# EUROPEAN PATENT APPLICATION

(11) **EP 2 123 752 A2**
(43) Date of publication of application: **25.11.2009**
(21) Application number: 07859120.3
(22) Date of filing: 18.12.2007
(51) Int. Cl.: C12N 15/09, A61K 31/7088, A61K 31/7105, A61K 31/713, A61K 35/76, A61K 48/00, A61P 9/10, A61P 11/00, A61P 13/08, A61P 13/12, A61P 29/00, A61P 35/00, A61P 37/00, A61P 37/08, C12N 1/15, C12N 1/19, C12N 1/21, C12N 5/10, C12Q 1/02, C12Q 1/68, G01N 33/15

(54) **NOVEL NUCLEIC ACID**

(30) Priority: 18.12.2006 JP 2006339997
(71) Applicant: Kyowa Hakko Kirin Co., Ltd., Tokyo 100-8185 (JP)
(72) Inventor: YAMADA, Yoji, Sunto-gun Shizuoka 411-8731 (JP); MIYAZAWA, Tatsuya, Machida-shi Tokyo 194-8533 (JP); YOSHIDA, Tetsuo, Machida-shi Tokyo 194-8533 (JP); NAKANO, Haruo, Machida-shi Tokyo 194-8533 (JP); KOSAKA, Kyoko, Machida-shi Tokyo 194-8533 (JP)
(74) Representative: Vossius & Partner
(86) International application number: PCT/IB2007/004005
(87) International publication number: WO 2008/084319

(57) **Abstract**

The present invention provides a nucleic acid such as a micro-RNA or a micro-RNA precursor, having a novel sequence. The nucleic acid of the present invention is useful for detecting the expression or a mutation of a micro-RNA, separating cells, suppressing the expression of a gene having a target sequence, screening for a substance that promotes or suppresses a function of a micro-RNA, and diagnosing or treating a disease caused by a mast cell abnormality, a disease caused by an abnormality of mesenchymal stem cell proliferation or differentiation, cancers, and a disease caused by abnormal proliferation of cells, tissue hyperplasia or the like.

SEQUENCE LISTING

<110> KYOWA HAKKO KIRIN CO., LTD.

<120> Novel nucleic acids

<130> 1911

<150> JP2006-339997
<151> 2006-12-18

<160> 2852

<170> PatentIn version 3.3

<210> 1
<211> 28
<212> RNA
<213> Homo sapiens

<400> 1
ccccccacaa ccgcgcuuga cuagcuug 28

<210> 2
<211> 19
<212> RNA
<213> Homo sapiens

<400> 2
gugaggggcc agauggcaa 19

<210> 3
<211> 21
<212> RNA
<213> Homo sapiens

<400> 3
uccccccaaa auucauaaug u 21

<210> 4
<211> 18
<212> RNA
<213> Homo sapiens

<400> 4
gcaggugagg uugugacg 18

<210> 5
<211> 17
<212> RNA
<213> Homo sapiens

<400> 5
gcagcugagc caagaag 17

<210> 6
<211> 17
<212> RNA
<213> Homo sapiens

<400> 6
uauaugugua auaagac 17

<210> 7
<211> 24
<212> RNA
<213> Homo sapiens

<400> 7

ugggcugaag ugauucuccu gccu 24

<210> 8
<211> 19
<212> RNA
<213> Homo sapiens

<400> 8
ugccugugcu cugcggucu 19

<210> 9
<211> 18
<212> RNA
<213> Homo sapiens

<400> 9
gcggagccgg agcccgag 18

<210> 10
<211> 18
<212> RNA
<213> Homo sapiens

<400> 10
cccgacgggg agccaaga 18

<210> 11
<211> 23
<212> RNA
<213> Homo sapiens

<400> 11
aaaaaccgcc auuacuuuug cac 23

<210> 12
<211> 17
<212> RNA
<213> Homo sapiens

<400> 12
agggcucaga gagaagg 17

<210> 13
<211> 18
<212> RNA
<213> Homo sapiens

<400> 13
aggccaucuu cagcaagg 18

<210> 14
<211> 22
<212> RNA
<213> Homo sapiens

<400> 14
auauaauaca accugcuaag ug 22

<210> 15
<211> 15
<212> RNA
<213> Homo sapiens

<400> 15
cggagaggga gccug 15

<210> 16
<211> 15
<212> RNA
<213> Homo sapiens

<400> 16
gaggaaugau gacaa 15

<210> 17
<211> 18
<212> RNA
<213> Homo sapiens

<400> 17
cagcggcacc agagagag 18

<210> 18
<211> 18
<212> RNA
<213> Homo sapiens

<400> 18
gcggcaagcu ggugucug 18

<210> 19
<211> 20
<212> RNA
<213> Homo sapiens

<400> 19
cuuccagacg cuccgcccca 20

<210> 20
<211> 21
<212> RNA
<213> Homo sapiens

<400> 20
ugaguguacu gugggcuucg g 21

<210> 21
<211> 24
<212> RNA
<213> Homo sapiens

<400> 21
uugcagcugc cugggaguga cuuc 24

<210> 22
<211> 19
<212> RNA
<213> Homo sapiens

<400> 22
ucuccccaag cuccagcgg 19

<210> 23
<211> 18
<212> RNA
<213> Homo sapiens

<400> 23
agguuucuaa ugagacaa 18

<210> 24
<211> 17
<212> RNA

<213>  Homo sapiens

<400>  24
ggcgggggcg gcgcgcg                                                    17


<210>  25
<211>  24
<212>  RNA
<213>  Homo sapiens

<400>  25
gacccgcggg cgcucuccag uccu                                            24


<210>  26
<211>  18
<212>  RNA
<213>  Homo sapiens

<400>  26
aucccuucgu gguugcca                                                   18


<210>  27
<211>  22
<212>  RNA
<213>  Homo sapiens

<400>  27
cacccggcug ugugcacaug ug                                              22


<210>  28
<211>  22
<212>  RNA
<213>  Homo sapiens

<400>  28
acucggcgug gcgucggucg ug                                              22


<210>  29
<211>  21
<212>  RNA
<213>  Homo sapiens

<400>  29
cugauguucg ccgaccguug a                                               21


<210>  30
<211>  22
<212>  RNA
<213>  Homo sapiens

<400>  30
aggagggcug agagggcccc ug                                              22


<210>  31
<211>  22
<212>  RNA
<213>  Homo sapiens

<400>  31
uugcagcugc cugggaguga cu                                              22


<210>  32
<211>  22
<212>  RNA
<213>  Homo sapiens

<400>  32

cgcuugacua gcuugcuguu uu                                                22

<210>  33
<211>  22
<212>  RNA
<213>  Homo sapiens

<400>  33
agcucugcug cucacuggca gu                                                22

<210>  34
<211>  22
<212>  RNA
<213>  Homo sapiens

<400>  34
gcagcugaug auacagcuuc uu                                                22

<210>  35
<211>  22
<212>  RNA
<213>  Homo sapiens

<400>  35
uccagcaggg cacgcacagc gu                                                22

<210>  36
<211>  22
<212>  RNA
<213>  Homo sapiens

<400>  36
ccaguuaccg cuuccgcuac cg                                                22

<210>  37
<211>  21
<212>  RNA
<213>  Homo sapiens

<400>  37
auuggccagu uuugucugau g                                                 21

<210>  38
<211>  21
<212>  RNA
<213>  Homo sapiens

<400>  38
uaugugccua guggcugcug u                                                 21

<210>  39
<211>  22
<212>  RNA
<213>  Homo sapiens

<400>  39
ucucucggcu ccucgcggcu cg                                                22

<210>  40
<211>  22
<212>  RNA
<213>  Homo sapiens

<400>  40
ccuccugaug auucuucuuc cu                                                22

<210> 41
<211> 22
<212> RNA
<213> Homo sapiens

<400> 41
uguccuugcu guuuggagau aa 22

<210> 42
<211> 22
<212> RNA
<213> Homo sapiens

<400> 42
accgucguuc ucaucggaac ug 22

<210> 43
<211> 22
<212> RNA
<213> Homo sapiens

<400> 43
auccccagau acaauggaca au 22

<210> 44
<211> 22
<212> RNA
<213> Homo sapiens

<400> 44
cuuccagacg cuccgcccca cg 22

<210> 45
<211> 22
<212> RNA
<213> Homo sapiens

<400> 45
ugacagcgcc cugccuggcu cg 22

<210> 46
<211> 22
<212> RNA
<213> Homo sapiens

<400> 46
ugccccaaca aggaaggaca ag 22

<210> 47
<211> 22
<212> RNA
<213> Homo sapiens

<400> 47
uauggagguc ucugucuggc uu 22

<210> 48
<211> 21
<212> RNA
<213> Homo sapiens

<400> 48
ccaaaacugc aguuacuuuu g 21

<210> 49
<211> 16
<212> RNA

<213> Homo sapiens

<400> 49
cggcggggcg cgggac 16

<210> 50
<211> 22
<212> RNA
<213> Homo sapiens

<400> 50
uucucacuac ugcacuugau ag 22

<210> 51
<211> 21
<212> RNA
<213> Homo sapiens

<400> 51
aaugaaucug aggaggcuac u 21

<210> 52
<211> 20
<212> RNA
<213> Homo sapiens

<400> 52
aagccaguuu cugucugaua 20

<210> 53
<211> 22
<212> RNA
<213> Homo sapiens

<400> 53
ccgcuaccgg cgcgacaggc cg 22

<210> 54
<211> 22
<212> RNA
<213> Homo sapiens

<400> 54
cuaaagagaa gucaaugcau ga 22

<210> 55
<211> 22
<212> RNA
<213> Homo sapiens

<400> 55
ugucuuuccu uguuggagca gg 22

<210> 56
<211> 21
<212> RNA
<213> Homo sapiens

<400> 56
aaucucuguc ucuuggaccu g 21

<210> 57
<211> 21
<212> RNA
<213> Homo sapiens

<400> 57

agaaguaauu gcgguuuugc c                                                21

<210>  58
<211>  16
<212>  RNA
<213>  Homo sapiens

<400>  58
ugggggcggg gagcgg                                                      16

<210>  59
<211>  22
<212>  RNA
<213>  Homo sapiens

<400>  59
aaucuaccuu gcaguguagg aa                                               22

<210>  60
<211>  22
<212>  RNA
<213>  Homo sapiens

<400>  60
cucggugccg gaagcggccg ug                                               22

<210>  61
<211>  22
<212>  RNA
<213>  Homo sapiens

<400>  61
ucaaggcccu gcugggaggg aa                                               22

<210>  62
<211>  21
<212>  RNA
<213>  Homo sapiens

<400>  62
cagacgcuca ggaaauagaa a                                                21

<210>  63
<211>  20
<212>  RNA
<213>  Homo sapiens

<400>  63
cgccgcuuuc ugggcucgcu                                                  20

<210>  64
<211>  20
<212>  RNA
<213>  Homo sapiens

<400>  64
gaugcagcuc ccuggggaca                                                  20

<210>  65
<211>  20
<212>  RNA
<213>  Homo sapiens

<400>  65
uuugcaguaa caggugugag                                                  20

<210> 66
<211> 22
<212> RNA
<213> Homo sapiens

<400> 66
aauagcucag aaugucaguu cu 22


<210> 67
<211> 22
<212> RNA
<213> Homo sapiens

<400> 67
auguauuccg cuucugaaca gg 22


<210> 68
<211> 22
<212> RNA
<213> Homo sapiens

<400> 68
ccacuccuga ccagcaggaa ug 22


<210> 69
<211> 22
<212> RNA
<213> Homo sapiens

<400> 69
ccuuugcaac acugagcaac ug 22


<210> 70
<211> 22
<212> RNA
<213> Homo sapiens

<400> 70
cucugcagga auaacggucc ug 22


<210> 71
<211> 22
<212> RNA
<213> Homo sapiens

<400> 71
cugguucugc cagcuccccu ga 22


<210> 72
<211> 22
<212> RNA
<213> Homo sapiens

<400> 72
cuguuuguug gagcccucca gg 22


<210> 73
<211> 22
<212> RNA
<213> Homo sapiens

<400> 73
cuuguaaagc auaguguuug cu 22


<210> 74
<211> 22
<212> RNA

<213>  Homo sapiens

<400>  74
guggggcugg ggcacccucc ga                                                22


<210>  75
<211>  22
<212>  RNA
<213>  Homo sapiens

<400>  75
ucauaaauuc uuugccaaga cu                                                22


<210>  76
<211>  22
<212>  RNA
<213>  Homo sapiens

<400>  76
ugcugcagug cucugagccc ua                                                22


<210>  77
<211>  22
<212>  RNA
<213>  Homo sapiens

<400>  77
uggucugugg acuucccaug cu                                                22


<210>  78
<211>  22
<212>  RNA
<213>  Homo sapiens

<400>  78
uugcaaguga gggugaagaa ga                                                22


<210>  79
<211>  22
<212>  RNA
<213>  Homo sapiens

<400>  79
uugggacagg gucuugcugu gu                                                22


<210>  80
<211>  22
<212>  RNA
<213>  Homo sapiens

<400>  80
uuucucauuu accucccugg aa                                                22


<210>  81
<211>  22
<212>  RNA
<213>  Homo sapiens

<400>  81
uuugugucuu gguuggcguc uu                                                22


<210>  82
<211>  22
<212>  RNA
<213>  Homo sapiens

<400>  82

uuuugcgagu acucaacacc aa                                                22

<210>  83
<211>  21
<212>  RNA
<213>  Homo sapiens

<400>  83
aaaaaguaau ugcuguuuuu g                                                 21

<210>  84
<211>  21
<212>  RNA
<213>  Homo sapiens

<400>  84
agacuucauu ccucuauguc u                                                 21

<210>  85
<211>  21
<212>  RNA
<213>  Homo sapiens

<400>  85
agcgccuggu gcgagaaauc g                                                 21

<210>  86
<211>  21
<212>  RNA
<213>  Homo sapiens

<400>  86
cggcuggagg gcuccucucu g                                                 21

<210>  87
<211>  21
<212>  RNA
<213>  Homo sapiens

<400>  87
gcgcccgccc gcccgcccgc c                                                 21

<210>  88
<211>  21
<212>  RNA
<213>  Homo sapiens

<400>  88
uucucggccu auccggaaug c                                                 21

<210>  89
<211>  21
<212>  RNA
<213>  Homo sapiens

<400>  89
uugcugggaa agggagaagu u                                                 21

<210>  90
<211>  20
<212>  RNA
<213>  Homo sapiens

<400>  90
aacuggagug uacccaaaac                                                   20

<210> 91
<211> 20
<212> RNA
<213> Homo sapiens

<400> 91
aacuguggac agauucucaa 20


<210> 92
<211> 20
<212> RNA
<213> Homo sapiens

<400> 92
ggugcuguug cugaaacgcu 20


<210> 93
<211> 20
<212> RNA
<213> Homo sapiens

<400> 93
ucucagccug gagucagggc 20


<210> 94
<211> 20
<212> RNA
<213> Homo sapiens

<400> 94
ucuuuucuuu gagacucacu 20


<210> 95
<211> 20
<212> RNA
<213> Homo sapiens

<400> 95
uggggacgua gcuggccaga 20


<210> 96
<211> 20
<212> RNA
<213> Homo sapiens

<400> 96
uuacuucagg gaguggcaga 20


<210> 97
<211> 20
<212> RNA
<213> Homo sapiens

<400> 97
uugccuuagg cuucuuucuu 20


<210> 98
<211> 19
<212> RNA
<213> Homo sapiens

<400> 98
aaaauccuuu uuguuuuuc 19


<210> 99
<211> 19
<212> RNA

<213>  Homo sapiens

<400>  99
aacucugccu cucccucug                                                    19


<210>  100
<211>  19
<212>  RNA
<213>  Homo sapiens

<400>  100
augcuugaug agaaugcca                                                    19


<210>  101
<211>  19
<212>  RNA
<213>  Homo sapiens

<400>  101
cagccuggag cucccugug                                                    19


<210>  102
<211>  19
<212>  RNA
<213>  Homo sapiens

<400>  102
uuuccucuuc uuaacacca                                                    19


<210>  103
<211>  18
<212>  RNA
<213>  Homo sapiens

<400>  103
aacucaguga gcucuugu                                                     18


<210>  104
<211>  18
<212>  RNA
<213>  Homo sapiens

<400>  104
acuguuuugu ggacaaau                                                     18


<210>  105
<211>  18
<212>  RNA
<213>  Homo sapiens

<400>  105
cucggugccu ggaaugag                                                     18


<210>  106
<211>  18
<212>  RNA
<213>  Homo sapiens

<400>  106
gccccggcgg cgggggca                                                     18


<210>  107
<211>  18
<212>  RNA
<213>  Homo sapiens

<400>  107

guaucccacu ucugacac 18

<210> 108
<211> 17
<212> RNA
<213> Homo sapiens

<400> 108
cagcuucagg auagguu 17

<210> 109
<211> 17
<212> RNA
<213> Homo sapiens

<400> 109
uuaaagccaa gaacacc 17

<210> 110
<211> 16
<212> RNA
<213> Homo sapiens

<400> 110
ucggagggaa gcugac 16

<210> 111
<211> 22
<212> RNA
<213> Homo sapiens

<400> 111
aacugguagu gucagccucc ug 22

<210> 112
<211> 22
<212> RNA
<213> Homo sapiens

<400> 112
agagcgagac uccaacucag aa 22

<210> 113
<211> 22
<212> RNA
<213> Homo sapiens

<400> 113
agucaccgac gagaccagaa gu 22

<210> 114
<211> 22
<212> RNA
<213> Homo sapiens

<400> 114
uccucgccca gggccaccga cu 22

<210> 115
<211> 22
<212> RNA
<213> Homo sapiens

<400> 115
uugggaaaga caaacucaga gu 22

<210> 116
<211> 21
<212> RNA
<213> Homo sapiens

<400> 116
acgaugagau guucuccgac a 21

<210> 117
<211> 21
<212> RNA
<213> Homo sapiens

<400> 117
auuuuguugc gagguucuga g 21

<210> 118
<211> 21
<212> RNA
<213> Homo sapiens

<400> 118
cagugccucg gcagugcagc c 21

<210> 119
<211> 21
<212> RNA
<213> Homo sapiens

<400> 119
uugugaagaa agaaauucuu a 21

<210> 120
<211> 20
<212> RNA
<213> Homo sapiens

<400> 120
agaaaugcug gacacaggaa 20

<210> 121
<211> 20
<212> RNA
<213> Homo sapiens

<400> 121
aggcggaacg auacggcagc 20

<210> 122
<211> 20
<212> RNA
<213> Homo sapiens

<400> 122
cauucguggg accgaguuuc 20

<210> 123
<211> 20
<212> RNA
<213> Homo sapiens

<400> 123
ucuccaggcc aaagccgcgu 20

<210> 124
<211> 19
<212> RNA

<213>  Homo sapiens

<400>  124
aaagaaucuu guuaugcuc                                                    19


<210>  125
<211>  19
<212>  RNA
<213>  Homo sapiens

<400>  125
aaggacgagg gaccgcacu                                                    19


<210>  126
<211>  17
<212>  RNA
<213>  Homo sapiens

<400>  126
aaagaaacag gagggac                                                      17


<210>  127
<211>  17
<212>  RNA
<213>  Homo sapiens

<400>  127
agcagcucuc agcauuc                                                      17


<210>  128
<211>  17
<212>  RNA
<213>  Homo sapiens

<400>  128
auaaaaacac agcacuc                                                      17


<210>  129
<211>  17
<212>  RNA
<213>  Homo sapiens

<400>  129
ccgacgagga agcgagg                                                      17


<210>  130
<211>  17
<212>  RNA
<213>  Homo sapiens

<400>  130
ugugcacaca aacuguu                                                      17


<210>  131
<211>  22
<212>  RNA
<213>  Homo sapiens

<400>  131
aaaaaccgca auuauuuuug cu                                                22


<210>  132
<211>  22
<212>  RNA
<213>  Homo sapiens

<400>  132

aaaccagagc uucccucauu ua 22

<210> 133
<211> 22
<212> RNA
<213> Homo sapiens

<400> 133
aacacggcgu gcgccuggug gg 22

<210> 134
<211> 22
<212> RNA
<213> Homo sapiens

<400> 134
aacagaggcc cugggugug cu 22

<210> 135
<211> 22
<212> RNA
<213> Homo sapiens

<400> 135
aacguccgag cgggcggccg ag 22

<210> 136
<211> 22
<212> RNA
<213> Homo sapiens

<400> 136
aacgugacag ugacugugag aa 22

<210> 137
<211> 22
<212> RNA
<213> Homo sapiens

<400> 137
aagagcuuca guguuaggac ug 22

<210> 138
<211> 22
<212> RNA
<213> Homo sapiens

<400> 138
aagcggcuuu uggaaagugg cu 22

<210> 139
<211> 22
<212> RNA
<213> Homo sapiens

<400> 139
aaguggcaca gccagggauu ga 22

<210> 140
<211> 22
<212> RNA
<213> Homo sapiens

<400> 140
agaagcugag cugacuaugc cu 22

<210> 141
<211> 22
<212> RNA
<213> Homo sapiens

<400> 141
agcgguuagg cguacggcca gg 22


<210> 142
<211> 22
<212> RNA
<213> Homo sapiens

<400> 142
agcugugguc ucugagagac ug 22


<210> 143
<211> 22
<212> RNA
<213> Homo sapiens

<400> 143
aggaagcgag uguguauuuc ug 22


<210> 144
<211> 22
<212> RNA
<213> Homo sapiens

<400> 144
aggcgcugcu gcgggccgug cg 22


<210> 145
<211> 22
<212> RNA
<213> Homo sapiens

<400> 145
aguacgugga gccacauaca ua 22


<210> 146
<211> 22
<212> RNA
<213> Homo sapiens

<400> 146
aguggaccgu ugaggagagc ga 22


<210> 147
<211> 22
<212> RNA
<213> Homo sapiens

<400> 147
aguuuuguuu cucuuucucc uu 22


<210> 148
<211> 22
<212> RNA
<213> Homo sapiens

<400> 148
aucauaccau uccuuggaug aa 22


<210> 149
<211> 22
<212> RNA

<213>  Homo sapiens

<400>  149
aucccuggcu gcuaagcccu ug                                                22


<210>  150
<211>  22
<212>  RNA
<213>  Homo sapiens

<400>  150
augcauuuuc ucugugaggc au                                                22


<210>  151
<211>  22
<212>  RNA
<213>  Homo sapiens

<400>  151
augcugcugc acucaaucuu gg                                                22


<210>  152
<211>  22
<212>  RNA
<213>  Homo sapiens

<400>  152
augggcuuug gucuuuuuga cu                                                22


<210>  153
<211>  22
<212>  RNA
<213>  Homo sapiens

<400>  153
augugcagga gcugccucug ga                                                22


<210>  154
<211>  22
<212>  RNA
<213>  Homo sapiens

<400>  154
auguuaugau gcacagcaga ag                                                22


<210>  155
<211>  22
<212>  RNA
<213>  Homo sapiens

<400>  155
auugaauaag aauggggaga gu                                                22


<210>  156
<211>  22
<212>  RNA
<213>  Homo sapiens

<400>  156
cacaggcccu ggaggucucc cu                                                22


<210>  157
<211>  22
<212>  RNA
<213>  Homo sapiens

<400>  157

cagacucgac cucccaggcu ua                                                22

<210>  158
<211>  22
<212>  RNA
<213>  Homo sapiens

<400>  158
cagagaugug gcugugucua gu                                                22

<210>  159
<211>  22
<212>  RNA
<213>  Homo sapiens

<400>  159
cagcaaaagu cagcuggaua uu                                                22

<210>  160
<211>  22
<212>  RNA
<213>  Homo sapiens

<400>  160
caggggacac ggaugccagg aa                                                22

<210>  161
<211>  22
<212>  RNA
<213>  Homo sapiens

<400>  161
caucgcccug cuggagcugg ag                                                22

<210>  162
<211>  22
<212>  RNA
<213>  Homo sapiens

<400>  162
ccaaccuugu gaggcugagc ug                                                22

<210>  163
<211>  22
<212>  RNA
<213>  Homo sapiens

<400>  163
ccaugcacug uugugaggaa aa                                                22

<210>  164
<211>  22
<212>  RNA
<213>  Homo sapiens

<400>  164
cccgguuccg ccugcggcuc ug                                                22

<210>  165
<211>  22
<212>  RNA
<213>  Homo sapiens

<400>  165
cgagacagua guucuugccu gg                                                22

<210> 166
<211> 22
<212> RNA
<213> Homo sapiens

<400> 166
   cgcuuccugg uggguccagu gu 22

<210> 167
<211> 22
<212> RNA
<213> Homo sapiens

<400> 167
cuaucaggac aggaauccag cu 22

<210> 168
<211> 22
<212> RNA
<213> Homo sapiens

<400> 168
cuccugaaac uuugcuguaa gu 22

<210> 169
<211> 22
<212> RNA
<213> Homo sapiens

<400> 169
cugcugcugc gggcacgccg ag 22

<210> 170
<211> 22
<212> RNA
<213> Homo sapiens

<400> 170
cuguguguga gaaauggcag gu 22

<210> 171
<211> 22
<212> RNA
<213> Homo sapiens

<400> 171
cuugcugaca cuuugucauc cg 22

<210> 172
<211> 22
<212> RNA
<213> Homo sapiens

<400> 172
cuuguuuggg ucuugagugu ag 22

<210> 173
<211> 22
<212> RNA
<213> Homo sapiens

<400> 173
gaagacagac ccccagacag gg 22

<210> 174
<211> 22
<212> RNA

<213>  Homo sapiens

<400>  174
gacccguggg acgcggcuga ga                                                22


<210>  175
<211>  22
<212>  RNA
<213>  Homo sapiens

<400>  175
gcacucugug ggccuggagg gu                                                22


<210>  176
<211>  22
<212>  RNA
<213>  Homo sapiens

<400>  176
gcccugcggg gccuaccgcc ug                                                22


<210>  177
<211>  22
<212>  RNA
<213>  Homo sapiens

<400>  177
gccgcuucug cuggcugagc ug                                                22


<210>  178
<211>  22
<212>  RNA
<213>  Homo sapiens

<400>  178
gccgggcacg guggcucacu uu                                                22


<210>  179
<211>  22
<212>  RNA
<213>  Homo sapiens

<400>  179
gguggccuga gcaguuccac ga                                                22


<210>  180
<211>  22
<212>  RNA
<213>  Homo sapiens

<400>  180
guucugccuc uguccagguc cu                                                22


<210>  181
<211>  22
<212>  RNA
<213>  Homo sapiens

<400>  181
ucaguugaau augagcuaua cu                                                22


<210>  182
<211>  22
<212>  RNA
<213>  Homo sapiens

<400>  182

uccuuucugu ggggcugagu cu                                                22

<210>  183
<211>  22
<212>  RNA
<213>  Homo sapiens

<400>  183
ugagugacuc uggacuggag aa                                                22

<210>  184
<211>  22
<212>  RNA
<213>  Homo sapiens

<400>  184
ugcuacuuuc acugcuauag ua                                                22

<210>  185
<211>  22
<212>  RNA
<213>  Homo sapiens

<400>  185
ugcuauguuc ucauccuaca gu                                                22

<210>  186
<211>  22
<212>  RNA
<213>  Homo sapiens

<400>  186
uggucacagu gccagcuuac uu                                                22

<210>  187
<211>  22
<212>  RNA
<213>  Homo sapiens

<400>  187
uguccucuag ggccugcagu cu                                                22

<210>  188
<211>  22
<212>  RNA
<213>  Homo sapiens

<400>  188
uugauuucag cauuuaacug ag                                                22

<210>  189
<211>  22
<212>  RNA
<213>  Homo sapiens

<400>  189
uugcaugugg aaaugucugc uu                                                22

<210>  190
<211>  22
<212>  RNA
<213>  Homo sapiens

<400>  190
uuuccggcuc gcgugggugu gu                                                22

<210>  191
<211>  21
<212>  RNA
<213>  Homo sapiens

<400>  191
aaaaguauuu gcggguuuug u                                                21


<210>  192
<211>  21
<212>  RNA
<213>  Homo sapiens

<400>  192
aaagcucucg gucaaguuca a                                                21


<210>  193
<211>  21
<212>  RNA
<213>  Homo sapiens

<400>  193
aagagccuuc uggccagccu g                                                21


<210>  194
<211>  21
<212>  RNA
<213>  Homo sapiens

<400>  194
accugaauua ccaaaagcuu u                                                21


<210>  195
<211>  21
<212>  RNA
<213>  Homo sapiens

<400>  195
agucacaauc ccuggaggaa g                                                21


<210>  196
<211>  21
<212>  RNA
<213>  Homo sapiens

<400>  196
augaguuugg ggaaagaguu g                                                21


<210>  197
<211>  21
<212>  RNA
<213>  Homo sapiens

<400>  197
auucucuggu cagcagacag g                                                21


<210>  198
<211>  21
<212>  RNA
<213>  Homo sapiens

<400>  198
cauuguaagu uaggaugcau g                                                21


<210>  199
<211>  21
<212>  RNA

<213>  Homo sapiens

<400>  199
ccccugggcu guuacuguuc c                                                21


<210>  200
<211>  21
<212>  RNA
<213>  Homo sapiens

<400>  200
cccuccuacc uuuucuccua a                                                21


<210>  201
<211>  21
<212>  RNA
<213>  Homo sapiens

<400>  201
cgaaaacagc aauuaccuuu g                                                21


<210>  202
<211>  21
<212>  RNA
<213>  Homo sapiens

<400>  202
gcucauuuaa guagucugau g                                                21


<210>  203
<211>  21
<212>  RNA
<213>  Homo sapiens

<400>  203
ucaucaggag caaaugauaa a                                                21


<210>  204
<211>  21
<212>  RNA
<213>  Homo sapiens

<400>  204
ucaugcccag uugcuuccug u                                                21


<210>  205
<211>  21
<212>  RNA
<213>  Homo sapiens

<400>  205
uccuggacag gcaccuccag g                                                21


<210>  206
<211>  21
<212>  RNA
<213>  Homo sapiens

<400>  206
ucucccuacu aucuucccua g                                                21


<210>  207
<211>  21
<212>  RNA
<213>  Homo sapiens

<400>  207

ugaagacccu gaccggcaag a                                                21

<210>  208
<211>  21
<212>  RNA
<213>  Homo sapiens

<400>  208
ugacgauuga gcacagucag g                                                21

<210>  209
<211>  21
<212>  RNA
<213>  Homo sapiens

<400>  209
ugccguguguu cuugcauaau u                                                21

<210>  210
<211>  21
<212>  RNA
<213>  Homo sapiens

<400>  210
ugcgggcacg aggagacggc c                                                21

<210>  211
<211>  21
<212>  RNA
<213>  Homo sapiens

<400>  211
uuaugccgga ugugcuuuuc u                                                21

<210>  212
<211>  21
<212>  RNA
<213>  Homo sapiens

<400>  212
uugccuuccu gaaaaguuuu u                                                21

<210>  213
<211>  20
<212>  RNA
<213>  Homo sapiens

<400>  213
aaaagucccu cugagcuuac                                                20

<210>  214
<211>  20
<212>  RNA
<213>  Homo sapiens

<400>  214
aacagaguga gacugugucg                                                20

<210>  215
<211>  20
<212>  RNA
<213>  Homo sapiens

<400>  215
aauggauugc ugguugugga                                                20

<210> 216
<211> 20
<212> RNA
<213> Homo sapiens

<400> 216
augcugccuu uggucucuga 20


<210> 217
<211> 20
<212> RNA
<213> Homo sapiens

<400> 217
cagguugcug ggcuuugccc 20


<210> 218
<211> 20
<212> RNA
<213> Homo sapiens

<400> 218
gagcagguuu ccgugaagcc 20


<210> 219
<211> 20
<212> RNA
<213> Homo sapiens

<400> 219
ucgcgguuug ugccagauga 20


<210> 220
<211> 20
<212> RNA
<213> Homo sapiens

<400> 220
ugucccgcug cguguuuucc 20


<210> 221
<211> 19
<212> RNA
<213> Homo sapiens

<400> 221
aacaggagaa aguuacuga 19


<210> 222
<211> 19
<212> RNA
<213> Homo sapiens

<400> 222
agcgagcgga gccgcgguc 19


<210> 223
<211> 19
<212> RNA
<213> Homo sapiens

<400> 223
agguaaauua auacucagc 19


<210> 224
<211> 19
<212> RNA

<213>  Homo sapiens

<400>  224
agucggcguc uccauccug                                                    19


<210>  225
<211>  19
<212>  RNA
<213>  Homo sapiens

<400>  225
aucugaaaca cuuccuaug                                                    19


<210>  226
<211>  19
<212>  RNA
<213>  Homo sapiens

<400>  226
aucugggugc cacagugga                                                    19


<210>  227
<211>  19
<212>  RNA
<213>  Homo sapiens

<400>  227
cuucuucagg ccgaauagg                                                    19


<210>  228
<211>  19
<212>  RNA
<213>  Homo sapiens

<400>  228
cuuuucauag agcaguuag                                                    19


<210>  229
<211>  19
<212>  RNA
<213>  Homo sapiens

<400>  229
uggccggcgg cgaccucgg                                                    19


<210>  230
<211>  19
<212>  RNA
<213>  Homo sapiens

<400>  230
uucuugugcu aagcagaac                                                    19


<210>  231
<211>  19
<212>  RNA
<213>  Homo sapiens

<400>  231
uugcugauuc uacugccaa                                                    19


<210>  232
<211>  18
<212>  RNA
<213>  Homo sapiens

<400>  232

aacuuggacg ucagagaa 18

<210> 233
<211> 18
<212> RNA
<213> Homo sapiens

<400> 233
aaggauguuc gucguggc 18

<210> 234
<211> 18
<212> RNA
<213> Homo sapiens

<400> 234
aaggcuuuaa cugagaac 18

<210> 235
<211> 18
<212> RNA
<213> Homo sapiens

<400> 235
agcagugaau guggauga 18

<210> 236
<211> 18
<212> RNA
<213> Homo sapiens

<400> 236
auaugagagg caggcuga 18

<210> 237
<211> 18
<212> RNA
<213> Homo sapiens

<400> 237
aucucccagu ucuguguc 18

<210> 238
<211> 18
<212> RNA
<213> Homo sapiens

<400> 238
aucucugugu gugaacug 18

<210> 239
<211> 18
<212> RNA
<213> Homo sapiens

<400> 239
cccucgaaag gacaucug 18

<210> 240
<211> 18
<212> RNA
<213> Homo sapiens

<400> 240
cucugcuggc caguguga 18

<210> 241
<211> 18
<212> RNA
<213> Homo sapiens

<400> 241
gaauggucuu agcacucu 18

<210> 242
<211> 18
<212> RNA
<213> Homo sapiens

<400> 242
gggagguaca gaauuggc 18

<210> 243
<211> 18
<212> RNA
<213> Homo sapiens

<400> 243
gggugcugua ggcuuuga 18

<210> 244
<211> 18
<212> RNA
<213> Homo sapiens

<400> 244
uccacggcau cugcgucu 18

<210> 245
<211> 18
<212> RNA
<213> Homo sapiens

<400> 245
uccgaggccu cagcgcag 18

<210> 246
<211> 18
<212> RNA
<213> Homo sapiens

<400> 246
ugaggaauuu ggccagac 18

<210> 247
<211> 18
<212> RNA
<213> Homo sapiens

<400> 247
ugcaaaccag ccagaaau 18

<210> 248
<211> 18
<212> RNA
<213> Homo sapiens

<400> 248
uggcuaagau acugcuga 18

<210> 249
<211> 17
<212> RNA

<213>  Homo sapiens

<400>  249
aagggcugga uugaggc                                                    17


<210>  250
<211>  17
<212>  RNA
<213>  Homo sapiens

<400>  250
agaaauuuug guuaaau                                                    17


<210>  251
<211>  17
<212>  RNA
<213>  Homo sapiens

<400>  251
caagagaaga gcucaac                                                    17


<210>  252
<211>  17
<212>  RNA
<213>  Homo sapiens

<400>  252
cagucccaag gguuugg                                                    17


<210>  253
<211>  17
<212>  RNA
<213>  Homo sapiens

<400>  253
cggcauggca aaggaaa                                                    17


<210>  254
<211>  17
<212>  RNA
<213>  Homo sapiens

<400>  254
cugaagggcc uugacga                                                    17


<210>  255
<211>  17
<212>  RNA
<213>  Homo sapiens

<400>  255
cuggcggggc ggggcgg                                                    17


<210>  256
<211>  17
<212>  RNA
<213>  Homo sapiens

<400>  256
guagcaccgu ggaaagc                                                    17


<210>  257
<211>  17
<212>  RNA
<213>  Homo sapiens

<400>  257

uacuccaaga guuaggg 17

<210> 258
<211> 16
<212> RNA
<213> Homo sapiens

<400> 258
cgggcggcgg aggggc 16

<210> 259
<211> 16
<212> RNA
<213> Homo sapiens

<400> 259
cuucaugggc cagaaa 16

<210> 260
<211> 16
<212> RNA
<213> Homo sapiens

<400> 260
gccaaagccu ccacca 16

<210> 261
<211> 15
<212> RNA
<213> Homo sapiens

<400> 261
agacuacacu cucag 15

<210> 262
<211> 22
<212> RNA
<213> Homo sapiens

<400> 262
aaaugcaaca guagauagca ga 22

<210> 263
<211> 22
<212> RNA
<213> Homo sapiens

<400> 263
aagcuuacug cucuggguua ga 22

<210> 264
<211> 22
<212> RNA
<213> Homo sapiens

<400> 264
aaugugugcu cuguaaguuu gu 22

<210> 265
<211> 22
<212> RNA
<213> Homo sapiens

<400> 265
accucugagc ucacucgcug gu 22

<210>  266
<211>  22
<212>  RNA
<213>  Homo sapiens

<400>  266
agaacgcugu ccgcagcuug cg                                                22


<210>  267
<211>  22
<212>  RNA
<213>  Homo sapiens

<400>  267
agagccauuu cacugcucgg uu                                                22


<210>  268
<211>  22
<212>  RNA
<213>  Homo sapiens

<400>  268
agagcgaggc cagccuagag gu                                                22


<210>  269
<211>  22
<212>  RNA
<213>  Homo sapiens

<400>  269
agagcucggc ugugauggcg cu                                                22


<210>  270
<211>  22
<212>  RNA
<213>  Homo sapiens

<400>  270
agcagcugag uggcuauuuc cu                                                22


<210>  271
<211>  22
<212>  RNA
<213>  Homo sapiens

<400>  271
aggacaaguu ugagaauuuu ug                                                22


<210>  272
<211>  22
<212>  RNA
<213>  Homo sapiens

<400>  272
aggccgccag gggggccggc ug                                                22


<210>  273
<211>  22
<212>  RNA
<213>  Homo sapiens

<400>  273
aggcugggcu cgaacuccug ga                                                22


<210>  274
<211>  22
<212>  RNA

<213>  Homo sapiens

<400>  274
agggcggugu cugagccugc cg                                                22


<210>  275
<211>  22
<212>  RNA
<213>  Homo sapiens

<400>  275
aguccuguug uuugcgagca aa                                                22


<210>  276
<211>  22
<212>  RNA
<213>  Homo sapiens

<400>  276
agugccacgu gucugggcca cu                                                22


<210>  277
<211>  22
<212>  RNA
<213>  Homo sapiens

<400>  277
aguguuuuug uccuuugggg ua                                                22


<210>  278
<211>  22
<212>  RNA
<213>  Homo sapiens

<400>  278
auccugcuga agcuuggaac uu                                                22


<210>  279
<211>  22
<212>  RNA
<213>  Homo sapiens

<400>  279
aucuuacugu cauuguccua aa                                                22


<210>  280
<211>  22
<212>  RNA
<213>  Homo sapiens

<400>  280
augaccuaag accuguucuu aa                                                22


<210>  281
<211>  22
<212>  RNA
<213>  Homo sapiens

<400>  281
augcugagcc ugcgaggccu ga                                                22


<210>  282
<211>  22
<212>  RNA
<213>  Homo sapiens

<400>  282

auuaggagug cucuguuguu ua                                                22

<210>  283
<211>  22
<212>  RNA
<213>  Homo sapiens

<400>  283
auugggcgcc uggucaccag gg                                                22

<210>  284
<211>  22
<212>  RNA
<213>  Homo sapiens

<400>  284
caagcuuggc ugcuuuccua ga                                                22

<210>  285
<211>  22
<212>  RNA
<213>  Homo sapiens

<400>  285
caucggcuga gagggcguag ga                                                22

<210>  286
<211>  22
<212>  RNA
<213>  Homo sapiens

<400>  286
ccuguaggca gugcuaaaaa au                                                22

<210>  287
<211>  22
<212>  RNA
<213>  Homo sapiens

<400>  287
cugcuuucuc cucuggcggg ag                                                22

<210>  288
<211>  22
<212>  RNA
<213>  Homo sapiens

<400>  288
cuucaugaac ccccuugccca cu                                                22

<210>  289
<211>  22
<212>  RNA
<213>  Homo sapiens

<400>  289
gaccggcagc acaggagaga uu                                                22

<210>  290
<211>  22
<212>  RNA
<213>  Homo sapiens

<400>  290
gacuguggcg gcggcggccu cg                                                22

<210> 291
<211> 22
<212> RNA
<213> Homo sapiens

<400> 291
gaggcugcag agcaucgugg ag 22


<210> 292
<211> 22
<212> RNA
<213> Homo sapiens

<400> 292
gaguggcaaa gucuuuccau au 22


<210> 293
<211> 22
<212> RNA
<213> Homo sapiens

<400> 293
gcggaggccu ggggcugcag cu 22


<210> 294
<211> 22
<212> RNA
<213> Homo sapiens

<400> 294
ggaaucaucc ugagagcuuc cu 22


<210> 295
<211> 22
<212> RNA
<213> Homo sapiens

<400> 295
ggagcuggag gugcuggaga gg 22


<210> 296
<211> 22
<212> RNA
<213> Homo sapiens

<400> 296
uaugaaccgu gcguguuuga uu 22


<210> 297
<211> 22
<212> RNA
<213> Homo sapiens

<400> 297
ugucaaacga ggcuucuguu uu 22


<210> 298
<211> 22
<212> RNA
<213> Homo sapiens

<400> 298
uguuuaaccu uuugccuguu gg 22


<210> 299
<211> 22
<212> RNA

<213>  Homo sapiens

<400>  299
uugggcagcc agaggaaugc au                                              22


<210>  300
<211>  22
<212>  RNA
<213>  Homo sapiens

<400>  300
uuggggaaac ggccgcugag ug                                              22


<210>  301
<211>  21
<212>  RNA
<213>  Homo sapiens

<400>  301
aaaauccugu cacugcaaug g                                               21


<210>  302
<211>  21
<212>  RNA
<213>  Homo sapiens

<400>  302
aaugccugga aacuuggugc u                                               21


<210>  303
<211>  21
<212>  RNA
<213>  Homo sapiens

<400>  303
accucguggg uccugaacug a                                               21


<210>  304
<211>  21
<212>  RNA
<213>  Homo sapiens

<400>  304
acugacccug cuuggugggg u                                               21


<210>  305
<211>  21
<212>  RNA
<213>  Homo sapiens

<400>  305
acuugaggau aggguuaaag u                                               21


<210>  306
<211>  21
<212>  RNA
<213>  Homo sapiens

<400>  306
aggagcugag cauuugugcu u                                               21


<210>  307
<211>  21
<212>  RNA
<213>  Homo sapiens

<400>  307

aggcccgagc auucugaagu g                                                21

<210>  308
<211>  21
<212>  RNA
<213>  Homo sapiens

<400>  308
aggcuggugc aaaaguaaug g                                                21

<210>  309
<211>  21
<212>  RNA
<213>  Homo sapiens

<400>  309
agggcccucc aggcccuccg g                                                21

<210>  310
<211>  21
<212>  RNA
<213>  Homo sapiens

<400>  310
agggcgggug ggcugaccac c                                                21

<210>  311
<211>  21
<212>  RNA
<213>  Homo sapiens

<400>  311
agucuccccu cauggaaggc c                                                21

<210>  312
<211>  21
<212>  RNA
<213>  Homo sapiens

<400>  312
agugccugag ggaguaagag c                                                21

<210>  313
<211>  21
<212>  RNA
<213>  Homo sapiens

<400>  313
aucuguuucg ugaggcucuc a                                                21

<210>  314
<211>  21
<212>  RNA
<213>  Homo sapiens

<400>  314
augagagcgu uugccgacgc a                                                21

<210>  315
<211>  21
<212>  RNA
<213>  Homo sapiens

<400>  315
cacucagggg aggcgggaaa g                                                21

<210> 316
<211> 21
<212> RNA
<213> Homo sapiens

<400> 316
cccggcgggc cgcccgccuc c 21


<210> 317
<211> 21
<212> RNA
<213> Homo sapiens

<400> 317
gagggcccuu ccgcggcccg g 21


<210> 318
<211> 21
<212> RNA
<213> Homo sapiens

<400> 318
gauggagucu ugcucuuugc c 21


<210> 319
<211> 21
<212> RNA
<213> Homo sapiens

<400> 319
gcacgacgau gaggugacag u 21


<210> 320
<211> 21
<212> RNA
<213> Homo sapiens

<400> 320
   gcuaccucuc ugggiucugu u 21


<210> 321
<211> 21
<212> RNA
<213> Homo sapiens

<400> 321
gcugcuuggu cuguuuacaa u 21


<210> 322
<211> 21
<212> RNA
<213> Homo sapiens

<400> 322
gcuuguggcu cuucugccug c 21


<210> 323
<211> 21
<212> RNA
<213> Homo sapiens

<400> 323
ggcgugggcc uuuucucugc a 21


<210> 324
<211> 21
<212> RNA

<213>  Homo sapiens

<400>  324
gggcgaguga cagccccggc u                                                21


<210>  325
<211>  21
<212>  RNA
<213>  Homo sapiens

<400>  325
guccaugcgc uccucgcggg c                                                21


<210>  326
<211>  21
<212>  RNA
<213>  Homo sapiens

<400>  326
uccguccgcg uguggaaggu c                                                21


<210>  327
<211>  21
<212>  RNA
<213>  Homo sapiens

<400>  327
ucuuugcugu ugugaacaau g                                                21


<210>  328
<211>  21
<212>  RNA
<213>  Homo sapiens

<400>  328
ugcucagcuc ugaccauguc u                                                21


<210>  329
<211>  21
<212>  RNA
<213>  Homo sapiens

<400>  329
uucuccgugg gcuacuagcg g                                                21


<210>  330
<211>  21
<212>  RNA
<213>  Homo sapiens

<400>  330
uucuccugac cuggggaguc g                                                21


<210>  331
<211>  21
<212>  RNA
<213>  Homo sapiens

<400>  331
uucuucucca gcaccugcag a                                                21


<210>  332
<211>  21
<212>  RNA
<213>  Homo sapiens

<400>  332

uuggccuugg cgcgggccuu g                                                21

<210>  333
<211>  21
<212>  RNA
<213>  Homo sapiens

<400>  333
uuucagaaau ugggagaagu g                                                21

<210>  334
<211>  20
<212>  RNA
<213>  Homo sapiens

<400>  334
aagauguuuc guguagaaag                                                  20

<210>  335
<211>  20
<212>  RNA
<213>  Homo sapiens

<400>  335
aagugaggga acugagauuu                                                  20

<210>  336
<211>  20
<212>  RNA
<213>  Homo sapiens

<400>  336
agcacucugg gauuuggcug                                                  20

<210>  337
<211>  20
<212>  RNA
<213>  Homo sapiens

<400>  337
aggggaugug agcauaugua                                                  20

<210>  338
<211>  20
<212>  RNA
<213>  Homo sapiens

<400>  338
aucgacgauu guccaggaaa                                                  20

<210>  339
<211>  20
<212>  RNA
<213>  Homo sapiens

<400>  339
augcaucucu uggcuguacu                                                  20

<210>  340
<211>  20
<212>  RNA
<213>  Homo sapiens

<400>  340
augcucaaua uucccagaau                                                  20

<210> 341
<211> 20
<212> RNA
<213> Homo sapiens

<400> 341
augggcuauu uuaaaaaauc 20

<210> 342
<211> 20
<212> RNA
<213> Homo sapiens

<400> 342
auucggagau ugcauaguag 20

<210> 343
<211> 20
<212> RNA
<213> Homo sapiens

<400> 343
cagccuccgg cagacacagg 20

<210> 344
<211> 20
<212> RNA
<213> Homo sapiens

<400> 344
cccucacaca gcugggagaa 20

<210> 345
<211> 20
<212> RNA
<213> Homo sapiens

<400> 345
ccuguguuaa ggagcaaccu 20

<210> 346
<211> 20
<212> RNA
<213> Homo sapiens

<400> 346
cugccaguca ggcaggaaug 20

<210> 347
<211> 20
<212> RNA
<213> Homo sapiens

<400> 347
ggcgcugccc gcugucacuc 20

<210> 348
<211> 20
<212> RNA
<213> Homo sapiens

<400> 348
uaacccaaac uagaguuguu 20

<210> 349
<211> 20
<212> RNA

<213>  Homo sapiens

<400>  349
uccccaggca ggcuuuggag                                                    20


<210>  350
<211>  20
<212>  RNA
<213>  Homo sapiens

<400>  350
uccugaccug gcaccugccc                                                    20


<210>  351
<211>  20
<212>  RNA
<213>  Homo sapiens

<400>  351
ucuaccugag gcuguugguc                                                    20


<210>  352
<211>  20
<212>  RNA
<213>  Homo sapiens

<400>  352
ugcucacuuc uuccugggga                                                    20


<210>  353
<211>  19
<212>  RNA
<213>  Homo sapiens

<400>  353
aagcacaggg cagcgagag                                                     19


<210>  354
<211>  19
<212>  RNA
<213>  Homo sapiens

<400>  354
accagaggag ccccaaaac                                                     19


<210>  355
<211>  19
<212>  RNA
<213>  Homo sapiens

<400>  355
augcugguca uugaugauu                                                     19


<210>  356
<211>  19
<212>  RNA
<213>  Homo sapiens

<400>  356
cagggaaugg uggcucacg                                                     19


<210>  357
<211>  19
<212>  RNA
<213>  Homo sapiens

<400>  357

ccuccacgcg uuccagguc                                                19

<210>  358
<211>  19
<212>  RNA
<213>  Homo sapiens

<400>  358
gcugcagcug cugggaugc                                                19

<210>  359
<211>  19
<212>  RNA
<213>  Homo sapiens

<400>  359
uccaccgcug agaguugaa                                                19

<210>  360
<211>  19
<212>  RNA
<213>  Homo sapiens

<400>  360
ugucccaucu gggucgcca                                                19

<210>  361
<211>  19
<212>  RNA
<213>  Homo sapiens

<400>  361
uugccugcuc ucuguggaa                                                19

<210>  362
<211>  18
<212>  RNA
<213>  Homo sapiens

<400>  362
aauaaacaug guguaucu                                                 18

<210>  363
<211>  18
<212>  RNA
<213>  Homo sapiens

<400>  363
aauguucuug uuacaaaa                                                 18

<210>  364
<211>  18
<212>  RNA
<213>  Homo sapiens

<400>  364
acugaagacu ggagcgcu                                                 18

<210>  365
<211>  18
<212>  RNA
<213>  Homo sapiens

<400>  365
agguugcuga auagacga                                                 18

<210> 366
<211> 18
<212> RNA
<213> Homo sapiens

<400> 366
augaagcaca ggggucga 18


<210> 367
<211> 18
<212> RNA
<213> Homo sapiens

<400> 367
cagagucaga aagcuaaa 18


<210> 368
<211> 18
<212> RNA
<213> Homo sapiens

<400> 368
cugugccgcg caguuagg 18


<210> 369
<211> 18
<212> RNA
<213> Homo sapiens

<400> 369
gaggagccug uucuaacc 18


<210> 370
<211> 18
<212> RNA
<213> Homo sapiens

<400> 370
gcauaagcua uuugcggc 18


<210> 371
<211> 18
<212> RNA
<213> Homo sapiens

<400> 371
gcucuggggc ggcauugc 18


<210> 372
<211> 18
<212> RNA
<213> Homo sapiens

<400> 372
ggcuggaaga gcaacaga 18


<210> 373
<211> 18
<212> RNA
<213> Homo sapiens

<400> 373
uaguucuugu ugcucuac 18


<210> 374
<211> 18
<212> RNA

<213>  Homo sapiens

<400>  374
uuggauuggc auggcggc                                                    18


<210>  375
<211>  18
<212>  RNA
<213>  Homo sapiens

<400>  375
uugucucucu guuucuca                                                    18


<210>  376
<211>  17
<212>  RNA
<213>  Homo sapiens

<400>  376
aaaaacaacg accuggg                                                     17


<210>  377
<211>  17
<212>  RNA
<213>  Homo sapiens

<400>  377
aaaccuacug ucucugu                                                     17


<210>  378
<211>  17
<212>  RNA
<213>  Homo sapiens

<400>  378
ccauacuugg ggauggg                                                     17


<210>  379
<211>  17
<212>  RNA
<213>  Homo sapiens

<400>  379
ccugaaauga ugacucu                                                     17


<210>  380
<211>  17
<212>  RNA
<213>  Homo sapiens

<400>  380
ggcgggcggc ggagggg                                                     17


<210>  381
<211>  16
<212>  RNA
<213>  Homo sapiens

<400>  381
aggaagcgcu ucaguc                                                      16


<210>  382
<211>  16
<212>  RNA
<213>  Homo sapiens

<400>  382

ccuacugggg gcacca 16

<210> 383
<211> 16
<212> RNA
<213> Homo sapiens

<400> 383
ucuuacucaa aggcaa 16

<210> 384
<211> 15
<212> RNA
<213> Homo sapiens

<400> 384
ccuggaggua ccacc 15

<210> 385
<211> 15
<212> RNA
<213> Homo sapiens

<400> 385
cgugcguccc uacag 15

<210> 386
<211> 22
<212> RNA
<213> Homo sapiens

<400> 386
aaaugacgug gcagcagcag ug 22

<210> 387
<211> 22
<212> RNA
<213> Homo sapiens

<400> 387
aaauuuuggu gaaguauuaa au 22

<210> 388
<211> 22
<212> RNA
<213> Homo sapiens

<400> 388
aaccugcguc ccaagaagaa gg 22

<210> 389
<211> 22
<212> RNA
<213> Homo sapiens

<400> 389
aacggccugc gcagggugcu gg 22

<210> 390
<211> 22
<212> RNA
<213> Homo sapiens

<400> 390
aacucccacu guguggacug cg 22

<210> 391
<211> 22
<212> RNA
<213> Homo sapiens

<400> 391
aacuugacaa gacugcugca aa 22


<210> 392
<211> 22
<212> RNA
<213> Homo sapiens

<400> 392
aauuccaagg ggaaaacugg ug 22


<210> 393
<211> 22
<212> RNA
<213> Homo sapiens

<400> 393
aauuuccagu uggaaaaauu ag 22


<210> 394
<211> 22
<212> RNA
<213> Homo sapiens

<400> 394
acuuugcuaa gacuugcuuu cu 22


<210> 395
<211> 22
<212> RNA
<213> Homo sapiens

<400> 395
agacuccgga gggcagaggc ug 22


<210> 396
<211> 22
<212> RNA
<213> Homo sapiens

<400> 396
agagaaggua cauagagaca uu 22


<210> 397
<211> 22
<212> RNA
<213> Homo sapiens

<400> 397
agagaggacu uuggccuaga ua 22


<210> 398
<211> 22
<212> RNA
<213> Homo sapiens

<400> 398
agguucugag ggaaguccuc ug 22


<210> 399
<211> 22
<212> RNA

<213>  Homo sapiens

<400>  399
augcugcauc ugccccugug cu                                                22


<210>  400
<211>  22
<212>  RNA
<213>  Homo sapiens

<400>  400
augcuggaag uuuguccaug au                                                22


<210>  401
<211>  22
<212>  RNA
<213>  Homo sapiens

<400>  401
augucuguaa gccuugagca ga                                                22


<210>  402
<211>  22
<212>  RNA
<213>  Homo sapiens

<400>  402
cacguaugua uaucuugccc uu                                                22


<210>  403
<211>  22
<212>  RNA
<213>  Homo sapiens

<400>  403
cacugaguuu ccugggugau uu                                                22


<210>  404
<211>  22
<212>  RNA
<213>  Homo sapiens

<400>  404
cagccugcca ggacucgcag ug                                                22


<210>  405
<211>  22
<212>  RNA
<213>  Homo sapiens

<400>  405
caugggcagg aggcccuuga ga                                                22


<210>  406
<211>  22
<212>  RNA
<213>  Homo sapiens

<400>  406
cauugauguu caucaugcau gg                                                22


<210>  407
<211>  22
<212>  RNA
<213>  Homo sapiens

<400>  407

ccaaagauug ccacguugca ag 22

<210> 408
<211> 22
<212> RNA
<213> Homo sapiens

<400> 408
cccggcgagg cagggcuucu aa 22

<210> 409
<211> 22
<212> RNA
<213> Homo sapiens

<400> 409
cccgggagca cuugagagcu gu 22

<210> 410
<211> 22
<212> RNA
<213> Homo sapiens

<400> 410
ccucucugcu gagcgggugg cu 22

<210> 411
<211> 22
<212> RNA
<213> Homo sapiens

<400> 411
cgauggggcu gggcccuggc cu 22

<210> 412
<211> 22
<212> RNA
<213> Homo sapiens

<400> 412
cgcguggggc ggagcuuccg ga 22

<210> 413
<211> 22
<212> RNA
<213> Homo sapiens

<400> 413
cgggcacugc agcagcucag gu 22

<210> 414
<211> 22
<212> RNA
<213> Homo sapiens

<400> 414
cggugacccg gcuagccggc cg 22

<210> 415
<211> 22
<212> RNA
<213> Homo sapiens

<400> 415
cuaugcagag cuggcgguuu ug 22

<210> 416
<211> 22
<212> RNA
<213> Homo sapiens

<400> 416
cucgaccgga ccucgaccgg cu 22

<210> 417
<211> 22
<212> RNA
<213> Homo sapiens

<400> 417
cucggagccg ccucgcuccu cu 22

<210> 418
<211> 22
<212> RNA
<213> Homo sapiens

<400> 418
cugccugcug uaucuuccau gu 22

<210> 419
<211> 22
<212> RNA
<213> Homo sapiens

<400> 419
cugcuuaaua cgcuccgcgg aa 22

<210> 420
<211> 22
<212> RNA
<213> Homo sapiens

<400> 420
cuggagaugc uggcuguuuu uu 22

<210> 421
<211> 22
<212> RNA
<213> Homo sapiens

<400> 421
cugggaggag uucagccgcg cu 22

<210> 422
<211> 22
<212> RNA
<213> Homo sapiens

<400> 422
cuuaauccca ggagccugca cg 22

<210> 423
<211> 22
<212> RNA
<213> Homo sapiens

<400> 423
gucaaggagu cgcaggucgc cu 22

<210> 424
<211> 22
<212> RNA

<213>  Homo sapiens

<400>  424
uccagacuga ggaagacccg ga                                                22


<210>  425
<211>  22
<212>  RNA
<213>  Homo sapiens

<400>  425
uccugugaga gucugaagga ua                                                22


<210>  426
<211>  22
<212>  RNA
<213>  Homo sapiens

<400>  426
ucugcucuga agucuugcau uu                                                22


<210>  427
<211>  22
<212>  RNA
<213>  Homo sapiens

<400>  427
ugagccaccg cgcacggccu gg                                                22


<210>  428
<211>  22
<212>  RNA
<213>  Homo sapiens

<400>  428
ugaguucagg caguggcag aa                                                 22


<210>  429
<211>  22
<212>  RNA
<213>  Homo sapiens

<400>  429
uggguuccug ccaggcuuga ag                                                22


<210>  430
<211>  22
<212>  RNA
<213>  Homo sapiens

<400>  430
ugucagcuug gaaggaccug uu                                                22


<210>  431
<211>  22
<212>  RNA
<213>  Homo sapiens

<400>  431
uucaaagcag agggcccuga ag                                                22


<210>  432
<211>  22
<212>  RNA
<213>  Homo sapiens

<400>  432

uugagcagcc uuucugcuga uu                                                22

<210>  433
<211>  22
<212>  RNA
<213>  Homo sapiens

<400>  433
uugcuuaacc uugcccucga aa                                                22

<210>  434
<211>  22
<212>  RNA
<213>  Homo sapiens

<400>  434
uuggcugguc cgaugguagu gg                                                22

<210>  435
<211>  22
<212>  RNA
<213>  Homo sapiens

<400>  435
uuuggcaguc cuuaccaagc aa                                                22

<210>  436
<211>  21
<212>  RNA
<213>  Homo sapiens

<400>  436
aaaaucaaug gagugguuuu u                                                 21

<210>  437
<211>  21
<212>  RNA
<213>  Homo sapiens

<400>  437
agagucugag ccugugagaa c                                                 21

<210>  438
<211>  21
<212>  RNA
<213>  Homo sapiens

<400>  438
aguccuccca cgaggcauac u                                                 21

<210>  439
<211>  21
<212>  RNA
<213>  Homo sapiens

<400>  439
augucccugc gggcggcugu c                                                 21

<210>  440
<211>  21
<212>  RNA
<213>  Homo sapiens

<400>  440
auguucaugg cauugcugua c                                                 21

<210> 441
<211> 21
<212> RNA
<213> Homo sapiens

<400> 441
auuguuuugc uagguguuca u 21

<210> 442
<211> 21
<212> RNA
<213> Homo sapiens

<400> 442
auuuugcuuu cuguuuacaa a 21

<210> 443
<211> 21
<212> RNA
<213> Homo sapiens

<400> 443
cagaggaggg gacggcagca c 21

<210> 444
<211> 21
<212> RNA
<213> Homo sapiens

<400> 444
cagucagcaa agccugggag a 21

<210> 445
<211> 21
<212> RNA
<213> Homo sapiens

<400> 445
ccaggagccu ucuuccuuga a 21

<210> 446
<211> 21
<212> RNA
<213> Homo sapiens

<400> 446
cccagcugcu uggaagucug a 21

<210> 447
<211> 21
<212> RNA
<213> Homo sapiens

<400> 447
ccuuccagac agagacucug u 21

<210> 448
<211> 21
<212> RNA
<213> Homo sapiens

<400> 448
cggcggcggc ggcgacucug c 21

<210> 449
<211> 21
<212> RNA

<213>  Homo sapiens

<400>  449
cguguucauu gcagcacugu u                                                21


<210>  450
<211>  21
<212>  RNA
<213>  Homo sapiens

<400>  450
cuuccagcga uuuugaaaua c                                                21


<210>  451
<211>  21
<212>  RNA
<213>  Homo sapiens

<400>  451
gccacgagag ggcacugcag g                                                21


<210>  452
<211>  21
<212>  RNA
<213>  Homo sapiens

<400>  452
gcugcuagcc cccaggggca c                                                21


<210>  453
<211>  21
<212>  RNA
<213>  Homo sapiens

<400>  453
ggucgcaacg cugugggcgu u                                                21


<210>  454
<211>  21
<212>  RNA
<213>  Homo sapiens

<400>  454
ucagaagcag uuugccuugu c                                                21


<210>  455
<211>  21
<212>  RNA
<213>  Homo sapiens

<400>  455
ucggaagagc gcuccggcca c                                                21


<210>  456
<211>  21
<212>  RNA
<213>  Homo sapiens

<400>  456
ucgggcgagc cucgggggcu g                                                21


<210>  457
<211>  21
<212>  RNA
<213>  Homo sapiens

<400>  457

ucuccagggg cagucugcca c                                                21

<210>  458
<211>  21
<212>  RNA
<213>  Homo sapiens

<400>  458
ugacccgccg ggacccgcuc g                                                21

<210>  459
<211>  21
<212>  RNA
<213>  Homo sapiens

<400>  459
uuccgcuucu ccgaggacgc c                                                21

<210>  460
<211>  21
<212>  RNA
<213>  Homo sapiens

<400>  460
uucggagccu ggagcacagg g                                                21

<210>  461
<211>  21
<212>  RNA
<213>  Homo sapiens

<400>  461
uuggugcauu cgucgcugaa c                                                21

<210>  462
<211>  20
<212>  RNA
<213>  Homo sapiens

<400>  462
aauggcagua cuuuaggaca                                                  20

<210>  463
<211>  20
<212>  RNA
<213>  Homo sapiens

<400>  463
aauuguaguu gcugccuagc                                                  20

<210>  464
<211>  20
<212>  RNA
<213>  Homo sapiens

<400>  464
   accgaggaau gucggggucu                                                 20

<210>  465
<211>  20
<212>  RNA
<213>  Homo sapiens

<400>  465
agaacucauc caguccuugu                                                  20

<210> 466
<211> 20
<212> RNA
<213> Homo sapiens

<400> 466
agaggagacu gggaggcagc 20


<210> 467
<211> 20
<212> RNA
<213> Homo sapiens

<400> 467
agcacgcaga ccgccgagga 20


<210> 468
<211> 20
<212> RNA
<213> Homo sapiens

<400> 468
agcgaggguu ccgccggcca 20


<210> 469
<211> 20
<212> RNA
<213> Homo sapiens

<400> 469
agcgaugugg ccugggaacg 20


<210> 470
<211> 20
<212> RNA
<213> Homo sapiens

<400> 470
auucccgggu gccuggcaac 20


<210> 471
<211> 20
<212> RNA
<213> Homo sapiens

<400> 471
cagcucggag cccggagcgu 20


<210> 472
<211> 20
<212> RNA
<213> Homo sapiens

<400> 472
caguuuccgu uacaagggua 20


<210> 473
<211> 20
<212> RNA
<213> Homo sapiens

<400> 473
cauguggcuu uggugacuca 20


<210> 474
<211> 20
<212> RNA

<213>  Homo sapiens

<400>  474
cuccuagugc acuaaccuac                                                    20


<210>  475
<211>  20
<212>  RNA
<213>  Homo sapiens

<400>  475
cucucaagga acucgcuguc                                                    20


<210>  476
<211>  20
<212>  RNA
<213>  Homo sapiens

<400>  476
ggcgcagcug gcugugagcg                                                    20


<210>  477
<211>  20
<212>  RNA
<213>  Homo sapiens

<400>  477
uccgguggug cccuggcggc                                                    20


<210>  478
<211>  20
<212>  RNA
<213>  Homo sapiens

<400>  478
uccuaaagua ccuggcaaag                                                    20


<210>  479
<211>  20
<212>  RNA
<213>  Homo sapiens

<400>  479
uggugaaugu gcaauguuag                                                    20


<210>  480
<211>  20
<212>  RNA
<213>  Homo sapiens

<400>  480
uucgcugagc ggcacggcua                                                    20


<210>  481
<211>  20
<212>  RNA
<213>  Homo sapiens

<400>  481
uuggcucaaa cagguugacc                                                    20


<210>  482
<211>  19
<212>  RNA
<213>  Homo sapiens

<400>  482

aacaucugug ggcgcugcc                                                    19

<210>  483
<211>  19
<212>  RNA
<213>  Homo sapiens

<400>  483
aaugucucau guuggaaag                                                    19

<210>  484
<211>  19
<212>  RNA
<213>  Homo sapiens

<400>  484
agaguuggac cagaaaagc                                                    19

<210>  485
<211>  19
<212>  RNA
<213>  Homo sapiens

<400>  485
aucgggagau uucuucauu                                                    19

<210>  486
<211>  19
<212>  RNA
<213>  Homo sapiens

<400>  486
cacggugcgg cugcuucac                                                    19

<210>  487
<211>  19
<212>  RNA
<213>  Homo sapiens

<400>  487
caggaggcug gauggcuuc                                                    19

<210>  488
<211>  19
<212>  RNA
<213>  Homo sapiens

<400>  488
caggcucugc agaggugac                                                    19

<210>  489
<211>  19
<212>  RNA
<213>  Homo sapiens

<400>  489
caguuagggu acaagcuaa                                                    19

<210>  490
<211>  19
<212>  RNA
<213>  Homo sapiens

<400>  490
cugacacucg aggggguuuc                                                    19

<210> 491
<211> 19
<212> RNA
<213> Homo sapiens

<400> 491
uugaguggga aaucacaag 19


<210> 492
<211> 18
<212> RNA
<213> Homo sapiens

<400> 492
aaaacuguga aaaugaug 18


<210> 493
<211> 18
<212> RNA
<213> Homo sapiens

<400> 493
aagaaaaagu uugccugc 18


<210> 494
<211> 18
<212> RNA
<213> Homo sapiens

<400> 494
aagauuugcc ggggcauc 18


<210> 495
<211> 18
<212> RNA
<213> Homo sapiens

<400> 495
agacagcugc gugugaga 18


<210> 496
<211> 18
<212> RNA
<213> Homo sapiens

<400> 496
aggacucggc cccaugga 18


<210> 497
<211> 18
<212> RNA
<213> Homo sapiens

<400> 497
augauuucag gauagguu 18


<210> 498
<211> 18
<212> RNA
<213> Homo sapiens

<400> 498
cagcuagacu cucaaugg 18


<210> 499
<211> 18
<212> RNA

<213>  Homo sapiens

<400>  499
ccgagccucg gcggcggc                                                    18


<210>  500
<211>  18
<212>  RNA
<213>  Homo sapiens

<400>  500
cucagucaac aaacacau                                                    18


<210>  501
<211>  18
<212>  RNA
<213>  Homo sapiens

<400>  501
cugggacaga ccugcgug                                                    18


<210>  502
<211>  18
<212>  RNA
<213>  Homo sapiens

<400>  502
cugugugaua ggaaaagc                                                    18


<210>  503
<211>  18
<212>  RNA
<213>  Homo sapiens

<400>  503
ugcaaggaga uggaagaa                                                    18


<210>  504
<211>  18
<212>  RNA
<213>  Homo sapiens

<400>  504
uucuuugcug cggagaac                                                    18


<210>  505
<211>  18
<212>  RNA
<213>  Homo sapiens

<400>  505
uuuggaauug guagcaau                                                    18


<210>  506
<211>  17
<212>  RNA
<213>  Homo sapiens

<400>  506
agcggcagcg gccgcag                                                     17


<210>  507
<211>  17
<212>  RNA
<213>  Homo sapiens

<400>  507

augaaagaga gaaaauu                                                    17

<210>  508
<211>  17
<212>  RNA
<213>  Homo sapiens

<400>  508
augagagcuc cacuguc                                                    17

<210>  509
<211>  17
<212>  RNA
<213>  Homo sapiens

<400>  509
auuggugcug cuuuuca                                                    17

<210>  510
<211>  17
<212>  RNA
<213>  Homo sapiens

<400>  510
cgaaguugaa auguuug                                                    17

<210>  511
<211>  17
<212>  RNA
<213>  Homo sapiens

<400>  511
cgaaugagaa ggagcgg                                                    17

<210>  512
<211>  17
<212>  RNA
<213>  Homo sapiens

<400>  512
cuaagacaua gggaaau                                                    17

<210>  513
<211>  17
<212>  RNA
<213>  Homo sapiens

<400>  513
cuucauacca cauuguc                                                    17

<210>  514
<211>  17
<212>  RNA
<213>  Homo sapiens

<400>  514
guggggagaa ggcuguc                                                    17

<210>  515
<211>  17
<212>  RNA
<213>  Homo sapiens

<400>  515
ugagguagua gauuaga                                                    17

<210> 516
<211> 16
<212> RNA
<213> Homo sapiens

<400> 516
cccacgugua gcuagu 16


<210> 517
<211> 16
<212> RNA
<213> Homo sapiens

<400> 517
uugacaagaa caggac 16


<210> 518
<211> 15
<212> RNA
<213> Homo sapiens

<400> 518
cucaguggcg gcuuu 15


<210> 519
<211> 22
<212> RNA
<213> Homo sapiens

<400> 519
aaaaagagug aguugagagg au 22


<210> 520
<211> 22
<212> RNA
<213> Homo sapiens

<400> 520
aaaagaugua ucuucucaaa ua 22


<210> 521
<211> 22
<212> RNA
<213> Homo sapiens

<400> 521
aaaucuuggg auuccuggag ug 22


<210> 522
<211> 22
<212> RNA
<213> Homo sapiens

<400> 522
aaaugagaga augugugaac ga 22


<210> 523
<211> 22
<212> RNA
<213> Homo sapiens

<400> 523
aacagaggac aacgcaacaa gg 22


<210> 524
<211> 22
<212> RNA

<213>  Homo sapiens

<400>  524
aacguggaca ucggauaguu cu                                                22


<210>  525
<211>  22
<212>  RNA
<213>  Homo sapiens

<400>  525
aacucccuga gcgcucccuc gu                                                22


<210>  526
<211>  22
<212>  RNA
<213>  Homo sapiens

<400>  526
aacuggaaga gaguuacacu cu                                                22


<210>  527
<211>  22
<212>  RNA
<213>  Homo sapiens

<400>  527
aagaaucuua accuaauaaa aa                                                22


<210>  528
<211>  22
<212>  RNA
<213>  Homo sapiens

<400>  528
aagcaccgug aaaacggacu gu                                                22


<210>  529
<211>  22
<212>  RNA
<213>  Homo sapiens

<400>  529
aagccugcag gacagccagu gu                                                22


<210>  530
<211>  22
<212>  RNA
<213>  Homo sapiens

<400>  530
aagcucaaug aggacucauu ag                                                22


<210>  531
<211>  22
<212>  RNA
<213>  Homo sapiens

<400>  531
aaggaaaacu guguuuucag aa                                                22


<210>  532
<211>  22
<212>  RNA
<213>  Homo sapiens

<400>  532

aaggccaucc gagggcaccu gg 22

<210> 533
<211> 22
<212> RNA
<213> Homo sapiens

<400> 533
aagggugagc ccuggugucg gg 22

<210> 534
<211> 22
<212> RNA
<213> Homo sapiens

<400> 534
aagucccagg cgggagcguc uu 22

<210> 535
<211> 22
<212> RNA
<213> Homo sapiens

<400> 535
aagucggcca ggaugucuca gg 22

<210> 536
<211> 22
<212> RNA
<213> Homo sapiens

<400> 536
aagucuguag gccuugucug uu 22

<210> 537
<211> 22
<212> RNA
<213> Homo sapiens

<400> 537
aaugagcagu gagacgacug ga 22

<210> 538
<211> 22
<212> RNA
<213> Homo sapiens

<400> 538
aaugucagau uuuugaaugu ua 22

<210> 539
<211> 22
<212> RNA
<213> Homo sapiens

<400> 539
aauguguaca gugaaguggu uu 22

<210> 540
<211> 22
<212> RNA
<213> Homo sapiens

<400> 540
accccgggca ggcaccccca gg 22

<210> 541
<211> 22
<212> RNA
<213> Homo sapiens

<400> 541
acccgccugc ugcugggcgg cg 22


<210> 542
<211> 22
<212> RNA
<213> Homo sapiens

<400> 542
accuccucca cuucucccuu ug 22


<210> 543
<211> 22
<212> RNA
<213> Homo sapiens

<400> 543
accugagcua gaauacaagu ag 22


<210> 544
<211> 22
<212> RNA
<213> Homo sapiens

<400> 544
accuguucgu gugcuccgac ug 22


<210> 545
<211> 22
<212> RNA
<213> Homo sapiens

<400> 545
acgcaccgcu cggugcuggc cg 22


<210> 546
<211> 22
<212> RNA
<213> Homo sapiens

<400> 546
acggcaguca caggaacuca gg 22


<210> 547
<211> 22
<212> RNA
<213> Homo sapiens

<400> 547
acuacgggga cgcugggccg gg 22


<210> 548
<211> 22
<212> RNA
<213> Homo sapiens

<400> 548
acuccuggug cugagaugga cg 22


<210> 549
<211> 22
<212> RNA

<213>  Homo sapiens

<400>  549
acugaugcca uccgaggaag au                                                22


<210>  550
<211>  22
<212>  RNA
<213>  Homo sapiens

<400>  550
acugcagucu ugaacuugcu gg                                                22


<210>  551
<211>  22
<212>  RNA
<213>  Homo sapiens

<400>  551
acuuuccuga gcaaaucuga ug                                                22


<210>  552
<211>  22
<212>  RNA
<213>  Homo sapiens

<400>  552
agaaacuuuc caaagugcuu ag                                                22


<210>  553
<211>  22
<212>  RNA
<213>  Homo sapiens

<400>  553
agaacucaga ccguaggugu gg                                                22


<210>  554
<211>  22
<212>  RNA
<213>  Homo sapiens

<400>  554
agacaggguc ugaaggacug ga                                                22


<210>  555
<211>  22
<212>  RNA
<213>  Homo sapiens

<400>  555
agauggaggc ugcagugagc cu                                                22


<210>  556
<211>  22
<212>  RNA
<213>  Homo sapiens

<400>  556
agcaauggau guggucuacg cg                                                22


<210>  557
<211>  22
<212>  RNA
<213>  Homo sapiens

<400>  557

agccccagag uagcugggau ua                                                22

<210>  558
<211>  22
<212>  RNA
<213>  Homo sapiens

<400>  558
agccccugcc uuagagcucu gg                                                22

<210>  559
<211>  22
<212>  RNA
<213>  Homo sapiens

<400>  559
agcgguggug guuccgcgau gg                                                22

<210>  560
<211>  22
<212>  RNA
<213>  Homo sapiens

<400>  560
aggaacagcc ugcuggcuca ga                                                22

<210>  561
<211>  22
<212>  RNA
<213>  Homo sapiens

<400>  561
aggaguuagc ugugccagug gu                                                22

<210>  562
<211>  22
<212>  RNA
<213>  Homo sapiens

<400>  562
aggccugggg cugccuccug gg                                                22

<210>  563
<211>  22
<212>  RNA
<213>  Homo sapiens

<400>  563
   aggguggggu gggcaugggg ug                                                22

<210>  564
<211>  22
<212>  RNA
<213>  Homo sapiens

<400>  564
aggugagcgg cgaccgguaa cu                                                22

<210>  565
<211>  22
<212>  RNA
<213>  Homo sapiens

<400>  565
agguuacaag agcagauauu cu                                                22

<210> 566
<211> 22
<212> RNA
<213> Homo sapiens

<400> 566
agugacauuc aucugcuggc ua 22

<210> 567
<211> 22
<212> RNA
<213> Homo sapiens

<400> 567
agugagcccc ugucucuguu uu 22

<210> 568
<211> 22
<212> RNA
<213> Homo sapiens

<400> 568
agugcuugga cggaacccgg cg 22

<210> 569
<211> 22
<212> RNA
<213> Homo sapiens

<400> 569
auacggccug cagaaucgug ag 22

<210> 570
<211> 22
<212> RNA
<213> Homo sapiens

<400> 570
aucacauaug agugagaaca ug 22

<210> 571
<211> 22
<212> RNA
<213> Homo sapiens

<400> 571
aucagcagca uugaacagaa ag 22

<210> 572
<211> 22
<212> RNA
<213> Homo sapiens

<400> 572
auccgauauu ccagaaggca ag 22

<210> 573
<211> 22
<212> RNA
<213> Homo sapiens

<400> 573
auguccugca guuaacacuc aa 22

<210> 574
<211> 22
<212> RNA

<213>  Homo sapiens

<400>  574
auguguuagc cauagcugaa aa                                                22


<210>  575
<211>  22
<212>  RNA
<213>  Homo sapiens

<400>  575
auuagaggcg ugagccgccg cg                                                22


<210>  576
<211>  22
<212>  RNA
<213>  Homo sapiens

<400>  576
auuugcuugu cuguaacgga uu                                                22


<210>  577
<211>  22
<212>  RNA
<213>  Homo sapiens

<400>  577
caagacuuga gcagugugua gu                                                22


<210>  578
<211>  22
<212>  RNA
<213>  Homo sapiens

<400>  578
caaggcgagg gaccuggccc ug                                                22


<210>  579
<211>  22
<212>  RNA
<213>  Homo sapiens

<400>  579
caccugagca agcuccuguu uu                                                22


<210>  580
<211>  22
<212>  RNA
<213>  Homo sapiens

<400>  580
cacugggccc gagccugaac ug                                                22


<210>  581
<211>  22
<212>  RNA
<213>  Homo sapiens

<400>  581
cagcaguagu cagccauccg ga                                                22


<210>  582
<211>  22
<212>  RNA
<213>  Homo sapiens

<400>  582

cagcgacuuc ugaagggccc cu                                                22

<210>  583
<211>  22
<212>  RNA
<213>  Homo sapiens

<400>  583
caggcaugag cugcuguguc ug                                                22

<210>  584
<211>  22
<212>  RNA
<213>  Homo sapiens

<400>  584
caggcgaccc ggaagguuug cg                                                22

<210>  585
<211>  22
<212>  RNA
<213>  Homo sapiens

<400>  585
cagugggcag gacacacacg uu                                                22

<210>  586
<211>  22
<212>  RNA
<213>  Homo sapiens

<400>  586
ccagggcacc accgaggccc ga                                                22

<210>  587
<211>  22
<212>  RNA
<213>  Homo sapiens

<400>  587
cccgagcacg ugcuggucaa ga                                                22

<210>  588
<211>  22
<212>  RNA
<213>  Homo sapiens

<400>  588
cccgggaggc agagguauug gu                                                22

<210>  589
<211>  22
<212>  RNA
<213>  Homo sapiens

<400>  589
cccgugaggc agaaguugua gu                                                22

<210>  590
<211>  22
<212>  RNA
<213>  Homo sapiens

<400>  590
cccuccggga cacugacgag gu                                                22

<210> 591
<211> 22
<212> RNA
<213> Homo sapiens

<400> 591
cccugcaggg cuuggccaac cg 22


<210> 592
<211> 22
<212> RNA
<213> Homo sapiens

<400> 592
ccgugagacc aacgcagcca ug 22


<210> 593
<211> 22
<212> RNA
<213> Homo sapiens

<400> 593
ccucggaaca acuguauugc ug 22


<210> 594
<211> 22
<212> RNA
<213> Homo sapiens

<400> 594
ccuucaacac cucucuuggu ug 22


<210> 595
<211> 22
<212> RNA
<213> Homo sapiens

<400> 595
cgacugcugg aagcugcagg ga 22


<210> 596
<211> 22
<212> RNA
<213> Homo sapiens

<400> 596
cgccugggga gccugccugg gg 22


<210> 597
<211> 22
<212> RNA
<213> Homo sapiens

<400> 597
cggccucgcu ucccgcgccu cg 22


<210> 598
<211> 22
<212> RNA
<213> Homo sapiens

<400> 598
cggcgccucu gugggcagac ug 22


<210> 599
<211> 22
<212> RNA

<213>  Homo sapiens

<400>  599
cgggaguugg gggcuagagu ga                                                22


<210>  600
<211>  22
<212>  RNA
<213>  Homo sapiens

<400>  600
cucacugccu ccgguggcug cu                                                22


<210>  601
<211>  22
<212>  RNA
<213>  Homo sapiens

<400>  601
cugagaguca gagggauggg ug                                                22


<210>  602
<211>  22
<212>  RNA
<213>  Homo sapiens

<400>  602
cuggaggacc aaccccugcu gu                                                22


<210>  603
<211>  22
<212>  RNA
<213>  Homo sapiens

<400>  603
cuggcuacga acucccuggg gg                                                22


<210>  604
<211>  22
<212>  RNA
<213>  Homo sapiens

<400>  604
cugggacgca ccuggggaca gg                                                22


<210>  605
<211>  22
<212>  RNA
<213>  Homo sapiens

<400>  605
cuggguccug cuggggcagg gu                                                22


<210>  606
<211>  22
<212>  RNA
<213>  Homo sapiens

<400>  606
   cuggguuuug ggggugcuga cg                                                22


<210>  607
<211>  22
<212>  RNA
<213>  Homo sapiens

<400>  607

cugugcagca ggccaaccga ga                                                22

<210>  608
<211>  22
<212>  RNA
<213>  Homo sapiens

<400>  608
cuuugcucau gggcuggaug gg                                                22

<210>  609
<211>  22
<212>  RNA
<213>  Homo sapiens

<400>  609
cuuuuugaua uuuguauuga ua                                                22

<210>  610
<211>  22
<212>  RNA
<213>  Homo sapiens

<400>  610
gaaauguuaa guaucaguug gu                                                22

<210>  611
<211>  22
<212>  RNA
<213>  Homo sapiens

<400>  611
gagagcgagc cgagccgagc ug                                                22

<210>  612
<211>  22
<212>  RNA
<213>  Homo sapiens

<400>  612
gagccaggga gcgcuggagg aa                                                22

<210>  613
<211>  22
<212>  RNA
<213>  Homo sapiens

<400>  613
gaguaucugc auccugcgcu cu                                                22

<210>  614
<211>  22
<212>  RNA
<213>  Homo sapiens

<400>  614
gauucugugg ggccguggga gg                                                22

<210>  615
<211>  22
<212>  RNA
<213>  Homo sapiens

<400>  615
gcaaaaaccu uaauuaccuu ug                                                22

<210> 616
<211> 22
<212> RNA
<213> Homo sapiens

<400> 616
gcagcgggcg gcugcagcug ug 22


<210> 617
<211> 22
<212> RNA
<213> Homo sapiens

<400> 617
gcaggcccuc acugucacuu ua 22


<210> 618
<211> 22
<212> RNA
<213> Homo sapiens

<400> 618
gcaggguaua aguggcucaa ug 22


<210> 619
<211> 22
<212> RNA
<213> Homo sapiens

<400> 619
gccccgagug cgggcgagcc uu 22


<210> 620
<211> 22
<212> RNA
<213> Homo sapiens

<400> 620
gccgcggcgg cuccucugcc gg 22


<210> 621
<211> 22
<212> RNA
<213> Homo sapiens

<400> 621
gccgcugggc cugagaggag cu 22


<210> 622
<211> 22
<212> RNA
<213> Homo sapiens

<400> 622
gccugaauuu cauaaaagaa ag 22


<210> 623
<211> 22
<212> RNA
<213> Homo sapiens

<400> 623
gcggaagcuc cggaccuccc ug 22


<210> 624
<211> 22
<212> RNA

<213>  Homo sapiens

<400>  624
gcggcuuaag acgcucggcg au                                                22


<210>  625
<211>  22
<212>  RNA
<213>  Homo sapiens

<400>  625
gcguuuggga ugccaacggu aa                                                22


<210>  626
<211>  22
<212>  RNA
<213>  Homo sapiens

<400>  626
gcugcagagc cgguaacggu gg                                                22


<210>  627
<211>  22
<212>  RNA
<213>  Homo sapiens

<400>  627
ggagcagaag agccuuccuu ug                                                22


<210>  628
<211>  22
<212>  RNA
<213>  Homo sapiens

<400>  628
ggcgcggagc uaagcggcuc gg                                                22


<210>  629
<211>  22
<212>  RNA
<213>  Homo sapiens

<400>  629
ggcuuccgcu gcccgugagc ua                                                22


<210>  630
<211>  22
<212>  RNA
<213>  Homo sapiens

<400>  630
gugaaguagu uggggccgua ga                                                22


<210>  631
<211>  22
<212>  RNA
<213>  Homo sapiens

<400>  631
guuucaucau guucggcagg cu                                                22


<210>  632
<211>  22
<212>  RNA
<213>  Homo sapiens

<400>  632

ucacagugcu uguguucaga ua                                                22

<210>  633
<211>  22
<212>  RNA
<213>  Homo sapiens

<400>  633
uccagggugg gaguaguucc cu                                                22

<210>  634
<211>  22
<212>  RNA
<213>  Homo sapiens

<400>  634
uccgcugggu caggcgcuga cg                                                22

<210>  635
<211>  22
<212>  RNA
<213>  Homo sapiens

<400>  635
uccggaggcg cuguuccagc cu                                                22

<210>  636
<211>  22
<212>  RNA
<213>  Homo sapiens

<400>  636
uccggcugga uggggccauu cg                                                22

<210>  637
<211>  22
<212>  RNA
<213>  Homo sapiens

<400>  637
ucgcagacag ggacacaugg ag                                                22

<210>  638
<211>  22
<212>  RNA
<213>  Homo sapiens

<400>  638
ucucuaccaa agcucaugug ga                                                22

<210>  639
<211>  22
<212>  RNA
<213>  Homo sapiens

<400>  639
ucucugucag gguacucaga gg                                                22

<210>  640
<211>  22
<212>  RNA
<213>  Homo sapiens

<400>  640
ucugcguggc ggugaggccc cg                                                22

<210> 641
<211> 22
<212> RNA
<213> Homo sapiens

<400> 641
ucuucgcuuc cagagcucug cg 22


<210> 642
<211> 22
<212> RNA
<213> Homo sapiens

<400> 642
ugagccuuga agguccagaa gg 22


<210> 643
<211> 22
<212> RNA
<213> Homo sapiens

<400> 643
ugagggauga gaugcgagcc uu 22


<210> 644
<211> 22
<212> RNA
<213> Homo sapiens

<400> 644
ugcaucgccu gcuccgcaaa gg 22


<210> 645
<211> 22
<212> RNA
<213> Homo sapiens

<400> 645
ugccaacggc cacggaggcc aa 22


<210> 646
<211> 22
<212> RNA
<213> Homo sapiens

<400> 646
ugcgagcggc ugcugagccg gg 22


<210> 647
<211> 22
<212> RNA
<213> Homo sapiens

<400> 647
ugcuggacgg cccugugcug ag 22


<210> 648
<211> 22
<212> RNA
<213> Homo sapiens

<400> 648
ugcugggcag cuugguccug cu 22


<210> 649
<211> 22
<212> RNA

<213>  Homo sapiens

<400>  649
uggccggagc uggugccugc gg                                                22


<210>  650
<211>  22
<212>  RNA
<213>  Homo sapiens

<400>  650
uggccggcug ggucuccucc cg                                                22


<210>  651
<211>  22
<212>  RNA
<213>  Homo sapiens

<400>  651
uggcugggag ucacugaugc ug                                                22


<210>  652
<211>  22
<212>  RNA
<213>  Homo sapiens

<400>  652
uguccgucau ccugggcguc gu                                                22


<210>  653
<211>  22
<212>  RNA
<213>  Homo sapiens

<400>  653
ugugccacca caccuggcuu aa                                                22


<210>  654
<211>  22
<212>  RNA
<213>  Homo sapiens

<400>  654
uuagccaauu guccaucuuu ag                                                22


<210>  655
<211>  22
<212>  RNA
<213>  Homo sapiens

<400>  655
uucaaggugg cguccuuccg ua                                                22


<210>  656
<211>  22
<212>  RNA
<213>  Homo sapiens

<400>  656
uucgggcgcg ugaccucggg gu                                                22


<210>  657
<211>  22
<212>  RNA
<213>  Homo sapiens

<400>  657

uucuaggcga gguccugccu ag                                                22

<210>  658
<211>  22
<212>  RNA
<213>  Homo sapiens

<400>  658
uucuccuggg gcucagagug uu                                                22

<210>  659
<211>  22
<212>  RNA
<213>  Homo sapiens

<400>  659
uucuucagca gaagagcuga gg                                                22

<210>  660
<211>  22
<212>  RNA
<213>  Homo sapiens

<400>  660
uucuuggaga gaaauaauaa au                                                22

<210>  661
<211>  22
<212>  RNA
<213>  Homo sapiens

<400>  661
uugaauuccu agcaaccuuc au                                                22

<210>  662
<211>  22
<212>  RNA
<213>  Homo sapiens

<400>  662
uugacauguu gcucuccaaa ag                                                22

<210>  663
<211>  22
<212>  RNA
<213>  Homo sapiens

<400>  663
uugcacuggc cugucccuca gg                                                22

<210>  664
<211>  22
<212>  RNA
<213>  Homo sapiens

<400>  664
uugcuuguuu aaacugcuca ag                                                22

<210>  665
<211>  22
<212>  RNA
<213>  Homo sapiens

<400>  665
   uugguguggg cuccuacucu ga                                                22

<210> 666
<211> 22
<212> RNA
<213> Homo sapiens

<400> 666
   uugucccugu ucugggggaa gg 22


<210> 667
<211> 22
<212> RNA
<213> Homo sapiens

<400> 667
uuugcagacu cacucugcau cu 22


<210> 668
<211> 22
<212> RNA
<213> Homo sapiens

<400> 668
uuugccacuu guuggccgug ga 22


<210> 669
<211> 21
<212> RNA
<213> Homo sapiens

<400> 669
aaaacgagaa ucuuaccaau u 21


<210> 670
<211> 21
<212> RNA
<213> Homo sapiens

<400> 670
aaaggcgagg uguccgcccg u 21


<210> 671
<211> 21
<212> RNA
<213> Homo sapiens

<400> 671
aagaguuuac ucugggaaaa c 21


<210> 672
<211> 21
<212> RNA
<213> Homo sapiens

<400> 672
aaggacugac cagggaccuu u 21


<210> 673
<211> 21
<212> RNA
<213> Homo sapiens

<400> 673
aaggugaaac acaacucugu a 21


<210> 674
<211> 21
<212> RNA

<213>  Homo sapiens

<400>  674
aaguaaggga cacggccucu c                                                21


<210>  675
<211>  21
<212>  RNA
<213>  Homo sapiens

<400>  675
aaguuccuaa agaaggcauu c                                                21


<210>  676
<211>  21
<212>  RNA
<213>  Homo sapiens

<400>  676
aauaauuugg cauucuuuaa g                                                21


<210>  677
<211>  21
<212>  RNA
<213>  Homo sapiens

<400>  677
aauaugcagg agggauuucc a                                                21


<210>  678
<211>  21
<212>  RNA
<213>  Homo sapiens

<400>  678
aaugcccucg ugaggccuca a                                                21


<210>  679
<211>  21
<212>  RNA
<213>  Homo sapiens

<400>  679
acaaaugccu ccugugcuuu g                                                21


<210>  680
<211>  21
<212>  RNA
<213>  Homo sapiens

<400>  680
acagccaggg uauccaggau g                                                21


<210>  681
<211>  21
<212>  RNA
<213>  Homo sapiens

<400>  681
acgacgaugu cugagcagaa u                                                21


<210>  682
<211>  21
<212>  RNA
<213>  Homo sapiens

<400>  682

acugauaccu uggaggcauu u                                                21

<210>  683
<211>  21
<212>  RNA
<213>  Homo sapiens

<400>  683
acugcccugg cacccagcac a                                                21

<210>  684
<211>  21
<212>  RNA
<213>  Homo sapiens

<400>  684
agacuguacg cuaaggguca u                                                21

<210>  685
<211>  21
<212>  RNA
<213>  Homo sapiens

<400>  685
   agagcccauc uccucaggac u                                               21

<210>  686
<211>  21
<212>  RNA
<213>  Homo sapiens

<400>  686
agaggggcaa uggcugcuuu c                                                21

<210>  687
<211>  21
<212>  RNA
<213>  Homo sapiens

<400>  687
agccucugcc cugggcggug u                                                21

<210>  688
<211>  21
<212>  RNA
<213>  Homo sapiens

<400>  688
agcgaggucu cggcuuuccg c                                                21

<210>  689
<211>  21
<212>  RNA
<213>  Homo sapiens

<400>  689
aggacaggcu gcugagcacc a                                                21

<210>  690
<211>  21
<212>  RNA
<213>  Homo sapiens

<400>  690
aggacuuguu ccugugggcu u                                                21

<210> 691
<211> 21
<212> RNA
<213> Homo sapiens

<400> 691
aggagucuug ccauguugcc c 21


<210> 692
<211> 21
<212> RNA
<213> Homo sapiens

<400> 692
aggccugcuc ugggccgccc u 21


<210> 693
<211> 21
<212> RNA
<213> Homo sapiens

<400> 693
aggcggacua ugacuuaguu g 21


<210> 694
<211> 21
<212> RNA
<213> Homo sapiens

<400> 694
agggcccggg ggaggcggcg g 21


<210> 695
<211> 21
<212> RNA
<213> Homo sapiens

<400> 695
agguuauggg cucuacauug u 21


<210> 696
<211> 21
<212> RNA
<213> Homo sapiens

<400> 696
agucagggag gcgaacugcu g 21


<210> 697
<211> 21
<212> RNA
<213> Homo sapiens

<400> 697
aguccagggg uuaaagcacc a 21


<210> 698
<211> 21
<212> RNA
<213> Homo sapiens

<400> 698
agugaacaga uaguguuaau u 21


<210> 699
<211> 21
<212> RNA

<213>  Homo sapiens

<400>  699
aucugggacc acgggcacuc u                                                21


<210>  700
<211>  21
<212>  RNA
<213>  Homo sapiens

<400>  700
auguuaaguu ucuacuaugu g                                                21


<210>  701
<211>  21
<212>  RNA
<213>  Homo sapiens

<400>  701
   auuggggugg augccucugc u                                                21


<210>  702
<211>  21
<212>  RNA
<213>  Homo sapiens

<400>  702
caaaaguacu ugcggauuuu g                                                21


<210>  703
<211>  21
<212>  RNA
<213>  Homo sapiens

<400>  703
caacagacac cagccugugg u                                                21


<210>  704
<211>  21
<212>  RNA
<213>  Homo sapiens

<400>  704
caccggggac aauggugacu a                                                21


<210>  705
<211>  21
<212>  RNA
<213>  Homo sapiens

<400>  705
   cacgaugagu cccugggggu u                                                21


<210>  706
<211>  21
<212>  RNA
<213>  Homo sapiens

<400>  706
cauagggucu gugggaaaug g                                                21


<210>  707
<211>  21
<212>  RNA
<213>  Homo sapiens

<400>  707

cauugagugu cugcagcucu u                                                21

<210>  708
<211>  21
<212>  RNA
<213>  Homo sapiens

<400>  708
ccccuccauc guggggcgcc c                                                21

<210>  709
<211>  21
<212>  RNA
<213>  Homo sapiens

<400>  709
ccgagugaug cuuggggaga c                                                21

<210>  710
<211>  21
<212>  RNA
<213>  Homo sapiens

<400>  710
ccgauaauuu gguggcggcg u                                                21

<210>  711
<211>  21
<212>  RNA
<213>  Homo sapiens

<400>  711
ccgccgccca acuugaagcc u                                                21

<210>  712
<211>  21
<212>  RNA
<213>  Homo sapiens

<400>  712
ccugacucag caugagccug g                                                21

<210>  713
<211>  21
<212>  RNA
<213>  Homo sapiens

<400>  713
   cgcccggcag ggaggcggcg g                                                21

<210>  714
<211>  21
<212>  RNA
<213>  Homo sapiens

<400>  714
cgcucaggcc cguggcgccg a                                                21

<210>  715
<211>  21
<212>  RNA
<213>  Homo sapiens

<400>  715
cgggcggcug gccucaccgc c                                                21

<210>  716
<211>  21
<212>  RNA
<213>  Homo sapiens

<400>  716
cggggguggug gggggcgggg a                                                21

<210>  717
<211>  21
<212>  RNA
<213>  Homo sapiens

<400>  717
cgugcugcgc uugcagugag c                                                21

<210>  718
<211>  21
<212>  RNA
<213>  Homo sapiens

<400>  718
cugcgggggu cccggccucu g                                                21

<210>  719
<211>  21
<212>  RNA
<213>  Homo sapiens

<400>  719
cuggcgcacc cguccgaguc c                                                21

<210>  720
<211>  21
<212>  RNA
<213>  Homo sapiens

<400>  720
cugggaguuc caggcucaga g                                                21

<210>  721
<211>  21
<212>  RNA
<213>  Homo sapiens

<400>  721
gagcugcugu ugccacaugu c                                                21

<210>  722
<211>  21
<212>  RNA
<213>  Homo sapiens

<400>  722
gccgcccugc ugcggcgggg c                                                21

<210>  723
<211>  21
<212>  RNA
<213>  Homo sapiens

<400>  723
gcgaagcccu gggcugcccu g                                                21

<210>  724
<211>  21
<212>  RNA

<213>  Homo sapiens

<400>  724
gcgggcaaug gccgccuggg u                                                21


<210>  725
<211>  21
<212>  RNA
<213>  Homo sapiens

<400>  725
ggagcccccc uggggaggcg g                                                21


<210>  726
<211>  21
<212>  RNA
<213>  Homo sapiens

<400>  726
ggcagauucc uggcagcugc c                                                21


<210>  727
<211>  21
<212>  RNA
<213>  Homo sapiens

<400>  727
ggcgccucca gcagcagcuc g                                                21


<210>  728
<211>  21
<212>  RNA
<213>  Homo sapiens

<400>  728
gggccgugga agguggccag g                                                21


<210>  729
<211>  21
<212>  RNA
<213>  Homo sapiens

<400>  729
ggggcucaug agcaggggaa g                                                21


<210>  730
<211>  21
<212>  RNA
<213>  Homo sapiens

<400>  730
gucccaggcc cggccggugc g                                                21


<210>  731
<211>  21
<212>  RNA
<213>  Homo sapiens

<400>  731
guggcucugg ggaaccuggg u                                                21


<210>  732.
<211>  21
<212>  RNA
<213>  Homo sapiens

<400>  732

gugggugggcc cccuguggug u                                                    21

<210>  733
<211>  21
<212>  RNA
<213>  Homo sapiens

<400>  733
uacacacauc ccugucuuac a                                                    21

<210>  734
<211>  21
<212>  RNA
<213>  Homo sapiens

<400>  734
ucagaggccg ccaucaaccg c                                                    21

<210>  735
<211>  21
<212>  RNA
<213>  Homo sapiens

<400>  735
ucccuugcug ugcccuccac c                                                    21

<210>  736
<211>  21
<212>  RNA
<213>  Homo sapiens

<400>  736
uccucuuccu ggugcugggc u                                                    21

<210>  737
<211>  21
<212>  RNA
<213>  Homo sapiens

<400>  737
ucucucugcc ugggauuccu c                                                    21

<210>  738
<211>  21
<212>  RNA
<213>  Homo sapiens

<400>  738
ucugcccuga guccugcaag a                                                    21

<210>  739
<211>  21
<212>  RNA
<213>  Homo sapiens

<400>  739
ucugggauac ugcugugucu c                                                    21

<210>  740
<211>  21
<212>  RNA
<213>  Homo sapiens

<400>  740
ucuuuuccuc accucguauu g                                                    21

<210> 741
<211> 21
<212> RNA
<213> Homo sapiens

<400> 741
ugagucaaug auucgggugc a 21


<210> 742
<211> 21
<212> RNA
<213> Homo sapiens

<400> 742
ugcagauucu ugggccucac c 21


<210> 743
<211> 21
<212> RNA
<213> Homo sapiens

<400> 743
ugcggucgcc gcgagccucg g 21


<210> 744
<211> 21
<212> RNA
<213> Homo sapiens

<400> 744
ugcucggcga gccucccucu a 21


<210> 745
<211> 21
<212> RNA
<213> Homo sapiens

<400> 745
uggaagaggu ggcccagagg u 21


<210> 746
<211> 21
<212> RNA
<213> Homo sapiens

<400> 746
uggagcugcu cuccuggccu c 21


<210> 747
<211> 21
<212> RNA
<213> Homo sapiens

<400> 747
uggauugcca gaaaugagag a 21


<210> 748
<211> 21
<212> RNA
<213> Homo sapiens

<400> 748
uggcagguga gccugagagu c 21


<210> 749
<211> 21
<212> RNA

<213>  Homo sapiens

<400>  749
ugugcgcagg gagaccucuc c                                                21


<210>  750
<211>  21
<212>  RNA
<213>  Homo sapiens

<400>  750
uucucagucc ucgaguccua c                                                21


<210>  751
<211>  21
<212>  RNA
<213>  Homo sapiens

<400>  751
uucuugcugc cccuucucug g                                                21


<210>  752
<211>  21
<212>  RNA
<213>  Homo sapiens

<400>  752
uugccacacu gcaacaccuu a                                                21


<210>  753
<211>  21
<212>  RNA
<213>  Homo sapiens

<400>  753
uugggcaggu cccaggcauu c                                                21


<210>  754
<211>  21
<212>  RNA
<213>  Homo sapiens

<400>  754
uugggcccga cggcaccggc c                                                21


<210>  755
<211>  20
<212>  RNA
<213>  Homo sapiens

<400>  755
aaacaaaugc cuacauguga                                                  20


<210>  756
<211>  20
<212>  RNA
<213>  Homo sapiens

<400>  756
aaagcauuua cuuuuuugac                                                  20


<210>  757
<211>  20
<212>  RNA
<213>  Homo sapiens

<400>  757

aacguucuga uagaaaugac                                                    20

<210>  758
<211>  20
<212>  RNA
<213>  Homo sapiens

<400>  758
aagccagugg gacucagcua                                                    20

<210>  759
<211>  20
<212>  RNA
<213>  Homo sapiens

<400>  759
aagcgaagug ugguggcuuc                                                    20

<210>  760
<211>  20
<212>  RNA
<213>  Homo sapiens

<400>  760
aauagguucu cuguaaaaca                                                    20

<210>  761
<211>  20
<212>  RNA
<213>  Homo sapiens

<400>  761
aaugaagcug gugugaccuu                                                    20

<210>  762
<211>  20
<212>  RNA
<213>  Homo sapiens

<400>  762
aaugcuuguu ggauucauga                                                    20

<210>  763
<211>  20
<212>  RNA
<213>  Homo sapiens

<400>  763
acuugcuaga gaaucuguuu                                                    20

<210>  764
<211>  20
<212>  RNA
<213>  Homo sapiens

<400>  764
agaaugcacu caagacaacc                                                    20

<210>  765
<211>  20
<212>  RNA
<213>  Homo sapiens

<400>  765
agagaccgug aagauuccaa                                                    20

<210> 766
<211> 20
<212> RNA
<213> Homo sapiens

<400> 766
agauuugaaa acacaugcau 20


<210> 767
<211> 20
<212> RNA
<213> Homo sapiens

<400> 767
agcaaggaug auucugauuu 20


<210> 768
<211> 20
<212> RNA
<213> Homo sapiens

<400> 768
agccaugugg aacugcaaau 20


<210> 769
<211> 20
<212> RNA
<213> Homo sapiens

<400> 769
agccugcgug agcuggaccu 20


<210> 770
<211> 20
<212> RNA
<213> Homo sapiens

<400> 770
aggaagcuga aggagaaaag 20


<210> 771
<211> 20
<212> RNA
<213> Homo sapiens

<400> 771
aggagaagca agcaggcgaa 20


<210> 772
<211> 20
<212> RNA
<213> Homo sapiens

<400> 772
agguguucga caauggcagc 20


<210> 773
<211> 20
<212> RNA
<213> Homo sapiens

<400> 773
agguuccuuu gguaucaucc 20


<210> 774
<211> 20
<212> RNA

<213>  Homo sapiens

<400>  774
agucccugug gaggaccugc                                                    20


<210>  775
<211>  20
<212>  RNA
<213>  Homo sapiens

<400>  775
agugggaugu ugaaguuucc                                                    20


<210>  776
<211>  20
<212>  RNA
<213>  Homo sapiens

<400>  776
aguuggggau uccaucauuu                                                    20


<210>  777
<211>  20
<212>  RNA
<213>  Homo sapiens

<400>  777
aucauaaucg gaggcuuugg                                                    20


<210>  778
<211>  20
<212>  RNA
<213>  Homo sapiens

<400>  778
augcuuugua agaaucguua                                                    20


<210>  779
<211>  20
<212>  RNA
<213>  Homo sapiens

<400>  779
augggggaac ugguuccagc                                                    20


<210>  780
<211>  20
<212>  RNA
<213>  Homo sapiens

<400>  780
auugcucuaa aauuucuaau                                                    20


<210>  781
<211>  20
<212>  RNA
<213>  Homo sapiens

<400>  781
cacaccuaca guggacuacc                                                    20


<210>  782
<211>  20
<212>  RNA
<213>  Homo sapiens

<400>  782

caggcaagga agaugcugcc                                                    20

<210>  783
<211>  20
<212>  RNA
<213>  Homo sapiens

<400>  783
caggccuggg cuccaccugc                                                    20

<210>  784
<211>  20
<212>  RNA
<213>  Homo sapiens

<400>  784
cagucuggcu aaagguuugu                                                    20

<210>  785
<211>  20
<212>  RNA
<213>  Homo sapiens

<400>  785
cccugcaggc guccacacau                                                    20

<210>  786
<211>  20
<212>  RNA
<213>  Homo sapiens

<400>  786
cgccacuagc cgggcauggc                                                    20

<210>  787
<211>  20
<212>  RNA
<213>  Homo sapiens

<400>  787
cggaaggaac ucauggcucu                                                    20

<210>  788
<211>  20
<212>  RNA
<213>  Homo sapiens

<400>  788
cggcagcagc agcagcugag                                                    20

<210>  789
<211>  20
<212>  RNA
<213>  Homo sapiens

<400>  789
cggggugucc cggcgaccca                                                    20

<210>  790
<211>  20
<212>  RNA
<213>  Homo sapiens

<400>  790
   cucuccugac cgugggggcc                                                     20

<210> 791
<211> 20
<212> RNA
<213> Homo sapiens

<400> 791
gacuuacagg ugcgugccac 20


<210> 792
<211> 20
<212> RNA
<213> Homo sapiens

<400> 792
gagcacagcu ggccgugguu 20


<210> 793
<211> 20
<212> RNA
<213> Homo sapiens

<400> 793
gagcccgcag ggcagcguca 20


<210> 794
<211> 20
<212> RNA
<213> Homo sapiens

<400> 794
gaggcccaag gucacggugu 20


<210> 795
<211> 20
<212> RNA
<213> Homo sapiens

<400> 795
gauuuacggc cgcaggugcg 20


<210> 796
<211> 20
<212> RNA
<213> Homo sapiens

<400> 796
gccagggagc uugcaggaag 20


<210> 797
<211> 20
<212> RNA
<213> Homo sapiens

<400> 797
gcuuuggcaa accaaguuuc 20


<210> 798
<211> 20
<212> RNA
<213> Homo sapiens

<400> 798
gguggcccga uucagcucuc 20


<210> 799
<211> 20
<212> RNA

<213>  Homo sapiens

<400>  799
gucauaaucc ugacccugag                                                    20


<210>  800
<211>  20
<212>  RNA
<213>  Homo sapiens

<400>  800
uaaauccucc cggccgcuca                                                    20


<210>  801
<211>  20
<212>  RNA
<213>  Homo sapiens

<400>  801
uagucuggga gcagacagag                                                    20


<210>  802
<211>  20
<212>  RNA
<213>  Homo sapiens

<400>  802
uccauuugcu gagcauacuu                                                    20


<210>  803
<211>  20
<212>  RNA
<213>  Homo sapiens

<400>  803
ugcagacagg gcaaagaaaa                                                    20


<210>  804
<211>  20
<212>  RNA
<213>  Homo sapiens

<400>  804
ugccccuucg cuccugccug                                                    20


<210>  805
<211>  20
<212>  RNA
<213>  Homo sapiens

<400>  805
uggcaagggc auccuggcug                                                    20


<210>  806
<211>  20
<212>  RNA
<213>  Homo sapiens

<400>  806
uggccgcucu cgggacuccc                                                    20


<210>  807
<211>  20
<212>  RNA
<213>  Homo sapiens

<400>  807

ugggcacccg cgacgacgag                                                    20

<210>  808
<211>  20
<212>  RNA
<213>  Homo sapiens

<400>  808
uugcuaaccc ugccuuccca                                                    20

<210>  809
<211>  20
<212>  RNA
<213>  Homo sapiens

<400>  809
uuggaauccc uguugcuaca                                                    20

<210>  810
<211>  20
<212>  RNA
<213>  Homo sapiens

<400>  810
uuguccggag aguauauugu                                                    20

<210>  811
<211>  20
<212>  RNA
<213>  Homo sapiens

<400>  811
uuuggaccca gcagaaagca                                                    20

<210>  812
<211>  19
<212>  RNA
<213>  Homo sapiens

<400>  812
aaagaccuca gaagcacuc                                                     19

<210>  813
<211>  19
<212>  RNA
<213>  Homo sapiens

<400>  813
aaggcccgca gccucuuug                                                     19

<210>  814
<211>  19
<212>  RNA
<213>  Homo sapiens

<400>  814
aaguauuguu gcuuugaaa                                                     19

<210>  815
<211>  19
<212>  RNA
<213>  Homo sapiens

<400>  815
acuuugccaa agcugucau                                                     19

<210> 816
<211> 19
<212> RNA
<213> Homo sapiens

<400> 816
agaagaagau ggucagccu 19

<210> 817
<211> 19
<212> RNA
<213> Homo sapiens

<400> 817
agaccucauc acaacagag 19

<210> 818
<211> 19
<212> RNA
<213> Homo sapiens

<400> 818
aggccaagcc aggcgucug 19

<210> 819
<211> 19
<212> RNA
<213> Homo sapiens

<400> 819
agugcgcagu augaggacc 19

<210> 820
<211> 19
<212> RNA
<213> Homo sapiens

<400> 820
cacaaccacu ugggacaga 19

<210> 821
<211> 19
<212> RNA
<213> Homo sapiens

<400> 821
cagaggcugg gaugguag 19

<210> 822
<211> 19
<212> RNA
<213> Homo sapiens

<400> 822
cgcuauucuc ugcugcagu 19

<210> 823
<211> 19
<212> RNA
<213> Homo sapiens

<400> 823
cucagcaucu ggugagggc 19

<210> 824
<211> 19
<212> RNA

<213>  Homo sapiens

<400>  824
cugggcuaca uguggccug                                                    19


<210>  825
<211>  19
<212>  RNA
<213>  Homo sapiens

<400>  825
gaaaaugcug agucagaau                                                    19


<210>  826
<211>  19
<212>  RNA
<213>  Homo sapiens

<400>  826
gaaagagaaa ugaaggaaa                                                    19


<210>  827
<211>  19
<212>  RNA
<213>  Homo sapiens

<400>  827
gacugaaugc cauuugucc                                                    19


<210>  828
<211>  19
<212>  RNA
<213>  Homo sapiens

<400>  828
gggaaaugcu gcaaagaau                                                    19


<210>  829
<211>  19
<212>  RNA
<213>  Homo sapiens

<400>  829
uccgacuugc cauggucgu                                                    19


<210>  830
<211>  19
<212>  RNA
<213>  Homo sapiens

<400>  830
ucuugcacag cacugcuaa                                                    19


<210>  831
<211>  19
<212>  RNA
<213>  Homo sapiens

<400>  831
uggacaucga ggcgcugaa                                                    19


<210>  832
<211>  19
<212>  RNA
<213>  Homo sapiens

<400>  832

uguugccagu gucugucug 19

<210> 833
<211> 19
<212> RNA
<213> Homo sapiens

<400> 833
uuccgcgacu accggcgug 19

<210> 834
<211> 19
<212> RNA
<213> Homo sapiens

<400> 834
uugcaguggg ugccaccca 19

<210> 835
<211> 18
<212> RNA
<213> Homo sapiens

<400> 835
aaaaaugcuc auugaaaa 18

<210> 836
<211> 18
<212> RNA
<213> Homo sapiens

<400> 836
aaagaaucac cagugcau 18

<210> 837
<211> 18
<212> RNA
<213> Homo sapiens

<400> 837
aacaaccacu guggaugu 18

<210> 838
<211> 18
<212> RNA
<213> Homo sapiens

<400> 838
aacccaggga acugaaac 18

<210> 839
<211> 18
<212> RNA
<213> Homo sapiens

<400> 839
aacugccuau gauucuuu 18

<210> 840
<211> 18
<212> RNA
<213> Homo sapiens

<400> 840
aaguugauug aauguugu 18

<210>  841
<211>  18
<212>  RNA
<213>  Homo sapiens

<400>  841
accaggcucc ugcagacc                                                    18


<210>  842
<211>  18
<212>  RNA
<213>  Homo sapiens

<400>  842
acuuaugggu ugcugaaa                                                    18


<210>  843
<211>  18
<212>  RNA
<213>  Homo sapiens

<400>  843
agacauggca acuuccug                                                    18


<210>  844
<211>  18
<212>  RNA
<213>  Homo sapiens

<400>  844
agagagaggc acccugga                                                    18


<210>  845
<211>  18
<212>  RNA
<213>  Homo sapiens

<400>  845
aggacuuugg ucgagcgg                                                    18


<210>  846
<211>  18
<212>  RNA
<213>  Homo sapiens

<400>  846
aguccuuccu ccccugcc                                                    18


<210>  847
<211>  18
<212>  RNA
<213>  Homo sapiens

<400>  847
aguugaccag ugcagagc                                                    18


<210>  848
<211>  18
<212>  RNA
<213>  Homo sapiens

<400>  848
auccugaugc uucaaaac                                                    18


<210>  849
<211>  18
<212>  RNA

<213>  Homo sapiens

<400>  849
augaaugugg ugcauuac                                                    18


<210>  850
<211>  18
<212>  RNA
<213>  Homo sapiens

<400>  850
   augcacagcc auggggca                                                     18


<210>  851
<211>  18
<212>  RNA
<213>  Homo sapiens

<400>  851
augggcuaug gaguugag                                                    18


<210>  852
<211>  18
<212>  RNA
<213>  Homo sapiens

<400>  852
   auuaaacuuu cucuuuuc                                                     18


<210>  853
<211>  18
<212>  RNA
<213>  Homo sapiens

<400>  853
auuccagagu gcugaggc                                                    18


<210>  854
<211>  18
<212>  RNA
<213>  Homo sapiens

<400>  854
cagagcuuug ggauguca                                                    18


<210>  855
<211>  18
<212>  RNA
<213>  Homo sapiens

<400>  855
cagcaccucu agugacca                                                    18


<210>  856
<211>  18
<212>  RNA
<213>  Homo sapiens

<400>  856
cagcuuggga agaaugag                                                    18


<210>  857
<211>  18
<212>  RNA
<213>  Homo sapiens

<400>  857

cccgccaugu ucugugug 18

<210> 858
<211> 18
<212> RNA
<213> Homo sapiens

<400> 858
   cgcccggcgc cggagucc 18

<210> 859
<211> 18
<212> RNA
<213> Homo sapiens

<400> 859
cggaccaaaa cugcagag 18

<210> 860
<211> 18
<212> RNA
<213> Homo sapiens

<400> 860
cucaacucug ugaugugg 18

<210> 861
<211> 18
<212> RNA
<213> Homo sapiens

<400> 861
cugacggugc aggagggu 18

<210> 862
<211> 18
<212> RNA
<213> Homo sapiens

<400> 862
cugccugcuc cugccugu 18

<210> 863
<211> 18
<212> RNA
<213> Homo sapiens

<400> 863
cuucucuucc uggcaggc 18

<210> 864
<211> 18
<212> RNA
<213> Homo sapiens

<400> 864
gaaccagcug cuaggccc 18

<210> 865
<211> 18
<212> RNA
<213> Homo sapiens

<400> 865
gacgugggug ucccugag 18

<210> 866
<211> 18
<212> RNA
<213> Homo sapiens

<400> 866
gagccgggcu caccaggu 18


<210> 867
<211> 18
<212> RNA
<213> Homo sapiens

<400> 867
gcaggagggc uggggaag 18


<210> 868
<211> 18
<212> RNA
<213> Homo sapiens

<400> 868
gcauuggagu caggcaga 18


<210> 869
<211> 18
<212> RNA
<213> Homo sapiens

<400> 869
gccauuuuga gagcgagc 18


<210> 870
<211> 18
<212> RNA
<213> Homo sapiens

<400> 870
gcggcggcgc gaggaggc 18


<210> 871
<211> 18
<212> RNA
<213> Homo sapiens

<400> 871
gugacucagc auccucaa 18


<210> 872
<211> 18
<212> RNA
<213> Homo sapiens

<400> 872
gugcgagagc uggacgga 18


<210> 873
<211> 18
<212> RNA
<213> Homo sapiens

<400> 873
uacccuugau guuuccuc 18


<210> 874
<211> 18
<212> RNA

<213>  Homo sapiens

<400>  874
ucauccaggc agagaggc                                                    18


<210>  875
<211>  18
<212>  RNA
<213>  Homo sapiens

<400>  875
ucugcccucu uaagcagc                                                    18


<210>  876
<211>  18
<212>  RNA
<213>  Homo sapiens

<400>  876
ucuuugugcu gcaguggu                                                    18


<210>  877
<211>  18
<212>  RNA
<213>  Homo sapiens

<400>  877
ugggccuauu ucuccuga                                                    18


<210>  878
<211>  18
<212>  RNA
<213>  Homo sapiens

<400>  878
uuaaagcgcu gauuagaa                                                    18


<210>  879
<211>  18
<212>  RNA
<213>  Homo sapiens

<400>  879
uuagaagaug gugcuauc                                                    18


<210>  880
<211>  18
<212>  RNA
<213>  Homo sapiens

<400>  880
uuagagacgg ggucuuuc                                                    18


<210>  881
<211>  18
<212>  RNA
<213>  Homo sapiens

<400>  881
uucgucuuug gucacagc                                                    18


<210>  882
<211>  18
<212>  RNA
<213>  Homo sapiens

<400>  882

uucuggcuca agggcuau                                                    18

<210>  883
<211>  18
<212>  RNA
<213>  Homo sapiens

<400>  883
uucuuccugc aucccgug                                                    18

<210>  884
<211>  18
<212>  RNA
<213>  Homo sapiens

<400>  884
uugccucuaa uccugaug                                                    18

<210>  885
<211>  18
<212>  RNA
<213>  Homo sapiens

<400>  885
uuugcagaug gugaagag                                                    18

<210>  886
<211>  17
<212>  RNA
<213>  Homo sapiens

<400>  886
aaacaaauuu uaugugu                                                     17

<210>  887
<211>  17
<212>  RNA
<213>  Homo sapiens

<400>  887
aaacaucugc aagaauu                                                     17

<210>  888
<211>  17
<212>  RNA
<213>  Homo sapiens

<400>  888
aaauuaguuu gggaaac                                                     17

<210>  889
<211>  17
<212>  RNA
<213>  Homo sapiens

<400>  889
aaccagggcu aucucuc                                                     17

<210>  890
<211>  17
<212>  RNA
<213>  Homo sapiens

<400>  890
aacuaucggg gcucugc                                                     17

<210> 891
<211> 17
<212> RNA
<213> Homo sapiens

<400> 891
aagaugaagc aagaugc 17


<210> 892
<211> 17
<212> RNA
<213> Homo sapiens

<400> 892
aagcaacugu gggcgac 17


<210> 893
<211> 17
<212> RNA
<213> Homo sapiens

<400> 893
acagaaaucu cauguag 17


<210> 894
<211> 17
<212> RNA
<213> Homo sapiens

<400> 894
accagggcug gagcuug 17


<210> 895
<211> 17
<212> RNA
<213> Homo sapiens

<400> 895
agaaaccagu gagaggu 17


<210> 896
<211> 17
<212> RNA
<213> Homo sapiens

<400> 896
agaagacucu guucauc 17


<210> 897
<211> 17
<212> RNA
<213> Homo sapiens

<400> 897
agaagagaac ucggaag 17


<210> 898
<211> 17
<212> RNA
<213> Homo sapiens

<400> 898
agcuggaaga uggccgc 17


<210> 899
<211> 17
<212> RNA

<213>  Homo sapiens

<400>  899
agggguaaug ccacuug                                                    17


<210>  900
<211>  17
<212>  RNA
<213>  Homo sapiens

<400>  900
augcuacaug gaaaauc                                                    17


<210>  901
<211>  17
<212>  RNA
<213>  Homo sapiens

<400>  901
auucuuucug ggauaaa                                                    17


<210>  902
<211>  17
<212>  RNA
<213>  Homo sapiens

<400>  902
auuggucaga cauccug                                                    17


<210>  903
<211>  17
<212>  RNA
<213>  Homo sapiens

<400>  903
cagcugcacu gauguug                                                    17


<210>  904
<211>  17
<212>  RNA
<213>  Homo sapiens

<400>  904
caggcccugu gcacccu                                                    17


<210>  905
<211>  17
<212>  RNA
<213>  Homo sapiens

<400>  905
cggcggcggg agaagac                                                    17


<210>  906
<211>  17
<212>  RNA
<213>  Homo sapiens

<400>  906
cucuguugac caucuuc                                                    17


<210>  907
<211>  17
<212>  RNA
<213>  Homo sapiens

<400>  907

cugguucgga uauuugg                                                    17

<210>  908
<211>  17
<212>  RNA
<213>  Homo sapiens

<400>  908
cuugaagugu caggagc                                                    17

<210>  909
<211>  17
<212>  RNA
<213>  Homo sapiens

<400>  909
gcaaggcgaa gcugaga                                                    17

<210>  910
<211>  17
<212>  RNA
<213>  Homo sapiens

<400>  910
gccuuguucu guuuaaa                                                    17

<210>  911
<211>  17
<212>  RNA
<213>  Homo sapiens

<400>  911
gguaguauug uacaguu                                                    17

<210>  912
<211>  17
<212>  RNA
<213>  Homo sapiens

<400>  912
uggucccugu ucaacac                                                    17

<210>  913
<211>  17
<212>  RNA
<213>  Homo sapiens

<400>  913
ugugaaaugu gcagaua                                                    17

<210>  914
<211>  17
<212>  RNA
<213>  Homo sapiens

<400>  914
uuguccuggg cgaaugg                                                    17

<210>  915
<211>  16
<212>  RNA
<213>  Homo sapiens

<400>  915
aaugccugug agccag                                                     16

<210> 916
<211> 16
<212> RNA
<213> Homo sapiens

<400> 916
acaauguucu ggcaca 16


<210> 917
<211> 16
<212> RNA
<213> Homo sapiens

<400> 917
accccauaaa caccaa 16


<210> 918
<211> 16
<212> RNA
<213> Homo sapiens

<400> 918
acccucagcc aguggg 16


<210> 919
<211> 16
<212> RNA
<213> Homo sapiens

<400> 919
agagacugag cuauaa 16


<210> 920
<211> 16
<212> RNA
<213> Homo sapiens

<400> 920
aggcccugga gagaau 16


<210> 921
<211> 16
<212> RNA
<213> Homo sapiens

<400> 921
aucaucuggc ucaguu 16


<210> 922
<211> 16
<212> RNA
<213> Homo sapiens

<400> 922
cacugcccag gcgacg 16


<210> 923
<211> 16
<212> RNA
<213> Homo sapiens

<400> 923
cagcgugcug acagug 16


<210> 924
<211> 16
<212> RNA

<213>  Homo sapiens

<400>  924
cccucucucc gccagg                                                        16


<210>  925
<211>  16
<212>  RNA
<213>  Homo sapiens

<400>  925
cggcaggcag caagga                                                        16


<210>  926
<211>  16
<212>  RNA
<213>  Homo sapiens

<400>  926
cggugguucu guaugg                                                        16


<210>  927
<211>  16
<212>  RNA
<213>  Homo sapiens

<400>  927
cuaacaggug caggcu                                                        16


<210>  928
<211>  16
<212>  RNA
<213>  Homo sapiens

<400>  928
cucauagucc uagcca                                                        16


<210>  929
<211>  16
<212>  RNA
<213>  Homo sapiens

<400>  929
cuggcucagg augaac                                                        16


<210>  930
<211>  16
<212>  RNA
<213>  Homo sapiens

<400>  930
cugguggagu cagggg                                                        16


<210>  931
<211>  16
<212>  RNA
<213>  Homo sapiens

<400>  931
cugucaaggc ugcuuu                                                        16


<210>  932
<211>  16
<212>  RNA
<213>  Homo sapiens

<400>  932

gaaacugugg gcuugg                                                    16

<210>  933
<211>  16
<212>  RNA
<213>  Homo sapiens

<400>  933
gcuccagggg caaagg                                                    16

<210>  934
<211>  16
<212>  RNA
<213>  Homo sapiens

<400>  934
gggcgcggua cucagc                                                    16

<210>  935
<211>  16
<212>  RNA
<213>  Homo sapiens

<400>  935
gucaagacug ccagcc                                                    16

<210>  936
<211>  16
<212>  RNA
<213>  Homo sapiens

<400>  936
guuacacuuc ccagaa                                                    16

<210>  937
<211>  16
<212>  RNA
<213>  Homo sapiens

<400>  937
uggcuucuga gacagc                                                    16

<210>  938
<211>  16
<212>  RNA
<213>  Homo sapiens

<400>  938
uugcacauga caucaa                                                    16

<210>  939
<211>  15
<212>  RNA
<213>  Homo sapiens

<400>  939
cuccgcgucc cccga                                                     15

<210>  940
<211>  15
<212>  RNA
<213>  Homo sapiens

<400>  940
cugagugcga gcucc                                                     15

<210> 941
<211> 15
<212> RNA
<213> Homo sapiens

<400> 941
gaugugguga cgucg 15

<210> 942
<211> 15
<212> RNA
<213> Homo sapiens

<400> 942
uaccccaucu gaaau 15

<210> 943
<211> 15
<212> RNA
<213> Homo sapiens

<400> 943
ucuugggucg aacau 15

<210> 944
<211> 22
<212> RNA
<213> Homo sapiens

<400> 944
aaaacauccu ugccucagag gg 22

<210> 945
<211> 22
<212> RNA
<213> Homo sapiens

<400> 945
aaacaacaau uaccugauuu au 22

<210> 946
<211> 22
<212> RNA
<213> Homo sapiens

<400> 946
aaacuguggu uugcuccaca ga 22

<210> 947
<211> 22
<212> RNA
<213> Homo sapiens

<400> 947
aaaguugugu caucugcaaa aa 22

<210> 948
<211> 22
<212> RNA
<213> Homo sapiens

<400> 948
aaaugagcaa cagcaaggga aa 22

<210> 949
<211> 22
<212> RNA

<213>  Homo sapiens

<400>  949
aaauguaauu gcgguuuuug cu                                                22


<210>  950
<211>  22
<212>  RNA
<213>  Homo sapiens

<400>  950
aacaaauccu ggaccaccug aa                                                22


<210>  951
<211>  22
<212>  RNA
<213>  Homo sapiens

<400>  951
aacuccccug gagauaagua ag                                                22


<210>  952
<211>  22
<212>  RNA
<213>  Homo sapiens

<400>  952
aacuggauug uuugucacac aa                                                22


<210>  953
<211>  22
<212>  RNA
<213>  Homo sapiens

<400>  953
aagcagggag gcaaagcucg cg                                                22


<210>  954
<211>  22
<212>  RNA
<213>  Homo sapiens

<400>  954
aagcggcuuc agcagcuagc gg                                                22


<210>  955
<211>  22
<212>  RNA
<213>  Homo sapiens

<400>  955
aaggccaugu cacugggccu ug                                                22


<210>  956
<211>  22
<212>  RNA
<213>  Homo sapiens

<400>  956
aaggccugau aauacacaaa au                                                22


<210>  957
<211>  22
<212>  RNA
<213>  Homo sapiens

<400>  957

aaggcucgaa gccgugugcg gg                                                22

<210>  958
<211>  22
<212>  RNA
<213>  Homo sapiens

<400>  958
aaggcuuugg cagaggccua aa                                                22

<210>  959
<211>  22
<212>  RNA
<213>  Homo sapiens

<400>  959
aaugaaaggu ccuauuuguu aa                                                22

<210>  960
<211>  22
<212>  RNA
<213>  Homo sapiens

<400>  960
aaugcacuga uguuuucaua au                                                22

<210>  961
<211>  22
<212>  RNA
<213>  Homo sapiens

<400>  961
acagcuucuc uaugugugga uu                                                22

<210>  962
<211>  22
<212>  RNA
<213>  Homo sapiens

<400>  962
accagucaga ccgauauguc aa                                                22

<210>  963
<211>  22
<212>  RNA
<213>  Homo sapiens

<400>  963
accaguccuc gcauguggac ga                                                22

<210>  964
<211>  22
<212>  RNA
<213>  Homo sapiens

<400>  964
accaugcuag gccacuugcu gg                                                22

<210>  965
<211>  22
<212>  RNA
<213>  Homo sapiens

<400>  965
accuuccugg gggccuuggc uu                                                22

<210>  966
<211>  22
<212>  RNA
<213>  Homo sapiens

<400>  966
acgcauuccg uaugaugauu au                                                22


<210>  967
<211>  22
<212>  RNA
<213>  Homo sapiens

<400>  967
acggcaucgu caccaacugg ga                                                22


<210>  968
<211>  22
<212>  RNA
<213>  Homo sapiens

<400>  968
agaagggguc cuggugacga gu                                                22


<210>  969
<211>  22
<212>  RNA
<213>  Homo sapiens

<400>  969
agagaaucaa augcuaacga aa                                                22


<210>  970
<211>  22
<212>  RNA
<213>  Homo sapiens

<400>  970
agaggcuuug ugcggauacg gg                                                22


<210>  971
<211>  22
<212>  RNA
<213>  Homo sapiens

<400>  971
agaggugaag uccccagugg ag                                                22


<210>  972
<211>  22
<212>  RNA
<213>  Homo sapiens

<400>  972
agaguguuca gccagagaua uu                                                22


<210>  973
<211>  22
<212>  RNA
<213>  Homo sapiens

<400>  973
agccguggcc cggcggccug gg                                                22


<210>  974
<211>  22
<212>  RNA

<213>  Homo sapiens

<400>  974
agcgcggccg gcgcuccagc gg                                                22


<210>  975
<211>  22
<212>  RNA
<213>  Homo sapiens

<400>  975
aggagaccgg gugauggugu ug                                                22


<210>  976
<211>  22
<212>  RNA
<213>  Homo sapiens

<400>  976
aggcccccgc ugccuguccg cg                                                22


<210>  977
<211>  22
<212>  RNA
<213>  Homo sapiens

<400>  977
aggcugcggg cggccggcua gg                                                22


<210>  978
<211>  22
<212>  RNA
<213>  Homo sapiens

<400>  978
agggcuccag gcguccccga au                                                22


<210>  979
<211>  22
<212>  RNA
<213>  Homo sapiens

<400>  979
   agggggagcg ggagggcagg ga                                               22


<210>  980
<211>  22
<212>  RNA
<213>  Homo sapiens

<400>  980
aggucgcugu ccccguggua gg                                                22


<210>  981
<211>  22
<212>  RNA
<213>  Homo sapiens

<400>  981
aguagccagu auuucugauu aa                                                22


<210>  982
<211>  22
<212>  RNA
<213>  Homo sapiens

<400>  982

agugccccga gugcuggcac ug                                                22

<210>  983
<211>  22
<212>  RNA
<213>  Homo sapiens

<400>  983
aguguccegg gcccguugag ga                                                22

<210>  984
<211>  22
<212>  RNA
<213>  Homo sapiens

<400>  984
aguuccuugc cugugaacgg gg                                                22

<210>  985
<211>  22
<212>  RNA
<213>  Homo sapiens

<400>  985
auaccugggu aaaggaaaug aa                                                22

<210>  986
<211>  22
<212>  RNA
<213>  Homo sapiens

<400>  986
aucacugagc caggagcugu gg                                                22

<210>  987
<211>  22
<212>  RNA
<213>  Homo sapiens

<400>  987
auguccccca gaacucgggg ag                                                22

<210>  988
<211>  22
<212>  RNA
<213>  Homo sapiens

<400>  988
auguguuggu gacuuuuugu uu                                                22

<210>  989
<211>  22
<212>  RNA
<213>  Homo sapiens

<400>  989
auugcaagag cagcgugacu ga                                                22

<210>  990
<211>  22
<212>  RNA
<213>  Homo sapiens

<400>  990
auuggcaaaa accgcaauua uu                                                22

<210> 991
<211> 22
<212> RNA
<213> Homo sapiens

<400> 991
auuugucaga cuuuuccuuu uu 22

<210> 992
<211> 22
<212> RNA
<213> Homo sapiens

<400> 992
cacagcucug cucggcuucu uu 22

<210> 993
<211> 22
<212> RNA
<213> Homo sapiens

<400> 993
cagcaaucag acugucgaca uu 22

<210> 994
<211> 22
<212> RNA
<213> Homo sapiens

<400> 994
caggcugaug agacuaaggc ga 22

<210> 995
<211> 22
<212> RNA
<213> Homo sapiens

<400> 995
cccagccugc aggugagcuc ug 22

<210> 996
<211> 22
<212> RNA
<213> Homo sapiens

<400> 996
cccugugcag aaacugucau ug 22

<210> 997
<211> 22
<212> RNA
<213> Homo sapiens

<400> 997
ccugagguau caggcugaag ug 22

<210> 998
<211> 22
<212> RNA
<213> Homo sapiens

<400> 998
cgccgccgcg gggaaggcgg gg 22

<210> 999
<211> 22
<212> RNA

<213>  Homo sapiens

<400>  999
cggggcccug gacgugggag cu                                                22


<210>  1000
<211>  22
<212>  RNA
<213>  Homo sapiens

<400>  1000
cucaggaguc acugggaaga ug                                                22


<210>  1001
<211>  22
<212>  RNA
<213>  Homo sapiens

<400>  1001
cucauuccca uugaguugug ug                                                22


<210>  1002
<211>  22
<212>  RNA
<213>  Homo sapiens

<400>  1002
cucggaggag caguucuggg ag                                                22


<210>  1003
<211>  22
<212>  RNA
<213>  Homo sapiens

<400>  1003
cucugaggcc cuggcaucuu uu                                                22


<210>  1004
<211>  22
<212>  RNA
<213>  Homo sapiens

<400>  1004
cugaccuugg uacccaccgu gg                                                22


<210>  1005
<211>  22
<212>  RNA
<213>  Homo sapiens

<400>  1005
cugccuguga uaagcaggag ga                                                22


<210>  1006
<211>  22
<212>  RNA
<213>  Homo sapiens

<400>  1006
cuggcggagc agcugcggcg cg                                                22


<210>  1007
<211>  22
<212>  RNA
<213>  Homo sapiens

<400>  1007

gaaaggagcc cgcaggcggc gg                                                22

<210>  1008
<211>  22
<212>  RNA
<213>  Homo sapiens

<400>  1008
gaacagcgag aggggaggaa gu                                                22

<210>  1009
<211>  22
<212>  RNA
<213>  Homo sapiens

<400>  1009
gacuagcagc acauaauggu uu                                                22

<210>  1010
<211>  22
<212>  RNA
<213>  Homo sapiens

<400>  1010
gaggcgccgg cgguguuccu gg                                                22

<210>  1011
<211>  22
<212>  RNA
<213>  Homo sapiens

<400>  1011
gaugcugccc ugcuacggua ua                                                22

<210>  1012
<211>  22
<212>  RNA
<213>  Homo sapiens

<400>  1012
gcacacugga augugggucc cu                                                22

<210>  1013
<211>  22
<212>  RNA
<213>  Homo sapiens

<400>  1013
gccagacgcg ccggagcggg cg                                                22

<210>  1014
<211>  22
<212>  RNA
<213>  Homo sapiens

<400>  1014
gccagcugug ccggcguuug uu                                                22

<210>  1015
<211>  22
<212>  RNA
<213>  Homo sapiens

<400>  1015
gccauucgau gaugauucca uu                                                22

<210>  1016
<211>  22
<212>  RNA
<213>  Homo sapiens

<400>  1016
gccugcuuuu cugguucucu gu                                                22


<210>  1017
<211>  22
<212>  RNA
<213>  Homo sapiens

<400>  1017
gcgagaaagg uuuucgccuc cu                                                22


<210>  1018
<211>  22
<212>  RNA
<213>  Homo sapiens

<400>  1018
gcucccagug ccaucagugu ga                                                22


<210>  1019
<211>  22
<212>  RNA
<213>  Homo sapiens

<400>  1019
gcucccuguc gcugccggag ag                                                22


<210>  1020
<211>  22
<212>  RNA
<213>  Homo sapiens

<400>  1020
ggagcaggaa gcuaugcuaa uu                                                22


<210>  1021
<211>  22
<212>  RNA
<213>  Homo sapiens

<400>  1021
gggaccugcc cuggaggcuc uu                                                22


<210>  1022
<211>  22
<212>  RNA
<213>  Homo sapiens

<400>  1022
gggagcaugg aggauagauu uu                                                22


<210>  1023
<211>  22
<212>  RNA
<213>  Homo sapiens

<400>  1023
gggcccaccc agaugagcuc au                                                22


<210>  1024
<211>  22
<212>  RNA

<213>  Homo sapiens

<400>  1024
ggguaucaau ggcaucaggg aa                                                22


<210>  1025
<211>  22
<212>  RNA
<213>  Homo sapiens

<400>  1025
ggucccugag caaggagggg gu                                                22


<210>  1026
<211>  22
<212>  RNA
<213>  Homo sapiens

<400>  1026
guccucuccu cccaagaguu gu                                                22


<210>  1027
<211>  22
<212>  RNA
<213>  Homo sapiens

<400>  1027
gugacaggcu ggagugcagu gg                                                22


<210>  1028
<211>  22
<212>  RNA
<213>  Homo sapiens

<400>  1028
gugacgaggc uaaggccgug ga                                                22


<210>  1029
<211>  22
<212>  RNA
<213>  Homo sapiens

<400>  1029
gugaugauuc ugaccuacaa au                                                22


<210>  1030
<211>  22
<212>  RNA
<213>  Homo sapiens

<400>  1030
gugccuggcc ugcggcccca gg                                                22


<210>  1031
<211>  22
<212>  RNA
<213>  Homo sapiens

<400>  1031
uacagccucc agaacccuga gg                                                22


<210>  1032
<211>  22
<212>  RNA
<213>  Homo sapiens

<400>  1032

ucaacccucu caggugacca aa                                                22

<210>  1033
<211>  22
<212>  RNA
<213>  Homo sapiens

<400>  1033
ucagcguugg uucccgucuu gg                                                22

<210>  1034
<211>  22
<212>  RNA
<213>  Homo sapiens

<400>  1034
uccccacauu cugugagcug ug                                                22

<210>  1035
<211>  22
<212>  RNA
<213>  Homo sapiens

<400>  1035
uccggaagcc gccgagcccg gg                                                22

<210>  1036
<211>  22
<212>  RNA
<213>  Homo sapiens

<400>  1036
uccugaccug cucggaguug aa                                                22

<210>  1037
<211>  22
<212>  RNA
<213>  Homo sapiens

<400>  1037
ucuggaaagc ggucugcccc ug                                                22

<210>  1038
<211>  22
<212>  RNA
<213>  Homo sapiens

<400>  1038
ugaccgugga aaaaguacag gg                                                22

<210>  1039
<211>  22
<212>  RNA
<213>  Homo sapiens

<400>  1039
ugagccucug cucccggccu aa                                                22

<210>  1040
<211>  22
<212>  RNA
<213>  Homo sapiens

<400>  1040
ugaugcuuca gucacgacau ga                                                22

<210> 1041
<211> 22
<212> RNA
<213> Homo sapiens

<400> 1041
ugcccagcaa gcugcgcucc cu 22


<210> 1042
<211> 22
<212> RNA
<213> Homo sapiens

<400> 1042
ugccccugag agggagcggc gg 22


<210> 1043
<211> 22
<212> RNA
<213> Homo sapiens

<400> 1043
ugcccguugu cuaggagaca ga 22


<210> 1044
<211> 22
<212> RNA
<213> Homo sapiens

<400> 1044
ugcuguccau gggcuucucu ga 22


<210> 1045
<211> 22
<212> RNA
<213> Homo sapiens

<400> 1045
uggaaaguca cccguaagga ug 22


<210> 1046
<211> 22
<212> RNA
<213> Homo sapiens

<400> 1046
uggaacgccc cggcugggug gu 22


<210> 1047
<211> 22
<212> RNA
<213> Homo sapiens

<400> 1047
ugggcugcgg ugggcucggc ug 22


<210> 1048
<211> 22
<212> RNA
<213> Homo sapiens

<400> 1048
ugggucuggc ugcugccgcc cg 22


<210> 1049
<211> 22
<212> RNA

<213>  Homo sapiens

<400>  1049
ugucauuucc aaaauacugg uu                                                22


<210>  1050
<211>  22
<212>  RNA
<213>  Homo sapiens

<400>  1050
uguucuccac gccgcacggg cu                                                22


<210>  1051
<211>  22
<212>  RNA
<213>  Homo sapiens

<400>  1051
uuccggaugg ugagcggcga ag                                                22


<210>  1052
<211>  22
<212>  RNA
<213>  Homo sapiens

<400>  1052
uugaccucac caagagggcg gg                                                22


<210>  1053
<211>  22
<212>  RNA
<213>  Homo sapiens

<400>  1053
uugcuuucca uagacauaga uu                                                22


<210>  1054
<211>  22
<212>  RNA
<213>  Homo sapiens

<400>  1054
uuggccagga agggcaggag ag                                                22


<210>  1055
<211>  22
<212>  RNA
<213>  Homo sapiens

<400>  1055
uugugaauuu ggcuccgcga ag                                                22


<210>  1056
<211>  22
<212>  RNA
<213>  Homo sapiens

<400>  1056
uugugcuccu uucauuccug aa                                                22


<210>  1057
<211>  22
<212>  RNA
<213>  Homo sapiens

<400>  1057

uuugcagccu gcaggggccu gg                                                22

<210>  1058
<211>  22
<212>  RNA
<213>  Homo sapiens

<400>  1058
uuugcagcuu ugaagcugcu gg                                                22

<210>  1059
<211>  22
<212>  RNA
<213>  Homo sapiens

<400>  1059
uuuguggaaa guugugaaga ua                                                22

<210>  1060
<211>  21
<212>  RNA
<213>  Homo sapiens

<400>  1060
aaaacuucac auaguuugau u                                                 21

<210>  1061
<211>  21
<212>  RNA
<213>  Homo sapiens

<400>  1061
aaacggcaau gacuuuugua c                                                 21

<210>  1062
<211>  21
<212>  RNA
<213>  Homo sapiens

<400>  1062
aaagacugcg ugucaagauu c                                                 21

<210>  1063
<211>  21
<212>  RNA
<213>  Homo sapiens

<400>  1063
aacgagcgcc ugcacagggc c                                                 21

<210>  1064
<211>  21
<212>  RNA
<213>  Homo sapiens

<400>  1064
aaggccaaga aggaggcgcc g                                                 21

<210>  1065
<211>  21
<212>  RNA
<213>  Homo sapiens

<400>  1065
aagucauugg aggguuugag c                                                 21

<210>  1066
<211>  21
<212>  RNA
<213>  Homo sapiens

<400>  1066
aagucgcuug ggcuucgccc g                                                21


<210>  1067
<211>  21
<212>  RNA
<213>  Homo sapiens

<400>  1067
acaccacuac uggacacggu c                                                21


<210>  1068
<211>  21
<212>  RNA
<213>  Homo sapiens

<400>  1068
accacuaagc gguucccuuc a                                                21


<210>  1069
<211>  21
<212>  RNA
<213>  Homo sapiens

<400>  1069
acggugcugg auguggccuu u                                                21


<210>  1070
<211>  21
<212>  RNA
<213>  Homo sapiens

<400>  1070
acucguccgc uccgcucugg a                                                21


<210>  1071
<211>  21
<212>  RNA
<213>  Homo sapiens

<400>  1071
acuucgaccu gagcugggag g                                                21


<210>  1072
<211>  21
<212>  RNA
<213>  Homo sapiens

<400>  1072
agaauuuugu uugaaauccu u                                                21


<210>  1073
<211>  21
<212>  RNA
<213>  Homo sapiens

<400>  1073
agaggagcga gcuggacgcc a                                                21


<210>  1074
<211>  21
<212>  RNA

<213>  Homo sapiens

<400>  1074
agaugcggcg uucccugccu c                                                21


<210>  1075
<211>  21
<212>  RNA
<213>  Homo sapiens

<400>  1075
agcccagacu ggacuagcaa g                                                21


<210>  1076
<211>  21
<212>  RNA
<213>  Homo sapiens

<400>  1076
agcgcaggga agccuggggg c                                                21


<210>  1077
<211>  21
<212>  RNA
<213>  Homo sapiens

<400>  1077
agcggaugcg ggaagccgga c                                                21


<210>  1078
<211>  21
<212>  RNA
<213>  Homo sapiens

<400>  1078
aggaagccgc gucguccggg g                                                21


<210>  1079
<211>  21
<212>  RNA
<213>  Homo sapiens

<400>  1079
aggacacuca uaaguacgag u                                                21


<210>  1080
<211>  21
<212>  RNA
<213>  Homo sapiens

<400>  1080
aggaggcucc ugcccucccg u                                                21


<210>  1081
<211>  21
<212>  RNA
<213>  Homo sapiens

<400>  1081
aggcaggggu agcccugggg u                                                21


<210>  1082
<211>  21
<212>  RNA
<213>  Homo sapiens

<400>  1082

aggccccugc aguauguggg c                                                    21

<210>  1083
<211>  21
<212>  RNA
<213>  Homo sapiens

<400>  1083
aggcgaagcu ggccgagcag g                                                    21

<210>  1084
<211>  21
<212>  RNA
<213>  Homo sapiens

<400>  1084
aggcggggcg aaggcggagc c                                                    21

<210>  1085
<211>  21
<212>  RNA
<213>  Homo sapiens

<400>  1085
aggugcuuug gucauuccuu u                                                    21

<210>  1086
<211>  21
<212>  RNA
<213>  Homo sapiens

<400>  1086
augcccucca ggcccugggg c                                                    21

<210>  1087
<211>  21
<212>  RNA
<213>  Homo sapiens

<400>  1087
augcugagcc ugcaauuugc u                                                    21

<210>  1088
<211>  21
<212>  RNA
<213>  Homo sapiens

<400>  1088
auugggcugg cagcaggacg a                                                    21

<210>  1089
<211>  21
<212>  RNA
<213>  Homo sapiens

<400>  1089
auuuucuggc cugagagaaa g                                                    21

<210>  1090
<211>  21
<212>  RNA
<213>  Homo sapiens

<400>  1090
cacuaugucc aaacuucuga c                                                    21

<210> 1091
<211> 21
<212> RNA
<213> Homo sapiens

<400> 1091
cagccucggc uacagcuucg g 21


<210> 1092
<211> 21
<212> RNA
<213> Homo sapiens

<400> 1092
caggcacaga gcugagcaag g 21


<210> 1093
<211> 21
<212> RNA
<213> Homo sapiens

<400> 1093
caggucuuca gacugugggg c 21


<210> 1094
<211> 21
<212> RNA
<213> Homo sapiens

<400> 1094
cagguuggcc uggcaugccc a 21


<210> 1095
<211> 21
<212> RNA
<213> Homo sapiens

<400> 1095
cgccaagcuc gccugcaccc c 21


<210> 1096
<211> 21
<212> RNA
<213> Homo sapiens

<400> 1096
cggcccagac cauccuggag a 21


<210> 1097
<211> 21
<212> RNA
<213> Homo sapiens

<400> 1097
cgggacccug uugggaacug u 21


<210> 1098
<211> 21
<212> RNA
<213> Homo sapiens

<400> 1098
cguggacgcc gccgaagaag c 21


<210> 1099
<211> 21
<212> RNA

<213>  Homo sapiens

<400>  1099
cucggacucc ugccucacuc a                                                21


<210>  1100
<211>  21
<212>  RNA
<213>  Homo sapiens

<400>  1100
cuggccaggc augguggcuc u                                                21


<210>  1101
<211>  21
<212>  RNA
<213>  Homo sapiens

<400>  1101
cugugagaau uuugggagug u                                                21


<210>  1102
<211>  21
<212>  RNA
<213>  Homo sapiens

<400>  1102
gaaacugggc aaaggagcag g                                                21


<210>  1103
<211>  21
<212>  RNA
<213>  Homo sapiens

<400>  1103
gaaugcucgu uagccaugug c                                                21


<210>  1104
<211>  21
<212>  RNA
<213>  Homo sapiens

<400>  1104
gagcaggcuc cgggggacag g                                                21


<210>  1105
<211>  21
<212>  RNA
<213>  Homo sapiens

<400>  1105
gagcgaagag ggaauagccc a                                                21


<210>  1106
<211>  21
<212>  RNA
<213>  Homo sapiens

<400>  1106
gcucgggaca cggggccacc a                                                21


<210>  1107
<211>  21
<212>  RNA
<213>  Homo sapiens

<400>  1107

ggcuccgcag cccgccggga g                                                21

<210>  1108
<211>  21
<212>  RNA
<213>  Homo sapiens

<400>  1108
ggcucugcaa gagcucccug g                                                21

<210>  1109
<211>  21
<212>  RNA
<213>  Homo sapiens

<400>  1109
gggcagggac cucgggggc u                                                21

<210>  1110
<211>  21
<212>  RNA
<213>  Homo sapiens

<400>  1110
ggggggagga gagagaagag a                                                21

<210>  1111
<211>  21
<212>  RNA
<213>  Homo sapiens

<400>  1111
gucuuggaca ccgguaucug a                                                21

<210>  1112
<211>  21
<212>  RNA
<213>  Homo sapiens

<400>  1112
ucccuggggcu ggcacucguu g                                                21

<210>  1113
<211>  21
<212>  RNA
<213>  Homo sapiens

<400>  1113
ucugcagcgc aacgccugag g                                                21

<210>  1114
<211>  21
<212>  RNA
<213>  Homo sapiens

<400>  1114
ugcggagcug cuucgccgcu g                                                21

<210>  1115
<211>  21
<212>  RNA
<213>  Homo sapiens

<400>  1115
uggacaggcc aaaacacaca c                                                21

<210> 1116
<211> 21
<212> RNA
<213> Homo sapiens

<400> 1116
ugggaggggu auggagaagu c 21


<210> 1117
<211> 21
<212> RNA
<213> Homo sapiens

<400> 1117
ugggcgagca gcggcggggg c 21


<210> 1118
<211> 21
<212> RNA
<213> Homo sapiens

<400> 1118
ugguagcaug uuggaaaaag u 21


<210> 1119
<211> 21
<212> RNA
<213> Homo sapiens

<400> 1119
ugugacugca uuaugaaaau u 21


<210> 1120
<211> 21
<212> RNA
<213> Homo sapiens

<400> 1120
uucuccuggc aaccucccug c 21


<210> 1121
<211> 21
<212> RNA
<213> Homo sapiens

<400> 1121
uugcccucca aagcucuagg c 21


<210> 1122
<211> 21
<212> RNA
<213> Homo sapiens

<400> 1122
uuugacuccg ggucucacau c 21


<210> 1123
<211> 20
<212> RNA
<213> Homo sapiens

<400> 1123
aaacggcugc acacuucagg 20


<210> 1124
<211> 20
<212> RNA

<213>  Homo sapiens

<400>  1124
aacggauaac cagaggccaa                                                    20


<210>  1125
<211>  20
<212>  RNA
<213>  Homo sapiens

<400>  1125
aagcaucauu ggugauaauc                                                    20


<210>  1126
<211>  20
<212>  RNA
<213>  Homo sapiens

<400>  1126
aaggacuugg acuacuacgg                                                    20


<210>  1127
<211>  20
<212>  RNA
<213>  Homo sapiens

<400>  1127
acaauguuca uugaaauugg                                                    20


<210>  1128
<211>  20
<212>  RNA
<213>  Homo sapiens

<400>  1128
acaggaguca acaagauaau                                                    20


<210>  1129
<211>  20
<212>  RNA
<213>  Homo sapiens

<400>  1129
accaguaugu cuggacgugg                                                    20


<210>  1130
<211>  20
<212>  RNA
<213>  Homo sapiens

<400>  1130
acugcggcaa ggccuucagc                                                    20


<210>  1131
<211>  20
<212>  RNA
<213>  Homo sapiens

<400>  1131
agaaggcugg auugaggcuc                                                    20


<210>  1132
<211>  20
<212>  RNA
<213>  Homo sapiens

<400>  1132

agaccgccuu ucaggacacu                                                    20

<210>  1133
<211>  20
<212>  RNA
<213>  Homo sapiens

<400>  1133
agagcgugaa ggcgaaugcc                                                    20

<210>  1134
<211>  20
<212>  RNA
<213>  Homo sapiens

<400>  1134
agcacugccc gcccaggcaa                                                    20

<210>  1135
<211>  20
<212>  RNA
<213>  Homo sapiens

<400>  1135
agcagucuga ggaggaccug                                                    20

<210>  1136
<211>  20
<212>  RNA
<213>  Homo sapiens

<400>  1136
agcggccuca ggccuguguc                                                    20

<210>  1137
<211>  20
<212>  RNA
<213>  Homo sapiens

<400>  1137
auccucuugu uuaaauugua                                                    20

<210>  1138
<211>  20
<212>  RNA
<213>  Homo sapiens

<400>  1138
augggaacgc agccuccagu                                                    20

<210>  1139
<211>  20
<212>  RNA
<213>  Homo sapiens

<400>  1139
auguccuggg uagauggcuu                                                    20

<210>  1140
<211>  20
<212>  RNA
<213>  Homo sapiens

<400>  1140
auuccacccc agaagugacu                                                    20

<210>  1141
<211>  20
<212>  RNA
<213>  Homo sapiens

<400>  1141
auugcacugu guguuaaaga                                                    20


<210>  1142
<211>  20
<212>  RNA
<213>  Homo sapiens

<400>  1142
cacaucgugc ccugcugggc                                                    20


<210>  1143
<211>  20
<212>  RNA
<213>  Homo sapiens

<400>  1143
cagaugugug ucaguugaaa                                                    20


<210>  1144
<211>  20
<212>  RNA
<213>  Homo sapiens

<400>  1144
ccuggugcug cuccagguca                                                    20


<210>  1145
<211>  20
<212>  RNA
<213>  Homo sapiens

<400>  1145
cgccacaugc ucugugauuc                                                    20


<210>  1146
<211>  20
<212>  RNA
<213>  Homo sapiens

<400>  1146
cucuucuggc uggcaccguc                                                    20


<210>  1147
<211>  20
<212>  RNA
<213>  Homo sapiens

<400>  1147
cuucgaagac accgccgcuu                                                    20


<210>  1148
<211>  20
<212>  RNA
<213>  Homo sapiens

<400>  1148
cuugaggcag ggguuagagg                                                    20


<210>  1149
<211>  20
<212>  RNA

<213>  Homo sapiens

<400>  1149
gagacggagu cuugcucaag                                                    20


<210>  1150
<211>  20
<212>  RNA
<213>  Homo sapiens

<400>  1150
gaggcagcgc agcccgugaa                                                    20


<210>  1151
<211>  20
<212>  RNA
<213>  Homo sapiens

<400>  1151
gauuuccugu ugacaugcca                                                    20


<210>  1152
<211>  20
<212>  RNA
<213>  Homo sapiens

<400>  1152
gcacagccuc cgggagcuac                                                    20


<210>  1153
<211>  20
<212>  RNA
<213>  Homo sapiens

<400>  1153
gcggggcuag ggcuaacagc                                                    20


<210>  1154
<211>  20
<212>  RNA
<213>  Homo sapiens

<400>  1154
gcuaaguagg gagccacaga                                                    20


<210>  1155
<211>  20
<212>  RNA
<213>  Homo sapiens

<400>  1155
gcuucgagag gcuucugcug                                                    20


<210>  1156
<211>  20
<212>  RNA
<213>  Homo sapiens

<400>  1156
gggucggcug uuuacucagc                                                    20


<210>  1157
<211>  20
<212>  RNA
<213>  Homo sapiens

<400>  1157

guguaaccuu gggcaauuug 20

<210> 1158
<211> 20
<212> RNA
<213> Homo sapiens

<400> 1158
uacaggcuaa cacagggaaa 20

<210> 1159
<211> 20
<212> RNA
<213> Homo sapiens

<400> 1159
ucugaggaag gaccaggaag 20

<210> 1160
<211> 20
<212> RNA
<213> Homo sapiens

<400> 1160
ucuggagucu gaaguagcua 20

<210> 1161
<211> 20
<212> RNA
<213> Homo sapiens

<400> 1161
uggacccgcc acgggagcuu 20

<210> 1162
<211> 20
<212> RNA
<213> Homo sapiens

<400> 1162
uuggccgaug ggaauggucu 20

<210> 1163
<211> 20
<212> RNA
<213> Homo sapiens

<400> 1163
uugucaacag guacuauuug 20

<210> 1164
<211> 20
<212> RNA
<213> Homo sapiens

<400> 1164
uuuggcucuc ugaccagcac 20

<210> 1165
<211> 19
<212> RNA
<213> Homo sapiens

<400> 1165
aaacgcuuuc ugauguacu 19

<210> 1166
<211> 19
<212> RNA
<213> Homo sapiens

<400> 1166
aaacucagau guccaaacu 19

<210> 1167
<211> 19
<212> RNA
<213> Homo sapiens

<400> 1167
aaaggaugug ugguuugcu 19

<210> 1168
<211> 19
<212> RNA
<213> Homo sapiens

<400> 1168
aaaggauuua ccugaggcc 19

<210> 1169
<211> 19
<212> RNA
<213> Homo sapiens

<400> 1169
acaagcaucu caaugacau 19

<210> 1170
<211> 19
<212> RNA
<213> Homo sapiens

<400> 1170
acaggucaau ggguuuuga 19

<210> 1171
<211> 19
<212> RNA
<213> Homo sapiens

<400> 1171
acuaccacag aggcuauga 19

<210> 1172
<211> 19
<212> RNA
<213> Homo sapiens

<400> 1172
acucugcuua ucaaguuuc 19

<210> 1173
<211> 19
<212> RNA
<213> Homo sapiens

<400> 1173
aggaggaagc uccuggcca 19

<210> 1174
<211> 19
<212> RNA

<213>  Homo sapiens

<400>  1174
agguucuccc uugcaugca                                                    19


<210>  1175
<211>  19
<212>  RNA
<213>  Homo sapiens

<400>  1175
augagcuggg acuggcagg                                                    19


<210>  1176
<211>  19
<212>  RNA
<213>  Homo sapiens

<400>  1176
auugaaggac uggaguuca                                                    19


<210>  1177
<211>  19
<212>  RNA
<213>  Homo sapiens

<400>  1177
auugggaagc ugggggaag                                                    19


<210>  1178
<211>  19
<212>  RNA
<213>  Homo sapiens

<400>  1178
cacagagcac auguauuag                                                    19


<210>  1179
<211>  19
<212>  RNA
<213>  Homo sapiens

<400>  1179
cggacccaga cugguagug                                                    19


<210>  1180
<211>  19
<212>  RNA
<213>  Homo sapiens

<400>  1180
cggcgccucg gagggcucg                                                    19


<210>  1181
<211>  19
<212>  RNA
<213>  Homo sapiens

<400>  1181
cugcgucuuu gugcuuuca                                                    19


<210>  1182
<211>  19
<212>  RNA
<213>  Homo sapiens

<400>  1182

gaaaaaggac aggaaaugu                                                    19

<210>  1183
<211>  19
<212>  RNA
<213>  Homo sapiens

<400>  1183
gaacuggugc ccaccgagg                                                    19

<210>  1184
<211>  19
<212>  RNA
<213>  Homo sapiens

<400>  1184
gagagcgagu gagcugggg                                                    19

<210>  1185
<211>  19
<212>  RNA
<213>  Homo sapiens

<400>  1185
gagcugaauc uuuguggag                                                    19

<210>  1186
<211>  19
<212>  RNA
<213>  Homo sapiens

<400>  1186
gccgcgcugg cauuuucuc                                                    19

<210>  1187
<211>  19
<212>  RNA
<213>  Homo sapiens

<400>  1187
gcucaggcga gggagauga                                                    19

<210>  1188
<211>  19
<212>  RNA
<213>  Homo sapiens

<400>  1188
gcuugauaac cccugcgcc                                                    19

<210>  1189
<211>  19
<212>  RNA
<213>  Homo sapiens

<400>  1189
ggacucagga aggcuucug                                                    19

<210>  1190
<211>  19
<212>  RNA
<213>  Homo sapiens

<400>  1190
gggcgaagcg cacgucgag                                                    19

<210> 1191
<211> 19
<212> RNA
<213> Homo sapiens

<400> 1191
gggcuaugac augcuagag 19


<210> 1192
<211> 19
<212> RNA
<213> Homo sapiens

<400> 1192
gguuuuguaa ucuagcuuc 19


<210> 1193
<211> 19
<212> RNA
<213> Homo sapiens

<400> 1193
gucccggaag ccccugugg 19


<210> 1194
<211> 19
<212> RNA
<213> Homo sapiens

<400> 1194
ucugcugggg caaaggagc 19


<210> 1195
<211> 19
<212> RNA
<213> Homo sapiens

<400> 1195
ucuggcaggu cugcucacc 19


<210> 1196
<211> 19
<212> RNA
<213> Homo sapiens

<400> 1196
ugagcgacag ggcaaggcc 19


<210> 1197
<211> 19
<212> RNA
<213> Homo sapiens

<400> 1197
ugaggagcug ccucgcgca 19


<210> 1198
<211> 19
<212> RNA
<213> Homo sapiens

<400> 1198
ugucgcggcu gcugagcgc 19


<210> 1199
<211> 19
<212> RNA

<213>  Homo sapiens

<400>  1199
uuguccacag cagaugagg                                                    19


<210>  1200
<211>  18
<212>  RNA
<213>  Homo sapiens

<400>  1200
aacuccgaca cuuguggg                                                     18


<210>  1201
<211>  18
<212>  RNA
<213>  Homo sapiens

<400>  1201
acagcuuugg augugagg                                                     18


<210>  1202
<211>  18
<212>  RNA
<213>  Homo sapiens

<400>  1202
agagagcaag caggaagc                                                     18


<210>  1203
<211>  18
<212>  RNA
<213>  Homo sapiens

<400>  1203
agaggaagau gaugauga                                                     18


<210>  1204
<211>  18
<212>  RNA
<213>  Homo sapiens

<400>  1204
agaugagaug ccgcugca                                                     18


<210>  1205
<211>  18
<212>  RNA
<213>  Homo sapiens

<400>  1205
aggacuugga cagagggc                                                     18


<210>  1206
<211>  18
<212>  RNA
<213>  Homo sapiens

<400>  1206
aggauguggg gaugcugc                                                     18


<210>  1207
<211>  18
<212>  RNA
<213>  Homo sapiens

<400>  1207
   aggggugaag gugggggc                                                    18

<210>  1208
<211>  18
<212>  RNA
<213>  Homo sapiens

<400>  1208
aucagcaugg uugucacg                                                   18

<210>  1209
<211>  18
<212>  RNA
<213>  Homo sapiens

<400>  1209
aucccuguac gcugcuuc                                                   18

<210>  1210
<211>  18
<212>  RNA
<213>  Homo sapiens

<400>  1210
auguagagca cauguuau                                                   18

<210>  1211
<211>  18
<212>  RNA
<213>  Homo sapiens

<400>  1211
auucugaaaa guucguga                                                   18

<210>  1212
<211>  18
<212>  RNA
<213>  Homo sapiens

<400>  1212
   ccgccccccg gccccagc                                                    18

<210>  1213
<211>  18
<212>  RNA
<213>  Homo sapiens

<400>  1213
cgaagagcug cggcacca                                                   18

<210>  1214
<211>  18
<212>  RNA
<213>  Homo sapiens

<400>  1214
cggcagccgg gacgcaga                                                   18

<210>  1215
<211>  18
<212>  RNA
<213>  Homo sapiens

<400>  1215
cggcgggugc gggggguaa                                                  18

<210>  1216
<211>  18
<212>  RNA
<213>  Homo sapiens

<400>  1216
cuucccuucc ugaagggc                                                    18


<210>  1217
<211>  18
<212>  RNA
<213>  Homo sapiens

<400>  1217
cuuugcagcg gcggcagc                                                    18


<210>  1218
<211>  18
<212>  RNA
<213>  Homo sapiens

<400>  1218
gaaaugccug uucuucag                                                    18


<210>  1219
<211>  18
<212>  RNA
<213>  Homo sapiens

<400>  1219
gaacccugag gaacagac                                                    18


<210>  1220
<211>  18
<212>  RNA
<213>  Homo sapiens

<400>  1220
gaacuguucu gcugcugg                                                    18


<210>  1221
<211>  18
<212>  RNA
<213>  Homo sapiens

<400>  1221
gcccagguag aggggucc                                                    18


<210>  1222
<211>  18
<212>  RNA
<213>  Homo sapiens

<400>  1222
gcccggugcc cgaggagg                                                    18


<210>  1223
<211>  18
<212>  RNA
<213>  Homo sapiens

<400>  1223
gcuccaccac agccaggc                                                    18


<210>  1224
<211>  18
<212>  RNA

<213>  Homo sapiens

<400>  1224
ggcggagccg cucugccu                                                    18


<210>  1225
<211>  18
<212>  RNA
<213>  Homo sapiens

<400>  1225
ggguuagggu ugcacacg                                                    18


<210>  1226
<211>  18
<212>  RNA
<213>  Homo sapiens

<400>  1226
ucaccccugu aauuucaa                                                    18


<210>  1227
<211>  18
<212>  RNA
<213>  Homo sapiens

<400>  1227
ucucaucccu gucggccg                                                    18


<210>  1228
<211>  18
<212>  RNA
<213>  Homo sapiens

<400>  1228
ugucaguuug ucaacagc                                                    18


<210>  1229
<211>  18
<212>  RNA
<213>  Homo sapiens

<400>  1229
uucuuucugc cucugaag                                                    18


<210>  1230
<211>  17
<212>  RNA
<213>  Homo sapiens

<400>  1230
aaauaguacu gugauac                                                     17


<210>  1231
<211>  17
<212>  RNA
<213>  Homo sapiens

<400>  1231
aaauuuucca gauacgg                                                     17


<210>  1232
<211>  17
<212>  RNA
<213>  Homo sapiens

<400>  1232

aagccccaca cuguccc 17

<210> 1233
<211> 17
<212> RNA
<213> Homo sapiens

<400> 1233
acugguggga gaggcaa 17

<210> 1234
<211> 17
<212> RNA
<213> Homo sapiens

<400> 1234
aggagucagg agcuggg 17

<210> 1235
<211> 17
<212> RNA
<213> Homo sapiens

<400> 1235
aggcacccug aggcaug 17

<210> 1236
<211> 17
<212> RNA
<213> Homo sapiens

<400> 1236
aggugaauga gugaaag 17

<210> 1237
<211> 17
<212> RNA
<213> Homo sapiens

<400> 1237
aucugccagg gauuucc 17

<210> 1238
<211> 17
<212> RNA
<213> Homo sapiens

<400> 1238
augcucugag uuaaaau 17

<210> 1239
<211> 17
<212> RNA
<213> Homo sapiens

<400> 1239
augugcggaa gggaaac 17

<210> 1240
<211> 17
<212> RNA
<213> Homo sapiens

<400> 1240
caguucagcc aagcaac 17

<210> 1241
<211> 17
<212> RNA
<213> Homo sapiens

<400> 1241
ccacgcggcg gggggag 17


<210> 1242
<211> 17
<212> RNA
<213> Homo sapiens

<400> 1242
cgccgcggcc cgggagc 17


<210> 1243
<211> 17
<212> RNA
<213> Homo sapiens

<400> 1243
cggcauccgg guuccuu 17


<210> 1244
<211> 17
<212> RNA
<213> Homo sapiens

<400> 1244
cuggcucaga uugaaca 17


<210> 1245
<211> 17
<212> RNA
<213> Homo sapiens

<400> 1245
gcccgccgcg gggcccc 17


<210> 1246
<211> 17
<212> RNA
<213> Homo sapiens

<400> 1246
uaccaaagca gucccac 17


<210> 1247
<211> 17
<212> RNA
<213> Homo sapiens

<400> 1247
ucagugcuuc caaccug 17


<210> 1248
<211> 17
<212> RNA
<213> Homo sapiens

<400> 1248
ucugagcaca gcgccuu 17


<210> 1249
<211> 16
<212> RNA

<213>  Homo sapiens

<400>  1249
aaaaacaagg uacuuu                                                    16


<210>  1250
<211>  16
<212>  RNA
<213>  Homo sapiens

<400>  1250
aaggaacuua gggaaa                                                    16


<210>  1251
<211>  16
<212>  RNA
<213>  Homo sapiens

<400>  1251
aaggguuucc uggcaa                                                    16


<210>  1252
<211>  16
<212>  RNA
<213>  Homo sapiens

<400>  1252
agggcucaga gcacug                                                    16


<210>  1253
<211>  16
<212>  RNA
<213>  Homo sapiens

<400>  1253
aucggcacug agacac                                                    16


<210>  1254
<211>  16
<212>  RNA
<213>  Homo sapiens

<400>  1254
auuggcccug cucagg                                                    16


<210>  1255
<211>  16
<212>  RNA
<213>  Homo sapiens

<400>  1255
caagcacuac uagauu                                                    16


<210>  1256
<211>  16
<212>  RNA
<213>  Homo sapiens

<400>  1256
ccucacgagu gucaca                                                    16


<210>  1257
<211>  16
<212>  RNA
<213>  Homo sapiens

<400>  1257

cggaaggguc ugcauc 16

<210> 1258
<211> 16
<212> RNA
<213> Homo sapiens

<400> 1258
cuggacaagg cggcug 16

<210> 1259
<211> 16
<212> RNA
<213> Homo sapiens

<400> 1259
cugugggagc cagguu 16

<210> 1260
<211> 16
<212> RNA
<213> Homo sapiens

<400> 1260
gccggucccc aaggcu 16

<210> 1261
<211> 15
<212> RNA
<213> Homo sapiens

<400> 1261
agaggauuga guacc 15

<210> 1262
<211> 15
<212> RNA
<213> Homo sapiens

<400> 1262
auugacugau guuga 15

<210> 1263
<211> 15
<212> RNA
<213> Homo sapiens

<400> 1263
ccuacagggc uauga 15

<210> 1264
<211> 15
<212> RNA
<213> Homo sapiens

<400> 1264
gcggucuccu gucca 15

<210> 1265
<211> 15
<212> RNA
<213> Homo sapiens

<400> 1265
ugccggggcc uugag 15

<210>  1266
<211>  15
<212>  RNA
<213>  Homo sapiens

<400>  1266
gcggcggcgg cggag                                                        15


<210>  1267
<211>  22
<212>  RNA
<213>  Homo sapiens

<400>  1267
aacaucacag caagucugug cu                                                22


<210>  1268
<211>  22
<212>  RNA
<213>  Homo sapiens

<400>  1268
gcccugugga cucaguucug gu                                                22


<210>  1269
<211>  21
<212>  RNA
<213>  Homo sapiens

<400>  1269
aacacaccua uucaaggauu c                                                 21


<210>  1270
<211>  21
<212>  RNA
<213>  Homo sapiens

<400>  1270
gcaguccaug ggcauauaca c                                                 21


<210>  1271
<211>  22
<212>  RNA
<213>  Homo sapiens

<400>  1271
cuuagcaggu uguauuauca uu                                                22


<210>  1272
<211>  22
<212>  RNA
<213>  Homo sapiens

<400>  1272
caacccuagg agagggugcc au                                                22


<210>  1273
<211>  22
<212>  RNA
<213>  Homo sapiens

<400>  1273
cugcccuggc ccgagggacc ga                                                22


<210>  1274
<211>  20
<212>  RNA

<213>  Homo sapiens

<400>  1274
cgggcggcgg cggcggcaga                                                    20


<210>  1275
<211>  22
<212>  RNA
<213>  Homo sapiens

<400>  1275
aauggaccuc cuuggagacc ga                                                 22


<210>  1276
<211>  22
<212>  RNA
<213>  Homo sapiens

<400>  1276
aguggcggcu gcagaaagcg ug                                                 22


<210>  1277
<211>  22
<212>  RNA
<213>  Homo sapiens

<400>  1277
aguucuugcg gcugugugaa cu                                                 22


<210>  1278
<211>  22
<212>  RNA
<213>  Homo sapiens

<400>  1278
ucuuguccug gugugcuaga gu                                                 22


<210>  1279
<211>  17
<212>  RNA
<213>  Homo sapiens

<400>  1279
uaugggcccg gagcgaa                                                       17


<210>  1280
<211>  22
<212>  RNA
<213>  Homo sapiens

<400>  1280
auaacauugu aaagcgcuuc uu                                                 22


<210>  1281
<211>  18
<212>  RNA
<213>  Homo sapiens

<400>  1281
cucagucugc agcagcuc                                                      18


<210>  1282
<211>  21
<212>  RNA
<213>  Homo sapiens

<400>  1282

acgaagcacu uccccgaguu c                                                21

<210>  1283
<211>  21
<212>  RNA
<213>  Homo sapiens

<400>  1283
aucuuggaag gcucugaacc u                                                21

<210>  1284
<211>  21
<212>  RNA
<213>  Homo sapiens

<400>  1284
caaggaaggc uuccggaugc u                                                21

<210>  1285
<211>  20
<212>  RNA
<213>  Homo sapiens

<400>  1285
acaaucagua uucaccucca                                                  20

<210>  1286
<211>  19
<212>  RNA
<213>  Homo sapiens

<400>  1286
agaaugaugu cguccgaau                                                   19

<210>  1287
<211>  19
<212>  RNA
<213>  Homo sapiens

<400>  1287
aucgcucggg uacgcucca                                                   19

<210>  1288
<211>  18
<212>  RNA
<213>  Homo sapiens

<400>  1288
cagggagaca aaauggag                                                    18

<210>  1289
<211>  17
<212>  RNA
<213>  Homo sapiens

<400>  1289
uccggugucu cugcaau                                                     17

<210>  1290
<211>  22
<212>  RNA
<213>  Homo sapiens

<400>  1290
uacgaaggcc uggucgcuuu ga                                               22

<210>  1291
<211>  21
<212>  RNA
<213>  Homo sapiens

<400>  1291
aguccuccgg gugagccgcc c                                                21


<210>  1292
<211>  21
<212>  RNA
<213>  Homo sapiens

<400>  1292
ucacccuaac aagccgcaac g                                                21


<210>  1293
<211>  18
<212>  RNA
<213>  Homo sapiens

<400>  1293
aaaaauguga uguggacc                                                    18


<210>  1294
<211>  21
<212>  RNA
<213>  Homo sapiens

<400>  1294
auacaaucua cugucuuucc u                                                21


<210>  1295
<211>  20
<212>  RNA
<213>  Homo sapiens

<400>  1295
cuauacgacc ugcugccuuu                                                  20


<210>  1296
<211>  20
<212>  RNA
<213>  Homo sapiens

<400>  1296
cuauacaguc uacugucuuu                                                  20


<210>  1297
<211>  19
<212>  RNA
<213>  Homo sapiens

<400>  1297
ucaguuauca cagugcuga                                                   19


<210>  1298
<211>  21
<212>  RNA
<213>  Homo sapiens

<400>  1298
uaccgcacug uggguacuug c                                                21


<210>  1299
<211>  19
<212>  RNA

<213>  Homo sapiens

<400>  1299
aggugagguu cuugggagc                                                    19


<210>  1300
<211>  21
<212>  RNA
<213>  Homo sapiens

<400>  1300
cggggccgua gcacugucug a                                                 21


<210>  1301
<211>  19
<212>  RNA
<213>  Homo sapiens

<400>  1301
cuuucccugu ugcacuacu                                                    19


<210>  1302
<211>  20
<212>  RNA
<213>  Homo sapiens

<400>  1302
aaccguggcu uucgauuguu                                                   20


<210>  1303
<211>  19
<212>  RNA
<213>  Homo sapiens

<400>  1303
cuuccaguac aguguugga                                                    19


<210>  1304
<211>  20
<212>  RNA
<213>  Homo sapiens

<400>  1304
aaaguucuga gacacuccga                                                   20


<210>  1305
<211>  21
<212>  RNA
<213>  Homo sapiens

<400>  1305
gcaggccaua uugugcugcc u                                                 21


<210>  1306
<211>  21
<212>  RNA
<213>  Homo sapiens

<400>  1306
ccaguauuaa cugugcugcu g                                                 21


<210>  1307
<211>  18
<212>  RNA
<213>  Homo sapiens

<400>  1307

aauauuacug ugcugcuu 18

<210> 1308
<211> 21
<212> RNA
<213> Homo sapiens

<400> 1308
ucgacagcac gacacugccu u 21

<210> 1309
<211> 19
<212> RNA
<213> Homo sapiens

<400> 1309
uugcagguuu gcauccagc 19

<210> 1310
<211> 22
<212> RNA
<213> Homo sapiens

<400> 1310
agccccugcc caccgcacac ug 22

<210> 1311
<211> 19
<212> RNA
<213> Homo sapiens

<400> 1311
aguucuucag uggcaagcu 19

<210> 1312
<211> 22
<212> RNA
<213> Homo sapiens

<400> 1312
accuggcaua caauguagau uu 22

<210> 1313
<211> 21
<212> RNA
<213> Homo sapiens

<400> 1313
cguguauuug acaagcugag u 21

<210> 1314
<211> 21
<212> RNA
<213> Homo sapiens

<400> 1314
gugccuacug agcugaaaca c 21

<210> 1315
<211> 19
<212> RNA
<213> Homo sapiens

<400> 1315
aggcggagac uugggcaau 19

<210>  1316
<211>  21
<212>  RNA
<213>  Homo sapiens

<400>  1316
ccuauucuug auuacuuguu u                                                21


<210>  1317
<211>  21
<212>  RNA
<213>  Homo sapiens

<400>  1317
ccuguucucc auuacuuggc u                                                21


<210>  1318
<211>  20
<212>  RNA
<213>  Homo sapiens

<400>  1318
acugauuucu uuugguguuc                                                  20


<210>  1319
<211>  22
<212>  RNA
<213>  Homo sapiens

<400>  1319
   gcugguuuca uauggugguu ua                                                22


<210>  1320
<211>  21
<212>  RNA
<213>  Homo sapiens

<400>  1320
cugggagagg guuguuuacu c                                                21


<210>  1321
<211>  18
<212>  RNA
<213>  Homo sapiens

<400>  1321
cagucagaug uuugcugc                                                    18


<210>  1322
<211>  21
<212>  RNA
<213>  Homo sapiens

<400>  1322
caauguuucc acagugcauc a                                                21


<210>  1323
<211>  16
<212>  RNA
<213>  Homo sapiens

<400>  1323
cucgacgaca gagccg                                                      16


<210>  1324
<211>  18
<212>  RNA

<213>  Homo sapiens

<400>  1324
aaagcaauga gacugauu                                                    18


<210>  1325
<211>  22
<212>  RNA
<213>  Homo sapiens

<400>  1325
aggggugcua ucugugauug ag                                               22


<210>  1326
<211>  19
<212>  RNA
<213>  Homo sapiens

<400>  1326
caaucagcaa guauacugc                                                   19


<210>  1327
<211>  22
<212>  RNA
<213>  Homo sapiens

<400>  1327
ucccccaggu gugauucuga uu                                               22


<210>  1328
<211>  22
<212>  RNA
<213>  Homo sapiens

<400>  1328
uaauccuugc uaccugggug ag                                               22


<210>  1329
<211>  21
<212>  RNA
<213>  Homo sapiens

<400>  1329
acugcugagc uagcacuucc c                                                21


<210>  1330
<211>  20
<212>  RNA
<213>  Homo sapiens

<400>  1330
uaccacaggg uagaaccacg                                                  20


<210>  1331
<211>  22
<212>  RNA
<213>  Homo sapiens

<400>  1331
ucgaggagcu cacagucuag ua                                               22


<210>  1332
<211>  20
<212>  RNA
<213>  Homo sapiens

<400>  1332

cucacugaac aaugaaugca 20

<210> 1333
<211> 24
<212> RNA
<213> Homo sapiens

<400> 1333
gcugguuuca caugguggcu uaga 24

<210> 1334
<211> 23
<212> RNA
<213> Homo sapiens

<400> 1334
accgauuucu ccugguguuc aga 23

<210> 1335
<211> 16
<212> RNA
<213> Homo sapiens

<400> 1335
ugaggggcag agagcg 16

<210> 1336
<211> 20
<212> RNA
<213> Homo sapiens

<400> 1336
aacaaaucac agucugccau 20

<210> 1337
<211> 78
<212> RNA
<213> Homo sapiens

<400> 1337
caguuggucc gaguguugug gguuauuguu aaguugauuu aacauugucu ccccccacaa 60

ccgcgcuuga cuagcuug 78

<210> 1338
<211> 88
<212> RNA
<213> Homo sapiens

<400> 1338
gggagagcca gcagcacauc cgugaggggc cagauggcaa ggucaagauc cauccauccc 60

cugcccccuc gggucacgug cuggcccc 88

<210> 1339
<211> 84
<212> RNA
<213> Homo sapiens

<400> 1339
ggcuguacug uguucccccc aaaauucaua auguugaagu ccucaccuuu agcauaugaa 60

uuuuuggggg gauacagugc agcc 84

<210> 1340
<211> 102
<212> RNA

<213>  Homo sapiens

<400>  1340
gcgcggagac cgaaggcugg cggcuggucg cguugcaggu gagguuguga cgcgcggugu      60

ggagccgcgg cccgggugcg ucccugucug guuccugggc gg                        102


<210>  1341
<211>  65
<212>  RNA
<213>  Homo sapiens

<400>  1341
agauagcagc ugagccaaga aggcaaagua agcccaguau cucuuccuua ugcucuacug      60

cuucu                                                                  65


<210>  1342
<211>  74
<212>  RNA
<213>  Homo sapiens

<400>  1342
uucuaaguuu aggacaugaa uauuauuuuu uucuggaaaa gaagaugagu auauguguaa      60

uaagacaagu agaa                                                        74


<210>  1343
<211>  88
<212>  RNA
<213>  Homo sapiens

<400>  1343
guugcccagg caauggcacg aucucagcuc acugcaaccu cugccuccug ggcugaagug      60

auucuccugc cucagccucc ugagcagc                                         88


<210>  1344
<211>  61
<212>  RNA
<213>  Homo sapiens

<400>  1344
ugggcugaag ugauucuccu gccucggccu cccaaaggca ugagccacug uccccagccc      60

a                                                                      61


<210>  1345
<211>  83
<212>  RNA
<213>  Homo sapiens

<400>  1345
gagagaugag acagggucuc gcuauauugc ccaggcuggu cuccaacucc ugggcugaag      60

ugauucuccu gccuuggccu cuc                                              83


<210>  1346
<211>  72
<212>  RNA
<213>  Homo sapiens

<400>  1346
cugggcugaa gugauucucc ugccucagcc ucccuaguag cugggacuac aggcacacac      60

caccaugccc ag                                                          72


<210>  1347

<211>  79
<212>  RNA
<213>  Homo sapiens

<400>  1347
aggcuggagu gcaaugacac aaucucagcu cacugcaacc uccaccucuu gggcugaagu      60

gauucuccug ccucagccu                                                   79


<210>  1348
<211>  79
<212>  RNA
<213>  Homo sapiens

<400>  1348
aggcuggagu gcaguggugu gaucucagcu uacuuuaucc uccaccuucu gggcugaagu      60

gauucuccug ccucggccu                                                   79


<210>  1349
<211>  73
<212>  RNA
<213>  Homo sapiens

<400>  1349
aggcuggaga auagcggugc aaucauagcu cacugcaguu uugaacuccu gggcugaagu      60

gauucuccug ccu                                                         73


<210>  1350
<211>  71
<212>  RNA
<213>  Homo sapiens

<400>  1350
cugggcugaa gugauucucc ugccuuagcc uuuugaggag ccagggcuuc aggaaugcac      60

uaucacaccu g                                                           71


<210>  1351
<211>  79
<212>  RNA
<213>  Homo sapiens

<400>  1351
aggcuggagu acagugguac aaucucggcu cacugcaacc ucugccuccu gggcugaagu      60

gauucuccug ccucagccu                                                   79


<210>  1352
<211>  81
<212>  RNA
<213>  Homo sapiens

<400>  1352
cccagaagau ccugccugug cucugcgguc ucacuguaga uccugagaaa uccgugcgag      60

accaggugag gcacagcugg g                                                81


<210>  1353
<211>  64
<212>  RNA
<213>  Homo sapiens

<400>  1353
gcucggugcu cuucuggugc uguccccuca ggugcugcug aggaagcgga gccggagccc      60

gagc                                                                   64

<210>  1354
<211>  79
<212>  RNA
<213>  Homo sapiens

<400>  1354
gccugccgcg ugcggagccg gagcccgagc cugaguggcg ccgggcccga cguggggcuc     60

cugggccgcg gcggcgggc                                                  79


<210>  1355
<211>  67
<212>  RNA
<213>  Homo sapiens

<400>  1355
gccccgacgg ggagccaaga agcggguucu caccagcaac cgcaucugcu ggccccuuga     60

ucugggc                                                               67


<210>  1356
<211>  76
<212>  RNA
<213>  Homo sapiens

<400>  1356
aggcuggugc aaaaguaaug gcgguuuuug ccguuaaagg uaauugcaaa aaccgccauu     60

acuuuugcac caaccu                                                     76


<210>  1357
<211>  76
<212>  RNA
<213>  Homo sapiens

<400>  1357
agguuggugc aaaaguaaug gcgguuuuug caauuaccuu uaacggcaaa aaccgccauu     60

acuuuugcac cagccu                                                     76


<210>  1358
<211>  80
<212>  RNA
<213>  Homo sapiens

<400>  1358
uuaguuuggu acaaaaguaa uggcguuuuu ugccauuacu uugaauggua aaaaccgcca     60

uuacuuuugc accaaccuaa                                                 80


<210>  1359
<211>  72
<212>  RNA
<213>  Homo sapiens

<400>  1359
guuggugcaa aaguaauggu caucuuugcc auugaaagaa auggcaaaaa ccgccauuac     60

uuuugcacca ac                                                         72


<210>  1360
<211>  79
<212>  RNA
<213>  Homo sapiens

<400>  1360
aggacggugc aaaaguaaug gcgguuuuag ccauuuuaaa aaguaauggc aaaaaccgcc     60

auuacuuuug caccaaccu 79

<210> 1361
<211> 76
<212> RNA
<213> Homo sapiens

<400> 1361
gcagugcuaa ugcccuggau ccaugaauug ccggcggggu gcacccaggg cucagagaga 60

aggguuuuga gacagc 76

<210> 1362
<211> 65
<212> RNA
<213> Homo sapiens

<400> 1362
ccuucucucu gcauugucuu gccucugcuu cucucugcag cucaggcagg ccaucuucag 60

caagg 65

<210> 1363
<211> 89
<212> RNA
<213> Homo sapiens

<400> 1363
gaaauccuac ucggauggau auaauacaac cugcuaagug uccuagcacu uagcagguug 60

uauuaucauu guccgugucu auggcucuc 89

<210> 1364
<211> 51
<212> RNA
<213> Homo sapiens

<400> 1364
uuccccugcc ccugagauuc ugauucauaa guccggagag ggagccugga a 51

<210> 1365
<211> 104
<212> RNA
<213> Homo sapiens

<400> 1365
gggcggggug ggccuggugg gcgggcgggg cucggggcgg gggcggagag ggagccuggu 60

gggcgggcgg ggcgcgucuu gcgggcuccc ucggguaccg gcgc 104

<210> 1366
<211> 53
<212> RNA
<213> Homo sapiens

<400> 1366
gagaggaaug augacaaagu ggcuugggga aggagcacgu gauuagaccu cuc 53

<210> 1367
<211> 66
<212> RNA
<213> Homo sapiens

<400> 1367
gaccagcggc accagagaga gaaacaugag caacaagcac cuucuucaac uguuuccccc 60

uccguc 66

<210> 1368
<211> 61
<212> RNA
<213> Homo sapiens

<400> 1368
gaggaggcgg caagcuggug ucuggggcag ugaggauuuc agaugcagcu ccugccuccu 60

u 61


<210> 1369
<211> 76
<212> RNA
<213> Homo sapiens

<400> 1369
gcggagcuuc cagacgcucc gccccacguc gcaugcgccc cgggaaagcg uggggcggag 60

cuuccggagg ccccgc 76


<210> 1370
<211> 76
<212> RNA
<213> Homo sapiens

<400> 1370
gcggagcuuc cagacgcucc gccccacguc gcaugcgccc cgggaacgcg uggggcggag 60

cuuccggagg ccccgc 76


<210> 1371
<211> 64
<212> RNA
<213> Homo sapiens

<400> 1371
ggaugagugu acugugggcu ucggagauca cgccacugcu gccgcccgcu gcccgccacc 60

aucu 64


<210> 1372
<211> 84
<212> RNA
<213> Homo sapiens

<400> 1372
ggggauugug gggggucgcu cuaggcaccg cagcacugug cuggggaugu ugcagcugcc 60

ugggagugac uucacacagu ccuc 84


<210> 1373
<211> 66
<212> RNA
<213> Homo sapiens

<400> 1373
uucucucccc aagcuccagc ggcuuuaacc cuuuaacuug gggccuugag acagcagggg 60

acagaa 66


<210> 1374
<211> 72
<212> RNA
<213> Homo sapiens

<400> 1374
auguagguuu cuaaugagac aaguggcaga gauaccuuua uuuggaauug guagcaauag 60

gggaagauac gu                                                          72

<210>  1375
<211>  52
<212>  RNA
<213>  Homo sapiens

<400>  1375
ggcgggggcg gcgcgcgcuc guuggaacag cccaggcggc ugcagccccg cc              52

<210>  1376
<211>  71
<212>  RNA
<213>  Homo sapiens

<400>  1376
ggccagcucg cgccgggccc ucccccgccc ucccgcggcc gggggcgggg gcggcgcgcg      60

gccggcuaac c                                                           71

<210>  1377
<211>  80
<212>  RNA
<213>  Homo sapiens

<400>  1377
ggguuguucg agacccgcgg gcgcucucca guccuuuuac cucucugggc gguucgagac      60

ccgcgggagc ucuccagucc                                                  80

<210>  1378
<211>  73
<212>  RNA
<213>  Homo sapiens

<400>  1378
gacagaauag caagcaaaag ugguccaaua uuacucagug cuuuggagau cccuucgugg      60

uugccaaaau guu                                                         73

<210>  1379
<211>  91
<212>  RNA
<213>  Homo sapiens

<400>  1379
cccggcugug uggacaugug cccagggccc gggacagcgc cacggaagag gacgcacccg      60

gcugugugca caugugccca gggcccggga c                                     91

<210>  1380
<211>  91
<212>  RNA
<213>  Homo sapiens

<400>  1380
cccggcugug ugcacaugug cccagggccc gggacagcgc cacggaagag gacgcacccg      60

gcugugugca caugugccca gggcccggga c                                     91

<210>  1381
<211>  91
<212>  RNA
<213>  Homo sapiens

<400>  1381
cccggcugug ugcacaugug cccagggccc gggacagcgc cauggaagag gacgcacccg      60

gcugugugca caugugccca gggcccggga c                                    91

<210>  1382
<211>  166
<212>  RNA
<213>  Homo sapiens

<400>  1382
agauaucucc cucuuccauu ucaucaagac ccagcugagu cacugucacu gccuaccaau      60

cucgaccgga ccucgaccgg cucgucugug uugccaaucg acucggcgug gcgucggucg     120

ugguagauag gcggucaugc auacgaauuu ucagcucuug uucugg                    166

<210>  1383
<211>  73
<212>  RNA
<213>  Homo sapiens

<400>  1383
uagauuuaca guccaaugcu ucacucagcc auuuuaccuc acccccacug auguucgccg      60

accguugacu auu                                                         73

<210>  1384
<211>  74
<212>  RNA
<213>  Homo sapiens

<400>  1384
gccggagguc auuaggaggg cugagagggc cccuguuagg ggccaugggc uggguuuuac      60

uauaugauag gcau                                                        74

<210>  1385
<211>  93
<212>  RNA
<213>  Homo sapiens

<400>  1385
ccgcagcacu gugcugggga uguugcagcu gccugggagu gacuucacac aguccucucu      60

gccuccaggg ucacccggaa cgggguugcg ggg                                  93

<210>  1386
<211>  98
<212>  RNA
<213>  Homo sapiens

<400>  1386
caggauaaca caguuggucc gaguguugug gguuauuguu aaguugauuu aacauugucu      60

ccccccacaa ccgcgcuuga cuagcuugcu guuuugca                              98

<210>  1387
<211>  126
<212>  RNA
<213>  Homo sapiens

<400>  1387
guaguggugu agagaggcau uaagaacgca gguggcaggg ccagccacca ggagggcugc      60

gugccacccg ggcagcucug cugcucacug gcagugucac cugcggaaac ucuccaucau     120

cuaucc                                                                126

<210>  1388
<211>  109

<212>  RNA
<213>  Homo sapiens

<400>  1388
ucuggagggg guaguagcug cuuuucagug uggguuuguc acccuacuuc uuguacgacu      60

caaauguggc cuuugcagcu gaugauacag cuucuuuccc caucagauc                 109


<210>  1389
<211>  102
<212>  RNA
<213>  Homo sapiens

<400>  1389
agugcagaua gugguccucu ccgugcuacc gcacuguggg uacuugcugc uccagcaggg      60

cacgcacagc guccguggag ggaaaggccu uuuccccacu uc                        102


<210>  1390
<211>  171
<212>  RNA
<213>  Homo sapiens

<400>  1390
ccgcgaggcg cacugccagu cucgcucgga ccugcucaag gccggcgggg gcgcgggcgg      60

caguggcggg agcggccccu cggccauccu ccgucugccc aguuaccgcu uccgcuaccg     120

ccgccgcucc cgcucuagcu cccgcuccag cgagccgucg ccgucgcggg a              171


<210>  1391
<211>  53
<212>  RNA
<213>  Homo sapiens

<400>  1391
caucagaucg acccuguuga ucucuacacu auuggccagu uuugucugau gca             53


<210>  1392
<211>  99
<212>  RNA
<213>  Homo sapiens

<400>  1392
agccugcagc gucccagaga guagccacua gccacauguc aguucaugcu uuuaaggcua      60

uaugugccua guggcugcug ucugggaugg ugcagcucc                             99


<210>  1393
<211>  78
<212>  RNA
<213>  Homo sapiens

<400>  1393
ugggacgcuc agacgccgcg cggggcgggg auuggucugu gguccucucu cggcuccucg      60

cggcucgcgg cggccgac                                                    78


<210>  1394
<211>  108
<212>  RNA
<213>  Homo sapiens

<400>  1394
ugggugucag ugggaagguu gcgucaugga cccagccagu gaggcuagga cuccccuggg      60

accccuggca gccccuccug augauucuuc uuccugagca cgcucaug                  108

<210>  1395
<211>  91
<212>  RNA
<213>  Homo sapiens

<400>  1395
gucagacgug ucauccccag auacaaugga caauaugcua uuauaaucgu auggcauugu      60

ccuugcuguu uggagauaau acugcugacu u                                     91


<210>  1396
<211>  100
<212>  RNA
<213>  Homo sapiens

<400>  1396
aaugagugag aacucauacc gucguucuca ucggaacuga gguccagcac auugccuccc      60

ccggggacug uaggagcgag ggacaacuuc cacugguucc                           100


<210>  1397
<211>  91
<212>  RNA
<213>  Homo sapiens

<400>  1397
gucagacgug ucauccccag auacaaugga caauaugcua uuauaaucgu auggcauugu      60

ccuugcuguu uggagauaau acugcugacu u                                     91


<210>  1398
<211>  122
<212>  RNA
<213>  Homo sapiens

<400>  1398
ccgacucuac agugcgacgg gcggagcuuc cagacgcucc gccccacguc gcaugcgccc      60

cgggaaagcg uggggcggag cuuccggagg ccccgcccug cugccgaccc uguggagcgg     120

ag                                                                    122


<210>  1399
<211>  81
<212>  RNA
<213>  Homo sapiens

<400>  1399
ugcgggcuga gcgcgggcug agcgcugcca gucagcgcuc acauuaaggc ugacagcgcc      60

cugccuggcu cggccggcga a                                                81


<210>  1400
<211>  131
<212>  RNA
<213>  Homo sapiens

<400>  1400
cugccucagc cccccaaaau gcuucuguac cccugcccca acaaggaagg acaagaggug      60

ugagccacac acacgccugg ccuccugucu uuccuuguug gagcagggau guagaagcac     120

uugccgcagc c                                                          131


<210>  1401
<211>  74
<212>  RNA
<213>  Homo sapiens

<400> 1401
gccuuuuguc uacagauugc agcgauccua cauaaauaua uggaggucuc ugucuggcuu 60

aggacagcug gcua 74


<210> 1402
<211> 70
<212> RNA
<213> Homo sapiens

<400> 1402
ggugcaaaag uaauugcggu uuuugccauu aaaaguaaug cggccaaaac ugcaguuacu 60

uuugcaccca 70


<210> 1403
<211> 116
<212> RNA
<213> Homo sapiens

<400> 1403
uuggugaaaa uguguugauu guaaugguuc cuauucugau caauaaacau gguuugagcc 60

uaguuacaau gaucuaaaau ucacggucca aaacugcagu uacuuuugca ccaacc 116


<210> 1404
<211> 116
<212> RNA
<213> Homo sapiens

<400> 1404
ggcugcccug gacuccccuc ccuccucccu cccgccuccu cgcccagggc ccggcucacc 60

uggcggcggg gcgcgggacg ccgcgggcgg gacggcgggg ggcuccgggg cgcucc 116


<210> 1405
<211> 84
<212> RNA
<213> Homo sapiens

<400> 1405
gcggcggcgg cggggcgcgg gacgcgggcg cuagcggggg agccggcccc gggcgcggcg 60

gcuccgcguc ugacugggcu gcgg 84


<210> 1406
<211> 93
<212> RNA
<213> Homo sapiens

<400> 1406
uuuucccuuc uucucacuac ugcacuugau agucuuaaga aaagaauauu ggagacuauu 60

auuuuaagug agguaacuca ggaauggaaa acc 93


<210> 1407
<211> 63
<212> RNA
<213> Homo sapiens

<400> 1407
gguccaauga aucugaggag gcuacucagu agauaguccc acccucacac gguucuuuac 60

cuu 63


<210> 1408
<211> 93
<212> RNA

<213>  Homo sapiens

<400>  1408
gcuggcauau augaugacuu agcuuuuuuc cccgacagau cgacuauguu gaucuaacuu      60

uucuaagcca guuucugucu gauaugccag cuu                                  93


<210>  1409
<211>  130
<212>  RNA
<213>  Homo sapiens

<400>  1409
cggaagcucu gcgccguggg ggaaaugcag cagcggggug uggacgcggg cgggagaugc      60

ggaccaggaa gugguucccg cuaccggcgc gacaggccgc agccgccgcc cccagcgagg     120

gauguccgcg                                                           130


<210>  1410
<211>  113
<212>  RNA
<213>  Homo sapiens

<400>  1410
cagccuguug aagcuuugaa gcuuugaugc caggcauuga cuucucacua gcugugaaag      60

uccuagcuaa agagaaguca augcaugaca ucuuguuuca auagauggcu guu           113


<210>  1411
<211>  131
<212>  RNA
<213>  Homo sapiens

<400>  1411
cugccucagc cccccaaaau gcuucuguac cccugcccca acaaggaagg acaagaggug      60

ugagccacac acacgccugg ccuccugucu uuccuuguug gagcagggau guagaagcac     120

uugccgcagc c                                                         131


<210>  1412
<211>  84
<212>  RNA
<213>  Homo sapiens

<400>  1412
ugucccgaca cauaaucucu gucucuugga ccugccacca ucacuuucug ggucaggauu      60

ggaauuggga uggaauggga cagu                                            84


<210>  1413
<211>  135
<212>  RNA
<213>  Homo sapiens

<400>  1413
cauuuuucuu ucuaagcaua uauaauuuau uuagauuggu gcagaaguaa uugcgguuuu      60

gccauggaaa guaauggcaa aaaccguaau uacuuuugua ccaaccuaau aauuucagcc     120

aacuauucau augca                                                     135


<210>  1414
<211>  144
<212>  RNA
<213>  Homo sapiens

<400>  1414

cagccuggag gcuuggguuu uuugagguuu cuuugaaauc cacuccuggc ugggccacug 60

gggucaaaau caggauuugg gccaggcagg acacagagug ggggcgggga gcggccaaag 120

gcaggcuggg cccccagagc ugca 144

<210> 1415
<211> 56
<212> RNA
<213> Homo sapiens

<400> 1415
gaacauggcc cuucucacug cacagaagaa guugggggcg gggagcggca guucuc 56

<210> 1416
<211> 69
<212> RNA
<213> Homo sapiens

<400> 1416
ggcucuuucu gcugggccug cccuucaccc ucuacauggu gagugugggg gcggggagcg 60

gguggcucc 69

<210> 1417
<211> 70
<212> RNA
<213> Homo sapiens

<400> 1417
gccgcugcuc gccgccgugg aaggugagcg agcgggcggg cgggcgggcg augggggcgg 60

ggagcggccg 70

<210> 1418
<211> 142
<212> RNA
<213> Homo sapiens

<400> 1418
gggcacuccg ggagauggca gcggauuggg ggcggggagc gggggggggc aaaggcggag 60

gauuggucga gggcggaggg acgggggguc gcgggggugc ugcugcacug caccgggagu 120

ucgaggcuac cgcagcgccc ag 142

<210> 1419
<211> 53
<212> RNA
<213> Homo sapiens

<400> 1419
uucaagcucu gcaacagggg gcaaagauua aucuaccuug caguguagga auu 53

<210> 1420
<211> 88
<212> RNA
<213> Homo sapiens

<400> 1420
ucccgggugu ccagaaacgc aggcugaacg cuggagcccg gggcguccuc ggugccggaa 60

gcggccgugc ggccggagcu ccggggag 88

<210> 1421
<211> 112
<212> RNA
<213> Homo sapiens

<400> 1421
ggucaaggcc cugcugggag ggaauguccg caugaugcug ucuggagggg ccccgcuauc 60

uccucagaca caccgauuca ugaaugucug cuucugcugc ccaauuggcc ag 112


<210> 1422
<211> 109
<212> RNA
<213> Homo sapiens

<400> 1422
ucauuuuccu uaucugcuuc cuaguccugu acgcccuuuu ccuaacacuc acaacaaaac 60

uaacuaauac uaacaucuca gacgcucagg aaauagaaac cgucugaac 109


<210> 1423
<211> 78
<212> RNA
<213> Homo sapiens

<400> 1423
gcccaagcug cgggcccugg gaguggagac gcggagcugc ggucgccgcu uucugggcuc 60

gcucaccagc ccgggugg 78


<210> 1424
<211> 117
<212> RNA
<213> Homo sapiens

<400> 1424
uccagggugc uguggccgcc ucacguaucc agagugaugc agcucccugg ggacacaggu 60

gcuuccuggg gagcccugag cacuugucag uguugcuggc cucaguccaa uugggca 117


<210> 1425
<211> 83
<212> RNA
<213> Homo sapiens

<400> 1425
uggguuuuga auuugcagua acagguguga gcauucuagc agcaguuuga ugaucaugua 60

ugauacugca aacaggaccu acg 83


<210> 1426
<211> 133
<212> RNA
<213> Homo sapiens

<400> 1426
uuaaagcauu ccuuucuuua auuuuguaau ucuuuacugu ggaauagcuc agaaugucag 60

uucuguuuua aguaacagaa uugauaacug agcaaggaaa cguaauuugg auuauaaaau 120

ucuugcuuua aua 133


<210> 1427
<211> 109
<212> RNA
<213> Homo sapiens

<400> 1427
cuccagauag cacuacgaug uauuccgcuu cugaacagga ucccaugcca guguggaaca 60

ccuucccaau cuuaguucag aaguggcuug uaugaccugu ucaggggguc 109

<210> 1428
<211> 71
<212> RNA
<213> Homo sapiens

<400> 1428
ccacuucugc agugucagug uggaacugua ucuauugcuc caguauccac uccugaccag 60

caggaauggc a 71


<210> 1429
<211> 72
<212> RNA
<213> Homo sapiens

<400> 1429
cugugugccu uugcaacacu gagcaacugg gcuugcagug cucuagugau guguagugcu 60

guaaccacag ua 72


<210> 1430
<211> 71
<212> RNA
<213> Homo sapiens

<400> 1430
cagguuugug uuuccuggag uggccgaaag agaucaguuc uaaccugcuc ugcaggaaua 60

acgguccugc c 71


<210> 1431
<211> 63
<212> RNA
<213> Homo sapiens

<400> 1431
ucggcggggc ugcgggaccg cgagugagug uggucgcucc ugguucugcc agcuccccug 60

aga 63


<210> 1432
<211> 70
<212> RNA
<213> Homo sapiens

<400> 1432
guguugcugu uuguuggagc ccuccaggag gaucuuggca cugagagaaa ucccuaaacu 60

agagucaugg 70


<210> 1433
<211> 61
<212> RNA
<213> Homo sapiens

<400> 1433
aguugauuuu auguauugca gcccauucaa aaauaaucuu guaaagcaua guguuugcuu 60

a 61


<210> 1434
<211> 76
<212> RNA
<213> Homo sapiens

<400> 1434
gguggcgugg ggcuggggca cccuccgacg gcagagccuc cuucagaaag ugcagcucaa 60

gucuuaaaga caccaa 76

<210> 1435
<211> 55
<212> RNA
<213> Homo sapiens

<400> 1435
gguuuuguug caauuguuuu ugaggacuug gucauaaauu cuuugccaag acuga 55


<210> 1436
<211> 79
<212> RNA
<213> Homo sapiens

<400> 1436
auuaggucca cuugucaauu uuguuuuugu ugcaguugcu uuugaggacu caucauaaau 60

ucuuugccaa gacugaugu 79


<210> 1437
<211> 70
<212> RNA
<213> Homo sapiens

<400> 1437
uuuagcugac ucgccacacu ccacggaagc aauaugaaau gaucugcugc agugcucuga 60

gcccuaggau 70


<210> 1438
<211> 103
<212> RNA
<213> Homo sapiens

<400> 1438
ugagugaugg agucccuggu gcuuccugga gagaacaaca aaugguaguc uugcaccacc 60

uugauuguua auauacuugg ucuguggacu ucccaugcuc aaa 103


<210> 1439
<211> 125
<212> RNA
<213> Homo sapiens

<400> 1439
auuuauaaau guuuucuuuu uuaucuacua acuaccuugu guugaaaucu agcaauuuaa 60

ugauaucuuu guugacucag gaguauguug caagugaggg ugaagaagau ccagaaguug 120

aauuu 125


<210> 1440
<211> 121
<212> RNA
<213> Homo sapiens

<400> 1440
uuacuuuuuu uuuuuuuugg gacagggucu ugcuguguug cccaggcugg agugcagugg 60

caccauuuca ccucacugca accuccacca ccaggguuca agcgauccuc augccuuaac 120

c 121


<210> 1441
<211> 72
<212> RNA
<213> Homo sapiens

<400> 1441
acauaggaaa uagauccucu uuuuuucuuu cuuuuuuuuu uuuuugguuu gggacagggu 60

cuugcugugu ug 72


<210> 1442
<211> 92
<212> RNA
<213> Homo sapiens

<400> 1442
uuucuuuuug ggacaggguc uugcuguguu gcccaggcug gucuugaacu ccugggcuca 60

agcgauucuc cugaauugcu ugagaagaaa ga 92


<210> 1443
<211> 80
<212> RNA
<213> Homo sapiens

<400> 1443
gggucaucuu cuccagggac auaaugggaa aggcuuuauc uaaugucuuu ucucauuuac 60

cucccuggaa ggugucccuu 80


<210> 1444
<211> 89
<212> RNA
<213> Homo sapiens

<400> 1444
ggaguuucua aaaguagacu guacgcuaag ggucauaucu uuuuuuguuu ugguuugugu 60

cuugguuggc gucuuaaaug uuaauccua 89


<210> 1445
<211> 96
<212> RNA
<213> Homo sapiens

<400> 1445
agcauauuuu gcgaguacuc aacaccaaca uccauguugg cagaaaauag ccuuugccau 60

cacugccauu gagggugugg gucgaagaua ugcuca 96


<210> 1446
<211> 108
<212> RNA
<213> Homo sapiens

<400> 1446
cacaaguaua uauuauguug gugaaaaagu aauugcuguu uuugccauua aaaauaauag 60

caaaacugca auuacuuuug caccaaccua uauaagacau auugugca 108


<210> 1447
<211> 63
<212> RNA
<213> Homo sapiens

<400> 1447
gggcaaggga ucugagugca gucagaccuc auuugggucc agacuucauu ccucuauguc 60

ucu 63


<210> 1448
<211> 87
<212> RNA

<213>  Homo sapiens

<400>  1448
agcugccguu ccagcgccug gugcgagaaa ucgcccaaga cuucaagacc gaucuucgcu      60

uccagagcuc ugcggugaug gcgcugc                                          87


<210>  1449
<211>  156
<212>  RNA
<213>  Homo sapiens

<400>  1449
ccugcuuccu guguggugag gggugaucag ggcgagugag ggaagcugug ggaggagccc      60

uuggcugcug gcagcaccgg cuggagggcu ccucucugcc caucccagcg aagcccugga     120

gcccucagcc ccagcaagcc ccccguggag caggug                               156


<210>  1450
<211>  94
<212>  RNA
<213>  Homo sapiens

<400>  1450
agcccccggg cccugcgccc gcccgcccgc ccgccaggcc aggccgcccg cugaccgguc      60

cuggagcugu aggucggguc gcucccgagg cucc                                  94


<210>  1451
<211>  83
<212>  RNA
<213>  Homo sapiens

<400>  1451
uuucuuccca caacacuuuc ucggccuauc cggaaugccc cgacguuacu cggacuaucc      60

cgaugcauac accacaugaa aua                                              83


<210>  1452
<211>  162
<212>  RNA
<213>  Homo sapiens

<400>  1452
ucuguuaguc uaugucaggu ugugucuauc aagacccgug ucauuucacc aacuucuccc      60

uuucccagaa gggucacuuc uuuuucacuu gcugggaaag ggagaaguuc auggaaugac     120

gaacgucuug auuucaugga augacgaagg ucuugauaga cu                        162


<210>  1453
<211>  66
<212>  RNA
<213>  Homo sapiens

<400>  1453
ucuuuuaguu auugggccag uucaauuguc uuacucauca acuggagugu acccaaaaca      60

aagauc                                                                 66


<210>  1454
<211>  93
<212>  RNA
<213>  Homo sapiens

<400>  1454
uggcugggag gggcagaacu guggacagau ucucaaugcc uuuuugaagu ucugacccac      60

caaagaccuu cugccuucac ccuccucccc acc 93

<210> 1455
<211> 127
<212> RNA
<213> Homo sapiens

<400> 1455
cacuucgguc gcgggcggug aggugcuguu gcugaaacgc ugccgcugag gguggacucg 60

auuucccagg gucccgccgc gggagucucc ggcgggcggg cgcgcgcgag ccaccgagcg 120

aggugau 127

<210> 1456
<211> 98
<212> RNA
<213> Homo sapiens

<400> 1456
ggucugggug aauggccauc ucagccugga gucagggcug ggcucagacg accagcaguc 60

uuugaccugc caugccggga gccacaucuc gugaccag 98

<210> 1457
<211> 93
<212> RNA
<213> Homo sapiens

<400> 1457
caucucagcc uggggucagg gcagggcuca gaauggucug gguggacggc cgucucagcc 60

uggagucagg gcagggcuca aggucugggu gaa 93

<210> 1458
<211> 62
<212> RNA
<213> Homo sapiens

<400> 1458
agucagggcu gggcugagaa cgcccugggu ggacggccgu cucagccugg agucagggcu 60

gg 62

<210> 1459
<211> 62
<212> RNA
<213> Homo sapiens

<400> 1459
agucagggcu gggcugagaa cgcccugggu ggacggucgu cucagccugg agucagggcu 60

gg 62

<210> 1460
<211> 156
<212> RNA
<213> Homo sapiens

<400> 1460
gggaggugag gaugaauugc ugguuuacuu auuacugaau uacuuauuac ugguagugag 60

ucucuaagaa aagaggaggu gguuguuuuc cucccucuuu cuuugagacu cacuaccaau 120

aauaagaaau acuacuaaug ggcauaagcu uccugu 156

<210> 1461
<211> 158

<212>  RNA
<213>  Homo sapiens

<400>  1461
ggccccuuug gaaaauagag gucguuaagc cugaccaccc ccggggucac cucucuggcc      60

gucuaccuuc cacacugaca agggccgugg ggacguagcu ggccagacag gugaccccag     120

agcggcgucc ccagguacuu cuugcuuucu ggugccug                             158


<210>  1462
<211>  55
<212>  RNA
<213>  Homo sapiens

<400>  1462
guuacuucag ggaguggcag aacugaagca cuggaucuac cucuccuagc agcau           55


<210>  1463
<211>  65
<212>  RNA
<213>  Homo sapiens

<400>  1463
caagugaggu aaaaggcguu gauccuauga uagauaauuu auuugccuua ggcuucuuuc      60

uugau                                                                  65


<210>  1464
<211>  69
<212>  RNA
<213>  Homo sapiens

<400>  1464
gggaaaaaaa aaaggauuug ucuuguagcc aggauauugu uuuaaagaaa auccuuuuug      60

uuuuuccag                                                              69


<210>  1465
<211>  77
<212>  RNA
<213>  Homo sapiens

<400>  1465
uggcuuugcg agggaguagg gagcuggggu cagacagcua cugugcuaac ucugccucuc      60

ccucugcaga ucccacu                                                     77


<210>  1466
<211>  56
<212>  RNA
<213>  Homo sapiens

<400>  1466
ugaguuagua aaugcuugau gagaaugcca uuuaggccuu cagugucaag aucaag          56


<210>  1467
<211>  90
<212>  RNA
<213>  Homo sapiens

<400>  1467
   cgccucccgc auccuccugc aacagccugg agcucccugu gcaggacgca gcggggggcg      60
   gggggcgguc uuaggaggcu gcggggcgca                                       90


<210>  1468
<211>  52

<212>  RNA
<213>  Homo sapiens

<400>  1468
uggaguuggg aaaaacuuuu cuuuggcccg guuuccucuu cuuaacacca aa          52


<210>  1469
<211>  106
<212>  RNA
<213>  Homo sapiens

<400>  1469
gagggaagca gcagccacag aguucuugcc ugacacaugg gguaguagau gagaacgugu    60

aucuccugga ucuuaacuca gugagcucuu guucuuuucc ccuccu                  106


<210>  1470
<211>  75
<212>  RNA
<213>  Homo sapiens

<400>  1470
uuguaucucc acaggaagua caugaccugc ucagugacua ugagcucaaa uacuguuuug    60

uggacaaaua caaag                                                     75


<210>  1471
<211>  67
<212>  RNA
<213>  Homo sapiens

<400>  1471
agauuuguuu gguuuguuaa uuuuacguga cgcuuuuuca cuguuuugug gacaaaucau    60

gguuuuu                                                              67


<210>  1472
<211>  59
<212>  RNA
<213>  Homo sapiens

<400>  1472
ggaccucggu gccuggaaug agucuuccag ccccagcuug uucuggcuuc uagguucuu     59


<210>  1473
<211>  70
<212>  RNA
<213>  Homo sapiens

<400>  1473
uccugcuggc cccgccugcu gggggcuccc cggcgccccc gggcccggcc ccggcggcgg    60

gggcaggagc                                                           70


<210>  1474
<211>  54
<212>  RNA
<213>  Homo sapiens

<400>  1474
guggcaucag agaucaagag ggaacugcuc uguaucccac uucugacacc acgu          54


<210>  1475
<211>  57
<212>  RNA
<213>  Homo sapiens

<400>  1475

gugucagcuu caggauaggu uuugaugugg gaagaguuaa gccuugaguu auuauca 57

<210> 1476
<211> 103
<212> RNA
<213> Homo sapiens

<400> 1476
cuguuuuaaa gccaagaaca cccuuucuuu guuccaaauu aacucuuaga agaaacccca 60

caaauaaagc aauucaauca aggcuguucu auuuaaauca gag 103

<210> 1477
<211> 143
<212> RNA
<213> Homo sapiens

<400> 1477
ugccggggga ggggaguugu aagcagagcg cugagccucg cagcucgcau ucggagggaa 60

gcugacaucc acaccaaguc gagacuucca gggauguggc cggggagcag ucacaugcug 120

uagcuuucau gagcacaggc auc 143

<210> 1478
<211> 64
<212> RNA
<213> Homo sapiens

<400> 1478
ggaacuggua gugucagccu ccuguaagga accagagcag gagccagugc cagcacaguu 60

ccag 64

<210> 1479
<211> 127
<212> RNA
<213> Homo sapiens

<400> 1479
cuugggaggc ugaggcagga gaauugcuug aacccaggag acggagguug cagugagcca 60

uacaucaugu cacccuuggg cgacagagcg agacuccaac ucagaaaaaa aagaaagaaa 120

gaaagaa 127

<210> 1480
<211> 164
<212> RNA
<213> Homo sapiens

<400> 1480
ucagcuacuc gggaggcuga ggcaggagag uugcuugaac cugggcgacg gagguugaag 60

ugagccaaga uugcgucacu gcacuccagc cugggcgaca gagcgagacu ccaacucaga 120

aaaaacaaaa caaaacaaac aaaaaaaccc caaaucuuuu gaaa 164

<210> 1481
<211> 70
<212> RNA
<213> Homo sapiens

<400> 1481
auuuuuaggc aagugucgug gacuucguac ugcuaggaag cuccguaguc accgacgaga 60

ccagaagugg 70

<210> 1482
<211> 91
<212> RNA
<213> Homo sapiens

<400> 1482
gguugaugag uacgcgccgc uggccgggac gcgccgggca guagcugcgc cgcaccuccu 60

cgcccagggc caccgacugc ucggcggcca c 91


<210> 1483
<211> 137
<212> RNA
<213> Homo sapiens

<400> 1483
ggauuagcac uuugucccuu cgccuaacag ccugugcuug ggaaagacaa acucagaguu 60

cccuucuugu gagcucagug ucuggauuuc cuagaauggc cugaagcaau guuuggcaga 120

auucaagcgg gaucugg 137


<210> 1484
<211> 54
<212> RNA
<213> Homo sapiens

<400> 1484
ggugaugggc ggcucugugu auccggcaga cgaugagaug uucuccgaca ucua 54


<210> 1485
<211> 57
<212> RNA
<213> Homo sapiens

<400> 1485
uauuuuguug cgagguucug agcguugucu guguuuaacc uugauuucag ucauguc 57


<210> 1486
<211> 92
<212> RNA
<213> Homo sapiens

<400> 1486
aggcgggcgg ccccgcggug cauugcuguu gcauugcacg ugugugaggc gggugcagug 60

ccucggcagu gcagcccgga gccggccccu gg 92


<210> 1487
<211> 170
<212> RNA
<213> Homo sapiens

<400> 1487
augaggaaag aaaaaaauaa uagacugaga uugcuaagac cuacuguaag caggucuaau 60

aagaauuucu uuuucuucac aauuaugaaa gaaaagaaau ugugaagaaa gaaauucuua 120

cuaguuuugc ugucaaaucu caaucuguaa aagugauagu uauaucgugu 170


<210> 1488
<211> 124
<212> RNA
<213> Homo sapiens

<400> 1488
ugaccugcug cuagacugua acuuccuguu ucugcaucac ccuucuccac uuaagccguu 60

ucucugaaaa caaaugagau agaaaugcug gacacaggaa cacucaguau gugccugugu 120

uaac                                                                 124

<210>  1489
<211>  57
<212>  RNA
<213>  Homo sapiens

<400>  1489
ccgcccaggc ggaacgauac ggcagccccg cggcgccucg guuggccucg gucggcc        57

<210>  1490
<211>  122
<212>  RNA
<213>  Homo sapiens

<400>  1490
gccggagggc cggcccacug cugucacgug gccuccaucc gugcgcuuua uuggcuggug     60

cuggcuuuac gggguugaau uuuggacgcu gccugccauu cgugggaccg aguuucaggc    120

ag                                                                   122

<210>  1491
<211>  55
<212>  RNA
<213>  Homo sapiens

<400>  1491
uuucuccagg ccaaagccgc guuuuuccug caaguuuggg guguguagag gaaaa          55

<210>  1492
<211>  61
<212>  RNA
<213>  Homo sapiens

<400>  1492
uuuagcauua aagaaucuug uuaugcuccc caggacgggc augcacuuuc ucagcagagc     60

a                                                                     61

<210>  1493
<211>  66
<212>  RNA
<213>  Homo sapiens

<400>  1493
ggcagucgga cuuccucgcc ccgcaaagcu ucuuuuccuu caaggacgag ggaccgcacu     60

aguuug                                                                66

<210>  1494
<211>  94
<212>  RNA
<213>  Homo sapiens

<400>  1494
aagaagcucu uucuguuacu gaacuagauc gagucuaugg aggucuuaca acuaaagucc     60

aagaaucucu aaagaaacag gagggacuuc uuaa                                 94

<210>  1495
<211>  80
<212>  RNA
<213>  Homo sapiens

<400>  1495
gccagugcuc aaaagaaaca ggagggaccg guaauguaaa aaucuggauu aguauucuau     60

uucuggguau guauuggcau                                                80

<210>  1496
<211>  114
<212>  RNA
<213>  Homo sapiens

<400>  1496
ugauaaauga agaagcucuu ucuguuacug aacuagaucg agucuaugga ggucuuacaa      60

cuaaagucca agaaucucua aagaaacagg agggacuucu uaaaaauauu cagg          114

<210>  1497
<211>  54
<212>  RNA
<213>  Homo sapiens

<400>  1497
gaaagccgaa guugaguugg aaaagucuuc uuaggagcag cucucagcau ucag          54

<210>  1498
<211>  66
<212>  RNA
<213>  Homo sapiens

<400>  1498
ccuggaguga gaccgagugc ugacuuguuu cugaugugau aaaaacacag cacucagcuc      60

ugggug                                                                66

<210>  1499
<211>  107
<212>  RNA
<213>  Homo sapiens

<400>  1499
gucgcggccc gggguucacg auguccgacg aggaagcgag gcagagcgga ggcuccucgc      60

aggccggcgu cgugacuguc agcgacgucc aggagcugau gcggcgc                  107

<210>  1500
<211>  118
<212>  RNA
<213>  Homo sapiens

<400>  1500
cugugcacac aaacuguucc uagguuguua gaugaaauug guuuguuauu ccuauaguuu      60

uuugguauca aaauuaaugu uuaaaauuuc uuuagagagu uuuaugguga cuuuuaga      118

<210>  1501
<211>  85
<212>  RNA
<213>  Homo sapiens

<400>  1501
uguuagguug gugcaaaagu acuugcggau uuugcuuuac uuuuaauggc aaaaaccgca      60

auuauuuuug cuucaaccua auaug                                          85

<210>  1502
<211>  106
<212>  RNA
<213>  Homo sapiens

<400>  1502
cucuuuuaug aagaagauga ggcaggcucu gcuacguccu gccugugaaa gaaucucuuc      60

aggaaaccag agcuucccuc auuuaccuuu ucuccuacaa agggaa 106

<210> 1503
<211> 112
<212> RNA
<213> Homo sapiens

<400> 1503
cgggcugcgg cgcuucgggu gcguggugug ccgcuggauc gcccaggacc ucagcagccu 60

ugcugggcuc cuggaccaac acggcgugcg ccuggugggc guagggcccg ag 112

<210> 1504
<211> 60
<212> RNA
<213> Homo sapiens

<400> 1504
uugguggacc ccaucugguc ccacucauaa ggagaacaga ggcccugggg ugugcuggga 60

<210> 1505
<211> 82
<212> RNA
<213> Homo sapiens

<400> 1505
cugcagcgcc aacguccgag cgggcggccg agcucccgga gcggccuggc cccgagcccc 60

gagcgggcgu cgcucagcag ca 82

<210> 1506
<211> 168
<212> RNA
<213> Homo sapiens

<400> 1506
gccugucuuc uucuucccac agccuggacu gccucguggu gggugggccc cgggaguuca 60

acgugacagu gacugugaga aaugauggug aggacuccua caggacacag gucaccuucu 120

ucuucccgcu ugaccugucc uaccggaagg uguccacgcu ccagguag 168

<210> 1507
<211> 148
<212> RNA
<213> Homo sapiens

<400> 1507
acugaaaaug aaccagcagg ucauccuuac auuaccuaaa agagcuucag uguuaggacu 60

gguacaagaa gaaagcaagc cuccuacauu uaggaaggac ucuuuauagg gcaagugguu 120

uugagcuaag ggaaguaugc uuuaguaa 148

<210> 1508
<211> 96
<212> RNA
<213> Homo sapiens

<400> 1508
uauuccaagc ggcuuuugga aaguggcuuu gacccaaaua uucgugacag caggggcaga 60

acaggccuuc accuugcagc agcucgaggg aaugua 96

<210> 1509
<211> 58
<212> RNA

<213>  Homo sapiens

<400>  1509
aguaaguggc acagccaggg auugaaccca ggucaucugg cuguagcaua uuggcucc        58


<210>  1510
<211>  76
<212>  RNA
<213>  Homo sapiens

<400>  1510
ugcuggcagg caccugagca gaacaucauu gcugucucuc uucgcagaag cugagcugac      60

uaugccuccc cgcauc                                                      76


<210>  1511
<211>  53
<212>  RNA
<213>  Homo sapiens

<400>  1511
guuagcgguu aggcguacgg ccagggcuau ugguugaaug aguaggcuga ugg              53


<210>  1512
<211>  93
<212>  RNA
<213>  Homo sapiens

<400>  1512
ccagcugugg ucucugagag acugaagauu cgaggcuccc uggccagggc agcccuucag      60

gagcuccuua guaaaggacu uaucaaacug guu                                   93


<210>  1513
<211>  124
<212>  RNA
<213>  Homo sapiens

<400>  1513
cagagagacu guuuucuaag uaccauagug uucccaaaca aaacugaggg uugggcuucu      60

auuucucaug ggccaauaac gagauacaca ugaacuaagg aggaagcgag uguguauuuc     120

ugca                                                                  124


<210>  1514
<211>  115
<212>  RNA
<213>  Homo sapiens

<400>  1514
ccaggcgcug cugcgggccg ugcgcugcca cucguugacg ccgaacuucc ugcagaugca      60

gcugcagaag ugcgagaucc ugcaguccga cucccgcugc aaggacuacc ugguc          115


<210>  1515
<211>  86
<212>  RNA
<213>  Homo sapiens

<400>  1515
ggacugauaa auugcaguuu auggauucca guauucauau uuggaaaaag uaguacgugg      60

agccacauac auaguuugaa guucac                                           86


<210>  1516
<211>  117
<212>  RNA

<213>  Homo sapiens

<400>  1516
cggcucagcc gcccgaggau ugugagugga ccguugagga gagcgaccga ccauccggcu      60

gguguccgga cucguacucu augguugucc gcgcucugcg cuuccucucu agccgcc      117


<210>  1517
<211>  123
<212>  RNA
<213>  Homo sapiens

<400>  1517
agugaagaaa uugagguuua aagaggugca guagcuugcc ugaccugacc uucauucucu      60

gcugacucac agcucauauu aguaguuuug uuucucuuuc uccuuuaaua auugcgucau     120

uac                                                                   123


<210>  1518
<211>  54
<212>  RNA
<213>  Homo sapiens

<400>  1518
guugucugaa gaaugguuuu augcccacau cauaccauuc cuuggaugaa accc            54


<210>  1519
<211>  65
<212>  RNA
<213>  Homo sapiens

<400>  1519
ucuguccagc agggggcagu aagugaucuu gguaguaucc cuggcugcua agcccuuggc      60

agggg                                                                  65


<210>  1520
<211>  69
<212>  RNA
<213>  Homo sapiens

<400>  1520
acaugcauuu ucucugugag gcauauauua caacuuucuu gugcuuaggg acacgagaca      60

gcacugugg                                                              69


<210>  1521
<211>  99
<212>  RNA
<213>  Homo sapiens

<400>  1521
ugagaggauc acuugagcuu gggugaggug aggcugcagu gaguccugau caugcugcug      60

cacucaaucu uggacaacag agcaagaccc ugucucaaa                             99


<210>  1522
<211>  67
<212>  RNA
<213>  Homo sapiens

<400>  1522
aguggaagca aagaaagaag aauccgagga gucugaugau gacaugggcu uuggucuuuu      60

ugacuaa                                                                67


<210>  1523

<211>  78
<212>  RNA
<213>  Homo sapiens

<400>  1523
aaaaggaaaa acaaagaaga aucugaggag ugugaugaug gcaugggcuu uggucuuuuu      60

gacuagaagu ucuuuuau                                                    78


<210>  1524
<211>  67
<212>  RNA
<213>  Homo sapiens

<400>  1524
aguggaagca aagaaagaag aauccgagga aucugaugag gacaugggcu uuggucuuuu      60

ugacuaa                                                                67


<210>  1525
<211>  54
<212>  RNA
<213>  Homo sapiens

<400>  1525
aguaugugca ggagcugccu cuggaagcag auggggcucu gcgcuuugug cucc            54


<210>  1526
<211>  84
<212>  RNA
<213>  Homo sapiens

<400>  1526
cacaaucuag gucuuugccu uuucuuguau uuuauaugca uuuauguuau gaugcacagc      60

agaagcaaaa uauccauuug uggg                                             84


<210>  1527
<211>  71
<212>  RNA
<213>  Homo sapiens

<400>  1527
aguacuauau ugaauaagaa uggggagagu gaacauccuu guauuguucc aguucuuaca      60

ggagaaacuu u                                                           71


<210>  1528
<211>  71
<212>  RNA
<213>  Homo sapiens

<400>  1528
ugugcacagg cccuggaggu cucccuggaa ggcagaggag gccaggcugg gaaggggcuu      60

cguggcacgc a                                                           71


<210>  1529
<211>  95
<212>  RNA
<213>  Homo sapiens

<400>  1529
aggcaggauu gcuugagccu gggaggucaa ggcugcagug ucguggucac agcuugcugc      60

agacucgacc ucccaggcuu aagcaauccu ccugc                                 95


<210>  1530

<211>  64
<212>  RNA
<213>  Homo sapiens

<400>  1530
auaguacaga gauguggcug ugucuaguac aacuuuuaga cacagaaauc ugaaugacau      60

auau                                                                    64


<210>  1531
<211>  80
<212>  RNA
<213>  Homo sapiens

<400>  1531
gauucagcaa aagucagcug gauauuauaa uucauagcuu gaagaaaugu gacaucuccc      60

ugcaauucuu guaagauccu                                                  80


<210>  1532
<211>  91
<212>  RNA
<213>  Homo sapiens

<400>  1532
ccaggggaca cggaugccag gaagugagag ggggugagca gaauggagcu ccagaggcug      60

gacaaacccg uguuugaucu gaucccuggg c                                     91


<210>  1533
<211>  78
<212>  RNA
<213>  Homo sapiens

<400>  1533
ggggcggaca ucgcccugcu ggagcuggag gagcccguga acaucuccag ccacauccac      60

acggucacgc ugcccccu                                                    78


<210>  1534
<211>  80
<212>  RNA
<213>  Homo sapiens

<400>  1534
uggaaaccaa ccuugugagg cugagcuggg agagagaaaa auccugccag uccucccugc      60

cuacuucucu ggauuccagu                                                  80


<210>  1535
<211>  161
<212>  RNA
<213>  Homo sapiens

<400>  1535
gauauucauu ugauuauacg gggagggguc agggaagaac gaaccuugac guugcagugc      60

aguuucacag aucguuguua gaucuuuauu uuuagccaug cacuguugug aggaaaaauu     120

accugucuug acugccaugu guucaucauc uuaaguauug u                         161


<210>  1536
<211>  134
<212>  RNA
<213>  Homo sapiens

<400>  1536
gcggggguuc uggggucucg ggcgccgccc cgguuccgcc ugcggcucug aacguggggc      60

ucggcgggag cucgggcggg ucugccccgc ggagcggguc gccggggugg cgcgucgagg 120

cuggugcccc gcgg 134

<210> 1537
<211> 145
<212> RNA
<213> Homo sapiens

<400> 1537
aggaaacgaa acuguggggg acucaguuuc caugauaggg aaaccaggca agaaauauug 60

ucuccucaag uugcgacgag acaguaguuc uugccugguu ucucuaucau ggagucuugg 120

uuggacucug cuaugcgucu cuuga 145

<210> 1538
<211> 72
<212> RNA
<213> Homo sapiens

<400> 1538
gccugggcuu agggcaggca gcgccaggga gcgcgcggcc gcuuccuggu ggguccagug 60

uccccggggu gg 72

<210> 1539
<211> 78
<212> RNA
<213> Homo sapiens

<400> 1539
gugggguugu aggcccguuc uguguccuga ggggcuguga uccuaucagg acaggaaucc 60

agcucggagc uccuauua 78

<210> 1540
<211> 57
<212> RNA
<213> Homo sapiens

<400> 1540
gucuccugaa acuuugcugu aagugcuuau uaguuacagg cgauuuuuug uugauuc 57

<210> 1541
<211> 54
<212> RNA
<213> Homo sapiens

<400> 1541
ggcugcaggc cuugugccca aagcagcacu gcugcugcgg gcacgccgag cccc 54

<210> 1542
<211> 119
<212> RNA
<213> Homo sapiens

<400> 1542
gcugggaggg uacaaguugg ucauuccuaa aucugugugu gagaaauggc aggucuaguu 60

ugggcauugu gauugcauug cagauuacua ggagaaggga auggugggua caccgguag 119

<210> 1543
<211> 87
<212> RNA
<213> Homo sapiens

<400> 1543

uagagaccag gauacagugg gaguaagaau gaccacuggg aguuugugac aaugcuugcu 60

gacacuuugu cauccgaaua cucuacc 87

<210> 1544
<211> 121
<212> RNA
<213> Homo sapiens

<400> 1544
cuguuuccuu guuugggucu ugaguguagg uuucuguguc uucuagucca gugccugugg 60

gcucucgaac ugauaauguc ugucuccuaa aauccuuauu auuugcaaaa aggaggcagu 120

a 121

<210> 1545
<211> 102
<212> RNA
<213> Homo sapiens

<400> 1545
aagaagacag acccccagac agggaaagug cgugugagcg gcgcacggug caaggauguc 60

cucacaggua ugccagguau cugggaggga gguauuucuu ag 102

<210> 1546
<211> 111
<212> RNA
<213> Homo sapiens

<400> 1546
cccguaguaa ccccgagucu gcggaagugg ugacccgugg gacgcggcug agacagguaa 60

ccuguucgcu cccuguuggg ucccugccuu ccucuccucc ggcgucggga u 111

<210> 1547
<211> 122
<212> RNA
<213> Homo sapiens

<400> 1547
gcccucucau guggccaccg ggcacucugu gggccuggag gguaacuggg ugccagugag 60

uggcauaaau guuaucuggg ugugaggggg aggcagagaa aaggaaggca aguggacggc 120

ca 122

<210> 1548
<211> 73
<212> RNA
<213> Homo sapiens

<400> 1548
agcagcccug cggggccuac cgccugccgc acgcacuccc gccggcgcgc gcgcacucgc 60

acucgcucgc ucg 73

<210> 1549
<211> 76
<212> RNA
<213> Homo sapiens

<400> 1549
cggccggcga gcggcggaag ugccgcggag uuggagcggg gccggcgccg cagccgcuuc 60

ugcuggcuga gcugcu 76

<210>  1550
<211>  82
<212>  RNA
<213>  Homo sapiens

<400>  1550
ggaugucuug gccgggcacg guggcucacu uugggaggcc gaggugggca gaucacgagg      60

uccggagaug aagaccaucc ug                                               82


<210>  1551
<211>  117
<212>  RNA
<213>  Homo sapiens

<400>  1551
uauuucaugc cgggcacggu ggcucacuuu gggaggccaa ggcgggagga ucacgaguuc      60

aagagaucaa gaccauccug gccaacaugg ugaaaccccg ucucuacuaa aaauaca        117


<210>  1552
<211>  62
<212>  RNA
<213>  Homo sapiens

<400>  1552
uucugccggg cacgguggcu cacuuuggga ggccgaggcg ggcggaucau gaggucagga      60

ga                                                                     62


<210>  1553
<211>  125
<212>  RNA
<213>  Homo sapiens

<400>  1553
auggguacuu uaaaaaucau gggcuguggc cgggcacggu ggcucacuuu gggaggcuga      60

ggcaggugga ucaccugagg ucaggaguuu gagaccagcc uggccaacau agugaaaccc     120

caucu                                                                 125


<210>  1554
<211>  67
<212>  RNA
<213>  Homo sapiens

<400>  1554
ggccgggcac gguggcucac uuugggcgga ucacaugagg ccaggaguuc acaacuagcc      60

uggcugg                                                                67


<210>  1555
<211>  131
<212>  RNA
<213>  Homo sapiens

<400>  1555
uuuuuauuaa aauguaacca gcuacagggc cgggcacggu ggcucacuuu uguaauccca      60

gcacuuuggg aggcugaggc gggcgagacc agccuggcca acguggcaaa accccgucuc     120

uaauaaaaau a                                                          131


<210>  1556
<211>  113
<212>  RNA
<213>  Homo sapiens

<400> 1556
guuucuguaa agugacaucu uucagauacu ucguggcuuu ucguauaugc auacccuugg 60

uggccugagc aguuccacga guguucuuaa agugaacacg aagauuggaa ccu 113


<210> 1557
<211> 88
<212> RNA
<213> Homo sapiens

<400> 1557
gggcggcugc gcagagggcu ggacucagcg gcggagcugg cugcuggccu caguucugcc 60

ucuguccagg uccuugugac ccgcccgc 88


<210> 1558
<211> 91
<212> RNA
<213> Homo sapiens

<400> 1558
auaguauagc ucauauucaa cugauauacc auuacuauca cauuauguga uaguaucaug 60

ugauaucagu ugaauaugag cuauacuaug a 91


<210> 1559
<211> 115
<212> RNA
<213> Homo sapiens

<400> 1559
ccucauagua uagcucauau ucaacugaua ucacaugaua cuaucacaua augugauagu 60

aaugguauau caguugaaua ugagcuauac uauuuucucu uuaaaguuug aggau 115


<210> 1560
<211> 52
<212> RNA
<213> Homo sapiens

<400> 1560
gcuuccuuuc uguggggcug agucucagcu cagcugccac ugcugggagc cc 52


<210> 1561
<211> 91
<212> RNA
<213> Homo sapiens

<400> 1561
augauucugu auugcauuac uaguugucca cucuaaggga ugagcgugaa auugagugac 60

ucuggacugg agaagugaua cuguaucaua a 91


<210> 1562
<211> 85
<212> RNA
<213> Homo sapiens

<400> 1562
ugcauauaua augcuacuuu cacugcuaua guaaaaugau ccagauuuag ucaccauagc 60

uaagaaugug aaaaaggcuu gcaag 85


<210> 1563
<211> 83
<212> RNA
<213> Homo sapiens

<400> 1563
gccugcggaa gguaggauua gaucauuagc ucagugaccu ccuaggguuu cgaugugcua 60

uguucucauc cuacaguugg uuu 83

<210> 1564
<211> 91
<212> RNA
<213> Homo sapiens

<400> 1564
agugcugugg ucacagugcc agcuuacuuu aaugacucuc aguuucaggu uaccaaagau 60

gcuggaacua uugcuggucu caauguacuu a 91

<210> 1565
<211> 174
<212> RNA
<213> Homo sapiens

<400> 1565
ggcgccugcc cuggucacca gccuggcuga gguaugcugu ugcgcugucc uuccucuggg 60

gagcaggcuc cgggggacag ggaaaagcac acaaggaacu uguccucuag ggccugcagu 120

cucaugggag agugacaugc accaggacca ccgcugugaa gggcuaagug cugu 174

<210> 1566
<211> 151
<212> RNA
<213> Homo sapiens

<400> 1566
auggguaucu uuaagcaaag ugaagaauuu cagcuuauca gugauguugu aaaaauaaau 60

gucugaacau augaaugcag uauugauuuc agcauuuaac ugagauaagc gcauugaaau 120

cuguuuaaca aaaauuaaaa uguaugcauc g 151

<210> 1567
<211> 87
<212> RNA
<213> Homo sapiens

<400> 1567
aguaguagaa uacuuucccu augcuaucac gaaaauucuu cuucaaaagc aaguagguug 60

cauguggaaa ugucugcuuc ucauucc 87

<210> 1568
<211> 109
<212> RNA
<213> Homo sapiens

<400> 1568
ccggcggggc gaagcccgcg gcugcuggac ccacccggcc gggaauagug cuccugguug 60

uuuccggcuc gcgugggugu gucggcggcg gggccugaca cccccggcc 109

<210> 1569
<211> 76
<212> RNA
<213> Homo sapiens

<400> 1569
gguuggugca aaaguauuug cggguuuugu cguagaaagu aauggcaaaa acugcaguua 60

cuugugcacc aaccaa 76

<210> 1570
<211> 67
<212> RNA
<213> Homo sapiens

<400> 1570
ucgaaguaaa gcucucgguc aaguucaaca gcaggaaguu cagcuugaag aggaugccgu 60

cccgaaa 67


<210> 1571
<211> 98
<212> RNA
<213> Homo sapiens

<400> 1571
gcugcucagg agcuggccac uuugcuguug ucccugccag caccugccag uguccagcag 60

caguccaaga gccuucuggc cagccugcac accagccg 98


<210> 1572
<211> 123
<212> RNA
<213> Homo sapiens

<400> 1572
uuggaagccu accauuuaug uccucuugag guaccugaau uaccaaaagc uuuauguauu 60

cugaaguuau ugaaaauaag agcuuuuggg aauucaggua guucaggagu gacuuuucua 120

aaa 123


<210> 1573
<211> 94
<212> RNA
<213> Homo sapiens

<400> 1573
ucuaagacca auuaagucac aaucccugga ggaaguaaaa ggcauauuuu uaaaguuccc 60

caggugauuc caaugugcag acaagucuag ggac 94


<210> 1574
<211> 61
<212> RNA
<213> Homo sapiens

<400> 1574
cuguaaucac acucucuucu uacuguauuu gggauaugag uuuggggaaa gaguugcagu 60

a 61


<210> 1575
<211> 69
<212> RNA
<213> Homo sapiens

<400> 1575
uaguuuccuu uuguuccuug aguccacgca gaauuccauu cucuggucag cagacaggcu 60

uaagcuaaa 69


<210> 1576
<211> 57
<212> RNA
<213> Homo sapiens

<400> 1576

gcacagugua auuaauucuu ucauuguuga ucauuguaag uuaggaugca uguguau 57

<210> 1577
<211> 126
<212> RNA
<213> Homo sapiens

<400> 1577
ggaaaagaug gccacauguc ugcuuuauaa ccagaagaga caguaacagc ccgggacagc 60

cuuucccaaa auaaaggccc cugggcuguu acuguucccu cuggcugugg ccagccacuu 120

cucucu 126

<210> 1578
<211> 109
<212> RNA
<213> Homo sapiens

<400> 1578
ggugucuuga guaagccaga gaguugggag agaagagaag agugcuuggu aucuugacaa 60

acccuccuac cuuuucuccu aacuacauag aucuugaaaa agccacuac 109

<210> 1579
<211> 181
<212> RNA
<213> Homo sapiens

<400> 1579
guguccuggg uuuuuggugg agaaagaguc cccuagauaa guuauuaggu gggugcaaag 60

guaauugcag uuuuucccau uauuuuaauu gcgaaaacag caauuaccuu ugcaccaacc 120

ugauggaguc ccccuugcca ugcuucucuc ugugaaaaac cccaagccga agucagcaua 180

a 181

<210> 1580
<211> 139
<212> RNA
<213> Homo sapiens

<400> 1580
uaagcauaca acauaagugc ucauuuaagu agucugaugc cuacuacuga ugacauacaa 60

uguaagugcu cauuuaggcg ucagacuacc uaaaugagca cuuacguuuc augucaauaa 120

aaaagggaaa aggauuuau 139

<210> 1581
<211> 100
<212> RNA
<213> Homo sapiens

<400> 1581
uuucaucagg agcaaaugau aaacugaaau ggugaggaag aauacgucua acuguaaaaa 60

uuuuucugaa guugucuguu uuguuugguu uuaugggaca 100

<210> 1582
<211> 84
<212> RNA
<213> Homo sapiens

<400> 1582
   uugguguuua agaucaugcc caguugcuuc cugucaccuc uugacaggug gugacugggc 60
   ugcugugggg cuggagcagc gagg 84

<210> 1583
<211> 82
<212> RNA
<213> Homo sapiens

<400> 1583
ccaggggcuu auccuggaca ggcaccucca ggcgccuacc cuggagcacc uggagcuuau 60

cccggagcac cugcaccugg ag 82


<210> 1584
<211> 72
<212> RNA
<213> Homo sapiens

<400> 1584
cuagggugga guguggguug aguccagaac ccgacauuga gagauggacu cucccuacua 60

ucuucccuag gg 72


<210> 1585
<211> 62
<212> RNA
<213> Homo sapiens

<400> 1585
ugguccugcg ucggagaggu gguaugcaga ucuucgugaa gacccugacc ggcaagacca 60

uc 62


<210> 1586
<211> 59
<212> RNA
<213> Homo sapiens

<400> 1586
uguucggggu ggccaguuuu uuauguaugc aaauugacga uugagcacag ucaggcauu 59


<210> 1587
<211> 103
<212> RNA
<213> Homo sapiens

<400> 1587
cauuugcuaa ugauuaauac acuguuuggg cuggccaguu uuucaugcau gcagcuugac 60

gauugagcac agucaggccu uuguauuaaa aaugaaaaau gaa 103


<210> 1588
<211> 69
<212> RNA
<213> Homo sapiens

<400> 1588
uuuugccgug ugucuugcau aauucauggc caguuuuaug aagcuuugga aggcacuaug 60

gacagaagc 69


<210> 1589
<211> 70
<212> RNA
<213> Homo sapiens

<400> 1589
ggcccucggc ugagcucuuc cccaucaucg uuagccagga cugcgggcac gaggagacgg 60

cccaggccau 70

<210> 1590
<211> 129
<212> RNA
<213> Homo sapiens

<400> 1590
ugguuuacac uuuaugccgg augugcuuuu cuccaauauc agugcucgag acacagugaa 60

gcaaauuaaa aaaaaaaaaa aaaaaaaucc cugaaugaug auuagagaca ucaccgcuaa 120

aaaacuaca 129

<210> 1591
<211> 61
<212> RNA
<213> Homo sapiens

<400> 1591
cuuugccuuc cugaaaaguu uuucuuuuga uucuacauau uuucagggga gggauaaaga 60

u 61

<210> 1592
<211> 63
<212> RNA
<213> Homo sapiens

<400> 1592
gugucuuuga uuugcauuuu auuaacaucg auuggcguau uaaaaguccc ucugagcuua 60

ccu 63

<210> 1593
<211> 103
<212> RNA
<213> Homo sapiens

<400> 1593
acaucgauug gcauauuaaa agucccucug agcuuaccuu cucuaaaaaa auguaagaaa 60

aagagccugu gggaaggacu gugaggcaga ggggcuggug uga 103

<210> 1594
<211> 103
<212> RNA
<213> Homo sapiens

<400> 1594
acaucgauug gcauauuaaa agucccucug agcuuaccuu cucuaaaaaa auguaagaaa 60

aaaggccugu gggaaggacu augaggcaga ggggcuggug uga 103

<210> 1595
<211> 101
<212> RNA
<213> Homo sapiens

<400> 1595
ugaggcagga gaauugcuug aauuugggag gcggagguug uagugagcua agaucgugcc 60

acugcacucc agccuggaca acagagugag acugugucgc a 101

<210> 1596
<211> 57
<212> RNA
<213> Homo sapiens

<400> 1596
auugcgccac uguacuccag ccuggcaaca gagugagacu gugucgcaaa aaaauaa 57

<210> 1597
<211> 61
<212> RNA
<213> Homo sapiens

<400> 1597
cugagucaca auggauugcu gguuguggac aaucugcagg uagugccuuu ggagaccagu 60

g 61

<210> 1598
<211> 129
<212> RNA
<213> Homo sapiens

<400> 1598
gacaggcugc ugauaugcug ccuuuggucu cugagccacc agguauagcg cugcauaaug 60

gaagaacaga ugaacauaag agugucuugg ggaagaugag cacaagacau caugggguug 120

uauuguuac 129

<210> 1599
<211> 76
<212> RNA
<213> Homo sapiens

<400> 1599
aaaaugcagg uugcugggcu uugccccaug gacugaaucc agaguucacg ggggagccca 60

ggaaccugua uuuuuc 76

<210> 1600
<211> 54
<212> RNA
<213> Homo sapiens

<400> 1600
aguggagcag uggaagggua cugugcucga gcagguuucc gugaagccca cucu 54

<210> 1601
<211> 84
<212> RNA
<213> Homo sapiens

<400> 1601
augcaaaagu aaucgcgguu ugugccagau gacgauuuga auuaauaaau ucauuuggua 60

uaaaccgcga uuauuuuugc auca 84

<210> 1602
<211> 89
<212> RNA
<213> Homo sapiens

<400> 1602
uucccggacu auaaagagag ccgccggcuu cugggcucca ccacgcuuuu caucuguccc 60

gcugcguguu uuccucuuga ucgggaacu 89

<210> 1603
<211> 78
<212> RNA
<213> Homo sapiens

<400> 1603
ucaaguacgc aaucucugua aacccacuag aaccagucca uggucggugg gcuucuuaac 60

aggagaaagu uacugaaa 78


<210> 1604
<211> 56
<212> RNA
<213> Homo sapiens

<400> 1604
ggagcaggaa guggcggugc gagggcugcu gcacagcgag cggagccgcg guccgg 56


<210> 1605
<211> 87
<212> RNA
<213> Homo sapiens

<400> 1605
cccaugaggu aaauuaauac ucagccaagc agagugaggc ccugaugcuc uugugguucu 60

gccauuaauu ugcuguaacc cagggac 87


<210> 1606
<211> 110
<212> RNA
<213> Homo sapiens

<400> 1606
ccggauguga cguugaccua ccuuagucac auuguuaggg aaggaagugu gccgcgccua 60

ccuaucugcc ccgccuugag ucucagccag ucggcgucuc cauccuggcg 110


<210> 1607
<211> 77
<212> RNA
<213> Homo sapiens

<400> 1607
uaucugaaac acuuccuaug caggcugggg ugaagguguc cuuccugcuc cugguucag 60

ugaggaauuu agauguc 77


<210> 1608
<211> 79
<212> RNA
<213> Homo sapiens

<400> 1608
cauagauaau cugggugcca caguggaucu guauauucuu aaucaucuaa ugaacccacc 60

caauggaaaa cgcugugaa 79


<210> 1609
<211> 60
<212> RNA
<213> Homo sapiens

<400> 1609
ccuaaggguu guagagggac aaaaauccau cuuuuguaac uucuucaggc cgaauaggug 60


<210> 1610
<211> 93
<212> RNA
<213> Homo sapiens

<400> 1610
uuuaacuauu cuuuucauag agcaguuagg aaacacucug uuuguaaagu cugcaagugg 60

auauuuugac cucuuugagg ccuucguugg aaa 93

<210> 1611
<211> 71
<212> RNA
<213> Homo sapiens

<400> 1611
gccggccgag gucucgccgc cggcgcccug gggaccucgu ggguguggcc ggcggcgacc 60

ucggcaggcg a 71

<210> 1612
<211> 54
<212> RNA
<213> Homo sapiens

<400> 1612
auucuugugc uaagcagaac uguaaaauag uuaaaauguc uuaucaagua auuc 54

<210> 1613
<211> 66
<212> RNA
<213> Homo sapiens

<400> 1613
auugauugca gccguaucca gaagaaaggg aacaaauguu uugcugauuc uacugccaag 60

aaguag 66

<210> 1614
<211> 94
<212> RNA
<213> Homo sapiens

<400> 1614
cacauccuug augcaacuuu uguuggaagc ugaagugcgg cagcucaccu ugggagcauu 60

acagcaguuc aacuuggacg ucagagaaug ugaa 94

<210> 1615
<211> 56
<212> RNA
<213> Homo sapiens

<400> 1615
ggcuucaagg ucaagaaugu gucugucaag gauguucguc guggcaacgu ugcugg 56

<210> 1616
<211> 56
<212> RNA
<213> Homo sapiens

<400> 1616
ggcuucaaug ucaagaaugu gucugucaag gauguucguc guggcaacgu cgcugg 56

<210> 1617
<211> 56
<212> RNA
<213> Homo sapiens

<400> 1617
ggcuucaaug ucaagaaugu gucugucaag gauguucguc guggcaacgu ugcugg 56

<210> 1618
<211> 56

<212>  RNA
<213>  Homo sapiens

<400>  1618
ggcuuccaug ucaagaaugu gucugucaag gauguucguc guggcaacgu ugcugg          56


<210>  1619
<211>  54
<212>  RNA
<213>  Homo sapiens

<400>  1619
gguuagguuu cgagcuuccu ggccguaagc gauaaggcuu uaacugagaa ccac          54


<210>  1620
<211>  131
<212>  RNA
<213>  Homo sapiens

<400>  1620
gugaggaagu agaagcagug aauguggaug acauuuugag gaauucugcc augaagaaga     60

auacacggaa ggggaggagg ugaaggggau uuuuuuaaaa gcaauuuaaa aguuuacugg    120

aaugucucau a                                                         131


<210>  1621
<211>  59
<212>  RNA
<213>  Homo sapiens

<400>  1621
cauaugagag gcaggcugag accaauuuuc uaccaggcca gcuaauucau cacaaugac      59


<210>  1622
<211>  59
<212>  RNA
<213>  Homo sapiens

<400>  1622
cccaucuuuc auggaaagga uuugguucca ggaucuccca guucuguguc ucuugggaa      59


<210>  1623
<211>  52
<212>  RNA
<213>  Homo sapiens

<400>  1623
agaaucucug ugugugaacu gcaggcaucu guaguuuuua caaagcuucu aa            52


<210>  1624
<211>  61
<212>  RNA
<213>  Homo sapiens

<400>  1624
gcagguccug uguucacugg ucacuuccac agccugaagc cccucgaaag gacaucugca     60

c                                                                     61


<210>  1625
<211>  107
<212>  RNA
<213>  Homo sapiens

<400>  1625
ugacuccgug uggccacgug gcuagcacag cugcagggac augcggacug aguauaucag     60

ugugccucug cuggccagug ugacguugau guggugugag ggucaga 107

<210> 1626
<211> 132
<212> RNA
<213> Homo sapiens

<400> 1626
   ugauguaagu uaagggucua acuucauucu aucccaugug gauauuccau uuuuccagca 60
   cuguuuguug aaaagaccau ccuuucuuca uugaaugguc uuagcacucu uguugaaaau 120
   aauuugauca ua 132

<210> 1627
<211> 91
<212> RNA
<213> Homo sapiens

<400> 1627
uagauaaaca guuuuaacaa gagcauuugu caaaaagacu guccuuuccu ccauugaaug 60

gucuuagcac ucuuguuaaa gauuauuuga c 91

<210> 1628
<211> 60
<212> RNA
<213> Homo sapiens

<400> 1628
ucgcuagcuu cuagucaccu cggcucuucc ucagccccgg gagguacaga auuggcgaga 60

<210> 1629
<211> 55
<212> RNA
<213> Homo sapiens

<400> 1629
ugggugcugu aggcuuugag ugcagaggca agaaccaugg aguauuauuc ccaua 55

<210> 1630
<211> 90
<212> RNA
<213> Homo sapiens

<400> 1630
agacacagcu uccggcggaa gcucuuaccg cacacgccac accggaagug ccucuguacc 60

cgagggcugc uccacggcau cugcgucucc 90

<210> 1631
<211> 52
<212> RNA
<213> Homo sapiens

<400> 1631
ccugccuagg cgucuggcgu ccguuugucu guccgaggcc ucagcgcagg ua 52

<210> 1632
<211> 62
<212> RNA
<213> Homo sapiens

<400> 1632
gguuucgaug gacgaggacu caaggcagaa aauggacaau uugaggaauu uggccagacu 60

uu 62

<210> 1633
<211> 57
<212> RNA
<213> Homo sapiens

<400> 1633
ucugcaaacc agccagaaau ugaccagcau uuagcaauga ugguugcuuu gcaggag 57


<210> 1634
<211> 98
<212> RNA
<213> Homo sapiens

<400> 1634
uucgccgucu ggcgagcccu uagccuugcu guagagacuu ccgucacccu ugguagaguu 60

uauuuuuuug auggcuaaga uacugcugau gcugaaau 98


<210> 1635
<211> 87
<212> RNA
<213> Homo sapiens

<400> 1635
aaaccaugua agggcuggau ugaggcuaca aauuucaggg agaacuuuuu ucucuauuca 60

gugccaaguu guuuauagcu gguuuuc 87


<210> 1636
<211> 75
<212> RNA
<213> Homo sapiens

<400> 1636
agaaaauuac agcuaucuua gucuaaaaug ugcuauuauu aaaagaaauu uugguuaaau 60

auuaugugag ucuaa 75


<210> 1637
<211> 62
<212> RNA
<213> Homo sapiens

<400> 1637
ucuuugcuca agagaagagc ucaaccacag ucaaggcucu gccacucuac aauacaggga 60

ug 62


<210> 1638
<211> 126
<212> RNA
<213> Homo sapiens

<400> 1638
acaagggagc cacaaggggg uucuguagcu ucuuggcuca gggcaccuag cauuuugaac 60

ucaagccagu cccaaggguu uggugaucuc agcugagucc ucacugccag ccugaauccu 120

cuguca 126


<210> 1639
<211> 78
<212> RNA
<213> Homo sapiens

<400> 1639
guucaguccc aaggguuugg uuguuuguuu auucuaaugu guugcugcaa uaggaaaccc 60

aaagcuaggg cuggacgc                                                    78

<210>  1640
<211>  75
<212>  RNA
<213>  Homo sapiens

<400>  1640
agcaacaaca cugccaguuc cguugcccug ucauucggca uggcaaagga aauuuggcuc      60

ugugcauuuu gcuuu                                                       75

<210>  1641
<211>  55
<212>  RNA
<213>  Homo sapiens

<400>  1641
accugaaggg ccuugacgau cuggacgccg augccgauga uggcugggca ggugg           55

<210>  1642
<211>  97
<212>  RNA
<213>  Homo sapiens

<400>  1642
gggcgcaccu gggcccgccc cuggugccgg ggaagaggcg gggcuugcgc gggccucgcc      60

cccaacagaa ccuggcgggg cggggcgggg cgucugc                               97

<210>  1643
<211>  97
<212>  RNA
<213>  Homo sapiens

<400>  1643
ggggcggggc cggcggcggg gaacgaccgu ugggucgcgg gauggggagg gacccggcug      60

gcggggcggg gcggagccgg cuguugccca gacccac                               97

<210>  1644
<211>  81
<212>  RNA
<213>  Homo sapiens

<400>  1644
cccggccggc cguagccgcc aguaccgugu ggaagcguga gagucgaggg guggcacgug      60

acuggcgggg cggggcgggg c                                                81

<210>  1645
<211>  157
<212>  RNA
<213>  Homo sapiens

<400>  1645
ggaggccgcc caggaagagg aagcugugag aagugagaag aggcgauaaa ggaagagggc      60

agagcuggcc cgcugcugug accggguagc accguggaaa gcgcccaugc ccagcuccau     120

ggcucgcugu gggugacccu gugcacuaag cuucuga                              157

<210>  1646
<211>  112
<212>  RNA
<213>  Homo sapiens

<400>  1646

cuaaugaguu uuauguaagu uauauggucu uaauuuggua cuuguaaaua gcacuaguua 60

gacucuuuag aauacuccaa gaguuagggc agcagagugg agcgauuuag aa 112

<210> 1647
<211> 70
<212> RNA
<213> Homo sapiens

<400> 1647
gcugacuggg gcgggcggcg gaggggcggg gaaggagggg gcucccaggg cagccuggcc 60

cagggagcag 70

<210> 1648
<211> 57
<212> RNA
<213> Homo sapiens

<400> 1648
ggcugcuggu ccggggucgc ccgcggggcc gggcggcgga ggggccgggg acgccgg 57

<210> 1649
<211> 86
<212> RNA
<213> Homo sapiens

<400> 1649
augcuuuuaa guuuuuauuu cuaauccuau augccccugg acguaguuaa gaggcuucau 60

gggccagaaa uucccauuuu gcauau 86

<210> 1650
<211> 53
<212> RNA
<213> Homo sapiens

<400> 1650
ucuccugggg acagcuucug gggauaagcc aaagccucca ccaccuggag gac 53

<210> 1651
<211> 72
<212> RNA
<213> Homo sapiens

<400> 1651
cacagacuga agacuacacu cucagcuucu cuacuuuuga gauuuuggga uucaggcugg 60

cuuccuugug ca 72

<210> 1652
<211> 93
<212> RNA
<213> Homo sapiens

<400> 1652
uauuuccaca ugcagagcag cuugugggcu agaagcagau gccagugguu acauaguagc 60

aaggguuuua gacuacacuc ucagggaaau guu 93

<210> 1653
<211> 112
<212> RNA
<213> Homo sapiens

<400> 1653
uacuguuuug ggaugguuag aaaugcaaca guagauagca gagauauuuu gauuuuugua 60

auuuuuugcu guaaccgucu ucuacuacau uaaauuaucu aaaacaugug aa 112

<210> 1654
<211> 75
<212> RNA
<213> Homo sapiens

<400> 1654
ucuaauaucu aagcuuacug cucuggguua gagccuaucu cuucuacuua gaugugugcc 60

ccaauauguu agaua 75

<210> 1655
<211> 85
<212> RNA
<213> Homo sapiens

<400> 1655
uuugggagua augugugcuc uguaaguuug uuuuaaauug cacuguuuuu aaagaaacug 60

uagaggagca acaaaaaucc aagca 85

<210> 1656
<211> 95
<212> RNA
<213> Homo sapiens

<400> 1656
gugaagcugg agaauccacc ucugagcuca cucgcugguu gcuggcaggc cuuaguccuc 60

aacagcugug ggccagaggc uucaguuucc ugcca 95

<210> 1657
<211> 81
<212> RNA
<213> Homo sapiens

<400> 1657
gccugcuacg aguagaacgc uguccgcagc uugcgcauuu cgcagccgcu gccgccucgc 60

cgcugcuccu ucguaaggcc a 81

<210> 1658
<211> 140
<212> RNA
<213> Homo sapiens

<400> 1658
uuaggucucu ccucccuuua gcaaucuucu ucuagagcca uuucacugcu cgguuucaau 60

cauguccaau ggaaacagga aacagagcca guuugacugc uuauuugaac ucuauggcug 120

auuaugcccu gacacugaag 140

<210> 1659
<211> 105
<212> RNA
<213> Homo sapiens

<400> 1659
gcugcugucc ucaucggagg ccagugcccu gggccaccuc agcuuccugg agcagcagca 60

gagcgaggcc agccuagagg uggcuucugg cagccacagc ggcag 105

<210> 1660
<211> 77
<212> RNA
<213> Homo sapiens

<400> 1660
cagcgucugg uacgugagau cgcgcaggac uucaagaccg accugcgcuu ccagagcucg 60

gcugugaugg cgcugca 77


<210> 1661
<211> 73
<212> RNA
<213> Homo sapiens

<400> 1661
ggaguacgug cagcagcuga guggcuauuu ccugcagcug aaauuugacc cagagcugcu 60

guucggugug cag 73


<210> 1662
<211> 55
<212> RNA
<213> Homo sapiens

<400> 1662
caacaauuuu aaaugaguaa uacugagaug gaggacaagu uugagaauuu uugcu 55


<210> 1663
<211> 101
<212> RNA
<213> Homo sapiens

<400> 1663
acggguggcc cucaacacgc uggcccgcug caguggcauu gccagugcug ccgccgcugc 60

aguggaggcc gccagggggg ccggcuggac ugggcacgug g 101


<210> 1664
<211> 86
<212> RNA
<213> Homo sapiens

<400> 1664
uacauugucu aggcugggcu cgaacuccug gacucaagua auccaccugu cucggccucc 60

caaaguccug gaauuacagg ugugag 86


<210> 1665
<211> 70
<212> RNA
<213> Homo sapiens

<400> 1665
cugccagggc ggugucugag ccugccggcg ccaccugggc cccggcccuu gagguacucc 60

cugagcagga 70


<210> 1666
<211> 58
<212> RNA
<213> Homo sapiens

<400> 1666
ucaguccugu uguuugcgag caaaaauuga caugaguagu aacaauggau gcaugaga 58


<210> 1667
<211> 106
<212> RNA
<213> Homo sapiens

<400> 1667

ucagugaguu ccaagcuggg auugagaagg gagcagugaa gggaccggga gguuauacuu 60

uccucugaug acuuccuguu agugccacgu gucugggcca cugaga 106

<210> 1668
<211> 89
<212> RNA
<213> Homo sapiens

<400> 1668
aagcuaugga uugguagugu uuuuguccuu ugggguauac aauaaauauu ugguuuucag 60

accuaggaaa uacucagaug aaugcuuuu 89

<210> 1669
<211> 61
<212> RNA
<213> Homo sapiens

<400> 1669
uuagaggcug ucuuuucagc uguaauggug gcuauccugc ugaagcuugg aacuucugga 60

c 61

<210> 1670
<211> 57
<212> RNA
<213> Homo sapiens

<400> 1670
uaucuuacug ucauuguccu aaaaccuuau uuuagaaaau ugaucaagua acauguu 57

<210> 1671
<211> 74
<212> RNA
<213> Homo sapiens

<400> 1671
uuugaggaua uggguuuguu guccaaacug uuuuuagaaa uggacuuaca ugaccuaaga 60

ccuguucuua aaaa 74

<210> 1672
<211> 81
<212> RNA
<213> Homo sapiens

<400> 1672
gggcacacac agggucacgc cguuaccgga ggcuucagug gaggaaguaa ugcugagccu 60

gcgaggccug acaucgcuua c 81

<210> 1673
<211> 84
<212> RNA
<213> Homo sapiens

<400> 1673
cacauugggu cacgccauua cugggggcuu caguggagga aguaaugcug agccugcgag 60

gccugacauc acuuaccagg uggg 84

<210> 1674
<211> 100
<212> RNA
<213> Homo sapiens

<400> 1674

cuggcguggc ugugggcucu gcuguggguc acgccauuac uggggcuuuc aggggaggaa 60

guaaugcuga gccugcgagg ccugacauca cuuaccagga 100

<210> 1675
<211> 61
<212> RNA
<213> Homo sapiens

<400> 1675
gucaugccau uacugggggc uucaguggag gaaguaaugc ugagccugcg aggccugaca 60

u 61

<210> 1676
<211> 73
<212> RNA
<213> Homo sapiens

<400> 1676
uauuaggagu gcucuguugu uuacaaacuc agguacccgc agggccuagc aagagacuua 60

aaugacugau aag 73

<210> 1677
<211> 126
<212> RNA
<213> Homo sapiens

<400> 1677
ucguauuggg cgccugguca ccagggcugc uuuuaacucu gguaaagugg auauuguugc 60

caucaaugac cccuucauug accucaacua cauggugagu gcuacauggu gagccccaaa 120

gcuggu 126

<210> 1678
<211> 131
<212> RNA
<213> Homo sapiens

<400> 1678
gagguguacg acaagaucug caaggccgcc aggcacugag cuggagcccg ccuggagaga 60

cagacacgug ugagugguca ggcaucuucc cuucacucaa gcuuggcugc uuuccuagau 120

ccacacuuuc a 131

<210> 1679
<211> 74
<212> RNA
<213> Homo sapiens

<400> 1679
uucaacucuu caucggcuga gagggcguag gaaucauguc uuuccuacuc aucgguugau 60

gguaugcucg aaca 74

<210> 1680
<211> 85
<212> RNA
<213> Homo sapiens

<400> 1680
agaaucccug uaggcagugc uaaaaaauaa uuuaagcauu ucuaaaugug uuucuuugaa 60

gucauugauc ccuuugguuu ucuag 85

<210> 1681
<211> 77
<212> RNA
<213> Homo sapiens

<400> 1681
agugguccuc caccagauca gaccuguuga uagaugaaaa cugucugcuu ucuccucugg 60

cgggagaaga cuauucu 77


<210> 1682
<211> 116
<212> RNA
<213> Homo sapiens

<400> 1682
gcggggggca accugguaug ugaggugaaa ccaaaagcac aaagcuuagu ugugagucau 60

cuguugauga auccacugug ccacugaucc cuucaugaac cccuugccca cugcua 116


<210> 1683
<211> 92
<212> RNA
<213> Homo sapiens

<400> 1683
uggguuacag ugaccggcag cacaggagag auuggcuucc ccuuuuugaa ggugaacugg 60

gucuuuuug uccuuuuuu aaguagccua aa 92


<210> 1684
<211> 58
<212> RNA
<213> Homo sapiens

<400> 1684
gccgggggcg gggcgcggcg gcugucagcu gacuguggcg gcggcggccu cgagguga 58


<210> 1685
<211> 72
<212> RNA
<213> Homo sapiens

<400> 1685
ucagcugaau cuccagggag gcugcagagc aucguggaga ccacuuucug ccucggccuu 60

uaaaugcuga cu 72


<210> 1686
<211> 97
<212> RNA
<213> Homo sapiens

<400> 1686
uucuucacuu uaaagagugg caaagucuuu ccauaugugu aacagacaua cauacauaca 60

uauggaaaga cuuugccacu cuugaaagug aagagug 97


<210> 1687
<211> 73
<212> RNA
<213> Homo sapiens

<400> 1687
aagaguggca aagucuuucc auauguaugu auguaugucu guuacacaua uggaaagacu 60

uugccacucu uua 73

<210> 1688
<211> 57
<212> RNA
<213> Homo sapiens

<400> 1688
ggcugccuag guccagugcc cagggaggac caggcggagg ccuggggcug cagcugc 57


<210> 1689
<211> 103
<212> RNA
<213> Homo sapiens

<400> 1689
gggcuuugug uuggggccuc ucaugguugc ugccucuggg ggacucuguc cucagacaca 60

gggucccgca ggggaaucau ccugagagcu uccuggggcc ccu 103


<210> 1690
<211> 60
<212> RNA
<213> Homo sapiens

<400> 1690
cuggaggagc uggaggugcu ggagaggaag cccgccgccg ggcuguccgc ggccccagug 60


<210> 1691
<211> 77
<212> RNA
<213> Homo sapiens

<400> 1691
uccagcagaa aacaagcaca agcuuccagu uguacccuca auaugaaccg ugcguguuug 60

auucuuugac augggag 77


<210> 1692
<211> 53
<212> RNA
<213> Homo sapiens

<400> 1692
aagagaguga gccgcaauug uucugaaaau gucaaacgag gcuucuguuu ugc 53


<210> 1693
<211> 104
<212> RNA
<213> Homo sapiens

<400> 1693
ccauucaaau auaccacagu uuguuuaacc uuuugccugu ugguugaaga ugccuuucaa 60

caggugacug guuagacaaa cugugguaua uacacaggau ggaa 104


<210> 1694
<211> 62
<212> RNA
<213> Homo sapiens

<400> 1694
gugaauaugc ggcucuugug uucagggucu uguugggcag ccagaggaau gcauggacac 60

aa 62


<210> 1695
<211> 135
<212> RNA
<213> Homo sapiens

<400> 1695
gccaucuuug uugauguguc agcuccgcag ggguuugggg aaacggccgc ugagugaggc 60

gucggcugug uuucucaccg cggucuuuuc cucccacucu uggcugguug gaccccgcua 120

uggaaaaguu ggccc 135


<210> 1696
<211> 101
<212> RNA
<213> Homo sapiens

<400> 1696
uguacaacag uuggcacauu uuagagacaa auuucccacc aucaucuccc aaauuacacu 60

gauaaaaucc ugucacugca auggcauucu ggaugaacau c 101


<210> 1697
<211> 86
<212> RNA
<213> Homo sapiens

<400> 1697
gguauuugcu ucauaggcag uuugacugca uguauuagag aaugaaaaga agauauuugu 60

aguaaugccu ggaaacuugg ugcuuu 86


<210> 1698
<211> 86
<212> RNA
<213> Homo sapiens

<400> 1698
cugaaacgca ugaccucgug gguccugaac ugaccucugg ucacaacacc cagggccugg 60

uucucccauu ggguaaaucu ucaggu 86


<210> 1699
<211> 113
<212> RNA
<213> Homo sapiens

<400> 1699
ugggggucac ugacccugcu uggugggguc ccccuuguaa caauaaaauc uauuuaaacu 60

uuacuucaga aauucauugu gccuucccuc cugccugggc ucaugccucc acu 113


<210> 1700
<211> 84
<212> RNA
<213> Homo sapiens

<400> 1700
ugugacuuga ggauaggguu aaagucaaau uaccacuuuu uuuuuuuuuu uuuuuuuuuu 60

uuuuuuuugu cuuuuggaaa cagu 84


<210> 1701
<211> 95
<212> RNA
<213> Homo sapiens

<400> 1701
gaugugaaug uaggauucgu aggagcugag cauuugugcu ucaggcauca aguugucuuu 60

gacucuucga cucuuccuug gucuuuuucc auucu 95

<210>  1702
<211>  102
<212>  RNA
<213>  Homo sapiens

<400>  1702
accuagggag cuucaaacga caaaugugca gccacgcccu ccagcaaguc ucaauguguu      60

ggucuuuggg aggcccgagc auucugaagu gcggcugggu uu                          102


<210>  1703
<211>  132
<212>  RNA
<213>  Homo sapiens

<400>  1703
cagggacugu gaaacucacu agaucuguua ggcuggugca aaaguaaugg cgguuuuugc      60

cguuaaaggu aauugcaaaa accgccauua cuuuugcacc aaccuucagg uucgggucua     120

cgcagcuuug uc                                                           132


<210>  1704
<211>  52
<212>  RNA
<213>  Homo sapiens

<400>  1704
uaggcuggug caaaaguaau ggcgguuuuu gccauuacuu uucauuuuua cc               52


<210>  1705
<211>  79
<212>  RNA
<213>  Homo sapiens

<400>  1705
ggcuuauguu uuauuaggcu ggugcaaaag uaauggcaaa aacugcaauu acuuuugcac      60

caaccuaaua uauugccaa                                                    79


<210>  1706
<211>  97
<212>  RNA
<213>  Homo sapiens

<400>  1706
gcagaacgca gcccgcuccu ccagggcccu ccaggcccuc cggccccggg ccggcgggug      60

aacugggggg ccccgggaca ggccgagccc ucugccc                                97


<210>  1707
<211>  60
<212>  RNA
<213>  Homo sapiens

<400>  1707
uccagggccc uccaggcccu ccggaccegg gccggcgggu gaacuggggg gccccgggac      60


<210>  1708
<211>  152
<212>  RNA
<213>  Homo sapiens

<400>  1708
gccugcccuc uuuggacagc agcuccagga cagggcgggu gggcugacca cccaaacccc      60

aucugggccc aggccccaug ccccgaggag ggguggucug aagcccacca gagcccccug      120

ccagacuguc ugccucccuu cugacugugg cc                                     152

<210> 1709
<211> 121
<212> RNA
<213> Homo sapiens

<400> 1709
aagcaagacc aacagucucc ccucauggaa ggcccacuga ucuaaaaugc ugguuccuuu 60

uggaccuuca gggcacuugg gggagaccuu ccugaggugc ugugcagugu cugguguuuc 120

u 121


<210> 1710
<211> 110
<212> RNA
<213> Homo sapiens

<400> 1710
gguauuuuga ugcuuugcug gcuggugcag ugccugaggg aguaagagcc cuguuguugu 60

cagauagugu cuuacucccu caggcacauc uccagcgagu cucuugcucu 110


<210> 1711
<211> 102
<212> RNA
<213> Homo sapiens

<400> 1711
ggccuguguu caucuguuuc gugaggcucu cagcaagucc uuccagugug gaaacucaug 60

uccuauccug ggaguuucuu gaaacauucc uuuucuugcc cu 102


<210> 1712
<211> 89
<212> RNA
<213> Homo sapiens

<400> 1712
uguuucgcca agugcacuca ccauacuucu guaccuuucc agugccccac cuuagaacag 60

uaugccauga gagcguuugc cgacgcacu 89


<210> 1713
<211> 89
<212> RNA
<213> Homo sapiens

<400> 1713
gggcucuccc uuuccccucu cuccuccauc uguaacccuc ucacucccag cuuacacuca 60

ggggaggcgg gaaagcaggu ugugucugu 89


<210> 1714
<211> 89
<212> RNA
<213> Homo sapiens

<400> 1714
gggcucuccc uuuccccucu cuccuccauc uguaacccuc ucacucccag cuuacacuca 60

ggggaggcgg gaaagcagga ugugucugu 89


<210> 1715
<211> 116
<212> RNA
<213> Homo sapiens

<400>  1715
gcgcccggcg gcggcggcgg cauggccaag gccagcgcgg cugagcugaa ggucuucaag      60

uccggcagcg uggacagccg uguccccggc gggccgcccg ccuccaaccu gcgcaa          116


<210>  1716
<211>  135
<212>  RNA
<213>  Homo sapiens

<400>  1716
cgccgcgcuu gccggagaua gcauugaggg cccuuccgcg gcccggcguu ucgucucuug      60

ggacgauuuu gugcguuccc ucgguccccg uggggcccau aguccucacu ucugccaccc     120

cagagucguc gcgcu                                                      135


<210>  1717
<211>  56
<212>  RNA
<213>  Homo sapiens

<400>  1717
uugugaugga gucuugcucu uugcccaggc uggagugcag uggcuugauc ucagcu          56


<210>  1718
<211>  53
<212>  RNA
<213>  Homo sapiens

<400>  1718
gcucgccugc aucuacucgg cccucauucu gcacgacgau gaggugacag uca             53


<210>  1719
<211>  130
<212>  RNA
<213>  Homo sapiens

<400>  1719
gcugggaaau cuccguuuug ugguuucucc ucaggcuacc ucucuggguu cuguuucuuc      60

ccuguaaaau acuuuagcaa cagaacccag agagucagcc ugaguggaaa gcacugaaau     120

gcccuggcuc                                                            130


<210>  1720
<211>  59
<212>  RNA
<213>  Homo sapiens

<400>  1720
auuucauaga gagcugaugg guucauuuua cagagagcug cuuggucugu uuacaaucc       59


<210>  1721
<211>  88
<212>  RNA
<213>  Homo sapiens

<400>  1721
agccucucuu ggcagggagg gugugggcag ccguuccagc uucagcgucu ggagcuugug      60

gcucuucugc cugccgaggc agagcucu                                         88


<210>  1722
<211>  71
<212>  RNA
<213>  Homo sapiens

<400> 1722
gugcaguaaa ggcaccagca uuuugcggca ccguagguga ggccggcggc gugggccuuu 60

ucucugcacg g 71


<210> 1723
<211> 78
<212> RNA
<213> Homo sapiens

<400> 1723
cgcagcuggg gccagcggug ccaagcgcag cuggacgagc ggcagcagcu gggcgaguga 60

cagccccggc uccgcgcg 78


<210> 1724
<211> 122
<212> RNA
<213> Homo sapiens

<400> 1724
ccgugccugc agacgcgcgg aucguccaug cgcuccucgc gggcagaaug cugggcagca 60

gcgucaagag cgugcagccc gagguggagc ugagcagcgg cggcggcgac gagggcgcgg 120

ac 122


<210> 1725
<211> 109
<212> RNA
<213> Homo sapiens

<400> 1725
uccgcgugug gaagguccgu ccgcgugugg aagguccguc cgcgugugga agguccguca 60

guguguggaa gguccguccg cguguggaag guccguccgc guguggaac 109


<210> 1726
<211> 97
<212> RNA
<213> Homo sapiens

<400> 1726
ucugucagcg uguggaaggu cuguccgcgu guggaagguc cguccgcgug uggaaggucc 60

guccgcgugu ggaagguccg ucagugugug gaagguc 97


<210> 1727
<211> 108
<212> RNA
<213> Homo sapiens

<400> 1727
uggaaggucu guccgcgugu ggaagguccg uccgcgugug gaagguccgu ccgcgugugg 60

aagguccguc agugugugga agguccgucc gcguguggaa gguccguc 108


<210> 1728
<211> 90
<212> RNA
<213> Homo sapiens

<400> 1728
ggguugguuc caagucuuug cuguugugaa caaugcugca guaaacauac augugcaugu 60

gucuuuauag uagaaugauu uauaacccuu 90


<210> 1729

<211>  110
<212>  RNA
<213>  Homo sapiens

<400>  1729
gguggggggu gcucagcucu gaccaugucu ggaugcaguu gggguggggg ugcccagcuc      60

ugaccauguc uggauacagu cggcgggggg cuuugcuuag cucugaccau      110


<210>  1730
<211>  108
<212>  RNA
<213>  Homo sapiens

<400>  1730
gucggggagg ggugcucagc ucugaccaug ucuggaugca gucggggggu gcccagcucu      60

gaccaugucu ggauacaguc gggcgggggg ggggcucagc ucugacca      108


<210>  1731
<211>  122
<212>  RNA
<213>  Homo sapiens

<400>  1731
gugcucagcu cugaccaugu cuggaugcag ucggggggug cccagcucug accaugucug      60

gauacagucg ggggggggug cccagcucug accaugucug gauacagucg ggggggggug      120

cc      122


<210>  1732
<211>  105
<212>  RNA
<213>  Homo sapiens

<400>  1732
gcugugggcg gcuucuccgu gggcuacuag cgggcccggc agcgaccagc uggucucggc      60

uuccagcucg cggguucagg gaaggugagu caugcguccc agcaa      105


<210>  1733
<211>  67
<212>  RNA
<213>  Homo sapiens

<400>  1733
   ggcuucuccu gaccugggga gucgcgcacu guuggacuug cucuuaaggc uggggguuug      60
   ggguugu      67


<210>  1734
<211>  53
<212>  RNA
<213>  Homo sapiens

<400>  1734
cugccccuga ggaugcucag aagaguucuu cuccagcacc ugcagacaua gcu      53


<210>  1735
<211>  53
<212>  RNA
<213>  Homo sapiens

<400>  1735
gaguucuucu ccagcaccug cagacauauc ucagacagua ugggaagacu uga      53


<210>  1736

<211> 92
<212> RNA
<213> Homo sapiens

<400> 1736
gagcgcgacu uggccuuggc gcgggccuug ccgccagucu ugccgcggcc cgacaugcua 60

gcgagguaga ccggugaagc acgacggcuc aa 92


<210> 1737
<211> 62
<212> RNA
<213> Homo sapiens

<400> 1737
gggaacugaa ggccagcgcg ggacgagcgc gacuuggccu uggcgcgggc cuugccuccu 60

ug 62


<210> 1738
<211> 95
<212> RNA
<213> Homo sapiens

<400> 1738
ugguaauucu auguuuaacu uucugaggaa cugagaauaa guuuuaugcc uuuaaucccc 60

ucuuagacau uucagaaauu gggagaagug ccaag 95


<210> 1739
<211> 162
<212> RNA
<213> Homo sapiens

<400> 1739
guugaaaagc uaauucauau uguccaguuu aaaagaaauc auaagauguu ucguguagaa 60

agcaaaauaa uuguaauuuu aguguuuaua cuugcucuuu ugcuaaaaga aaggucaguu 120

ugguggugau uauagaacuu gguauagaau ucuaauuaaa cu 162


<210> 1740
<211> 129
<212> RNA
<213> Homo sapiens

<400> 1740
aauaaccucc uaccaauuuu aauucaguuu aaauuccuaa uauucccaug agguagguaa 60

aauggauuau cauuauccca uuauauaagu gagggaacug agauuuuaag agguuaaaag 120

gcuuguuua 129


<210> 1741
<211> 143
<212> RNA
<213> Homo sapiens

<400> 1741
ucucaguagu guuaaagagu cugacacuag ccuggccacc accagcacuc ugggauuugg 60

cugcaaauaa gcaaaugcuc cagggugaac aauguuuaca gguugcaagg uguagaaaga 120

uaguaaaugc ugauucugag aac 143


<210> 1742
<211> 115
<212> RNA
<213> Homo sapiens

<400> 1742
   ccgcacagaa gauuggauuu ucgucaaggu uuacaacugu gcccucugga gacggggaaa 60
   uuaggaguug gaggaguagg ggaugugagc auauguauuc auaggcuguu uggcu 115


<210> 1743
<211> 81
<212> RNA
<213> Homo sapiens

<400> 1743
ugugaggaaa auaucgacga uuguccagga aacaacugca agaacggggg ugccugugug 60

gacggcguga acaccuacaa c 81


<210> 1744
<211> 113
<212> RNA
<213> Homo sapiens

<400> 1744
gaauuauaca gguagagaug uaugcagaug uguccauaua uguccauauu uacauuuuga 60

uagccauuga uguaugcauc ucuuggcugu acuauaagaa cacauuaauu caa 113


<210> 1745
<211> 76
<212> RNA
<213> Homo sapiens

<400> 1745
auuaugcuca auauucccag aauaguuuuc aauguauuaa ugaagugauu aauuggcucc 60

auauuuagac uaauaa 76


<210> 1746
<211> 84
<212> RNA
<213> Homo sapiens

<400> 1746
uuuguagaga ggaaaggagg uuugucaaaa uaauuuuaua acaaugggcu auuuuaaaaa 60

auccacuuaa uuauacuuca agga 84


<210> 1747
<211> 110
<212> RNA
<213> Homo sapiens

<400> 1747
agaagcuagc aguuauacau acauucggag auugcauagu aguaauaaga auaauauuca 60

uuguuacugu ucuuaaauug acauauuucu aguuggacug aaguuucucu 110


<210> 1748
<211> 73
<212> RNA
<213> Homo sapiens

<400> 1748
uccugcgcac uacagccucc ggcagacaca ggcagggcac aucgcgccgg ggugugcugc 60

agcccgcggg gac 73


<210> 1749
<211> 72

<212>  RNA
<213>  Homo sapiens

<400>  1749
uguucucucc ugcuggugau ggacuaggcc uuuuagaacu agcaagaucc cucacacagc      60

ugggagaaca ca                                                          72


<210>  1750
<211>  70
<212>  RNA
<213>  Homo sapiens

<400>  1750
agccuguguu aaggagcaac cuuucggugg ccagauggag ccggggcauc cuucuucaaa      60

uaauggcuuu                                                             70


<210>  1751
<211>  135
<212>  RNA
<213>  Homo sapiens

<400>  1751
gcuucccaca gacagagaug ugaguccccu ccccagcucc acuugcuccu gguauaaacg      60

uacauuaaug aagacugcca gucaggcagg aaugggccca gaccccuauc ucaacaaccc     120

ccacgugaaa agccu                                                      135


<210>  1752
<211>  111
<212>  RNA
<213>  Homo sapiens

<400>  1752
ggcccuguga gggaggcugg caggcgcugc ccgcugucac uccaugccgg ugcccuuugc      60

uccacuucag uggccacgug accaggcccc auuccugccc ucuaggguug u              111


<210>  1753
<211>  119
<212>  RNA
<213>  Homo sapiens

<400>  1753
guucggcuca agugaauugu cugcaaauau uaaaaauucu aguuugggcu aagauuuaug      60

uguagcuuaa cccaaacuag aguuguuaau acuuucagaa aauaaaaccu ccccaaugu      119


<210>  1754
<211>  56
<212>  RNA
<213>  Homo sapiens

<400>  1754
agcccuccug ggcugggcuu gggcacaaau ccccaggcag gcuuuggagu uguuuc          56


<210>  1755
<211>  54
<212>  RNA
<213>  Homo sapiens

<400>  1755
gggcuggucc caggcguggg gcggcuucuu gguccugacc uggcaccugc ccca            54


<210>  1756
<211>  67

<212>  RNA
<213>  Homo sapiens

<400>  1756
ggauauuggu cuaccugagg cuguugguca aacuguacag guuucugguc agacggaaca      60

acuccac                                                                67


<210>  1757
<211>  66
<212>  RNA
<213>  Homo sapiens

<400>  1757
gugcucacuu cuuccugggg acacucaucu guugaguacu ccugccgggg aagcuucaga      60

gugccc                                                                 66


<210>  1758
<211>  114
<212>  RNA
<213>  Homo sapiens

<400>  1758
gccacaaacu ugccagucug caacaaagca cagggcagcg agagaaggcg gcauggcagc      60

ucagcuggga guuucaccgc ugcccugugg aagagaauag uuggugugag gcaa           114


<210>  1759
<211>  78
<212>  RNA
<213>  Homo sapiens

<400>  1759
gggccugcgg cacuugcggg ccucccugcu ggcuacugau gccaguacca gaggagcccc      60

aaaacuuuga cagccucg                                                    78


<210>  1760
<211>  102
<212>  RNA
<213>  Homo sapiens

<400>  1760
uaugcugguc auugaugauu uauuuacgua acaaaaaucu gagguucaug cuucauguuu      60

aaccucuuug cccugugucu gucccccaaa gcugugcaug ca                        102


<210>  1761
<211>  81
<212>  RNA
<213>  Homo sapiens

<400>  1761
uagcgcgggg cuucuguacg cugcuaggaa gccgaauuac aaaaaggucg ggugggcagg      60

gaaugguggc ucacgccuau a                                                81


<210>  1762
<211>  104
<212>  RNA
<213>  Homo sapiens

<400>  1762
ggacuccauc uguuuccccg uuaugcccuc cacgcguucc agguccucca ccuggcggga      60

caggaucgau aggggacagg gucucggggg uacccggucu cccc                      104

<210> 1763
<211> 72
<212> RNA
<213> Homo sapiens

<400> 1763
auggcuggag cugcagcugc ugggaugcag ggugccaugu cccaaggcuu ugcagagccc 60

acaaaaccau uu 72


<210> 1764
<211> 59
<212> RNA
<213> Homo sapiens

<400> 1764
cacuccucca ccgcugagag uugaauagcu uuucuucugc aaugggaguu gggagugau 59


<210> 1765
<211> 68
<212> RNA
<213> Homo sapiens

<400> 1765
gcuauugugu cccaucuggg ucgccaaaau guguccaaaa uugguuccuu ccgguggguu 60

cuuggucu 68


<210> 1766
<211> 60
<212> RNA
<213> Homo sapiens

<400> 1766
aggcaaaugu cccaucuggg ucgccaaaau guguccagaa uugguggguu cuuggucuca 60


<210> 1767
<211> 66
<212> RNA
<213> Homo sapiens

<400> 1767
ggcaauaggc gauuguccca ucugggucgc caaaaugugu cccaucuggg ucgccaaaug 60

uguccc 66


<210> 1768
<211> 76
<212> RNA
<213> Homo sapiens

<400> 1768
uuuguugccu uuucuuugca guuagcuucu ggaauuuaua auuuugccug cucucugugg 60

aaucaccaca cagaca 76


<210> 1769
<211> 61
<212> RNA
<213> Homo sapiens

<400> 1769
uuaaagaaua aacauggugu aucuuuguau cacccuguug cauucucucu ccuuuuuaaa 60

a 61


<210> 1770
<211> 94

<212>  RNA
<213>  Homo sapiens

<400>  1770
ugguuugauc aauuuuuuuc aagcaaguaa uuugauaauu cuuuucagac agaaaauguc     60

auaauguucu uguuacaaaa uugaaagaac caaa                                94


<210>  1771
<211>  109
<212>  RNA
<213>  Homo sapiens

<400>  1771
ugugaaguuc aauauuccug agagaguaau uucuaauguu cuuguuacaa aaauguaaau     60

guuugaagug augauauauu aagcuaauuu aaucuuucug cauugcaau               109


<210>  1772
<211>  131
<212>  RNA
<213>  Homo sapiens

<400>  1772
auggacugcu ccagcuccug aguucacugc uacucggccu gagguugcag acuggucuga     60

aggugugcag gugcccucug ugccuauuca gcaguucccu acugaagacu ggagcgcuca    120

gccugccaua g                                                         131


<210>  1773
<211>  69
<212>  RNA
<213>  Homo sapiens

<400>  1773
gugugcaggu gcccucugug ccuauucagc aguucccuac ugaagacugg agcgcucagc     60

cugccacgg                                                             69


<210>  1774
<211>  72
<212>  RNA
<213>  Homo sapiens

<400>  1774
acggcgugcg ggugcccucu gugccuauuc agcuguuccc uacugaagac uggagcgcuc     60

agccugccgu gg                                                         72


<210>  1775
<211>  60
<212>  RNA
<213>  Homo sapiens

<400>  1775
caggugcccu cugugccuau ucagcaguuc ccuacugaag acuggagcgc ucagccugcc     60


<210>  1776
<211>  69
<212>  RNA
<213>  Homo sapiens

<400>  1776
ugugcaggug cacucugugc cuauucagca guucccuacu gaagacugga gcgcuuggcc     60

ugccacaaa                                                             69

<210> 1777
<211> 78
<212> RNA
<213> Homo sapiens

<400> 1777
ggucugcagg ugcccucugu gucuauucag uaguucccua cugaagacug gagcgcucag 60

ccugccacgg aagacugg 78


<210> 1778
<211> 99
<212> RNA
<213> Homo sapiens

<400> 1778
gagcuuguug ucaagguugc ugaauagacg acugcauagu gaagacacuc ccacugaguu 60

uucguuuguu uguuugguuu uuuugagucg gagucucgc 99


<210> 1779
<211> 134
<212> RNA
<213> Homo sapiens

<400> 1779
ggugggcugc acccuccugc cuauucagga uguggaugcc acaggagagc agcaggcagu 60

ggaaacuuca guugcacugg uucuccuggu ggcaaaggca ugaagcacag gggucgauua 120

auccaggcua cuag 134


<210> 1780
<211> 142
<212> RNA
<213> Homo sapiens

<400> 1780
uuaaagaaau gauuaauuug guauuuuccu uucuaggauc aagcgugcuu uccuuaucga 60

ggagcagaaa aucguuguga aaguguugaa ggcacaagca cagagucaga aagcuaaaua 120

aaaaaaugaa acuuuuuuga gu 142


<210> 1781
<211> 60
<212> RNA
<213> Homo sapiens

<400> 1781
gucuaucgcu gcgguugcga gcgcuguagg gagccugugc ugugccgcgc aguuaggcag 60


<210> 1782
<211> 76
<212> RNA
<213> Homo sapiens

<400> 1782
cuagaggagc cuguucuaac ccaauacaaa gaugcggaga aacuugauaa aagguuagag 60

ggguugcuaa cuagaa 76


<210> 1783
<211> 122
<212> RNA
<213> Homo sapiens

<400> 1783
cacuuguggu aaauuguuac uuaagaaaua acuaaugcau aagcuauuug cggcauucca 60

gcuucaaaaa gauggugcuu guauuagauu ugugguaagg aagauggaga gaaaugagug 120

ga 122

<210> 1784
<211> 58
<212> RNA
<213> Homo sapiens

<400> 1784
ugcucugggg cggcauugcc agccggcugu aggcauucag ggcagugucu ucugcauc 58

<210> 1785
<211> 76
<212> RNA
<213> Homo sapiens

<400> 1785
ggcccacugc ugcugcucuc cagccugcag uuuccucuaa ggcucuggau uggcuggaag 60

agcaacagag ggcugg 76

<210> 1786
<211> 111
<212> RNA
<213> Homo sapiens

<400> 1786
uaauucuuuu caaaagaacu ugaaguaaag caauaagaac ugagagagaa ugguauuagu 60

ucuaucuuag uucuuguugc ucuacuucaa guucuuuuca aaagaauuag u 111

<210> 1787
<211> 67
<212> RNA
<213> Homo sapiens

<400> 1787
cggggcggag aggaugcugg gaauuguagu uuugaaguuu ggauuggcau ggcggccgug 60

ccccggu 67

<210> 1788
<211> 123
<212> RNA
<213> Homo sapiens

<400> 1788
gugucucaaa aaaaaaaaaa aaaaaaagaa agaaagaaaa agaaaagaaa agaaauauaa 60

gaauuuuuca ugauuuagaa cugaaaaaua aguuucuugu cucucuguuu cucaggaaca 120

cca 123

<210> 1789
<211> 57
<212> RNA
<213> Homo sapiens

<400> 1789
gcuggaaaaa acaacgaccu gggcucucag uacggcugcu ucuuuccaac aaggcac 57

<210> 1790
<211> 117
<212> RNA
<213> Homo sapiens

<400> 1790
agguuuuuaa accuacuguc ucugucauaa cuugucaaca cugcuguaug aagcacaaaa 60

gcagccauag acaauacaua aacaauacgg gcguggcuuu guuccaguaa aacuuug 117


<210> 1791
<211> 55
<212> RNA
<213> Homo sapiens

<400> 1791
guuaaaaugu uugaaaaugg uuuccuauca gaagccauac uuggggaugg ggcag 55


<210> 1792
<211> 75
<212> RNA
<213> Homo sapiens

<400> 1792
agaacagggu aauuuggaug gagcaguguu ucacagccug aaaugaugac ucuuuaaaaa 60

auuucauguc ucuuc 75


<210> 1793
<211> 81
<212> RNA
<213> Homo sapiens

<400> 1793
acacccugaa augaugacuc uguugcucuc agcuaggacu auggggagca agaggagugu 60

auuaguuuuc uguggcuguc a 81


<210> 1794
<211> 169
<212> RNA
<213> Homo sapiens

<400> 1794
gcgcggagag gcggggaggg cgggcguggg gaggcgggcg gcggcgggcg gggggagcca 60

aggaggcucg gagccucgcg gcgcgcgggc gggcggcgga gggggcgcgg ggagcccccc 120

gccccacgaa ggaggagucu ggcucccacu ugcagccucg gacgcgcgg 169


<210> 1795
<211> 71
<212> RNA
<213> Homo sapiens

<400> 1795
cuuugucagg aaggucagga agcgcuucag ucaauguggc uugagcugaa augugauggc 60

uggaaaaggg g 71


<210> 1796
<211> 76
<212> RNA
<213> Homo sapiens

<400> 1796
uuucaaaacc uacugggggc accauuucuu cugcagagua ggcugggaag gaggagcugu 60

ccuggguucu gaaaug 76


<210> 1797
<211> 62
<212> RNA

<213>  Homo sapiens

<400>  1797
ccguauugcu auuuuugacu ggagugaggg ucuucuuacu caaaggcaaa uaggucccgg      60

cu                                                                    62


<210>  1798
<211>  94
<212>  RNA
<213>  Homo sapiens

<400>  1798
ugcuguaguu ugcugggggu ccacuccagu cccuagucac cucagauuuu ccaguaccug      60

gagguaccac caguaaagac uucaaaacag caaa                                 94


<210>  1799
<211>  100
<212>  RNA
<213>  Homo sapiens

<400>  1799
auccgugcgu cccuacaggg agggcggucg cuaugaacgc acagcuggga ggguggaguu      60

ggagcugggg acccucgacu ggcagggagg acgcggaugc                          100


<210>  1800
<211>  88
<212>  RNA
<213>  Homo sapiens

<400>  1800
aaaguaaguu aaaugacgug gcagcagcag ugaacaagac gcuguuagcu guuaagauaa      60

uuuacuuagc caagaacaaa agcuuuau                                        88


<210>  1801
<211>  118
<212>  RNA
<213>  Homo sapiens

<400>  1801
uuuuucaacc ugauauuuuu ucccuaacau augcugacac augccaaauu uaacccagua      60

auauaaaagc agucaaaagc aaauuuuggu gaaguauuaa auauuaaauu gagaagcc      118


<210>  1802
<211>  103
<212>  RNA
<213>  Homo sapiens

<400>  1802
ccugucucug ugcaggugcu augcucgccu ucacccucgu gcugucaacu gccgcaagaa      60

gaaguguggu cacaccaaca accugcgucc caagaagaag guc                      103


<210>  1803
<211>  56
<212>  RNA
<213>  Homo sapiens

<400>  1803
ucaacggccu gcgcagggug cuggaugagc ugacccuggc caggaccgac cuggag         56


<210>  1804
<211>  121
<212>  RNA

<213>  Homo sapiens

<400>  1804
ggcccugcag ucgauccgga acaugcgcgg gaacucccac uguguggacu gcgagaccca      60

gaguaagugu gugcgguggu agcaggggcu ggcgcugugu agcuggagac cacauggucc     120

c                                                                     121


<210>  1805
<211>  58
<212>  RNA
<213>  Homo sapiens

<400>  1805
caacuugaca agacugcugc aaacuucaau uugucaacca caacuugaca agguugcu        58


<210>  1806
<211>  68
<212>  RNA
<213>  Homo sapiens

<400>  1806
auuuauuuuu aauuuacccu guggaagauu uuauuagaau uaauuccaag gggaaaacug      60

gugaaucu                                                              68


<210>  1807
<211>  72
<212>  RNA
<213>  Homo sapiens

<400>  1807
uugagccugg auuuuguaau acuuuggaua uuggaauauc cuaauuucca guuggaaaaa      60

uuaguaucaa au                                                         72


<210>  1808
<211>  163
<212>  RNA
<213>  Homo sapiens

<400>  1808
gguaucauau uuuuuuccuu ugcaguuuug guaagacucc cccauuacuu ugcuaagacu      60

ugcuuucuca accaguggga ugaaacgagu gccagugugu ugacugagcc uucuuaggac     120

uuaaggggcu cugggagagg ccaaggcagc aguugaaaac ugc                       163


<210>  1809
<211>  85
<212>  RNA
<213>  Homo sapiens

<400>  1809
ccuguagacu ccggagggca gaggcuggaa ccaucucauu cacugcuuga gcauuccuag      60

cccucuuucu ggcucacuau aggug                                           85


<210>  1810
<211>  77
<212>  RNA
<213>  Homo sapiens

<400>  1810
acuuugcacu ucauagagaa gguacauaga gacauugcaa aaccugucuc cauuugcuau      60

ccugauaauu aagguuu                                                    77

<210> 1811
<211> 66
<212> RNA
<213> Homo sapiens

<400> 1811
auuacagaga ggacuuuggc cuagauaagg auaauaacug guuaaaggcc cacaauucau 60

uaauga 66


<210> 1812
<211> 59
<212> RNA
<213> Homo sapiens

<400> 1812
guucugagga agguuuuccg gggaugcauu agguucugag ggaaguccuc ugugaaugc 59


<210> 1813
<211> 68
<212> RNA
<213> Homo sapiens

<400> 1813
gcauuagguc cugagggaag uucucuggga acacauuagg uucugaggga aguccucuga 60

gaaugcau 68


<210> 1814
<211> 67
<212> RNA
<213> Homo sapiens

<400> 1814
gccccucuuc augcugcauc ugccccugug cuauuaggcc augagggcag agaccauguu 60

cuggcca 67


<210> 1815
<211> 65
<212> RNA
<213> Homo sapiens

<400> 1815
aacaccaaag auguagccuc agaugugaau cuacucacau gcuggaaguu uguccaugau 60

guuga 65


<210> 1816
<211> 105
<212> RNA
<213> Homo sapiens

<400> 1816
aguggucuua uuugggaagc augcugauau ugggguaggg gaggcaacag agcucauguc 60

uguaagccuu gagcagaaga ggugauggua gaaaagauag acuuu 105


<210> 1817
<211> 57
<212> RNA
<213> Homo sapiens

<400> 1817
uaaaaggcaa gauauacaua uguaugugac acacguaugu auaucuugcc cuuuacu 57


<210> 1818

<211>  101
<212>  RNA
<213>  Homo sapiens

<400>  1818
aacgauuugg ggcacugagu uuccugggug auuucuuucu uucacaacau agauugaaac      60

uauccacaca ggcuuuccuc auuuaaacau gaaaucguuc c                         101


<210>  1819
<211>  70
<212>  RNA
<213>  Homo sapiens

<400>  1819
ugacagccug ccaggacucg cagugccuac agagcguggu gcgugcccuc cguaaccugg      60

cagacucacc                                                             70


<210>  1820
<211>  81
<212>  RNA
<213>  Homo sapiens

<400>  1820
cuagccagga cucgcuggcc aucaucccau cuggucccag gaagcauggg caggaggccc      60

uugagacuuc acucacuagc c                                                81


<210>  1821
<211>  90
<212>  RNA
<213>  Homo sapiens

<400>  1821
aagcuuuuug augugcugcu ggauucgguu ugccagaauu uuauugagga uuguugcauu      60

gauguucauc augcauggcc cgaaguuuuc                                       90


<210>  1822
<211>  82
<212>  RNA
<213>  Homo sapiens

<400>  1822
gcaugccaaa gauugccacg uugcaagcac ugguauuauu ucugaaugug ucuugcaccc      60

aguggauuuu auccuugugu ca                                               82


<210>  1823
<211>  92
<212>  RNA
<213>  Homo sapiens

<400>  1823
aaagccccgg cgaggcaggg cuucuaagcc ucugagccuc uucuuagcag agaccaagau      60

gcuugagagc cauuggcucu uggaggcuuu cu                                    92


<210>  1824
<211>  80
<212>  RNA
<213>  Homo sapiens

<400>  1824
gccuuccaag agaaauccuc ucccagucac uuucgggcca gcagagaguc ccgggagcac      60

uugagagcug uugguggccu                                                  80

<210> 1825
<211> 101
<212> RNA
<213> Homo sapiens

<400> 1825
ccuuggaugg agagcuagac cugcuagagc aggacccucu ggugccaccu ccacccucac 60

aggcaccccu cucugcugag cggguggcuu uucccaaggg c 101


<210> 1826
<211> 67
<212> RNA
<213> Homo sapiens

<400> 1826
cgcgaugggg cugggcccug gccuccuaau ggggcugccg ucuggggcuc ucaccccugc 60

ccgcgac 67


<210> 1827
<211> 105
<212> RNA
<213> Homo sapiens

<400> 1827
ccgggaacgc guggggcgga gcuuccggag gccccgcccu gcugccgacc cuguggagcg 60

gagggugaag ccuccggaug ccagucccuc aucgcuggcc cgguc 105


<210> 1828
<211> 105
<212> RNA
<213> Homo sapiens

<400> 1828
ccgggaacgc guggggcgga gcuuccggag gccccgcucu gcugccgacc cuguggagcg 60

gagggugaag ccuccggaug ccagucccuc aucgcuggcc ugguc 105


<210> 1829
<211> 61
<212> RNA
<213> Homo sapiens

<400> 1829
acugguaaga guuaaugcag ggaaaucagc uaggggucgg gcacugcagc agcucaggua 60

a 61


<210> 1830
<211> 68
<212> RNA
<213> Homo sapiens

<400> 1830
ggccuaccgg ugacccggcu agccggccgu gcuccugcuu gagccgccug cuggggcccg 60

cgggccug 68


<210> 1831
<211> 72
<212> RNA
<213> Homo sapiens

<400> 1831
gcugguauca gguuccgggu ucugucugga ggcuccgcuu ccuaugcaga gcuggcgguu 60

uuguaucagc ac 72

<210> 1832
<211> 168
<212> RNA
<213> Homo sapiens

<400> 1832
cagauaucuc ccucuuccau uucaucaaga cccagcugag ucacugucac ugccuaccaa 60

ucucgaccgg accucgaccg gcucgucugu guugccaauc gacucggcgu ggcgucgguc 120

gugguagaua ggcggucaug cauacgaauu uucagcucuu guucuggu 168

<210> 1833
<211> 55
<212> RNA
<213> Homo sapiens

<400> 1833
ccucggagcc gccucgcucc ucuucaggga cuuugcugag aagggcucuc gggcg 55

<210> 1834
<211> 114
<212> RNA
<213> Homo sapiens

<400> 1834
ccuguguugc cugccugcug uaucuuccau gucuuucuua uucaacuucu agaaguagag 60

aagguuggga cuugagugua gaagaaacau cagaauaauu ucaaaaaaca ggaa 114

<210> 1835
<211> 106
<212> RNA
<213> Homo sapiens

<400> 1835
ucgcaggccc ccugcugguc gugggguggg agacacucuc uccucugcgc cugcgcaggg 60

agcuccugcu uaauacgcuc cgcggaaggg ucaagcacug ucgaaa 106

<210> 1836
<211> 70
<212> RNA
<213> Homo sapiens

<400> 1836
gugucugccu ggagaugcug gcuguuuuuu uaaucucugg aagcaccuuu gaguguguuc 60

ugccccaccc 70

<210> 1837
<211> 129
<212> RNA
<213> Homo sapiens

<400> 1837
ggccacgaug ccgcaguacc agaccuggga ggaguucagc cgcgcugccg agaagcuuua 60

ccucgcugac ccuaugaagg cacguguggu ucucaaauau aggcauucug augggaacuu 120

guguguuaa 129

<210> 1838
<211> 100
<212> RNA
<213> Homo sapiens

<400>  1838
ccugggagga guucagccgc gcugccgaga agcuuuaccu cgcugacccu augaagguaa      60

aucgcuggcg gggccgcugc uuugggcccc agcccaggau                            100


<210>  1839
<211>  56
<212>  RNA
<213>  Homo sapiens

<400>  1839
cugucagccu gagaacaggg cuuguuugug ucuuaauccc aggagccugc acggcc          56


<210>  1840
<211>  60
<212>  RNA
<213>  Homo sapiens

<400>  1840
ggggcuucaa ccgggaguac uacucgauug ucaaagucaa ggagucgcag gucgccucgc      60


<210>  1841
<211>  62
<212>  RNA
<213>  Homo sapiens

<400>  1841
aguuuuuuuu uucgucuggg cugccaacau gccauccaga cugaggaaga cccggaaacu      60

ua                                                                     62


<210>  1842
<211>  58
<212>  RNA
<213>  Homo sapiens

<400>  1842
gguaugcaga ccucacagaa gaucagcuac ccuccuguga gagucugaag gauacuau        58


<210>  1843
<211>  82
<212>  RNA
<213>  Homo sapiens

<400>  1843
gcuuaggauc gcagguaugc agaccucaca gaagaucagc uacccuccug ugagagucug      60

aaggauacua uugccagagc uc                                               82


<210>  1844
<211>  63
<212>  RNA
<213>  Homo sapiens

<400>  1844
ggaaaauaau cugcucugaa gucuugcauu ucugugugug uaauacucuu gcgggcgcuc      60

cac                                                                    63


<210>  1845
<211>  73
<212>  RNA
<213>  Homo sapiens

<400>  1845
caggcgugag ccaccgcgca cggccuggag ucucuucugu ugccaggcug gagugcagug      60

gcccaagcuu ggc                                                          73

<210>  1846
<211>  149
<212>  RNA
<213>  Homo sapiens

<400>  1846
ggugcauaaa ugggggcagg gucugauguc ugaaugcuac uguuuaugcc ugucuucuug      60

guuuauuuug gugcagaaug uagccgugag uucaggcagu gggcagaaug aucuggaaaa     120

gaugaccagc auccuggaag cugugccac                                       149

<210>  1847
<211>  77
<212>  RNA
<213>  Homo sapiens

<400>  1847
cuucuggacc cuggcuucuc cccacuguaa cgccuguuac acacaaacug uuguggguuc      60

cugccaggcu ugaagaa                                                     77

<210>  1848
<211>  64
<212>  RNA
<213>  Homo sapiens

<400>  1848
agacauggau uccugaaguu caugaucaga aagcagaugu gucagcuugg aaggaccugu      60

uugu                                                                   64

<210>  1849
<211>  90
<212>  RNA
<213>  Homo sapiens

<400>  1849
ggcuucaaag cagagggccc ugaaguggau gugaaccugc ccaaggcuga cguugauguc      60

ucaggcccca aaguggacgu ugaaggcccu                                       90

<210>  1850
<211>  90
<212>  RNA
<213>  Homo sapiens

<400>  1850
ggcuucaaag cagagggccc ugaaguagac gucaacuugc cuaaggcuga cguugacauc      60

ucaggaccca agguggacau ugaaggcccu                                      90

<210>  1851
<211>  86
<212>  RNA
<213>  Homo sapiens

<400>  1851
ugauaaaaag aagugguuau aucaaacucc ucuacagaug ugaaggagug gacaagaguu      60

gagcagccuu ucugcugauu aucaca                                           86

<210>  1852
<211>  84
<212>  RNA
<213>  Homo sapiens

<400>  1852
agucagugua uuuggacggu aagguuaagc aaggugcguc guaucuugcu uaaccuugcc      60

cucgaaauac acugacugga uuuu                                              84


<210>  1853
<211>  66
<212>  RNA
<213>  Homo sapiens

<400>  1853
agcuuuuggc ugguccgaug guaguggguu aucagaacuc auuaacauua gugucacuga      60

aguuga                                                                  66


<210>  1854
<211>  66
<212>  RNA
<213>  Homo sapiens

<400>  1854
aguuacuuuu caguuugccc aaauugagcc caucucaaca uuuggcaguc cuuaccaagc      60

aacuac                                                                  66


<210>  1855
<211>  71
<212>  RNA
<213>  Homo sapiens

<400>  1855
aguucaaaau caauggagug guuuuucauu ccuuuuaaug uaggaucaag uucauuuuga      60

uuugguacuc a                                                            71


<210>  1856
<211>  73
<212>  RNA
<213>  Homo sapiens

<400>  1856
uuccacaggc cuggccucca guugggacuc cuccaucauc agagucugag ccugugagaa      60

ccagcaggga aca                                                          73


<210>  1857
<211>  65
<212>  RNA
<213>  Homo sapiens

<400>  1857
gccacguagu ccucccacga ggcauacuca gucagugugu ccgugcggua ggggauccag      60

ggcau                                                                   65


<210>  1858
<211>  70
<212>  RNA
<213>  Homo sapiens

<400>  1858
gcgaagacca gaucauccuc cugaaggggu gcugcaugga gaucaugucc cugcgggcgg      60

cuguccgcua                                                              70


<210>  1859
<211>  99

<212>  RNA
<213>  Homo sapiens

<400>  1859
gccugugugc aagguccagg aucaguugcu cuuccuuugc agguacugac agacccagaa      60

gcagcuaaau auguucaugg cauugcugua cauugguac                            99


<210>  1860
<211>  59
<212>  RNA
<213>  Homo sapiens

<400>  1860
uauuguuuug cuagguguuc auccuauaag aagcaggauu guauaggcag aaaaaugau      59


<210>  1861
<211>  86
<212>  RNA
<213>  Homo sapiens

<400>  1861
cuucguucuu uuuuacaagg cugaguaaua uuucauuggu uguauauaac auauuuugcu      60

uucuguuuac aaauauuuau ggagug                                          86


<210>  1862
<211>  113
<212>  RNA
<213>  Homo sapiens

<400>  1862
aagugaggag ggcagccagc agcuuccccu ucucugcccu gcuccaggca ccaggcucuu      60

uccccuucag ugucucagag gaggggacgg cagcaccaug gacccccgcu ugu           113


<210>  1863
<211>  115
<212>  RNA
<213>  Homo sapiens

<400>  1863
ugcuacauua ccgcugccau guaagaaaga cagucagcaa agccugggag agcuccagcu      60

ccucccuccc ugcucugcuc aacuucacuc uccuccucgg uucccuugga guacc          115


<210>  1864
<211>  119
<212>  RNA
<213>  Homo sapiens

<400>  1864
uggggcagga aguaggggug ggguuggagc ugcagcagaa gugggcaggg ccagauggca      60

aggaaacaaa ugguccacug aggcuggggc caggagccuu cuuccuugaa caauccagc      119


<210>  1865
<211>  100
<212>  RNA
<213>  Homo sapiens

<400>  1865
gguggcagau gccuguaauc ccagcugcuu ggaagucuga ggaagggaga aucacuugaa      60

cccaggaggc ggggguugca gugagccaua gcgccacugc                            100


<210>  1866
<211>  54

<212>  RNA
<213>  Homo sapiens

<400>  1866
uuacccgagu gugguggugg gcgccugugg ucccagcugc uuggaagucu gagg          54


<210>  1867
<211>  75
<212>  RNA
<213>  Homo sapiens

<400>  1867
agucaaggug augugaugug gcugagcagg gaggccaucu uccuuccaga cagagacucu    60

gucacuuugg acugg                                                     75


<210>  1868
<211>  82
<212>  RNA
<213>  Homo sapiens

<400>  1868
ggcucgcucc uccggcggcg gcggcggcgg cgacucugca ggcggaguuu cgcggcggcg    60

gcaccagggu uacgccagcc cc                                             82


<210>  1869
<211>  52
<212>  RNA
<213>  Homo sapiens

<400>  1869
gaucguucug ucauaaagac acaugcacac guguucauug cagcacuguu ca            52


<210>  1870
<211>  52
<212>  RNA
<213>  Homo sapiens

<400>  1870
uucuuccagc gauuuugaaa uaccuuuauu uauuuguuuu ugagacaggg uc            52


<210>  1871
<211>  85
<212>  RNA
<213>  Homo sapiens

<400>  1871
ggcaugcagg cuuugccugg cugcuaucuc uagaggcaag ucugccacga gagggcacug    60

caggcgaagc aguugugcuu gcuca                                          85


<210>  1872
<211>  67
<212>  RNA
<213>  Homo sapiens

<400>  1872
gucugucugc cuccugacac uguccaugcc gcccaggcug cuagccccca ggggcaccug    60

caggcca                                                              67


<210>  1873
<211>  54
<212>  RNA
<213>  Homo sapiens

<400>  1873

aaccggucac uggugggucc uggcacuggc gggucgcaac gcugugggcg uucc 54

<210> 1874
<211> 83
<212> RNA
<213> Homo sapiens

<400> 1874
ggccugguga agucugcuac ucuguccuua cugcugaaca uccugcuugu aucaggaaac 60

ucagaagcag uuugccuugu caa 83

<210> 1875
<211> 94
<212> RNA
<213> Homo sapiens

<400> 1875
   ggcggcucgg aagagcgcuc cggccaccgg cggugucaag aagccccauc gcuaucggcc 60
   ugguacagug gcucuccgcg agauucgccg cuac 94

<210> 1876
<211> 94
<212> RNA
<213> Homo sapiens

<400> 1876
gggcagccag gagucgggcg agccucgggg gcugcagaau ggggucgcgg ccgcgaugcc 60

ccugacccuc gccggcccca cccaggccgc cccc 94

<210> 1877
<211> 62
<212> RNA
<213> Homo sapiens

<400> 1877
gucuccaggg gcagucugcc acccauccuu ccugacugcc uggcccuguc cccugcagac 60

cg 62

<210> 1878
<211> 110
<212> RNA
<213> Homo sapiens

<400> 1878
ggccagcucc gggaugaccc gccgggaccc gcucgcaaau aagguggccc ugguaacggc 60

cuccaccgac gggugagugc uccggccgga guuucugagg cccuggcugc 110

<210> 1879
<211> 111
<212> RNA
<213> Homo sapiens

<400> 1879
uguguccugu uccgcuucuc cgaggacgcc uucaacucca cuuuuaucuc caccauaggu 60

aacgggaccg gggaauggcu gggggcgccg gaggcccggg cugggcgcgc c 111

<210> 1880
<211> 85
<212> RNA
<213> Homo sapiens

<400> 1880

ugaagggaac cccacuucgg agccuggagc acagggcgag cucuccuccg cccugcagcc 60

ucagguaagg auccggaccu ucacu 85

<210> 1881
<211> 89
<212> RNA
<213> Homo sapiens

<400> 1881
ggacgcugug gcgagaagcc gggcuuagcu ggaaggaauu ucuaccugaa ggccaggaca 60

uuggugcauu cgucgcugaa cagguuugg 89

<210> 1882
<211> 61
<212> RNA
<213> Homo sapiens

<400> 1882
ugugguuaaa gacugaauuu guaaacccau ucagauaaau ggcaguacuu uaggacacac 60

a 61

<210> 1883
<211> 144
<212> RNA
<213> Homo sapiens

<400> 1883
cuuuuaaccc gcaacuacug aaaauaagag ucagcaugua gcagcauuaa ugaccccuga 60

auuugaaaug auuucuguuu auuuauaccu aauuguaguu gcugccuagc cuaauaaauc 120

uguaguuuuc aaaaguuaaa agaa 144

<210> 1884
<211> 73
<212> RNA
<213> Homo sapiens

<400> 1884
cgcgaggguc gggggugggg guccuaggug gggacagaca auaaauaccg aggaaugucg 60

gggucucagu gca 73

<210> 1885
<211> 57
<212> RNA
<213> Homo sapiens

<400> 1885
auaaacuaau agguuuucag aauuaugaau uuuucagaac ucauccaguc cuuguaa 57

<210> 1886
<211> 82
<212> RNA
<213> Homo sapiens

<400> 1886
cucuucugcc aucccuuguc ucccugaggc caucaucaaa caggacagag uuggaagagg 60

agacugggag gcagcaagag gg 82

<210> 1887
<211> 136
<212> RNA
<213> Homo sapiens

<400> 1887
ggcaggacga cuguucagca cgcagaccgc cgaggacaag gaggaacccc ugcacucgau 60

uaucagcagc acagagagcg ugcaggguag ccuuggcucg ugcuuugguc ugggagggcu 120

uuuugguuca cugcug 136


<210> 1888
<211> 77
<212> RNA
<213> Homo sapiens

<400> 1888
ggugacgggg ggagagcgag gguuccgccg gccaccgcgg ugguggccga gcgcggcucg 60

ucgccuacug uggccgg 77


<210> 1889
<211> 83
<212> RNA
<213> Homo sapiens

<400> 1889
gcgaugcgca cggcucccag gcaggcaggc gcgcucgggc gagguaggag cgauguggcc 60

ugggaacgcc uggcgcgccg cac 83


<210> 1890
<211> 82
<212> RNA
<213> Homo sapiens

<400> 1890
uccgcuacgu auucccguau ucccgggugc cuggcaacag ugaugugcuu uaggccuuuu 60

uuagaaugca uacaaacgga ac 82


<210> 1891
<211> 58
<212> RNA
<213> Homo sapiens

<400> 1891
gcggcccucg gugccuccuu ccugaccucg cacccagcuc ggagcccgga gcgugccu 58


<210> 1892
<211> 58
<212> RNA
<213> Homo sapiens

<400> 1892
uguuuccuuu aaggagggug caaaguuguu cuaacaguuu ccguuacaag gguacaaa 58


<210> 1893
<211> 58
<212> RNA
<213> Homo sapiens

<400> 1893
uggggaaagc cgagagccac aggccuucuu cugaggcaug uggcuuuggu gacucagg 58


<210> 1894
<211> 100
<212> RNA
<213> Homo sapiens

<400> 1894

uucucuguag aguuuccucu agagagauuu ugaccauaau aaccagcugg cccucagcuu 60

cccauuucca ucuccuagug cacuaaccua cugaugaaau 100

<210> 1895
<211> 95
<212> RNA
<213> Homo sapiens

<400> 1895
cacuugugcc aggccuugug cugggcaccg uaggggcuau ggaaggagua agacacaguc 60

augcucucaa ggaacucgcu guccagcaga gugau 95

<210> 1896
<211> 115
<212> RNA
<213> Homo sapiens

<400> 1896
guuuccccgc augguuccgg aagagcgcgg cgcagcuggc ugugagcgca gggcuauccc 60

ggcggccgcu ucggcagcca gggcggcgcg gaggggcagg gccagaggga agcgc 115

<210> 1897
<211> 61
<212> RNA
<213> Homo sapiens

<400> 1897
aggguuccgg uggugcccug gcggccaucg cuggaggccg cgguugcuau gggagaccug 60

g 61

<210> 1898
<211> 69
<212> RNA
<213> Homo sapiens

<400> 1898
cuuccuuauu uccuaaagua ccuggcaaag aauucugcaa cuuuguuuag uaccagcaau 60

uaucaagug 69

<210> 1899
<211> 105
<212> RNA
<213> Homo sapiens

<400> 1899
gacuggugaa ugugcaaugu uaguaauuag aaguuuuacc acauggaaaa uuugugguuu 60

uaaacuuucu aaaucauggu gacuucauug aaagccauua guugc 105

<210> 1900
<211> 67
<212> RNA
<213> Homo sapiens

<400> 1900
uuucgcugag cggcacggcu acaucaaggg caucgucaag gugcggaacg cuggccgggg 60

cgggagg 67

<210> 1901
<211> 76
<212> RNA
<213> Homo sapiens

<400> 1901
acucucuauu ggcucaaaca gguugaccuc aauccaaguu uacucuugau aucacucugu 60

uggcugaagg agguaa 76


<210> 1902
<211> 64
<212> RNA
<213> Homo sapiens

<400> 1902
uggugaagcc acagguguuc uguauuucag cuccgggagu ucaacaucug ugggcgcugc 60

cagg 64


<210> 1903
<211> 116
<212> RNA
<213> Homo sapiens

<400> 1903
aacagcuuaa ugucucaugu uggaaagagu aaaggaaaug ucacugucuu auccucuugg 60

uaggaguaug acuugguaua aucuuuccac agagcaacau cuauuugaga aguuaa 116


<210> 1904
<211> 121
<212> RNA
<213> Homo sapiens

<400> 1904
gccuucuuuu uauagaguug gaccagaaaa gcuuaaugca gcagagucau gcagugaagg 60

cagauucaug ggcuugcauc cuggcuucag gucuuacuag augugugaaa aaauuaggcc 120

c 121


<210> 1905
<211> 76
<212> RNA
<213> Homo sapiens

<400> 1905
agugaagggc ccgacgcaau aacuucugga uaaagccuaa guccucccuu uuguuaucgg 60

gagauuucuu cauuca 76


<210> 1906
<211> 70
<212> RNA
<213> Homo sapiens

<400> 1906
uucacggugc ggcugcuuca caucuucacg gucaacaaac agcuggggcc caagaucguc 60

aucgugagca 70


<210> 1907
<211> 93
<212> RNA
<213> Homo sapiens

<400> 1907
cuuuccagga ggcuggaugg cuuccagggu cacuccuugc ccuggguggc ucccaggcuc 60

cuggaacuuc ugccuccacc ucgcugggga gcc 93

<210> 1908
<211> 72
<212> RNA
<213> Homo sapiens

<400> 1908
uguaaccuuc ugugccgggg aggauauuug cauuuaagca gaaaguguuu caggcucugc 60

agaggugaca cu 72


<210> 1909
<211> 79
<212> RNA
<213> Homo sapiens

<400> 1909
auggaagauu ucgaucaguu aggguacaag cuaaauaguu auguucuugu uguuugaguu 60

ggauuaggug uuuuuaucg 79


<210> 1910
<211> 79
<212> RNA
<213> Homo sapiens

<400> 1910
uggcaagucc gacugacacu cgagggguuu cccuuuagcu gccacccuca aucccagaag 60

ugacagugau ucuguuacc 79


<210> 1911
<211> 67
<212> RNA
<213> Homo sapiens

<400> 1911
ggccccuuug acgucuugag ugggaaauca caagauacug cuccagauuu ucccauuuua 60

uggcuaa 67


<210> 1912
<211> 52
<212> RNA
<213> Homo sapiens

<400> 1912
agauaaaacu gugaaaauga ugacccacua ugcuuacagu uuacacauuu ac 52


<210> 1913
<211> 54
<212> RNA
<213> Homo sapiens

<400> 1913
uaagaaaaag uuugccugcu uuugagcuag aaaauaaacu uuaugaauuu uaua 54


<210> 1914
<211> 66
<212> RNA
<213> Homo sapiens

<400> 1914
uccccauucc uuggcagauu gggggcacug aggaauauaa gauuugccgg ggcaucaagg 60

agggcc 66


<210> 1915
<211> 100

<212>  RNA
<213>  Homo sapiens

<400>  1915
cgcugcucca gcuugacaca gcgguuuagu gcgguagaaa cgaccggggg acugguuggg      60

cucuugacca ucagacagcu gcgugugaga uggaagugcc                            100


<210>  1916
<211>  76
<212>  RNA
<213>  Homo sapiens

<400>  1916
gcccgcgcgc cgccccauau aaaggcgccg ccguccuuac ccccaggacu cggccccaug      60

gagacgccgc cggccg                                                       76


<210>  1917
<211>  73
<212>  RNA
<213>  Homo sapiens

<400>  1917
aaaccauuau caagugaucu aguugucuuc aaccauacaa aagaaaauug acaugauuuc      60

aggauagguu uag                                                          73


<210>  1918
<211>  141
<212>  RNA
<213>  Homo sapiens

<400>  1918
ucgcucuugc ugguguagac acggucagcu agacucucaa uggcaaucca cuugacuggc      60

aucuuggcga uacgucccug gcgguaguag uccccauugu agaucuucuu ggagagcccg     120

aaguccgcca cacacacgga c                                                141


<210>  1919
<211>  83
<212>  RNA
<213>  Homo sapiens

<400>  1919
gggucccgca gcgggacccg agccucggcg gcggcggcgg cggcggcagg ggcgaggguc      60

ggggccaccg cgcggcgacc ucg                                               83


<210>  1920
<211>  67
<212>  RNA
<213>  Homo sapiens

<400>  1920
aguauccccc gcgcgcccaa cucggagcgc ccugcugggc gggcccgagc cucggcggcg      60

gcgcuga                                                                 67


<210>  1921
<211>  117
<212>  RNA
<213>  Homo sapiens

<400>  1921
gccccgaccu gggggugggc gguggcgggc cggcggccgc ucuggcgcgg cugggaugca      60

ccuugagcgg cgccuuucgu ugcagcgcgc gggcggcccg agccucggcg gcggcgg        117

<210> 1922
<211> 127
<212> RNA
<213> Homo sapiens

<400> 1922
uccucauugg uuugugcuga aauaugagug caauuuccug uccccucagc aguguugguu 60

ucucuucuug acuugaugca ggcacagauu uaucaagcuc cucagucaac aaacacauca 120

ccggaag 127

<210> 1923
<211> 70
<212> RNA
<213> Homo sapiens

<400> 1923
cagccuggga cagaccugcg ugcugaucgu gaucuucaca gugcuccugc agucucucug 60

uguggcugua 70

<210> 1924
<211> 114
<212> RNA
<213> Homo sapiens

<400> 1924
gggucuauug uugucaagaa ggaaaaaaua aagagaaagg uuagauauga auauaaggcu 60

gauauuucua auuuuuuuuu aaaaggagac cucuguguga uaggaaaagc uuag 114

<210> 1925
<211> 63
<212> RNA
<213> Homo sapiens

<400> 1925
cgauugucau gacuccugug uugcagacag augcaaggag auggaagaag gguaaagguc 60

gaa 63

<210> 1926
<211> 67
<212> RNA
<213> Homo sapiens

<400> 1926
agugucuauu ucuuugcugc ggagaacaaa uuccuaaaua guuuuaguaa aggagcugag 60

aagcuag 67

<210> 1927
<211> 60
<212> RNA
<213> Homo sapiens

<400> 1927
uguagguuuc uaaugauaca aguggcagag auaccuuuau uuggaauugg uagcaauagg 60

<210> 1928
<211> 54
<212> RNA
<213> Homo sapiens

<400> 1928
acuggcuucc gcagccacag cagccgcccu gggcagcggc agcggccgca gugg 54

<210> 1929
<211> 65
<212> RNA
<213> Homo sapiens

<400> 1929
uucugauaua auccacuucu cuuuccuuau aaaguguuau uaugaaagag agaaaauuga 60

gaggc 65


<210> 1930
<211> 103
<212> RNA
<213> Homo sapiens

<400> 1930
cucuucauaa aaagcugcua uuuuauuugu ucauuugauu aacagcugag uauuauacuu 60

ucuaagcaaa ugaaagagag aaaauuuagg gguggcuugg gcu 103


<210> 1931
<211> 99
<212> RNA
<213> Homo sapiens

<400> 1931
uaaucccugg ugcaacaauu ugugaggugg ggcccaaugg gaaguauuua ggucaugaga 60

gcuccacugu caggaaugga uggauuaaug uggauuauc 99


<210> 1932
<211> 159
<212> RNA
<213> Homo sapiens

<400> 1932
aaugcaaugg uauuaagagg uauggccuau aggaggugau ugaguuauga gagcuccacu 60

gucaugaaug ggauuaggug cccuuauaaa aaggcaugac agagggaguu cauuccuuuu 120

acccucuguc ccuucugcca ugugaagaca uagcauucc 159


<210> 1933
<211> 119
<212> RNA
<213> Homo sapiens

<400> 1933
uaccuaacac ugcugaacug uauacuucaa aaugguuaag augguaaguu ucacguuagu 60

guauuuuacc acaauuuuaa aaauuggugc ugcuuuucau guauuuaaau auggguaaa 119


<210> 1934
<211> 83
<212> RNA
<213> Homo sapiens

<400> 1934
agccgaaguu gaaauguuug uguugcuaaa aucuucucuu agucagacuu cauugaacaa 60

gccaaacaau uaacuuuugu uug 83


<210> 1935
<211> 140
<212> RNA
<213> Homo sapiens

<400> 1935
ugcucuuuuu aaaaaucucc uuccuaugau cuagggcgaa aagcgaauga gaaggagcgg 60

caggcagccc ugcagguggc cgagggcuuc aucucgcgca ugcaguacgc cccgaacacu 120

caggugagga gaugcgcaca 140

<210> 1936
<211> 82
<212> RNA
<213> Homo sapiens

<400> 1936
gcuugcuaag acauagggaa auuugugguu aaaauuuccu gagggaaaug aucacugauu 60

ucaguuuugg auuuuauggu cu 82

<210> 1937
<211> 99
<212> RNA
<213> Homo sapiens

<400> 1937
ugacuucaua ccacauuguc agccaccccc caccccaccc ccaggcuagg cuggggauuu 60

cugagggggu gacagcauug cugaguacgg aaugucagc 99

<210> 1938
<211> 93
<212> RNA
<213> Homo sapiens

<400> 1938
auccaggcac aggccuuucc uugccuuccu gggccuuccu ggggcuucag gagcugggcc 60

guggaguggg ggagaggcug ucagccugga ucc 93

<210> 1939
<211> 54
<212> RNA
<213> Homo sapiens

<400> 1939
uuuaacuugu ggcuccuuuu gccucccaag cagaaugagg uaguagauua gagc 54

<210> 1940
<211> 79
<212> RNA
<213> Homo sapiens

<400> 1940
cuggcccauc uuccuauucc cacguguagc uagugucuag ugucagcuuu gcucagugug 60

guggaaacau uuugcagaa 79

<210> 1941
<211> 66
<212> RNA
<213> Homo sapiens

<400> 1941
gucauaaugc cgucuuuaua uucuuaauaa auagcacuuu gacaagaaca ggacuguaaa 60

ugauga 66

<210> 1942
<211> 55
<212> RNA

<213>  Homo sapiens

<400>  1942
guuaagagua ggccagucau cuuugucccu caguggcggc uuuuccacca ggcug          55

<210>  1943
<211>  88
<212>  RNA
<213>  Homo sapiens

<400>  1943
acaucaaaau cuuuuguguc auuuaucaga uuaauuacuu uuuccauaaa aagagugagu     60

ugagaggaua acaagaaacu cccugucc                                         88

<210>  1944
<211>  100
<212>  RNA
<213>  Homo sapiens

<400>  1944
uucuuggaau caccaccuua aacaucaaaa ucuuuugggu cauuuaucag auuaauuacu     60

uuuuccauaa aaagagugag uugagaggau aacaagaaac                           100

<210>  1945
<211>  73
<212>  RNA
<213>  Homo sapiens

<400>  1945
uauuugaccc aagauuucuu uaaugaagua accauuccag uguaaaugua aaagauguau     60

cuucucaaau aug                                                         73

<210>  1946
<211>  69
<212>  RNA
<213>  Homo sapiens

<400>  1946
gguaggaaaa ucuugggauu ccuggaguga cagcgaagaa gaauuuuuug aaugccuaag     60

ugauacuga                                                              69

<210>  1947
<211>  129
<212>  RNA
<213>  Homo sapiens

<400>  1947
uguugauaaa agauuguguu ugccauucuc auuuauaaug caguuucucc uuaagccugg     60

aguuuuuuga augagugcau gaguaaauga gagaaugugu gaacgaacau uuaugaagua     120

ucuaacaug                                                              129

<210>  1948
<211>  79
<212>  RNA
<213>  Homo sapiens

<400>  1948
aucuggacac ucugaauguu uuuguuauca guguuuauua auuuuacaga acagaggaca     60

acgcaacaag gccaggugu                                                   79

<210>  1949

<211>  71
<212>  RNA
<213>  Homo sapiens

<400>  1949
ggagccuggc cucuguuuuu gucucugugu guugugugua cacacugaac guggacaucg      60

gauaguucuu u                                                          71


<210>  1950
<211>  119
<212>  RNA
<213>  Homo sapiens

<400>  1950
cucacggacu ucucgcccaa cucccugagc gcucccucgu uucgaucuuu agaaaacccc      60

gcuuucuuuc uggggccgug acgaggggca gggagcggcg agcaaggaug cguugagga      119


<210>  1951
<211>  57
<212>  RNA
<213>  Homo sapiens

<400>  1951
uugaaguuaa ugucagcuug cccagguuca aacuggaaga gaguuacacu cucaacu         57


<210>  1952
<211>  96
<212>  RNA
<213>  Homo sapiens

<400>  1952
caaaaaaaaa aaaaaaaaga aucuuaaccu aauaaaaacu cugccuuaca ucuguguuuc      60

uuuugggcca gauuuuugug aucauauaug uuugag                                96


<210>  1953
<211>  172
<212>  RNA
<213>  Homo sapiens

<400>  1953
uuauuacaac uugcauguau uuugucuguu uacucauuau uucgucuguc ucaccguucc      60

aagguaagca ccgugaaaac ggacuguguc uuuuuaguuc uuuacugugu uucugccauc     120

aagcccagug uugacacaug guagguacuc aagacaugcu ggcugaauga au             172


<210>  1954
<211>  69
<212>  RNA
<213>  Homo sapiens

<400>  1954
gcaagccugc aggacagcca guguggaggg gcuaccggaa gcuucaugug ggguacacug      60

cguuuguau                                                              69


<210>  1955
<211>  90
<212>  RNA
<213>  Homo sapiens

<400>  1955
acagucucac aagcaaugau ucuggugagu uuagaaauga aucaggaugu gcuaagcuca      60

augaggacuc auuagccuug gauaauguga                                       90

<210> 1956
<211> 87
<212> RNA
<213> Homo sapiens

<400> 1956
ccagauaaua uaagcaagga aaacuguguu uucagaagcu cacugauugg cauaguuugu 60

gguguuuucu aucccaguuc uuuggcu 87


<210>. 1957
<211> 84
<212> RNA
<213> Homo sapiens

<400> 1957
uccgcauguc ccuucgcggg aaggcugugg ugcugauggg caagaacacc augaugcgca 60

aggccauccg agggcaccug gaaa 84


<210> 1958
<211> 84
<212> RNA
<213> Homo sapiens

<400> 1958
uccgcauguc ccuucgcggg aaggucgugg ugcugauggg caagaacacc augaugcgca 60

aggccauccg agggcaccug gaaa 84


<210> 1959
<211> 103
<212> RNA
<213> Homo sapiens

<400> 1959
ucucaacggc ucugggcgga ugacuugcag gguggcuucc cccgaaacac ugggguggag 60

gcugaggcuc ccagcaaggg ugagcccugg ugucggggag gaa 103


<210> 1960
<211> 64
<212> RNA
<213> Homo sapiens

<400> 1960
gccuccugga cccggcauac ccccagcuua cucaaguccc aggcgggagc gucuucguug 60

gccc 64


<210> 1961
<211> 82
<212> RNA
<213> Homo sapiens

<400> 1961
aucugggcga ucgcuuccug guccucgccu ccuccgcugu cucccuggag uucuugcaag 60

ucggccagga ugucucaggu ac 82


<210> 1962
<211> 66
<212> RNA
<213> Homo sapiens

<400> 1962
uggcugccau ggcuguacua cggguguguc caagaaggaa uaagucugua ggccuugucu 60

guuaau                                                              66


<210>  1963
<211>  76
<212>  RNA
<213>  Homo sapiens

<400>  1963
acuggcggga gugucuuuag cauguuagcg cauuauauaa uuaguguaua augagcagug      60

agacgacugg agguca                                                      76


<210>  1964
<211>  120
<212>  RNA
<213>  Homo sapiens

<400>  1964
aaguuauuuu aaugucagau uuuugaaugu uauuauauau uaccuguaug auggaaggau      60

uaccacugua cacaaaucua aucaauaaaa acguuagaac cuucugcuua gaguacuuuu     120


<210>  1965
<211>  107
<212>  RNA
<213>  Homo sapiens

<400>  1965
cauacagugu accuauuuaa uguguacagu gaagugguuu uuaguauauu cacagauaua      60

ugccaccauc auucucucac uuccuuauuu uagcugugcu gaaugaa                   107


<210>  1966
<211>  61
<212>  RNA
<213>  Homo sapiens

<400>  1966
gggcuaccca ggggcuuccu auccuggggc cuaccccggg caggcacccc caggggcuua      60

u                                                                      61


<210>  1967
<211>  102
<212>  RNA
<213>  Homo sapiens

<400>  1967
gcuguacccc gagcguggcc acgggcugca cgcagacaag ggcgcuggcg ucaaggugga      60

gucggagcug cugugcaccc gccugcugcu gggcggcggc uc                        102


<210>  1968
<211>  52
<212>  RNA
<213>  Homo sapiens

<400>  1968
gcaccagugu ggagggcggu cccacccacc uccuccacuu cucccuuugc au              52


<210>  1969
<211>  85
<212>  RNA
<213>  Homo sapiens

<400>  1969
uauucauugu cuaccugagc uagaauacaa guaguuggcg ucuucagaga cacuuguaug      60

cuagcucagg uagauauuga augaa                                          85

<210>  1970
<211>  88
<212>  RNA
<213>  Homo sapiens

<400>  1970
cauggacgag aaccuguucg ugugcuccga cugcgggaag gccuuccugg aagcccacga      60

gcuggagcag caccggguga uccaugag                                         88

<210>  1971
<211>  84
<212>  RNA
<213>  Homo sapiens

<400>  1971
gccgcgaguu ccccacgcac cgcucggugc uggccgccug cagccaguac uucaagaagc      60

uguucacguc gggcgccgug gugg                                             84

<210>  1972
<211>  62
<212>  RNA
<213>  Homo sapiens

<400>  1972
gagccaccag guuuguguaa uucgcuacua cggcagucac aggaacucag gcaccauuuc      60

uc                                                                     62

<210>  1973
<211>  90
<212>  RNA
<213>  Homo sapiens

<400>  1973
ggcgcacuac ggggacgcug ggccgggcac cgggcugugu ggagaaguga gcgcgcucgu      60

cugacccccg ugcccgccac cccgagccac                                        90

<210>  1974
<211>  134
<212>  RNA
<213>  Homo sapiens

<400>  1974
agaagucccu cuaccuggca augggcccuc caccgagaac uccuggugcu gagauggacg      60

gaucaugauu uccuucuguc ugcaccaggc uuugcuuagg agagaaaaug ccagaaacaa     120

aaaggcuuuu cuca                                                       134

<210>  1975
<211>  104
<212>  RNA
<213>  Homo sapiens

<400>  1975
cgcuguggcc acgcugguuc cucaauugcu ggggcgugca gugucuguaa gggaggcuac      60

ugaugccauc cgaggaagau guaagguuuc gugugggcag cgag                      104

<210>  1976
<211>  101
<212>  RNA
<213>  Homo sapiens

<400> 1976
uagcucacug cagucuugaa cuugcuggcu caagugaucc ucccacuuca gccccccaag 60

gagcuaggac uacaggcaca caccaccaca accuggcuaa u 101


<210> 1977
<211> 55
<212> RNA
<213> Homo sapiens

<400> 1977
uuuaauagga augccauuac uagagaauua cuuuccugag caaaucugau gaaua 55


<210> 1978
<211> 56
<212> RNA
<213> Homo sapiens

<400> 1978
uuaaguuguu gcuuggaagu gagaaguugc uuagaaacuu uccaaagugc uuagaa 56


<210> 1979
<211> 56
<212> RNA
<213> Homo sapiens

<400> 1979
aguucccagu ucccaccugc ugagaguuca gaacucagac cguaggugug ggcuua 56


<210> 1980
<211> 63
<212> RNA
<213> Homo sapiens

<400> 1980
guuucagaca gggucugaag gacuggauug gccaaacauc agaccugucu uccaaggaga 60

cca 63


<210> 1981
<211> 94
<212> RNA
<213> Homo sapiens

<400> 1981
gucguggugc acaccuguag ucccagcuac uggggaagcu gaggugggag gauuauuuga 60

guccaggaga uggaggcugc agugagccug auca 94


<210> 1982
<211> 68
<212> RNA
<213> Homo sapiens

<400> 1982
accgugacag caauggaugu ggucuacgcg cugaagcgac agggacgcac ucuuuacggc 60

uucggugg 68


<210> 1983
<211> 58
<212> RNA
<213> Homo sapiens

<400> 1983
ccucccaggu ucaagcgauu cucaugccuc agccccagag uagcugggau uacaggca 58

<210> 1984
<211> 73
<212> RNA
<213> Homo sapiens

<400> 1984
ugugaucugc agcuggaccu ccuggguuca agugguccuc ccuccucagc cccagaguag 60

cugggauuac agu 73


<210> 1985
<211> 123
<212> RNA
<213> Homo sapiens

<400> 1985
gugcaugcac acacacacac acacacacaa ucuugcacac aaccucagag guaggucuau 60

caauuaacag auucugguua agagccccug ccuuagagcu cugguagguu cucagcagca 120

ucu 123


<210> 1986
<211> 54
<212> RNA
<213> Homo sapiens

<400> 1986
uuggagcgga agcgcacgcc ugcuaggauc agcgguggug guuccgcgau ggua 54


<210> 1987
<211> 97
<212> RNA
<213> Homo sapiens

<400> 1987
gccuggagug gauguaggaa cagccugcug gcucagaagc uuaaaaaaag cucugugugu 60

uuucaguuuu ucucuucuua guucucaaug aagguuu 97


<210> 1988
<211> 73
<212> RNA
<213> Homo sapiens

<400> 1988
cccacccaga guaacacaca uuuaugacuu uuucuccagg aguuagcugu gccagugguu 60

caugacgggg gcg 73


<210> 1989
<211> 60
<212> RNA
<213> Homo sapiens

<400> 1989
gugaaccaug ccccagugag cccccggcca ggccuggggc ugccuccugg ggccccaccc 60


<210> 1990
<211> 81
<212> RNA
<213> Homo sapiens

<400> 1990
   gcugucauua guaaugggga acauuaagau acucugugag gugcaagacu ucaggguggg 60
   gugggcaugg ggugggggua u 81

<210> 1991
<211> 79
<212> RNA
<213> Homo sapiens

<400> 1991
guccucgguc gguacagucu cugcaccucg cgccccagca ggugagcggc gaccgguaac 60

uggcgagugg uggggauag 79


<210> 1992
<211> 76
<212> RNA
<213> Homo sapiens

<400> 1992
ucugggagaa cuuuaucuua accuggagau caagggaaca aauggacagg uuacaagagc 60

agauauucuu cagguu 76


<210> 1993
<211> 57
<212> RNA
<213> Homo sapiens

<400> 1993
uuuagugaca uucaucugcu ggcuaugaug uacaguggag aaauguguua cugggga 57


<210> 1994
<211> 83
<212> RNA
<213> Homo sapiens

<400> 1994
gaagcuagaa gaaagaggau uacuugaggc caggaguuca aggccagccu ggccaacaua 60

gugagccccu gucucuguuu uau 83


<210> 1995
<211> 68
<212> RNA
<213> Homo sapiens

<400> 1995
gcuucaacag ugcuuggacg gaacccggcg cucgucccgc acccgggccg gccguccaua 60

gccagccc 68


<210> 1996
<211> 71
<212> RNA
<213> Homo sapiens

<400> 1996
gacguucuuc gccgagaguc gucgggguuu ccugcuucaa cagugcuugg acggaacccg 60

gcgcucguuc c 71


<210> 1997
<211> 71
<212> RNA
<213> Homo sapiens

<400> 1997
gacguuccuc gccgggaguc gucgggguuu ccugcuucaa cagugcuugg acggaacccg 60

gcguucgucc c 71

<210> 1998
<211> 65
<212> RNA
<213> Homo sapiens

<400> 1998
ggccucccca gaagcugagc agaugccagu gccaugcuua uacggccugc agaaucguga 60

gccaa 65


<210> 1999
<211> 84
<212> RNA
<213> Homo sapiens

<400> 1999
aguaucccau uuuacuuuuu acuucuauga gaucaacuuu uuuuagcuau cacauaugag 60

ugagaacaug ugguguuuaa cuuu 84


<210> 2000
<211> 132
<212> RNA
<213> Homo sapiens

<400> 2000
agcugacaga ugaagaaaga aaccuccuau cuguugcaua ugagaaugug auuggagcua 60

gaagaaccuc cuggagaaua aucagcagca uugaacagaa agaagaaaac aagggaggag 120

aaaacaagcu ag 132


<210> 2001
<211> 86
<212> RNA
<213> Homo sapiens

<400> 2001
agcaugagug cuucugcuga uguguuggcc cuggcgaaaa ucgaaaucaa guuauccgau 60

auuccagaag gcaagaacau ggcuuu 86


<210> 2002
<211> 64
<212> RNA
<213> Homo sapiens

<400> 2002
uuuggguuuu uucuuagcuc acgugucaga cggcacugca uguccugcag uuaacacuca 60

agga 64


<210> 2003
<211> 79
<212> RNA
<213> Homo sapiens

<400> 2003
auuuucaaga cuaugauaca acuccauuuc aagcaaaaac ccaaugaaca gcuuaugugu 60

uagccauagc ugaaaauaa 79


<210> 2004
<211> 79
<212> RNA
<213> Homo sapiens

<400> 2004

gggauuagag gcgugagccg ccgcgccggg ccuccccccu uccccuccgc cgccgccuuc 60

ucaucgcccu uccuccuuc 79

<210> 2005
<211> 80
<212> RNA
<213> Homo sapiens

<400> 2005
aggagcucuu guaaggcagg ccugguggug auaaaaucuc uuagcauuug cuugucugua 60

acggauuuua uuucuccuuu 80

<210> 2006
<211> 80
<212> RNA
<213> Homo sapiens

<400> 2006
aggagcucuu guaaggcagg ccugguggug augaaaucuc ucagcauuug cuugucugua 60

acggauuuua uuucuccuuc 80

<210> 2007
<211> 80
<212> RNA
<213> Homo sapiens

<400> 2007
aggagcucuu uuaaggcagg ccugguggug acaaaauccc ucagcauuug cuugucugua 60

acggauuuua uuucuccuuc 80

<210> 2008
<211> 154
<212> RNA
<213> Homo sapiens

<400> 2008
ccgguuguuc uuuuccaugu uuagugcuuc cuucaggagc ucuuguaagg caggccuggu 60

ugugacaaaa ucugucacca uuugcuuguc uguaacggau uuuauuucuc cuucacuuau 120

gaagcuuugu uuggcuggau augaaauucu gggu 154

<210> 2009
<211> 80
<212> RNA
<213> Homo sapiens

<400> 2009
aggagcucuu uuagggcagg ccugguggug acaaaaucuc ucagcauuug cuugucugua 60

acggauuuua uuucuccuuc 80

<210> 2010
<211> 91
<212> RNA
<213> Homo sapiens

<400> 2010
uacacuguug ccucuuuuaa aagcaaagcc acuuucuugu ucuaagauua caucaagacu 60

ugagcagugu guaguaauua aaaugugugc u 91

<210> 2011
<211> 74

<212>  RNA
<213>  Homo sapiens

<400>  2011
cagaacaucu ucgccuggug ugguggaaag uccaacaucc uggaacgcaa caaggcgagg      60

gaccuggccc uggc                                                        74


<210>  2012
<211>  81
<212>  RNA
<213>  Homo sapiens

<400>  2012
uccaggcaug augccaggug caccaacuua cuuaauccuc auagccacca ccugagcaag      60

cuccuguuuu auaaauggac c                                                81


<210>  2013
<211>  77
<212>  RNA
<213>  Homo sapiens

<400>  2013
cuggugcccc ugcucacugg gcccgagccu gaacugagcc ccagcgggcu ggcgggccuu      60

uuuccugcgu cccagcc                                                     77


<210>  2014
<211>  103
<212>  RNA
<213>  Homo sapiens

<400>  2014
agauuuaaaa uugagcaggu uucuuuccag caguagucag ccauccggac agaaccguuc      60

cugugauggu guuacacugc ugggagagga augucuuguc uuc                       103


<210>  2015
<211>  113
<212>  RNA
<213>  Homo sapiens

<400>  2015
gcugggaggc ccagaggcug ggcugggcga guaucucuuc gaaaggcuca cucucaagca      60

cgucuaagag ccuuaugagc ccagcgacuu cugaagggcc ccuugcaaag uaa            113


<210>  2016
<211>  75
<212>  RNA
<213>  Homo sapiens

<400>  2016
cugggcucaa guguccugcc uuccucggcc ucccaaagug cugggauuua caggcaugag      60

cugcuguguc uggcc                                                       75


<210>  2017
<211>  77
<212>  RNA
<213>  Homo sapiens

<400>  2017
uccuggccuc aagugauccu cuugucuugg ccucccaaag ugcugggauu acaggcauga      60

gcugcugugu cuggaca                                                     77

<210>  2018
<211>  130
<212>  RNA
<213>  Homo sapiens

<400>  2018
gccucuuaaa uuacugagau uacaggcaug agcugcugug ucuggcccua uuccuguauu      60

cugacuccau aucucccuuc caggagguuc agcuucugug uguccaugcc uuuucaguuu     120

cuccaggccu                                                            130


<210>  2019
<211>  91
<212>  RNA
<213>  Homo sapiens

<400>  2019
cgccgccgcc gccgccgccg ccucggcgcu ugcagaaccc agaagugaac agcaggcgac      60

ccggaagguu ugcgcgcggc uccgcagcga g                                     91


<210>  2020
<211>  64
<212>  RNA
<213>  Homo sapiens

<400>  2020
gcaugauaca ugugguuuag guucugauga uaaagcagug ggcaggacac acacguucuu      60

guuc                                                                   64


<210>  2021
<211>  75
<212>  RNA
<213>  Homo sapiens

<400>  2021
ggccgagacc cagggcacca ccgaggcccg agaccugcau ugucuccugg ucacggugag      60

ucgggccucu gcccu                                                       75


<210>  2022
<211>  80
<212>  RNA
<213>  Homo sapiens

<400>  2022
ugcugcugcu gcccgagcac gugcugguca agaucuucag cuuccugccc acgcgcgcgc      60

uggccgcccu caagugcacc                                                  80


<210>  2023
<211>  135
<212>  RNA
<213>  Homo sapiens

<400>  2023
ucaggaggcu gaggcaggag aaucacuuga acccgggagg cagagguauu ggugagccaa      60

gaucgugcca uugcacucca gccugggcaa caagcaugaa acuccgucua aaaauguaua     120

caaaguaaac ugaca                                                      135


<210>  2024
<211>  94
<212>  RNA
<213>  Homo sapiens

<400>  2024
ggcguggugg cgccugccug uaaucccagc uacucagggg aggaacugcu ugaacccgug      60

aggcagaagu uguagugggc cgagaucgcg ccac                                  94


<210>  2025
<211>  57
<212>  RNA
<213>  Homo sapiens

<400>  2025
accccaucau gucgcggggc uccaucgaga uuccccuccg ggacacugac gagguaa         57


<210>  2026
<211>  69
<212>  RNA
<213>  Homo sapiens

<400>  2026
ggccaccaug acccugcagg gcuuggccaa ccgcugcaug uuccuaguag cccugaggcu      60

gcuggccaa                                                              69


<210>  2027
<211>  88
<212>  RNA
<213>  Homo sapiens

<400>  2027
uguccgcuac cccguaguug cccugucuug ugggcgccug caaaagcgca gagauuuccg      60

ugagaccaac gcagccaugu cuaauaaa                                         88


<210>  2028
<211>  77
<212>  RNA
<213>  Homo sapiens

<400>  2028
gccccucgga acaacuguau ugcuguggag ccucuugagg aguucuccgg gcguggaacg      60

ggucuguuuc cgggcuc                                                     77


<210>  2029
<211>  68
<212>  RNA
<213>  Homo sapiens

<400>  2029
ugggggugag cacucugguc uggggcuuga ggaugccuuc aacaccucuc uugguugaag      60

cucucagu                                                               68


<210>  2030
<211>  99
<212>  RNA
<213>  Homo sapiens

<400>  2030
gccgcgucuu cccggaggcg acugcuggaa gcugcaggga acagcugcgg ugcucugcca      60

uauugugucu uccccggccg gucccucgcc ucccggccg                             99


<210>  2031
<211>  119
<212>  RNA
<213>  Homo sapiens

<400>  2031
ggcccguggg agggaggcag accccugggc uuuccugcug gccacggaga cucugcuccu      60

gcauggaaag ggagccuggg agccagcagc ccacgccugg ggagccugcc uggggccau      119


<210>  2032
<211>  101
<212>  RNA
<213>  Homo sapiens

<400>  2032
gggggaggga gcgcgggcuc gaggccggcg ccggcacgaa gcgcgcgucc cgccaacccc      60

cggcacccgc cggccucgcu ucccgcgccu cgcugcuccg g                         101


<210>  2033
<211>  86
<212>  RNA
<213>  Homo sapiens

<400>  2033
ugcaagcucc ucauguaggg ccauauugaa ggaggugagc cauucaccug uacccaguga      60

cggcgccucu gugggcagac ugcauc                                           86


<210>  2034
<211>  98
<212>  RNA
<213>  Homo sapiens

<400>  2034
ggcaugguga cacauaccua cagucccagc uacucaggag ggugaggcag ggggaucacu      60

ugagcccggg aguugggggc uagagugaac cauguucu                               98


<210>  2035
<211>  125
<212>  RNA
<213>  Homo sapiens

<400>  2035
gcccugaucu cuaugguuuc cgcggcucgc uagaaggagu gugguccacc ggaggacucc      60

cuacauucuu ccuccauuac cucacugccu ccgguggcug cugggauacc ggaggacucg     120

ggucc                                                                 125


<210>  2036
<211>  91
<212>  RNA
<213>  Homo sapiens

<400>  2036
uuccgcggcu cgcuagaagg agugcggucc acuggagguc ucccuacauu cuuccuccau      60

uaucucacug ccuccggugg cugcugggau a                                     91


<210>  2037
<211>  91
<212>  RNA
<213>  Homo sapiens

<400>  2037
uuccgcggcu cgcuagaagg aguguggucc acuggaggac ucccuacauu cuuccuccau      60

uaccucacug ccuccggugg cugcugggau a                                     91


<210>  2038

<211>  110
<212>  RNA
<213>  Homo sapiens

<400>  2038
gagugaaaac cacuccuuag gcaaugauua agcugagagu cagagggaug ggugggaggc      60

cagcuguccc aucugagcug cacuugauca cuagggaauu uaucauuucu                110


<210>  2039
<211>  78
<212>  RNA
<213>  Homo sapiens

<400>  2039
aagccgugag ucaggccugg guuggccacc ugggucacug gaggaccaac cccugcuguc      60

caaaacacca cugcuucc                                                    78


<210>  2040
<211>  105
<212>  RNA
<213>  Homo sapiens

<400>  2040
cgagucacgg gcuggcuacg aacuccucgg gggcgaaggu ggcggagagg gauggguucc      60

aggacguccu ggcgcccggg gaaggcucgg cgggacggau uugcg                     105


<210>  2041
<211>  123
<212>  RNA
<213>  Homo sapiens

<400>  2041
cugggucagg aauaccccau gucuucuccc caccuguagc accaguuuuu gacccauucc      60

cuagcaggga ggagcuguac cugggacgca ccuggggaca ggcaccuagg uucaugccua     120

guc                                                                   123


<210>  2042
<211>  61
<212>  RNA
<213>  Homo sapiens

<400>  2042
agcucaguua cgaaguaggc ugggccacau agaggacugg guccugcugg ggcaggguuc      60

c                                                                      61


<210>  2043
<211>  107
<212>  RNA
<213>  Homo sapiens

<400>  2043
cugcugacua caaacacagg cuggguuuug ggggugcuga cgcagcuuug uugugcugcu      60

gcuuccaguu cugccggcuc agugccuugu gaccggggcc gguggcu                   107


<210>  2044
<211>  121
<212>  RNA
<213>  Homo sapiens

<400>  2044
cuugcugucc acccagcagg cgcagguccu gugcagcagg ccaaccgaga agcgccugcg      60

ucucccauuu ucgggcuggc cugcugcucc ggaccugugc cugaucuuaa ugcugcagga 120

a 121

<210> 2045
<211> 59
<212> RNA
<213> Homo sapiens

<400> 2045
ucuuugcuca ugggcuggau gggaauuacc ccuccuccag ugcaauguca ucauagacu 59

<210> 2046
<211> 138
<212> RNA
<213> Homo sapiens

<400> 2046
ugucuuuuau ucucuuucca ucuuucuuug ugucuuuuug auauuuguau ugauaggcuu 60

ugacuuauuu uguuuugugu auuuauacaa auauuuuugu gguacauugg ggauuacaua 120

uaaccucuaa aagauaca 138

<210> 2047
<211> 105
<212> RNA
<213> Homo sapiens

<400> 2047
ucacugccuu augcugugcu gccucagaaa uguuaaguau caguugguag ucugauacca 60

acuggaaggg aauguuucug uucagaucuc ugcuugcuag ugggu 105

<210> 2048
<211> 128
<212> RNA
<213> Homo sapiens

<400> 2048
ggcucggcgc ccgcccgcuu gcucuccucg cucgcucgcu cgcuccgcgg ccccccaugc 60

ggaugugaca uuucacgccg ccgccauuuu gagagcgagc cgagccgagc ugccgggcgc 120

cgcguccg 128

<210> 2049
<211> 74
<212> RNA
<213> Homo sapiens

<400> 2049
agugaaggcu ggagccaggg agcgcuggag gaaaccagcc cuuuagccag cagccccucc 60

accacaggca cugc 74

<210> 2050
<211> 55
<212> RNA
<213> Homo sapiens

<400> 2050
ggaguaucug cauccugcgc ucugggaacu cccgugaggc aggagauccg uccac 55

<210> 2051
<211> 58
<212> RNA
<213> Homo sapiens

<400> 2051
gcuggcccag gcccauguaa gaaagaacac uuggauucug uggggccgug ggaggugg 58

<210> 2052
<211> 102
<212> RNA
<213> Homo sapiens

<400> 2052
auguacgcau uaccauuagg uuggggcaaa gguaauugcg guuuuuucca uugaaaguaa 60

uggcaaaaac cuuaauuacc uuugcaccaa gcuaauacau cu 102

<210> 2053
<211> 57
<212> RNA
<213> Homo sapiens

<400> 2053
ccagggccug caccggaucc auguggaugg gagcagcggg cggcugcagc uguggca 57

<210> 2054
<211> 56
<212> RNA
<213> Homo sapiens

<400> 2054
ccuggaagca cagccuuggg gacugagcag gcccucacug ucacuuuaag aaggga 56

<210> 2055
<211> 139
<212> RNA
<213> Homo sapiens

<400> 2055
agacuacaag ggcacugggu gcuugaucau gcaggguaua aguggcucaa ugauaacagc 60

auaugauggu ucauaugcuc auugcucuuu uguauuguau uagacuccug auuagugucu 120

cacugucugg guugucuuu 139

<210> 2056
<211> 107
<212> RNA
<213> Homo sapiens

<400> 2056
cggggagaag ccguacgugu gccccgagug cgggcgagcc uucagccaga acaugcaccu 60

gaccgagcac cagcgcacgc acaccgggga gaagccguac gccugca 107

<210> 2057
<211> 66
<212> RNA
<213> Homo sapiens

<400> 2057
ccggggccgc ggcggcuccu cugccggguc gcgccggacg acuggcuucg ggcggcggcc 60

ccggcg 66

<210> 2058
<211> 67
<212> RNA
<213> Homo sapiens

<400> 2058

agccgccgcu uccgcccggc aguggggcaa gcugaaguga gaagccgcug ggccugagag 60

gagcucu 67

<210> 2059
<211> 146
<212> RNA
<213> Homo sapiens

<400> 2059
agggcuucuu uaaauuaauu cggcugggug auuaauuaua accccuccga auugccugaa 60

uuucauaaaa gaaagaaaaa ugucuagcuu uuaugaaauu uaguaaauuc agguggguua 120

uuucuucaga uauuuuuugc aucuuu 146

<210> 2060
<211> 71
<212> RNA
<213> Homo sapiens

<400> 2060
gggcgggggc ggccgggauu cccgugcggg guuggggcuc guggcggaag cuccggaccu 60

cccuggcccg a 71

<210> 2061
<211> 53
<212> RNA
<213> Homo sapiens

<400> 2061
auuuccaggg cugagcagag cacuauguug cggcuuaaga cgcucggcga uuu 53

<210> 2062
<211> 108
<212> RNA
<213> Homo sapiens

<400> 2062
gguuuccggc cagugcuuau uuccggucuu ucggaugcug acgcucucuu ccugucuuug 60

uggcuccgga aaggcguuug ggaugccaac gguaaguccg gaggcuua 108

<210> 2063
<211> 105
<212> RNA
<213> Homo sapiens

<400> 2063
cggcugcaga gccgguaacg gugguggcgg cuguugggcc aaaggcgaaa gacgaagagg 60

aggaggaaga ggagccgcug ccaccgugcg aggcggugcg cuggg 105

<210> 2064
<211> 85
<212> RNA
<213> Homo sapiens

<400> 2064
ggggcccuca gccucagaug accugcccug ggcucuccag uuacagggag cagaagagcc 60

uuccuuuggg cuagaaaggu ucugc 85

<210> 2065
<211> 64
<212> RNA
<213> Homo sapiens

<400>  2065
ggguguaccu ggcugagucu cuguggccgc ggaggcgcgg agcuaagcgg cucggauuau      60

ccug                                                                    64


<210>  2066
<211>  57
<212>  RNA
<213>  Homo sapiens

<400>  2066
acccggcucc gagcggcggc ggcccggcuu ccgcugcccg ugagcuaagg acggucc         57


<210>  2067
<211>  81
<212>  RNA
<213>  Homo sapiens

<400>  2067
agugcuucau gcuguuguca acacuaacuu uacaggugac ccaggaaauu gugaaguagu      60

uggggccgua gaacagacuu c                                                 81


<210>  2068
<211>  136
<212>  RNA
<213>  Homo sapiens

<400>  2068
cuccgugccc agcuaauuuu uacauuuuua guacagaugg gguuucauca uguucggcag      60

gcuggccucg aacuccugag cuugagcgau cugcccaccu cagcuuccca aagugcucgg     120

auuacaggca ugagcc                                                      136


<210>  2069
<211>  157
<212>  RNA
<213>  Homo sapiens

<400>  2069
agucacuuua guggccaccu ugcuuaucag auugacuguc gcaauaucac agugcuugug      60

uucagauaac ccuuauucua cucaguaaug gccucaaagu acaaaaauag ugauguuggc     120

aauuuagaua ugcuaaagag aagccuuaaa cugcuuc                               157


<210>  2070
<211>  64
<212>  RNA
<213>  Homo sapiens

<400>  2070
cggaagcucc agggugggag uaguucccug cuaagggagg guagacuguc caaccuguuc      60

cggc                                                                    64


<210>  2071
<211>  118
<212>  RNA
<213>  Homo sapiens

<400>  2071
cccaggaagg aggcuccgcu gguuccgcug ggucaggcgc ugacgggacc gggcugcggc      60

aaucguuagc gggucauguc ggccgcccag ggcugggaca ggaaccgccg gaggggag       118

<210> 2072
<211> 68
<212> RNA
<213> Homo sapiens

<400> 2072
guguccggag gcgcuguucc agccuuccuu ccuggguaug gaaucuugcg gcauccacga 60

gaccacgu 68


<210> 2073
<211> 53
<212> RNA
<213> Homo sapiens

<400> 2073
cgagaaccca uuccucaaug gagaggucau ccggcuggau ggggccauuc gua 53


<210> 2074
<211> 66
<212> RNA
<213> Homo sapiens

<400> 2074
aucgcagaca gggacacaug gagaacgccc ccaccaguuc ccacgugcuu cucuuuccgc 60

agguac 66


<210> 2075
<211> 69
<212> RNA
<213> Homo sapiens

<400> 2075
gucucuacca aagcucaugu ggaauguuaa ugggaaaugu uaaaggcggg ggcucgugga 60

aggugauuu 69


<210> 2076
<211> 66
<212> RNA
<213> Homo sapiens

<400> 2076
gucucuacca aagcucaugu ggaauguuaa ugggaaaugu uaaagguggg ggcuggugga 60

agauga 66


<210> 2077
<211> 134
<212> RNA
<213> Homo sapiens

<400> 2077
ggcaucauug cauuuugcaa ugugagaaag acaugaacuu ugggggacca gggacagaau 60

aauauguuuu ggcucucugu cucuaccaaa gcucaugugg aauauuaaug ggaaauguua 120

aaaguggggg cugg 134


<210> 2078
<211> 168
<212> RNA
<213> Homo sapiens

<400> 2078
ugagagacuc uagggaaggc aucauugcau uuugcaaugu gagaaagaca ugaacuuugg 60

gggaccaggg acagaauaau auguuuuggc ucucugucuc uaccaaagcu cauguggaau 120

auuaauggga aauguuaaaa gugggggcug guagaaggug auuucauc 168

<210> 2079
<211> 54
<212> RNA
<213> Homo sapiens

<400> 2079
cucucuguca ggguacucag aggaaagguc agggaaccag ggcauguggg agaa 54

<210> 2080
<211> 111
<212> RNA
<213> Homo sapiens

<400> 2080
ggccccgggg ccuccagcgg caccagcgga gaccacggug agcucgucgu ccggauugcc 60

agucuggaag uggagaacca gagucugcgu ggcggugagg ccccgggcca g 111

<210> 2081
<211> 87
<212> RNA
<213> Homo sapiens

<400> 2081
agcugccguu ccagcgccug gugcgagaaa ucgcccaaga cuucaagacc gaucuucgcu 60

uccagagcuc ugcggugaug gcgcugc 87

<210> 2082
<211> 54
<212> RNA
<213> Homo sapiens

<400> 2082
gugagccuug aagguccaga agggaagcug aagggcccca aguuuaagau gccu 54

<210> 2083
<211> 54
<212> RNA
<213> Homo sapiens

<400> 2083
gugagccuug aagguccaga agggaagcug aagggcccca agcuuaagau gccu 54

<210> 2084
<211> 58
<212> RNA
<213> Homo sapiens

<400> 2084
ccagcuagug gacaucuccc aggacaacgc ccugagggau gagaugcgag ccuuggca 58

<210> 2085
<211> 64
<212> RNA
<213> Homo sapiens

<400> 2085
ggccgagugc aucgccugcu ccgcaaaggc aacuaugcgg agcgggucgg ugcuggagcg 60

ccgg 64

<210> 2086
<211> 59

<212>  RNA
<213>  Homo sapiens

<400>  2086
ucugccggcu gcgggucguu gggggggccc caugccaacg gccacggagg ccaaggacc      59


<210>  2087
<211>  68
<212>  RNA
<213>  Homo sapiens

<400>  2087
ccugcgagcg gcugcugagc cggguggccg cccuguuccc cgcgcugcgg ccuggcggcu      60

uccaggcg                                                              68


<210>  2088
<211>  72
<212>  RNA
<213>  Homo sapiens

<400>  2088
ccggcugcag caggaagggg agcggauccg cagugugcug gacggcccug ugcugagcaa      60

cauugaccgg au                                                         72


<210>  2089
<211>  97
<212>  RNA
<213>  Homo sapiens

<400>  2089
ugcucaagcc ggaagagugc ugggcagcuu gguccugcug cuugcuguca caagcagggc      60

ccugcugacc ugccuucugg acaacucugc uugcagc                              97


<210>  2090
<211>  111
<212>  RNA
<213>  Homo sapiens

<400>  2090
cccggcuucu ucgucacagg agaccgcgcc gguggccgga gcuggugccu gcggcgggug      60

gggaugagcg ccggguggcu gcugcuggaa gaugggugcg agguacgggg g             111


<210>  2091
<211>  92
<212>  RNA
<213>  Homo sapiens

<400>  2091
ggcuggccgg cugggucucc ucccgggcuc agugggccug gggccucucu gacguggugc      60

cugcuggagg gaggcucguc gucaccagcu ga                                   92


<210>  2092
<211>  68
<212>  RNA
<213>  Homo sapiens

<400>  2092
ggccuguucu gucucugagc ucuguguccu ccguucauuc cauggcuggg agucacugau      60

gcugccuc                                                              68


<210>  2093
<211>  88

<212>  RNA
<213>  Homo sapiens

<400>  2093
ccuucaagau gaagaugucc gucauccugg gcgucgugca cauggccuuu gggguggucc      60

ucggagucuu caaccacgug ugagggcc                                          88


<210>  2094
<211>  179
<212>  RNA
<213>  Homo sapiens

<400>  2094
aacuucugag cuuaagccaa caucuugcuu gucuccugag uagagggacu auaggcaugu      60

gccaccacac cuggcuuaaa ugugucauuu aaaaauucag gccaggcaca guggcucaug     120

ccuguaaucc cagcaauuug ggaggcugag gcaggcggau caccugaggu caggaguuc      179


<210>  2095
<211>  83
<212>  RNA
<213>  Homo sapiens

<400>  2095
gcgacuauag gcaugugcca ccacaccugg cuuaaaugug ucauuuaaaa auucaggcca      60

ggcacagugg cucaugccug uaa                                               83


<210>  2096
<211>  146
<212>  RNA
<213>  Homo sapiens

<400>  2096
auggcugcaa cagaaaaagc cauuguguac cacagugucu auuuagccaa uuguccaucu      60

uuagcuauuc ugaaugccua aagauagaca auuggcuaaa uagaaauugu gguacaucca     120

uacaauggaa uaccaugaag ccaucg                                           146


<210>  2097
<211>  112
<212>  RNA
<213>  Homo sapiens

<400>  2097
ggccgcuaaa gugugacgag cccauccuca gcaaccgauc uggagaccac cguggcaaau      60

ucaagguggc guccuuccgu aaaaaguaug agcucagggc cccgguggcu gg               112


<210>  2098
<211>  82
<212>  RNA
<213>  Homo sapiens

<400>  2098
ccagagaguc cuucgggcgc gugaccucgg gguugucggg cugauacggg acccccaccg      60

uggccgaggc cagaaccugg uu                                                82


<210>  2099
<211>  101
<212>  RNA
<213>  Homo sapiens

<400>  2099
gacagaguuu gaaacaguca ucugggggaa cucacauaga aaauagaaau cucagcuauu      60

cuaggcgagg uccugccuag aauacuguug aacagugucu c                    101

<210>  2100
<211>  87
<212>  RNA
<213>  Homo sapiens

<400>  2100
ugugcaggga agaaaugaga aauacugugc ccugaggcug gacaagggga auuucuccug    60

gggcucagag uguuguacuu gcacaac                                        87

<210>  2101
<211>  72
<212>  RNA
<213>  Homo sapiens

<400>  2101
aguaacaaga aauaccgugc ccugagguug gacguggggg aauuucuccu ggggcucaga    60

guguuguacu cg                                                        72

<210>  2102
<211>  59
<212>  RNA
<213>  Homo sapiens

<400>  2102
cucccaaagc aaccugucau caugaaggcc aaauucuuca gcagaagagc ugaggagaa    59

<210>  2103
<211>  122
<212>  RNA
<213>  Homo sapiens

<400>  2103
cacuagugaa gaagcagaug aggacccugu uuauuguuuc ucuccaagaa auucuccaag    60

aauauuguuu cuuggagaga aauaauaaau aaacaagaca auuucugaaa gcaauaagug   120

ca                                                                  122

<210>  2104
<211>  53
<212>  RNA
<213>  Homo sapiens

<400>  2104
guggagccag gaauuacauc uuauuuauuu ugaauuccua gcaaccuuca uug          53

<210>  2105
<211>  88
<212>  RNA
<213>  Homo sapiens

<400>  2105
cacagcuccc uagaggugaa guugccaggu cauagacuag aauaguuuca cuagaucuug    60

acauguugcu cuccaaaagg guuguguc                                       88

<210>  2106
<211>  128
<212>  RNA
<213>  Homo sapiens

<400>  2106
uaucccgugg uuggcagugu ccuguccauu cuucuugcac uggccugucc cucaggagac    60

aggugugagu gguccugagc uugggccagu guaagaguag gggacagguu ucucuccuug     120

uaggugucc                                                              128

<210>  2107
<211>  73
<212>  RNA
<213>  Homo sapiens

<400>  2107
uuugaugugu agacauugug auaagguaga uaauugugau aacuauuuau ugcuuguuua      60

aacugcucaa gua                                                          73

<210>  2108
<211>  129
<212>  RNA
<213>  Homo sapiens

<400>  2108
cggcuacgug guaaaggaga caauuggugu gggcuccuac ucugagugca agcgcugugu      60

ccacaaggcc accaacaugg aguaugcugu caaggugggc cuccugacca cgucucggcc     120

aaggcugcu                                                              129

<210>  2109
<211>  64
<212>  RNA
<213>  Homo sapiens

<400>  2109
gaccacuguc uccugguacu ggacuaucua ccugccuugu cccuguucug ggggaaggug      60

ucca                                                                    64

<210>  2110
<211>  92
<212>  RNA
<213>  Homo sapiens

<400>  2110
agaaugcagg aaagagaucg aagagagaga aaagaaaggu gacguggugc caugguuugg      60

ggggcuucuu ugcagacuca cucugcaucu ga                                     92

<210>  2111
<211>  92
<212>  RNA
<213>  Homo sapiens

<400>  2111
aguggcuaca gcacuggcuu uagaggccgc ccugagugca agucuuggcu uugccacuug      60

uuggccgugg aaccauucga gcaaguuacu ca                                     92

<210>  2112
<211>  106
<212>  RNA
<213>  Homo sapiens

<400>  2112
uucugaguaa auuauuuuuu auuggaggau ucuuuuuuuu uuaaauugga gaagcuguga      60

agguguuaaa acgagaaucu uaccaauuca caauacgaua agaaau                    106

<210>  2113

<211>  107
<212>  RNA
<213>  Homo sapiens

<400>  2113
ggccuggccg aggacaucga uaaaggcgag guguccgccc gucaggagcu caagcagcgg      60

gcgcgcuacc uggccgagaa guacgagugg gacguggcug aggcccg                   107


<210>  2114
<211>  92
<212>  RNA
<213>  Homo sapiens

<400>  2114
auuauuugau aaagcacagg ucuuucucag agcacacuca augaaagagu acuaagaguu      60

uacucuggga aaaccaaauc uaaaaauaau ua                                    92


<210>  2115
<211>  71
<212>  RNA
<213>  Homo sapiens

<400>  2115
gaaccguacu ugagcagguu cuuccaauau agcuaucuga gcuccccaag gacugaccag      60

ggaccuuucc a                                                           71


<210>  2116
<211>  87
<212>  RNA
<213>  Homo sapiens

<400>  2116
ucccaguaag gugaaacaca acucuguaua ccaagauaua uccaugguac cucgauauac      60

auauuugcag guuucuuuuu ugggagg                                          87


<210>  2117
<211>  59
<212>  RNA
<213>  Homo sapiens

<400>  2117
ugugacaagg acacguucgg uccccacuuu acaaguaagg gacacggccu cucagcaac       59


<210>  2118
<211>  72
<212>  RNA
<213>  Homo sapiens

<400>  2118
uuguuuagag gagauggcau uuaaaacagg acuugacacu uuaaguuccu aaagaaggca      60

uuccaaucag aa                                                          72


<210>  2119
<211>  121
<212>  RNA
<213>  Homo sapiens

<400>  2119
ucauagacug accuucuuuu ggaauuucug agucauuuau uuuccuuagc uuuuuccacu      60

caaauuaagg gcaagcgaaa aaguaauaau uuggcauucu uuaagccuac agaaugugau     120

u                                                                     121

<210> 2120
<211> 68
<212> RNA
<213> Homo sapiens

<400> 2120
gugggcuucu uucucuggca cauuuuuuaa augcuccuuu aguuaauaug caggagggau 60

uuccaugu 68


<210> 2121
<211> 74
<212> RNA
<213> Homo sapiens

<400> 2121
uuucagaaaa cucaggcgag gcaguugcga gggcacaacu caaugcccuc gugaggccuc 60

aacucucugg ggcc 74


<210> 2122
<211> 52
<212> RNA
<213> Homo sapiens

<400> 2122
gguaaggcag uugcgagggc acaacucaau gcccucguga ggccucaacu cu 52


<210> 2123
<211> 109
<212> RNA
<213> Homo sapiens

<400> 2123
ccccaguccc ugggccucag ggugaaaggc acauggggcu gaguccacuu ggacaaaugc 60

cuccugugcu uugcaauggc ccugaacucc ccuccuagga acaagggug 109


<210> 2124
<211> 85
<212> RNA
<213> Homo sapiens

<400> 2124
cacagccagg guauccagga ugccaaccgu ugggucccua cccuccucca uacccaccgc 60

cugccccugg aaucccuccu gugaa 85


<210> 2125
<211> 59
<212> RNA
<213> Homo sapiens

<400> 2125
ugugccacag ccaggguauc caggaugcca auccucgggu cccuacccuc cuccauacc 59


<210> 2126
<211> 85
<212> RNA
<213> Homo sapiens

<400> 2126
cacagccagg guauccagga ugccaauccu cgggucccca ccuuccucca uacccaccgc 60

cugccccugg aaucccuccu gugaa 85


<210> 2127

<211>  108
<212>  RNA
<213>  Homo sapiens

<400>  2127
gugccacagc caggguaucc aggaugccaa uccucggguc ccuacccucc uccauaccca      60

ccaccugccc cuggaugccu ccugugaauc ccuuggcucc uggcaugu                 108


<210>  2128
<211>  107
<212>  RNA
<213>  Homo sapiens

<400>  2128
gguagaccau ucugugcgga ucaauucugu cggcagcacc gcuuccuccu cccagccucu      60

gcuugugcac gacgaugucu gagcagaauc aguguuuggg ucacccc                  107


<210>  2129
<211>  67
<212>  RNA
<213>  Homo sapiens

<400>  2129
gacugauacc uuggaggcau uuuaucuaag auacacacaa agcaaaugcc ucuaagguau      60

caguuua                                                               67


<210>  2130
<211>  61
<212>  RNA
<213>  Homo sapiens

<400>  2130
uguacccugg cauugccgac aggaugcaga aggagaucac ugcccuggca cccagcacaa      60

u                                                                     61


<210>  2131
<211>  70
<212>  RNA
<213>  Homo sapiens

<400>  2131
gcgcuuuccc cugacuuucu ggaguuucua aaaguagacu guacgcuaag ggucauaucu      60

uuuuuuguuu                                                            70


<210>  2132
<211>  61
<212>  RNA
<213>  Homo sapiens

<400>  2132
ccgcaguuug caugagauga accccucuga ccagagccca ucuccucagg acuccacggg      60

g                                                                     61


<210>  2133
<211>  92
<212>  RNA
<213>  Homo sapiens

<400>  2133
auuuguccac aaucagccau uggccugucu ggggaguuuu uggaagacag caaagagggg      60

caauggcugc uuucagcuuu gaggacagau gc                                   92

<210> 2134
<211> 105
<212> RNA
<213> Homo sapiens

<400> 2134
gucgucagcc ucugcccugg gcggugucaa aguggagagg caggucuuug gggaggccac 60

caagcagcca ggcaucaccu ucaucgcagc caaguucgau ggcau 105


<210> 2135
<211> 65
<212> RNA
<213> Homo sapiens

<400> 2135
gcagcgaggu cucggcuuuc cgccugcuuu aagccacgcg gaaacugcug cacccuugac 60

ugcac 65


<210> 2136
<211> 80
<212> RNA
<213> Homo sapiens

<400> 2136
   caggacaggc ugcugagcac cacuacauug agcaugaugu gucuccugua ugugugguuc 60
   augugaagua uuguucugau 80


<210> 2137
<211> 55
<212> RNA
<213> Homo sapiens

<400> 2137
uuguaggacu uguuccugug ggcuucagug augggauagu acacuucacu cagag 55


<210> 2138
<211> 124
<212> RNA
<213> Homo sapiens

<400> 2138
ccugggcuca agcaauccuc cugccucagc uuccaaguag cuaggacuac aggcuugugc 60

caccagaccc agcuaauuuu uuuuuuuuuu gguagaguag gagucuugcc auguugccca 120

ggcu 124


<210> 2139
<211> 84
<212> RNA
<213> Homo sapiens

<400> 2139
ccagccuggg ugacagagca agauuccauc caaaaaaaau uuuuuuauag agacaggagu 60

cuugccaugu ugcccaggcu gguc 84


<210> 2140
<211> 72
<212> RNA
<213> Homo sapiens

<400> 2140
gcagagguac aggcuugguu acagggcuuc gcguuccuuu ccagcacagg ccugcucugg 60

gccgcccugc cu                                                          72

<210>  2141
<211>  93
<212>  RNA
<213>  Homo sapiens

<400>  2141
uaggcggacu augacuuagu ugcguuacac ccuuucuuga caaaaccuaa cuugcgcaga      60

aaacaagaug agauuggcau ggcuuuauuu guu                                   93

<210>  2142
<211>  127
<212>  RNA
<213>  Homo sapiens

<400>  2142
cagccggacu gcaucucccg gcgugccccg cggcgggcgc ugggccggag ccggagccca      60

agccagagcg gcgcggccug gaagaggcca gggcccgggg gaggcggcgg cagcggcggc     120

ggcuggg                                                               127

<210>  2143
<211>  69
<212>  RNA
<213>  Homo sapiens

<400>  2143
uugggaagga gaggcacaua acuuuuuuua acauuuggua acuagguuau gggcucuaca      60

uugucagcu                                                              69

<210>  2144
<211>  63
<212>  RNA
<213>  Homo sapiens

<400>  2144
cucgggguuc cucagcuggc ugagguggag ucagugucag ucagggaggc gaacugcuga      60

gca                                                                    63

<210>  2145
<211>  121
<212>  RNA
<213>  Homo sapiens

<400>  2145
agggggaugg gguggggggg uugggugcug cuggagcccc ugcucugcug cuacccagug      60

ccuggcaguc cagggguuaa agcaccacac aaagccagcu caggcaaucu ucuccuccuc     120

c                                                                     121

<210>  2146
<211>  98
<212>  RNA
<213>  Homo sapiens

<400>  2146
uuuuugcaua caucagaaaa acaacuacug uguccaguuc ccugauugac cucagcagau      60

gaagugaaca gauaguguua auucagauug gagaaauu                              98

<210>  2147
<211>  80

<212> RNA
<213> Homo sapiens

<400> 2147
gcugccgaga uccuccugug cucaacggau ccucccaccu cagccgccga guaucuggga 60

ccacgggcac ucuccagcac 80


<210> 2148
<211> 59
<212> RNA
<213> Homo sapiens

<400> 2148
uauguuaagu uucuacuaug ugcuguguau ugguacuaua aauaugaaua agacauaag 59


<210> 2149
<211> 84
<212> RNA
<213> Homo sapiens

<400> 2149
caggggcaca uuggggugga ugccucugcu ccccaaauug gggguagcau cacuggcccc 60

aguguggaag uucaggcccc ugac 84


<210> 2150
<211> 85
<212> RNA
<213> Homo sapiens

<400> 2150
uguuagguug gugcaaaagu acuugcggau uuugcuuuac uuuuaauggc aaaaaccgca 60

auuauuuuug cuucaaccua auaug 85


<210> 2151
<211> 99
<212> RNA
<213> Homo sapiens

<400> 2151
agcucugggc uucagcacaa cagacaccag ccugugggucu cucagaagcc agggagacca 60

caucgggcuc aggacguuuu acccaaaguc cagaguuuu 99


<210> 2152
<211> 56
<212> RNA
<213> Homo sapiens

<400> 2152
cgggcgccgc cucaucguug uccucggaca ccggggacaa uggugacuac accgag 56


<210> 2153
<211> 94
<212> RNA
<213> Homo sapiens

<400> 2153
cuugcaggua gaugagauuu accacgauga gucccugggg guucauauaa auauugcccu 60

cguccgcuug aucaugguug gcuaccgaca ggua 94


<210> 2154
<211> 88
<212> RNA
<213> Homo sapiens

<400> 2154
gcccaccaua gggucugugg gaaauggaaa uggugacucc acacaggaaa cacggcucca 60

ccucaugaga agccugggaa acgggcau 88


<210> 2155
<211> 122
<212> RNA
<213> Homo sapiens

<400> 2155
gucuugugca guuuugcccc uguggcuuug caggguacag accuccuccu ggcugcuuuc 60

aagggcuguc auugaguguc ugcagcucuu ccagguucac agugcaaguu aucaguggau 120

cu 122


<210> 2156
<211> 98
<212> RNA
<213> Homo sapiens

<400> 2156
cccgggccgu cuuccccucc aucguggggc gccccaggca ccagguaggg gagcuggcug 60

gguggggcag ccccgggagc gggcgggagg caagggcg 98


<210> 2157
<211> 75
<212> RNA
<213> Homo sapiens

<400> 2157
gaagacaggc cgagugaugc uuggggagac caauccagca gauucuaagc caggcaccau 60

ucguggggac uuuug 75


<210> 2158
<211> 95
<212> RNA
<213> Homo sapiens

<400> 2158
gcgcuugcgc cgaucucggc cucagcguga gcaugcgcag guccccgccc ucgcugcguu 60

ugcgcuugag cgccgauaau uugguggcgg cgucc 95


<210> 2159
<211> 116
<212> RNA
<213> Homo sapiens

<400> 2159
agcggaaguc acggggagcu ggcggcuggg uugggcggcg gaucugcaga guuucgaggu 60

ucugucaagu ucgcugcccg ccgcccaacu ugaagccuca uuuaccuuuc ggcuac 116


<210> 2160
<211> 54
<212> RNA
<213> Homo sapiens

<400> 2160
gccugacuca gcaugagccu ggcuucuccu gccggccggc acaggggugg guac 54


<210> 2161
<211> 124

<212>  RNA
<213>  Homo sapiens

<400>  2161
gucgccgggg gagcgggugg cguguggauu cgcccuccug cccgccccuc cgggccgcgg      60

acacacgccc ggcagggagg cggcggcggc cccugcguau uugcuucccu ccauuugggg     120

acug                                                                  124


<210>  2162
<211>  106
<212>  RNA
<213>  Homo sapiens

<400>  2162
ugcuucucuc uuucgcucag gcccguggcg ccgacaggau gggugagcug uuguggccgg      60

uuuaagggcg cugcaagcgg gacuuggggu cuuggggacg ggcggg                    106


<210>  2163
<211>  62
<212>  RNA
<213>  Homo sapiens

<400>  2163
ggcccgggcg gcuggccuca ccgcccacug gguaaggagg cucaagcuga gcccggcgcu      60

gc                                                                     62


<210>  2164
<211>  159
<212>  RNA
<213>  Homo sapiens

<400>  2164
cuuuguccug cggcgggccc gauaagaagu ccuccuggcg gggcucgggg ugguggggg      60

cggggagaug aacgcugcgg ccagcagcua ccccauggcc ucccuguacg ugggcgaccu     120

gcauucggac gucaccgagg ccaugcugua cgaaaaguu                            159


<210>  2165
<211>  62
<212>  RNA
<213>  Homo sapiens

<400>  2165
guaacgcgug cugcgcuugc agugagccua cugggcuuuc ugcagcuggg ggauugguau      60

gg                                                                     62


<210>  2166
<211>  82
<212>  RNA
<213>  Homo sapiens

<400>  2166
ggaggcugcg ggggucccgg ccucugcggu cucucuggcc ggccgcagcu guggcgcggg      60

ugaaacgccc gcccuccucc uu                                               82


<210>  2167
<211>  134
<212>  RNA
<213>  Homo sapiens

<400>  2167
ccugcaguac guucuggcgc acccguccga gucccuggag aagaugacgc ugcccaaccu      60

uccgcggcug agcgcguggg uccgagagca gugcccgggg ccggguucac ggugcaccaa 120

caucaucgcg gggg 134

<210> 2168
<211> 163
<212> RNA
<213> Homo sapiens

<400> 2168
uggcugagca gcgugcccgg gcccggcucu cuagcgcuua ucugcuggga guuccaggcu 60

cagagcagcc ugaccgcgcu ggcagccugg agcuucgugg cuucaagaau aagcuguacg 120

uggccguggu cggggacaaa gugcagcucu acaagaaucu aga 163

<210> 2169
<211> 84
<212> RNA
<213> Homo sapiens

<400> 2169
ggcuguggcu uguaggggag gcagaaaugg cugaggugcg gauacgagac cugagucacu 60

ugagcugcug uugccacaug uccc 84

<210> 2170
<211> 77
<212> RNA
<213> Homo sapiens

<400> 2170
gccaggucgg aaguagugug aggcucgugg gcggagccaa gcgccgccau guccgccgcc 60

cugcugcggc ggggccu 77

<210> 2171
<211> 101
<212> RNA
<213> Homo sapiens

<400> 2171
gcagggucug ccuggcgaag cccugggcug cccugcgggu gugggcaccc ccgugccagc 60

agauggcacu cagacccuua ccugugcaca caccucugcu c 101

<210> 2172
<211> 116
<212> RNA
<213> Homo sapiens

<400> 2172
ggaggaggcu ccggcgcagg cgcagacgca gggcgggcaa uggccgccug ggucucucgg 60

gcagcccugc ccaccucucu cgcgucuccu ggucuuggcc ugcggcucuc uuucug 116

<210> 2173
<211> 137
<212> RNA
<213> Homo sapiens

<400> 2173
uccagaguac gggucgagcc cgggccaugg agccccccug gggaggcggc accagggagc 60

cugggcgccc ggggcuccgc cgcgacccca ucggguagac cacagaagcu ccgggacccu 120

uccggcaccu cuggaca 137

<210>  2174
<211>  70
<212>  RNA
<213>  Homo sapiens

<400>  2174
gggccauggc agauuccugg cagcugccca gagcccugcu caugagccag cuccaggggc     60

cccagccccc                                                            70


<210>  2175
<211>  67
<212>  RNA
<213>  Homo sapiens

<400>  2175
aggcgccucc agcagcagcu cgcucagcag augcagcagc ucaacacugc caccuggggg     60

aaccuga                                                               67


<210>  2176
<211>  139
<212>  RNA
<213>  Homo sapiens

<400>  2176
cgccgcaggu cgaguaccgg guggcaaagc agcuggcccg ggccguggaa gguggccagg     60

ccaccgcggc cugugacgcg caccucggcg cccaaggccc guagccgcgc aguuuccucg    120

ggcgucaggc cgccgcgca                                                 139


<210>  2177
<211>  103
<212>  RNA
<213>  Homo sapiens

<400>  2177
gggggcucau gagcagggga agugggaagg gaaauuuggu ggacacucau cccaguuaag     60

caaggccauc acccucuccc uucccagggc uuccugccuu cuc                      103


<210>  2178
<211>  53
<212>  RNA
<213>  Homo sapiens

<400>  2178
cugcucgcug ggccgcggcu cccggguguc ccaggcccgg ccggugcgca gag            53


<210>  2179
<211>  133
<212>  RNA
<213>  Homo sapiens

<400>  2179
cccucuuacg ucucugugcu uguggcucug gggaaccugg gucugcuggu ggugacccuc     60

uggaggaggc uggccccagg aaaggacgag caggucccca uccggguggu gcaggugcug    120

ggcauggugg gca                                                       133


<210>  2180
<211>  62
<212>  RNA
<213>  Homo sapiens

<400>  2180

uccgugggug gcccccugug guguccccag cauugaacac agcagcacgu cccucauggg 60

ug 62

<210> 2181
<211> 105
<212> RNA
<213> Homo sapiens

<400> 2181
uccacggggg gcugaggaca gggguaaguc uggggagaug gggggagcuc ugcugagggu 60

gcacaaggcc cuggcucuac acacaucccu gucuuacaga ggaua 105

<210> 2182
<211> 114
<212> RNA
<213> Homo sapiens

<400> 2182
aagacucaga ggccgccauc aaccgccaga ucaaccugga gcucuacgcc uccuccauuu 60

accugugcgu ggcuuacuac uuugacagcg augauguggc uuugaagaac uuug 114

<210> 2183
<211> 77
<212> RNA
<213> Homo sapiens

<400> 2183
ggugagcaga gcccagccca guggaaaugg ccaugauaca gugaucgcgu cucaucccuu 60

gcugugcccu ccaccca 77

<210> 2184
<211> 66
<212> RNA
<213> Homo sapiens

<400> 2184
uggccaagga gcuggaggag aagagugacc gucugcucaa gguccucuuc cuggugcugg 60

gcuaca 66

<210> 2185
<211> 108
<212> RNA
<213> Homo sapiens

<400> 2185
ugucaagcag ggcgguucua gagagagugg agguggagcc uuucccugcc uguccaacag 60

ccuccgagau uuugccucuc ucugccuggg auuccuccuc uuggcguc 108

<210> 2186
<211> 135
<212> RNA
<213> Homo sapiens

<400> 2186
aagcuggucu guuaucugcc cugaguccug caagaacauc ucaagaaaaa uaccaaaaac 60

augcaagaca aaugaggguc cuucuuuagu guguucuuga agcacugagg ugccugaaag 120

uuauauauag uuucu 135

<210> 2187
<211> 76

<212>  RNA
<213>  Homo sapiens

<400>  2187
agcucugaca ggaggugcgu ggcccaggag gagccaucag gccaguucuc ugggauacug      60

cugugucucc agcucu                                                      76


<210>  2188
<211>  82
<212>  RNA
<213>  Homo sapiens

<400>  2188
gggcaguagc aucuuuuccu caccucguau ugccaguaac uggagcaaua ugagguaggg      60

agaagauguc uccaugaccu gc                                               82


<210>  2189
<211>  95
<212>  RNA
<213>  Homo sapiens

<400>  2189
agauccugag ucaaugauuc gggugcaagu agauuucuuc ugggagccuu ucuuuaauaa      60

agcacuugca ccugagucau ugacucagga ucuau                                 95


<210>  2190
<211>  74
<212>  RNA
<213>  Homo sapiens

<400>  2190
aaugcagauu cuugggccuc acccagagcu gccaagcaga agucccuugg guggggccua      60

ggaaucugca uucc                                                        74


<210>  2191
<211>  143
<212>  RNA
<213>  Homo sapiens

<400>  2191
uggcagucuc agcaucaccu gcaaacuugu uagaaaugca gauucuuggg ccucaccuca      60

uacuuacugc aucagaagug ccaggaucca ggcccagcca ccuguguuuc agcaagcccu     120

gaugcagcua caccacugcu aua                                             143


<210>  2192
<211>  75
<212>  RNA
<213>  Homo sapiens

<400>  2192
aaaugcagau ucuugggccu caccccacag uacugaauca gaaacucuga gggugggccc      60

agagucugug uuuga                                                       75


<210>  2193
<211>  85
<212>  RNA
<213>  Homo sapiens

<400>  2193
ggcgcccugc ugcggucgcc gcgagccucg gccucugucu ccucccccuc ccgcccuuac      60

cuccacgcgg gaccgcccgc gccag                                            85

<210> 2194
<211> 68
<212> RNA
<213> Homo sapiens

<400> 2194
gguggacagg cucuggcagc cgggcagugc ccuccccguc ccaugcucgg cgagccuccc 60

ucuacucg 68


<210> 2195
<211> 63
<212> RNA
<213> Homo sapiens

<400> 2195
cuggaagagg uggcccagag gucccaugcu gugggauccc caggcagccc ccucauucug 60

gag 63


<210> 2196
<211> 92
<212> RNA
<213> Homo sapiens

<400> 2196
gcgugcucga aggaugccau ggucagggca gggccuccag agcagguguu gugccuggag 60

cugcucuccu ggccuccuug gauuuaucgc aa 92


<210> 2197
<211> 92
<212> RNA
<213> Homo sapiens

<400> 2197
gcgugcucga aggaugccau ggucagggca gagccuccag agcagguguu gugccuggag 60

cugcucuccu ggccuccuug gauuuaucgc aa 92


<210> 2198
<211> 143
<212> RNA
<213> Homo sapiens

<400> 2198
cuucccccca acucccuccu ucugaucucu ucuuuuuaau cuguucccau uuuccgaguu 60

aaguucuauu uccaaaacug uucucuggag cuauaagugg auugccagaa augagagauu 120

aggagcuggg agaagaggaa gcg 143


<210> 2199
<211> 62
<212> RNA
<213> Homo sapiens

<400> 2199
ggcccuggca ggugagccug agaguccgcg accgauacug gaugcacuuc ugcgggggcu 60

cc 62


<210> 2200
<211> 72
<212> RNA
<213> Homo sapiens

<400> 2200
ugggucaguu cagagguccu cggggcgcgc gucgagucag ccgugugcgc agggagaccu 60

cucccaccca ca 72


<210> 2201
<211> 87
<212> RNA
<213> Homo sapiens

<400> 2201
ugcuuccuaa uucucagucc ucgaguccua ccuccgaucu caagaaaagg ugaggauuuc 60

agagaggcca uaaaaaggag aagcaag 87


<210> 2202
<211> 64
<212> RNA
<213> Homo sapiens

<400> 2202
ggcaguucuu gcugccccuu cucuggcugu gcuuugccug agggaagagu uggcaagagg 60

ccag 64


<210> 2203
<211> 114
<212> RNA
<213> Homo sapiens

<400> 2203
acaauugucu aacuuuuuau uuuuggucug gcuguugugg ugugcaaaac uccguacauu 60

gcuauuuugc cacacugcaa caccuuacag auguggaaga ugugaaauuu guca 114


<210> 2204
<211> 134
<212> RNA
<213> Homo sapiens

<400> 2204
aggaugcugc ucucauggga gcuggccucu gaccugucuu gggcaggucc caggcauucc 60

ucaucuguua gaggccagca augcccaggu cugcccuaga caggccagca ggaauggaga 120

ggaggguuc cuca 134


<210> 2205
<211> 120
<212> RNA
<213> Homo sapiens

<400> 2205
ggcgaggugu ccgcccguca ggagcucaag cagcgggcgc gcuaccuggc cgagaaguac 60

gagugggacg uggcugaggc ccgcaagauc uggugcuuug ggcccgacgg caccggcccc 120


<210> 2206
<211> 138
<212> RNA
<213> Homo sapiens

<400> 2206
ggggucaaug ugaugagagg cacaaagcuu guggccuccc uacaaacaaa ugccuacaug 60

ugaagaggaa aaaaaauuau agaaucuggg uagugggugu auggguguuc acuguauaau 120

ucuuucaaca aucuccua 138

<210> 2207
<211> 95
<212> RNA
<213> Homo sapiens

<400> 2207
agacuuuucu uuuuaaggaa gugcuguuuu uuuuugaggg uuuucaaaac auuuugaaaa 60

gcauuuacuu uuuugaccac gagccaugag uuuuc 95


<210> 2208
<211> 131
<212> RNA
<213> Homo sapiens

<400> 2208
gucauuggca uguaagagaa auauaccugc auguuagucu aacguucuga uagaaaugac 60

augcauuuau gcugccauuu guuacuauca ggacucgacu cgugugcgga cauuucuguu 120

aauaaugaca g 131


<210> 2209
<211> 54
<212> RNA
<213> Homo sapiens

<400> 2209
uuagcuccca gccuacuccc ucugccguca gaagccagug ggacucagcu aaaa 54


<210> 2210
<211> 64
<212> RNA
<213> Homo sapiens

<400> 2210
gccuugagag uuaagcgaag ugugguggcu uccaagguac cgacacuugc cguaggcugg 60

gcuu 64


<210> 2211
<211> 58
<212> RNA
<213> Homo sapiens

<400> 2211
auuuggagau ggaagaagcu augaaagcaa acuaauaggu ucucuguaaa acaaguag 58


<210> 2212
<211> 61
<212> RNA
<213> Homo sapiens

<400> 2212
aguguacaau gaagcuggug ugaccuucac auagauggau cauggcugac uuccucacua 60

u 61


<210> 2213
<211> 55
<212> RNA
<213> Homo sapiens

<400> 2213
acacuaaugc uuguuggauu caugaggugg ccagaccaac guguuuuuuu uguuu 55


<210> 2214

<211>  95
<212>  RNA
<213>  Homo sapiens

<400>  2214
uuacuugcua gagaaucugu uuuaauauag ugguuuggcc ucugauuauu uauagguuuu      60

auaaauuuua gaaucaauuu cucuuuaagg uggcu                                 95


<210>  2215
<211>  60
<212>  RNA
<213>  Homo sapiens

<400>  2215
aauggauuuu cugagaaguu cuaccuaauu guaauagaau gcacucaaga caaccguuca      60


<210>  2216
<211>  85
<212>  RNA
<213>  Homo sapiens

<400>  2216
gcacagagac cgugaagauu ccaaguucag gagaacucga ccggucaguu uccuuuccua      60

agagucucuu accugcuugu uguac                                            85


<210>  2217
<211>  52
<212>  RNA
<213>  Homo sapiens

<400>  2217
aguguuuguu gauuuuuguu ccucaguuua gauuugaaaa cacaugcauu aa              52


<210>  2218
<211>  108
<212>  RNA
<213>  Homo sapiens

<400>  2218
ggaacaaagu cacuccagug uuauauuuga auuaugggcc cuacgguucu uaugcaccgc      60

auuaugacuc cacauuugcg aauaucagca aggaugauuc ugauuuaa                  108


<210>  2219
<211>  90
<212>  RNA
<213>  Homo sapiens

<400>  2219
aagaugugac ucgcuacucc uugccuucca cuaucguugu gaggccucug cagccaugug      60

gaacugcaaa uucuccauua aaccucuuuc                                       90


<210>  2220
<211>  99
<212>  RNA
<213>  Homo sapiens

<400>  2220
acauagugug ugaguugcuc cuccuugccu ucugccauga uugugaagcc uccccagcca      60

uguggaacug caaaugcaau aaaccucuuu cuuuuguaa                             99


<210>  2221
<211>  68
<212>  RNA

<213>  Homo sapiens

<400>  2221
agaauuguaa guuucuugag gcuuccccag ccauguggaa cugcaaauca auuaaaccuc      60

uuuucuuu                                                              68


<210>  2222
<211>  57
<212>  RNA
<213>  Homo sapiens

<400>  2222
auuaucaccu cccaccauga uucugaggcc uccccagcca uguggaacug caaaucc        57


<210>  2223
<211>  121
<212>  RNA
<213>  Homo sapiens

<400>  2223
gcagccucgc cgcaacccug uuggccaacc acagccugcg ugagcuggac cucagcaaca      60

acugccuggg ggacgccggc auccugcagc ugguggagag cguccggcag ccgggcugcc     120

u                                                                     121


<210>  2224
<211>  65
<212>  RNA
<213>  Homo sapiens

<400>  2224
uauuaagguu cuccuugccc ugaagcuuca ggaugaagga caggaagcug aaggagaaaa      60

guaca                                                                 65


<210>  2225
<211>  59
<212>  RNA
<213>  Homo sapiens

<400>  2225
uuuuuuuuuu uuuuuuuucu ggucacagcg auugaacagg agaagcaagc aggcgaauc       59


<210>  2226
<211>  125
<212>  RNA
<213>  Homo sapiens

<400>  2226
ccagcaggug uucgacaaug gcagcaucua caacccugaa gugcuugaua ucacagagga      60

aacucugcau ucucgcuucc uggagguaua ugcugucagc cuugcccccu cuuacacuga     120

ugguu                                                                 125


<210>  2227
<211>  68
<212>  RNA
<213>  Homo sapiens

<400>  2227
uacuuauguc uagguuccuu ugguaucauc cacaagugag ggguacacag caucucaacc      60

cuuguacc                                                              68


<210>  2228

<211> 101
<212> RNA
<213> Homo sapiens

<400> 2228
acuaagguca ucuuuuauag ggucgucuaa ggacaguugc cuuaauguga agacuccuau 60

agucccugug gaggaccugc ccuacacacu cagcaugguc g 101


<210> 2229
<211> 90
<212> RNA
<213> Homo sapiens

<400> 2229
aguggagauu aggucugaug uuucuuuguu gauuuucuuu cuggaagauc uguccagugc 60

ugaaaguggg auguugaagu uuccagcuau 90


<210> 2230
<211> 78
<212> RNA
<213> Homo sapiens

<400> 2230
uguccagugc ugaaaguggg auguugaagu uuccagcaau uacuguguug gaguccaucu 60

cucuuuagcu cuaauaau 78


<210> 2231
<211> 58
<212> RNA
<213> Homo sapiens

<400> 2231
aaagugggau guugaaguuu ccagcuauua uuguauuggg gucaaucucu cucuuuag 58


<210> 2232
<211> 57
<212> RNA
<213> Homo sapiens

<400> 2232
ugaguugggg auuccaucau uucauuuaag aggugaagag ggauuuaauu auuuaau 57


<210> 2233
<211> 64
<212> RNA
<213> Homo sapiens

<400> 2233
agucucuuuc aaaggagugu gauuuaaaag cccaucauaa ucggaggcuu uggcaacugg 60

cuag 64


<210> 2234
<211> 90
<212> RNA
<213> Homo sapiens

<400> 2234
guuauguuuu uucuauauug augcuguuuc uuccaaagca auugcuaugc uuuguaagaa 60

ucguuaguca aauauagauu uuuuugauuu 90


<210> 2235
<211> 200
<212> RNA

<213>  Homo sapiens

<400>  2235
guguucuuuu uuccuucccu aauucuucac cucccccucc ucugcugccu ccuugcucuc      60

cucgcagguc aaguuuaccc augggggaac ugguuccagc caaaccgcac caaccugugg     120

cagguaagac cuacguuccu ccuuucacca agcauugaca gagaccugcu ggaggcuggg     180

cacuguguug uuaggccgcg                                                 200


<210>  2236
<211>  57
<212>  RNA
<213>  Homo sapiens

<400>  2236
aauuauugcu cuaaaauuuc uaauauuuau aguuagaacu cuagguaaga gaauucu         57


<210>  2237
<211>  65
<212>  RNA
<213>  Homo sapiens

<400>  2237
ucacaccuac aguggacuac ccgauuuuuc gcuucucuuc agggaugagu cauguggugg      60

ugaaa                                                                  65


<210>  2238
<211>  74
<212>  RNA
<213>  Homo sapiens

<400>  2238
ggcacuuacc gccagcucuu ccacccugag caacucauca caggcaagga agaugcugcc      60

aauaacuaug cccg                                                        74


<210>  2239
<211>  99
<212>  RNA
<213>  Homo sapiens

<400>  2239
ucuggcugga uccuauaucc cagcaggccu gggcuccacc ugccacaucc cuguccuagg      60

gugaggccag gccucugcuu gguugacaca ucccagguu                             99


<210>  2240
<211>  99
<212>  RNA
<213>  Homo sapiens

<400>  2240
uauucucucu uuuuuuucuu uaucagucug gcuaaagguu ugucaauucu uuuuaucuuu      60

ucgaaaaaca aacuuuuagu uuugauaauc uuuuauauu                             99


<210>  2241
<211>  81
<212>  RNA
<213>  Homo sapiens

<400>  2241
uuguaauguc uccuuuauuu cuguuuuauu uauuuagauc gucccucuuu auuuaucagu      60

cuggcuaaag guuugucaau u                                                81

<210> 2242
<211> 63
<212> RNA
<213> Homo sapiens

<400> 2242
ucagucuggc uaaagguuug ucaauuuuau cuuuucaaaa accaacguuu uguuucauug 60

auc 63


<210> 2243
<211> 68
<212> RNA
<213> Homo sapiens

<400> 2243
ucagucuggc uaaagguuug ucaauuuugu uuaacuuuuc aaaaaaacaa cuuuuuguuu 60

cauugauc 68


<210> 2244
<211> 72
<212> RNA
<213> Homo sapiens

<400> 2244
ucagucuggc uaaagguuug uugauuuugu uuaucgucgc aaaaaaaaaa ccaacuuuuc 60

auuucauuga cu 72


<210> 2245
<211> 93
<212> RNA
<213> Homo sapiens

<400> 2245
cagccuccau uuuucuuagu cagucuggcu aaagguuugu caauuuuguu uaacuuuuca 60

aaaaaaaccu uuuuguuuca uugauauuuu gua 93


<210> 2246
<211> 77
<212> RNA
<213> Homo sapiens

<400> 2246
acgucuucuu uuucaucccu gauuuuauuu auuuggguuu ucucucuuuu uuuuucaguc 60

uggcuaaagg uuuguca 77


<210> 2247
<211> 94
<212> RNA
<213> Homo sapiens

<400> 2247
uugucagucu ggcuaaaggu uugucaauuu uguuuaucuu uucagaaaaa ccaauauuuu 60

gugucauuga ucuuuuguuu ucuuuguuuc aauu 94


<210> 2248
<211> 144
<212> RNA
<213> Homo sapiens

<400> 2248
gcccgcugcu uuugcccacc cucuuccucu cuccuuacac ccugcaggcg uccacacauc 60

ucuaucaguu ggaaccccag ccugggccug ugcuggaagc uggagccuca ugggagccag 120

ccaggccugg gagcaagcag gcuc 144

<210> 2249
<211> 108
<212> RNA
<213> Homo sapiens

<400> 2249
agccgccgcu gccaaagcug ccgccacuag ccgggcaugg ccauggcguc cccggccauc 60

gggcagcgcc cguacccgcu acuauuggac cccgagccgc cgcgcuau 108

<210> 2250
<211> 107
<212> RNA
<213> Homo sapiens

<400> 2250
guggcugggg ugccauuggg auuuuggaau gggagcaguu ugauuccucc cucggaagga 60

acucauggcu cucacaaacc agugucugug gcagugacaa gucacag 107

<210> 2251
<211> 121
<212> RNA
<213> Homo sapiens

<400> 2251
cugcaccacc ccuccuccuc cuccgccucc uccugcugcu gcugccgcac cuccuccccg 60

caggacuuca guaagcggca gcagcagcag cugagcagcc agaagaggaa agaguucagc 120

c 121

<210> 2252
<211> 87
<212> RNA
<213> Homo sapiens

<400> 2252
uggguuccgg gagacccgcg ggucuggcug cgagagguga gggcagagcc ggggcggggc 60

ccuagcgggg ugucccggcg acccaga 87

<210> 2253
<211> 77
<212> RNA
<213> Homo sapiens

<400> 2253
uggccugcau ggcuccuacg ugcugagggu ggaggcugaa aggcugacuc uccugaccgu 60

gggggcccag agucaga 77

<210> 2254
<211> 112
<212> RNA
<213> Homo sapiens

<400> 2254
   uggcaugauc ucggcucacu gcaaccucug ccucccaguu ucaaguuauu cucgugccuc     60
   agccucccga guagcuagga cuuacaggug cgugccacca cacccagcua au            112

<210> 2255
<211> 86

<212>  RNA
<213>  Homo sapiens

<400>  2255
agcgauggca ugcuacuguu uaggccucau uuccccaaaa aacagcgagu cccaggaagg      60

gcuugagcac agcuggccgu gguuca                                            86


<210>  2256
<211>  67
<212>  RNA
<213>  Homo sapiens

<400>  2256
ccgugagccc gcagggcagc gucacaggcu ugugcagcag ccgugggcag ccaagcaggu      60

cgcgggg                                                                 67


<210>  2257
<211>  99
<212>  RNA
<213>  Homo sapiens

<400>  2257
gccuuuaggg accugaggag ccccgggauc agccaagcca gacuucucuu gugaucggga      60

aggcaacuga ggcccaaggu cacgguguca gcaaggugu                              99


<210>  2258
<211>  55
<212>  RNA
<213>  Homo sapiens

<400>  2258
ucgauuuacg gccgcaggug cgcgcgcuuu uuugcucucg cugggaaugg cggag            55


<210>  2259
<211>  104
<212>  RNA
<213>  Homo sapiens

<400>  2259
guggcucaaa uuugcucccu gucuucugac ccuguaugug uggugaccca gaacguguuc      60

cucuucauau uagccaggga gcuugcagga agcagagcuc accc                       104


<210>  2260
<211>  144
<212>  RNA
<213>  Homo sapiens

<400>  2260
uguucagucu gaggaccuca agcuuggguc aggccagcau guuuuaggua uggauguggu      60

gacuuaaagu acuugauugu cuugauuuca cccugaaagc uuuggcaaac caaguuucaa      120

cccuuacuuu aaaaaucuga cacu                                             144


<210>  2261
<211>  91
<212>  RNA
<213>  Homo sapiens

<400>  2261
ugguacagaa ggauuaaaug ucacuugcuc aaagucacau ccugugaggg gccaggauuc      60

aaacucaggu ggcccgauuc agcucuccau c                                      91

<210> 2262
<211> 80
<212> RNA
<213> Homo sapiens

<400> 2262
ggccaauggg aggacaaaug ccccucugaa cugucuucug ggcagugaca gucaugguca 60

uaauccugac ccugagccag 80


<210> 2263
<211> 138
<212> RNA
<213> Homo sapiens

<400> 2263
ccgggcguca gggugcauug ugggaaggag gcggcagcgu cucgggcggg cgggaggugc 60

accagcggcg gcggcggcgg uaaauccucc cggccgcuca cagcacugug gagccgcguc 120

cccagcccgg ccucggac 138


<210> 2264
<211> 64
<212> RNA
<213> Homo sapiens

<400> 2264
uucuagucug ggagcagaca gagaaaacaa aauuaagguu uuugaaugcu uuggauggag 60

aaag 64


<210> 2265
<211> 103
<212> RNA
<213> Homo sapiens

<400> 2265
gccuauacau cuaguguugu cuaguguugu cauggcugca gucuuugcag uuuucagugc 60

aucucaucca uuugcugagc auacuucucu gauguaaugg uuu 103


<210> 2266
<211> 135
<212> RNA
<213> Homo sapiens

<400> 2266
gcauuuucau ggagaaaugg gcccuuucug uugaccaaug ccagcugaag gaguugcagu 60

uuucagugca ucucauccau uugcugagca uacuucucag auguaauggu uucaccagga 120

uucagaaagc uguag 135


<210> 2267
<211> 122
<212> RNA
<213> Homo sapiens

<400> 2267
cccugauuuu uuucccccua uauucucauc cuugcuugcc uguuaggugg gugugccugc 60

ugcuuuggac augaugcuga cugguagaag cauucgugca gacagggcaa agaaaauggg 120

ac 122


<210> 2268
<211> 68
<212> RNA

<213>  Homo sapiens

<400>  2268
uggggcccag ggaggauggg gagggagugg cccaggccug ccccuucgcu ccugccugca      60

uuuccaga                                                                68


<210>  2269
<211>  68
<212>  RNA
<213>  Homo sapiens

<400>  2269
gcagaagaag gagcugucug acaucgcuca ccgcaucgug gcaccuggca agggcauccu      60

ggcugcag                                                                68


<210>  2270
<211>  56
<212>  RNA
<213>  Homo sapiens

<400>  2270
agcccgagcc ccggcaccgg gcggucugug gccgcucucg ggacucccca ggcuau          56


<210>  2271
<211>  121
<212>  RNA
<213>  Homo sapiens

<400>  2271
uccucggccg cgcaaugggc acccgcgacg acgaguacga cuaccucuuu aaaggugagg      60

ccaugggcuc ucgcacucua cacaguccuc guucggggac ccgggccacu cccgguggac     120

c                                                                      121


<210>  2272
<211>  74
<212>  RNA
<213>  Homo sapiens

<400>  2272
ggccccagag gggcaggagg ggcuggcaag gcccguucca gacuuugcua acccugccuu      60

cccacagggg cuuc                                                         74


<210>  2273
<211>  61
<212>  RNA
<213>  Homo sapiens

<400>  2273
aguuucccua gcgggccauu ccuuagcaaa auguuggaau cccuguugcu acauugacua      60

a                                                                       61


<210>  2274
<211>  112
<212>  RNA
<213>  Homo sapiens

<400>  2274
gaguauugau uggugguauu gguuaugguu cauuguccgg agaguauauu guugaagagg      60

auagcuauua gaaggauuau ggaugcgguu gcuugcguga ggaaauacuu ga             112


<210>  2275

<211>  53
<212>  RNA
<213>  Homo sapiens

<400>  2275
uaacuaccuu ucuuugcucc aagugaaguu uggacccagc agaaagcauu auu          53


<210>  2276
<211>  83
<212>  RNA
<213>  Homo sapiens

<400>  2276
uuuuuguacc aaaaauacuu aaagaccuca gaagcacucu uuuacuuuua agaaauugcu    60

uuuuugaacu uuauucagaa gca                                            83


<210>  2277
<211>  58
<212>  RNA
<213>  Homo sapiens

<400>  2277
agccaaggcc cgcagccucu uugaugccaa ccgagcugag cuguuugccc agagcugc      58


<210>  2278
<211>  56
<212>  RNA
<213>  Homo sapiens

<400>  2278
gauauucuuu uaacucauau guauugucaa guauuguugc uuugaaauga agucuu        56


<210>  2279
<211>  104
<212>  RNA
<213>  Homo sapiens

<400>  2279
gccacuuaaa uaagcuuaua cuuugccaaa gcugucaucu uguaauauau gcucuagaaa    60

ugacauaaag guuuaugggg aagcaaagag agauuuugug gcaa                    104


<210>  2280
<211>  140
<212>  RNA
<213>  Homo sapiens

<400>  2280
ggcgccucau ggauuugcug cagaagaaga uggucagccu agacaucugu cgcuaccugg    60

cccuggacga ggcugaccgc augaucgaca ugggcuucga gggugacauc cguaccaucu   120

ucuccuacuu caaggugccg                                               140


<210>  2281
<211>  61
<212>  RNA
<213>  Homo sapiens

<400>  2281
cuagaccuca ucacaacaga gacaaggucu gaugaugcuc uguguggccu ugaugcuggc    60

u                                                                    61


<210>  2282
<211>  132
<212>  RNA

<213>  Homo sapiens

<400>  2282
ugagacuugc caguucccac aggccaagcc aggcgucugc accccauucu cuugguuccg      60

acgguguguc ucugcgaugu uuuggcgguc ucuccucucc auccccuuaa uaauacuggu     120

cucuguuuca cc                                                          132

<210>  2283
<211>  60
<212>  RNA
<213>  Homo sapiens

<400>  2283
gaugagugcg caguaugagg accgcuggcc gggacccagu gagcgcggga cuacaauucc      60

<210>  2284
<211>  72
<212>  RNA
<213>  Homo sapiens

<400>  2284
ucugcccccu guggcugcac cugguccucc guaccaaccc cuggggguau ccacaaccac      60

uugggacaga ag                                                          72

<210>  2285
<211>  111
<212>  RNA
<213>  Homo sapiens

<400>  2285
cccaggggug uccacaacca cuugggacag aggugaaggu ggaauuucaa agagaagcuu      60

ccuuggaucu ucggugccaa gcuuggauga gcuacagccc caacaugggc c              111

<210>  2286
<211>  112
<212>  RNA
<213>  Homo sapiens

<400>  2286
cccaggggug uccacaacca cuugggacag aaggugaaag uggaauuuca aagagaagcu      60

ugcuuggauc uucggugaca ggcuuggaug aguuacggca ccaagcuggg cc             112

<210>  2287
<211>  108
<212>  RNA
<213>  Homo sapiens

<400>  2287
ucuguuccgu guaacuggaa augugcucug aacuugucug ucucccuugg cugcuccugg      60

cccuuguuac uggcugucag ggauguccac aaccacuugg gacagaag                  108

<210>  2288
<211>  67
<212>  RNA
<213>  Homo sapiens

<400>  2288
uaccagaggc ugggaugggu agugcaagag uacaaggggc uauagucaau aauaaucuaa      60

ugguaca                                                                67

<210>  2289

<211>  53
<212>  RNA
<213>  Homo sapiens

<400>  2289
auuacugugg agaaugccuu uucgcuauuc ucugcugcag ucagauauga ugc          53


<210>  2290
<211>  53
<212>  RNA
<213>  Homo sapiens

<400>  2290
ucugcucagc aucuggugag ggccuucuuu gcaucuuugu aacauggcag aag          53


<210>  2291
<211>  91
<212>  RNA
<213>  Homo sapiens

<400>  2291
aagaaauuua uuuggcucac aguucuggag gcugggaagu ccacaggcau ggugccggca    60

ucugcucagc aucuggugag ggccuucuug c                                   91


<210>  2292
<211>  104
<212>  RNA
<213>  Homo sapiens

<400>  2292
auacaggaga aauguauuug gcucacagau cuggaggcug ggaaguccaa gggcguagug    60

cuggcuucua cucagcaucu ggugagggcc uucuugcugu guca                    104


<210>  2293
<211>  77
<212>  RNA
<213>  Homo sapiens

<400>  2293
cacaguuuca gaggcuggga aguucaagau caagaugcca gcagacucag caucugguga    60

gggcucuccg cugugua                                                   77


<210>  2294
<211>  90
<212>  RNA
<213>  Homo sapiens

<400>  2294
gcaucugcuc agcaucuggu gagggccuuc uugcuacauc auaauauggc agagggcguc    60

auauaaccag aaggcaagaa ugggauguca                                     90


<210>  2295
<211>  80
<212>  RNA
<213>  Homo sapiens

<400>  2295
auucaauguc auccugggcu acauguggcc ugauagcuuc agguugaaca aguuugucuu    60

auuaucauua uauugaauua                                                80


<210>  2296
<211>  96
<212>  RNA

<213>  Homo sapiens

<400>  2296
uacuguauuc caaacauuuu caucucugga aauuuuuagu uuucuaacac cuuuggaaaa      60

ugcugaguca gaaucacuaa gaugauguuc aguaua                                96


<210>  2297
<211>  75
<212>  RNA
<213>  Homo sapiens

<400>  2297
uguuacaugg guuacuguua uuguuuuagg ccugaucugu uuagaaagga aagagaaaug      60

aaggaaauag auaga                                                       75


<210>  2298
<211>  61
<212>  RNA
<213>  Homo sapiens

<400>  2298
uuggcaguug gcugcuaauc uuaugaacag uggugugaga cugaaugcca uuuguccagg      60

c                                                                      61


<210>  2299
<211>  58
<212>  RNA
<213>  Homo sapiens

<400>  2299
gguugagcag ucuuuucuuc uacucuccaa uuuacaggga aaugcugcaa agaauuuc        58


<210>  2300
<211>  97
<212>  RNA
<213>  Homo sapiens

<400>  2300
gcccgcggaa ggagugcgca ggcgcggaau cuccgacuug ccauggucgu cuuugucugc      60

cggugaguug gguuucggcc cugugggacu uggguuu                               97


<210>  2301
<211>  71
<212>  RNA
<213>  Homo sapiens

<400>  2301
acugcaggcu gguuuuaggu cuugaauuau guaagaaucu ugcacagcac ugcuaaugua      60

aauuucaguu g                                                           71


<210>  2302
<211>  92
<212>  RNA
<213>  Homo sapiens

<400>  2302
agaagacuag aaccuccacu gcaggcuggu uugaggucgu gauuuauguc agaaucuugc      60

acagcacugc uaauguaaau uucagcuuuu uc                                    92


<210>  2303
<211>  93
<212>  RNA

<213>  Homo sapiens

<400>  2303
cacauggaca ucgaggcgcu gaaaaaacuc aacaagaaua aaaaacuggu caagaagcug      60

ggugaguccg gccgcugugg uuuugcaugu gag                                   93


<210>  2304
<211>  74
<212>  RNA
<213>  Homo sapiens

<400>  2304
uugcaucaua ccugguggaa guuuuuguau cuuuaagagu aaguccgcaa uguugccagu      60

gucugucugc agau                                                        74


<210>  2305
<211>  69
<212>  RNA
<213>  Homo sapiens

<400>  2305
agcuggagga cuuccgcgac uaccggcgug ugcacaagcc gcccaaggug caggagaagu      60

gccagcugg                                                              69


<210>  2306
<211>  76
<212>  RNA
<213>  Homo sapiens

<400>  2306
uguuugcagu gggugccacc cacagugcca gcauccagga ggaaacugag gaggugguga      60

acucacagua agcacc                                                      76


<210>  2307
<211>  86
<212>  RNA
<213>  Homo sapiens

<400>  2307
uacaauauuu uuguuggucu aaaauuugua caguuaauuu uuucuuaaaa aaugcucauu      60

gaaaauaauu aauaaaaaua gguaau                                           86


<210>  2308
<211>  122
<212>  RNA
<213>  Homo sapiens

<400>  2308
gaaauagcau guauguguug cuuugugaug uauccucagu auccaucagc uuucaaguua      60

aaagcucauu caaguuaaua uugauucaga aucuuuggca aagaaucacc agugcauuuu     120

uu                                                                    122


<210>  2309
<211>  87
<212>  RNA
<213>  Homo sapiens

<400>  2309
agagugugua augagccauc caacaaccac uguggaugug uccagacugu cucaaaggcu      60

guuggauauu ugcugggucc gcucugg                                          87

<210> 2310
<211> 53
<212> RNA
<213> Homo sapiens

<400> 2310
aaacccaggg aacugaaaca aauuacuuaa uuuuucaguu ucuggaaauu uag 53

<210> 2311
<211> 103
<212> RNA
<213> Homo sapiens

<400> 2311
uucaacaacu gccuaugauu cuuuuuacuc auuuccccag gaaauacauu guucucucug 60

ggauagcucu gagucaaguc aaauaaggau agcauuguga guu 103

<210> 2312
<211> 123
<212> RNA
<213> Homo sapiens

<400> 2312
agaucaaguu gauugaaugu uguuuagguc aacuacaucu uuauugguuu ucuaccuguu 60

ugaucuguca auuacugaga aagaaauguu gaagucucca uauaauaaua gugaauuugu 120

cua 123

<210> 2313
<211> 99
<212> RNA
<213> Homo sapiens

<400> 2313
gagucccucu cccagaugcu guccaugggc uucucugaug aaggcggcug gcucaccagg 60

cuccugcaga ccaagaacua ugacaucgga gcggcucug 99

<210> 2314
<211> 58
<212> RNA
<213> Homo sapiens

<400> 2314
uguuugagcu gauguguggg gaacugcuuu uggauuacuu auggguugcu gaaauaua 58

<210> 2315
<211> 77
<212> RNA
<213> Homo sapiens

<400> 2315
agugaaguga aagugaaagg cagaauugaa aguguuaagg cagacauggc aacuuccugu 60

cucuucuaca gaacucc 77

<210> 2316
<211> 96
<212> RNA
<213> Homo sapiens

<400> 2316
aaccugugua uggugacugg aggugcuaac cuguguauga agaauuggug ugaucaccaa 60

cagagagagg cacccuggau cuuuugacgu gguuca 96

<210> 2317
<211> 55
<212> RNA
<213> Homo sapiens

<400> 2317
gauucgcugu aggacuuugg ucgagcggca gugcuucccu gagccucagc guugc 55


<210> 2318
<211> 68
<212> RNA
<213> Homo sapiens

<400> 2318
agcaguuagu ccuuccuccc cugcccucag ccagaugcuc agcaaggagg ccuggaugug 60

gcggcucc 68


<210> 2319
<211> 96
<212> RNA
<213> Homo sapiens

<400> 2319
ccaugcagau uaccaguuga ccagugcaga gcucuccucc uuaccagccu uuaguucacc 60

uggggggcug ucgcuaggca augucacugc cuggca 96


<210> 2320
<211> 59
<212> RNA
<213> Homo sapiens

<400> 2320
ggauccugau gcuucaaaac cggaagacug ggaugagcgg gccaagaucg augauccca 59


<210> 2321
<211> 117
<212> RNA
<213> Homo sapiens

<400> 2321
ugucggaagc uuugugucua uugggaugau caugugaguu ugcccuuuau ucuauucuau 60

ccuaugaaug uggugcauua cauugauuuu cuuacauuga accaauugug ggacaaa 117


<210> 2322
<211> 136
<212> RNA
<213> Homo sapiens

<400> 2322
ccugcaacuc ugucucccac aggaaauguu caucaugcuc accauccuug ccuuucgugu 60

uuuccuugag accacugggg aagagaccuc agugagcaga augcacagcc auggggcaga 120

ggucugagug ugggga 136


<210> 2323
<211> 68
<212> RNA
<213> Homo sapiens

<400> 2323
caggcacuuu uaauucucag ugucuauugc auaaugguca ccagaugggc uauggaguug 60

agccuggg 68

<210> 2324
<211> 67
<212> RNA
<213> Homo sapiens

<400> 2324
ucugaaauua aacuuucucu uuucucucgg cuuuucuaaa aagaguuaau gguuuaauaa 60

uuagauu 67


<210> 2325
<211> 119
<212> RNA
<213> Homo sapiens

<400> 2325
cuggguccaa uuaggagaga ugguuuguca uuucaucuau uaaacuuucu cuuuucaaac 60

uacugucuga aaauagaaac uuuaauagaa gaaauuacua acuauaacaa gacuuagcu 119


<210> 2326
<211> 64
<212> RNA
<213> Homo sapiens

<400> 2326
ugucauucca gagugcugag gccagggcug auggucuuaa augcucauuu ucugguuugg 60

caug 64


<210> 2327
<211> 63
<212> RNA
<213> Homo sapiens

<400> 2327
auucagagcu uugggauguc aggaauuaac ccccaguggg caucacucau caaguauuaa 60

uua 63


<210> 2328
<211> 54
<212> RNA
<213> Homo sapiens

<400> 2328
ggucagcacc ucuagugacc acccaggcug gggucccugu ggggcacuug cccc 54


<210> 2329
<211> 98
<212> RNA
<213> Homo sapiens

<400> 2329
agcgagugca uucuguucuc ugcagcuugg gaagaaugag ccuuaaaaac acacugguca 60

cugcccauag cagcccuggg caguggguac ugagcucu 98


<210> 2330
<211> 139
<212> RNA
<213> Homo sapiens

<400> 2330
uaauagcgcc ggccuucggc gaugucacag ugacgcuaca gugccuggag cugggcuguu 60

uucuccucgg uuuggagccu ggugaccgug acuuccccgc cauguucugu guguugcugg 120

cuggagaagg guaguugac                                                139

<210>  2331
<211>  87
<212>  RNA
<213>  Homo sapiens

<400>  2331
gccccgcccc ggcgaccuca ggcgggcggg gaagaccgag ucuucccgga gccgcuucgu      60

ccgcccggcg ccggaguccc acggccg                                          87

<210>  2332
<211>  57
<212>  RNA
<213>  Homo sapiens

<400>  2332
cccgcugcua ggguguaguu aguccgaggc cggaccaaaa cugcagagac augggug         57

<210>  2333
<211>  74
<212>  RNA
<213>  Homo sapiens

<400>  2333
aguuucucaa cucugugaug uggcuucuga ugugagggaa cuuggagcua aauaaaauaa      60

guauguguca cuca                                                        74

<210>  2334
<211>  108
<212>  RNA
<213>  Homo sapiens

<400>  2334
aggggccaga gucacugacg gugcaggagg gucugugcgu ccucguaccc ugcagauugc      60

ccacuacccu uccagccucg uacuaugguu auggcuacug guuccugg                  108

<210>  2335
<211>  90
<212>  RNA
<213>  Homo sapiens

<400>  2335
gggcagccug ccugcuccug ccucugagcc cauccucuuc uuccaggagu ccagcccaga      60

guggccacag cagacgugua ggccucccug                                       90

<210>  2336
<211>  52
<212>  RNA
<213>  Homo sapiens

<400>  2336
ugcaguaccu gcccaguggu gaccggcccc uucucuuccu ggcaggcgca ca              52

<210>  2337
<211>  85
<212>  RNA
<213>  Homo sapiens

<400>  2337
gcuugcgucc agaaccagcu gcuaggcccu gaugagaacg cuaagaccua caucgccagc      60

cagggguguc uggaggccac gguca                                            85

<210> 2338
<211> 76
<212> RNA
<213> Homo sapiens

<400> 2338
agaggaggac gugggugucc cugagcugau gugaaccuug ccucaacucc uuggcuuucu 60

cccaguccuu cucuuc 76


<210> 2339
<211> 105
<212> RNA
<213> Homo sapiens

<400> 2339
gcugacggag ccgggcucac caggucgcug ccgcgaggga guugcugugc uggggccugg 60

guggcggcug gaggccugag uugggcucgc ggcggggguc ggcag 105


<210> 2340
<211> 93
<212> RNA
<213> Homo sapiens

<400> 2340
ggcugcagga gggcugggga agccagacuc aguccccuag accugggagc ugggcuccuu 60

gcccauccag cagccauucc ccacugcagc cca 93


<210> 2341
<211> 65
<212> RNA
<213> Homo sapiens

<400> 2341
ucugaauuuc ugguccuaag cauuuuggau aagugauacu caacagcauu ggagucaggc 60

agaug 65


<210> 2342
<211> 117
<212> RNA
<213> Homo sapiens

<400> 2342
gccgccgcca uuuugagagc gagccgagcc gagcugccgg gcgccgcguc cgcugccgga 60

gccccgacga cgacgccgag gaggcggagg ccgcggcucu cggaacgcgg ccggccc 117


<210> 2343
<211> 63
<212> RNA
<213> Homo sapiens

<400> 2343
gccgccuccu gcugcuccgg ggcccccaag auggcgggcc cgggcggcgg cgcgaggagg 60

cca 63


<210> 2344
<211> 63
<212> RNA
<213> Homo sapiens

<400> 2344
gccgccuccu gcugccccgg ggcccccaag auggcgggcc cgggcggcgg cgcgaggagg 60

cca                                                                 63

<210>  2345
<211>  60
<212>  RNA
<213>  Homo sapiens

<400>  2345
aggccgggug uaacaggguc aucucaguca ucacugugac ucagcauccu caaagcuucu      60

<210>  2346
<211>  141
<212>  RNA
<213>  Homo sapiens

<400>  2346
uguguggaag uuugcaucug agagugcgag agcuggacgg aguguuacag agccgcgaug      60

ccuucucguu uuguuuuguu uuguuuuuga uacagugucu cgcucuuccg cccagucucg     120

agugcagugg ugagaacacg g                                               141

<210>  2347
<211>  74
<212>  RNA
<213>  Homo sapiens

<400>  2347
ugucuucagc aaaaauaccg uggggucagu uuagccagaa uccuccuuac ccuugauguu      60

uccucuuaga uaau                                                        74

<210>  2348
<211>  100
<212>  RNA
<213>  Homo sapiens

<400>  2348
uccuuuuuuu uuuucuucau ccaggcagag aggccuacug ggacuuaaau ccaaggagcu      60

gaaaucuguu uugggauggg guggagucac auucuggaac                           100

<210>  2349
<211>  126
<212>  RNA
<213>  Homo sapiens

<400>  2349
uuuguagcug uccugcaggu gccggagaca ucuauagggu cuggugguga uaaauugaug      60

cuaacacugg uugguuggua ccagcccacc cugaagcucu gcccucuuaa gcagcaguua     120

gaaacu                                                                126

<210>  2350
<211>  82
<212>  RNA
<213>  Homo sapiens

<400>  2350
ugcauuuuuu ucccuccagc auuucaaaga cauuacucaa auuuuguuug uucuuugugc      60

ugcagugguc uuacuuggca ac                                               82

<210>  2351
<211>  56
<212>  RNA
<213>  Homo sapiens

<400> 2351
gcgacuuggu gaaaacagga aucaaaacuu gggccuauuu cuccugaggu cugcuu 56

<210> 2352
<211> 80
<212> RNA
<213> Homo sapiens

<400> 2352
gacuuucuca uagaauguuc uugaaaggug auuggcaaag ucaugcaggc ccgccuuaaa 60

gcgcugauua gaacagucca 80

<210> 2353
<211> 115
<212> RNA
<213> Homo sapiens

<400> 2353
uccugaucau gugcucagcc cuagcuuuag aagauggugc uauccaaacu ccaugaauga 60

acagugaagg aaggaccauu guucaaaagu aaguggaaau gcuguuucca ggaaa 115

<210> 2354
<211> 115
<212> RNA
<213> Homo sapiens

<400> 2354
uccugaucau gugcucagcu cuagcuuuag aagauggugc uauccaaacu ccaugaauga 60

acagugaagg aaggaccauu guucaaaagu aaguggaaau gcuguuucca ggaaa 115

<210> 2355
<211> 93
<212> RNA
<213> Homo sapiens

<400> 2355
uuuuuuuuuu uuauuuagag acggggucuu ucuacauugc ucaggcuagu cuugaacuuc 60

uaggcucaaa uuucuugauu ucuuccaaaa gua 93

<210> 2356
<211> 93
<212> RNA
<213> Homo sapiens

<400> 2356
ccuugucuuu cuccaccuuu cgucuuuggu cacagcgcca ucucaugguu agcugaaacu 60

uugaaagagg auucauucuu cagaggcaag gaa 93

<210> 2357
<211> 57
<212> RNA
<213> Homo sapiens

<400> 2357
guucuggcuc aagggcuaug aacagaaaug auauugggca cucuugggcc aaagcau 57

<210> 2358
<211> 138
<212> RNA
<213> Homo sapiens

<400> 2358

ucuugguugc cugucacugu uucuuccugc aucccguggg guuucuucgc ucucgucacc 60

uucugggaga agaacccugg ggaagaagcc aaaugcggau ggacagcagg auacacaauu 120

cucuagugcu cucaggga 138

<210> 2359
<211> 55
<212> RNA
<213> Homo sapiens

<400> 2359
agcucaggau gagagaguga gaagggaagg ggcuugccuc uaauccugau gcuau 55

<210> 2360
<211> 66
<212> RNA
<213> Homo sapiens

<400> 2360
ugcuggccau uagcgcgacu uggggguuau caguacacuu cuuguuugca gauggugaag 60

agcgcu 66

<210> 2361
<211> 52
<212> RNA
<213> Homo sapiens

<400> 2361
uguguauaua uguaucauuu guauauaaac aaauuuuaug uguguauaua au 52

<210> 2362
<211> 73
<212> RNA
<213> Homo sapiens

<400> 2362
uuggaaauca aguucuugag cacuuuugaa aacagacuaa uccaaaacau cugcaagaau 60

uuuaucuuua aaa 73

<210> 2363
<211> 93
<212> RNA
<213> Homo sapiens

<400> 2363
gugcuuccca aacugggguu ucccagaaca cuuuuucuaa gagagguuaa uagguaggcu 60

guucucacgg uuaaauuagu uugggaaaca ugc 93

<210> 2364
<211> 76
<212> RNA
<213> Homo sapiens

<400> 2364
ugacugccuu guccuuaacc agggcuaucu cucagaagca ggggccagcc ugguggacca 60

aaaacuggaa cucaac 76

<210> 2365
<211> 95
<212> RNA
<213> Homo sapiens

<400> 2365

uuccuaacac caaugaccca ugugcagaua acuaucgggg cucugcacca gaguccgaga 60

aagagacgaa agcugucacu aauuucauua gaagc 95

<210> 2366
<211> 97
<212> RNA
<213> Homo sapiens

<400> 2366
guccaguaga aggcuauuua auuuucacaa cugcuugaau uuugacauac aagaugaagc 60

aagaugccuc aagaaaugcu gccuacacug uggauug 97

<210> 2367
<211> 67
<212> RNA
<213> Homo sapiens

<400> 2367
gccacuacaa acaagcaacu gugggcgaca uaaauacagg uaugcagagc gggguugga 60

agggcau 67

<210> 2368
<211> 76
<212> RNA
<213> Homo sapiens

<400> 2368
cuuggugauu uaaaacaaua uguuuauuuu cuuccaguuu uauagaacag aaaucucaug 60

uagcucucac ugagag 76

<210> 2369
<211> 83
<212> RNA
<213> Homo sapiens

<400> 2369
agccuggaga ccagggcugg agcuugaagg ucacacugaa gucauggacu agaguuucaa 60

gucaaacuag ggauucaugc uac 83

<210> 2370
<211> 65
<212> RNA
<213> Homo sapiens

<400> 2370
uggcacaucu caccaggagc ucccccgagu cacccccacc agggcuggag cuugagcucg 60

ucacu 65

<210> 2371
<211> 64
<212> RNA
<213> Homo sapiens

<400> 2371
ugcagggcca accuaacggu cucugaugau auuaacaggc uggaccaggg cuggagcuug 60

cagc 64

<210> 2372
<211> 56
<212> RNA
<213> Homo sapiens

<400> 2372
uauuuuucaa acugaguguu uuagaagcca agguauagaa accagugaga ggugaa 56

<210> 2373
<211> 97
<212> RNA
<213> Homo sapiens

<400> 2373
uugaagcagg uguacagugu aaagcugccc uggaugagca guuugaaccu cagaagacuc 60

uguucaucca gugcgaugug gcugaccagc aacaacu 97

<210> 2374
<211> 68
<212> RNA
<213> Homo sapiens

<400> 2374
gcugcugagc uucgugcuua ucugaaaucu aaaggagcug agauuucaga agagaacucg 60

gaaggugg 68

<210> 2375
<211> 57
<212> RNA
<213> Homo sapiens

<400> 2375
cagcagaggc ucaucuuugc aggcaagcag cuggaagaug gccgcacucu uucugac 57

<210> 2376
<211> 54
<212> RNA
<213> Homo sapiens

<400> 2376
gaccagcaga ggcucaucuu ugcaggcaag cagcuggaag auggccgcag ucuu 54

<210> 2377
<211> 67
<212> RNA
<213> Homo sapiens

<400> 2377
cagccccgac cagcagaggc ucaucuuugc aggcaagcag cuggaagaug gccgcacucu 60

uucugac 67

<210> 2378
<211> 112
<212> RNA
<213> Homo sapiens

<400> 2378
acuaugcugg gcacuuugug auaucauuua acccucccgc aaaaagcauc auucucuuuu 60

gacagaugag guagcugaag uucaaagggg uaaugccacu uguccagggu ca 112

<210> 2379
<211> 97
<212> RNA
<213> Homo sapiens

<400> 2379
agcucuggua uuauggagaa cauuuguuua ugcuacaugg aaaaucucua acugugauuc 60

cauacaagug ugaaguguca ucauuagcag gugcucu 97

<210> 2380
<211> 65
<212> RNA
<213> Homo sapiens

<400> 2380
guuucuccuc ugauuuuuga gaauguaaau cagcaaguuc cuuucauucu uucugggaua 60

aaucu 65

<210> 2381
<211> 70
<212> RNA
<213> Homo sapiens

<400> 2381
aaccuggaag guaugacuau gucugugaau ccucauugga agaaguuuua uuggucagac 60

auccuguucc 70

<210> 2382
<211> 70
<212> RNA
<213> Homo sapiens

<400> 2382
agccuggaag guaugacuau gucugugaau ccuuauugga agaaguuuua uuggucagac 60

auccuguucc 70

<210> 2383
<211> 122
<212> RNA
<213> Homo sapiens

<400> 2383
gcuuauaaau cagaacaugu gguauuugau uucuuggucc uacaucaauu agcuuagaau 60

aauggccucc agcugcacug auguugcuac gaaauacaug aauucauucu uuuuuauagu 120

ug 122

<210> 2384
<211> 140
<212> RNA
<213> Homo sapiens

<400> 2384
guguuuaucu cccgcuuaua aaucagaaca ugugguauuu gguuucuugg uccuacauca 60

auuagcuuag aauaauggcc uccagcugca cugauguugc uacgaaauac augaauucau 120

ucuuuuuuau aguugcauag 140

<210> 2385
<211> 81
<212> RNA
<213> Homo sapiens

<400> 2385
cuugagcugg uucagcagcg ucguggcagg gcaggacagg acccugccag gcccugugca 60

cccucuccac agcuucaggc a 81

<210> 2386
<211> 97

<212>  RNA
<213>  Homo sapiens

<400>  2386
gccgcccgcg gcggcgggag aagacgccgc ggggcgcggg guuauguaau gguccccccu      60

aggcgucgac gccggcccgg cugcuggacc gcggugg                               97


<210>  2387
<211>  105
<212>  RNA
<213>  Homo sapiens

<400>  2387
ugaauagcua cucuuauuuc cauuauuaaa aucagguaga uggcagcaga aucuggagga      60

gauucacccu cuguugacca ucuucuguaa ggugcacuuc ucagu                     105


<210>  2388
<211>  131
<212>  RNA
<213>  Homo sapiens

<400>  2388
ggcaggcagu gguccugguu cggauauuug gcugaaaaca cagcuccucu uugucacccu      60

caggaagcca ucccaaaccg cucgaggagu ccuugaagcc aguguuacca cuaggacagc     120

aaguuugccu g                                                          131


<210>  2389
<211>  131
<212>  RNA
<213>  Homo sapiens

<400>  2389
ggcaggcagu gguccugguu cggauauuug gcugaaaaca cagcuccucu uugucacccu      60

caggaagcca ucccaaaccg cucgaggagu ccuugaagcc aauguuacca cuaggacagc     120

aaguuugccu g                                                          131


<210>  2390
<211>  63
<212>  RNA
<213>  Homo sapiens

<400>  2390
gcuugaagug ucaggagcca cacuggagac ccggugugac cucucacacu agaacuuagg      60

cag                                                                    63


<210>  2391
<211>  117
<212>  RNA
<213>  Homo sapiens

<400>  2391
gaggagggca aggcgaagcu gagagucccc ucuagccuug cugagauaug augccagggu      60

uuccuacgaa gacaggccaa gcuauagagg cuucagcuuc accuauaaac uguucau        117


<210>  2392
<211>  121
<212>  RNA
<213>  Homo sapiens

<400>  2392
uagaaaauug ugaauguaac augucuuucu aaguaggacu gcuaaaauau gucauuggcc      60

uuguucuguu uaaaaaacua aaacauguuu auuugaccca uaauagagaa aauauucuaa     120

a                                                                     121

<210>  2393
<211>  61
<212>  RNA
<213>  Homo sapiens

<400>  2393
gaaugauaau aacuuccugc cuucuggucu ugauuccuua ugguaguauu guacaguucc      60

a                                                                      61

<210>  2394
<211>  116
<212>  RNA
<213>  Homo sapiens

<400>  2394
cuggucccug uucaacacac ucuucaucaa uugcugcuuc cugagacuug ucacauaaaa      60

uuaacccaug aagucuaggg accaaaagau ggcaggccau gugacugggc ccagac         116

<210>  2395
<211>  108
<212>  RNA
<213>  Homo sapiens

<400>  2395
ugacacuugg ccuacugggc aaaccaucaa uacuaaagcu auaaacauga ccuuuauuaa      60

acagcuagaa aagauuguga aaugugcaga uaaaccagag acgucaau                  108

<210>  2396
<211>  97
<212>  RNA
<213>  Homo sapiens

<400>  2396
cugcacgugu uuguucuggg cgaauggcag cccaucgagg uacgacuaag aggauggugu      60

ugaccuagga aguguuuguc cugggcgaau ggcagac                               97

<210>  2397
<211>  60
<212>  RNA
<213>  Homo sapiens

<400>  2397
uuuaacaauc aaugccugug agccaguaug aaggggugca gcauaccgcu gacuuaggcc      60

<210>  2398
<211>  92
<212>  RNA
<213>  Homo sapiens

<400>  2398
caaaguacuu acaauguucu ggcacauagu aauuaauuaa gaaaaucgag cacccuuaau      60

uaccuagaau gcaggguugu uaguuuuuug gu                                    92

<210>  2399
<211>  55
<212>  RNA
<213>  Homo sapiens

<400> 2399
caccccauaa acaccaauuc uucuaucuug uucaggaaau ggugauuaug gugca 55

<210> 2400
<211> 113
<212> RNA
<213> Homo sapiens

<400> 2400
guccagggcg ugggggggcc gguuuguugu ggucgccauu uugcugguug cauuacuggg 60

uaaucggggc ccuggcuugc cgcguccgcc ggauacccuc agccaguggg cag 113

<210> 2401
<211> 53
<212> RNA
<213> Homo sapiens

<400> 2401
uugagagacu gagcuauaaa guaaauagcu uuauaguguu gucucauuua aga 53

<210> 2402
<211> 68
<212> RNA
<213> Homo sapiens

<400> 2402
uuuguacauu aucuucucau cuaauaccuc aaugaaaagg cccuggagag aauaaaggaa 60

aucaaaag 68

<210> 2403
<211> 66
<212> RNA
<213> Homo sapiens

<400> 2403
ggcagguccu cucauggagu guucaguucu cagcagagag gaggcccugg agagaauggc 60

ugcucu 66

<210> 2404
<211> 86
<212> RNA
<213> Homo sapiens

<400> 2404
gcagguccca cucccuccag aggcccugga gagaaucuau cuuugucucc ugguggcugc 60

ugagauuccu ugguuugugg cugcau 86

<210> 2405
<211> 104
<212> RNA
<213> Homo sapiens

<400> 2405
gcagagguug gugaggcccu ggagagaaug aggaaaacag gggaggcauu ugcugagacc 60

uggggucacu ucgugaagcu ggggccugga ggaaagggcu gcgg 104

<210> 2406
<211> 179
<212> RNA
<213> Homo sapiens

<400> 2406
cagccugcuc uccauccgag gccccauuuc uguacugagg ccuccagagc ugagcaagac 60

caaaggcccu ggagagaaug aagcuccgua gcaucaucuc cagguccucu ggugcucccc 120

ucccugaggu gucacaaagg cccaugguac aagaaagcac uuccaaugca gagugcugc 179

<210> 2407
<211> 52
<212> RNA
<213> Homo sapiens

<400> 2407
uuucaggccc uggagagaau cuuacauucc ugucuauuug guuauagaaa ag 52

<210> 2408
<211> 59
<212> RNA
<213> Homo sapiens

<400> 2408
uguuaucauc uggcucaguu ugugucucug uggaaacugg ggcccaagaa aaugauaca 59

<210> 2409
<211> 141
<212> RNA
<213> Homo sapiens

<400> 2409
auacauacua uuaaaugugu auaggccuuc caucaucugg cucaguuuaa ucuaccauua 60

guaucccauu gaaauguuca gcuguagcag aagaugccau gcuauuuuau aucuuuacac 120

auaacuaaau uuuuuguaug a 141

<210> 2410
<211> 97
<212> RNA
<213> Homo sapiens

<400> 2410
aguuugccaa ccugcuagga gccagauccu ggccuuugug uuucucugcc aucguggucc 60

acaucuguua ucaucuggcu caguuugugc uuauuag 97

<210> 2411
<211> 56
<212> RNA
<213> Homo sapiens

<400> 2411
cugcguccac ugcccaggcg acgcaagcua cagcggcguc aacgccuggg ucggcg 56

<210> 2412
<211> 61
<212> RNA
<213> Homo sapiens

<400> 2412
ucacacugga gcagcuggca cagcugggga cguuuagagc agcgugcuga cagugcugga 60

g 61

<210> 2413
<211> 56
<212> RNA
<213> Homo sapiens

<400> 2413
guuuuuuccu augagcacag cuccucucca gcgugcugac agugacacuu aaaccc 56

<210> 2414
<211> 105
<212> RNA
<213> Homo sapiens

<400> 2414
gggcucagag cagcuccccu cacccucgcc cucucuccgc caggccacca gcagcggcgg 60

gggcuggccc augaaugugu ggcugacucc caaccuccag cccuu 105


<210> 2415
<211> 57
<212> RNA
<213> Homo sapiens

<400> 2415
uccuaagcug gaugcaaaga aggugucaca gagccagcuc ggcaggcagc aaggagg 57


<210> 2416
<211> 76
<212> RNA
<213> Homo·sapiens

<400> 2416
gcuggggacc cagcuugugc ccuggcgucg uggccgccgg caggcagcaa ggaagggggu 60

guaaagagcc cgguua 76


<210> 2417
<211> 80
<212> RNA
<213> Homo sapiens

<400> 2417
ggcccgccgc ggcgcccagg cggcaggcag caaggacagc cuugugcgag cccgaucugg 60

gccuuccugg gcgcggcuac 80


<210> 2418
<211> 65
<212> RNA
<213> Homo sapiens

<400> 2418
gcagaugcaa uaacgguggu ucuguauggc aaacucccuu ccaugcauga ugcuggcucc 60

ugugc 65


<210> 2419
<211> 142
<212> RNA
<213> Homo sapiens

<400> 2419
guuuuccucc ccuuggggga uuguguugag ugggaggcaa cgggaaugca ggcuguaucu 60

gcagggcauu gugcuaacag gugcaggcug cagaccuguc acaggccgcu gagaccaggu 120

ucccuacugu ggcugaugau ca 142


<210> 2420
<211> 96
<212> RNA
<213> Homo sapiens

<400> 2420
gguaugugcu cauaguccua gccacugagg aggcugauac gggaggauug cuucagccca 60

ggaaguaaag guugcaguga ggugagauca uacccc 96

<210> 2421
<211> 69
<212> RNA
<213> Homo sapiens

<400> 2421
gcaccuguuc cuagucaugc ccagggaaua ugugaaacuc uggcucagga ugaacgucug 60

uugguguca 69

<210> 2422
<211> 54
<212> RNA
<213> Homo sapiens

<400> 2422
guuagucagu cugggagucc uggccguucg uguggccugg cucaggauga acaa 54

<210> 2423
<211> 68
<212> RNA
<213> Homo sapiens

<400> 2423
gugcauucaa cugccugugg acuagcagaa acuacuggug gagucagggg cucugcagca 60

gugcacac 68

<210> 2424
<211> 121
<212> RNA
<213> Homo sapiens

<400> 2424
gguuuccacc caacuuuaca aucugucaag gcugcuuuga aucugcuuca gucacccauu 60

guguuggagc ucccucccag ccagcagcca ucugcagggu gaagauggcu uccucuaccu 120

c 121

<210> 2425
<211> 60
<212> RNA
<213> Homo sapiens

<400> 2425
ucaguacuga ggcuauugaa ccaacaggca aaauucuguc aaggcugcuu uaaaauggca 60

<210> 2426
<211> 87
<212> RNA
<213> Homo sapiens

<400> 2426
ggcccuugug gagagcaucc ugugccagcc ucgcccccuc ccaguguucu ggggccccuc 60

ugucaaggcu gcuuucucca aggcuga 87

<210> 2427
<211> 63
<212> RNA
<213> Homo sapiens

<400> 2427
cauguuuuca acugucaagg cugcuuuuag aauccagagg aggccuguaa guacaaagau 60

gua 63

<210> 2428
<211> 81
<212> RNA
<213> Homo sapiens

<400> 2428
ugaaacugug ggcuugggag cucucaaauu auuauuauua uuauuauugu uauuuuuacu 60

cccaauggaa uauuguucaa u 81

<210> 2429
<211> 73
<212> RNA
<213> Homo sapiens

<400> 2429
uccagcucug gaguuggaac uuggcauuaa aaguauuuau aacgugugug aguagaaacu 60

gugggcuugg aaa 73

<210> 2430
<211> 98
<212> RNA
<213> Homo sapiens

<400> 2430
uauucuuaac acaagugucc acaucaggca guauagauuc aaggaccaug gcugggcaga 60

aacugugggc uuggugaagg augcacagga ggagugug 98

<210> 2431
<211> 96
<212> RNA
<213> Homo sapiens

<400> 2431
acccuuucag aguggugcuc caggggcaaa ggagauuagg agaaacuggc acagucuugc 60

gucaugaucu ggcauugugu cauuuggauu uggugg 96

<210> 2432
<211> 71
<212> RNA
<213> Homo sapiens

<400> 2432
gccgaucggg cgcgguacuc agccuuuuaa agcuugaggu gagaggggug ggguguuccc 60

ucuucugguu u 71

<210> 2433
<211> 66
<212> RNA
<213> Homo sapiens

<400> 2433
cuggagaaau agguuugccu ggguuaugcu acacggucaa gacugccagc cacuguucau 60

ucaggu 66

<210> 2434
<211> 66
<212> RNA
<213> Homo sapiens

<400> 2434
ggucaagacu gccagccuuu ggagaggaac uaccccauca gcucuccugg gucucuaguu 60

ggccaa 66


<210> 2435
<211> 89
<212> RNA
<213> Homo sapiens

<400> 2435
aggcauaccu cuguuacacu ucccagaaca gccucgagua guucugagag uugcuucgug 60

guagaguggu gucacucagg uguucuuuu 89


<210> 2436
<211> 54
<212> RNA
<213> Homo sapiens

<400> 2436
ucacuggcuu cugagacagc cucaggaugg gugugcuguu gacagguggu ggcg 54


<210> 2437
<211> 53
<212> RNA
<213> Homo sapiens

<400> 2437
ugucugggug ugcuugugau ccccuagccu ggcuucugag acagcagugc agc 53


<210> 2438
<211> 62
<212> RNA
<213> Homo sapiens

<400> 2438
cuuugcacau gacaucaacu cuuucuccag gcauagagaa gaaggggagu uaauguagag 60

au 62


<210> 2439
<211> 82
<212> RNA
<213> Homo sapiens

<400> 2439
gcaggaucag ggucgcggcc ugggaagcag gguuggucca ggcuugggcu ccuccgcguc 60

ccccgacccc caaccccugc cc 82


<210> 2440
<211> 70
<212> RNA
<213> Homo sapiens

<400> 2440
aggucggccu guuccuuuuc agcagcagac augggagggc ccagccugag cgcugagugc 60

gagcuccuuc 70


<210> 2441
<211> 90
<212> RNA
<213> Homo sapiens

<400> 2441
augucauggu gaagugguug uguugugaug uggugacguc guggugaugu guuguuguua 60

uggugaggug gugaugucau agugacguga                                    90

<210>  2442
<211>  58
<212>  RNA
<213>  Homo sapiens

<400>  2442
gugauguggu gacgucgugg ugauguggug uuguuauggu gagguaguga ugucauag      58

<210>  2443
<211>  82
<212>  RNA
<213>  Homo sapiens

<400>  2443
cgugaugugg ugguauagug gugaugucau ggugacgucg uggugaugug gugacgucgu      60

ggugaugugg ugaugucacg gu                                              82

<210>  2444
<211>  57
<212>  RNA
<213>  Homo sapiens

<400>  2444
uauuguuaga uggaguaugu ucucuuccca ccuaauucau accccaucug aaaugcu       57

<210>  2445
<211>  141
<212>  RNA
<213>  Homo sapiens

<400>  2445
acuuauaauc cuauuauuua auucuuucca gcugcugaca uauaguacau aauuucagau      60

guuuuaguau guuugaugaa uauuucuuuu uuuuucaauu uaccccaucu gaaauuacuu     120

cauagucuuu ccagcuaguc u                                              141

<210>  2446
<211>  102
<212>  RNA
<213>  Homo sapiens

<400>  2446
gccuauaaua ggaucuaucu uagagagugu uccauaugcu aaugagaaaa auguauauuc      60

ugcaguucuu gggucgaaca uucuguaaau uuuguuaggu ac                       102

<210>  2447
<211>  54
<212>  RNA
<213>  Homo sapiens

<400>  2447
uuaaaacauc cuugccucag agggacaaau cccauuugaa cauggcaagu aauc           54

<210>  2448
<211>  60
<212>  RNA
<213>  Homo sapiens

<400>  2448
gauaggaaca uguaacuauu uuuuguuucc auuaaacaac aauuaccuga uuuauauuca      60

<210> 2449
<211> 71
<212> RNA
<213> Homo sapiens

<400> 2449
acgaugggca uaaaacugug guuugcucca cagauuucug agcauuggcc guaguauacc 60

ccuggucgug u 71


<210> 2450
<211> 87
<212> RNA
<213> Homo sapiens

<400> 2450
uguuucuugu agcuuuugcu uugauuggaa uaauuuuacu cuaggaauaa aguuguguca 60

ucugcaaaaa ugaaucauau guacaca 87


<210> 2451
<211> 138
<212> RNA
<213> Homo sapiens

<400> 2451
gauauguauc uuuuuuaaau guuucauuca ggguaagcaa augagcaaca gcaagggaaa 60

aucacauuuu aguauuuuuc ucccuguuaa agaucguuuu gugcuuuuua acuaggauac 120

cagaaagcaa aguauuuc 138


<210> 2452
<211> 74
<212> RNA
<213> Homo sapiens

<400> 2452
aagcaaauga gcaacagcaa gggaaaaaca cauuuuagua uuuuucuccc uguuagacau 60

ugcuuggugc uuuu 74


<210> 2453
<211> 157
<212> RNA
<213> Homo sapiens

<400> 2453
gucucaaaaa aaaaauaagu aaaagccugc uucugacuau uagguuggug caaauguaau 60

ugcgguuuuu gcuaucacuu uuaauggcaa aaaccacaau uacauuugac caaccuaaua 120

uccuaaagaa caaagcugcc auaccugucc augacug 157


<210> 2454
<211> 117
<212> RNA
<213> Homo sapiens

<400> 2454
acaaccagcc ucagggcugu aauacguucu aaucacugaa aaauacuaau gcauaucucu 60

acuccucagu cuucuauuag guuggugcaa auguaauugc gguuuuugcu auuguuu 117


<210> 2455
<211> 159
<212> RNA
<213> Homo sapiens

<400> 2455
caguagaccc cagggagcag accacacuag aagaaguuau gagggacccg ugggugaaca 60

guggucagga guugccucug acaacaugaa gaacaaaucc uggaccaccu gaaucccaaa 120

acaacccagc uuuugguggc caugggauuc caggcugag 159


<210> 2456
<211> 52
<212> RNA
<213> Homo sapiens

<400> 2456
gcuaacaucu ucagggucug cacugugaac uccccuggag auaaguaagu cc 52


<210> 2457
<211> 79
<212> RNA
<213> Homo sapiens

<400> 2457
   auaaccaaaa gaguauaacu ggauuguuug ucacacaaga uaaugcuuga ggugauaggu 60
   acuccauuua uccuuaugc 79


<210> 2458
<211> 97
<212> RNA
<213> Homo sapiens

<400> 2458
gggcguggca agcagggagg caaagcucgc gccaaggcca agacccgcuc uucucgggcc 60

gggcuucagu uucccguagg ccgagugcau cgccugc 97


<210> 2459
<211> 97
<212> RNA
<213> Homo sapiens

<400> 2459
gggcguggua agcagggagg caaagcucgc gccaaggcca agacccgcuc uucucgggcc 60

gggcuucagu uucccguagg ccgagugcau cgccugc 97


<210> 2460
<211> 100
<212> RNA
<213> Homo sapiens

<400> 2460
gcggcaagca gggaggcaaa gcucgcgcca aagccaagac ccgcucuucu cgugccgguc 60

uccaguuccc cgugggccga gugcaccgac ugcuccgcaa 100


<210> 2461
<211> 76
<212> RNA
<213> Homo sapiens

<400> 2461
ucgcccucca gcugcuggag cugcagcccg accgcgagcg ugccaagcgg cuucagcagc 60

uagcggagcg guggcg 76


<210> 2462
<211> 76
<212> RNA

<213>  Homo sapiens

<400>  2462
ucgcccucca gcugcugcag cugcagcccg accgcgagcg ugccaagcgg cuucagcagc      60

uagcggagcg guggcg                                                      76


<210>  2463
<211>  155
<212>  RNA
<213>  Homo sapiens

<400>  2463
gagcucaagu cacagcugcc ccagguuaca guaaaugggg aaacccgguc uccuagcaag      60

gccaugucac ugggccuuga cucagaaagg caccagaggu auggauggga ggcugcuucc     120

uccuucagcc caugggaaaa ugaccuagag uucac                                155


<210>  2464
<211>  70
<212>  RNA
<213>  Homo sapiens

<400>  2464
augaucaagg ccugauaaua cacaaaauua auuaagaaau uuaguguagg ccgggcgcag      60

uggcucaugc                                                             70


<210>  2465
<211>  89
<212>  RNA
<213>  Homo sapiens

<400>  2465
ucugucccca cucuagcacc uguacaaugc ugcugccuuc aaagcccgga ccaaggcucg      60

aagccgugug cgggacaagc gggcagaca                                        89


<210>  2466
<211>  59
<212>  RNA
<213>  Homo sapiens

<400>  2466
aacuaugagg uuugucuuag uuuuuuauaa ggcuuuggca gaggccuaaa gcauguuuu       59


<210>  2467
<211>  104
<212>  RNA
<213>  Homo sapiens

<400>  2467
guugaaaucu aauguaaaug gaagauuuga uaugucaggu agaguuucaa augugucuaa      60

ugaaaggucc uauuuguuaa auccuagugc augugaauaa augu                      104


<210>  2468
<211>  75
<212>  RNA
<213>  Homo sapiens

<400>  2468
ucuuucauaa agagaccuca guuuauagcg uaacagacua cacaaugcac ugauguuuuc      60

auaauguuua aggga                                                       75


<210>  2469

<211>  149
<212>  RNA
<213>  Homo sapiens

<400>  2469
uuccuggugg gcugcagaca cuaauggugu uggggggucu uggaacagcu ucucuaugug      60

uggauucgug uaaaugcgaa gaguucacau auaaagaagu gacuuugauu cugugauuau      120

auugauuugg uacacaguaa augggaguu                                        149


<210>  2470
<211>  57
<212>  RNA
<213>  Homo sapiens

<400>  2470
uccggcacca gucagaccga uaugucaaaa uuaagguaug ugguccuggg auggaaa         57


<210>  2471
<211>  83
<212>  RNA
<213>  Homo sapiens

<400>  2471
cagcgcgcgc uauaccaguc cucgcaugug gacgaaaaug acgugcagac ggugucgcac      60

aagugccugg uggugggccu gga                                              83


<210>  2472
<211>  83
<212>  RNA
<213>  Homo sapiens

<400>  2472
cagugcgcgc uauaccaguc cucgcaugug gacgaaaaug acgugcagac ggugucgcac      60

aagugccugg uggugggccu gga                                              83


<210>  2473
<211>  80
<212>  RNA
<213>  Homo sapiens

<400>  2473
uuugugcccc cuaccugcca uccugauguc cccgcgugcc cgcagcgcgu gcuauaccag      60

uccucgcaug uggacgaaaa                                                  80


<210>  2474
<211>  76
<212>  RNA
<213>  Homo sapiens

<400>  2474
ccucggacca ugcuaggcca cuugcugggg ugucaaccua ccaccaaagg ggucuuuuag      60

caaaccucau ggggaa                                                      76


<210>  2475
<211>  100
<212>  RNA
<213>  Homo sapiens

<400>  2475
ggcugaugga agacuuggac cggaacaagg accaggaggu gaacuuccag gaguauguca      60

ccuuccuggg ggccuuggcu uugaucuaca augaagcccu                            100

<210>  2476
<211>  121
<212>  RNA
<213>  Homo sapiens

<400>  2476
augaccauua agcaauacgc auuccguaug augauuauua uguaaaucua aguauauuuc      60

augauuacua uguaauauau auuaucuauu gugauuauua uguaaaugca agugagcauc     120

u                                                                     121


<210>  2477
<211>  88
<212>  RNA
<213>  Homo sapiens

<400>  2477
uccuaugugg gcgacgaggc ccagagcaag agaggcaucc ucacccugaa guaccccauc      60

gagcacggca ucgucaccaa cugggacg                                         88


<210>  2478
<211>  88
<212>  RNA
<213>  Homo sapiens

<400>  2478
uccuaugugg gugacgaggc ccagagcaag agaggcaucc ugacccugaa guaccccauc      60

gagcacggca ucgucaccaa cugggacg                                         88


<210>  2479
<211>  83
<212>  RNA
<213>  Homo sapiens

<400>  2479
gcgccgcaga agggguccug gugacgaguc ccgcguucuc uccuugaauc cacucgccag      60

cccgccgccc ucugccgccg cac                                              83


<210>  2480
<211>  81
<212>  RNA
<213>  Homo sapiens

<400>  2480
uauaaucaac auugaauuau cuguuggcag auugaacuuu uagagaauca aaugcuaacg      60

aaaaauuuaa acaaguauau u                                                81


<210>  2481
<211>  143
<212>  RNA
<213>  Homo sapiens

<400>  2481
uggggucccg cauggcuaaa uaacuccuug gcgccuccug cucugcugug ccgccagggc      60

cuccccuagc gcgccuucug gagaggcuuu gugcggauac ggggcuggag gccugguggg     120

gcuguguccg gggcucgcc ggg                                              143


<210>  2482
<211>  84
<212>  RNA
<213>  Homo sapiens

<400> 2482
gccccaaggc aggccacgcu cauuucucuu uccccuugua cucguagccu ccagagguga 60

aguccccagu ggagccuaug gcua 84


<210> 2483
<211> 99
<212> RNA
<213> Homo sapiens

<400> 2483
uuaugauaga guguucagcc agagauauuu uaguuaaggu auguuuagcu uuuguauacu 60

aguuaaggua uguuuagcuu uucuauacuu uacauaaaa 99


<210> 2484
<211> 118
<212> RNA
<213> Homo sapiens

<400> 2484
ggccggaggc ggugcuggcg guggcggcgg cagccguggc ccggcggccu gggacagaau 60

cagagucaac gguggccucg gcggcggcgg cggcugccgc ggcgguggcg acggccgc 118


<210> 2485
<211> 57
<212> RNA
<213> Homo sapiens

<400> 2485
ccggaggaga gcucgcugcg caugcgcagg gagagcgcgg ccggcgcucc agcgguc 57


<210> 2486
<211> 65
<212> RNA
<213> Homo sapiens

<400> 2486
ugacugaaug auacucuucu guucuucaag gcaggagacc gggugauggu guugaaccgg 60

ucagg 65


<210> 2487
<211> 86
<212> RNA
<213> Homo sapiens

<400> 2487
gggcccgggg cuccaggggg gcgagagcgg cgcgaauccc gcggggccca cugcgcuagg 60

cccccgcugc cuguccgcgg uuccca 86


<210> 2488
<211> 90
<212> RNA
<213> Homo sapiens

<400> 2488
ggcccccgcu uccgccgggc cccgcugagc ucuagacaaa ccuccgcuuc agaaauaggc 60

ugcgggcggc cggcuaggag gcuuggcccc 90


<210> 2489
<211> 107
<212> RNA
<213> Homo sapiens

<400> 2489
ggcagggcuc caggcguccc cgaaugcucg ucguggaguu uuccugaucc ugucgggucc 60

cuaaggaacg cuuggcgggg agcguggacg cccuggggcc aggccac 107


<210> 2490
<211> 119
<212> RNA
<213> Homo sapiens

<400> 2490
ccuugauuuc cuuugcucug agcagcuucc uucaggcaca ggggggugua gaucuugaug 60

ggcuuaggcu cugcuuugcc cagggggagc gggaggggag ggaaugcucu uuagaggaa 119


<210> 2491
<211> 82
<212> RNA
<213> Homo sapiens

<400> 2491
gccaucagug gcuucacugu aguacacgga gaugcggucc agcugcaggu cgcugucccc 60

gugguaggug ccaguggggu cg 82


<210> 2492
<211> 86
<212> RNA
<213> Homo sapiens

<400> 2492
auuuucagua gccaguauuu cugauuaacu aaguugaaac ucuuauuaga aacuuucagu 60

uggugauauu guauucuaga agauau 86


<210> 2493
<211> 146
<212> RNA
<213> Homo sapiens

<400> 2493
agaggcaggc cggggccaag ugcuagugcc ccgagugcug gcacuguuga ccaaguuggu 60

ggcggaggug aaguggccuc uaccuugggg ggcagagggu guaugcaggg aggcaggcgg 120

uagccauggc ggggcuaacc cucucu 146


<210> 2494
<211> 76
<212> RNA
<213> Homo sapiens

<400> 2494
ccucugccca gcuugggccc agcugggcuc ugccacccua ccuguucagu gucccgggcc 60

cguugaggau gaggcc 76


<210> 2495
<211> 73
<212> RNA
<213> Homo sapiens

<400> 2495
uugucugugu gacccugggc aagucauuuc cccuccuugg gacucaguuc cuugccugug 60

aacggggaca gug 73

<210>  2496
<211>  118
<212>  RNA
<213>  Homo sapiens

<400>  2496
uaugucagau guccauaccu ggguaaagga aaugaagaua aucuuaauuu guucagugcu      60

uugagggaau uuucuguaga uuuucuuuuu agcucuaguu uacaauguca cccauaag      118


<210>  2497
<211>  86
<212>  RNA
<213>  Homo sapiens

<400>  2497
cucaucacug agccaggagc uguggacacu gcaauggcca gugcagacuu agugccuguu      60

cuuguggacu uuacagugga ggagag                                           86


<210>  2498
<211>  94
<212>  RNA
<213>  Homo sapiens

<400>  2498
agcauguccc ccagaacucg gggagcgcag gcaggacagg cuuagagaag acgcgguccc      60

cagcgcuugg gccacggacg ucccaccccg cucc                                  94


<210>  2499
<211>  170
<212>  RNA
<213>  Homo sapiens

<400>  2499
uaggcacuca aauauuuuug cuguuuuugu uauuauuaag cuaauaagcc aauuugguac      60

cacugacaca gucugcuauu uaaaugugau uucauguguu ggugacuuuu uguuuguuug     120

uuuaauuugu ccuagcugca guuuaaacag cauugaaguc agaaccuggu                170


<210>  2500
<211>  113
<212>  RNA
<213>  Homo sapiens

<400>  2500
cuuucuaaag aguuuuuaug agugauuuuu gcaauaaaau auucuuuuuu cuguauaucu      60

uucaggcauu aagggaucau ugcaagagca gcgugacuga cauuaugaag gcc            113


<210>  2501
<211>  58
<212>  RNA
<213>  Homo sapiens

<400>  2501
aaaaguaauu gcaguuuuug ccauuucauu ggcaaaaacc gcaauuauua uuauuuuu        58


<210>  2502
<211>  59
<212>  RNA
<213>  Homo sapiens

<400>  2502
uauuugucag acuuuuccuu uuuaauaugu agugaugaag gcccagaagu aggaguguc       59

<210> 2503
<211> 84
<212> RNA
<213> Homo sapiens

<400> 2503
gucacagcuc ugcucggcuu cuuuugcaaa ccggucacac caugugggaa ggaaaauagc 60

cauugaggua gcuuuuggug gccu 84


<210> 2504
<211> 57
<212> RNA
<213> Homo sapiens

<400> 2504
ucucagcaau cagacugucg acauuccaga aaaugucgac auuacucuga agggacg 57


<210> 2505
<211> 58
<212> RNA
<213> Homo sapiens

<400> 2505
gggcaggcug augagacuaa ggcgaaugcg acuccgugcu cucuggcccu uggcuccu 58


<210> 2506
<211> 81
<212> RNA
<213> Homo sapiens

<400> 2506
gaggauggcc acugaugugu guuuguggau accugggcau cgacccagcc ugcaggugag 60

cucugggcca uguugccuug a 81


<210> 2507
<211> 101
<212> RNA
<213> Homo sapiens

<400> 2507
uucuagagau aaucuccagu cccugugcag aaacugucau ugcacuuucu gcugaaaugg 60

caguuucuuc ucagcaaggu gagauuaugg aauccagaau c 101


<210> 2508
<211> 75
<212> RNA
<213> Homo sapiens

<400> 2508
aaggugggcc agagcuuccu gagguaucag gcugaaguga gccuccagcu gagaccacag 60

ucuugcuuag uuuuc 75


<210> 2509
<211> 72
<212> RNA
<213> Homo sapiens

<400> 2509
uccugcucuc cccuagggcg cgcgucacaa ucucuggucc cccguagacg ccgccgcggg 60

gaaggcgggg cu 72


<210> 2510
<211> 54

<212>  RNA
<213>  Homo sapiens

<400>  2510
aagcuccuuu cucagacagc cacucauucc ggggcccugg acgugggagc uuuc          54


<210>  2511
<211>  122
<212>  RNA
<213>  Homo sapiens

<400>  2511
ggccuccuuc ucagguucag cucuguucag cuccuugggg aucuccuguu ucacaucuca     60

ggagucacug ggaagaugac cacagaaacu gccucugagg augauaacuu uggaacugcc    120

ca                                                                   122


<210>  2512
<211>  81
<212>  RNA
<213>  Homo sapiens

<400>  2512
uagccccaca auuugaugga aauggcaggc uguuguauca ugugggacaa uggcucauuc     60

ccauugaguu gugugguuag u                                               81


<210>  2513
<211>  81
<212>  RNA
<213>  Homo sapiens

<400>  2513
uagccccaca auuugaugga aauggcaggc uguuguauca ugugggacga uggcucauuc     60

ccauugaguu gugugguuag u                                               81


<210>  2514
<211>  62
<212>  RNA
<213>  Homo sapiens

<400>  2514
ucucggagga gcaguucugg gaggccugcg ccgagcucca gcagcccgcu cuggccgggg     60

cc                                                                    62


<210>  2515
<211>  58
<212>  RNA
<213>  Homo sapiens

<400>  2515
ucucugaggc ccuggcaucu uuuaauguaa guuguaugga gcuggaagga aggagagu       58


<210>  2516
<211>  75
<212>  RNA
<213>  Homo sapiens

<400>  2516
ggggaagcug caggaguucg gcguggguga uggcagcaag cugaccuugg uacccaccgu     60

ggaagcgggc cucau                                                      75


<210>  2517
<211>  133

<212>  RNA
<213>  Homo sapiens

<400>  2517
gggucaccua cugguuuuca uggcauugau uuauaguucc ccuuccuguu uuuguuuccu      60

caagaggcac ugagcauuac augacuuucu uucuuuaacu cugccuguga uaagcaggag      120

gaagaggacu cug                                                          133


<210>  2518
<211>  70
<212>  RNA
<213>  Homo sapiens

<400>  2518
ugcuggcgga gcagcugcgg cgcgacgcgg agggcggccc gggcgcgugg cggcugucac      60

gggcggcggc                                                              70


<210>  2519
<211>  119
<212>  RNA
<213>  Homo sapiens

<400>  2519
gcgccccucu gggugacuug gccgugagaa agugaaagga gcccgcaggc ggcggcgaca      60

gcugcgcugg gaggggcuug uuuccaggcu cagaggcccc gcgcacucug cgggugcgg      119


<210>  2520
<211>  56
<212>  RNA
<213>  Homo sapiens

<400>  2520
caggaacagc gagaggggag gaagugcuac acacuuuuuu uuuuuuuuuu uuugag          56


<210>  2521
<211>  111
<212>  RNA
<213>  Homo sapiens

<400>  2521
ccuugaggca ggcuggcuuc cucaccaccu ugugcacugc ugcucccaca ccacagugac      60

uagcagcaca uaaugguuug aauuaaagcu gaaguaaaaa auauccaggu c               111


<210>  2522
<211>  137
<212>  RNA
<213>  Homo sapiens

<400>  2522
cggaugugcc gccgcuuuuu aguuucugag gcgccggcgg uguuccuggu agcgucagcc      60

uccuggcuuu uccuuuuccu ggacgcuuca gggcuccgcg gccugcgcua gacuggaggg      120

uccgcgucac ucucgcc                                                      137


<210>  2523
<211>  93
<212>  RNA
<213>  Homo sapiens

<400>  2523
ggaugcugcc cugcuacggu auacacaaug acaccuaccg caaagaagua ucuggcguca      60

aacaucagua gugcccaagu ugggcagcuc ugc                                    93

<210> 2524
<211> 124
<212> RNA
<213> Homo sapiens

<400> 2524
gcuguguacc uggguggaua ugugcacacu ggaauguggg ucccugugug agguuguaac 60

auuugcuugc augugaggau gcauaucuau guguaaaugc ucucaugucu ugcauguuua 120

gugu 124


<210> 2525
<211> 148
<212> RNA
<213> Homo sapiens

<400> 2525
cccaggccga ggaaccccca gcccagggac uagacaccac cagggccggg cggagccaga 60

gccagacgcg ccggagcggg cgccucuacg ccgugagucc gagucugggg cccgaggcgg 120

gguggggcuu ggggcucagc cgcgggaa 148


<210> 2526
<211> 91
<212> RNA
<213> Homo sapiens

<400> 2526
ggcccagcca gcugugccgg cguuuguugg cugcccugcg cccggcccuc cagccagccu 60

ucugccggcc ccgccgcgau ggaggugccc c 91


<210> 2527
<211> 94
<212> RNA
<213> Homo sapiens

<400> 2527
ucgaguacau ucgaugauga uuccauucga guacauucga uggugauucc auucgaugcc 60

auucgaugau gauuccauuc uauuccauuc gaug 94


<210> 2528
<211> 76
<212> RNA
<213> Homo sapiens

<400> 2528
ugagguacau auaucuaaug gaguaaucac aagauacaga ugccugcuuu ucugguucuc 60

uguuuaagac uucacu 76


<210> 2529
<211> 73
<212> RNA
<213> Homo sapiens

<400> 2529
ggcaguguag cgagaaaggu uuucgccucc uguuucagcg gugacggcuc uuggguuuuc 60

ucgggguggc uuu 73


<210> 2530
<211> 73
<212> RNA

<213>  Homo sapiens

<400>  2530
ggcaaugucg cgagaaaggu uuucgccucc uguuucagcg gugacggcuc uuggguuuuc      60

ucgggguggc uuu                                                        73


<210>  2531
<211>  72
<212>  RNA
<213>  Homo sapiens

<400>  2531
ggcaugucgc gagaaagguu uucgccuccu guuucagcgg ugacggcucu uggguuuucu      60

cgggguggcu uu                                                         72


<210>  2532
<211>  65
<212>  RNA
<213>  Homo sapiens

<400>  2532
uuaccaaggu gacauaugcc acucaggggg acgcucccag ugccaucagu gugaacagug      60

ugaca                                                                 65


<210>  2533
<211>  136
<212>  RNA
<213>  Homo sapiens

<400>  2533
ggggucaguc agucgcuccc ugucgcugcc ggagagucuc ugcuuccccc uuccuacgcg      60

cuccgcggcg guagcucggg cucuccggag gagggggcga cagaguaaaa gagaggcuga     120

cuuaauaaag uuuccu                                                    136


<210>  2534
<211>  73
<212>  RNA
<213>  Homo sapiens

<400>  2534
ggagggaugg ugugggauuu uggccgaggg aagcaggaca gagaaggagc aggaagcuau      60

gcuaauuuuc cug                                                        73


<210>  2535
<211>  134
<212>  RNA
<213>  Homo sapiens

<400>  2535
gaaggaaaaa ugcaagagaa agaggagcac caggagaucu aucaugccga ggcugcagcu      60

gugcaggucg gaagacaggc ugcagcacgu ggcgggaccu gcccuggagg cucuuagcug     120

uggggagucu ucau                                                      134


<210>  2536
<211>  55
<212>  RNA
<213>  Homo sapiens

<400>  2536
gaaguauauu cuguaguagc ucuucaaaaa ggggagcaug gaggauagau uuuug          55

<210> 2537
<211> 67
<212> RNA
<213> Homo sapiens

<400> 2537
uggauuaggg cccacccaga ugagcucauu uuaccuaaau gaccucuuuc aggacccaac 60

cuccaaa 67


<210> 2538
<211> 89
<212> RNA
<213> Homo sapiens

<400> 2538
cuccacaaua gggguaucaa uggcaucagg gaaggccaua uuguccuaaa acacuucuau 60

aagauuccau cuccauucau uugggagaa 89


<210> 2539
<211> 75
<212> RNA
<213> Homo sapiens

<400> 2539
aggcuccucc ccuaauccua aucucauggc uuuucauuag uuuuuggucc cugagcaagg 60

agggggugag uuuua 75


<210> 2540
<211> 85
<212> RNA
<213> Homo sapiens

<400> 2540
gguuuuuggu cccugagcaa ggaggggguu aguuuuaugg agggauuuac uuuuacccuu 60

gcuuuuaagu uaaacuauaa acuau 85


<210> 2541
<211> 70
<212> RNA
<213> Homo sapiens

<400> 2541
   aauccuaauc uuguggccuu ucauuaccaa gacaaaggcg auuuuggucc cugagcaagg 60
   aggggguucg 70


<210> 2542
<211> 79
<212> RNA
<213> Homo sapiens

<400> 2542
   ggccccccua uccuaaucuu guggccuuuc uuuaguuuua aaaagguggu uuuugguccc 60
   ugagcaagga ggggguuag 79


<210> 2543
<211> 107
<212> RNA
<213> Homo sapiens

<400> 2543
uaauccccug cuacuuguua gaggagcugg augacacuuc gugggcucau ucucauaaau 60

aagcugcuga auggaguccu cuccucccaa gaguugucgg uguugca 107

<210> 2544
<211> 70
<212> RNA
<213> Homo sapiens

<400> 2544
uuugugacag gcuggagugc aguggcacga ucucagcuca cuguaaccuc caccucccag 60

guuccaagcu 70

<210> 2545
<211> 71
<212> RNA
<213> Homo sapiens

<400> 2545
uccccgcccu aaguucucug uguguguccu gggggaccag cagcacugug acgaggcuaa 60

ggccguggau a 71

<210> 2546
<211> 125
<212> RNA
<213> Homo sapiens

<400> 2546
ucucuuccau uguagagcaa auccaucccc acacccuguu cacuccuuug cugauugguu 60

ucguaaucgu agcuggcaug augugcauua uugugaugau ucugaccuac aaauauuuac 120

aggua 125

<210> 2547
<211> 69
<212> RNA
<213> Homo sapiens

<400> 2547
gccuggccug ccucaccaac cugggugacg uccacgucuu cucggugccu ggccugcggc 60

cccaggugc 69

<210> 2548
<211> 71
<212> RNA
<213> Homo sapiens

<400> 2548
ccugaggccu ucccagaagc ugaaggaauu uggcgcuaug cuuccuauac agccuccaga 60

acccugaggc a 71

<210> 2549
<211> 89
<212> RNA
<213> Homo sapiens

<400> 2549
gggagugagu gcaaaaagcu gaguauggug agagaagccu gugcccugau ccaaguuuac 60

ucaacccucu caggugacca aaaucccccu 89

<210> 2550
<211> 72
<212> RNA
<213> Homo sapiens

<400>  2550
ggccggcggg acgggagcug cggcgcuggc uacgagagug acccagucag cguugguucc      60

cgucuuggcc au                                                          72


<210>  2551
<211>  62
<212>  RNA
<213>  Homo sapiens

<400>  2551
ugcuuucccc acauucugug agcuguguuc ccacaccugg aaggaaggag gaggagagcg      60

aa                                                                     62


<210>  2552
<211>  65
<212>  RNA
<213>  Homo sapiens

<400>  2552
cccgggggcg gcagagagga agaggaggga gaggagagcg guccggaagc cgccgagccc      60

gggca                                                                  65


<210>  2553
<211>  133
<212>  RNA
<213>  Homo sapiens

<400>  2553
aggaaguagg caguccaguc cugaccugcu cggaguugaa gcagucggcg uuggaagagc      60

cugaacaccc ggccuaccac uuguccuucc aucucugggc cuucaggaca gagcggacuc     120

cccucucccc uaa                                                        133


<210>  2554
<211>  103
<212>  RNA
<213>  Homo sapiens

<400>  2554
uuaguagcug uggaggaaga ugcagaguca gaagaugaag aggaggagga ugugaaacuc      60

ugaaguauau cuggaaagcg gucugccccu ggagguggua gca                       103


<210>  2555
<211>  97
<212>  RNA
<213>  Homo sapiens

<400>  2555
uuagcugugg aggaagaugc agagucagaa gaugaagagg aggaggaugu gaaacucuua      60

aguauaucug gaaagcgguc ugccccugga gguggua                               97


<210>  2556
<211>  103
<212>  RNA
<213>  Homo sapiens

<400>  2556
uuaguagcug uggaggaaga ugcagaguca gaagaugaag aggaggagga ugugaaacuc      60

uuaaguauau cuggaaagcg gucugccccu ggagguggua gca                       103

<210> 2557
<211> 60
<212> RNA
<213> Homo sapiens

<400> 2557
ucuuaaguau aucuggaaag cggucugccc cuggaggugg uagcaaguuu ccagaggaaa 60

<210> 2558
<211> 65
<212> RNA
<213> Homo sapiens

<400> 2558
uucugaccaa uauucacccu caaccuuauu uuuauaauag augaccgugg aaaaaguaca 60

gggua 65

<210> 2559
<211> 99
<212> RNA
<213> Homo sapiens

<400> 2559
ugcugaaaau gcaaaaauua gcuggucgug guggccucgg ccucccaaag uguugggauu 60

acaggcguga gccucugcuc ccggccuaau uuuuguauu 99

<210> 2560
<211> 125
<212> RNA
<213> Homo sapiens

<400> 2560
uucucucuga ugcuucaguc acgacaugaa ucuauaaggu gcuuugaaag gaauaauaau 60

ucuuucaaau aauuguacuu ccauuuaauu cauuauaguc aaacuauuag uaaucaaaga 120

gaaua 125

<210> 2561
<211> 63
<212> RNA
<213> Homo sapiens

<400> 2561
ccaggugggu gugggcaggg gcugcauuuu augaaacugc ccagcaagcu gcgcucccug 60

ggg 63

<210> 2562
<211> 92
<212> RNA
<213> Homo sapiens

<400> 2562
ccgcggcgcg gcgcugcccc ugagagggag cggcggcggc cuccuccgcc ccgaguccuc 60

gcucgggggc cgcgcgagga cggcgcccgg cc 92

<210> 2563
<211> 68
<212> RNA
<213> Homo sapiens

<400> 2563
uccugaugcc cguugucuag gagacagagc caagcacugc ucacgucucu gccgccugcg 60

uuuggagg 68

<210> 2564
<211> 67
<212> RNA
<213> Homo sapiens

<400> 2564
ucggcagagg cugacccgcg gcugauugag ucccucuccc agaugcuguc caugggcuuc 60

ucugaug 67


<210> 2565
<211> 95
<212> RNA
<213> Homo sapiens

<400> 2565
ugccaauuuc ugguuggaau agugacaaca ugcuggagcc aagugcuaac augccuuggu 60

ucaagggaug gaaagucacc cguaaggaug gcaau 95


<210> 2566
<211> 115
<212> RNA
<213> Homo sapiens

<400> 2566
gggcuuugcc uugaaacccg ggacgccagg ggcgcucccg caaguggggg uccuccggga 60

cuuggaacgc cccggcuggg ugguguccgg gcguccuuuc cccgcuucuu cccac 115


<210> 2567
<211> 109
<212> RNA
<213> Homo sapiens

<400> 2567
ugcugcccga gcugggcgug cacggacagc ugcgccuggg gaccgcgugc gugcuaaucg 60

ugggcugcgg ugggcucggc uguccacuag cgcaguacuu ggcagcggc 109


<210> 2568
<211> 138
<212> RNA
<213> Homo sapiens

<400> 2568
gccgcgccgc ucuugccgug ggugccgacg gcggcgccgg gacgggcgug gggguaggg 60

cgagucauau gauccgcucg gcuuccuggg ucuggcugcu gccgcccgcc gguguccgcc 120

cgugucgcgc cggggcac 138


<210> 2569
<211> 99
<212> RNA
<213> Homo sapiens

<400> 2569
gcaaauuugu cauuuccaaa auacugguuc uuuuuuuucu uucuuuuuua cuacuucuuu 60

agaaaaagaa ccaguauuuu ggaaaugaca aauuuguac 99


<210> 2570
<211> 97
<212> RNA
<213> Homo sapiens

<400> 2570
caaauuuguc auuuccaaaa uacugguucu uuuucuaaag aaguaguaaa aaagaaagaa 60

aaaaaagaac caguauuuug gaaaugacaa auuugcu 97


<210> 2571
<211> 122
<212> RNA
<213> Homo sapiens

<400> 2571
guguucucca cgccgcacgg gcucgaggug cacgugcgca ggucccacag cgguaccaga 60

cccuuugccu gcgagaugug cggcaagacc uucgggcacg cggugagccu ggagcagcac 120

aa 122


<210> 2572
<211> 53
<212> RNA
<213> Homo sapiens

<400> 2572
ugccgcgcgc gcgcuccccc cugggguucc ggauggugag cggcgaaggu aag 53


<210> 2573
<211> 77
<212> RNA
<213> Homo sapiens

<400> 2573
uguggaugga agauacgcuu augugguguu gaggaaaaca gacauugacc ucaccaagag 60

ggcgggagac ucacgga 77


<210> 2574
<211> 169
<212> RNA
<213> Homo sapiens

<400> 2574
auauucuagu aaagauuugg ggaggggagg guagccacag aaagaaucuu guuuaucuaa 60

agcaggaacu ugaucuuggu uuugcuggau ugcuuuccau agacauagau ucuuauuaaa 120

uauuuguuuu gguacuaauu auuuuaauua uucuuuaauu agaguguua 169


<210> 2575
<211> 66
<212> RNA
<213> Homo sapiens

<400> 2575
ccuagggcuc uuccuguggc cccugacccc ucaaauuggc caggaagggc aggagagccu 60

ugggca 66


<210> 2576
<211> 79
<212> RNA
<213> Homo sapiens

<400> 2576
cagaguacag uugugaauuu ggcuccgcga aguauuaugc acuguguggc uuuggugggg 60

ucuuaaguug uggucugac 79


<210> 2577

<211>  106
<212>  RNA
<213>  Homo sapiens

<400>  2577
ccuguguugg ccaauggaau gugagcagac agggugugcg cagaggcguu aaaugcgcuu      60

gcaagguucu ucucucuugu gcuccuuuca uuccugaaga caggag                    106


<210>  2578
<211>  64
<212>  RNA
<213>  Homo sapiens

<400>  2578
cauuugcagc cugcaggggc cuggcaaucu uuuuugcuac cuguuuacag ccuggaacca      60

ugau                                                                  64


<210>  2579
<211>  116
<212>  RNA
<213>  Homo sapiens

<400>  2579
aagcccagca uuuugcagcu uugaagcugc uggggaucac gacugucucu guggcccuua      60

ccuggccaca gauguacccu ggcaacccgc ucacagugcc cugcgugcag guuuau         116


<210>  2580
<211>  53
<212>  RNA
<213>  Homo sapiens

<400>  2580
uauacuuuua auuuuagaau aguuuuaaau uuguggaaag uugugaagau agu             53


<210>  2581
<211>  125
<212>  RNA
<213>  Homo sapiens

<400>  2581
ugaaagagua gugcaguagu ucaggucuga gugugugaug uguuggagga ugggcaauug      60

uccauuuaag uaucagugga uaauuguuau uuuuaaaacu ucacauaguu ugauucucuc     120

ucaaa                                                                 125


<210>  2582
<211>  125
<212>  RNA
<213>  Homo sapiens

<400>  2582
uggcaguuau uuuauuaggu cggugcaaaa guaauugcug uuuuugccau uaaaaauaau      60

ggcauuaaaa guaauggcaa aaacggcaau gacuuuugua ccaaucuaau aucucugcug     120

cuaua                                                                 125


<210>  2583
<211>  89
<212>  RNA
<213>  Homo sapiens

<400>  2583
auaguuaagu gauuuaaaga cugcguguca agauuccaua auguuauaag guuauuuuaa      60

aguacucauu cuuuucacca augcuguuu 89

<210> 2584
<211> 80
<212> RNA
<213> Homo sapiens

<400> 2584
cuuguguucc agguggcaga gaugcauggc gagcugauug aguucaacga gcgccugcac 60

agggcccugg uagccaagga 80

<210> 2585
<211> 52
<212> RNA
<213> Homo sapiens

<400> 2585
gcggcacuac cugcugaagc gccugcgcaa ggccaagaag gaggcgccgc cc 52

<210> 2586
<211> 90
<212> RNA
<213> Homo sapiens

<400> 2586
uagguaauuc cuccacucaa aacccuucag ugacuuccau gacaugaaau aggaagucau 60

uggaggguuu gagcagagga augaccuguu 90

<210> 2587
<211> 183
<212> RNA
<213> Homo sapiens

<400> 2587
cccuguggcg ugcggcgggg ugagaggggc ggggccugcg cgcgagggac ccgggacgug 60

cgagcgggcg aagucggcgc uuggggugge ucuggucccu aagucgcuug ggcuucgccc 120

gaucccugca caucccuacu ccccgccguc gucccccuuc ccagggcccc agagccuagg 180

guu 183

<210> 2588
<211> 57
<212> RNA
<213> Homo sapiens

<400> 2588
gggacaccac uacuggacac ggucuuguag aggcagcugu ccaguuuugg ugcuuga 57

<210> 2589
<211> 136
<212> RNA
<213> Homo sapiens

<400> 2589
ggucaguugc uuuugggaga ccgcaguggu cccucggucu uaucuuuuag augugguuga 60

aguaaacccu gccaguuuaa uuaaccacua agcgguuccc uucacuccug ccucccuccc 120

ucccucccuc ugccag 136

<210> 2590
<211> 143
<212> RNA
<213> Homo sapiens

<400> 2590
gcuggcucug cccaccuguc ccgcuggccu ggcaggugac ggugcuggau guggccuuuu 60

ugccuuuucu aaaggccaca uuuuccagcc cauucaaccu uccagagccc ucugaagugg 120

ccacaggccc caucaaccag uga 143

<210> 2591
<211> 74
<212> RNA
<213> Homo sapiens

<400> 2591
agucaggggc agagcagggc ggucugagcg ugcgggcgac gcgggucuca cucguccgcu 60

ccgcucugga cugc 74

<210> 2592
<211> 83
<212> RNA
<213> Homo sapiens

<400> 2592
uggccggggu cagcuacgcg gcgcccuggu gggugagccu ccugcaccgg cugccccacu 60.

ucgaccugag cugggaggcc acu 83

<210> 2593
<211> 111
<212> RNA
<213> Homo sapiens

<400> 2593
ucuguuuuga uuuucuaagg cauauagaua gauuugucag ucuuuaccuu uaugguuugu 60

acuuauagaa uuuuguuuga aauccuuccu cacccugaau ucauacagau a 111

<210> 2594
<211> 147
<212> RNA
<213> Homo sapiens

<400> 2594
gcccaguucc uaagggugca acuuacagca agccugucca ucaugguguu aaccagcuaa 60

aguuugcucg aagccuucag uccguugcag aggagcgagc uggacgccac cauggggcuc 120

ugagcguccu gaauucuuac aggguug 147

<210> 2595
<211> 120
<212> RNA
<213> Homo sapiens

<400> 2595
aggguguaac uuacggcaag ccuguccauc augguguuaa ccagcuaaac uuugcucgaa 60

gccuucaguc cuuuguagag gagcgagcug gacgccacuu uggggcucua agaauccuca 120

<210> 2596
<211> 95
<212> RNA
<213> Homo sapiens

<400> 2596
uccaggccau cacagagcca ugaaccagca gcacaugaug ccuucccaag ccuuccagau 60

gcggcguucc cugccuccag augacaucca ggaug 95

<210> 2597
<211> 62
<212> RNA
<213> Homo sapiens

<400> 2597
gccacugucu cuagucuggg ugacuacaga ugcccagagc ccagacugga cuagcaaggc 60

cc 62


<210> 2598
<211> 55
<212> RNA
<213> Homo sapiens

<400> 2598
gcagcgcagg gaagccuggg ggccagaggu cgccgcugcc gccaugccgc ugcuc 55


<210> 2599
<211> 124
<212> RNA
<213> Homo sapiens

<400> 2599
ggcagcccua gucccagcac cggguccuug cgcugagucu cgggaccaca gccggggagg 60

cgggguccuu cucuggggcg gucgcguugg cagcggaugc gggaagccgg acucugggcg 120

ucau 124


<210> 2600
<211> 87
<212> RNA
<213> Homo sapiens

<400> 2600
cagccuccug aguugucuuc uucagcaucc cccagguaaa guccggggcu ugugucaucc 60

uaggaagccg cgucguccgg ggcugcu 87


<210> 2601
<211> 132
<212> RNA
<213> Homo sapiens

<400> 2601
cccuguauuu uugaauucac cuacucacuc aaauguauuu guaacccuca aaccagcaug 60

cucagugccu uucuggucau uuuaggacac ucauaaguac gagucucaaa ugugagguca 120

aguaagaggg ca 132


<210> 2602
<211> 103
<212> RNA
<213> Homo sapiens

<400> 2602
gggccugggg ggcugggacg cgggagaugc cuuccugccc cccccccccc cccacuucug 60

agcgcccuag gaggcuccug cccucccguc ccaccagguc cug 103


<210> 2603
<211> 70
<212> RNA
<213> Homo sapiens

<400> 2603
accugggaag gcagggguag cccugggguc cugggccuca cuggaaggcc cgcugcucca 60

uggagggugu 70


<210> 2604
<211> 57
<212> RNA
<213> Homo sapiens

<400> 2604
agaagguucu gcgccucacu gagugccuua ggccccugca guaugugggc cuucucc 57


<210> 2605
<211> 72
<212> RNA
<213> Homo sapiens

<400> 2605
ccugccggag ccgcugccgc uauggaugau cgagaggauc ugguguacca ggcgaagcug 60

gccgagcagg cu 72


<210> 2606
<211> 72
<212> RNA
<213> Homo sapiens

<400> 2606
ccugcuggag ccacugcugc uguggaugau cgagaggauc ugguguacca ggcgaagcug 60

gccgagcagg cu 72


<210> 2607
<211> 53
<212> RNA
<213> Homo sapiens

<400> 2607
ugcaggcggg gcgaaggcgg agccccggga ccguuugucc cccgaaucgc agc 53


<210> 2608
<211> 84
<212> RNA
<213> Homo sapiens

<400> 2608
ucuacagguu gauaaaaauu aaagaguggg uggacaagua ugacccaggu gcuuugguca 60

uuccuuuuag uggggccuug gaac 84


<210> 2609
<211> 55
<212> RNA
<213> Homo sapiens

<400> 2609
aggcccacgg gggagggugu uggcagacag augcccucca ggcccugggg ccucc 55


<210> 2610
<211> 95
<212> RNA
<213> Homo sapiens

<400> 2610
gcuguuauaa auaaaauaca ugcugagccu gcaauuugcu cuuaaugaag gccacuuguc 60

aguagacggu gcugcuaaug cguguccugc agcaa 95

<210> 2611
<211> 66
<212> RNA
<213> Homo sapiens

<400> 2611
gcuggaagcu auugggcugg cagcaggacg acuggcuguc cuccugccca cccacacuga 60

cggcau 66


<210> 2612
<211> 71
<212> RNA
<213> Homo sapiens

<400> 2612
ggcagguuuc uacaauggcc ugggaaugga cugauuauuu uuauacauuu ucuggccuga 60

gagaaagcca a 71


<210> 2613
<211> 144
<212> RNA
<213> Homo sapiens

<400> 2613
agagugacac ccauuuauua ucucaugguu ccauagguca gaaguuugga uguagugugg 60

cuggguaaga cccugcacag aggacccagc cacacuaugu ccaaacuucu gaccucggag 120

augugagcca gggcugcauu cuca 144


<210> 2614
<211> 127
<212> RNA
<213> Homo sapiens

<400> 2614
aggcagcagu uauuaacacg ggaacauggc ggccgcagcc ucggcuacag cuucggcggc 60

gaaggucagc gccgacggca gccggcaccu gacggcguga ccgacccgag ccgguaaccg 120

cgccuuc 127


<210> 2615
<211> 117
<212> RNA
<213> Homo sapiens

<400> 2615
cauaaccauc uuccuagcug auugugcaau guuagcauuu gucucugaac ccgaccccau 60

gguggguuca gagcacagag uuacaggcac agagcugagc aaggcacagg cuguguu 117


<210> 2616
<211> 74
<212> RNA
<213> Homo sapiens

<400> 2616
cugucuucuc uggcccaugg acaucggaac accuggucuc aggucuucag acuguggggc 60

uuacacgagc agcc 74


<210> 2617
<211> 60
<212> RNA

<213>  Homo sapiens

<400>  2617
gugugaauga ugaacaggua gguugguggu agguaccagg uuggccuggc augcccacuu      60


<210>  2618
<211>  143
<212>  RNA
<213>  Homo sapiens

<400>  2618
cccgaagagu guaagauguu cgccugcgcc aagcucgccu gcacccccuc ucuggugcgu      60

agcccaggca gggucggggg gauugagggg ugggcguuuu ggccuagagc guccacguug     120

uugaaugggg caaccuucgg gcg                                             143


<210>  2619
<211>  55
<212>  RNA
<213>  Homo sapiens

<400>  2619
uucccccagu uuguccgguu cagcuggcgc acggcccaga ccauccugga gaaag           55


<210>  2620
<211>  52
<212>  RNA
<213>  Homo sapiens

<400>  2620
gacgcaacaa gggugcugcc cgaguuaucg ggacccuguu gggaacuguc aa              52


<210>  2621
<211>  58
<212>  RNA
<213>  Homo sapiens

<400>  2621
cgagagacgc aacgagggug cugcccgagu uaucgggacc cuguugggaa cugucgac        58


<210>  2622
<211>  105
<212>  RNA
<213>  Homo sapiens

<400>  2622
uaguugaaau auuuuucugg ccuuucugcc cguggacgcc gccgaagaag caucguuaaa      60

gucucucuuc acccugccgu caugucuaag ucagagucuc cuaaa                     105


<210>  2623
<211>  96
<212>  RNA
<213>  Homo sapiens

<400>  2623
gcuugcuccu uucugcccgu ggacgccgcc gaagaagcau cguuaaaguc ucucuucacc      60

cugccgucau gucuaaguca gaggugaguu aggcgc                                96


<210>  2624
<211>  95
<212>  RNA
<213>  Homo sapiens

<400>  2624
ucuucuccuu ucugcccgug gacgccgccg aagaagcauc guuaaagucu cucuucaccc      60

ugccgucaug ucuaagucag agucuccuaa agagc 95

<210> 2625
<211> 98
<212> RNA
<213> Homo sapiens

<400> 2625
ccucggacuc cugccucacu cauuuacacc aaccacccaa cuaucuauaa accuagccau 60

ggccaucccc uuaugagcgg gcgcagugau uauaggcu 98

<210> 2626
<211> 88
<212> RNA
<213> Homo sapiens

<400> 2626
agcaucuggc caggcauggu ggcucucaua uguaauccca gcacuuuggg aggcugaggu 60

ggguggauca cuugagguca ggaguuca 88

<210> 2627
<211> 69
<212> RNA
<213> Homo sapiens

<400> 2627
caggaguuug agaccagccu gggcaacaug gcaagacccc gucucuggcc aggcauggug 60

gcucuugcc 69

<210> 2628
<211> 120
<212> RNA
<213> Homo sapiens

<400> 2628
acagggauaa gcuucuacca gccugguucu ggagcuggca agugaacaaa ucccacaagu 60

ggcuuucuga ucaugauaaa agcagcagcc cuggccaggc augguggcuc ucgccuguaa 120

<210> 2629
<211> 80
<212> RNA
<213> Homo sapiens

<400> 2629
ccuggccagg caugguggcu cucgccugua aucccaguac uuugggaggc cgaggcgggc 60

uuaucaccug agguuaggag 80

<210> 2630
<211> 129
<212> RNA
<213> Homo sapiens

<400> 2630
guugcaguga gcugagaucc ugccacugca cuccagccug ggugacagag ucagacccug 60

ucucaacaac aaaaagaaaa gaacccaggc cuggccaggc augguggcuc ucaccuauaa 120

ucccaauac 129

<210> 2631
<211> 61
<212> RNA

<213>  Homo sapiens

<400>  2631
uacugugaga auuuugggag ugucuauuag cuugcagcac uuuucuugga uucuguguau      60

u                                                                     61


<210>  2632
<211>  82
<212>  RNA
<213>  Homo sapiens

<400>  2632
caggggaaac ugggcaaagg agcaggcugg uaggauggaa aaaugaugug ccaacuuuca      60

uuugaauagu uuuucuccug ca                                              82


<210>  2633
<211>  60
<212>  RNA
<213>  Homo sapiens

<400>  2633
agcaaugaau gcucguuagc caugugccug gaggaagguc acagcucagc cagcagcuag      60


<210>  2634
<211>  193
<212>  RNA
<213>  Homo sapiens

<400>  2634
cuggagaacc cuggcgccug cccuggucac cagccuggcu gagguaugcu guugcgcugu      60

ccuuccucug gggagcaggc uccgggggac agggaaaagc acacaaggaa cuuguccucu      120

agggccugca gucucauggg agagugacau gcaccaggac caccgcugug aagggcuaag     180

ugcuguccug gag                                                        193


<210>  2635
<211>  78
<212>  RNA
<213>  Homo sapiens

<400>  2635
caaggaaggg agcgaagagg gaauagccca cagcagccca guggaaagca uggcauuucu      60

uuaaaguuua uucuugua                                                   78


<210>  2636
<211>  91
<212>  RNA
<213>  Homo sapiens

<400>  2636
ggccggggca ccuucgucgg ccgugcccgu gccucugugc cguggacgcg cucgggacac      60

ggggccacca cgugggucgc uuaccggcca g                                    91


<210>  2637
<211>  70
<212>  RNA
<213>  Homo sapiens

<400>  2637
cggggcggcu ccgcagcccg ccgggagcuc ugaccgaggc gccucgcugg ggcggggacc      60

uugccuugcc                                                            70

<210> 2638
<211> 73
<212> RNA
<213> Homo sapiens

<400> 2638
gccacggagg cagggucucu ggggacuguc gggggguaca gagggagaag gcucugcaag 60

agcucccugg caa 73


<210> 2639
<211> 79
<212> RNA
<213> Homo sapiens

<400> 2639
ggagcagggu cgcuaagguc cugcccacug aggggacagc cuucugggca gggaccucgg 60

ggggcuucaa gggcucugc 79


<210> 2640
<211> 66
<212> RNA
<213> Homo sapiens

<400> 2640
ucuccucccu ucacacucau uuccccuucg ccgaggagag aaggggggga ggagagagaa 60

gagacc 66


<210> 2641
<211> 141
<212> RNA
<213> Homo sapiens

<400> 2641
guugccuccu aguggcugug ucugucuugg acaccgguau cugagaacug agaacaauau 60

uuguuccaua aaugaggcau gaauauuacu cuuuaaagau ucccuguuga aggcaggcac 120

aguggcucac acguguaauc u 141


<210> 2642
<211> 57
<212> RNA
<213> Homo sapiens

<400> 2642
cauccaagag gagaaucuuc cccaggguuu aguccugggg cuggcacucg uugugac 57


<210> 2643
<211> 66
<212> RNA
<213> Homo sapiens

<400> 2643
cccgacgcca cgcagcugcg cgcagcuggu ucccgcucug cagcgcaacg ccugaggcag 60

ugggcg 66


<210> 2644
<211> 129
<212> RNA
<213> Homo sapiens

<400> 2644
uccgcaggcc gcggcguacg gcaggugcgu gcaggccucc acggccccgg gcggccgccu 60

gaguaaggac uucugcgcgc gggaguucga ggcccugcgg agcugcuucg ccgcugcggu 120

aggugggcg 129

<210> 2645
<211> 102
<212> RNA
<213> Homo sapiens

<400> 2645
aguaguagug gcugggucuu ccuugccuac aucaucaaaa gugaaugcaa cuguaccaag 60

uaauaugaug ucaguuaaug gacaggccaa aacacacacu ga 102

<210> 2646
<211> 67
<212> RNA
<213> Homo sapiens

<400> 2646
agcaugggag ggguauggag aaguccaagu gagcacucuu ucuccaguac ucucaaaaua 60

cuguuau 67

<210> 2647
<211> 121
<212> RNA
<213> Homo sapiens

<400> 2647
cggcugacca ggaaccuggg cgagcagcgg cgggggcccg aggggucagu accaguaggc 60

gcggcucaca cgcucgggaa gacugcgccc ucucccccau cccuccucgg aggcagccgc 120

a 121

<210> 2648
<211> 86
<212> RNA
<213> Homo sapiens

<400> 2648
ggcuuuggua gcauguugga aaaaguuugu ccccacugca guuucuguuu uaguucuugg 60

cauuuuccau guauccaauu agccuu 86

<210> 2649
<211> 78
<212> RNA
<213> Homo sapiens

<400> 2649
cacacauaca auaauauuca uaaugcaauc acacacaauc accaugugac ugcauuauga 60

aaauucuucu agugugau 78

<210> 2650
<211> 102
<212> RNA
<213> Homo sapiens

<400> 2650
caggugccca cacauacuac aauaauuuuc auaaugcaau cacacacaau caccguguga 60

cugcauuaug aaaauucuuc uagugugauu uacagcucug uc 102

<210> 2651
<211> 72

<212>  RNA
<213>  Homo sapiens

<400>  2651
ugggacugug caggguggau ccagggugag gguacagucg gccccuucuc cuggcaaccu      60

cccugcccua aa                                                          72


<210>  2652
<211>  92
<212>  RNA
<213>  Homo sapiens

<400>  2652
gggggugagg aaggcagauc uuagagcuuu ggcaggacgg caaggaggcc uuuccuugcc      60

cuccaaagcu cuaggccuuu ccuaacuuuu au                                    92


<210>  2653
<211>  74
<212>  RNA
<213>  Homo sapiens

<400>  2653
auguucuccu uugacuccgg gucucacauc caggucacgc ugaugaaaga gguggguuaa      60

aaugacucac auga                                                        74


<210>  2654
<211>  66
<212>  RNA
<213>  Homo sapiens

<400>  2654
ggucuguaaa cggcugcaca cuucaggcac uguaguugcu gaagaugcuu uguuaaaugu      60

gaccuu                                                                 66


<210>  2655
<211>  60
<212>  RNA
<213>  Homo sapiens

<400>  2655
cagcugaccu uuuacguuaa cuggggcaaa gccugaacgg auaaccagag gccaacugcc      60


<210>  2656
<211>  95
<212>  RNA
<213>  Homo sapiens

<400>  2656
cacagcggug uucauacaag caucauuggu gauaaucaaa aaggggaaac aucccaaaug      60

accaucagcu ggugacugcc uaaacaaaau gugcu                                 95


<210>  2657
<211>  64
<212>  RNA
<213>  Homo sapiens

<400>  2657
ugcugugaag gugcugauga aguucaugga gauguugucu aaggacuugg acuacuacgg      60

caca                                                                   64


<210>  2658
<211>  76

<212>  RNA
<213>  Homo sapiens

<400>  2658
acacaauguu cauugaaauu ggcuuucauc ugaggaaucc caggguggcu ccugcuucau      60

gaugugcauu cugucu                                                      76


<210>  2659
<211>  85
<212>  RNA
<213>  Homo sapiens

<400>  2659
uguuuagcua uuuuacagga gucaacaaga uaauuaaaua ucuuggugug uuguuucuau      60

uguguggcuu uuuaaaaaug acaaa                                            85


<210>  2660
<211>  87
<212>  RNA
<213>  Homo sapiens

<400>  2660
ggcgcugccu uuccucguug gcuacuuuca guaaguugug accaguaugu cuggacgugg      60

caagcaaggc gguaaagcuc gcgccaa                                          87


<210>  2661
<211>  64
<212>  RNA
<213>  Homo sapiens

<400>  2661
gugcacacgg gcgagaagcc cuaugccugc ggggacugcg gcaaggccuu cagccagcgc      60

auga                                                                   64


<210>  2662
<211>  94
<212>  RNA
<213>  Homo sapiens

<400>  2662
aauagaacuc aaguuucaag guacaguuuu cugaccccaa ggcaacuaug gucuaccccc      60

agaaggcugg auugaggcuc uagcauuuug uuuc                                  94


<210>  2663
<211>  150
<212>  RNA
<213>  Homo sapiens

<400>  2663
uacaaauaga gugugcaaac aauaguacug auuaagugac aaauguugag gccugggaac      60

ugauuuuugg cacugacaaa uuaaggcaga uuagaccgcc uuucaggaca cuuuugauaa     120

ugcuacgugu augugaaaga agaagguauc                                      150


<210>  2664
<211>  90
<212>  RNA
<213>  Homo sapiens

<400>  2664
guaacaaaug gcuucuuuug uccuucccuu uguuuacuac uccaaccaga ucuuuaagau      60

agagcgugaa ggcgaaugcc agcguuacaa                                       90

<210> 2665
<211> 78
<212> RNA
<213> Homo sapiens

<400> 2665
acccuuuccu ccugccgggc ugcagggaca gugacaguga gcagcacugc ccgcccaggc 60

aaccuggaac cagggucg 78


<210> 2666
<211> 120
<212> RNA
<213> Homo sapiens

<400> 2666
ggccggcucu acguucccug uucucgccug cagcuccgcc auggcuccua aaggcagcuc 60

caaacagcag ucugaggagg accugcuccu gcaggauuuc agccgcaauc ucucggccaa 120


<210> 2667
<211> 71
<212> RNA
<213> Homo sapiens

<400> 2667
ugaagcggcc ucaggccugu guccacggcc uguguugguc cagguguaca cagcacuggg 60

guccugucac c 71


<210> 2668
<211> 107
<212> RNA
<213> Homo sapiens

<400> 2668
gugaauccuc uuguuuaaau uguaaaucau guggcaucuc uguucucugu gguaacauag 60

gaaaaagguc gggguucuu uggucaaaau aggggugggc uucaugc 107


<210> 2669
<211> 133
<212> RNA
<213> Homo sapiens

<400> 2669
agccuguccu caccgucucg gggguugugg cccccgcccc cucccuauau gcaccccugg 60

aaccagcaag ucccagacaa ggagagcgga ggaggaaguc augggaacgc agccuccagu 120

uguagcaggu uuc 133


<210> 2670
<211> 70
<212> RNA
<213> Homo sapiens

<400> 2670
aggugaggau auucauacua gaaugugggu ggcacagacc cugauguccu ggguagaugg 60

cuuuauuucu 70


<210> 2671
<211> 85
<212> RNA
<213> Homo sapiens

<400> 2671
uggcacauuc caccccagaa gugacuguug gagagaugag aggaaaaagg cagagcagag 60

cuagguggag augugacacu ucaga 85


<210> 2672
<211> 82
<212> RNA
<213> Homo sapiens

<400> 2672
acucugaucu uuuuuuaaaa aacacacagu ggcagaguca uuucacuauu gcacugugug 60

uuaaagaaug aauaaggagu uu 82


<210> 2673
<211> 106
<212> RNA
<213> Homo sapiens

<400> 2673
uguggagacc gcagucagua acuuugugau auauggaaua uuuagaggac aguaacccau 60

uucccuacgc ccuaccugau cacaucgugc ccugcugggc cauacc 106


<210> 2674
<211> 59
<212> RNA
<213> Homo sapiens

<400> 2674
uuucuuuauu gcacauuugg ggcuuggggg auagagucag auguguguca guugaaacu 59


<210> 2675
<211> 116
<212> RNA
<213> Homo sapiens

<400> 2675
uacuucgccu ccacuucugu uccucccugc ccuggugcug cuccagguca cauacucguc 60

cugagccggc uucagccugu ccgcgcagaa gucucccgga gccauggccu aguacu 116


<210> 2676
<211> 143
<212> RNA
<213> Homo sapiens

<400> 2676
ugcuuaauga agggagccag acacaagacg ccacaugcuc ugugauuccg uuuguaugaa 60

auguucaaag uagaugaauc cauagagacg gagugcagau ugauggucgc uagggcuugu 120

gggaaggggc acccgagagc agc 143


<210> 2677
<211> 72
<212> RNA
<213> Homo sapiens

<400> 2677
gcugcucuuc uggcuggcac cgucaccccu gcccgacccc aggccccgcg ugugucccgg 60

acggagcagu cc 72


<210> 2678
<211> 65
<212> RNA

<213>  Homo sapiens

<400>  2678
uuucuucgaa gacaccgccg cuuccacagc gaagggagcu gcuccgggca cgucgcggag      60

ggagu                                                                  65


<210>  2679
<211>  70
<212>  RNA
<213>  Homo sapiens

<400>  2679
aguggggucc uaacugucaa agcaggggac accauuaggc uugaggcagg gguuagaggc      60

aaaccauucc                                                             70


<210>  2680
<211>  129
<212>  RNA
<213>  Homo sapiens

<400>  2680
caccuccagc cugggcgaca gaaacagacu cugucucaga gaaaaaaaaa aaaaaagcga      60

aaaaacguua uuuauuuauu uauuuauuua gagacggagu cuugcucaag uugcccaggc     120

uggagugca                                                             129


<210>  2681
<211>  80
<212>  RNA
<213>  Homo sapiens

<400>  2681
ugcgcgcagg ggaggcagcg cagcccguga acacccccga gcccggugcg ggccgagcag      60

uucuccgcac cuccgguaaa                                                  80


<210>  2682
<211>  52
<212>  RNA
<213>  Homo sapiens

<400>  2682
aaguguuaau cucaguguga cucauuugau uuccuguuga caugccauuu cu              52


<210>  2683
<211>  64
<212>  RNA
<213>  Homo sapiens

<400>  2683
guuugcacag ccuccgggag cuaccaauau gauagguuac auggggcaga cugccuacca      60

auac                                                                   64


<210>  2684
<211>  88
<212>  RNA
<213>  Homo sapiens

<400>  2684
ggguugggca aggugcgggg cuagggcuaa cagcagucuu acugaagguu uccuggaaac      60

cacgcacaug cuguugccac uaaccuca                                         88


<210>  2685

<211>  60
<212>  RNA
<213>  Homo sapiens

<400>  2685
gcuucucuga uugcucagac uauugcuugg uugcuaagua gggagccaca gaccuggucc      60


<210>  2686
<211>  109
<212>  RNA
<213>  Homo sapiens

<400>  2686
gcgagcggac ccuccgaggc ugcggccagg ugggcggaca ccccacgcug cagccccugc      60

acggcgcuca cugcccguuc cccacagcuu cgagaggcuu cugcugcac                 109


<210>  2687
<211>  119
<212>  RNA
<213>  Homo sapiens

<400>  2687
ggcuuucuug ucacguacaa ccucgggucg gcuguuuacu cagccaagga gagaagauag      60

aguagggcug ggaggcugag ggcggacugg ggcggaaaug uggcagagau cccagucccc     119


<210>  2688
<211>  67
<212>  RNA
<213>  Homo sapiens

<400>  2688
agcuguguaa ccuugggcaa uuugcuucaa cucugggucu cagauuccuu auuauaaagu      60

aagcuuu                                                                67


<210>  2689
<211>  64
<212>  RNA
<213>  Homo sapiens

<400>  2689
gcuacaggcu aacacaggga aagagacaga aucuucuagu cccuacaguu agucuaagua      60

gcaa                                                                   64


<210>  2690
<211>  115
<212>  RNA
<213>  Homo sapiens

<400>  2690
ucuaacccuc cugcucgauc ucagagcuca gacacagagc agcaaucccc uacaaguggu      60

gggggaaaag uagcuccagc ucaaccaucu gaggaaggac caggaaggaa agaug          115


<210>  2691
<211>  72
<212>  RNA
<213>  Homo sapiens

<400>  2691
aucuggaguc ugaaguagcu auaaagcagc uauaaaacag aaauacaugc auagcugcag      60

aaaccaugau ag                                                          72


<210>  2692

<211> 59
<212> RNA
<213> Homo sapiens

<400> 2692
ggaguggacc cgccacggga gcuuugucaa uaagcccacg cggggcuggc ugcauccca 59


<210> 2693
<211> 72
<212> RNA
<213> Homo sapiens

<400> 2693
uguuuggguc uguuuacagg gucuuggaug ucggguucga ccacuuggcc gaugggaaug 60

gucugucaca gu 72


<210> 2694
<211> 148
<212> RNA
<213> Homo sapiens

<400> 2694
cagugaugau cgcaucacag uugagugaaa uggccuuccu guucagcugu uagagacuga 60

agauuguuag ggcaccuuag aaugucucau cuuuucuagg uugucaacag guacuauuug 120

ucacauaacu aacuuucgag gcacugga 148


<210> 2695
<211> 118
<212> RNA
<213> Homo sapiens

<400> 2695
aucaauaaau aaaaauauau caccuuuuug gcucucugac cagcaccaug gcaguuggca 60

agaacaagug ccuuacgaaa ggcggcaaaa agggagccaa gaagaaagug guugauca 118


<210> 2696
<211> 60
<212> RNA
<213> Homo sapiens

<400> 2696
uguaaacgcu uucugaugua guacucaaac ugauagaaaa uauuuucaug aaacuacaug 60


<210> 2697
<211> 68
<212> RNA
<213> Homo sapiens

<400> 2697
gggaaacuca gauguccaaa cuaauuuuaa caaacgcauu aagagguuua uuuggguaca 60

uggcccgc 68


<210> 2698
<211> 75
<212> RNA
<213> Homo sapiens

<400> 2698
gguuuaauca aaggaugugu gguuugcuaa ccuuggcaac aggcaaggcc aauguagucu 60

aaagucaagg acucu 75


<210> 2699

<211>  142
<212>  RNA
<213>  Homo sapiens

<400>  2699
ugguuuaagu uauuaauagu ugaauugaaa uuguggccaa ggaugagaac ucuaaucuga      60

uuuuaugugc uucugcugug auggauuaaa ggauuuaccu gaggccaaau ugguaugcag     120

gaauuuauua aauggaauca ua                                              142


<210>  2700
<211>  73
<212>  RNA
<213>  Homo sapiens

<400>  2700
gaaugucauu gagaugcuug uaggacaggg accgcugucc uacaagcauc ucaaugacau      60

ucauucuguu ccc                                                         73


<210>  2701
<211>  73
<212>  RNA
<213>  Homo sapiens

<400>  2701
gaaugucauu gagaugcuug uaggacagcg gucccugucc uacaagcauc ucaaugacau      60

ucauucuguu ccc                                                         73


<210>  2702
<211>  100
<212>  RNA
<213>  Homo sapiens

<400>  2702
gguacacuuu guuuuguaca ggucaauggg uuuugacagu guauauaaug acaugugucu      60

accauugugu aaugccacgu aucugucauu acaguaccau                           100


<210>  2703
<211>  112
<212>  RNA
<213>  Homo sapiens

<400>  2703
ccccugaacc uucuggagga acaucuaaau guacacagcu cuguuuugua gguauuaaag      60

acuaccacag aggcuaugau ugaggucaac auuaacaaga auuuaguggg cu             112


<210>  2704
<211>  103
<212>  RNA
<213>  Homo sapiens

<400>  2704
uaguuuuugu agacaugggc cacucugcuu aucaaguuuc uguaugugca uuuaauagag      60

gaaaacugaa aguaaguaua uauuuuuuuu ccagaagacu guu                       103


<210>  2705
<211>  83
<212>  RNA
<213>  Homo sapiens

<400>  2705
ugcaggagga agcuccuggc cacggguccu gcagagugac aguucuuccu gaaguggacu      60

ccguaggcuc uuccuccugc aau                                                83

<210>  2706
<211>  56
<212>  RNA
<213>  Homo sapiens

<400>  2706
cuagacuagg aggaagcucc uggccacagc cucugacuca ucuccuggcc cugguu          56

<210>  2707
<211>  70
<212>  RNA
<213>  Homo sapiens

<400>  2707
gcagguucuc ccuugcaugc aagaugggug cuggcccacu gugaggcuuc ugcucgggga      60

aguucugugg                                                              70

<210>  2708
<211>  61
<212>  RNA
<213>  Homo sapiens

<400>  2708
gggacccgcc acgguuggcc aagcucaggc cauaugagcu gggacuggca ggcaguuccu      60

g                                                                       61

<210>  2709
<211>  61
<212>  RNA
<213>  Homo sapiens

<400>  2709
gggacccgcc gcgguuggcc aagcucaggc cauaugagcu gggacuggca ggcaguuccu      60

g                                                                       61

<210>  2710
<211>  120
<212>  RNA
<213>  Homo sapiens

<400>  2710
uggcagacag aguaacauga gcugggacug gcaggcuaga agccucccau uggcugcugg      60

ugguccaagc uaugggcaag gagcugcagc ugggacuggc acaggaaaca cccugcuagg     120

<210>  2711
<211>  114
<212>  RNA
<213>  Homo sapiens

<400>  2711
uugcaacuua agcuucaugu aggucuuuau aauguguuuu cuccuccuac cagaaagcaa      60

uaaaggagaa agagauuccu auugaaggac uggaguucau gggccauggc aaag           114

<210>  2712
<211>  126
<212>  RNA
<213>  Homo sapiens

<400>  2712
ccucuuggug ggaggcuuug auugggaagc ugggggaagg uggcuagugg gugggguucg      60

ggaguacugg ggaccucucc caguguccuu ccuccagcca guccuuggca gagagcugag 120

aaggug 126

<210> 2713
<211> 98
<212> RNA
<213> Homo sapiens

<400> 2713
cugcagccug guagaauugg gaagcugggg gaaggguggu cugagcaccc ccucauuccc 60

cucgugugac ucucuuggau uauuuaugug uugugguu 98

<210> 2714
<211> 61
<212> RNA
<213> Homo sapiens

<400> 2714
aguuugaauu gggaagcugg gggaaguuaa auaugagcca cuggguguac cagugcauua 60

a 61

<210> 2715
<211> 63
<212> RNA
<213> Homo sapiens

<400> 2715
uccugucuag ugcagaaagc aggcagugcc cagcacagag cacauguauu agguaaaugg 60

acc 63

<210> 2716
<211> 63
<212> RNA
<213> Homo sapiens

<400> 2716
ccggacccag acugguagug aggcuuugga ccccgagccg cugcaaugcc gcuggagcug 60

gag 63

<210> 2717
<211> 84
<212> RNA
<213> Homo sapiens

<400> 2717
cggcccgcgc gccuagcguc augggcgcca cgcgccggag gggcggggcu acggcgccuc 60

ggagggcucg cagaugcggc cggc 84

<210> 2718
<211> 100
<212> RNA
<213> Homo sapiens

<400> 2718
agcugcgucu uugugcuuuc agcuucuuua cagugcugcc uuguagcauu caggucaagc 60

agcauuguac agggcuauga aagaaccaag aaugggcugc 100

<210> 2719
<211> 80
<212> RNA
<213> Homo sapiens

<400> 2719
aucuaaagau gaaaaaggac aggaaaugug gaaaaugaaa ucuggauucu gcguguucug 60

uuguuugccu cccugggucu 80


<210> 2720
<211> 100
<212> RNA
<213> Homo sapiens

<400> 2720
aggguguggu gaggauuugg ugaacuggug cccaccgagg guggugaaga accuugguau 60

acaguaagug cucacuaaau ccuggcggcu aggauccuca 100


<210> 2721
<211> 121
<212> RNA
<213> Homo sapiens

<400> 2721
cuucugccua cucgguccag caggcucugc uuggcugaug cucccggccc agaucccacg 60

ccugccaagg gcgggucagg ugcagagcag caaggggugu gagagcgagu gagcugggga 120

u 121


<210> 2722
<211> 77
<212> RNA
<213> Homo sapiens

<400> 2722
ccacugagag cgggacgcgg ggcuagguca gaguaggaag gagucgcuga gcugaaucuu 60

uguggagugg agugggg 77


<210> 2723
<211> 56
<212> RNA
<213> Homo sapiens

<400> 2723
gcugccgcgc uggcauuuuc uccuggacaa ggagagagug cggcugcuga gagccg 56


<210> 2724
<211> 104
<212> RNA
<213> Homo sapiens

<400> 2724
ugcucuccuc ucagaagcug cagccgugau ggaaguuuga auguugagcc gcugugaggc 60

gaggccgggc ucaggcgagg gagaugagag acagagacgg cggc 104


<210> 2725
<211> 54
<212> RNA
<213> Homo sapiens

<400> 2725
cugggcagcc gcggguaaag gccuagcggg cuugauaacc ccugcgcccu ggcu 54


<210> 2726
<211> 63
<212> RNA
<213> Homo sapiens

<400> 2726
ccaggcuuuc uuccccacuc ccccaggccc aagcccagcu gggacucagg aaggcuucug 60

gaa 63


<210> 2727
<211> 59
<212> RNA
<213> Homo sapiens

<400> 2727
gcuggggugu uuguuuggac uggagaaggg aggcggcggg cgaagcgcac gucgagcgg 59


<210> 2728
<211> 57
<212> RNA
<213> Homo sapiens

<400> 2728
agggcuauga caugcuagag gaucccaagu ucccugaggg caucuacaca gaucuga 57


<210> 2729
<211> 128
<212> RNA
<213> Homo sapiens

<400> 2729
ggccuuuuca cagaggguuu uguaaucuag cuucccuuuu ucaagggaug auuguucugc 60

uuuguggaga guagagaaca gaauguagga guggaagauu ggaagcggaa agaucaguaa 120

gaggcuaa 128


<210> 2730
<211> 58
<212> RNA
<213> Homo sapiens

<400> 2730
gcucgcgacu ggagggcgga acuccgggcu ggagucccgg aagccccugu ggcagcuu 58


<210> 2731
<211> 60
<212> RNA
<213> Homo sapiens

<400> 2731
gugcccgucu gcuggggcaa aggagccugu ccuguguuug aauguggcaa cguggugcuc 60


<210> 2732
<211> 52
<212> RNA
<213> Homo sapiens

<400> 2732
caccaagucu ggcaggucug cucaccaggu ggcuagauac cgcccacgug cc 52


<210> 2733
<211> 69
<212> RNA
<213> Homo sapiens

<400> 2733
ggcgagggac cuggcccugg ccauccggga cagugagcga cagggcaagg cccaggugga 60

gauugucac 69

<210> 2734
<211> 120
<212> RNA
<213> Homo sapiens

<400> 2734
cccggagaau guauccauug agacgcucgc uguuuguauc cauugaggag cugccucgcg 60

cagggggugu gcgaggcuga guccaagaga uagcaaaucg agucuuaaau aauccgggga 120


<210> 2735
<211> 60
<212> RNA
<213> Homo sapiens

<400> 2735
gcgcggcggc aacagcagac augucggggg uccggggccu gucgcggcug cugagcgcuc 60


<210> 2736
<211> 64
<212> RNA
<213> Homo sapiens

<400> 2736
cuguccugcu gggacaguca ugggacugca ucuucguccu uguccacagc agaugaggac 60

agug 64


<210> 2737
<211> 103
<212> RNA
<213> Homo sapiens

<400> 2737
uugcuaagua cgaccugaaa aucugaugca aacuccgaca cuugugggcu gagcccagca 60

guguccagca guuugcaagg aauagccgcg gcucugggca guu 103


<210> 2738
<211> 74
<212> RNA
<213> Homo sapiens

<400> 2738
ccuccucauc cugggcuggg cuuuuccucu uggcacacag uggugugggg agaaacagcu 60

uuggauguga ggcc 74


<210> 2739
<211> 106
<212> RNA
<213> Homo sapiens

<400> 2739
ugauccucgu gauaugagga acuggcuucc ccagagcaag uggucuuaga agaagagagc 60

aagcaggaag ccacagugcc uuuaucacuu ggccucugac aucaca 106


<210> 2740
<211> 72
<212> RNA
<213> Homo sapiens

<400> 2740
uggaauugaa gaggaagaug augaugauaa ugaaauugau gaugaugaua caauuucuga 60

cuggaaucua ag 72

<210> 2741
<211> 72
<212> RNA
<213> Homo sapiens

<400> 2741
uggaauugaa gaggaagaug augaugauaa ugaaauugau ggugaugaua caauuucuga 60

cuggaaucua ag 72


<210> 2742
<211> 90
<212> RNA
<213> Homo sapiens

<400> 2742
ugaugaagau ggaauugaag aggaagauga ugaugaugau gaaauugaug augaugauac 60

aauuucugac uggaaucuaa guaagucaga 90


<210> 2743
<211> 120
<212> RNA
<213> Homo sapiens

<400> 2743
ggugcacgug cgcaguagcg gcggccacgc cucuuccgcc cgcccucucc cagccugccc 60

gcuugaucgc cggcuguugg cggagaugag augccgcugc acgguguuug uccguacuac 120


<210> 2744
<211> 74
<212> RNA
<213> Homo sapiens

<400> 2744
gcuccccaca ggacuuggac agagggccca cagggggaug ggcuggcugu ggccagguuu 60

agggcagggg gcau 74


<210> 2745
<211> 60
<212> RNA
<213> Homo sapiens

<400> 2745
gcaggaugug gggaugcugc agcucauuga guugagaugg gguccugaua gucacugcag 60


<210> 2746
<211> 105
<212> RNA
<213> Homo sapiens

<400> 2746
gccuaguaaa aaaucagaaa aaauguaugc agagcaguuu ucaaugacuu ccaagaugcc 60

ugcaggaugu ggggaugcug cuguggcuga gugagucugu gguca 105


<210> 2747
<211> 53
<212> RNA
<213> Homo sapiens

<400> 2747
ugcugucguc cacgcccugc uggaccggcc uuagggguga aggugggggc aca 53


<210> 2748

<211>  123
<212>  RNA
<213>  Homo sapiens

<400>  2748
cauccguuau gccucgguca ucagcauggu ugucacgguc ucucuggcuc acgggcugcu      60

gcggcucaca gccuucccuc acuugccugc aaccagcuga gagccucccu ggugaugggu     120

guu                                                                   123


<210>  2749
<211>  85
<212>  RNA
<213>  Homo sapiens

<400>  2749
gccaugauca ucccuguacg cugcuucacu uguggcaaga ucgucggcaa caagugggag      60

gcuuaccugg ggcugcugca ggccg                                            85


<210>  2750
<211>  91
<212>  RNA
<213>  Homo sapiens

<400>  2750
gugaccgugc guguaaaggg ugaggcguau ggagcugugg cggggcggaa guucacaugu      60

gugguaugua gagcacaugu uaugguuauc a                                     91


<210>  2751
<211>  74
<212>  RNA
<213>  Homo sapiens

<400>  2751
gggauucuga aaaguucgug agcugaaaca aauaaauuga guuggaagau ucauguugac      60

uguuuguauc ccaa                                                        74


<210>  2752
<211>  65
<212>  RNA
<213>  Homo sapiens

<400>  2752
gcgcgaggaa cuggagcccg gcuggccccc gccuccgccu ccgccccccg gccccagcug      60

cgcgg                                                                  65


<210>  2753
<211>  105
<212>  RNA
<213>  Homo sapiens

<400>  2753
gguggggcgc ugcaggcuag ccucacugcc cuguauucca cgcucacagg gaguccugac      60

cugggggcug uggagcugcg caucgaagag cugcggcacc acuuc                     105


<210>  2754
<211>  56
<212>  RNA
<213>  Homo sapiens

<400>  2754
uuugcgggcg cucgcgguaa guuugcgcca agugcgcggc agccgggacg cagacg          56

<210> 2755
<211> 159
<212> RNA
<213> Homo sapiens

<400> 2755
uccuaauaau gggccuuccu cacucugaaa acuauuuuuu uuuuggcggc gggugcgggg 60

guaauggaau guuucugaua ugguuuggau cugugucccc accuaaaucu cgucuugaau 120

uguaauccca augugucaag ggagggaccu ggugggagg 159

<210> 2756
<211> 124
<212> RNA
<213> Homo sapiens

<400> 2756
gcagggacca uucuggaagu accucuucac ccugggguca uucaggaagg agacagucuc 60

uggaaucagu cacuucccuu ccugaagggc cagcugggug gaggaacgga gcugccaccu 120

gcug 124

<210> 2757
<211> 108
<212> RNA
<213> Homo sapiens

<400> 2757
gccuucucuc cuucccgccc ucagggucca cggccaccau ggcguaucag gggcagcagu 60

accuguggca gcauuggccu uugcagcggc ggcagcagca ccaggcuc 108

<210> 2758
<211> 63
<212> RNA
<213> Homo sapiens

<400> 2758
cggccgagga ggagaacaug gcggccgcgg agagcggcug aaaugccugu ucuucaggcc 60

ggg 63

<210> 2759
<211> 73
<212> RNA
<213> Homo sapiens

<400> 2759
ugucuuccug uagggccucc ucuaagucuu gagcccgcag uuccugagag aagaacccug 60

aggaacagac guu 73

<210> 2760
<211> 84
<212> RNA
<213> Homo sapiens

<400> 2760
ugcgggaccu ccccuaaguc uugagaccuc aguuucugag agaagaaccc ugaggaacag 60

acguucccug gcggcccugg cgcc 84

<210> 2761
<211> 167
<212> RNA
<213> Homo sapiens

<400> 2761
cagagagggg agaaaggaga uuugccucug cuuguuaucc ggagcuguag gugaacuguu 60

cugcugcugg uaauugaagu cuaucugcag aacaguuauc aagggcagua accuuuuuuc 120

uagcaauggc cucagauaaa acuuguuuuu cuuauuaaua auuuguu 167

<210> 2762
<211> 75
<212> RNA
<213> Homo sapiens

<400> 2762
uucaaaacaa aggauuauuu guaucuguua caguugggau cugcccaggu agaggggucc 60

uaccaaucuu ggaau 75

<210> 2763
<211> 106
<212> RNA
<213> Homo sapiens

<400> 2763
ugcccggugc ccgaggaggc agccaagcuc guagccugcc acuccgugcg ugaguacggg 60

gcgcgggccg gggguuggga ggucguaguc ccugggaccc ugcauc 106

<210> 2764
<211> 106
<212> RNA
<213> Homo sapiens

<400> 2764
ugcccggugc ccgaggaggc agccaagcuc guggccugcc ucugcgugcg ugaguacggg 60

gcgcgggccg gggguuggga ggucguaguc ucugggaccc ugcauc 106

<210> 2765
<211> 180
<212> RNA
<213> Homo sapiens

<400> 2765
gauccagcau caggccaaca gcagacaccc cauuucugcu cccaguccuc gugcagcuua 60

guggcgcagu ggcggcagca gcaugagcac cccccggccu gcuccaccac agccaggcgc 120

ugcugagggg gcuggaggcc cagaagggaa gguaugcgcu cgggacccug ggggggucgg 180

<210> 2766
<211> 57
<212> RNA
<213> Homo sapiens

<400> 2766
ggcggccgcg ggcggggcgg gagcacucgg cggagccgcu cugccugcgu ccgcucu 57

<210> 2767
<211> 59
<212> RNA
<213> Homo sapiens

<400> 2767
ugcgccugcg caaauuguag gucaagcuug ggccgggguu aggguugcac acgcguaaa 59

<210> 2768
<211> 63

<212>  RNA
<213>  Homo sapiens

<400>  2768
cccuguaaau uauuuuuguu ccagguacgg uggcucaccc cuguaauuuc aauacuuugg      60

gag                                                                    63


<210>  2769
<211>  85
<212>  RNA
<213>  Homo sapiens

<400>  2769
uggauggcug guguggcuuc uugggggagg guaggcaggu gggccccagc ucuucucauc      60

ccugucggcc gcaggcaugu ccaac                                            85


<210>  2770
<211>  53
<212>  RNA
<213>  Homo sapiens

<400>  2770
uaaaauaagu uauugcaucu gacauacuuu gucaguuugu caacagcuuu gaa             53


<210>  2771
<211>  56
<212>  RNA
<213>  Homo sapiens

<400>  2771
uaccugcuuc uuucugccuc ugaagcaagu uucacugagc ggugguagca ggugcc          56


<210>  2772
<211>  88
<212>  RNA
<213>  Homo sapiens

<400>  2772
gcaguagcag ccuguaugua cuggucuugu uugccaaagu aauacaguua aauagauuaa      60

aaaugcaaau aguacuguga uacuguga                                         88


<210>  2773
<211>  68
<212>  RNA
<213>  Homo sapiens

<400>  2773
uuuagacugu cuuugcuuca ucaucugaag guaaaauuuu ccagauacgg cagacggcuu      60

ucagagua                                                               68


<210>  2774
<211>  121
<212>  RNA
<213>  Homo sapiens

<400>  2774
gugcgcagag gcuacugggg gaacaagauc ggcaagcccc acacuguccc uugcaaggug      60

acaggcugcu gcggcucugu gcugguacgc cucaucccug cacccagggg cacuggcauc     120

g                                                                     121


<210>  2775
<211>  60

<212>  RNA
<213>  Homo sapiens

<400>  2775
cggcaagccc cacacugucc cuugcaaggu aggcuggugg caggugaugg gccugccgug      60


<210>  2776
<211>  61
<212>  RNA
<213>  Homo sapiens

<400>  2776
aucggcaagc cccacacugu cccuugcaag gugacaggcc gcugcggcuc ugugcuggug      60

c                                                                      61


<210>  2777
<211>  72
<212>  RNA
<213>  Homo sapiens

<400>  2777
uucggcagcu cccauacugg ugggagaggc aaacuugcag ucugcucauc uuaagauggu      60

gaauguugag au                                                          72


<210>  2778
<211>  93
<212>  RNA
<213>  Homo sapiens

<400>  2778
ccugcugccu ggagaagcug ccugggaugg gcucuggauu ucucugugga gggggcuggg      60

agggcuggaa ugggaggagu caggagcugg gcu                                   93


<210>  2779
<211>  147
<212>  RNA
<213>  Homo sapiens

<400>  2779
gaguuauugg aguguuuuga gcagagcugu uguguagucg gauuauucug gcugcuggac      60

ugucauagac uguuggaggc caagggugga aacaguaaua ggagucagga gcuggggcau     120

ucugggugag agaugacagu ggcuugg                                         147


<210>  2780
<211>  111
<212>  RNA
<213>  Homo sapiens

<400>  2780
ugcccagagc cacacugccu ggcucucuug aagggccugg gcuugccgcc ugcacuucca      60

gggcagccaa cagcccauag gagucaggag cugggaggug ggaggggcau a              111


<210>  2781
<211>  105
<212>  RNA
<213>  Homo sapiens

<400>  2781
cccucauccu gacccuugug cagauagccc gggucaucuu ggaguauauu gaccacaagc      60

ucagagguga gucuggaguc ggaguaggag ucaggagcug gggaa                     105

<210> 2782
<211> 137
<212> RNA
<213> Homo sapiens

<400> 2782
aagcaggcag gcuagacuca gcacacccug gaccucacuc acaaccaagc aucccagagc 60

ccacugggcc ucccuuugga agcugacaga cuggaaggag ucaggagcug gguuccaaag 120

cuaauagagc cucuuac 137


<210> 2783
<211> 84
<212> RNA
<213> Homo sapiens

<400> 2783
ugagggugag ggcacccagg cacccugagg cauggacacu cuucugaggc cuggguccug 60

ggaccugcgu ccguuccccu caca 84


<210> 2784
<211> 101
<212> RNA
<213> Homo sapiens

<400> 2784
cugggcaccg ggagccucuu cggcccuccu ccagcccagc auacaggaag ugcuccguaa 60

gugucugauc aggugaauga gugaaaggua cccucccagc a 101


<210> 2785
<211> 59
<212> RNA
<213> Homo sapiens

<400> 2785
gaugucaucu gccagggauu uccagaaaga gcucucagau ugccucaaaa acacauugg 59


<210> 2786
<211> 54
<212> RNA
<213> Homo sapiens

<400> 2786
ucggauaugc uuuaacucug aguuggcccg caugcucuga guuaaaauuu gaac 54


<210> 2787
<211> 71
<212> RNA
<213> Homo sapiens

<400> 2787
gcauuuauuc ugguaaauau aauuaaaugg uuaguuaaua aaaaguaauu augcucugag 60

uuaaaaugua a 71


<210> 2788
<211> 61
<212> RNA
<213> Homo sapiens

<400> 2788
auaaugguua uuauuuauug agcauuuaca ugugucaugc ucugaguuaa aauuuuuaua 60

u 61

<210> 2789
<211> 98
<212> RNA
<213> Homo sapiens

<400> 2789
uugcuguaua cacauuuugc cucucuauuc aacgaauucu uaugcccucu uguggugauu 60

uuaaugugcg gaagggaaac aauagaaauu uugcaauu 98


<210> 2790
<211> 57
<212> RNA
<213> Homo sapiens

<400> 2790
guuggugagu uugucaagca acuaucgacu ugaucaguuc agccaagcaa cugacaa 57


<210> 2791
<211> 77
<212> RNA
<213> Homo sapiens

<400> 2791
gcucggaggg ugcguggccu ucucgcagcg cggggcuucc cuugaggcca cgcggcgggg 60

ggaggcaccg cuggcaa 77


<210> 2792
<211> 152
<212> RNA
<213> Homo sapiens

<400> 2792
ucccgccgcu gccgccgccg ccgccgccuc ccgcucccgc cgccgcgacc cgcgggaagg 60

cgccugcgca cugcgcgcug cggugggggc gggggcgucc cgccgcggcc cgggagcggg 120

gggcgcgcgg cacguggcgc ucgaguggga gg 152


<210> 2793
<211> 133
<212> RNA
<213> Homo sapiens

<400> 2793
ucugggccug cgcaugcgcg ccacggacga ggcaggccgg gugccugggc cugggagcca 60

uaagccggag agucuggcgg gcuaagcuca gcggcauccg gguuccuugu ggccgccgca 120

gcagcucgag aac 133


<210> 2794
<211> 132
<212> RNA
<213> Homo sapiens

<400> 2794
aagaguaacc aguggaaaau accagcgucc auuuggagga cuugcuggcu cagauugaac 60

agcucuguca cagcugcuag ccacauccuc ugaggggucu cuguguugaa auugguuguu 120

gauguuucuu uu 132


<210> 2795
<211> 115
<212> RNA
<213> Homo sapiens

<400> 2795
gcugcccgcc gcggggcccc aggcgcagca cgcucucgcg cgugggccgc agcuggcagc 60

acaggaaguc cagguggaag agcggcggcg ugggcggccc ggcgcggcgc ggcga 115


<210> 2796
<211> 71
<212> RNA
<213> Homo sapiens

<400> 2796
guggguugag cugcugugag gauuaaauga agcauugcuu gcaaggucau guuaccaaag 60

cagucccaca g 71


<210> 2797
<211> 64
<212> RNA
<213> Homo sapiens

<400> 2797
cugacagacu uguaagugga guuggaaaga ugguugucuc uucagugcuu ccaaccuguc 60

agag 64


<210> 2798
<211> 55
<212> RNA
<213> Homo sapiens

<400> 2798
ggaggcugcu uggcccaggg gcucauauag ccuuuucuga gcacagcgcc uucag 55


<210> 2799
<211> 77
<212> RNA
<213> Homo sapiens

<400> 2799
cuaauacaau uaaguuaaaa acaagguacu uuaccuucau guguugccuc cuguuuguuu 60

uuaguuuguu auuggga 77


<210> 2800
<211> 102
<212> RNA
<213> Homo sapiens

<400> 2800
accagaggaa gggcacagcu guaaggaacu uagggaaaca gugaagucua uuuagguucu 60

uccuuuuuuu uuucuucuuu uuuuuuuuug ccaaucuggu aa 102


<210> 2801
<211> 57
<212> RNA
<213> Homo sapiens

<400> 2801
ucaaaggguu uccuggcaau aaaagaguuu uugugaauga cuacagcauc uuugaga 57


<210> 2802
<211> 55
<212> RNA
<213> Homo sapiens

<400> 2802
ccaucgugca uuggcuaccu gggccacugc guggccaggg cucagagcac uggga 55

<210> 2803
<211> 57
<212> RNA
<213> Homo sapiens

<400> 2803
caguguuaua accuuugaag auggcuacag acuaacacca gggcucagag cacugcu 57

<210> 2804
<211> 58
<212> RNA
<213> Homo sapiens

<400> 2804
gaagggcuca gagcacugcg cauguauaag augagauuag ggagcugggg uauuuccu 58

<210> 2805
<211> 158
<212> RNA
<213> Homo sapiens

<400> 2805
agucaggccu gcggggcuga gccguucugc cuucgucugg ccucacacag ggcucagagc 60

acugccccaa cccuccccca accaggaaaa gcagugcaag cugaguccag aagguucuca 120

ggguuugagu ggcacagcag cuguugaaca acugcuua 158

<210> 2806
<211> 76
<212> RNA
<213> Homo sapiens

<400> 2806
cuuccugaug gcacaucggc acugagacac uccuugugau uuuucauagc ugcguucucu 60

cccacgguag ggagaa 76

<210> 2807
<211> 130
<212> RNA
<213> Homo sapiens

<400> 2807
cugcugcugc caacauuggc ccugcucagg cagcagcggu cccaggaugc caggcugucc 60

uggcuugcug gccuccagca ccgaguggca uggggggccc uggucugggc agccaccugg 120

cagcggcgga 130

<210> 2808
<211> 99
<212> RNA
<213> Homo sapiens

<400> 2808
agagcaccca uguauucaag cacuacuaga uucuuauauc caacaacaac aacaaaacag 60

uuauugggaa ccuacugugg gccaggcaau uuguucuga 99

<210> 2809
<211> 82
<212> RNA
<213> Homo sapiens

<400> 2809
gacaaaccuc acgaguguca caaagaaccg ugcagauaag guaaauggug ccguuugugg 60

caugugaacu caggcguguc ag 82

<210> 2810
<211> 60
<212> RNA
<213> Homo sapiens

<400> 2810
cucaucccgg uccagaccca gcaccccaua cggaaggguc ugcaucacaa gguaagagcc 60

<210> 2811
<211> 84
<212> RNA
<213> Homo sapiens

<400> 2811
ccuccuggac aaggcggcug gcugggcagg gggcugaccc ccccaccccc cucccggacg 60

ggucggcugc cgggcagaga ggcu 84

<210> 2812
<211> 73
<212> RNA
<213> Homo sapiens

<400> 2812
ggauguacga gggcuucugg gggagcugcc agcaggaaag ggagggugcu gugggagcca 60

gguuuucauc ucc 73

<210> 2813
<211> 125
<212> RNA
<213> Homo sapiens

<400> 2813
ggaguuggca gcccuggugu cugcgcucua auuagacuau ugcugugaca agcucuuggc 60

uguguucuca gcuucccagc uugguccccg gugcuguggg agccagguuu ucuagccuac 120

uucuu 125

<210> 2814
<211> 95
<212> RNA
<213> Homo sapiens

<400> 2814
agggacugca gaagccaugg cuccauugca agccgguccc caaggcucag gaaagcugcc 60

cggggcgggu ggagucucau cuguguguac ccuac 95

<210> 2815
<211> 63
<212> RNA
<213> Homo sapiens

<400> 2815
cugauucacu uuucauccuu uccuuguuug uuuagacugg accaagagga uugaguacca 60

gcc 63

<210> 2816
<211> 83
<212> RNA
<213> Homo sapiens

<400> 2816
uaauuucaua caagaauuga cugauguuga ccuuaagaaa uuauuacuca uauacaaugu 60

acauuaguuc cuaugacauu agc 83


<210> 2817
<211> 109
<212> RNA
<213> Homo sapiens

<400> 2817
gaauugacug auguugauga uuuuaaaagu gaaaccauau gccaaauugc cucucaagag 60

cuaagaaaua aacucccgcu gauguucucu cagccuuugu cuuguuuga 109


<210> 2818
<211> 61
<212> RNA
<213> Homo sapiens

<400> 2818
gugaguugcu ggacaccgcg uuggguuugg agcauugacu gauguugagc aaacuccgca 60

c 61


<210> 2819
<211> 86
<212> RNA
<213> Homo sapiens

<400> 2819
gauauuugca uuuuccuauu gacugauguu gagcacguuu ucaugugcuu auuugguauc 60

caguuaucuu cuuugguaua uauuug 86


<210> 2820
<211> 56
<212> RNA
<213> Homo sapiens

<400> 2820
uauagauaau ugacugaugu ugaaacucaa gcccaauuua uagaugauuc ugugua 56


<210> 2821
<211> 56
<212> RNA
<213> Homo sapiens

<400> 2821
aggcccaucu cagccccugu gaaacugcac cucauccuac agggcuauga accuac 56


<210> 2822
<211> 88
<212> RNA
<213> Homo sapiens

<400> 2822
gggcaguccu cacugaccuc cgugcaugcg gucuccuguc cacugccugu ugcagaggcu 60

guucccugga ugguccucac ugaccucu 88


<210> 2823
<211> 97
<212> RNA
<213> Homo sapiens

<400> 2823
ggcaguagcc aaacaguggu ugccggggcc uugagcaaga cucagugcug ugcuggcuuc 60

agguuugacc cugugcaguu ccaguggugg uggccaa 97

<210> 2824
<211> 94
<212> RNA
<213> Homo sapiens

<400> 2824
cggcggcggc ggcggaggcg ggggcggggg uggugggggc ggcaggaaau ccuccuccgc 60

cgccgccucc uccuccgccu ccuccuccuc ggcg 94

<210> 2825
<211> 103
<212> RNA
<213> Homo sapiens

<400> 2825
ugccuugggc gggcggcugu uaagacuugc agugauguuu aacuccucuc cacgugaaca 60

ucacagcaag ucugugcugc uucccguccc uacgcugccu ggg 103

<210> 2826
<211> 105
<212> RNA
<213> Homo sapiens

<400> 2826
gaacuuuggc caccuggcac ugagaacuga auuccauagg cugugagcuc uagcaaugcc 60

cuguggacuc aguucuggug cccggcagug cuacaacauc aaugc 105

<210> 2827
<211> 111
<212> RNA
<213> Homo sapiens

<400> 2827
ucuugucugc ucccccucuu gaauccuugg aaccuaggug ugagugcuau uucagugcaa 60

cacaccuauu caaggauuca aagaggcuga gccuugucua cauguagaag g 111

<210> 2828
<211> 111
<212> RNA
<213> Homo sapiens

<400> 2828
ugccggcgag cgccugcgcc cuucccuggc gugaggguau gugccuuugg acuacaucgu 60

ggaagccagc accaugcagu ccaugggcau auacacuugc cucaaggccu a 111

<210> 2829
<211> 105
<212> RNA
<213> Homo sapiens

<400> 2829
agaaauccua cucggaugga uauaauacaa ccugcuaagu guccuagcac uuagcagguu 60

guauuaucau uguccguguc uauggcucuc gucuaccaga cuuua 105

<210> 2830
<211> 111
<212> RNA
<213> Homo sapiens

<400> 2830
cgccacgaau ggcuaugcac ugcacaaccc uaggagaggg ugccauucac auagacuaua 60

auugaauggc gccacuaggg uugugcagug cacaaccuac acaacuguac u 111


<210> 2831
<211> 105
<212> RNA
<213> Homo sapiens

<400> 2831
gaauuguugg uguuagcccu gcggccccac gcaccagggu aagagagacu cucgcuuccu 60

gcccuggccc gagggaccga cuggcugggc cugccuucug cccag 105


<210> 2832
<211> 101
<212> RNA
<213> Homo sapiens

<400> 2832
gagcgggcgg gcggcggcgg cggcagagua ggcccuuccu caggccggga gaaggcggcg 60

gcggagcggc ggcugugcgg ccccgggcuc cguguguucg g 101


<210> 2833
<211> 109
<212> RNA
<213> Homo sapiens

<400> 2833
ucucuuucac cuccuuuucu cuuuccagga gaggaaauuu gugggcggau cuggucaagu 60

gagugagcgg auaauggacc uccuuggaga ccgagugaag cuggagagg 109


<210> 2834
<211> 83
<212> RNA
<213> Homo sapiens

<400> 2834
gcggcugcag uggcggcugc agaaagcgug gagugaaugg accagagacc gagccgcgcu 60

gcugccgccg ccgccgccgc cgc 83


<210> 2835
<211> 100
<212> RNA
<213> Homo sapiens

<400> 2835
uuaugaacaa ugucuuugua guucuugcgg cugugugaac uaguuuaucu cugauuaagg 60

uucaugcugc auuuccugcu auauaugaac auuguccuga 100


<210> 2836
<211> 71
<212> RNA
<213> Homo sapiens

<400> 2836
cacagcucuu guccuggugu gcuagaguuc ucgaugaauc uacuggucuu gauucacugg 60

caggagcagu g 71


<210> 2837
<211> 108
<212> RNA

<213>  Homo sapiens

<400>  2837
cagaauuguc acccccaagu guuaugggcc cggagcgaag acaguaguac ucccgagcgg      60

gaggaugauu ucucggaggu ugguagaggu uucuuggagg uuguuuug                  108


<210>  2838
<211>  63
<212>  RNA
<213>  Homo sapiens

<400>  2838
uagauaacau uguaaagcgc uucuuucgcg guugggcugg agcaacucuu uacaauguuu      60

cua                                                                    63


<210>  2839
<211>  107
<212>  RNA
<213>  Homo sapiens

<400>  2839
uggugugcuc acccucaguc ugcagcagcu cgaagcccuc cugaugagag ccgacacagg      60

caggggccag agucgcuuuu gcagcgcugg aaggcugagu ucugcca                   107


<210>  2840
<211>  66
<212>  RNA
<213>  Homo sapiens

<400>  2840
acgaagcacu uccccgaguu caggugaauu ggaugcagug ucccuguuag ucaugggcug      60

uuuugu                                                                 66


<210>  2841
<211>  82
<212>  RNA
<213>  Homo sapiens

<400>  2841
aucuuggaag gcucugaacc ucagaaagga uucacaguaa guuaaccaug uagaucugag      60

aggagaguag cuucuuguag au                                               82


<210>  2842
<211>  110
<212>  RNA
<213>  Homo sapiens

<400>  2842
gcaaacugug ucucaggaag cagccccuau ccuggaaugc cggucgauuc uaaguucuac      60

aagaugauca aggaaggcuu ccggaugcuc agcccugaac acgcaccugc                110


<210>  2843
<211>  108
<212>  RNA
<213>  Homo sapiens

<400>  2843
ggugguggug gagguggagg agguggaggu aaugcagaua ucccugugga ggaaaagggg      60

gacaaucagu auucaccucc acagcugccc ugcacacacg cugcugcu                  108


<210>  2844

<211> 81
<212> RNA
<213> Homo sapiens

<400> 2844
aagcagaaug augucguccg aauucucccc ugcaaguaug ucaacuucau uuguuugaga 60

aagaaugaua uuaaugugcu u 81


<210> 2845
<211> 110
<212> RNA
<213> Homo sapiens

<400> 2845
cggccgggcc ggggcagccg ggaagcgggu gggguggugu guuacccagu agcuccuggg 60

acaucgcucg gguacgcucc acgccgucgc agccacugcu guggucgccg 110


<210> 2846
<211> 75
<212> RNA
<213> Homo sapiens

<400> 2846
aacagcauga uggcauuuga ugcuuggauu uaauguuagu uucagggaga caaaauggag 60

ugcuagaggg cuguu 75


<210> 2847
<211> 60
<212> RNA
<213> Homo sapiens

<400> 2847
gccgaccugc agacuggggc cuaaguuucu uuuaguuucc ggugucucug caauggcggc 60


<210> 2848
<211> 106
<212> RNA
<213> Homo sapiens

<400> 2848
cagcagaggg gaaauugugg ucauuggugc uacgaacagg cuagauucua uagauccugc 60

uuuacgaagg ccuggucgcu uugauagaga auuccucuuu agccug 106


<210> 2849
<211> 69
<212> RNA
<213> Homo sapiens

<400> 2849
cccgggacgc ggcggagccc ggggacagcg gggacgccgc ccaguccucc gggugagccg 60

ccccugagg 69


<210> 2850
<211> 79
<212> RNA
<213> Homo sapiens

<400> 2850
gaaucacccu aacaagccgc aacguaaaau ccuuggaaaa gggucagugc auucguuaug 60

cgggggggag ggggaauug 79


<210> 2851

<211>  89
<212>  RNA
<213>  Homo sapiens

<400>  2851
gcacuuggug auaaacguuu ucuaaauuuu uaguuaagua uauggauaaa aaguuaugaa      60

cuauuaaaaa ugugaugugg accaaaggc                                        89


<210>  2852
<211>  23
<212>  RNA
<213>  Homo sapiens

<400>  2852
cccccacaac cgcgcuugac uag                                              23

## Description

### Technical Field

The present invention relates to a novel nucleic acid, a method of expressing or suppressing the nucleic acid, and a diagnostic reagent or a therapeutic agent comprising the nucleic acid.

### Background Art

A micro-RNA (miRNA), which is one type of nucleic acid, is a small non-coding single-stranded RNA of about 22 nucleotides that is not translated into a protein, and has been confirmed as being present in many types in organisms, including humans (non-patent documents 1 and 2).
A micro-RNA is produced from a gene transcribed to a single or clustered micro-RNA precursor. Specifically, first, a primary-microRNA (pri-miRNA), which is a primary transcript, is transcribed from the gene, then, in stepwise processing from the pri-miRNA to a mature type micro-RNA, a precursor-microRNA (pre-miRNA) of about 70 nucleotides having a characteristic hairpin structure is produced from the pri-miRNA. Furthermore, the mature type micro-RNA is produced from the pre-miRNA by Dicer-mediated processing (non-patent document 3).

A mature type micro-RNA is thought to be involved in the post-transcriptional control of gene expression by complementarily binding to a target mRNA to suppress the translation of the mRNA, or to degrade the mRNA. As of May 2006, in the micro-RNA database miRBase (http://microrna.sanger.ac.uk/), 455 species of micro-RNAs were registered for humans, and 3685 species for all organisms. Of the micro-RNAs expressed in mammals, including humans, only some have their physiological functions elucidated to date, including miR-181, which is involved in hematopoietic lineage differentiation (non-patent document 4), miR-375, which is involved in insulin secretion (non-patent document 5), and the like; many have their bioactivities unclarified. However, studies using nematodes or Drosophila have shown that micro-RNAs play various important roles in the development and differentiation in organisms, and a report of the relation to human diseases has been presented suggesting a profound relation to cancers (non-patent document 6).

For identification of micro-RNAs, there are a method wherein a low-molecular RNA is cloned from a cell, a method wherein bioinformatics is applied to genome sequence information, and the like. Registration of any micro-RNA in the miRBase requires both information on the expression and information on the biosynthesis and structure; a structural prediction from genome sequence information only does not suffice approval as a micro-RNA (non-patent document 7).

Mast cells are known to be activated by various stimuli to undergo degranulation and release or produce many inflammatory mediators (non-patent documents 8 to 10). For example, it is known that when an antigen is recognized by a mast cell, histamine and tryptase are quickly released upon degranulation, and chemical mediators such as prostaglandin D2 (PGD2), leukotriene (LT), and platelet activation factor (PAF), various chemokines such as macrophage inflammatory protein (MIP)-1α, and various cytokines such as granulocyte macrophage colony stimulation factor (GM-CSF) are newly synthesized and released. Regarding major basic proteins, which are cytotoxic proteins that have been thought to be produced by eosinophils, it has recently been shown that in the case of humans, they are produced in large amounts by mast cells (non-patent document 11).

Hence, mast cells are thought to play major roles in the pathogenesis of various allergic diseases; therefore, it is thought that by controlling a function of mast cells, treatment of allergic diseases is possible.
However, it is known that rodent mast cells and human mast cells have different reactivities to drugs (non-patent document 9). Specifically, sodium cloroglycate, which is used as a suppressant of inflammatory mediator release, remarkably suppresses the IgE-dependent release of inflammatory mediators in rat abdominal mast cells, but the action thereof on human mast cells is not potent (non-patent document 12). Azelastine hydrochloride, at high concentrations, suppresses the release of histamine, PGD2, and LT and production of GM-CSF and MIP-1α, from human mast cells in culture, but none of these activities are potent. Suplatast tosilate, which is used as an anti-cytokine drug, exhibits inflammatory mediator release suppressive action on rat mast cells, but lacks action on human mast cells (non-patent document 12).

As a result of a comparison of genes expressed in human mast cells and mouse mast cells, it is known that the genes expressed by various stimuli do not always agree (non-patent document 13).
Regarding micro-RNAs, micro-RNAs expressed in mouse bone marrow derived mast cells have been reported (non-patent document 14), but no relationship is known between a micro-RNA and a function of mast cells. No report is available on a micro-RNA expressed in human mast cells; taking into account the above-described interspecific differences between humans and mice, it is difficult to predict information on the expression of micro-RNAs in human mast cells on the basis of information on the expression of micro-RNAs in mouse mast cells.

Mesenchymal stem cells are present in mammalian bone marrow, fat tissue, umbilical blood and the like, and are known as multipotent stem cells that differentiate into adipocytes, chondrocytes, osteocytes and the like. Mesenchymal stem cells, because of the multipotency thereof, are attracting attention as graft materials for regenerative medicine for many tissues, including bones, cartilage, tendons, muscles, fat, and periodontal tissue (non-patent document 15).
Mesenchymal stem cells can be differentiated into particular cells in vitro by the addition of a drug, a cytokine and the like; for example, differentiation into adipocytes can be induced by allowing 1-methyl-3-isobutylxanthine, dexamethasone, insulin and indomethacin to act, and differentiation into osteoblasts can be induced by allowing dexamethasone, β-glycerol phosphate, and ascorbic acid to act (non-patent document 16). However, details of the molecular mechanisms in these differentiation processes are unknown. From the results of gene expression analyses on gene-knockout mice and in the differentiation stage, it is known that differentiation into adipocytes is mediated by the PPARγ and C/EBP families, and that during osteoblast differentiation, the expression of genes such as Cbfa1/Runx2 and Osterix is involved (non-patent document 17); however, the mechanisms of differentiation from mesenchymal stem cells cannot be explained solely on the basis of these genes, and artificial control of the differentiation and proliferation has not been realized. Also, no micro-RNA is known to act on the differentiation and proliferation of mesenchymal stem cells.

Because micro-RNAs are involved in the control of the expression of a wide variety of genes, abnormalities of micro-RNAs are supposed to be involved in various human diseases. Particularly in cancers, research has been advanced; it has been reported that in many cancers, the expression of micro-RNAs differs from that in normal tissues, that classification of cancers is enabled by expression profile analyses of micro-RNAs, and the like (non-patent document 18). It is also known that about half of the human micro-RNAs that have been found so far are present in chromosome aberrations or fragile portions of chromosomes known in human cancers (non-patent document 19). Examples of relationships between cancers and micro-RNAs that have been reported so far include the finding that the miR-15a/miR-16 cluster is contained in chromosome 13q14, which is deleted in B cell chronic lymphatic leukemia (B-CLL), the deletion being supposed to be a cause of B-CLL (non-patent document 20), the finding that in lung cancer, the expression of Let-7, which is a micro-RNA, is decreased, one of the targets thereof being Ras, which is known as a carcinogenic gene (non-patent documents 21 and 22), and the like. Many micro-RNAs have their expression decreased in cancer cells; conversely, however, there are some micro-RNAs with gene amplification or overexpression in cancers. For example, in regions where gene amplification is seen in malignant lymphoma, a cluster consisting of six species of micro-RNAs (miR-17-92) is present; it has been reported that when this miRNA cluster gene is forcibly expressed in a mouse model of human B cell lymphoma, the onset of lymphoma is promoted (non-patent document 23). It has also been shown that a gene called BIC, which does not encode a protein and has been regarded as a candidate for the cancer gene that is overexpressed in Hodgkin lymphoma, encodes miR-155 (non-patent document 24).

As stated above, relationships between cancers and micro-RNAs have recently been reported in many cases, but most of them show expression abnormalities in cancer cells; there are only a few studies showing a function of a micro-RNA, including a report that the proliferation of a cancer cell line was inhibited by forcibly expressing Let-7 in a lung cancer cell line (non-patent document 25) and the like. Currently, no report is available that cancer growth was suppressed in an animal model by administering a micro-RNA or a precursor thereof, or an antisense oligonucleotide thereof, from outside the body to increase or decrease the expression of the micro-RNA.
non-patent document 1: Science, 294, 853-858 (2001)
non-patent document 2: Cell, 113, 673-676 (2003)
non-patent document 3: Nature Reviews Genetics, 5, 522-531 (2004)
non-patent document 4: Science, 303, 83-86 (2004)
non-patent document 5: Nature, 432, 226-230 (2004)
non-patent document 6: Nature Reviews Cancer, 6, 259-269 (2006)
non-patent document 7: RNA, 9, 277-279 (2003)
non-patent document 8: Himan Saibo no Rinsho, ed. Motohito Kurosawa, Sentan Igaku-sha Ltd., p 142 (2001)
non-patent document 9: Himan Saibo no Rinsho, ed. Motohito Kurosawa, Sentan Igaku-sha Ltd., p 559 (2001)
non-patent document 10: Crit. Rev. Immunol., 22, 115-140 (2002)]
non-patent document 11: Blood, 98, 1127-1134 (2001)
non-patent document 12: Clin. Exp. Allergy, 28, 1228-1236 (1998)
non-patent document 13: Blood, 100, 3861-3868 (2002)
non-patent document 14: Genome Biology, 6, R71 (2005)
non-patent document 15: Idenshi Igaku, vol. 4, p. 58-61 (2000)
non-patent document 16: Science, 284, 143-147 (1999)
non-patent document 17: Jikken Igaku, vol. 20, p. 2459-2464 (2000)
non-patent document 18: Nature, 435, 839-843 (2005)
non-patent document 19: Proc.Natl.Acad.Sci.USA, 101, 2999-3004 (2004)
non-patent document 20: Proc.Natl.Acad.Sci.USA, 99, 15524-15529 (2002)
non-patent document 21: Cancer Research, 64, 3753-3756, (2004)
non-patent document 22: Cell, 120, 635-647, (2005)
non-patent document 23: Nature, 435, 823-833 (2005)
non-patent document 24: Proc.Natl.Acad.Sci.USA, 102, 3627-3632 (2005)
non-patent document 25: Cancer Research, 64, 3753-3756, (2004)

### [Disclosure of the Invention]

### [Problems to Be Solved by the Invention]

It is expected that by identifying micro-RNAs expressed in various human organs, and analyzing the functions thereof to elucidate their relations to diseases, new therapeutic agents and diagnostic reagents will be developed.
Finding a micro-RNA that acts in mast cells is expected to lead to the functional elucidation of differentiation, degranulation, inflammatory mediator production, cytokine production, chemokine production and the like in mast cells, and to lead to the development of methods of isolation, cultivation, differentiation control, degranulation control, inflammatory mediator production control, cytokine production control, and chemokine production control for mast cells, as well as new therapies for allergic diseases and the like based thereon.

In addition, finding a micro-RNA that acts in mesenchymal stem cells is expected to lead to the functional elucidation of differentiation and proliferation of mesenchymal stem cells, and to lead to the development of a method of controlling the differentiation from mesenchymal stem cells to particular cells and a new therapy based on differentiation control.
Furthermore, finding a micro-RNA that causes cancer cell proliferation or suppression is expected not only to help to understand the mechanisms of carcinogenesis, but also to lead to the development of diagnostic reagents and therapeutic agents for human cancers, and new diagnostic methods and therapies for cancers based thereon. Furthermore, regarding diseases other than cancers, the same is expected to contribute to the development of diagnostic reagents and therapeutic agents for diseases caused by abnormal proliferation of cells, tissue hyperplasia and the like, such as arteriosclerosis, rheumatoid arthritis, prostatic hyperplasia, blood vessel restenosis after percutaneous transvascular coronary angioplasty, fibroid lung, glomerulonephritis, and autoimmune diseases, and diagnostic methods and therapies based thereon.

It is an object of the present invention to acquire micro-RNAs, and to provide nucleic acids that are useful in isolation, cultivation, differentiation control, degranulation control, inflammatory mediator production control, cytokine production control, and chemokine production control in mast cells, diagnosis and treatment of allergic diseases, control of mesenchymal stem cell differentiation and proliferation, control of cancer cell differentiation and proliferation, and diagnosis and treatment of diseases such as cancers, as well as methods of utilizing the same.

### [Means of Solving the Problems]

The present invention relates to (1) to (64) below.
(1) A nucleic acid consisting of the nucleotide sequence of any one of SEQ ID NOs:1 to 1336.
(2) A nucleic acid consisting of a nucleotide sequence having an identity of 90% or more to the nucleotide sequence of any one of SEQ ID NOs:1 to 1336.
(3) A nucleic acid that hybridizes under stringent conditions with a strand complementary to a nucleic acid consisting of the nucleotide sequence of any one of SEQ ID NOs:1 to 1336.
(4) A nucleic acid comprising the nucleic acid described in any one of (1) to (3).
(5) A nucleic acid consisting of the nucleotide sequence of any one of SEQ ID NOs:1337 to 2851.
(6) A nucleic acid consisting of a nucleotide sequence having an identity of 80% or more to the nucleotide sequence of any one of SEQ ID NOs:1337 to 2851.
(7) A nucleic acid that hybridizes under stringent conditions with a strand complementary to a nucleic acid consisting of the nucleotide sequence of any one of SEQ ID NOs:1337 to 2851.
(8) A nucleic acid consisting of a nucleotide sequence complementary to the nucleic acid described in any one of (1) to (7).
(9) A double-stranded nucleic acid consisting of the nucleic acid described in any one of (1) to (7) and a nucleic acid consisting of a nucleotide sequence complementary to the nucleotide sequence of the nucleic acid.
(10) A vector which expresses the nucleic acid described in any one of (1) to (9).
(11) A method of detecting the expression or mutation of the nucleic acid described in any one of (1) to (9), comprising using the nucleic acid described in any one of (1) to (9).
(12) A method for screening a substance that promotes or suppresses the expression or function of the nucleic acid described in any one of (1) to (9), comprising using the nucleic acid described in any one of (1) to (9).
(13) A method of separating a cell that expresses the nucleic acid described in any one of (1) to (9), comprising using the nucleic acid described in any one of (1) to (9).
(14) A method of suppressing the expression of a target gene of the nucleic acid described in any one of (1) to (9), comprising using the nucleic acid described in any one of (1) to (9).
(15) A diagnostic reagent or a therapeutic agent for a disease caused by a mast cell abnormality, comprising the nucleic acid described in any one of (1) to (9) as an active ingredient.
(16) A diagnostic reagent or a therapeutic agent for a disease caused by a mast cell abnormality, comprising a substance that promotes or suppresses the expression or function of the nucleic acid described in any one of (1) to (9) as an active ingredient.
(17) A method for screening a diagnostic reagent or a therapeutic agent for a disease caused by a mast cell abnormality, wherein promotion or suppression of the expression or function of the nucleic acid described in any one of (1) to (9) serves as an index.
(18) A diagnostic reagent or a therapeutic agent for a disease caused by a mast cell abnormality, comprising a substance that suppresses the expression of a target gene of the nucleic acid described in any one of (1) to (9) as an active ingredient.
(19) A method for screening a diagnostic reagent or a therapeutic agent for a disease caused by a mast cell abnormality, wherein suppression of the expression of a target gene of the nucleic acid described in any one of (1) to (9) serves as an index.
(20) A cell incorporating the nucleic acid or vector described in any one of (1) to (10).
(21) A mast cell degranulation promoter comprising the nucleic acid described in any one of (1) to (4) wherein the SEQ ID NO: is any one of 1, 2, 3, 8, 14, 20, 22, 25, 32 and 36, as an active ingredient.
(22) A mast cell degranulation promoter comprising the nucleic acid described in any one of (5) to (7) wherein the SEQ ID NO: is any one of 1337, 1338, 1339, 1352, 1363, 1371, 1373, 1377, 1386 and 1390, as an active ingredient.
(23) A mast cell degranulation suppressant comprising a nucleic acid consisting of a nucleotide sequence complementary to the nucleic acid described in (21) or (22) as an active ingredient.
(24) A mast cell degranulation promoter or degranulation suppressant comprising a double-stranded nucleic acid consisting of the nucleic acid described in (21) or (22) and a nucleic acid consisting of a nucleotide sequence complementary to the nucleotide sequence of the nucleic acid, as an active ingredient.
(25) A mast cell degranulation promoter or degranulation suppressant comprising a substance that promotes or suppresses the expression or function of the nucleic acid described in (21) or (22) as an active ingredient.
(26) A method for screening a mast cell degranulation promoter or degranulation suppressant, wherein promotion or suppression of the expression or function of the nucleic acid described in (21) or (22) serves as an index.
(27) A mast cell degranulation promoter or degranulation suppressant comprising a substance that suppresses or promotes the expression of a target gene of the nucleic acid described in (21) or (22) as an active ingredient.
(28) A method for screening a mast cell degranulation promoter or degranulation suppressant, wherein suppression or promotion of the expression of a target gene of the nucleic acid described in (21) or (22) serves as an index.
(29) A diagnostic reagent or a therapeutic agent for a disease caused by an abnormality of mesenchymal stem cell proliferation and/or differentiation, comprising the nucleic acid described in any one of (1) to (4) wherein the SEQ ID NO: is any one of 1, 8, 21 and 36, as an active ingredient.
(30) A diagnostic reagent or a therapeutic agent for a disease caused by an abnormality of mesenchymal stem cell proliferation and/or differentiation, comprising the nucleic acid described in any one of (5) to (7) wherein the SEQ ID NO: is any one of 1337, 1352, 1372 and 1390, as an active ingredient.
(31) A diagnostic reagent or a therapeutic agent for a disease caused by an abnormality of mesenchymal stem cell proliferation and/or differentiation, comprising a nucleic acid consisting of a nucleotide sequence complementary to the nucleic acid described in (29) or (30) as an active ingredient.
(32) A diagnostic reagent or a therapeutic agent for a disease caused by an abnormality of mesenchymal stem cell proliferation and/or differentiation, comprising a double-stranded nucleic acid consisting of the nucleic acid described in (29) or (30) and a nucleic acid consisting of a nucleotide sequence complementary to the nucleotide sequence of the nucleic acid as an active ingredient.
(33) A diagnostic reagent or a therapeutic agent for a disease caused by an abnormality of mesenchymal stem cell proliferation and/or differentiation, comprising a substance that promotes or suppresses the expression or function of the nucleic acid described in (29) or (30) as an active ingredient.
(34) A method for screening a diagnostic reagent or a therapeutic agent for a disease caused by an abnormality of mesenchymal stem cell proliferation and/or differentiation, wherein promotion or suppression of the expression or function of the nucleic acid described in (29) or (30) serves as an index.
(35) A diagnostic reagent or a therapeutic agent for a disease caused by an abnormality of mesenchymal stem cell proliferation and/or differentiation, comprising a substance that suppresses or promotes the expression of a target gene of the nucleic acid described in (29) or (30) as an active ingredient.
(36) A method for screening a diagnostic reagent or a therapeutic agent for a disease caused by an abnormality of mesenchymal stem cell proliferation and/or differentiation, wherein suppression or promotion of the expression of a target gene of the nucleic acid described in (29) or (30) serves as an index.
(37) A mesenchymal stem cell proliferation promoter comprising the nucleic acid described in any one of (1) to (4) wherein the SEQ ID NO: is 1, as an active ingredient.
(38) A mesenchymal stem cell proliferation promoter comprising the nucleic acid described in any one of (5) to (7) wherein the SEQ ID NO: is 1337, as an active ingredient.
(39) A mesenchymal stem cell proliferation suppressant comprising a nucleic acid consisting of a nucleotide sequence complementary to the nucleic acid described in (37) or (38) as an active ingredient.
(40) A mesenchymal stem cell proliferation suppressant comprising the nucleic acid described in any one of (1) to (4) wherein the SEQ ID NO: is any one of 8, 21 and 36, as an active ingredient.
(41) A mesenchymal stem cell proliferation suppressant comprising the nucleic acid described in any one of (5) to (7) wherein the SEQ ID NO: is any one of 1352, 1372 and 1390, as an active ingredient.
(42) A mesenchymal stem cell proliferation promoter comprising a nucleic acid consisting of a nucleotide sequence complementary to the nucleic acid described in (37) or (38) as an active ingredient.
(43) A mesenchymal stem cell proliferation promoter or proliferation suppressant comprising a double-stranded nucleic acid consisting of the nucleic acid described in any one of (37) to (42) and a nucleic acid consisting of a nucleotide sequence complementary to the nucleotide sequence of the nucleic acid, as an active ingredient.
(44) A mesenchymal stem cell proliferation promoter or proliferation suppressant comprising a substance that promotes or suppresses the expression or function of the nucleic acid described in any one of (37) to (42), as an active ingredient.
(45) A method for screening a mesenchymal stem cell proliferation promoter or proliferation suppressant, wherein promotion or suppression of the expression or function of the nucleic acid described in any one of (37) to (42) serves as an index.
(46) A mesenchymal stem cell proliferation promoter or proliferation suppressant comprising a substance that suppresses or promotes the expression of a target gene of the nucleic acid described in any one of (37) to (42) as an active ingredient.
(47) A method for screening a mesenchymal stem cell proliferation promoter or proliferation suppressant, wherein suppression or promotion of the expression of a target gene of the nucleic acid described in any one of (37) to (42) serves as an index.
(48) A diagnostic reagent or a therapeutic agent for a disease caused by a cell proliferation abnormality, comprising the nucleic acid described in any one of (1) to (4) wherein the SEQ ID NO: is any one of 1, 3, 8, 20, 21, 22, 32 and 36, as an active ingredient.
(49) A diagnostic reagent or a therapeutic agent for a disease caused by a cell proliferation abnormality, comprising the nucleic acid described in any one of (5) to (7) wherein the SEQ ID NO: is any one of 1337, 1339, 1352, 1371, 1372, 1373, 1386 and 1390, as an active ingredient.
(50) A diagnostic reagent or a therapeutic agent for a disease caused by a cell proliferation abnormality, comprising a nucleic acid consisting of a nucleotide sequence complementary to the nucleic acid described in (48) or (49) as an active ingredient.
(51) A diagnostic reagent or a therapeutic agent for a disease caused by a cell proliferation abnormality, comprising a double-stranded nucleic acid consisting of the nucleic acid described in (48) or (49) and a nucleic acid consisting of a nucleotide sequence complementary to the nucleic acid as an active ingredient.
(52) A diagnostic reagent or a therapeutic agent for a disease caused by a cell proliferation abnormality, comprising a substance that promotes or suppresses the expression or function of the nucleic acid described in (48) or (49) as an active ingredient.
(53) A method for screening a diagnostic reagent or a therapeutic agent for a disease caused by a cell proliferation abnormality, wherein promotion or suppression of the expression or function of the nucleic acid described in (48) or (49) serves as an index.
(54) A diagnostic reagent or a therapeutic agent for a disease caused by a cell proliferation abnormality, comprising a substance that suppresses or promotes the expression of a target gene of the nucleic acid described in (48) or (49) as an active ingredient.
(55) A method for screening a diagnostic reagent or a therapeutic agent for a disease caused by a cell proliferation abnormality, wherein suppression or promotion of the expression of a target gene of the nucleic acid described in (48) or (49) serves as an index.
(56) The diagnostic reagent, the therapeutic agent or the screening method described in any one of (48) to (55), wherein the disease caused by a cell proliferation abnormality is a disease selected from the group consisting of cancers, arteriosclerosis, rheumatoid arthritis, prostatic hyperplasia, blood vessel restenosis after percutaneous transvascular coronary angioplasty, fibroid lung, glomerulonephritis and autoimmune diseases.
(57) A cell proliferation suppressant comprising the nucleic acid described in any one of (1) to (4) wherein the SEQ ID NO: is any one of 1, 3, 8, 20, 21, 22, 32 and 36, as an active ingredient.
(58) A cell proliferation suppressant comprising the nucleic acid described in any one of (5) to (7) wherein the SEQ ID NO: is any one of 1337, 1339, 1352, 1371, 1372, 1373, 1386 and 1390, as an active ingredient.
(59) A cell proliferation promoter comprising a nucleic acid consisting of a nucleotide sequence complementary to the nucleic acid described in (57) or (58) as an active ingredient.
(60) A cell proliferation suppressant or proliferation promoter comprising a double-stranded nucleic acid consisting of the nucleic acid described in (57) or (58) and a nucleic acid consisting of a nucleotide sequence complementary to the nucleotide sequence of the nucleic acid as an active ingredient.
(61) A cell proliferation suppressant or proliferation promoter comprising a substance that promotes or suppresses the expression or function of the nucleic acid described in (57) or (58) as an active ingredient.
(62) A method for screening a cell proliferation suppressant or proliferation promoter, wherein promotion or suppression of the expression or function of the nucleic acid described in (57) or (58) serves as an index.
(63) A cell proliferation suppressant or proliferation promoter comprising a substance that suppresses or promotes the expression of a target gene of the nucleic acid described in (57) or (58) as an active ingredient.
(64) A method for screening a cell proliferation suppressant or proliferation promoter, wherein suppression or promotion of the expression of a target gene by the nucleic acid described in (57) or (58) serves as an index.

### [Effect of the Invention]

According to the present invention, it is possible to provide a novel nucleic acid, a vector that expresses the nucleic acid, a method of detecting the expression and mutation of the nucleic acid, a screening method for a substance that controls the nucleic acid, a method of separating a cell that expresses the nucleic acid, a method of controlling the expression of a target gene of the nucleic acid, a diagnostic reagent or pharmaceutical comprising the nucleic acid or a substance that controls the nucleic acid as an active ingredient, and a diagnostic reagent or pharmaceutical comprising a substance that controls the expression of a target gene of the nucleic acid, as well as a diagnostic reagent or a therapeutic agent for a disease caused by an abnormality of mast cells or mesenchymal stem cells and the like, and diseases such as cancers, an agent that controls cell differentiation or proliferation, and the like.

### [Brief Description of the Drawings]

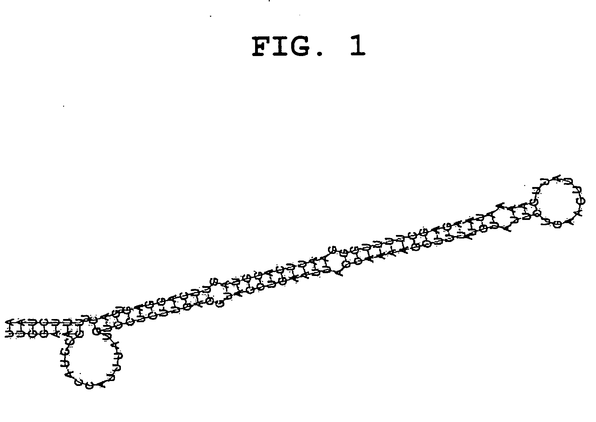

[FIG. 1] shows the secondary structure of the nucleotide sequence of KHK_miR_1194 of SEQ ID NO:1580.

### [Best Mode for Carrying out the Invention]

As the nucleic acid in the present invention, the following nucleic acids can be mentioned. The nucleic acid is preferably a micro-RNA or a derivative thereof, a micro-RNA precursor or a derivative thereof, or a double-stranded nucleic acid (hereinafter also referred to as a nucleic acid of the present invention).
(1) A nucleic acid consisting of the nucleotide sequence of any one of SEQ ID NOs:1 to 1336.
(2) A nucleic acid consisting of a nucleotide sequence having an identity of 90% or more to the nucleotide sequence of any one of SEQ ID NOs:1 to 1336.
(3) A nucleic acid that hybridizes under stringent conditions with a strand complementary to a nucleic acid consisting of the nucleotide sequence of any one of SEQ ID NOs:1 to 1336.
(4) A nucleic acid comprising the nucleic acid described in any one of (1) to (3).
(5) A nucleic acid consisting of the nucleotide sequence of any one of SEQ ID NOs:1337 to 2851.
(6) A nucleic acid consisting of a nucleotide sequence having an identity of 80% or more to the nucleotide sequence of any one of SEQ ID NOs:1337 to 2851.
(7) A nucleic acid that hybridizes under stringent conditions with a strand complementary to a nucleic acid consisting of the nucleotide sequence of any one of SEQ ID NOs:1337 to 2851.
(8) A nucleic acid consisting of a nucleotide sequence complementary to the nucleic acid described in any one of (1) to (7).
(9) A double-stranded nucleic acid consisting of the nucleic acid described in any one of (1) to (7) and a nucleic acid consisting of a nucleotide sequence complementary to the nucleotide sequence of the nucleic acid.

In the present invention, a micro-RNA refers to an RNA that is a cell-derived single-stranded RNA, that has a sequence wherein the surrounding genome sequence, including the sequence, is capable of forming a hairpin structure, and that is capable of being cleaved out from either one chain of the hairpin. The length of the micro-RNA is preferably 15 to 28 nucleotides, more preferably 16 to 28 nucleotides, still more preferably 16 to 26 nucleotides, and particularly preferably 16 to 24 nucleotides. A micro-RNA complementarily binds to an mRNA being a target thereof, to degrade the mRNA or to suppress the translation of the mRNA, and to make post-transcriptional control of gene expression. As a micro-RNA of the present invention, a nucleic acid consisting of the nucleotide sequence of any one of SEQ ID NOs:1 to 1336, and a nucleic acid consisting of a nucleotide sequence having an identity of 90% or more, preferably 95% or more, to the nucleotide sequence of any one of SEQ ID NOs:1 to 1336 can be mentioned. A nucleic acid that hybridizes under stringent conditions with a strand complementary to a nucleic acid consisting of the nucleotide sequence of any one of SEQ ID NOs:1 to 1336 can also be mentioned. As another micro-RNA of the present invention, a nucleic acid comprising these nucleic acids can be mentioned.

In the present invention, a micro-RNA precursor is a nucleic acid, including a micro-RNA, that is about 50 to about 200 nucleotides, preferably about 70 to about 100 nucleotides, long, and that is capable of forming a hairpin structure. A micro-RNA is produced from a micro-RNA precursor via processing by a protein called Dicer. As micro-RNA precursors of the present invention, a nucleic acid consisting of the nucleotide sequence of any one of SEQ ID NOs:1337 to 2851, a nucleic acid consisting of a nucleotide sequence having an identity of 80% or more, preferably 90% or more, and still more preferably 95% or more, to the nucleotide sequence of any one of SEQ ID NOs:1337 to 2851, and a nucleic acid that hybridizes under stringent conditions with a strand complementary to a nucleic acid consisting of the nucleotide sequence of any one of SEQ ID NOs:1337 to 2851 can be mentioned. Because a micro-RNA precursor comprises a sequence of a micro-RNA, and it exhibits a function as a precursor if the micro-RNA is produced from the micro-RNA precursor via processing, any nucleic acid having a homology of 80% or more to the nucleotide sequence of any one of SEQ ID NOs:1337 to 2851 is thought to exhibit a function as a micro-RNA precursor.

Shown in Tables 1-1 to 1-40 are relationships between the nucleotide sequences of SEQ ID NOs:1 to 1336, specifically mentioned as micro-RNAs, and the nucleotide sequences of micro-RNA precursors mentioned as precursors thereof.

**[Table 1-1]**

| nucleic acid name | micro-RNA | micro-RNA precursor |
|---|---|---|
| KHK_miR_1001 | SEQ ID NO:1 | SEQ ID NO:1337 |
| KHK_miR_1002 | SEQ ID NO:2 | SEQ ID NO:1338 |
| KHK_miR_1003 | SEQ ID NO:3 | SEQ ID NO:1339 |
| KHK-miR_1004 | SEQ ID NO:4 | SEQ ID NO:1340 |
| KHK_miR_1005 | SEQ ID NO:5 | SEQ ID NO:1341 |
| KHK_miR_1006 | SEQ ID NO:6 | SEQ ID NO:1342 |
| KHK_miR_1007 | SEQ ID NO:7 | SEQ ID NO:1343 |
| | | SEQ ID NO:1344 |
| | | SEQ ID NO:1345 |
| | | SEQ ID NO:1346 |
| | | SEQ ID NO:1347 |
| | | SEQ ID NO:1348 |
| | | SEQ ID NO:1349 |
| | | SEQ ID NO:1350 |
| | | SEQ ID NO:1351 |
| KHK_miR_1008 | SEQ ID NO:8 | SEQ ID NO:1352 |
| KHK_miR_1009 | SEQ ID NO:9 | SEQ ID NO:1353 |
| | | SEQ ID NO:1354 |
| KHK_miR_1010 | SEO ID NO:10 | SEQ ID NO:1355 |
| KHK_miR_1011 | SEQ ID NO:11 | SEQ ID NO:1356 |
| | | SEQ ID NO:1357 |
| | | SEQ ID NO:1358 |
| | | SEQ ID NO:1359 |
| | | SEQ ID NO:1360 |
| KHK_miR_1012 | SEQ ID NO:12 | SEQ ID NO:1361 |
| KHK_miR_1013 | SEQ ID NO:13 | SEQ ID NO:2.3 62 |
| KHK_miR_1014 | SEQ ID NO:14 | SEQ ID NO:1363 |
| KHK_miR_1015 | SEO ID NO:15 | SEQ ID NO:1364 |
| | | SEQ ID NO:1365 |
| KHK_miR_1016 | SEQ ID NO:16 | SEQ ID NO:1366 |
| KHK_miR_1017 | SEQ ID NO:17 | SEQ ID NO:1367 |
| KHK_miR_1018 | SEQ ID NO:18 | SEQ ID NO:1369 |
| KHK_miR_1019 | SEQ ID NO:19 | SEQ ID NO:1369 |
| | | SEQ ID NO:1370 |
| KHK_miR_1020 | SEQ ID NO:20 | SEQ ID NO:1371 |
| KHK_miR_1021 | SEQ ID NO:21 | SEQ ID NO:1372 |
| KHK_miR_1022 | SEQ ID NO:22 | SEO ID NO:1373 |
| KHK_miR_1023 | SEQ ID NO:23 | SEQ ID NO:1374 |
| KHK_miR_1024 | SEQ ID NO:24 | SEQ ID NO:1375 |

**[Table 1-2]**

| nucleic acid name | micro-RNA | micro-RNA precursor |
|---|---|---|
| | | SEQ ID NO:1376 |
| KHK_miR_1025 | SEQ ID NO:25 | SEQ ID NO:1377 |
| KHK_miR_1026 | SEQ ID NO:26 | SEQ ID NO:1378 |
| KHK_miR_1027 | SEQ ID NO:27 | SEQ ID NO:1379 |
| | | SEQ ID NO:1380 |
| | | SEQ ID NO:3381 |
| KHK_miR_1028 | SEQ ID NO:28 | SEQ ID NO:1382 |
| KHK_miR_1029 | SEQ ID NO:29 | SEQ ID NO:1383 |
| KHK_miR_1030 | SEQ ID NO:30 | SEQ ID NO:1384 |
| KHK_miR_1031 | SEQ ID NO:31 | SEQ ID NO:1385 |
| KHK_miR_1032 | SEQ ID NO:32 | SEQ ID NO:1386 |
| KHK_miR_1033 | SEQ ID NO:33 | SEQ ID NO:1387 |
| KHK_miR_1034 | SEQ ID NO:34 | SEQ ID NO:1388 |
| KHK_miR_1035 | SEQ ID NO:35 | SEQ ID NO:1389 |
| KHK_miR_1036 | SEQ ID NO:36 | SEQ ID NO:1390 |
| KHK_miR_1037 | SEQ ID NO:37 | SEQ ID NO:1391 |
| KHK_miR_1038 | SEQ ID NO:38 | SEQ ID NO:1392 |
| KHK_miR_1039 | SEQ ID NO:39 | SEQ ID NO:1393 |
| KHK_miR_1040 | SEQ ID NO:40 | SEQ ID NO:1394 |
| KHK_miR_1041 | SEQ ID NO:41 | SEQ ID NO:1395 |
| KHK_miR_1042 | SEQ ID NO:42 | SEQ ID NO:1396 |
| KHK_miR_1043 | SEQ ID NO:43 | SEQ ID NO:1397 |
| KHK_miR_1044 | SEQ ID NO:44 | SEQ ID NO:1398 |
| KHK_miR_1045 | SEQ ID NO:45 | SEQ ID NO:1399 |
| KHK_miR_1046 | SEQ ID NO:46 | SEQ ID NO:1400 |
| KHK_miR_1047 | SEQ ID NO:47 | SEQ ID NO:1401 |
| KHK_miR_1048 | SEQ ID NO:48 | SEQ ID NO:1402 |
| | | SEQ ID NO:1403 |
| KHK_miR_1049 | SEQ ID NO:49 | SEQ ID NO:1404 |
| | | SEQ ID NO:1405 |
| KHK_miR_1050 | SEQ ID NO:50 | SEQ ID NO:1406 |
| KHK_miR_1051 | SEQ ID NO:51 | SEQ ID NO:1407 |
| KHK_miR_1052 | SEQ ID NO:52 | SEQ ID NO:1408 |
| KHK_miR_1053 | SEQ ID NO:53 | SEQ ID NO:1409 |
| KHK_miR_1054 | SEQ ID NO:54 | SEQ ID NO:1410 |
| KHK_miR_1055 | SEQ ID NO:55 | SEQ ID NO:1411 |
| KHK_miR_1056 | SEQ ID NO:56 | SEQ ID NO:1412 |
| KHK_miR_1057 | SEQ ID NO:57 | SEQ ID NO:1413 |
| KHK_miR_1058 | SEQ ID NO:58 | SEQ ID NO:1414 |

**[Table 1-3]**

| nucleic acid name | micro-RNA | micro-RNA precursor |
|---|---|---|
| | | SEQ ID NO:1415 |
| | | SEQ ID NO:1416 |
| | | SEQ ID NO:1417 |
| | | SEQ ID NO:1418 |
| KHK_miR_1059 | SEQ ID NO:59 | SEQ ID NO:1419 |
| KHK_miR_1060 | SEQ ID NO:60 | SEQ ID NO:1420 |
| KHK_miR_1061 | SEQ ID NO:61 | SEQ ID NO:1421 |
| KHK_miR_1062 | SEQ ID NO:62 | SEQ ID NO:1422 |
| KHK_miR_1063 | SEQ ID NO:63 | SEQ ID NO:1423 |
| KHK_miR_1064 | SEQ ID NO:64 | SEQ ID NO:1424 |
| KHK_miR_1065 | SEQ ID NO:65 | SEQ ID NO:1425 |
| KHK_miR_1066 | SEQ ID NO:66 | SEQ ID NO:1426 |
| KHK_miR_1067 | SEQ ID NO:67 | SEQ ID NO:1427 |
| KHK_miR_1068 | SEQ ID NO:68 | SEQ ID NO:1428 |
| KHK_miR_1069 | SEQ ID NO:69 | SEQ ID NO:1429 |
| KHK_miR_1070 | SEQ ID NO:70 | SEQ ID NO:1430 |
| KHK_miR_1071 | SEQ ID NO:71 | SEQ ID NO:1431 |
| KHK_miR_1072 | SEQ ID NO:72 | SEQ ID NO:1432 |
| KHK_miR_1073 | SEQ ID NO:73 | SEQ ID NO:1433 |
| KHK_miR_1074 | SEQ ID NO:74 | SEQ ID NO:1434 |
| KHK_miR_1075 | SEQ ID NO:75 | SEQ ID NO:1435 |
| | | SEQ ID NO:1436 |
| KHK_miR_1076 | SEQ ID NO:76 | SEQ ID NO:1437 |
| KHK_miR_1077 | SEQ ID NO:77 | SEQ ID NO:1438 |
| KHK_miR_1078 | SEQ ID NO:78 | SEQ ID NO:1439 |
| KHK_miR_1079 | SEQ ID NO:79 | SEQ ID NO:1440 |
| | | SEQ ID NO:1441 |
| | | SEQ ID NO:1442 |
| KHK_miR_1080 | SEQ ID NO:80 | SEQ ID NO:1443 |
| KHK_miR_1081 | SEQ ID NO:81 | SEQ ID NO:1444 |
| KHK_miR_1082 | SEQ ID NO:82 | SEQ ID NO:1445 |
| KHK_miR_1083 | SEQ ID NO:83 | SEQ ID NO:1446 |
| KHK_miR_1084 | SEQ ID NO:84 | SEQ ID NO:1447 |
| KHK_miR_1095 | SEQ ID NO:85 | SEQ ID NO:1448 |
| KHK_miR_1086 | SEQ ID NO:86 | SEQ ID NO:1449 |
| KHK_miR_1087 | SEQ ID NO:87 | SEQ ID NO:1450 |
| KHK_miR_1088 | SEQ ID NO:88 | SEQ ID NO:1451 |
| KHK_miR_1089 | SEQ ID NO:89 | SEQ ID NO:1452 |
| KHK_miR_1090 | SEQ ID NO:90 | SEQ ID NO:1453 |

**[Table 1-4]**

| nucleic acid name | micro-RNA | micro-RNA precursor |
|---|---|---|
| KHK_miR_1091 | SEQ ID NO:91 | SEQ ID NO:1454 |
| KHK_miR_1092 | SEQ ID NO:92 | SEQ ID NO:1455 |
| KHK_miR_1093 | SEQ ID NO:93 | SEQ ID NO:1456 |
| | | SEQ ID NO:1457 |
| | | SEQ ID NO:1458 |
| | | SEQ ID NO:1459 |
| KHK_miR_1094 | SEQ ID NO:94 | SEQ ID NO:1460 |
| KHK_miR_1095 | SEQ ID NO:95 | SEQ ID NO:1461 |
| KHK_miR_1096 | SEQ ID NO:96 | SEQ ID NO:1462 |
| KHK_miR_1097 | SEQ ID NO:97 | SEQ ID NO:1463 |
| KHK_miR_1098 | SEQ ID NO:98 | SEQ ID NO:1464 |
| KHK_miR_1099 | SEQ ID NO:99 | SEQ ID NO:1465 |
| KHK_miR_1100 | SEQ ID NO:100 | SEQ ID NO:1466 |
| KHK_miR_1101 | SEQ ID NO:101 | SEQ ID NO:1467 |
| KHK_miR_1102 | SEQ ID NO:102 | SEQ ID NO:1468 |
| KHK_miR_1103 | SEQ ID NO:103 | SEQ ID NO:1469 |
| KHK_miR_1104 | SEQ ID NO:104 | SEQ ID NO:1470 |
| | | SEQ ID NO:1471 |
| KHK_miR_1105 | SEQ ID NO:105 | SEQ ID NO:1472 |
| KHK_miR_1106 | SEQ ID NO:106 | SEQ ID NO:1473 |
| KHK_miR_1107 | SEQ ID NO:107 | SEQ ID NO:1474 |
| KHK_miR_1108 | SEQ ID NO:108 | SEQ ID NO:1475 |
| KHK_miR_1109 | SEQ ID NO:109 | SEQ ID NO:1476 |
| KHK_miR_1110 | SEQ ID NO:110 | SEQ ID NO:1477 |
| KHK_miR_1111 | SEQ ID NO:111 | SEQ ID NO:1478 |
| KHK_miR_1112 | SEQ ID NO:112 | SEQ ID NO:1479 |
| | | SEQ ID NO:1480 |
| KHK_miR_1113 | SEQ ID NO:113 | SEQ ID NO:1481 |
| KHK_miR_1114 | SEQ ID NO:114 | SEQ ID NO:1482 |
| KHK_miR_1115 | SEQ ID NO:115 | SEQ ID NO:1483 |
| KHK_miR_1116 | SEQ ID NO:116 | SEQ ID NO:1484 |
| KHK_miR_1117 | SEQ ID NO:117 | SEQ ID NO:1485 |
| KHK_miR_1118 | SEQ ID NO:118 | SEQ ID NO:1486 |
| KHK_miR_1119 | SEQ ID NO:119 | SEQ ID NO:1487 |
| KHK_miR_1120 | SEQ ID NO:120 | SEQ ID NO:1488 |
| KHK_miR_1121 | SEQ ID NO:121 | SEQ ID NO:1489 |
| KHK_miR_1122 | SEQ ID NO:122 | SEQ ID NO:1490 |
| KHK_miR_1123 | SEQ ID NO:123 | SEQ ID NO:1491 |
| KHK_miR_1124 | SEQ ID NO:124 | SEQ ID NO:1492 |

**[Table 1-5]**

| nucleic acid name | micro-RNA | micro-RNA precursor |
|---|---|---|
| KHK_miR_1125 | SEQ ID NO:125 | SEQ ID NO:1493 |
| KHK_miR_1126 | SEQ ID NO:126 | SEQ ID NO:1494 |
| | | SEQ ID NO:1495 |
| | | SEQ ID NO:1496 |
| KHK_miR_1127 | SEQ ID NO:127 | SEQ ID NO:1497 |
| KHK_miR_1128 | SEQ ID NO:128 | SEQ ID NO:1498 |
| KHK_miR_1129 | SEQ ID NO:129 | SEQ ID NO:1499 |
| KHK_miR_1130 | SEQ ID NO:130 | SEQ ID NO:1500 |
| KHK_miR_1131 | SEQ ID NO:131 | SEQ ID NO:1501 |
| KHK_miR_1132 | SEQ ID NO:132 | SEQ ID NO:1502 |
| KHK_miR_1133 | SEQ ID NO:133 | SEQ ID NO:1503 |
| KHK_miR_1134 | SEQ ID NO:134 | SEQ ID NO:1504 |
| KHK_miR_1135 | SEQ ID NO:135 | SEQ ID NO:1505 |
| KHK_miR_1136 | SEQ ID NO:136 | SEQ ID NO:1506 |
| KHK_miR-1137 | SEQ ID NO:137 | SEQ ID NO:1507 |
| KHK_miR_1138 | SEQ ID NO:138 | SEQ ID NO:1508 |
| KHK_miR_1139 | SEQ ID NO:139 | SEQ ID NO:1509 |
| KHK_miR_1140 | SEQ m NO:140 | SEQ ID NO:1510 |
| KHK_miR_1141 | SEQ ID NO:141 | SEQ ID NO:1511 |
| KHK_miR_1142 | SEQ ID NO:142 | SEQ ID NO:1512 |
| KHK_miR_1143 | SEQ ID NO:143 | SEQ ID NO:1513 |
| KHK_miR_1144 | SEQ ID NO:144 | SEQ ID NO:1514 |
| KHK_miR_1145 | SEQ ID NO:145 | SEQ ID NO:1515 |
| KHK_miR_1146 | SEQ ID NO:146 | SEQ ID NO:1516 |
| KHK_miR_1147 | SEQ ID NO:147 | SEQ ID NO:1517 |
| KHK_miR_1148 | SEQ ID NO:148 | SEQ ID NO:1518 |
| KHK_miR_1149 | SEQ ID NO:149 | SEQ ID NO:1519 |
| KHK_miR_1150 | SEQ ID NO:150 | SEQ ID NO:1520 |
| KHK_miR_1151 | SEQ ID NO:151 | SEQ ID NO:1521 |
| KHK_miR_1152 | SEQ ID NO:152 | SEQ ID NO:1522 |
| | | SEQ ID NO:1523 |
| | | SEQ ID NO:1524 |
| KHK_miR_1153 | SEQ ID NO:153 | SEQ ID NO:1525 |
| KHK_miR_1154 | SEQ ID NO:154 | SEQ ID NO:1526 |
| KHK_miR_1155 | SEQ ID NO:155 | SEQ ID NO:1527 |
| KHK_miR_1156 | SEQ ID NO:156 | SEQ ID NO:1528 |
| KHK_miR_1157 | SEQ ID NO:157 | SEQ ID NO:1529 |
| KHK_miR_1158 | SEQ ID NO:158 | SEQ ID NO:1530 |
| KHK_miR_1159 | SEQ ID NO:159 | SEQ ID NO:1531 |

**[Table 1-6]**

| nucleic acid name | micro-RNA | micro-RNA precursor |
|---|---|---|
| KHK_miR_1160 | SEQ ID NO:160 | SEQ ID NO:1532 |
| KHK_miR_1161 | SEQ ID NO:161 | SEQ ID NO:1533 |
| KHK_miR_1162 | SEQ ID NO:162 | SEQ ID NO:1534 |
| KHK_miR_1163 | SEQ ID NO:163 | SEQ ID NO:1535 |
| KHK_miR_1164 | SEQ ID NO:164 | SEQ ID NO:1536 |
| KHK_miR_1165 | SEQ ID NO:165 | SEQ ID NO:1537 |
| KHK_miR_1166 | SEQ ID NO:166 | SEQ ID NO:1538 |
| KHK_miR_1167 | SEQ ID NO:167 | SEQ ID NO:1539 |
| KHK_miR_1168 | SEQ ID NO:168 | SEQ ID NO:1540 |
| KHK_miR_1169 | SEQ ID NO:169 | SEQ ID NO:1541 |
| KHK_miR_1170 | SEQ ID NO:170 | SEQ ID NO:1542 |
| KHK_miR_1171 | SEQ ID NO:171 | SEQ ID NO:1543 |
| KHK_miR_1172 | SEQ ID NO:172 | SEQ ID NO:1544 |
| KHK_miR_1173 | SEQ ID NO:173 | SEQ ID NO:1545 |
| KHK_miR_1174 | SEQ ID NO:174 | SEQ ID NO:1546 |
| KHK_miR_1175 | SEQ ID NO:175 | SEQ ID NO:1547 |
| KHK_miR_1176 | SEQ ID NO:176 | SEQ ID NO:1548 |
| KHK_miR_1177 | SEQ ID NO:177 | SEQ ID NO:1549 |
| KHK_miR_1178 | SEQ ID NO:178 | SEQ ID NO:1550 |
| | | SEQ ID NO:1551 |
| | | SEQ ID NO:1552 |
| | | SEQ ID NO:1553 |
| | | SEQ ID NO:1554 |
| | | SEQ ID NO:1555 |
| KHK_miR_1179 | SEQ ID NO:179 | SEQ ID NO:1556 |
| KHK_miR_1180 | SEQ ID NO:180 | SEQ ID NO:1557 |
| KHK_miR_1181 | SEQ ID NO:181 | SEQ ID NO:1558 |
| | | SEQ ID NO:1559 |
| KHK_miR_1182 | SEQ ID NO:182 | SEQ ID NO:1560 |
| KHK_miR_1183 | SEQ ID NO:183 | SEQ ID NO:1561 |
| KHK_miR_1184 | SEQ ID NO:184 | SEQ ID NO:1562 |
| KHK_miR_1185 | SEQ ID NO:185 | SEQ ID NO:1563 |
| KHK_miR_1186 | SEQ ID NO:186 | SEQ ID NO:1564 |
| KHK_miR_1187 | SEQ ID NO:187 | SEQ ID NO:1565 |
| KHK_miR_1188 | SEQ ID NO:188 | SEQ ID NO:1566 |
| KHK_miR_1189 | SEQ ID NO:189 | SEQ ID NO:1567 |
| KHK_miR_1190 | SEQ ID NO:190 | SEQ ID NO:1568 |
| KHK_miR_1191 | SEQ ID NO:191 | SEQ ID NO:1569 |
| KHK_miR_1192 | SEQ ID NO:192 | SEQ ID NO:1570 |

**[Table 1-7]**

| nucleic acid name | micro-RNA | micro-RNA precursor |
|---|---|---|
| KHK_miR_1193 | SEQ ID NO:193 | SEQ ID NO:1571 |
| KHK_miR_1194 | SEQ ID NO:194 | SEQ ID NO:1572 |
| KHK_miR_1195 | SEQ ID NO:195 | SEQ ID NO:1573 |
| KHK_miR_1196 | SEQ ID NO:196 | SEQ ID NO:1574 |
| KHK_miR_1197 | SEQ ID NO:197 | SEQ ID NO:1575 |
| KHK_miR_1198 | SEQ ID NO:198 | SEQ ID NO:1576 |
| KHK_miR_1199 | SEQ ID NO:199 | SEQ ID NO:1577 |
| KHK_miR_1200 | SEQ ID NO:200 | SEQ ID NO:1578 |
| KHK_miR_1201 | SEQ ID NO:201 | SEQ ID NO:1579 |
| KHK_miR_1202 | SEQ ID NO:202 | SEQ ID NO:1580 |
| KHK_miR_1203 | SEQ ID NO:203 | SEQ ID NO:1581 |
| KHK_miR_1204 | SEQ ID NO:204 | SEQ ID NO:1582 |
| KHK_miR_1205 | SEQ ID NO:205 | SEQ ID NO:1583 |
| KHK_miR_1206 | SEQ ID NO:206 | SEQ ID NO: 1584 |
| KHK_miR_1207 | SEQ ID NO:207 | SEQ ID NO:1585 |
| KHK_miR_1208 | SEQ ID NO:208 | SEQ ID NO:1586 |
| | | SEQ ID NO:1587 |
| KHK_miR_1209 | SEQ ID NO:209 | SEQ ID NO:1588 |
| KHK_miR_1210 | SEQ ID NO:210 | SEQ ID NO:1589 |
| KHK_miR_1211 | SEQ ID NO:211 | SEQ ID NO:1590 |
| KHK_miR_1212 | SEQ ID NO:212 | SEQ ID NO:1591 |
| KHK_miR_1213 | SEQ ID NO:213 | SEQ ID NO:1592 |
| | | SEQ ID NO:1593 |
| | | SEQ ID NO:1594 |
| KHK_miR_1214 | SEQ ID NO:214 | SEQ ID NO:1595 |
| | | SEQ ID NO:1596 |
| KHK_miR_1215 | SEQ ID NO:215 | SEQ ID NO:1597 |
| KHK_miR_1216 | SEQ ID NO:216 | SEQ ID NO:1598 |
| KHK_nMR_1217 | SEQ ID NO:217 | SEQ ID NO:1599 |
| KHK_miR_1218 | SEQ ID NO:218 | SEQ ID NO:1600 |
| KHK_miR_1219 | SEQ ID NO:219 | SEQ ID NO:1601 |
| KHK_miR_1220 | SEQ ID NO:220 | SEQ ID NO:1602 |
| KHK_miR_1221 | SEQ ID NO:221 | SEQ ID NO:1603 |
| KHK_miR_1222 | SEQ ID NO:222 | SEQ ID NO:1604 |
| KHK_miR_1223 | SEQ ID NO:223 | SEQ ID NO:1605 |
| KHK_miR_1224 | SEQ ID NO:224 | SEQ ID NO:1606 |
| KHK_miR_1225 | SEQ ID NO:225 | SEQ ID NO:1607 |
| KHK_miR-1226 | SEQ ID NO:226 | SEQ ID NO:1608 |
| KHK_miR-1227 | SEQ ID NO:227 | SEQ ID NO:1609 |

**[Table 1-8]**

| nucleic acid name | micro-RNA | micro-RNA precursor |
|---|---|---|
| KHK_miR_1228 | SEQ ID NO:228 | SEQ ID NO:1610 |
| KHK_miR_1229 | SEQ ID NO:229 | SEQ ID NO:1611 |
| KHK_miR_1230 | SEQ ID NO:230 | SEQ ID NO:1612 |
| KHK_miR_1231 | SEQ ID NO:231 | SEQ ID NO:1613 |
| KHK_miR_1232 | SEQ ID NO:232 | SEQ ID NO:1614 |
| KHK_miR_1233 | SEQ ID NO:233 | SEQ ID NO:1615 |
| | | SEQ ID NO:1616 |
| | | SEQ ID NO:1617 |
| | | SEQ ID NO:1618 |
| KHK_miR_1234 | SEQ ID NO:234 | SEQ ID NO:1619 |
| KHK_miR_1235 | SEQ ID NO:235 | SEQ ID NO:1620 |
| KHK_miR_1236 | SEQ ID NO:236 | SEQ ID NO:1621 |
| KHK_miR_1237 | SEQ ID NO:237 | SEQ ID NO:1622 |
| KHK_miR_1238 | SEQ ID NO:238 | SEQ ID NO:1623 |
| KHK_miR_1239 | SEQ ID NO:239 | SEQ ID NO:1624 |
| KHK_miR_1240 | SEQ ID NO:240 | SEQ ID NO:1625 |
| KHK_miR_1241 | SEQ ID NO:241 | SEQ ID NO:1626 |
| | | SEQ ID NO:1627 |
| KHK_miR_1242 | SEQ ID NO:242 | SEQ ID NO:1628 |
| KHK_miR_1243 | SEQ ID NO:243 | SEQ ID NO:1629 |
| KHK_miR_1244 | SEQ ID NO:244 | SEQ ID NO:1630 |
| KHK_miR_1245 | SEQ ID NO:245 | SEQ ID NO:1631 |
| KHK_miR_1246 | SEQ ID NO:246 | SEQ ID NO:1632 |
| KHK_miR_1247 | SEQ ID NO:247 | SEQ ID NO:1633 |
| KHK_miR_1248 | SEQ ID NO:248 | SEQ ID NO:1634 |
| KHK_miR_1249 | SEQ ID NO:249 | SEQ ID NO:1635 |
| KHK_miR_1250 | SEQ ID NO:250 | SEQ ID NO:1636 |
| KHK_miR_1251 | SEQ ID NO:251 | SEQ ID NO:1637 |
| KHK_miR_1252 | SEQ ID NO:252 | SEQ ID NO:1638 |
| | | SEQ ID NO:1639 |
| KHK_miR_1253 | SEQ ID NO:253 | SEQ ID NO:1640 |
| KHK_miR_1254 | SEQ ID NO:254 | SEQ ID NO:1641 |
| KHK_miR_1255 | SEQ ID NO:255 | SEQ ID NO:1642 |
| | | SEQ ID NO:1643 |
| | | SEQ ID NO:1644 |
| KHK_miR_1256 | SEQ ID NO:256 | SEQ ID NO:1645 |
| KHK_miR_1257 | SEQ ID NO:257 | SEQ ID NO:1646 |
| KHK_miR_1258 | SEQ ID NO:258 | SEQ ID NO:1647 |
| | | SEQ ID NO:1648 |

**[Table 1-9]**

| nucleic acid name | micro-RNA | micro-RNA precursor |
|---|---|---|
| KHK_miR_1259 | SEQ ID NO:259 | SEQ ID NO:1649 |
| KHK_miR_1260 | SEQ ID NO:260 | SEQ ID NO:1650 |
| KHK_miR_1261 | SEQ ID NO:261 | SEQ ID NO:1651 |
| | | SEQ ID NO:1652 |
| KHK_miR_1262 | SEQ ID NO:262 | SEQ ID NO:1653 |
| KHK_miR_1263 | SEQ ID NO:263 | SEQ ID NO:1654 |
| KHK_miR_1264 | SEQ ID NO:264 | SEQ ID NO:1655 |
| KHK_miR_1265 | SEQ ID NO:265 | SEQ ID NO:1656 |
| KHK_miR_1266 | SEQ ID NO:266 | SEQ ID NO:1657 |
| KHK_miR_1267 | SEQ ID NO:267 | SEQ ID NO:1658 |
| KHK_miR_1268 | SEQ ID NO:268 | SEQ ID NO:1659 |
| KHK_miR_1269 | SEQ ID NO:269 | SEQ ID NO:1660 |
| KHK_miR_1270 | SEQ ID NO:270 | SEQ ID NO:1661 |
| KHK_miR_1271 | SEQ ID NO:271 | SEQ ID NO:1662 |
| KHK_miR_1272 | SEQ ID NO:272 | SEQ ID NO:1663 |
| KHK_miR_1273 | SEQ ID NO:273 | SEQ ID NO:1664 |
| KHK_miR_1274 | SEQ ID NO:274 | SEQ ID NO:1665 |
| KHK_miR_1275 | SEQ ID NO:275 | SEQ ID NO:1666 |
| KHK_miR_1276 | SEQ ID NO:276 | SEQ ID NO:1667 |
| KHK_miR_1277 | SEQ ID NO:277 | SEQ ID NO:1668 |
| KHK_miR_1278 | SEQ ID NO:278 | SEQ ID NO:1669 |
| KNK_miR_1279 | SEQ ID NO:279 | SEQ ID NO:1670 |
| KHK_miR_1280 | SEQ ID NO:280 | SEQ ID NO:1671 |
| KHK_miR_1281 | SEQ ID NO:281 | SEQ ID NO:1672 |
| | | SEQ ID NO:1673 |
| | | SEQ ID NO:1674 |
| | | SEQ ID NO:1675 |
| KHK_miR_1282 | SEQ ID NO:282 | SEQ ID NO:1676 |
| KHK_miR_1283 | SEQ ID MO:283 | SEQ ID NO:1677 |
| KHK_miR_1284 | SEQ ID NO:284 | SEQ ID No:1678 |
| KHK_miR_1285 | SEQ ID NO:285 | SEQ ID NO:1679 |
| KHK_miR_1286 | SEQ ID NO:286 | SEQ ID NO:1660 |
| KHK_miR_1287 | SEQ ID NO:287 | SEQ ID NO:1681 |
| KHK_miR_1288 | SEQ ID NO:228 | SEQ ID NO:1682 |
| KHK_miR_1289 | SEQ ID NO:289 | SEQ ID NO:1683 |
| KHK_miR_1290 | SEQ ID NO:290 | SEQ ID NO:1684 |
| KHK_miR_1291 | SEQ ID NO:291 | SEQ ID NO:1685 |
| KHK_miR_1292 | SEQ ID NO:292 | SEQ ID NO:1686 |
| | | SEQ ID NO:1687 |

**[Table 1-10]**

| nucleic acid name | micro-RNA | micro-RNA precursor |
|---|---|---|
| KHK_miR_1293 | SEQ ID NO:293 | SEQ ID NO:1688 |
| KHK_miR_1294 | SEQ ID NO:294 | SEQ ID NO:1689 |
| KHK_miR_1295 | SEQ ID NO:295 | SEQ ID NO:1690 |
| KHK_miR_1296 | SEQ ID NO:296 | SEQ ID NO:1691 |
| KHK_miR_1297 | SEQ ID NO:297 | SEQ ID NO:1692 |
| KHK_miR_1298 | SEQ ID NO:298 | SEQ ID NO:1693 |
| KHK_miR_1299 | SEQ ID NO:299 | SEQ ID NO:1694 |
| KHK_miR_1300 | SEQ ID NO:300 | SEQ ID NO:1695 |
| KHK_miR_1301 | SEQ ID NO:301 | SEQ ID NO:1696 |
| KHK_miR_1302 | SEQ ID NO:302 | SEQ ID NO:1697 |
| KHK_miR_1303 | SEQ ID NO:303 | SEQ ID NO:1698 |
| KHK_miR_1304 | SEQ ID NO:304 | SEQ ID NO:1699 |
| KHK_miR_1305 | SEQ ID NO:305 | SEQ ID NO:1700 |
| KHK_miR_1306 | SEQ ID NO:306 | SEQ ID NO:1701 |
| KHK_miR_1307 | SEQ ID NO:307 | SEQ ID NO:1702 |
| KHK_miR_1308 | SEQ ID NO:308 | SEQ ID NO:1703 |
| | | SEQ ID NO:1704 |
| | | SEQ ID NO:1705 |
| KHK_miR_1309 | SEQ ID NO:309 | SEQ ID NO:1706 |
| | | SEQ ID NO:1707 |
| KHK_miR_1310 | SEQ ID NO:310 | SEQ ID NO:1708 |
| KHK_miR_1311 | SEQ ID NO:311 | SEQ ID NO:1709 |
| KHK_miR_1312 | SEQ ID NO:312 | SEQ ID NO:1710 |
| KHK_miR_1313 | SEQ ID NO:313 | SEQ ID NO:1711 |
| KHK_miR_1314 | SEQ ID NO:314 | SEQ ID NO:1712 |
| KHK_miR_1315 | SEQ ID NO:315 | SEQ ID NO:1713 |
| | | SEQ ID NO:1714 |
| KHK_miR_1316 | SEQ ID NO:316 | SEQ ID NO:1715 |
| KHK_miR_1317 | SEQ ID NO:317 | SEQ ID NO:1716 |
| KHK_miR-1318 | SEQ ID NO:318 | SEQ ID NO:1717 |
| KHK_miR_1319 | SEQ ID NO:319 | SEQ ID NO:1718 |
| KHK_miR_1320 | SEQ ID NO:320 | SEQ ID NO:1719 |
| KHK_miR_1321 | SEQ ID NO:321 | SEQ ID NO:1720 |
| KHK_miR_1322 | SEQ ID NO:322 | SEQ ID NO:1721 |
| KHK_miR_1323 | SEQ ID NO:323 | SEQ ID NO:1722 |
| KHK_miR_1324 | SEQ ID NO:324 | SEQ ID NO:1723 |
| KHK_miR_1325 | SEQ ID NO:325 | SEQ ID NO:1724 |
| KHK_miR_1326 | SEQ ID NO:326 | SEQ ID NO:1725 |
| | | SEQ ID NO:1726 |

**[Table 1-11]**

| nucleic acid name | micro-RNA | micro-RNA precursor |
|---|---|---|
| | | SEQ ID NO:1727 |
| KHK_miR_1327 | SEQ ID NO:327 | SEQ ID NO:1728 |
| KHK_miR_1328 | SEQ ID NO:328 | SEQ ID NO:1729 |
| | | SEQ ID NO:1730 |
| | | SEQ ID NO:1731 |
| KHK_miR_1329 | SEQ ID NO:329 | SEQ ID NO:1732 |
| KHK_miR_1330 | SEQ ID NO:330 | SEQ ID NO:1733 |
| KHK_miR_1331 | SEQ ID NO:331 | SEQ ID NO:1734 |
| | | SEQ ID NO:1735 |
| KHK_miR_1332 | SEQ ID NO:332 | SEQ ID NO:1736 |
| | | SEQ ID NO:1737 |
| KHK_miR_1333 | SEQ ID NO:333 | SEQ ID NO:1738 |
| KHK_miR_1334 | SEQ ID NO:334 | SEQ ID NO:1739 |
| KHK_miR_1335 | SEQ ID NO:335 | SEQ ID NO:1740 |
| KHK_miR_1336 | SEQ ID NO:336 | SEQ ID NO:1741 |
| KHK_miR_1337 | SEQ ID NO:337 | SEQ ID NO:1742 |
| KHK_miR_1338 | SEQ ID NO:338 | SEQ ID NO:1743 |
| KHK_miR_1339 | SEQ ID NO:339 | SEQ ID NO:1744 |
| KHK_miR_1340 | SEQ ID NO:340 | SEQ ID NO:1745 |
| KHK_miR_1341 | SEQ ID NO:341 | SEQ ID NO:1746 |
| KHK_miR_1342 | SEQ ID NO:342 | SEQ ID NO:1747 |
| KHK_miR_1343 | SEQ ID NO:343 | SEQ ID NO:1748 |
| KHK_miR_1344 | SEQ ID NO:344 | SEQ ID NO:1749 |
| KHK_miR_1345 | SEQ ID NO:345 | SEQ ID NO:1750 |
| KHK_miR_1346 | SEQ ID NO:346 | SEQ ID NO:1751 |
| KHK_miR_1347 | SEQ ID NO:347 | SEQ ID NO:1752 |
| KHK_miR_1348 | SEQ ID NO:348 | SEQ ID NO:1753 |
| KHK_miR_1349 | SEQ ID NO:349 | SEQ ID NO:1754 |
| KHK_miR_1350 | SEQ ID NO:350 | SEQ ID NO:1755 |
| KHK_miR_1351 | SEQ ID NO:351 | SEQ ID NO:1756 |
| KHK_miR_1352 | SEQ ID NO:352 | SEQ ID NO:1757 |
| KHK_miR_1353 | SEQ ID NO:353 | SEQ ID NO:1758 |
| KHK_miR_1354 | SEQ ID NO:354 | SEQ ID NO:1759 |
| KHK_miR_1355 | SEQ ID NO:355 | SEQ ID NO:1760 |
| KHK_miR_1356 | SEQ ID NO:356 | SEQ ID NO:1761 |
| KHK_miR_1357 | SEQ ID NO:357 | SEQ ID NO:1762 |
| KHK_miR_1358 | SEQ ID NO:358 | SEQ ID NO:1763 |
| KHK_miR_1359 | SEQ ID NO:359 | SEQ ID NO:1764 |
| KHK_miR_1360 | SEQ ID NO:360 | SEQ ID NO:1765 |

**[Table 1-12]**

| nucleic acid name | micro-RNA | micro-RNA precursor |
|---|---|---|
| | | SEQ ID NO:1766 |
| | | SEQ ID NO:1767 |
| KHK_miR_1361 | SEQ ID NO:361 | SEQ ID NO:1768 |
| KHK_miR_1362 | SEQ ID NO:362 | SEQ ID NO:1769 |
| KHK_miR_1363 | SEQ ID NO:363 | SEQ ID NO:1770 |
| | | SEQ ID NO:1771 |
| KHK_miR_1364 | SEQ ID NO:364 | SEQ ID NO:1772 |
| | | SEQ ID NO:1773 |
| | | SEQ ID NO:1774 |
| | | SEQ ID NO:1775 |
| | | SEQ ID NO:1776 |
| | | SEQ ID NO:1777 |
| KHK_miR_1365 | SEQ ID NO:365 | SEQ ID NO:1778 |
| KHK_miR_1366 | SEQ ID NO:366 | SEQ ID NO:1779 |
| KHK_miR_1367 | SEQ ID NO:367 | SEQ ID NO:1780 |
| KHK_miR_1368 | SEQ ID NO:368 | SEQ ID NO:1781 |
| KHK_miR_1369 | SEQ ID NO:369 | SEQ ID NO:1782 |
| KHK_miR_1370 | SEQ ID NO:370 | SEQ ID NO:1783 |
| KHK_miR_1371 | SEQ ID NO:371 | SEQ ID NO:1784 |
| KHK_miR_1372 | SEQ ID NO:372 | SEQ ID NO:1785 |
| KHK_miR_1373 | SEQ ID NO:373 | SEQ ID NO:1786 |
| KHK_miR_1374 | SEQ ID NO:374 | SEQ ID NO:1787 |
| KHK_miR_1375 | SEQ ID NO:375 | SEQ ID NO:1788 |
| KHK_miR_1376 | SEQ ID NO:376 | SEQ ID NO:1789 |
| KHK_miR_1377 | SEQ ID NO:377 | SEQ ID NO:1790 |
| KHK_miR_1378 | SEQ ID NO:378 | SEQ ID NO:1791 |
| KHK_miR_1379 | SEQ ID NO:379 | SEQ ID NO:1792 |
| | | SEQ ID NO:1793 |
| KHK_miR_1380 | SEQ ID NO:380 | SEQ ID NO:1794 |
| KHK_miR_1381 | SEQ ID NO:381 | SEQ ID NO:1795 |
| KHK_miR_1382 | SEQ ID NO:382 | SEQ ID NO:1796 |
| KHK_miR_1383 | SEQ ID NO:383 | SEQ ID NO:1797 |
| KHK_miR_1384 | SEQ ID NO:384 | SEQ ID NO:1798 |
| KHK_miR_1385 | SEQ ID NO:385 | SEQ ID NO:1799 |
| KHK_miR_1386 | SEQ ID NO:386 | SEQ ID NO:1800 |
| KHK_miR_1387 | SEQ ID NO:387 | SEQ ID NO:1801 |
| KHK_miR_1388 | SEQ ID NO:388 | SEQ ID NO:1802 |
| KHK_miR_1389 | SEQ ID NO:389 | SEQ ID NO:1803 |
| KHK_miR_1390 | SEQ ID NO:390 | SEQ ID NO:1804 |

**[Table 1-13]**

| nucleic acid name | micro-RNA | micro-RNA precursor |
|---|---|---|
| KHK_miR_1391 | SEQ ID NO:391 | SEQ ID NO:1805 |
| KHK_miR_1392 | SEQ ID NO:392 | SEQ ID NO:1806 |
| KHK_miR_1393 | SEQ ID NO:393 | SEQ ID NO:1807 |
| KHK_miR_1394 | SEQ ID NO:394 | SEQ ID NO:1808 |
| KHK_miR_1395 | SEQ ID NO:395 | SEQ ID NO:1809 |
| KHK_miR-1396 | SEQ ID NO:396 | SEQ ID NO:1810 |
| KHK_miR_1397 | SEQ ID NO:397 | SEQ ID NO:1811 |
| KHK_miR_1398 | SEQ ID NO:398 | SEQ ID NO:1812 |
| | | SEQ ID NO:1813 |
| KHK_miR_1399 | SEQ ID NO:399 | SEQ ID NO:1814 |
| KHK_miR_1400 | SEQ ID NO:400 | SEQ ID NO:1815 |
| KHK_miR_1401 | SEQ ID NO:401 | SEQ ID NO:1816 |
| KHK_miR_1402 | SEQ ID NO:402 | SEQ ID NO:1817 |
| KHK_miR_1403 | SEQ ID NO:403 | SEQ ID NO:1818 |
| KHK_miR_1404 | SEQ ID NO:404 | SEQ ID NO:1819 |
| KHK_miR_1405 | SEQ ID NO:405 | SEQ ID NO:1820 |
| KHK_miR_1406 | SEQ ID NO:406 | SEQ ID NO:1821 |
| KHK_miR_1407 | SEQ ID NO:407 | SEQ ID NO:1822 |
| KHK_miR_1408 | SEQ ID NO:408 | SEQ ID NO:1823 |
| KHK_miR_1409 | SEQ ID NO:409 | SEQ ID NO:1824 |
| KHK_miR_1410 | SEQ ID NO:410 | SEQ ID NO:1825 |
| KHK_miR_1411 | SEQ ID NO:411 | SEQ ID NO:1826 |
| KHK_miR_1412 | SEQ ID NO:412 | SEQ ID NO:1827 |
| | | SEQ ID NO:1828 |
| KHK_miR_1413 | SEQ ID NO:413 | SEQ ID NO:1829 |
| KHK_miR_1414 | SEQ ID NO:414 | SEQ ID NO:1830 |
| KHK_miR_1415 | SEQ ID NO:415 | SEQ ID NO:1831 |
| KHK_miR_1416 | SEQ ID NO:416 | SEQ ID NO:1832 |
| KHK_miR_1417 | SEQ ID NO:417 | SEQ ID NO:1833 |
| KHK_miR_1418 | SEQ ID NO:418 | SEQ ID NO:1834 |
| KHK_miR_1419 | SEQ ID NO:419 | SEQ ID NO:1835 |
| KHK_miR_1420 | SEQ ID NO:420 | SEQ ID NO:1836 |
| KHK_miR_1421 | SEQ ID NO:421 | SEQ ID NO:1837 |
| | | SEQ ID NO:1838 |
| KHK_miR_1422 | SEQ ID NO:422 | SEQ ID NO:1839 |
| KHK_miR_1423 | SEQ ID NO:423 | SEQ ID NO:1840 |
| KHK_miR_1424 | SEQ ID NO:424 | SEQ ID NO:1841 |
| KHK_miR_1425 | SEQ ID NO:425 | SEQ ID NO:1842 |
| | | SEQ ID NO:1843 |

**[Table 1-14]**

| nucleic acid name | micro-RNA | micro-RNA precursor |
|---|---|---|
| KHK_miR_1426 | SEQ ID NO:426 | SEQ ID NO:1844 |
| KHK_miR_1427 | SEQ ID NO:427 | SEQ ID NO:1845 |
| KHK_miR_1428 | SEQ ID NO:428 | SEQ ID NO:1846 |
| KHK_miR_1429 | SEQ ID NO:429 | SEQ ID NO:1847 |
| KHK_miR_1430 | SEQ ID NO:430 | SEQ ID NO:1848 |
| KHK_miR_1431 | SEQ ID NO:431 | SEQ ID NO:1849 |
| | | SEQ ID NO:1850 |
| KHK_miR_1432 | SEQ ID NO:432 | SEQ ID NO:1851 |
| KHK_miR_1433 | SEQ ID NO:433 | SEQ ID NO:1852 |
| KHK_miR_1434 | SEQ ID NO:434 | SEQ ID NO:1853 |
| KHK_miR_1435 | SEQ ID NO:435 | SEQ ID NO:1854 |
| KHK_miR_1436 | SEQ ID NO:436 | SEQ ID NO:1855 |
| KHK_miR_1437 | SEQ ID NO:437 | SEQ ID NO:1856 |
| KHK_miR_1438 | SEQ ID NO:438 | SEQ ID NO:1857 |
| KHK_miR_1439 | SEQ ID NO:439 | SEQ ID NO:1858 |
| KHK_miR_1440 | SEQ ID NO:440 | SEQ ID NO:1859 |
| KHK_miR_1441 | SEQ ID NO:441 | SEQ ID NO:1860 |
| KHK_miR_1442 | SEQ ID NO:442 | SEQ ID NO:1861 |
| KHK_miR_1443 | SEQ ID NO:443 | SEQ ID NO:1862 |
| KHK_miR_1444 | SEQ ID NO:444 | SEQ ID NO:1863 |
| KHK_miR_1445 | SEQ ID NO:445 | SEQ ID NO:1864 |
| KHK_miR_1446 | SEQ ID NO:446 | SEQ ID NO:1865 |
| | | SEQ ID NO:1866 |
| KHK_miR_1447 | SEQ ID NO:447 | SEQ ID NO:1867 |
| KHK_miR_1448 | SEQ ID NO:448 | SEQ ID NO:1868 |
| KHK_miR_1449 | SEQ ID NO:449 | SEQ ID NO:1869 |
| KHK_miR_1450 | SEQ ID NO:450 | SEQ ID NO:1870 |
| KHK_miR_1451 | SEQ ID NO:451 | SEQ ID NO:1871 |
| KHK_miR_1452 | SEQ ID NO:452 | SEQ ID NO:1872 |
| KHK_miR_1453 | SEQ ID NO:453 | SEQ ID NO:1873 |
| KHK_miR_1454 | SEQ ID NO:454 | SEQ ID NO:1874 |
| KHK_miR_1455 | SEQ ID NO:455 | SEQ ID NO:1875 |
| KHK_miR_1456 | SEQ ID NO:456 | SEQ ID NO:1876 |
| KHK_miR_1457 | SEQ ID NO:457 | SEQ ID NO:1877 |
| KHK_miR_1458 | SEQ ID NO:458 | SEQ ID NO:1878 |
| KHK_miR_1459 | SEQ ID NO:459 | SEQ ID NO:1879 |
| KHK_miR_1460 | SEQ ID NO:460 | SEQ ID NO:1880 |
| KHK_miR_1461 | SEQ ID NO:461 | SEQ ID NO:1881 |
| KHK_miR_1462 | SEQ ID NO:462 | SEQ ID NO:1882 |

**[Table 1-15]**

| nucleic acid name | micro-RNA | micro-RNA precursor |
|---|---|---|
| KHK_miR_1463 | SEQ ID NO:463 | SEQ ID NO:1883 |
| KHK_miR_1464 | SEQ ID NO:464 | SEQ ID NO:1884 |
| KHK_miR_1465 | SEQ ID NO:465 | SEQ ID NO:1885 |
| KHK_miR_1466 | SEQ ID NO:466 | SEQ ID NO:1886 |
| KHK_miR_1467 | SEQ ID NO:467 | SEQ ID NO:1887 |
| KHK_miR_1468 | SEQ ID NO:468 | SEQ ID NO:1888 |
| KHK_miR_1469 | SEQ ID NO:469 | SEQ ID NO:1889 |
| KHK_miR_1470 | SEQ ID NO:470 | SEQ ID NO:1890 |
| KHK_miR_1471 | SEQ ID NO:471 | SEQ ID NO:1891 |
| KHK_miR_1472 | SEQ ID NO:472 | SEQ ID NO:1892 |
| KHK_miR_1473 | SEQ ID NO:473 | SEQ ID NO:1893 |
| KHK_miR_1474 | SEQ ID NO:474 | SEQ ID NO:1894 |
| KHK_miR_1475 | SEQ ID NO:475 | SEQ ID NO:1895 |
| KHK_miR_1476 | SEQ ID NO:476 | SEQ ID NO:1896 |
| KHK_miR_1477 | SEQ ID NO:477 | SEQ ID NO:1897 |
| KHK_miR_1478 | SEQ ID NO:478 | SEQ ID NO:1898 |
| KHK_miR_1479 | SEQ ID NO:479 | SEQ ID NO:1899 |
| KHK_miR_1480 | SEQ ID NO:480 | SEQ ID NO:1900 |
| KHK_miR_1481 | SEQ ID NO:481 | SEQ ID NO:1901 |
| KHK_miR_1482 | SEQ ID NO:482 | SEQ ID NO:1902 |
| KHK_miR_1483 | SEQ ID NO:483 | SEQ ID NO:1903 |
| KHK_miR_1484 | SEQ ID NO:484 | SEQ ID NO:1904 |
| KHK_miR_1485 | SEQ ID NO:485 | SEQ ID NO:1905 |
| KHK_miR_1488 | SEQ ID NO:486 | SEQ ID NO:1906 |
| KHK_miR_1487 | SEQ ID NO:487 | SEQ ID NO:1907 |
| KHK_miR_1488 | SEQ ID NO:488 | SEQ ID NO:1908 |
| KHK_miR_1489 | SEQ ID NO:489 | SEQ ID NO:1909 |
| KHK_miR_1490 | SEQ ID NO:490 | SEQ ID NO:1910 |
| KHK_miR_1491 | SEQ ID NO:491 | SEQ ID NO:1911 |
| KHK_miR_1492 | SEQ ID NO:492 | SEQ ID NO:1912 |
| KHK_miR_1493 | SEQ ID NO:493 | SEQ ID NO:1913 |
| KHK_miR_1494 | SEQ ID NO:494 | SEQ ID NO:1914 |
| KHK_miR_1495 | SEQ ID NO:495 | SEQ ID NO:1915 |
| KHK_miR_1498 | SEQ ID NO:496 | SEQ ID NO:1916 |
| KHK_miR_1497 | SEQ ID NO:497 | SEQ ID NO:1917 |
| KHK_miR_1498 | SEQ ID NO:498 | SEQ ID NO:1918 |
| KHK_miR_1499 | SEQ ID NO:499 | SEQ ID NO:1919 |
| | | SEQ ID NO:1920 |
| | | SEQ ID NO:1921 |

**[Table 1-16]**

| nucleic acid name | micro-RNA | micro-RNA precursor |
|---|---|---|
| KHK_miR_1500 | SEQ ID NO:500 | SEQ ID NO:1922 |
| KHK_miR_1501 | SEQ ID NO:501 | SEQ ID NO:1923 |
| KHK_miR_1502 | SEQ ID NO:502 | SEQ ID NO:1924 |
| KHK_miR_1503 | SEQ ID NO:503 | SEQ ID NO:1925 |
| KHK_miR_1504 | SEQ ID NO:504 | SEQ ID NO:1926 |
| KHK_miR_1505 | SEQ ID NO:505 | SEQ ID NO:1927 |
| KHK_miR_1506 | SEQ ID NO:506 | SEQ ID NO:1928 |
| KHK_miR_1507 | SEQ ID NO:507 | SEQ ID NO:1929 |
| | | SEQ ID NO:1930 |
| KHK_miR_1508 | SEQ ID NO:508 | SEQ ID NO:1931 |
| | | SEQ ID NO:1932 |
| KHK_miR_1509 | SEQ ID NO:509 | SEQ ID NO:1933 |
| KHK_miR_1510 | SEQ ID NO:510 | SEQ ID NO:1934 |
| KHK_miR_1511 | SEQ ID NO:511 | SEQ ID NO:1935 |
| KHK_miR_1512 | SEQ ID NO:512 | SEQ ID NO:1936 |
| KHK_miR_1513 | SEQ ID NO:513 | SEQ ID NO:1937 |
| KHK_miR_1514 | SEQ ID NO:514 | SEQ ID NO:1938 |
| KHK_miR_1515 | SEQ ID NO:515 | SEQ ID NO:1939 |
| KHK_miR_1516 | SEQ ID NO:516 | SEQ ID NO:1940 |
| KHK_miR_1517 | SEQ ID NO:517 | SEQ ID NO:1941 |
| KHK_miR_1518 | SEQ ID NO:518 | SEQ ID NO:1942 |
| KHK_miR_1519 | SEQ ID NO:519 | SEQ ID NO:1943 |
| | | SEQ ID NO:1944 |
| KHK_miR_1520 | SEQ ID NO:520 | SEQ ID NO:1945 |
| KHK_miR_1521 | SEQ ID NO:521 | SEQ ID NO:1946 |
| KHK_miR_1522 | SEQ ID NO:522 | SEQ ID NO:1947 |
| KHK_miR_1523 | SEQ ID NO:523 | SEQ ID NO:1948 |
| KHK_miR_1524 | SEQ ID NO:524 | SEQ ID NO:1949 |
| KHK_miR_1525 | SEQ ID NO:525 | SEQ ID NO:1950 |
| KHK_miR_1528 | SEQ ID NO:526 | SEQ ID NO:1951 |
| KHK_miR_1527 | SEQ ID NO:527 | SEQ ID NO:1952 |
| KHK_miR_1528 | SEQ ID NO:528 | SEQ ID NO:1953 |
| KHK_miR_1529 | SEQ ID NO:529 | SEQ ID NO:1954 |
| KHK_miR_1530 | SEQ ID NO:530 | SEQ ID NO:1955 |
| KHK_miR_1531 | SEQ ID NO:531 | SEQ ID NO:1956 |
| KHK_miR_1532 | SEQ ID NO:532 | SEQ ID NO:1957 |
| | | SEQ ID NO:1958 |
| KHK_miR_1533 | SEQ ID NO:533 | SEQ ID NO:1959 |
| KHK_miR_1534 | SEQ ID NO:534 | SEQ ID NO:1960 |

**[Table 1-17]**

| nucleic acid name | micro-RNA | micro-RNA precursor |
|---|---|---|
| KHK_miR_1535 | SEQ ID NO:535 | SEQ ID NO:1961 |
| KHK_miR_1536 | SEQ ID NO:536 | SEQ ID NO:1962 |
| KHK_miR_1537 | SEQ ID NO:537 | SEQ ID NO:1963 |
| KHK_miR_1538 | SEQ ID NO:538 | SEQ ID NO:1964 |
| KHK_miR_1539 | SEQ ID NO:539 | SEQ ID NO:1965 |
| KHK_miR_1544 | SEQ ID NO:540 | SEQ ID NO:1966 |
| KHK_miR_1541 | SEQ ID NO:541 | SEQ ID NO:1967 |
| KHK_miR_1542 | SEQ ID NO:542 | SEQ ID NO:1968 |
| KHK_miR_1543 | SEQ ID NO:543 | SEQ ID NO:1969 |
| KHK_miR_1544 | SEQ ID NO:544 | SEQ ID NO:1970 |
| KHK_miR_1545 | SEQ ID NO:545 | SEQ ID NO:1971 |
| KHK_miR_1546 | SEQ ID NO:546 | SEQ ID NO:1972 |
| KHK_miR_1547 | SEQ ID NO:547 | SEQ ID NO:1973 |
| KHK_miR_1548 | SEQ ID NO:548 | SEQ ID NO:1974 |
| KHK_miR_1549 | SEQ ID NO:549 | SEQ ID NO:1975 |
| KHK_miR_1550 | SEQ ID NO:550 | SEQ ID NO:1976 |
| KHK_miR_1551 | SEQ ID NO:551 | SEQ ID NO:1977 |
| KHK_miR_1552 | SEQ ID NO:552 | SEQ ID NO:1978 |
| KHK_miR_1553 | SEQ ID NO:553 | SEQ ID NO:1979 |
| KHK_miR_1554 | SEQ ID NO:554 | SEQ ID NO:1980 |
| KHK_miR_1555 | SEQ ID NO:555 | SEQ ID NO:1981 |
| KHK_miR_1556 | SEQ ID NO:556 | SEQ ID NO:1982 |
| KHK_miR_1557 | SEQ ID NO:557 | SEQ ID NO:1983 |
| | | SEQ ID NO:1984 |
| KHK_miR_1558 | SEQ ID NO:558 | SEQ ID NO:1985 |
| KHK_miR_1559 | SEQ ID NO:559 | SEQ ID NO:1986 |
| KHK_miR_1580 | SEQ ID NO:560 | SEQ ID NO:1987 |
| KHK_miR_1561 | SEQ ID NO:561 | SEQ ID NO:1988 |
| KHK_miR_1562 | SEQ ID NO:562 | SEQ ID NO:1989 |
| KHK_miR_1563 | SEQ ID NO:563 | SEQ ID NO:1990 |
| KHK_miR_1564 | SEQ ID NO:564 | SEQ ID NO:1991 |
| KHK_miR_1565 | SEQ ID NO:565 | SEQ ID NO:1992 |
| KHK_miR_1566 | SEQ ID NO:566 | SEQ ID NO:1993 |
| KHK_miR_1567 | SEQ ID NO:567 | SEQ ID NO:1994 |
| KHK_miR_1568 | SEQ ID NO:568 | SEQ ID NO:1995 |
| | | SEQ ID NO:1996 |
| | | SEQ ID NO:1997 |
| KHK_miR_1569 | SEQ ID HO:569 | SEQ ID NO:1998 |
| KHK_miR_1570 | SEQ ID NO:570 | SEQ ID NO:1999 |

**[Table 1-18]**

| nucleic acid name | micro-RNA | micro-RNA precursor |
|---|---|---|
| KHK_miR_1571 | SEQ ID NO:571 | SEQ ID NO:2000 |
| KHK_miR_1572 | SEQ ID NO:572 | SEQ ID NO:2001 |
| KHK_miR_1573 | SEQ ID NO:573 | SEQ ID NO:2002 |
| KHK_miR_1574 | SEQ ID NO:574 | SEQ ID NO:2003 |
| KHK_miR_1575 | SEQ ID NO:575 | SEQ ID NO:2004 |
| KHK_miR_1576 | SEQ ID NO:576 | SEQ ID NO:2005 |
| | | SEQ ID NO:2006 |
| | | SEQ ID NO:2007 |
| | | SEQ ID NO:2008 |
| | | SBQ ID NO:2009 |
| KHK_miR_1577 | SEQ ID NO:577 | SEQ ID NO:2010 |
| KHK_miR_1578 | SEQ ID NO:578 | SEQ ID NO:2011 |
| KHK_miR_1579 | SEQ ID NO:579 | SEQ ID NO:2012 |
| KHK_miR_1580 | SEQ ID NO:580 | SEQ ID NO:2013 |
| KHK_miR_1581 | SEQ ID NO:581 | SEQ ID NO:2014 |
| KHK_miR_1582 | SEQ ID NO:582 | SEQ ID NO:2015 |
| KHK_miR_1583 | SEQ ID NO:583 | SEQ ID NO:2016 |
| | | SEQ ID NO:2017 |
| | | SEQ ID NO:2018 |
| KHK_miR_1584 | SEQ ID NO:584 | SEQ ID NO:2019 |
| KHK_miR_1585 | SEQ ID NO:585 | SEQ ID NO:2020 |
| KHK_miR_1586 | SEQ ID NO:586 | SEQ ID NO:2021 |
| KHK_miR_1587 | SEQ ID NO:587 | SEQ ID NO:2022 |
| KHK_miR_1588 | SEQ ID NO:588 | SEQ ID NO:2023 |
| KHK_miR_1589 | SEQ ID NO:589 | SEQ ID NO:2024 |
| KHK_miR_1590 | SEQ ID NO:590 | SEQ ID NO:2025 |
| KHK_miR_1591 | SEQ ID NO:591 | SEQ ID NO:2026 |
| KHK_miR_1592 | SEQ ID NO:592 | SEQ ID NO:2027 |
| KHK_miR_1593 | SEQ ID NO:593 | SEQ ID NO:2028 |
| KHK_miR_1594 | SEQ ID NO:594 | SEQ ID NO:2029 |
| KHK_miR_1595 | SEQ ID NO:595 | SEQ ID NO:2030 |
| KHK_miR_1596 | SEQ ID NO:596 | SEQ ID NO:2031 |
| KHK_miR_1597 | SEQ ID NO:597 | SEQ ID NO:2032 |
| KHK_miR_1598 | SEQ ID NO:598 | SEQ ID NO:2033 |
| KHK_miR_1599 | SEQ ID NO:599 | SEQ ID NO:2034 |
| KHK_miR_1600 | SEQ ID NO:600 | SEQ ID NO:2035 |
| | | SEQ ID NO:2036 |
| | | SEQ ID NO:2037 |
| KHK_miR_1601 | SEQ ID NO:601 | SEQ ID NO:2038 |

**[Table 1-19]**

| nucleic acid name | micro-RNA | micro-RNA precursor |
|---|---|---|
| KHK_miR_1602 | SEQ ID NO:602 | SEQ ID NO:2039 |
| KHK_miR_1603 | SEQ ID NO:603 | SEQ ID NO:2040 |
| KHK_miR_1604 | SEQ ID NO:604 | SEQ ID NO:2041 |
| KHK_miR_1605 | SEQ ID NO:605 | SEQ ID NO:2042 |
| KHK_miR_1606 | SEQ ID NO:606 | SEQ ID NO:2043 |
| KHK_miR_1607 | SEQ ID NO:607 | SEQ ID NO:2044 |
| KHK_miR_1608 | SEQ ID NO:608 | SEQ ID NO:2045 |
| KHK_miR_1609 | SEQ ID NO:609 | SEQ ID NO:2046 |
| KHK_miR_1610 | SEQ ID NO:610 | SEQ ID NO:2047 |
| KHK_miR_1611 | SEQ ID NO:611 | SEQ ID NO:2048 |
| KHK_miR_1612 | SEQ ID NO:612 | SEQ ID NO:2049 |
| KHK_miR_1613 | SEQ ID NO:613 | SEQ ID NO:2050 |
| KHK_miR_1614 | SEQ ID No:614 | SEQ ID NO:2051 |
| KHK_miR_1615 | SEQ ID NO:615 | SEQ ID NO:2052 |
| KHK_miR_1616 | SEQ ID NO:616 | SEQ ID NO:2053 |
| KHK_miR_1617 | SEQ ID NO:617 | SEQ ID NO:2054 |
| KHK_miR_1618 | SEQ ID NO:618 | SEQ ID NO:2055 |
| KHK_miR_1619 | SEQ ID NO:619 | SEQ ID NO:2056 |
| KHK_miR_1620 | SEQ ID NO:620 | SEQ ID NO:2057 |
| KHK_miR_1621 | SEQ ID NO:621 | SEQ ID NO:2058 |
| KHK_miR_1622 | SEQ ID NO:622 | SEQ ID NO:2059 |
| KHK_miR_1623 | SEQ ID NO:623 | SEQ ID NO:2060 |
| KHK_miR_1624 | SEQ ID NO:624 | SEQ ID NO:2061 |
| KHK_miR_1625 | SEQ ID NO:625 | SEQ ID NO:2062 |
| KHK_miR_1626 | SEQ ID NO:626 | SEQ ID NO:2063 |
| KHK_miR_1627 | SEQ ID NO:627 | SEQ ID NO:2064 |
| KHK_miR_1628 | SEQ ID NO:628 | SEQ ID NO:2065 |
| KHK_miR_1629 | SEQ ID NO:629 | SEQ ID NO:2066 |
| KHK_miR_1630 | SEQ ID NO:630 | SEQ ID NO:2067 |
| KHK_miR_1631 | SEQ ID NO:631 | SEQ ID NO:2068 |
| KHK_miR_1632 | SEQ ID NO:632 | SEQ ID NO:2069 |
| KHK_miR_1633 | SEQ ID NO:633 | SEQ ID NO:2070 |
| KHK_miR_1634 | SEQ ID NO:634 | SEQ ID NO:2071 |
| KHK_miR_1635 | SEQ ID NO:635 | SEQ ID NO:2072 |
| KHK_miR_1636 | SEQ ID NO:636 | SEQ ID NO:2073 |
| KHK_miR_1637 | SEQ ID NO:637 | SEQ ID NO:2074 |
| KHK_miR_1638 | SEQ ID NO:638 | SEQ ID NO:2075 |
| | | SEQ ID NO:2076 |
| | | SEQ ID NO:2077 |

**[Table 1-20]**

| nucleic acid name | micro-RNA | micro-RNA precursor |
|---|---|---|
| | | SEQ ID NO:2078 |
| KHK_miR_1639 | SEQ ID NO:639 | SEQ ID NO:2079 |
| KHK_miR_1640 | SEQ ID NO:640 | SEQ ID NO:2080 |
| KHK_miR_1641 | SEQ ID NO:641 | SEQ ID NO:2081 |
| KHK_miR_1642 | SEQ ID NO:642 | SEQ ID NO:2082 |
| | | SEQ ID NO:2083 |
| KHK_miR_1643 | SEQ ID NO:643 | SEQ ID NO:2084 |
| KHK_miR_1644 | SEQ ID NO:644 | SEQ ID NO:2085 |
| KHK_miR_1645 | SEQ ID NO:645 | SEQ ID NO:2086 |
| KHK_miR_1648 | SEQ ID NO:646 | SEQ ID NO:2087 |
| KHK_miR_1647 | SEQ ID NO:647 | SEQ ID NO:2088 |
| KHK_miR_1648 | SEQ ID NO:648 | SEQ ID NO:2089 |
| KHK_miR_1849 | SEQ ID NO:649 | SEQ ID NO:2090 |
| KHK_miR_1650 | SEQ ID NO:650 | SEQ ID NO:2091 |
| KHK_miR_1651 | SEQ ID NO:651 | SEQ ID NO:2092 |
| KHK_miR_1652 | SEQ ID NO:652 | SEQ ID NO:2093 |
| KHK_miR_1653 | SEQ ID NO:653 | SEQ ID NO:2094 |
| | | SEQ ID NO:2095 |
| KHK_miR_1654 | SEQ ID NO:654 | SEQ ID NO:2096 |
| KHK_miR_1655 | SEQ ID NO:655 | SEQ ID NO:2097 |
| KHK_miR_1656 | SEQ ID NO:656 | SEQ ID NO:2098 |
| KHK_miR_1657 | SEQ ID NO:657 | SEQ ID NO:2099 |
| KHK_miR_1658 | SEQ ID NO:658 | SEQ ID NO:2100 |
| | | SEQ ID NO:2101 |
| KHK_miR_1659 | SEQ ID NO:659 | SEQ ID NO:2102 |
| KHK_miR_1660 | SEQ ID NO:660 | SEQ ID NO:2103 |
| KHK_miR_1661 | SEQ ID NO:661 | SEQ ID NO:2104 |
| KHK_miR_1662 | SEQ ID NO:662 | SEQ ID NO:2105 |
| KHK_miR_1663 | SEQ ID NO:663 | SEQ ID NO:2106 |
| KHK_miR_1664 | SEQ ID NO:664 | SEQ ID NO:2107 |
| KHK_miR_1665 | SEQ ID NO:665 | SEQ ID NO:2108 |
| KHK_miR_1666 | SEQ ID NO:666 | SEQ ID NO:2109 |
| KHK_miR_1667 | SEQ ID NO:667 | SEQ ID NO:2110 |
| KHK_miR_1668 | SEQ ID NO:668 | SEQ ID NO:2111 |
| KHK_miR_1669 | SEQ ID NO:669 | SEQ ID NO:2112 |
| KHK-miR_1670 | SEQ ID NO:670 | SEQ ID NO:2113 |
| KHK_miR_1671 | SEQ ID NO:671 | SEQ ID NO:2114 |
| KHK_miR_1672 | SEQ ID NO:672 | SEQ ID NO:2115 |
| KHK_miR_1673 | SEQ ID NO:673 | SEQ ID NO:2116 |

**[Table 1-21]**

| | | |
|---|---|---|
| nucleic acid name | micro-RNA | micro-RNA precursor |
| KHK_miR_1674 | SEQ ID NO:674 | SEQ ID NO:2117 |
| KHK_miR_1675 | SEQ ID NO:675 | SEQ ID NO:2118 |
| KHK_miR_1676 | SEQ ID NO:676 | SEQ ID NO:2119 |
| KHK_miR_1677 | SEQ ID NO:677 | SEQ ID NO:2120 |
| KHK_miR_1678 | SEQ ID NO:678 | SEQ ID NO:2121 |
| | | SEQ ID NO:2122 |
| KHK_miR_1679 | SEQ ID NO:679 | SEQ ID NO:2123 |
| KHK_miR_1680 | SEQ ID NO:680 | SEQ ID NO:2124 |
| | | SEQ ID NO:2125 |
| | | SEQ ID NO:2126 |
| | | SEQ ID NO:2127 |
| KHK_miR_1681 | SEQ ID NO:681 | SEQ ID NO:2128 |
| KHK_miR_1682 | SEQ ID NO:682 | SEQ ID NO:2129 |
| KHK_miR_1683 | SEQ ID NO:683 | SEQ ID NO:2130 |
| KHK_miR_1684 | SEQ ID NO:684 | SEQ ID NO:2131 |
| KHK_miR_1685 | SEQ ID NO:685 | SEQ ID NO:2132 |
| KHK_miR_1686 | SEQ ID NO:686 | SEQ ID NO:2133 |
| KHK_miR_1687 | SEQ ID NO:687 | SEQ ID NO:2134 |
| KHK_miR_1688 | SEQ ID NO:688 | SEQ ID NO:2135 |
| KHK_miR_1689 | SEQ ID NO:689 | SEQ ID NO:2136 |
| KHK_miR_1690 | SEQ ID NO:690 | SEQ ID NO:2137 |
| KHK_miR_1691 | SEQ ID NO:691 | SEQ ID NO:2138 |
| | | SEQ ID NO:2139 |
| KHK_miR_1692 | SEQ ID NO:692 | SEQ ID NO:2140 |
| KHK_miR_1693 | SEQ ID NO:693 | SEQ ID NO:2141 |
| KHK_miR_1694 | SEQ ID NO:694 | SEQ ID NO:2142 |
| KHK_miR_1695 | SEQ ID NO:695 | SEQ ID NO:2143 |
| KHK_miR_1696 | SEQ ID NO:696 | SEQ ID NO:2144 |
| KHK_miR_1697 | SEQ ID NO:697 | SEQ ID NO:2145 |
| KHK_miR_1698 | SEQ ID NO:698 | SEQ ID NO:2146 |
| KHK_miR_1699 | SEQ ID NO:699 | SEQ ID NO:2147 |
| KHK_miR_1700 | SEQ ID NO:700 | SEQ ID NO:2148 |
| KHK_miR_1701 | SEQ ID NO:701 | SEQ ID NO:2149 |
| KHK_miR_1702 | SEQ ID NO:702 | SEQ ID NO:2150 |
| KHK_miR_1703 | SEQ ID NO:703 | SEQ ID NO:2151 |
| KHK_miR_1704 | SEQ ID NO:704 | SEQ ID NO:2152 |
| KHK_miR_1705 | SEQ ID NO:705 | SEQ ID NO:2153 |
| KHK_miR_1706 | SEQ ID NO:706 | SEQ ID NO:2154 |
| KHK_miR_1707 | SEQ ID NO:707 | SEQ ID NO:2155 |

**[Table 1-22]**

| nucleic acid name | micro-RNA | micro-RNA precursor |
|---|---|---|
| HK_miR_1708 | SEQ ID NO:708 | SEQ ID NO:2156 |
| KHK_miR_1709 | SEQ ID NO:709 | SEQ ID NO:2157 |
| KHK_miR_1710 | SEQ ID NO:710 | SEQ ID NO:2158 |
| KHK_miR_1711 | SEQ ID NO:711 | SEQ ID NO:2159 |
| KHK_miR_1712 | SEQ ID NO:712 | SEQ ID NO:2160 |
| KHK_miR_1713 | SEQ ID NO:713 | SEQ ID NO:2161 |
| KHK_miR_1714 | SEQ ID NO:714 | SEQ ID NO:2162 |
| KHK_miR_1715 | SEQ ID NO:715 | SEQ ID NO:2163 |
| KHK_miR_1716 | SEQ ID NO:716 | SEQ ID NO:2164 |
| KHK_miR_1717 | SEQ ID NO:717 | SEQ ID NO:2165 |
| KHK_miR_1718 | SEQ ID NO:718 | SEQ ID NO:2166 |
| KHK_miR_1719 | SEQ ID NO:719 | SEQ ID NO:2167 |
| KHK_miR_1720 | SEQ ID NO:720 | SEQ ID NO:2168 |
| KHK_miR_1721 | SEQ ID NO:721 | SEQ ID NO:2169 |
| KHK_miR_1722 | SEQ ID NO:722 | SEQ ID NO:2170 |
| KHK_miR_1723 | SEQ ID NO:723 | SEQ ID NO:2171 |
| KHK_miR_1724 | SEQ ID NO:724 | SEQ ID NO:2172 |
| KHK_miR_1725 | SEQ ID NO:725 | SEQ ID NO:2173 |
| KHK_miR_1726 | SEQ ID NO:726 | SEQ ID NO:2174 |
| KHK_miR_1727 | SEQ ID NO:727 | SEQ ID NO:2175 |
| KHK_miR_1728 | SEQ ID NO:728 | SEQ ID NO:2176 |
| KHK_miR_1729 | SEQ ID NO:729 | SEQ ID NO:2177 |
| KHK_miR_1730 | SEQ ID NO:730 | SEQ ID NO:2178 |
| KHK_miR_1731 | SEQ ID NO:731 | SEQ ID NO:2179 |
| KHK_miR_1732 | SEQ ID NO:732 | SEQ ID NO:2180 |
| KHK_miR_1733 | SEQ ID NO:733 | SEQ ID NO:2181 |
| KHK_miR_1734 | SEQ ID NO:734 | SEQ ID NO:2182 |
| KHK_miR_1735 | SEQ ID NO:735 | SEQ ID NO:2183 |
| KHK_miR_1736 | SEQ ID NO:736 | SEQ ID NO:2184 |
| KHK_miR_1737 | SEQ ID NO:737 | SEQ ID NO:2185 |
| KHK_miR_1738 | SEQ ID NO:738 | SEQ ID NO:2186 |
| KHK_miR_1739 | SEQ ID NO:739 | SEQ ID NO:2187 |
| KHK_miR_1740 | SEQ ID NO:740 | SEQ ID NO:2188 |
| KHK_miR_1741 | SEQ ID NO:741 | SEQ ID NO:2189 |
| KHK_miR_1742 | SEQ ID NO:742 | SEQ ID NO:2190 |
| | | SEQ ID NO:2191 |
| | | SEQ ID NO:2192 |
| KHK_miR_1743 | SEQ ID NO:743 | SEQ ID NO:2193 |
| KHK_miR_1744 | SEQ ID NO:744 | SEQ ID NO:2194 |

**[Table 1-23]**

| nucleic acid name | micro-RNA | micro-RNA precursor |
|---|---|---|
| KHK_miR_1745 | SEQ ID NO:745 | SEQ ID NO:2195 |
| KHK_miR_1746 | SEQ ID NO:746 | SEQ ID NO:2196 |
| | | SEQ ID NO:2197 |
| KHK_miR_1747 | SEQ ID NO:747 | SEQ ID NO:2198 |
| KHK_miR_1748 | SEQ ID NO.748 | SEQ ID NO:2199 |
| KHK_miR_1749 | SEQ ID NO:749 | SEQ ID NO:2200 |
| KHK_miR_1750 | SEQ ID NO:750 | SEQ ID NO:2201 |
| KHK_miR_1751 | SEQ ID NO:751 | SEQ ID NO:2202 |
| KHK_miR_1752 | SEQ ID NO:752 | SEQ ID NO:2203 |
| KHK_miR_1753 | SEQ ID NO:753 | SEQ ID NO:2204 |
| KHK_miR_1754 | SEQ ID NO:754 | SEQ ID NO:2205 |
| KHK_miR_1755 | SEQ ID NO:755 | SEQ ID NO:2206 |
| KHK_miR_1756 | SEQ ID NO:756 | SEQ ID NO:2207 |
| KHK_miR_1757 | SEQ ID NO:757 | SEQ ID NO:2208 |
| KHK_miR_1758 | SEQ ID NO:758 | SEQ ID NO:2209 |
| KHK_miR_1759 | SEQ ID NO:759 | SEQ ID NO:2210 |
| KHK_miR_1760 | SEQ ID NO:760 | SEQ ID NO:2211 |
| KHK_miR_1761 | SEQ ID NO:761 | SEQ ID NO:2212 |
| KHK_miR_1762 | SEQ ID NO:762 | SEQ ID NO:2213 |
| KHK_miR_1763 | SEQ ID NO:763 | SEQ ID NO:2214 |
| KHK_miR_1764 | SEQ ID NO:764 | SEQ ID NO:2215 |
| KHK_miR_1765 | SEQ ID NO:765 | SEQ ID NO:2216 |
| KHK_miR_1766 | SEQ ID NO:766 | SEQ ID NO:2217 |
| KHK_miR_1767 | SEQ ID NO:767 | SEQ ID NO:2218 |
| KHK_miR_1768 | SEQ ID NO:768 | SEQ ID NO:2219 |
| | | SEQ ID NO:2220 |
| | | SEQ ID NO:2221 |
| | | SEQ ID NO:2222 |
| KHK_miR_1769 | SEQ ID NO:769 | SEQ ID NO:2223 |
| KHk_miR_1770 | SEQ ID NO:770 | SEQ ID NO:2224 |
| KHK_miR_1771 | SEQ ID NO:771 | SEQ ID NO:2225 |
| KHK_miR_1772 | SEQ ID NO:772 | SEQ ID NO:2226 |
| KHK_miR_1773 | SEQ ID NO:773 | SEQ ID NO:2227 |
| KHK_miR_1774 | SEQ ID NO:774 | SEQ ID NO:2228 |
| KHK_miR_1775 | SEQ ID NO:775 | SEQ ID NO:2229 |
| | | SEQ ID NO:2230 |
| | | SEQ ID NO:2231 |
| KHK_miR_1776 | SEQ ID NO:776 | SEQ ID NO:2232 |
| KHK_miR_1777 | SEQ ID NO:777 | SEQ ID NO:2233 |

**[Table 1-24]**

| nucleic acid name | micro-RNA | micro-RNA precursor |
|---|---|---|
| KHK_miR_1778 | SEQ ID NO:778 | SEQ ID NO:2234 |
| KHK_miR_1779 | SEQ ID NO:779 | SEQ ID NO:2235 |
| KHK_miR_1780 | SEQ ID NO:780 | SEQ ID NO:2236 |
| KHK_miR_1781 | SRQ ID NO:781 | SEQ ID NO:2237 |
| KHK_miR_1782 | SEQ ID NO:782 | SEQ ID NO:2238 |
| KHK_miR_1783 | SEQ ID NO:783 | SEQ ID NO:2239 |
| KHK_miR_1784 | SEQ ID NO:784 | SEQ ID NO.2240 |
| | | SEQ ID NO:2241 |
| | | SEQ ID NO:2242 |
| | | SEQ ID NO:2243 |
| | | SEQ ID NO:2244 |
| | | SEQ ID NO:2245 |
| | | SEQ ID NO:2246 |
| | | SEQ ID NO:2247 |
| KHK_miR_1785 | SEQ ID NO:785 | SEQ ID NO:2248 |
| KHK_miR_1786 | SEQ ID NO:786 | SEQ ID NO:2249 |
| KHK_miR_1787 | SEQ ID NO:787 | SEQ ID NO:2250 |
| KHK_miR_1788 | SEQ ID NO:788 | SEQ ID NO:2251 |
| KHK_miR_1789 | SEQ ID NO:789 | SEQ ID NO:2252 |
| KHK_miR_1790 | SEQ ID NO:790 | SEQ ID NO:2253 |
| KHK_miR_1791 | SEQ ID NO:791 | SEQ ID NO:2254 |
| KHK_miR_1792 | SEQ ID NO:792 | SEQ ID NO:2255 |
| KHK_miR_1793 | SEQ ID NO:793 | SEQ ID NO:2256 |
| KHK_miR_1794 | SEQ ID NO:794 | SEQ ID NO:2257 |
| KHK_miR_1795 | SEQ ID NO:795 | SEQ ID NO:2258 |
| KHK_miR_1796 | SEQ ID NO:796 | SEQ ID NO:2259 |
| KHK_miR_1797 | SEQ ID NO:797 | SEQ ID NO:2260 |
| KHK_miR_1798 | SEQ ID NO:798 | SEQ ID NO:2261 |
| KHK_miR-1799 | SEQ ID NO:799 | SEQ ID NO:2262 |
| KHK_miR_1800 | SEQ ID NO:800 | SEQ ID NO:2263 |
| KHK_miR_1801 | SEQ ID NO:801 | SEQ ID NO:2264 |
| KHK_miR_1802 | SEQ ID NO:802 | SEQ ID NO:2265 |
| | | SEQ ID NO:2266 |
| KHK_miR_1803 | SEQ ID NO:803 | SEQ ID NO:2267 |
| KHK_miR_1804 | SEQ ID NO:804 | SEQ ID NO:2268 |
| KHK_miR_1805 | SEQ ID NO:805 | SEQ ID NO:2269 |
| KHK_miR_1808 | SEQ ID NO:806 | SEQ ID NO:2270 |
| KHK_miR_1807 | SEQ ID NO:807 | SEQ ID NO:2271 |
| KHK_miR_1808 | SEQ ID NO:808 | SEQ ID NO:2272 |

**[Table 1-25]**

| nucleic acid name | micro-RNA | micro-RNA precursor |
|---|---|---|
| KHK_miR_1809 | SEQ ID NO:809 | SEQ ID NO:2273 |
| KHK_miR_1810 | SEQ ID NO:810 | SEQ ID NO:2274 |
| KHK_miR_1811 | SEQ ID NO:811 | SEQ ID NO:2275 |
| KHK_miR_1812 | SEQ ID NO:812 | SEQ ID NO:2276 |
| KHK_miR_1813 | SEQ ID NO:813 | SEQ ID NO:2277 |
| KHK_miR_1814 | SEQ ID NO:814 | SEQ ID NO:2278 |
| KHK_miR_1815 | SEQ ID NO:815 | SEQ ID NO:2279 |
| KHK_miR_1818 | SEQ ID NO:816 | SEQ ID NO:2280 |
| KHK_miR_1817 | SEQ ID NO:817 | SEQ ID NO:2281 |
| KHK_miR_1818 | SEQ ID NO:818 | SEQ ID NO:2282 |
| KHK_miR_1819 | SEQ ID NO:819 | SEQ ID NO:2283 |
| KHK_miR_1820 | SEQ ID NO:820 | SEQ ID NO:2284 |
| | | SEQ ID NO:2285 |
| | | SEQ ID NO:2286 |
| | | SEQ ID NO:2287 |
| KHK_miR_1821 | SEQ ID NO:821 | SEQ ID NO:2288 |
| KHK_miR_1822 | SEQ ID NO:822 | SEQ ID NO:2289 |
| KHK_miR_1823 | SEQ ID NO:823 | SEQ ID NO:2290 |
| | | SEQ ID NO:2291 |
| | | SEQ ID NO:2292 |
| | | SEQ ID NO:2293 |
| | | SEQ ID NO:2294 |
| KHK_miR_1824 | SEQ ID NO:824 | SEQ ID NO:2295 |
| KHK_miR_1825 | SEQ ID NO:825 | SEQ ID NO:2296 |
| KHK_miR_1826 | SEQ ID NO:826 | SEQ ID NO:2297 |
| KHK_miR_1827 | SEQ ID NO:827 | SEQ ID NO:2298 |
| KHK_miR_1828 | SEQ ID RO:828 | SEQ ID NO:2299 |
| KHK_miR_1829 | SEQ ID NO:829 | SEQ ID NO:2300 |
| KHK_miR_1830 | SEQ ID NO:830 | SEQ ID NO:2301 |
| | | SEQ ID NO:2302 |
| KHK_miR_1831 | SEQ ID NO:831 | SEQ ID NO:2303 |
| KHK_miR_1832 | SEQ ID NO:832 | SEQ ID NO:2304 |
| KHK_miR_1833 | SEQ ID NO:833 | SEQ ID NO:2305 |
| KHK_miR_1834 | SEQ ID NO:834 | SEQ ID NO:2306 |
| KHK_miR_1835 | SEQ ID NO:835 | SEQ ID NO:2307 |
| KHK_miR_1836 | SEQ ID NO.836 | SEQ ID NO:2308 |
| KHK_miR_1837 | SEQ ID NO:837 | SEQ ID NO:2309 |
| KHK_miR_1838 | SEQ ID No:838 | SEQ ID NO:2310 |
| KHK_miR_1839 | SEQ ID NO:839 | SEQ ID NO:2311 |

**[Table 1-26]**

| nucleic acid name | micro-RNA | micro-RNA precursor |
|---|---|---|
| KHK_miR_1840 | SEQ ID NO:840 | SEQ ID NO:2312 |
| KHK_miR_1841 | SEQ ID NO:841 | SEQ ID NO:2313 |
| KHK_miR_1842 | SEQ ID NO:842 | SEQ ID NO:2314 |
| KHK_miR_1843 | SEQ ID NO:843 | SEQ ID NO:2315 |
| KHK_miR_1844 | SEQ ID NO:844 | SEQ ID NO:2316 |
| KHK_miR_1845 | SEQ ID NO:845 | SEQ ID NO:2317 |
| KHK_miR_1846 | SEQ ID NO:846 | SEQ ID NO:2318 |
| KHK_miR_1847 | SEQ ID NO:847 | SEQ ID NO:2319 |
| KHK_miR_1848 | SEQ ID NO:848 | SEQ ID NO:2320 |
| KHK_miR_1849 | SEQ ID NO:849 | SEQ ID NO:2321 |
| KHK_miR_1850 | SEQ ID NO:850 | SEQ ID NO:2322 |
| KHK_miR_1851 | SEQ ID NO:851 | SEQ ID NO:2323 |
| KHK_miR_1852 | SEQ ID NO:852 | SEQ ID NO:2324 |
| | | SEQ ID NO:2325 |
| KHK_miR_1853 | SEQ ID NO:853 | SEQ ID NO:2326 |
| KHK_miR_1854 | SEQ ID NO:854 | SEQ ID NO:2327 |
| KHK_miR_1855 | SEQ ID NO:855 | SEQ ID NO:2328 |
| KHK_miR_1856 | SEQ ID NO:856 | SEQ ID NO:2329 |
| KHK_miR_1857 | SEQ ID NO:857 | SEQ ID NO:2330 |
| KHK_miR_1858 | SEQ ID NO:858 | SEQ ID NO:2331 |
| KHK_mik_1859 | SEQ ID NO:859 | SEQ ID NO:2332 |
| KHK_miR_1860 | SEQ ID NO:860 | SEQ ID NO:2333 |
| KHK_miR_1861 | SEQ ID NO:861 | SEQ ID NO:2334 |
| KHK_miR_1862 | SEQ ID NO:862 | SEQ ID NO:2335 |
| KHK_miR_1863 | SEQ ID NO:863 | SEQ ID NO:2336 |
| KHK_miR_1864 | SEQ ID NO:864 | SEQ ID NO:2337 |
| KHK_miR_1865 | SEQ ID NO:865 | SEQ ID NO:2338 |
| KHK_miR_1866 | SEQ ID NO:866 | SEQ ID NO:2339 |
| KHK_miR_1867 | SEQ ID NO:867 | SEQ ID NO:2340 |
| KHK_miR_1868 | SEQ ID NO:868 | SEQ ID NO:2341 |
| KHK_miR_1869 | SEQ ID NO:869 | SEQ ID NO:2342 |
| KHK_miR_1870 | SEQ ID NO:870 | SEQ ID NO:2343 |
| | | SEQ ID NO:2344 |
| KHK_miR_1871 | SEQ ID NO:871 | SEQ ID NO:2345 |
| KHK_miR_1872 | SEQ ID NO:872 | SEQ ID NO:2346 |
| KHK_miR_1873 | SEQ ID NO:873 | SEQ ID NO:2347 |
| KHK_miR_1874 | SEQ ID NO:874 | SEQ ID NO:2348 |
| KHK_miR_1875 | SEQ ID NO:875 | SEQ ID NO:2349 |
| KHK_miR_1876 | SEQ ID NO:876 | SEQ ID NO:2350 |

**[Table 1-27]**

| nucleic acid name | micro-RNA | micro-RBA precursor |
|---|---|---|
| KHK_miR_1877 | SEQ ID NO:877 | SEQ ID NO:2351 |
| KHK_miR_1878 | SEQ ID NO:878 | SEQ ID NO:2352 |
| KHK_miR_1879 | SEQ ID NO:879 | SEQ ID NO:2353 |
| | | SEQ ID NO:2354 |
| KHK_miR_1880 | SEQ ID NO:880 | SEQ ID NO:2355 |
| KHK_miR_1881 | SEQ ID NO:881 | SEQ ID NO:2356 |
| KHK_miR_1882 | SEQ ID NO:882 | SEQ ID NO:2357 |
| KHK_miR_1883 | SEQ ID NO:883 | SEQ ID NO:2358 |
| KHK_miR_1884 | SEQ ID NO:884 | SEQ ID NO:2359 |
| KHK_miR_1885 | SEQ ID NO:885 | SEQ ID NO:2360 |
| KHK_miR_1886 | SEQ ID NO:886 | SEQ ID NO:2361 |
| KHK_miR_1887 | SEQ ID NO:887 | SEQ ID NO:2362 |
| KHK_miR_1888 | SEQ ID NO:888 | SEQ ID NO:2363 |
| KHK_miR_1889 | SEQ ID NO:889 | SEQ ID NO:2364 |
| KHK_miR_1890 | SEQ ID NO:890 | SEQ ID NO:2365 |
| KHK_miR_1891 | SEQ ID NO:891 | SEQ ID NO:2366 |
| KHK_miR_1892 | SEQ ID NO:892 | SEQ ID NO:2367 |
| KHK_miR_1893 | SEQ ID NO:893 | SEQ ID NO:2368 |
| KHK_miR_1894 | SEQ ID NO:894 | SEQ ID NO:2369 |
| | | SEQ ID NO:2370 |
| | | SEQ ID NO:2371 |
| KHK_miR_1895 | SEQ ID NO:895 | SEQ ID NO:2372 |
| KHK_miR_1896 | SEQ ID NO:896 | SEQ ID NO:2373 |
| KHK_miR_1897 | SEQ ID NO:897 | SEQ ID NO:2374 |
| KHK_miR_1898 | SEQ ID NO:898 | SEQ ID NO:2375 |
| | | SEQ ID NO:2376 |
| | | SEQ ID NO:2377 |
| KHK_miR_1899 | SEQ ID NO:899 | SEQ ID NO:2378 |
| KHK_miR_1900 | SEQ ID NO:900 | SEQ ID NO:2379 |
| KHK_miR_1901 | SEQ ID NO:901 | SEQ ID NO:2380 |
| KHK_miR_1902 | SEQ ID NO:902 | SEQ ID NO:2381 |
| | | SEQ ID NO:2382 |
| KHK_miR_1903 | SEQ ID NO:903 | SEQ ID NO:2383 |
| | | SEQ ID NO:2384 |
| KHK_miR_1904 | SEQ ID NO:904 | SEQ ID NO:2385 |
| KHK_miR_1905 | SEQ ID NO:905 | SEQ ID NO:2386 |
| KHK_miR_1906 | SEQ ID NO:906 | SEQ ID NO:2387 |
| KHK_miR_1907 | SEQ ID NO:907 | SEQ ID NO:2388 |
| | | SEQ ID NO:2389 |

**[Table 1-28]**

| nucleic acid name | micro-RNA | micro-RNA precursor |
|---|---|---|
| KHK_miR_1908 | SEQ ID NO:908 | SEQ ID NO:2390 |
| KHK_miR_1909 | SEQ ID NO:909 | SEQ ID NO:2391 |
| KHK_miR_1910 | SEQ ID NO:910 | SEQ ID NO:2392 |
| KHK_miR_1911 | SEQ ID NO:911 | SEQ ID NO:2393 |
| KHK_miR_1912 | SEQ ID NO:912 | SEQ ID NO:2394 |
| KHK_miR_1913 | SEQ ID NO:913 | SEQ ID NO:2395 |
| KHK_miR_1914 | SEQ ID NO:914 | SEQ ID NO:2396 |
| KHK_miR_1915 | SEQ ID NO:915 | SEQ ID NO:2397 |
| KHK_miR_1916 | SEQ ID NO:916 | SEQ ID NO:2398 |
| KHK_miR_1917 | SEQ ID NO:917 | SEQ ID NO:2399 |
| KHK_miR_1918 | SEQ ID NO:918 | SEQ ID NO:2400 |
| KHK_miR_1919 | SEQ ID NO:919 | SEQ ID NO:2401 |
| KHK_miR_1920 | SEQ ID NO:920 | SEQ ID NO:2402 |
| | | SEQ ID NO:2403 |
| | | SEQ ID NO:2404 |
| | | SEQ ID NO:2405 |
| | | SEQ ID NO:2406 |
| | | SEQ ID NO:2407 |
| KHK_miR_1921 | SEQ ID NO:921 | SEQ ID NO:2408 |
| | | SEQ ID NO:2409 |
| | | SEQ ID NO:2410 |
| KHK_miR_1922 | SEQ ID NO:922 | SEQ ID NO:2411 |
| KHK_miR_1923 | SEQ ID NO:923 | SEQ ID NO:2412 |
| | | SEQ ID NO:2413 |
| KHK_miR_1924 | SEQ ID NO:924 | SEQ ID NO:2414 |
| KHK_miR_1925 | SEQ ID NO:925 | SEQ ID NO:2415 |
| | | SEQ ID NO:2416 |
| | | SEQ ID NO:2417 |
| KHK_miR_1926 | SEQ ID NO:926 | SEQ ID NO:2418 |
| KHK_miR_1927 | SEQ ID NO:927 | SEQ ID NO:2419 |
| KHK_miR_1928 | SEQ ID NO:928 | SEQ ID NO:2420 |
| KHK_miR_1929 | SEQ ID NO:929 | SEQ ID NO:2421 |
| | | SEQ ID NO:2422 |
| KHK_miR_1930 | SEQ ID NO:930 | SEQ ID NO:2423 |
| KHK_miR_1931 | SEQ ID NO:931 | SEQ ID NO:2424 |
| | | SEQ ID NO:2425 |
| | | SEQ ID NO:2426 |
| | | SEQ ID NO:2427 |
| KHK_miR_1932 | SEQ ID NO:932 | SEQ ID NO:2428 |

**[Table 1-29]**

| nucleic acid name | micro-RNA | micro-RNA precursor |
|---|---|---|
| | | SEQ ID NO:2429 |
| | | SEQ ID NO:2430 |
| KHK_miR_1933 | SEQ ID NO:933 | SEQ ID NO:2431 |
| KHK_miR_1934 | SEQ ID NO:934 | SEQ ID NO:2432 |
| KHK_miR_1935 | SEQ ID NO:935 | SEQ ID NO:2433 |
| | | SEQ ID NO:2434 |
| KHK_miR_1936 | SEQ ID NO:936 | SEQ ID NO:2435 |
| KHK_miR_1937 | SEQ ID NO:937 | SEQ ID NO:2436 |
| | | SEQ ID NO:2437 |
| KHK_miR_1938 | SEQ ID NO:938 | SEQ ID NO:2438 |
| KHK_miR_1939 | SEQ ID NO:939 | SEQ ID NO:2439 |
| KHK_miR_1940 | SEQ ID NO:940 | SEQ ID NO:2440 |
| KHK_miR_1941 | SEQ ID NO:941 | SEQ ID NO:2441 |
| | | SEQ ID NO:2442 |
| | | SEQ ID NO:2443 |
| KHK_miR_1942 | SEQ ID NO:942 | SEQ ID NO:2444 |
| | | SEQ ID NO:2445 |
| KHK_miR_1943 | SEQ ID NO:943 | SEQ ID NO:2446 |
| KHK_miR_1944 | SEQ ID NO:944 | SEQ ID NO:2447 |
| KHK_miR_1945 | SEQ ID NO:945 | SEQ ID NO:2448 |
| KHK_miR_1946 | SEQ ID NO:946 | SEQ ID NO:2449 |
| KHK_miR_1947 | SEQ ID NO:947 | SEQ ID NO:2450 |
| KHK_miR_1948 | SEQ ID NO:948 | SEQ ID NO:2451 |
| | | SEQ ID NO:2452 |
| KHK_miR_1949 | SEQ ID NO:949 | SEQ ID NO:2453 |
| | | SEQ ID NO:2454 |
| KHK_miR_1950 | SEQ ID NO:950 | SEQ ID NO:2455 |
| KHK_miR_1951 | SEQ ID NO:951 | SEQ ID NO:2456 |
| KHK_miR_1952 | SEQ ID NO:952 | SEQ ID NO:2457 |
| KHK_miR_1953 | SEQ ID NO:953 | SEQ ID NO:2458 |
| | | SEQ ID NO:2459 |
| | | SEQ ID NO:2460 |
| KHK_miR_1954 | SEQ ID NO:954 | SEQ ID NO:2461 |
| | | SEQ ID NO:2462 |
| KHK_miR_1955 | SEQ ID NO:955 | SEQ ID NO:2463 |
| KHK_miR_1956 | SEQ ID NO:956 | SEQ ID NO:2464 |
| KHK_miR_1957 | SEQ ID NO:957 | SEQ ID NO:2465 |
| KHK_miR_1958 | SEQ ID NO:958 | SEQ ID NO:2466 |
| KHK_miR_1959 | SEQ ID NO:959 | SEQ ID NO:2467 |

**[Table 1-30]**

| nucleic acid name | micro-RNA | micro-RNA precursor |
|---|---|---|
| KHK_miR_1960 | SEQ ID NO:960 | SEQ ID NO:2468 |
| KHK_miR_1961 | SEQ ID NO:961 | SEQ ID NO:2469 |
| KHK_miR_1962 | SEQ ID NO:962 | SEQ ID NO:2470 |
| KHK_miR_1963 | SEQ ID NO:963 | SEQ ID NO:2471 |
| | | SEQ ID NO:2472 |
| | | SEQ ID NO:2473 |
| KHK_miR_1964 | SEQ ID NO:964 | SEQ ID NO:2474 |
| KHK_miR_1965 | SEQ ID NO:965 | SEQ ID NO:2475 |
| KHK_miR_1966 | SEQ ID NO:966 | SEQ ID NO:2476 |
| KHK_miR_1967 | SEQ ID NO:967 | SEQ ID NO:2477 |
| | | SEQ ID NO:2478 |
| KHK_miR_1968 | SEQ ID NO:968 | SEQ ID NO:2479 |
| KHK_miR_1969 | SEQ ID NO:969 | SEQ ID NO:2480 |
| KHK_miR_1970 | SEQ ID NO:970 | SEQ ID NO:2481 |
| KHK_miR_1971 | SEQ ID NO:971 | SEQ ID NO:2482 |
| KHK_miR_1972 | SEQ ID NO:972 | SEQ ID NO:2483 |
| KHK_miR_1973 | SEQ ID NO:973 | SEQ ID NO:2484 |
| KHK_miR_1974 | SEQ ID NO:974 | SEQ ID NO:2485 |
| KHK_miR_1975 | SEQ ID NO:975 | SEQ ID NO:2486 |
| KHK_miR_1976 | SEQ ID NO:976 | SEQ ID NO:2487 |
| KHK_miR_1977 | SEQ ID NO:977 | SEQ ID NO:2488 |
| KHK_miR_1978 | SEQ ID NO:978 | SEQ ID NO:2489 |
| KHK_miR_1979 | SEQ ID NO:979 | SEQ ID NO:2490 |
| KHK_miR_1980 | SEQ ID NO:980 | SEQ ID NO:2491 |
| KHK_miR_1981 | SEQ ID NO:981 | SEQ ID NO:2492 |
| KHK_miR_1982 | SEQ ID NO:982 | SEQ ID NO:2493 |
| KHK_miR_1983 | SEQ ID NO:983 | SEQ ID NO:2494 |
| KHK_miR_1984 | SEQ ID NO:984 | SEQ ID NO:2495 |
| KHK_miR_1985 | SEQ ID NO:985 | SEQ ID NO:2496 |
| KHK_miR_1986 | SEQ ID NO:986 | SEQ ID NO:2497 |
| KHK_miR_1987 | SEQ ID NO:987 | SEQ ID NO:2498 |
| KHK_miR_1988 | SEQ ID NO:988 | SEQ ID NO:2499 |
| KHK_miR_1989 | SEQ ID NO:989 | SEQ ID NO:2500 |
| KHK_miR_1990 | SEQ ID NO:990 | SEQ ID NO:2501 |
| KHK_miR_1991 | SEQ ID NO:991 | SEQ ID NO:2502 |
| KHK_miR_1992 | SEQ ID NO:992 | SEQ ID NO:2503 |
| KHK_miR_1993 | SEQ ID NO:993 | SEQ ID NO:2504 |
| KHK_miR_1994 | SEQ ID NO:994 | SEQ ID NO:2505 |
| KHK_miR_1995 | SEQ ID NO:995 | SEQ ID NO:2506 |

**[Table 1-31]**

| nucleic acid name | micro-RNA | micro-RNA precursor |
|---|---|---|
| KHK_miR_1996 | SEQ ID NO:996 | SEQ ID NO:2507 |
| KHK_miR_1997 | SEQ ID NO:997 | SEQ ID NO:2508 |
| KHK_miR_1998 | SEQ ID NO:998 | SEQ ID NO:2509 |
| KHK_miR_1999 | SEQ ID NO:999 | SEQ ID NO:2510 |
| KHK_miR_2000 | SEQ ID NO:1000 | SEQ ID NO:2511 |
| KHK_miR_2001 | SEQ ID NO:1001 | SEQ ID NO:2512 |
| | | SEQ ID NO:2513 |
| KHK_miR_2002 | SEQ ID NO:1002 | SEQ ID NO:2514 |
| KHK_miR_2003 | SEQ ID NO:1003 | SEQ ID NO:2515 |
| KHK_miR_2004 | SEQ ID NO:1004 | SEQ ID NO:2516 |
| KHK_miR_2005 | SEQ ID NO:1005 | SEQ ID NO:2517 |
| KHK_miR_2006 | SEQ ID NO:1006 | SEQ ID NO:2518 |
| KHK_miR_2007 | SEQ ID NO:1007 | SEQ ID NO:2519 |
| KHK_miR_2008 | SEQ ID NO:1008 | SEQ ID NO:2520 |
| KHK_miR_2009 | SEQ ID NO:1009 | SEQ ID NO:2521 |
| KHK_miR_2010 | SEQ ID NO:1010 | SEQ ID NO:2522 |
| KHK_miR_2011 | SEQ ID NO:1011 | SEQ ID NO:2523 |
| KHK_miR_2012 | SEQ ID NO:1012 | SEQ ID NO:2524 |
| KHK_miR_2013 | SEQ ID NO:1013 | SEQ ID NO:2525 |
| KHK_miR_2014 | SEQ ID NO:1014 | SEQ ID NO:2526 |
| KHK_miR_2015 | SEQ ID NO:1015 | SEQ ID NO:2527 |
| KHK_miR_2016 | SEQ ID NO:1016 | SEQ ID NO:2528 |
| KHK_miR_2017 | SEQ ID NO:1017 | SEQ ID NO:2529 |
| | | SEQ ID NO:2530 |
| | | SEQ ID NO:2531 |
| KHK_miR_2018 | SEQ ID NO:1018 | SEQ ID NO:2532 |
| KHK_miR_2019 | SEQ ID NO:1019 | SEQ ID NO:2533 |
| KHK_miR_2020 | SEQ ID NO:1020 | SEQ ID NO:2534 |
| KHK_miR_2021 | SEQ ID NO:1021 | SEQ ID NO:2535 |
| KHK_miR_2022 | SEQ ID NO:1022 | SEQ ID NO:2536 |
| KHK_miR_2023 | SEQ ID NO:1023 | SEQ ID NO:2537 |
| KHK_miR_2024 | SEQ ID NO:1024 | SEQ ID NO:2538 |
| KHK_miR_2025 | SEQ ID NO:1025 | SEQ ID NO:2539 |
| | | SEQ ID NO:2540 |
| | | SEQ ID NO:2541 |
| | | SEQ ID NO:2542 |
| KHK_miR_2026 | SEQ ID NO:1026 | SEQ ID NO:2543 |
| KHK_miR_2027 | SEQ ID NO:1027 | SEQ ID NO:2544 |
| KHK_miR_2028 | SEQ ID NO:1028 | SEQ ID NO:2545 |

**[Table 1-32]**

| nucleic acid name | micro-RNA | micro-RNA precursor |
|---|---|---|
| KHK_miR_2029 | SEQ ID NO:1029 | SEQ ID NO:2546 |
| KHK_miR_2030 | SEQ ID NO:1030 | SEQ ID NO:2547 |
| KHK_miR_2031 | SEQ ID NO:1031 | SEO ID NO:2548 |
| KHK_miR_2032 | SEQ ID NO:1032 | SEQ ID NO:2549 |
| KHK_miR_2033 | SEQ ID NO:1033 | SEQ ID NO:2550 |
| KHK_miR_2034 | SEQ ID NO:1034 | SEQ ID NO:2551 |
| KHK_miR_2035 | SEQ ID NO:1035 | SEQ ID NO:2552 |
| KHK_miR_2036 | SEQ ID NO:1036 | SEQ ID NO:2553 |
| KHK_miR_2037 | SEQ ID NO:1037 | SEQ ID NO:2554 |
| | | SEQ ID NO:2555 |
| | | SEQ ID NO:2556 |
| | | SEQ ID NO:2557 |
| KHK_miR_2038 | SEQ ID NO:1038 | SEQ ID NO:2558 |
| KHK_miR_2039 | SEQ ID NO:1039 | SEQ ID NO:2559 |
| KHK_miR_2040 | SEQ ID No:1040 | SEQ ID NO:2560 |
| KHK_miR_2041 | SEQ ID NO:1041 | SEQ ID NO:2561 |
| KHK_miR_2042 | SEQ ID NO:1042 | SEQ ID NO:2562 |
| KHK_miR_2043 | SEQ ID NO:1043 | SEQ ID NO:2563 |
| KHK_miR_2044 | SEQ ID NO:1044 | SEQ ID NO:2564 |
| KHK_miR_2045 | SEQ ID NO:1045 | SEQ ID NO:2565 |
| KHK_miR_2046 | SEQ ID NO:1046 | SEQ ID NO:2566 |
| KHK_miR_2047 | SEQ ID NO:1047 | SEQ ID NO:2567 |
| KHK_miR_2048 | SEQ ID NO:1048 | SEQ ID NO:2568 |
| KHK_miR_2049 | SEQ ID NO:1040 | SEQ ID NO:2569 |
| | | SEQ ID NO:2570 |
| KHK_miR_2050 | SEQ ID NO:1050 | SEQ ID NO:2571 |
| KHK_miR_2051 | SEQ ID NO:1051 | SEQ ID NO:2572 |
| KHK_miR_2052 | SEQ ID NO:1052 | SEQ ID NO:2573 |
| KHK_miR_2053 | SEQ ID NO:1053 | SEQ ID NO:2574 |
| KHK_miR_2054 | SEQ ID NO:1054 | SEQ ID NO:2575 |
| KHK_miR_2055 | SEQ ID NO:1055 | SEQ ID NO:2576 |
| KHK_miR_2056 | SEQ ID NO:1056 | SEQ ID NO:2577 |
| KHK_miR_2057 | SEQ ID NO:1057 | SEQ ID NO:2578 |
| KHK_miR_2058 | SEQ ID NO:1058 | SEQ ID NO:2579 |
| KHK_miR_2059 | SEQ ID NO:1059 | SEQ ID NO:2580 |
| KHK_miR_2060 | SEQ ID NO:1060 | SEQ ID NO:2581 |
| KHK_miR_2061 | SEQ ID NO:1061 | SEQ ID NO:2582 |
| KHK_miR_2062 | SEQ ID NO:1062 | SEQ ID NO:2583 |
| KHK_miR_2063 | SEQ ID NO:1063 | SEQ ID NO:2584 |

**[Table 1-33]**

| nucleic acid name | micro-RNA | micro-RNA precursor |
|---|---|---|
| KHK_miR_2064 | SEQ ID NO:1064 | SEQ ID NO:2585 |
| KHK_miR_2065 | SEQ ID NO:1065 | SEQ ID NO:2586 |
| KHK_miR_2066 | SEQ ID NO:1066 | SEQ ID NO:2587 |
| KHK_miR_2067 | SEQ ID NO:1067 | SEQ ID NO:2588 |
| KHK_miR_2068 | SEQ ID NO:1068 | SEQ ID NO:2589 |
| KHK_miR_2069 | SEQ ID NO:1069 | SEQ ID NO:2590 |
| KHK_miR_2070 | SEQ ID NO:1070 | SEQ ID NO:2591 |
| KNK_miR_2071 | SEQ ID NO:1071 | SEQ ID NO:2592 |
| KHK_miR_2072 | SEQ ID NO:1072 | SEQ ID NO:2593 |
| KHK_miR_2073 | SEQ ID NO:1073 | SEQ ID NO:2594 |
| | | SEQ ID NO:2595 |
| KHK_miR_2074 | SEQ ID NO:1074 | SEQ ID NO:2596 |
| KHK_miR_2075 | SEQ ID NO:1075 | SEQ ID NO:2597 |
| KHK_miR_2076 | SEQ ID NO:1076 | SEQ ID NO:2598 |
| KHK_miR_2077 | SEQ ID NO:1077 | SEQ ID NO:2599 |
| KHK_miR_2078 | SEQ ID NO:1078 | SEQ ID NO:2600 |
| KHK_miR_2079 | SEQ ID NO:1079 | SEQ ID NO:2601 |
| KHK_miR_2080 | SEQ ID NO:1080 | SEQ ID NO:2602 |
| KHK_miR_2081 | SEQ ID NO:1081 | SEQ ID NO:2603 |
| KHK_miR_2082 | SEQ ID NO:1082 | SEQ ID NO:2604 |
| KHK_miR_2083 | SEQ ID NO:1083 | SEQ ID NO:2605 |
| | | SEQ ID NO:2606 |
| KHK_miR_2084 | SEQ ID NO:1084 | SEQ ID NO:2607 |
| KHK_miR_2085 | SEQ ID NO:1085 | SEQ ID NO:2608 |
| KHK_miR_2086 | SEQ ID NO:1086 | SEQ ID NO:2609 |
| KHK_miR_2087 | SEQ ID NO:1087 | SEQ ID NO:2610 |
| KHK_miR_2088 | SEQ ID NO:1088 | SEQ ID NO:2611 |
| KHK_miR_2089 | SEQ ID NO:1089 | SEQ ID NO:2612 |
| KHK_miR_2090 | SEQ ID NO:1090 | SEQ ID NO:2613 |
| KHK_miR_2091 | SEQ ID NO:1091 | SEQ ID NO:2614 |
| KHK_miR_2092 | SEQ ID NO:1092 | SEQ ID NO:2615 |
| KHK_miR_2093 | SEQ ID NO:1093 | SEQ ID NO:2616 |
| KHK_miR_2094 | SEQ ID NO:1094 | SEQ ID NO:2617 |
| KHK_miR_2095 | SEQ ID NO:1095 | SEQ ID NO:2618 |
| KHK_miR_2096 | SEQ ID NO:1096 | SEQ ID NO:2619 |
| KHK_miR_2097 | SEQ ID NO:1097 | SEQ ID NO:2620 |
| | | SEQ ID NO:2621 |
| KHK_miR_2098 | SEQ ID NO:1098 | SEQ ID NO:2622 |
| | | SEQ ID NO:2623 |

**[Table 1-34]**

| nucleic acid name | micro-RNA | micro-RNA precursor |
|---|---|---|
| | | SEQ ID NO:2624 |
| KHK_miR_2099 | SEQ ID NO:1099 | SEQ ID NO:2625 |
| KHK_miR_2100 | SEQ ID NO:1100 | SEQ ID NO:2626 |
| | | SEQ ID NO:2627 |
| | | SEQ ID NO:2628 |
| | | SEQ ID NO:2629 |
| | | SEQ ID NO:2630 |
| KHK_miR_2101 | SEQ ID NO:1101 | SEQ ID NO:2631 |
| KHK_miR_2102 | SEQ ID NO:1102 | SEQ ID NO:2632 |
| KHK_miR_2103 | SEQ ID NO:1103 | SEQ ID NO:2633 |
| KHK_miR_2104 | SEQ ID NO:1104 | SEQ ID NO:2634 |
| KHK_miR_2105 | SEQ ID NO:1105 | SEQ ID NO:2635 |
| KHK_miR_2108 | SEQ ID NO:1106 | SEQ ID NO:2636 |
| KHK_miR_2107 | SEQ ID NO:1107 | SEQ ID NO:2637 |
| KHK_miR_2108 | SEQ ID NO:1108 | SEQ ID NO:2638 |
| KHK_miR_2109 | SEQ ID NO:1109 | SEQ ID NO:2639 |
| KHK_miR_2110 | SEQ ID NO:1110 | SEQ ID NO:2640 |
| KHK_miR_2111 | SEQ ID NO:1111 | SEQ ID NO:2641 |
| KHK_miR_2112 | SEQ ID NO:1112 | SEQ ID NO:2642 |
| KHK_miR_2113 | SEQ ID NO:1113 | SEQ ID NO:2643 |
| KHK_miR_2114 | SEQ ID NO:1114 | SEQ ID NO:2644 |
| KHK_miR_2115 | SEQ ID NO:1115 | SEQ ID NO:2645 |
| KHK_miR_2116 | SEQ ID NO:1116 | SEQ ID NO:2646 |
| KHK_miR_2117 | SEQ ID NO:1117 | SEQ ID NO:2647 |
| KHK_miR_2118 | SEQ ID NO:1118 | SEQ ID NO:2648 |
| KHK_miR_2119 | SEQ ID NO:1119 | SEQ ID NO:2649 |
| | | SEQ ID NO:2650 |
| KHK_miR_2120 | SEQ ID NO:1120 | SEQ ID NO:2651 |
| KHK_miR_2121 | SEQ ID NO:1121 | SEQ ID NO:2652 |
| KHK_miR_2122 | SEQ ID NO:1122 | SEQ ID NO:2653 |
| KHK_miR_2123 | SEQ ID NO:1123 | SEQ ID NO:2654 |
| KHK_miR_2124 | SEQ ID NO:1124 | SEQ ID NO:2655 |
| KHK_miR_2125 | SEQ ID NO:1125 | SEQ ID NO:2656 |
| KHK_miR_2126 | SEQ ID NO:1126 | SEQ ID NO:2657 |
| KHK_miR_2127 | SEQ ID NO:1127 | SEQ ID NO:2658 |
| KHK_miR_2128 | SEQ ID NO:1128 | SEQ ID NO:2659 |
| KHK_miR_2129 | SEQ ID NO:1129 | SEQ ID NO:2660 |
| KHK_miR_2130 | SEQ ID NO:1130 | SEQ ID NO:2661 |
| KHK_miR_2131 | SEQ ID NO:1131 | SEQ ID NO:2662 |

**[Table 1-35]**

| nucleic acid name | micro-RNA | micro-RNA precursor |
|---|---|---|
| KHK_miR_2132 | SEQ ID NO:1132 | SEQ ID NO:2663 |
| KHK_miR_2133 | SEQ ID NO:1133 | SEQ ID NO:2664 |
| KHK_miR_2134 | SEQ ID NO:1134 | SEQ ID NO:2665 |
| KHK_miR_2135 | SEQ ID NO:1135 | SEQ ID NO:2666 |
| KHK_miR_2136 | SEQ ID NO:1136 | SEQ ID NO:2667 |
| KHK_miR_2137 | SEQ ID NO:1137 | SEQ ID NO:2668 |
| KHK_miR_2138 | SEQ ID NO:1138 | SEQ ID NO:2669 |
| KHK_miR_2139 | SEQ ID NO:1139 | SEQ ID NO:2670 |
| KHK_miR_2140 | SEQ ID NO:1140 | SEQ ID NO:2671 |
| KHK_miR_2141 | SEQ ID NO:1141 | SEQ ID NO:2672 |
| KHK_miR_2142 | SEQ ID NO:1142 | SEQ ID NO:2673 |
| KHK_miR_2143 | SEQ ID NO:1143 | SEQ ID NO:2674 |
| KHK_miR_2144 | SEQ ID NO:1144 | SEQ ID NO:2675 |
| KHK_miR_2145 | SEQ ID NO:1145 | SEQ ID NO:2676 |
| KHK_miR_2146 | SEQ ID NO:1146 | SEQ ID NO:2677 |
| KHK_miR_2147 | SEQ ID NO:1147 | SEQ ID NO:2678 |
| KHK_miR_2148 | SEQ ID NO:1148 | SEQ ID NO:2679 |
| KHK_miR_2149 | SEQ ID NO:1149 | SEQ ID NO:2680 |
| KHK_miR_2150 | SEQ ID NO:1150 | SEQ ID NO:2601 |
| KHK_miR_2151 | SEQ ID NO:1151 | SEQ ID NO:2682 |
| KHK_miR_2152 | SEQ ID NO:1152 | SEQ ID NO:2683 |
| KHK_miR_2153 | SEQ ID NO:1153 | SEQ ID NO:2684 |
| KHK_miR_2154 | SEQ ID NO:1154 | SEQ ID NO:2685 |
| KHK_miR_2155 | SEQ ID NO:1155 | SEQ ID NO:2686 |
| KHK_miR_2158 | SEQ ID NO:1156 | SEQ ID NO:2687 |
| KHK_miR_2157 | SEQ ID NO:1157 | SEQ ID NO:2688 |
| KHK_miR_2158 | SEQ ID NO:1158 | SEQ ID NO:2689 |
| KHK_miR_2159 | SEQ ID NO:1159 | SEQ ID NO:2690 |
| KHK_miR_2160 | SEQ ID NO:1160 | SEQ ID NO:2691 |
| KHK_miR_2161 | SEQ ID NO:1161 | SEQ ID NO:2692 |
| KHK_miR_2162 | SEQ ID NO:1162 | SEQ ID NO:2693 |
| KHK_miR-2163 | SEQ ID NO:1163 | SEQ ID NO:2694 |
| KHK_miR_2164 | SEQ ID NO:1164 | SEQ ID NO:2695 |
| KHK_miR_2165 | SEQ ID NO:1165 | SEQ ID NO:2696 |
| KHK_miR_2166 | SEQ ID NO:1166 | SEQ ID NO:2697 |
| KHK_miR_2167 | SEQ ID NO:1167 | SEQ ID NO:2698 |
| KHK_miR_2168 | SEQ ID NO:1168 | SEQ ID NO:2699 |
| KHK_miR_2169 | SEQ ID NO:1169 | SEQ ID NO:2700 |
| | | SEQ ID NO:2701 |

**[Table 1-36]**

| nucleic acid name | micro-RNA | micro-RNA precursor |
|---|---|---|
| KHK_miR_2170 | SEQ ID NO:117 | SEQ ID NO:2702 |
| KHK_miR_2171 | SEQ ID NO:1171 | SEQ ID NO:2703 |
| KHK_miR_2172 | SEQ ID NO:1172 | SEQ ID NO:2704 |
| KHK_miR_2173 | SEQ ID NO:1173 | SEQ ID NO:2705 |
| | | SEQ ID NO:2706 |
| KHK_miR_2174 | SEQ ID NO:1174 | SEQ ID NO:2707 |
| KHK_miR_2175 | SEQ ID NO:1175 | SEQ ID NO:2708 |
| | | SEQ ID NO:2709 |
| | | SEQ ID NO:2710 |
| KHK_miR_2176 | SEQ ID NO:1176 | SEQ ID NO:2711 |
| KHK_miR_2177 | SEQ ID NO:1177 | SEQ ID NO:2712 |
| | | SEQ ID NO:2713 |
| | | SEQ ID NO:2714 |
| KHK_miR_2178 | SEQ ID NO:1178 | SEQ ID NO:2715 |
| KHK_miR_2179 | SEQ ID NO:1179 | SEQ ID NO:2716 |
| KHK_miR_2180 | SEQ ID NO:1180 | SEQ ID NO:2717 |
| KHK_miR_2181 | SEQ ID NO:1181 | SEQ ID NO:2718 |
| KHK_miR_2182 | SEQ ID NO:1182 | SEQ ID NO:2719 |
| KHK_miR_2183 | SEQ ID NO:1183 | SEQ ID NO:2720 |
| KHK_miR_2184 | SEQ ID NO:1184 | SEQ ID NO:2721 |
| KHK_miR_2185 | SEQ ID NO:1185 | SEQ ID NO:2722 |
| KHK_miR_2186 | SEQ ID NO:1186 | SEQ ID NO:2723 |
| KHK_miR_2187 | SEQ ID NO:1187 | SEQ ID NO:2724 |
| KHK_miR_2188 | SEQ ID NO:1188 | SEQ ID NO:2725 |
| KHK_miR_2189 | SEQ ID NO:1189 | SEQ ID NO:2726 |
| KHK_miR_2190 | SEQ ID NO:1190 | SEQ ID NO:2727 |
| KHK_miR_2191 | SEQ ID NO:1191 | SEQ ID NO:2728 |
| KHK_miR_2192 | SEQ ID NO:1192 | SEQ ID NO:2729 |
| KHK_miR_2193 | SEQ ID NO:1193 | SEQ ID NO:2730 |
| KHK_miR_2194 | SEQ ID NO:1194 | SEQ ID NO:2731 |
| KHK_miR_2195 | SEQ ID NO:1195 | SEQ ID NO:2732 |
| KHK_miR_2196 | SEQ ID NO:1196 | SEQ ID NO:2733 |
| KHK_miR_2197 | SEQ ID NO:1197 | SEQ ID NO:2734 |
| KHK_miR_2198 | SEQ ID NO:1198 | SEQ ID NO:2735 |
| KHK_miR_2199 | SEQ ID NO:1199 | SEQ ID NO:2736 |
| KHK_miR_2200 | SEQ ID NO:1200 | SEQ ID NO:2737 |
| KHK_miR_2201 | SEQ ID NO:1201 | SEQ ID NO:2738 |
| KHK_miR_2202 | SEQ ID NO:1202 | SEQ ID NO:2739 |
| KHK_miR_2203 | SEQ ID NO:1203 | SEQ ID NO:2740 |

**[Table 1-37]**

| | | |
|---|---|---|
| nucleic acid name | micro-RNA | micro-RNA precursor |
| | | SEQ ID NO:2741 |
| | | SEQ ID NO:2742 |
| KHK_miR_2204 | SEQ ID NO:1204 | SEQ ID NO:2743 |
| KHK_miR_2205 | SEQ ID NO:1205 | SEQ ID NO:2744 |
| KHK_miR_2206 | SEQ ID NO:1206 | SEQ ID NO:2745 |
| | | SEQ ID NO:2746 |
| KHK_miR_2207 | SEQ ID NO:1207 | SEQ ID NO:2747 |
| KHK_miR_2208 | SEQ ID NO:1208 | SEQ ID NO:2748 |
| KHK_miR_2209 | SEQ ID NO:1209 | SEQ ID NO:2749 |
| KHK_miR_2210 | SEQ ID NO:1210 | SEQ ID NO:2750 |
| KHK_miR_2211 | SEQ ID NO:1211 | SEQ ID NO:2751 |
| KHK_miR_2212 | SEQ ID NO:1212 | SEQ ID NO:2752 |
| KHK_miR_2213 | SEQ ID NO:1213 | SEQ ID NO:2753 |
| KHK_miR_2214 | SEQ ID NO:1219 | SEQ ID NO:2754 |
| KHK_miR_2215 | SEQ ID NO:1215 | SEQ ID NO:2755 |
| KHK_miR_2216 | SEQ ID NO:1216 | SEQ ID NO:2756 |
| KHK_miR_2217 | SEQ ID NO:1217 | SEQ ID NO:2757 |
| KHK_miR_2218 | SEQ ID NO:1218 | SEQ ID NO:2758 |
| KHK_miR_2219 | SEQ ID NO:1219 | SEQ ID NO:2759 |
| | | SEQ ID NO:2760 |
| KHK_miR_2220 | SEQ ID NO:1220 | SEQ ID NO:2761 |
| KHK_miR_2221 | SEQ ID NO:1221 | SEQ ID NO:2762 |
| KHK_miR_2222 | SEQ ID NO:1222 | SEQ ID NO:2763 |
| | | SEQ ID NO:2764 |
| KHK_miR_2223 | SEQ ID NO:1223 | SEQ ID NO:2765 |
| KHK_miR_2224 | SEQ ID NO:1224 | SEQ ID NO:2766 |
| KHK_miR_2225 | SEQ ID NO:1225 | SEQ ID NO:2767 |
| KHK_miR_2226 | SEQ ID NO:1226 | SEQ ID ND:2768 |
| KHK_miR_2227 | SEQ ID NO:1227 | SEQ ID NO:2769 |
| KHK_miR_2228 | SEQ ID NO:1228 | SEQ ID NO:2770 |
| KHK_miR_2229 | SEQ ID NO:1229 | SEQ ID NO:2771 |
| KHK_miR_2230 | SEQ ID NO:1230 | SEQ ID NO:2772 |
| KHK_miR_2231 | SEQ ID NO:1231 | SEQ ID NO:2773 |
| KHK_miR_2232 | SEQ ID NO:1232 | SEQ ID NO:2774 |
| | | SEQ ID NO:2775 |
| | | SEQ ID NO:2776 |
| KHK_miR_2233 | SEQ ID NO:1233 | SEQ ID NO:2777 |
| KHK_miR_2234 | SEQ ID No:1234 | SEQ ID NO:2778 |
| | | SEQ ID NO:2779 |

**[Table 1-38]**

| nucleic acid name | micro-RNA | micro-FNA precursor |
|---|---|---|
| | | SEQ ID NO:2780 |
| | | SEQ ID NO:2781 |
| | | SEQ ID NO:2782 |
| KHK_miR_2235 | SEQ ID NO:1235 | SEQ ID NO:2783 |
| KHK_miR_2236 | SEQ ID NO:1236 | SEQ ID NO:2784 |
| KHK_miR_2237 | SEQ ID NO:1237 | SEQ ID NO:2785 |
| KHK_miR_2238 | SEQ ID NO:1238 | SEQ ID NO:2786 |
| | | SEQ ID NO:2787 |
| | | SEQ ID NO:2788 |
| KHK_miR_2239 | SEQ ID NO:1239 | SEQ ID NO:2789 |
| KHK_miR_2240 | SEQ ID NO:1240 | SEQ ID ND:2790 |
| KHK_miR_2241 | SEQ ID NO:1241 | SEQ ID NO:2791 |
| KHK_miR_2242 | SEQ ID NO:1242 | SEQ ID NO:2792 |
| KHK_miR_2243 | SEQ ID NO:1243 | SEQ ID NO:2793 |
| KHK_miR_2244 | SEQ ID NO:1244 | SEQ ID NO:2794 |
| KHK_miR_2245 | SEQ ID NO:1245 | SEQ ID NO:2795 |
| KHK_miR_2246 | SEQ ID NO:1246 | SEQ ID NO:2796 |
| KHK_miR_2247 | SEQ ID NO:1247 | SEQ ID NO:2797 |
| KHK_miR_2248 | SEQ ID NO:1248 | SEQ ID NO:2798 |
| KHK_miR_2249 | SEQ ID NO:1249 | SEQ ID NO:2799 |
| KHK_miR_2250 | SEQ ID NO:1250 | SEQ ID NO:2800 |
| KHK_miR_2251 | SEQ ID NO:1251 | SEQ ID NO:2801 |
| KHK_miR_2252 | SEQ ID NO:1252 | SEQ ID NO:2802 |
| | | SEQ ID NO:2803 |
| | | SEQ ID NO:2804 |
| | | SEQ ID NO:2805 |
| KHK_miR_2253 | SEQ ID NO:2253 | SEQ ID NO:2806 |
| KHK_miR_2254 | SEQ ID NO:1254 | SEQ ID NO:2807 |
| KHK_miR_2255 | SEQ ID NO:1255 | SEQ ID NO:29013 |
| KHK_miR_2258 | SEQ ID NO:1256 | SEQ ID NO:2809 |
| KHK_miR_2257 | SEQ ID NO:1257 | SEQ ID NO:2810 |
| KHK_miR_2258 | SEQ ID NO:1258 | SEQ ID NO:2811 |
| KHK_miR_2259 | SEQ ID NO:1259 | SEQ ID NO:2812 |
| | | SEQ ID NO:2813 |
| KHK_miR_2260 | SEQ ID NO:1260 | SEQ ID NO:2814 |
| KHK_miR_2261 | SEQ ID NO:1261 | SEQ ID NO:2815 |
| KHK_miR_2262 | SEQ ID NO:1262 | SEQ ID NO:2816 |
| | | SEQ ID NO:2817 |
| | | SEQ ID NO:2818 |

**[Table 1-39]**

| nucleic acid name | micro-RNA | micro-RNA precursor |
|---|---|---|
| | | SEQ ID NO:2819 |
| | | SEQ ID NO:2820 |
| KHK_miR_2263 | SEQ ID NO:1263 | SEQ ID NO:2821 |
| KHK_miR_2264 | SEQ ID RO:1264 | SEQ ID NO:2822 |
| KHK_miR_2265 | SEQ ID NO:1265 | SEQ ID NO:2823 |
| KHK_miR_2267 | SEQ ID NO:1266 | SEQ ID NO:2824 |
| KHK_miR_2269 | SEQ ID NO:1267 | SEQ ID NO:2825 |
| KHK_miR_2271 | SEQ ID NO:1268 | SEQ ID NO:2826 |
| KHK_miR_2272 | SEQ ID NO:1269 | SEQ ID NO:2827 |
| KHK_miR_2273 | SEQ ID NO:1270 | SEQ ID NO:2828 |
| KHK_miR_2274 | SEQ ID NO:1271 | SEQ ID NO:2829 |
| KHK_miR_2278 | SEQ ID NO:1272 | SEQ ID NO:2830 |
| KHK_miR_2280 | SEQ ID NO:1273 | SEQ ID NO:2831 |
| KHK_miR_2281 | SEQ ID NO:1274 | SEQ ID NO:2832 |
| KHK_miR_2282 | SEQ ID NO:1275 | SEQ ID NO:2833 |
| KHK_miR_2283 | SEQ ID NO:1276 | SEQ ID NO:2834 |
| KHK_miR_2284 | SEQ ID NO:1277 | SEQ ID NO:2835 |
| KHK_miR_2285 | SEQ ID NO:1278 | SEQ ID NO:2836 |
| KHK_miR_2286 | SEQ ID NO:1279 | SEQ ID NO:2837 |
| KHK_miR_2287 | SEQ ID NO:1280 | SEQ ID NO:2838 |
| KHK_miR_2288 | SEQ ID NO:1281 | SEQ ID NO:2939 |
| KHK_miR_2289 | SEQ ID NO:1282 | SEQ ID NO:2840 |
| KHK_miR_2290 | SEQ ID NO:1283 | SEQ ID NO:2841 |
| KHK_miR_2291 | SEQ ID NO:1284 | SEQ ID NO:2842 |
| KHK_miR_2292 | SEQ ID NO:1285 | SEQ ID NO:2843 |
| KHK_miR_2293 | SEQ ID NO:1286 | SEQ ID NO:2844 |
| KHK_miR_2294 | SEQ ID NO:1287 | SEQ ID NO:2845 |
| KHK_miR_2295 | SEQ ID NO:1288 | SEQ ID NO:2846 |
| KHK_miR_2296 | SEQ ID NO:1289 | SEQ ID NO:2847 |
| KHK_miR_2297 | SEQ ID NO:1290 | SEQ ID NO:2848 |
| KHK_miR_2298 | SEQ ID NO:1291 | SEQ ID NO:2849 |
| KHK_miR_2299 | SEQ ID NO:1292 | SEQ ID NO:2850 |
| KHK_miR_2300 | SEQ ID NO:1293 | SEQ ID NO:2851 |
| KHK_miR_2301 | SEQ ID NO:1294 | pre-hsa-et-7a-1 |
| KHK_miR_2302 | SEQ ID NO:1295 | pre-hsa-let-7d |
| KHK_miR_2303 | SEQ ID NO: 1296 | pre-hsa-let-7f-2 |
| KHK_miR_2304 | SEQ ID NO:1297 | pre-hsa-mir-101-1 |
| KHK_miR_2305 | SEQ ID NO:1298 | pre-hsa-mir-106b |
| KHK_miR_2306 | SEQ ID NO:1299 | pre-hsa-mir-125a |

**[Table 1-40]**

| nucleic acid name | micro-RNA | micro-RNA precursor |
|---|---|---|
| KHK_mifR_2307 | SEQ ID NO:1300 | pre-hsa-mir-128a |
| KHK_miR_2308 | SEQ ID NO:1301 | pre-hsa-mir-130b |
| KHK_miR_2309 | SEQ ID NO:1302 | pre-hsa-mir-132 |
| KHK_miR_2310 | SEQ ID NO:1303 | pre-hsa-mir-141 |
| KHK-miR_2311 | SEQ ID NO:1304 | pre-hsa-mir-148a |
| KHK_MiR_2312 | SEQ ID NO:1305 | pre-hsa-mir-15a |
| KHK_miR_2313 | SEQ ID NO:1306 | pre-hsa-mir-16-1 |
| KHK_miR_2314 | SEQ ID NO:1307 | pre-hsa-mir-16-2 |
| KHK_miR_2315 | SEQ ID NO:1308 | pre-hsa-mim-196b |
| KHK_miR_2316 | SEQ ID NO:1309 | pre-hsa-mir-19b-1 |
| KHK_miR_2317 | SEQ ID NO:1310 | pre-hsa-mir-210 |
| KHK_miR_2318 | SEQ ID NO:1311 | pre-hsa-mir-22 |
| KHK_miR_2319 | SEQ ID NO:1312 | pre-hsa-mir-221 |
| KHK_miR_2320 | SEQ ID NO:1313 | pre-hsa-mir-223 |
| KHK_miR_2321 | SEQ ID NO:1314 | pre-hsa-mir-24-2 |
| KHK_miR_2322 | SEQ ID NO: 1315 | pre-hsa-mir-25 |
| KHK_miR_2323 | SEQ ID NO:1316 | pre-hsa-mir-26a-2 |
| KHK_miR_2324 | SEQ ID NO:1317 | pre-hsa-mir-26b |
| KHK_miR_2325 | SEQ ID NO:1318 | pre-hsa-mir-29a |
| KHK_miR_2326 | SEQ ID NO:1319 | pre-hsa-mir-29b-1 |
| KHK_miR_2327 | SEQ ID NO:1320 | pre-hsa-mir-30c-1 |
| KHK_miR_2328 | SEQ ID NO:1321 | pre-hsa-mir-30d |
| KHK_miR_2329 | SEQ ID NO:1322 | pre-hsa-mir-33 |
| KHK_miR_2330 | SEQ ID NO:1323 | pre-hsa-mir-339 |
| KHK_miR_2331 | SEQ ID NO:1324 | pre-hsa-mir-340 |
| KHK_miR_2332 | SEQ ID NO:1325 | pre-hsa--mir-342 |
| KHK_miR_2333 | SEQ ID No:1326 | pre-hsa-mir-34a |
| KHK_miR_334 | SEQ ID NO:1327 | pre-hsa-mir-361 |
| KHK_miR_2336 | SEQ ID NO:1328 | pre-hsa-mir-500 |
| KHK_miR_2337 | SEQ ID NO:1329 | pre-hsa--mir-93 |
| KHK_miR_2338 | SEQ ID NO:1330 | pre-hsa-mir-140 |
| KHK_miR_2339 | SEQ ID NO:1331 | pre-hsa-mir-151 |
| KHK_miR_2340 | SEQ ID NO:1332 | pre-hse-mir-181b-1 |
| KHK_miR_2341 | SEQ ID NO:1333 | pre-hsa-mir-29b-2 |
| KHK_miR_2342 | SEQ ID NO:1334 | pre-hsa-mir-29c |
| KHK_miR_2343 | SEQ ID NO:1335 | pre-hsa-mir-423 |
| KHK_miR_2344 | SEQ ID NO:1336 | pre-hsa-mir-7-1 |

As nucleic acids of the present invention, a nucleic acid consisting of a nucleotide sequence complementary to a nucleic acid mentioned above, and a double-stranded nucleic acid consisting of a nucleic acid mentioned above and a nucleic acid consisting of a nucleotide sequence complementary to the nucleotide sequence of the nucleic acid can also be mentioned.
In the present invention, a nucleic acid consisting of a nucleotide sequence having an identity of 90% or more to the nucleotide sequence of any one of SEQ ID NOs:1 to 1336 means a nucleic acid having an identity of at least 90% or more, preferably 91% or more, more preferably 92% or more, still more preferably 93% or more, particularly preferably 94% or more, and most preferably 95% or more, to a nucleic acid consisting of the nucleotide sequence of any one of SEQ ID NOs:1 to 1336, as calculated using an analytical software program such as BLAST [J. Mol. Biol., 215, 403 (1990)] or FASTA [Methods in Enzymology, 183, 63 (1990)]. In the present invention, a nucleic acid consisting of a nucleotide sequence having an identity of 80% or more to the nucleotide sequence of any one of SEQ ID NOs:1337 to 2851 means a nucleic acid having an identity of at least 80% or more, preferably 85% or more, more preferably 90% or more, still more preferably 92% or more, particularly preferably 95% or more, and most preferably 96% or more, to a nucleic acid consisting of the nucleotide sequence of any one of SEQ ID NOs:1337 to 2851, as calculated using an analytical software program such as BLAST [J. Mol. Biol., 215, 403 (1990)] or FASTA [Methods in Enzymology, 183, 63 (1990)].

In the present invention, a nucleic acid that hybridizes under stringent conditions includes, for example, a nucleic acid that can be identified by adding a probe RNA labeled γ-³² p-ATP to a Hybridization buffer consisting of 7.5 mL of 20xSSC, 0.6 mL of 1 M Na₂HPO₄ (pH 7.2), 21 mL of 10% SDS, 0.6 mL of 50xDenhardt's solution, and 0.3 mL of 10 mg/mL sonicated salmon sperm DNA, wherein the probe is a nucleic acid having the nucleotide sequence of any of SEQ ID NOs:1 to 2851 or a partial fragment thereof, carrying out a reaction at 50°C overnight, thereafter washing the membrane with 5xSSC/5% SDS liquid at 50°C for 10 minutes, and further washing the same with 1xSSc/1% SDS liquid at 50°C for 10 minutes, thereafter taking out the membrane, and applying it to an X-ray film.

In the present invention, the nucleic acid may be any molecule, as far as it is a molecule resulting from polymerization of a nucleotide or a molecule functionally equivalent to the nucleotide; for example, an RNA, which is a ribonucleotide polymer, a DNA, which is a deoxyribonucleotide polymer, a mixed polymer of RNA and DNA, and a nucleotide polymer, including a nucleotide analogue, can be mentioned; furthermore, the nucleic acid may be a nucleotide polymer, including a nucleic acid derivative, and may be a single-stranded nucleic acid or a double-stranded nucleic acid. A micro-RNA or a derivative thereof and a micro-RNA precursor or a derivative thereof are also included in nucleic acids of the present invention.

In the present invention, the nucleotide analogue may be any molecule, as far as it is a molecule prepared by modifying a ribonucleotide, a deoxyribonucleotide, an RNA or a DNA in order to improve the nuclease resistance thereof, to stabilize the same, to increase the affinity thereof for a complementary chain nucleic acid, to increase the cell permeability thereof, or to visualize the same, compared with the RNA or DNA; the analogue may be a naturally occurring molecule or a non-natural molecule; for example, a nucleotide analogue modified at the sugar moiety thereof, a nucleotide analogue modified by phosphodiester binding and the like can be mentioned.

The nucleotide analogue modified at the sugar moiety thereof may be any one, as far as an optionally chosen chemical structural substance has been added to, or substituted for, a portion or all of the chemical structure of the sugar of the nucleotide; for example, a nucleotide analogue substituted by 2'-O-methylribose, a nucleotide analogue substituted by 2'-O-propylribose, a nucleotide analogue substituted by 2'-methoxyethoxyribose, a nucleotide analogue substituted by 2'-O-methoxyethylribose, a nucleotide analogue substituted by 2'-O-[2-(guanidium)ethyl]ribose, a nucleotide analogue substituted by 2'-O-fluororibose, a bridged nucleic acid (BNA) having two cyclic structures as a result of introduction of a bridging structure into the sugar moiety, more specifically a locked nucleic acid (LNA) wherein the oxygen atom at the 2' position and the carbon atom at the 4' position have been bridged via methylene, and an ethylene bridged nucleic acid (ENA) [Nucleic Acid Research, 32, e175 (2004)] can be mentioned, and a peptide nucleic acid (PNA) [Acc. Chem. Res., 32, 624 (1999)], an oxypeptide nucleic acid (OPNA) [J. Am. Chem. Soc., 123, 4653 (2001)], and a peptide ribonucleic acid (PRNA) [J. Am. Chem. Soc., 122, 6900 (2000)] and the like can also be mentioned.

The nucleotide analogue modified by phosphodiester binding may be any one, as far as an optionally chosen chemical substance has been added to, or substituted for, a portion or all of the chemical structure of the phosphodiester bond of the nucleotide; for example, a nucleotide analogue substituted by a phosphorothioate bond, a nucleotide analogue substituted by an N3'-P5' phosphoamidate bond, and the like can be mentioned [SAIBO KOGAKU, 16, 1463-1473 (1997)] [RNAi Method and Antisense Method, Kodansha (2005)].

In the present invention, the nucleic acid derivative may be any molecule, as far as it is a molecule prepared by adding another chemical substance to the nucleic acid in order to improve the nuclease resistance thereof, to stabilize the same, to increase the affinity thereof for a complementary chain nucleic acid, to increase the cell permeability thereof, or to visualize the same, compared with the nucleic acid; for example, a 5'-polyamine addition derivative, a cholesterol addition derivative, a steroid addition derivative, a bile acid addition derivative, a vitamin addition derivative, a Cy5 addition derivative, a Cy3 addition derivative, a 6-FAM addition derivative, a biotin addition derivative and the like can be mentioned.

As examples of the other nucleic acid derivatives, specifically as derivatives modified at the sugar moiety, an oligonucleotide derivative substituted by 2'-O-propylribose, an oligonucleotide derivative substituted by 2'-methoxyethoxyribose, an oligonucleotide derivative substituted by 2'-O-methylribose, an oligonucleotide derivative substituted by 2'-O-methoxyethylribose, an oligonucleotide derivative substituted by 2'-O-[2-(guanidium)ethyl]ribose, an oligonucleotide derivative substituted by 2'-O-fluororibose and the like can be mentioned; as derivatives modified at the phosphate group, an oligonucleotide derivative wherein a phosphodiester bond in an oligonucleotide has been converted to a phosphorothioate bond, an oligonucleotide derivatives wherein a phosphodiester bond in an oligonucleotide has been converted to an N3'-P5'phosphoamidate bond and the like can be mentioned [SAIBO KOGAKU, 16, 1463-1473 (1997)] [RNAi Method and Antisense Method, Kodansha (2005)].

In the present invention, the micro-RNA derivative may be any polymer comprising a molecule, other than a ribonucleotide, that is functionally equivalent to the micro-RNA; for example, a DNA, which is a deoxyribonucleotide polymer, a mixed polymer of RNA and DNA, and a nucleotide polymer, including a nucleotide analogue, can be mentioned; furthermore, the micro-RNA derivative may be a nucleotide polymer, including a nucleic acid derivative, and may be a single-stranded nucleic acid or a double-stranded nucleic acid.

The micro-RNA precursor derivative may be any one, as far as it is a polymer comprising a molecule, other than a ribonucleotide, that is functionally equivalent to the micro-RNA precursor; for example, a DNA, which is a deoxyribonucleotide polymer, a mixed polymer of RNA and DNA, and a nucleotide polymer, including a nucleotide analogue, can be mentioned; furthermore, the micro-RNA precursor derivative may be a nucleotide polymer, including a nucleic acid derivative, and may be a single-stranded nucleic acid or a double-stranded nucleic acid.

The method of producing a nucleic acid of the present invention is not particularly limited; the same can be produced by a method using a known chemical synthesis, or an enzymatic transcription method and the like. As methods using a known chemical synthesis, the phosphoroamidite method, the phosphorothioate method, the phosphotriester method, the CEM method [Nucleic Acid Research, 35, 3287 (2007)] and the like can be mentioned; for example, the same can be synthesized using the ABI3900 high throughput nucleic acid synthesizer (manufactured by Applied Biosystems). As an enzymatic transcription method, transcription with a plasmid or DNA having a desired nucleotide sequence as the template using a typical phage RNA polymerase, for example, T7, T3, or SP6RNA polymerase, can be mentioned.

The method of detecting the expression of a nucleic acid such as a micro-RNA or a micro-RNA precursor using a nucleic acid of the present invention may be any method that enables detection of a nucleic acid in a sample; for example, (1) Northern hybridization, (2) dot blot hybridization, (3) in situ hybridization, (4) quantitative PCR, (5) differential hybridization, (6) microarray, (7) ribonuclease protection assay and the like can be mentioned.

The method of detecting a mutation of a nucleic acid such as a micro-RNA or a micro-RNA precursor using a nucleic acid of the present invention may be any method that enables detection of a mutation of the nucleotide sequence of a nucleic acid in a sample; for example, a method wherein a heteroduplex formed by hybridization of a nucleic acid having a non-mutated nucleotide sequence and a nucleic acid having a mutated nucleotide sequence are detected, or a method wherein a sample-derived nucleotide sequence is directly sequenced to detect the presence or absence of a mutation and the like can be mentioned.

The method of separating a cell that expresses a nucleic acid such as a micro-RNA or a micro-RNA precursor using a nucleic acid of the present invention may be any method that enables separation of a cell that expresses a nucleic acid such as a micro-RNA or a micro-RNA precursor from a mixture of various cells; for example, a method wherein a probe prepared by fluorescently labeling a nucleic acid having a sequence complementary to the nucleotide sequence of a nucleic acid of the present invention is introduced into a cell to cause hybridization with the probe, and only the cells that have hybridized with the labeled probe are separated using a flow cytometer with sorting function, and the like can be mentioned.

A vector that expresses a nucleic acid of the present invention refers to a vector designed for a nucleic acid of the present invention to be biosynthesized by being transcribed in a cell or in vitro, and the vector may be any vector having a promoter capable of transcribing a nucleic acid of the present invention in a cell or in vitro. Specifically, pcDNA6.2-GW/miR (manufactured by Invitrogen), pSilencer 4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned.

The method of suppressing the expression of a gene having a target nucleotide sequence of a nucleic acid, such as a micro-RNA, of the present invention (hereinafter referred to as a target gene) may be any method that suppresses the expression of a target gene by means of the activity to suppress the expression of an mRNA having a target nucleotide sequence using a nucleic acid, such as a micro-RNA, of the present invention. Here, to suppress the expression encompasses a case where the translation of an mRNA is suppressed, and a case where cleavage or decomposition of an mRNA results in a decreased amount of protein translated from the mRNA.

A target nucleotide sequence refers to the nucleotide sequence of a nucleic acid consisting of several nucleotides recognized by a nucleic acid, such as a micro-RNA, of the present invention. The translation of an mRNA having the nucleotide sequence is suppressed by a nucleic acid, such as a micro-RNA, of the present invention. Because an mRNA having a nucleotide sequence complementary to the sequence of the 2nd to 8th nucleotides on the 5' terminal side of a micro-RNA undergoes suppression of the translation thereof by the micro-RNA [Current Biology, 15, R458-R460 (2005)], a nucleotide sequence complementary to the sequence of the 2nd to 8th nucleotides on the 5' terminal side of a nucleic acid, such as a micro-RNA, of the present invention, can be mentioned as a target nucleotide sequence of the nucleic acid, such as the micro-RNA. For example, by providing a target sequence complementary to the 2nd to 8th nucleotides on the 5' terminal side of a micro-RNA, and selecting an mRNA comprising a sequence completely identical to a set of 3' UTR nucleotide sequences of human mRNAs by a method such as character sequence search, the target nucleotide sequence can be determined. A set of 3' UTR nucleotide sequences of human mRNAs can be prepared using information on genome sequences and gene positions that can be acquired from "UCSC Human Genome Browser Gateway (http://genome.ucsc.edu/cgi-bin/hgGateway)". As specific examples of genes having a target nucleotide sequence of a micro-RNA of SEQ ID NOs:1 to 1336, the genes shown in Tables 2-1 to 2-35, represented by names (Official Symbols and Gene IDs) used in the EntreGene database (http://www.ncbi.nlm.nih.gov/Entrez/) of the US National Center for Biotechnology Information (NCBI), can be mentioned. The gene names used are names in the EntreGene database as of March 2006.

**[Table 2-1]**

| **SEQ ID NO** | **Target gene** |
|---|---|
| 1 | PRKCZ(5590);TNFRSF14(8764);TNFRSF9(3604);RCC2(55920);NBL1(4681);LDLRAD2(401944);CDC42(998);E2 F2(1870);RCC1(1104);SDC3(9672);KHDRBS1(10657);HPCA(3208);RIMS3(9783);TOE1(114034);SERBP1(26135) ;SYPL2(284612);SLC6A17(388662);MCL1(4170);TNRC4(11189);C1orf60(65123);IL6R(3570);ZBTB7B(51043);LM NA(4000);MEF2D(4209);PEA15(8682);DEDD(9191);QSCN6(5768);ADORA1(134);IL24(11009);TMEM63A(9725);R HOB(388);KCNK3(3777);ZFP36L2(678);HTRA2(27429);ADRA2B(151);BRRN1(23397);BCL2L11(10018);BBS5(12 9880);CASP10(843);ALS2CR19(117583);INHA(3623);HDAC4(9759);KIF1A(547);SEPT2(4735);NEU4(129807);ZA K(51776);OGG1(4968);HDAC11(79885);ZNF651(92999);ZNF445(353274);DAG1(1605);HEMK1(51409);RBM15B( 29890);RNF7(9616);ALG3(10195);TP73L(8626);FLJ37478(339983);LRPAP1(4043);APBB2(323);PRLR(5618);AR SB(411);SEMA6A(57556);RAD50(10111);PFDN1(5201);WDR55(54853);N4BP3(23138);ATXN1(6310);E2F3(1871) ;IER3(8870);MCCD1(401250);C6orf47(57827);ZBTB12(221527);CREBL1(1388);PBX2(5089);BAK1(578);IHPK3(1 17283);CDKN1A(1026);ZFAND3(60685);KCNK5(8645);NFYA(4800);FOXO3A(2309);SNX8(29886);TRIAD3(54476 );CARD4(10392);INHBA(3624);DKFZp761I2123(83637);ADCY1(107);YWHAG(7532);FLJ39237(375607);LRRC4(6 4101);EPHA1(2041);CDK5(1020);TNFRSF10B(8795);TNFRSF10C(8794);PTK2B(2185);KIF13B(23303);UNC5D(1 37970);EXT1(2131);DGAT1(8694);MGC70857(414919);GAS1(2619);SEMA4D(10507);TRAF1(7185);CDK9(1025); ST6GALNAC6(30815);ABL1(25);BRD3(8019);TRAF2(7186);C9orf140(89958);FLJ20245(54863);FRMD4A(55691 );SPOCK2(9806);LOC439985(439985);PTEN(5728);SLIT1(6585);NOLC1(9221);C10orf46(143384);FAM53B(9679 );FLJ46300(399827);MUCDHL(53841);TOLLIP(54472);BRSK2(9024);IGF2(3481);OSBPL5(114879);C11orf49(79 096);VEGFB(7423);CDCA5(113130);DPF2(5977);SSSCA1(10534);RELA(5970);PPP1CA(5499);RPS6KB2(6199); CCND1(595);FADD(8772);INPPL1(3636);GAB2(9846);SCN4B(6330);ABCG4(64137);EI24(9538);ST3GAL4(6484); CACNA1C(775);TSPAN9(10867);CCND2(894);ING4(51147);PTMS(5763);FGD4(121512);FLJ20489(55652);HDA C7A(51564);RND1(27289);MCRS1(10445);FAIM2(23017);RARG(5916);GDF11(10220);CENTG1(116986);PPTC7( 160760);PPP1CC(5501);SCARB1(949);RAN(5901);DIP13B(55198);SLC7A1(6541);FLJ40296(122183);POU4F1(5 457);LAMP1(3916);BCL2L2(599);TM9SF1(10548);FLJ39779(400223);ERH(2079);VASH1(22846);CHES1(1112);T RAF3(7187);FLJ42486(388021);AKT1(207);NDN(4692);KLF13(51621);BMF(90427);BAHD1(22893);CSK(1445);IG F1R(3480);PIGQ(9091);KIAA1924(197335);CCDC78(124093);C1QTNF8(390664);NME3(4832);TBL3(10607);TSC 2(7249);TRAF7(84231);CCNF(899);TNFRSF12A(51330);MMPL1(4328);N-PAC(84656);GPRC5B(51704);PLK1(5 347);MAPK3(5595);FBXL19(54620);POLR2C(5432);TRADD(8717);LOC283849(283849);E2F4(1874);SLC12A4(6 560);SLC7A6(9057);SMPD3(55512);TERF2(7014);KIAA0513(9764);PCOLN3(5119);PRDM7(11105);PARD6A(508 55);ALOX15B(247);PAFAH1B1(5048);DHX33(56919);VAMP2(6844);GAS7(8522);CORO6(84940);GOSR1(9527); RASL10B(91608);DUSP3(1845);UBTF(7343);SLC4A1(6521);MAPT(4137);CDK5RAP3(80279);CROP(51747);MSI 2(124540);ERN1(2081);PRKCA(5578);SPHK1(8877);TMC6(11322);BIRC5(332);SOCS3(9021);FLJ21865(64772); CARD14(79092);CIDEA(1149);CABLES1(91768);MAPRE2(10982);BCL2(596);TXNL4A(10907);APC2(10297);MK NK2(2872);C19orf36(113177);FZR1(51343);MATK(4145);DAPK3(1613);ZBTB7A(51341);TNFAIP8L1(126282);M AP2K7(5609);CDKN2D(1032);TNPO2(30000);GADD45GIP1(90480);BTBD14B(112939);DPF1(8193);MAP3K10(4 294);AKT2(208);PRX(57716);AXL(558);ARHGEF1(9138);ERF(2077);IGSF4C(199731);APOE(348);SPHK2(56848); RPS11(6205);SPIB(6689);LOC284361(284361);SAPS1(22870);HMG20B(10362);GRLF1(2909);CSNK2A1(1457); TM9SF4(9777);E2F1(1869);GGTL3(2686);LPIN3(64900);MYBL2(4605);DIDO1(11083);BIRC7(79444);ARFGAP1( 55738);PLAC4(191585);FLJ41733(400870);C21orf123(378832);BCL2L13(23786);TSSK2(23617);DGCR14(8220); LOC128977(128977);SEPT5(5413);HIC2(23119);MAPK1(5594);SMARCB1(6598);ADORA2A(135);MN1(4330);GA S2L1(10634);CABP7(164633);KIAA1904(114794);CBX6(23466);CBX7(23492);MGAT3(4248);PPARA(5465);FLJ4 1993(400935);SAPS2(9701);SBF1(6305);MAPK8IP2(23542);MGC3731(79159);PCTK1(5127);SMC1L1(8243);SN X12(29934);BTK(695);AGTR2(186);ZDHHC9(51114);EMD(2010);AFF2(2334); |
| 2 | E2F2(1870);CCDC21(64793);SDC3(9672);RBBP4(5928);KIAA0319L(79932);C1orf84(149469);AGBL4(84871);CE LSR2(1952);RAP1A(5906);SV2A(9900);C1orf60(65123);SHC1(6464);UBQLN4(56893);MEF2D(4209);BCAN(6382 7);IGSF4B(57863);NAV1(89796);PPP1R12B(4660);RIPK5(25778);C1orf95(375057);YPEL5(51646);TEX261(1134 19);KCNIP3(30818);IMP4(92856);PNKD(25953);ATG9A(79065);COPS7B(64708);ATP2B2(491);HEMK1(51409);R AB6B(51560);SLCO2A1(6578);SLC26A1(10861);EVC(2121);FLJ46481(389197);SETD7(80854);KIAA1909(15347 8);PCDH1(5097);PDGFRB(5159);NDST1(3340);CPLX2(10814);N4BP3(23138);RNF5(6048);PBX2(5089);BAK1(57 8);PACSIN1(29993);MOCS1(4337);FOXP4(116113);MGC45491(221416);TRAM2(9697);PSCD3(9265);AQP1(358 );POM121(9883);CASP2(835);VIPR2(7434);C9orf25(203259);TPM2(7169);NTRK2(4915);C9orf47(286223);ST6G ALNAC6(30815);SPOCK2(9806);SH3PXD2A(9644);VAX1(11023);MTG1(92170);SPRN(503542);C11orf11(747);P ACS1(55690);LOC399947(399947);BCL9L(283149);CACNA1C(775);FLJ20489(55652);FMNL3(91010);CBX5(23 468);MYL6(4637);GLS2(27165);CTDSP2(10106);DDX54(79039);B4GALNT1(2583);SLC8A3(6547);PCNX(22990); KIAA0125(9834);BMF(90427);TLN2(83660);PML(5371);NEIL1(79661);ABHD2(11057);EMP2(2013);UBPH(56061); SBK1(388228);ATP2A1(487);N4BP1(9683);CCL22(6367);POLR2C(5432);AFG3L1(172);SMG6(23293);GAS7(852 2);C17orf63(55731);ACACA(31);MLLT6(4302);LASP1(3927);WIRE(147179);PTRF(284119);MAPT(4137);PNPO(5 5163);NGFR(4804);NXPH3(11248);LIMD2(80774);CDC42EP4(23580);C17orf28(283987);SEPT9(10801);PCYT2(5 833);MAFG(4097);RNF165(494470);FZR1(51343);TUBB4(10382);PDE4A(5141);NFIX(4784);RAB8A(4218);ZNF58 5A(199704);APOE(348);DMWD(1762);SHANK1(50944);MGC2752(65996);LOC389286(389286);C20orf27(54976); SOX12(6666);ProSAPiP1(9762);RIMS4(140730);DIDO1(11083);BTBD4(140685);TRPM2(7226);POFUT2(23275); MICAL-L1(85377);MGAT3(4248);TEF(7008);NFAM1(150372);TTLL12(23170);SSX4B(548313);SYN1(6853);SSX |

**[Table 2-2]**

| | |
|---|---|
| | 6(280657):RP11-114H20.1(401589):SSX2(6757);SMCILI(8243); |
| 3 | TNFRSF14(8764);NBL1(4681);CDC42(998);LIN28(79727);KHDRBS1(10657);JMJD2A(9682);GFI1(2672);CDC14 A(8556);AMIGO1(57463);MCL1(4170);RORC(6097);MEF2D(4209);PEA15(8682);UHMK1(127933);ADORA1(134); NUCKS1(64710);TP53BP2(7159);C1orf69(200205);KIF21B(23046);RHOB(388);KCNK3(3777);PRKCE(5581);MX D1(4084);KCNIP3(30818);BRRN1(23397);BBS5(129880);CFLAR(8837);CASP10(843);CTDSP1(58190);INHA(362 3);PAX3(5077);HDAC4(9759);KIF1A(547);SRGAP3(9901);VHL(7428);RBMS3(27303);AXUD1(64651);ZNF651(92 999);RBM15B(29890);MAGI1(9223);FXR1(8087);TP73L(8626);PPP1R2(5504);G3BP2(9908);PRLR(5618);FLJ391 55(133584);ITGA2(3673);RAD17(5884);SEMA6A(57556);RAD50(10111);PHF15(23338);HNRPA0(10949);CDC23( 8697);CAMK2A(815);CD74(972);NDST1(3340);DDR1(780);ZBTB12(221527);SKIV2L(6499);SYNGAP1(8831);BA K1(578);MAPK13(5603);ZFAND3(60685);TRAM2(9697);FOXO3A(2309);L3MBTL3(84456);STX7(8417);TNFAIP3( 7128);PDE10A(10846);PDCD2(5134);TRIAD3(54476);SCRN1(9805);IGFBP3(3486);EGFR(1956);YWHAG(7532);L EP(3952);FASTK(10922);TNFRSF10B(8795);TNFRSF10A(8797);TRIM35(23087);CLU(1191);KIF13B(23303);DUS P4(1846);UBE2V2(7336);MTSS1(9788);CDC37L1(55664);CDK9(1025);C9orf140(89958);GPR107(57720);ADARB 2(105);SPOCK2(9806);NOLC1(9221);TCF7L2(6934);C10orf46(143384);TIAL1(7073);LRRC56(115399);IGF2(348 1);IPO7(10527);API5(8539);PRPF19(27339);GANAB(23193);CDCA5(113130);DPF2(5977);SSSCA1(10534);NPA S4(266743);PPP1CA(5499);FGF4(2249);SESN3(143686);YAP1(10413);PPP2R1B(5519);FLJ25530(220296);EI24 (9538);ARF3(377);C12orf22(81566);LOC401720(401720);GDF11(10220);SUDS3(64426);HNRPA1(3178);LATS2( 26524);FLJ40296(122183);POU4F1(5457);BCL2L2(599);JPH4(84502);ERH(2079);VASH1(22846);CHES1(1112); ANKRD9(122416);AKT1(207);KLF13(51621);TPM1(7168);CSK(1445);IGF1R(3480);CCDC78(124093);C1QTNF8( 390664);TSC2(7249);TRAF7(84231);TNFRSF12A(51330);ERN2(10595);MAPK3(5595);FBXL19(54620);CIAPIN1( 57019);CMTM4(146223);TRADD(8717);LOC283849(283849);SLC12A4(6560);SMPD3(55512);KIAA0513(9764);C DT1(81620);RPL13(6137);ALOX15B(247);MNT(4335);PAFAH1B1(5048);DLG4(1742);TNFSF12(8742);GOSR1(9 527);NF1(4763);RAB11FIP4(84440);RASL10B(91608);NR1D1(9572);HDAC5(10014);CRHR1(1394);MAPT(4137); NGFR(4804);SPHK1(8877);TMC6(11322);SOCS3(9021);PCYT2(5833);CIDEA(1149);CABLES1(91768);PHLPP(2 3239);BCL2(596);TXNL4A(10907);FZR1(51343);ZBTB7A(51341);SEMA6B(10501);TNFSF9(8744);CDC37(11140 );CDKN2D(1032);ILF3(3609);DNM2(1785);RAB3D(9545);LPPR2(64748);ELAVL3(1995);TNPO2(30000);BTBD14 B(112939);F2RL3(9002);ELL(8178);DPF1(8193);MAP3K10(4294);ERF(2077);IGSF4C(199731);BCL3(602);APOE( 348);FOSB(2354);BBC3(27113);SLC8A2(6543);BAX(581);CD37(951);BCL2L12(83596);PPP2R1A(5518);MYADM (91663);CACNG7(59284);U2AF2(11338);ZNF264(9422);ZNF17(7565);GRLF1(2909);SCRT2(85508);E2F1(1869); UBE2V1(7335);CABLES2(81928);DIDO1(11083);ADARB1(104);BCL2L13(23786);DGCR14(8220);SEPT5(5413); MAPK1(5594);SMARCB1(6598);MN1(4330);GAS2L1(10634);LIF(3976);MCM5(4174);KIAA1904(114794);CARD10 (29775);C22orf5(25829);CBX6(23466);CBX7(23492);TCF20(6942);PHF21B(112885);PPARA(5465);SAPS2(9701 );SBF1(6305);MAPK8IP2(23542);RS1(6247);PCTK1(5127);ELK1(2002);ZNF81(347344);SMC1L1(8243);RP1-112 K5.2(90121);MLLT7(4303);BRWD3(254065);SEPT6(23157);MTCP1(4515);AFF2(2334); |
| 4 | BOK(666) |
| 5 | GAS7(8522) |
| 6 | PI15(51050) |
| 7 | TAGLN(6876) |
| 8 | SAMD11(148398);CDC2L2(985);TP73(7161);HES2(54626);THAP3(90326);TNFRSF9(3604);LDLRAP1(26119);N UDC(10726);RCC1(1104);TRIM62(55223);KIAA0319L(79932);MPL(4352);CDC20(991);SH3GLB1(51100);PROK1 (84432);KCNJ9(3765);DEDD(9191);GPR161(23432);TNNI1(7135);TMEM63A(9725);C1orf95(375057);C1orf69(20 0205);MTR(4548);RHOB(388);CGREF1(10669);BRRN1(23397);BCL2L11(10018);WDR33(55339);PLEKHB2(5504 1);MCM6(4175);KIF1A(547);RAF1(5894);AXUD1(64651);RASSF1(11186);NEK4(6787);TMEM110(375346);CLSTN 2(64084);LOC285382(285382);LETM1(3954);SH3BP2(6452);HD(3064);BTC(685);UNC5C(8633);NEK1(4750);TP PP(11076);ERBB2IP(55914);MRPS27(23107);CDC23(8697);PDGFRB(5159);HMP19(51617);DHX16(8449);AIF1(1 99);MAPK13(5603);CDKN1A(1026);LATS1(9113);ESR1(2099);RPS6KA2(6196);LOC90639(90639);MAD1L1(8379 );FOXK1(221937);TRIAD3(54476);HDAC9(9734);INHBA(3624);TMED4(222068);ADCY1(107);IGFBP3(3486);YWH AG(7532);KIAA0773(9715);LOC155060(155060);RBM33(155435);MAFK(7975);CLN8(2055);BIN3(55909);TNFRS F10C(8794);CLU(1191);BAG1(573);SHB(6461);C9orf47(286223);TRIM14(9830);TRAF1(7185);ASB6(140459);FN BP1(23048);ABL1(25);TSC1(7248);QSCN6L1(169714);UNC5B(219699);C10orf104(119504);FAS(355);LZTS2(84 445);SUFU(51684);SPRN(503542);IGF2(3481);KCNQ1(3784);WT1(7490);SLC15A3(51296);LOC144097(144097); CDC42EP2(10435);BRMS1(25855);CCND1(595);FADD(8772);LRRC51(220074);GAB2(9846);SESN3(143686);CA SP5(838);EXPH5(23086);TAGLN(6876);ETV6(2120);FLJ20489(55652);HDAC7A(51564);MCRS1(10445);FMNL3( 91010);FAIM2(23017);CBX5(23468);RBMS2(5939);BTG1(694);SOCS2(8835);GAS2L3(283431);TCF1(6927);C12 orf43(64897);DIABLO(56616);SCARB1(949);LATS2(26524);WDFY2(115825);LTB4R2(56413);SPTB(6710);RAB1 5(376267);LTBP2(4053);VASH1(22846);SLC24A4(123041);TRAF3(7187);PACS2(23241);KIAA0125(9834);FLJ43 339(388115);CA12(771);DAPK2(23604);PDCD7(10081);CLN6(54982);ARNT2(9915);C15orf38(348110);IDH2(341 8);IGF1R(3480);LOC440313(440313);LRRK1(79705);RAB40C(57799);KIAA1924(197335);CCDC78(124093);CLC N7(1186);TRAF7(84231);CCNF(899);PKMYT1(9088);BTBD12(84464);DNAJA3(9093);C16orf5(29965);GSPT1(29 35);XYLT1(64131);RPS15A(6210);PLK1(5347);SBK1(388228);STX1B2(112755);FTS(64400);GNAO1(2775);LIN1 |

**[Table 2-3]**

| | |
|---|---|
| | 0(80262);LOC283849(283849);RANBP10(57610);SLC12A4(6560);SMPD3(55512);KIAA0513(9764);FLJ45121(40 0556);CDT1(81620);CDK10(8558);ANAPC11(51529);ALOX15B(247);TUSC5(286753);HIC1(3090);TNFSF12(874 2);GAS7(8522);MFAP4(4239);WSB1(26118);POLDIP2(26073);HCP1(113235);EZH1(2145);MAPT(4137);NFE2L1( 4779);CROP(51747);KIAA0195(9772);EXOC7(23265);TBC1D16(125058);BAIAP2(10458);DUS1L(64118);MAPK4 (5596);DCC(1630);MBP(4155);HMHA1(23526);ZBTB7A(51341);MAP2K7(5609);ELAVL3(1995);PRDX2(7001);F2 RL3(9002);KIAA0892(23383);ZNF43(7594);MGC20255(90324);APOE(348);LOC400707(400707);CARD8(22900); RPS11(6205);HKR2(342945);SNPH(9751);KCNK15(60598);CSE1L(1434);UBE2V1(7335);LAMA5(3911);DIDO1(1 1083);CHRNA4(1137);AGPAT3(56894);TRPM2(7226);UBE2G2(7327);COL18A1(80781);C22orf25(128989);HTF9 C(27037);MAPK1(5594);PPM1F(9647);SMARCB1(6598);GAS2L1(10634);NF2(4771);SSTR3(6753);TOB2(10766); SEPT3(55964);PPARA(5465);GTSE1(51512);ADM2(79924);APOL6(80830);ZC3H12B(340554);RNF12(51132);M ECP2(4204); |
| 9 | GM632(57473) |
| 10 | CEBPA(1050) |
| 11 | RBPSUH(3516) |
| 12 | BOK(666) |
| 13 | PACS2(23241) |
| 14 | KIF1B(23095);SPATA6(54558);LEPR(3953);GBP6(163351);LRRC8C(84230);ABCD3(5825);AGL(178);CDC14A(8 556);UHMK1(127933);RGS4(5999);YOD1(55432);NCOA1(8648);CRIM1(51232);FLJ13910(64795);HNMT(3176);L OC339745(339745);ITGA4(3676);PGAP1(80055);CREB1(1385);TRPM8(79054);MGAT4A(11320);IGSF4D(25355 9);VGLL3(389136);ZPLD1(131368);IFT57(55081);NCK1(4690);DBR1(51163);DHX36(170506);PPAT(5471);TMPR SS11B(132724);RASSF6(166824);CXCL5(6374);PAQR3(152559);FGF5(2250);AFF1(4299);DAPP1(27071);DNAJ B14(79982);BBS7(55212);SLC7A11(23657);FLJ38482(201931);ASB5(140458);IL7R(3575);LIFR(3977);FLJ21657 (64417);HCN1(348980);ITGA2(3673);ELOVL7(79993);R7BP(401190);TNPO1(3842);POLK(51426);HOMER1(945 6);LOC153364(153364);ACSL6(23305);FLJ37562(134553);DCDC2(51473);ICK(22858);ASCC3(10973);C6orf68(1 16150);AHI1(54806);TXLNB(167838);PDE10A(10846);MGC42090(256130);CREB5(9586);LOC441257(441257);S TEAP4(79689);STEAP2(261729);BCAP29(55973);WNT16(51384);NDUFA5(4698);CALD1(800);PLAG1(5324);YT HDF3(253943);PMP2(5375);ANGPT1(284);TRPS1(7227);KLHL9(55958);TRPM6(140803);FRMD3(257019);NTRK 2(4915);C9orf5(23731);PAPPA(5069);RP11-142I17.1(26095);PLEKHK1(219790);FAS(355);IFIT5(24138);ADD3(1 20);PGM2L1(283209);HBXAP(51773);PANX1(24145);SESN3(143686);KIAA0999(23387);CBL(867);KRAS(3845); STK38L(23012);MON2(23041);LIN7A(8825);SLC41A2(84102);TMEM132B(114795);MGC40069(348035);KIAA13 33(55632);MIPOL1(145282);C14orf138(79609);SOS2(6655);PIGH(5283);PCNX(22990);GTF2A1(2957);SEL1L(64 00);UBE3A(7337);PLDN(26258);MYO5A(4644);USP31(57478);NF1(4763);MSI2(124540);C18orf1(753);DSG2(182 9);SMAD2(4087);FVT1(2531);DOK6(220164);NETO1(81832);ZNF568(374900);BMP2(650);RAB22A(57403);BTB D4(140685);CXADR(1525);PKNOX1(5316);KAL1(3730);DMD(1756);SCML1(6322); |
| 15 | RAB40C(57799) |
| 16 | NFATS(10725) |
| 17 | LAMP1(3916) |
| 18 | PRX(57716) |
| 19 | GIPC3(126326) |
| 20 | TNFRSF1B(7133);RCC2(55920);TTMB(399474);FABP3(2170);GIPC2(54810);CDC14A(8556);CDC42SE1(56882) ;DEDD(9191);FASLG(356);ZBTB41(360023);TNNI1(7135);LPGAT1(9926);C1orf140(400804);NID1(4811);AKT3(1 0000);C1orf173(127254);SOX11(6664);CAD(790);FOSL2(2355);REEP1(65055);IL1RL1(9173);IL1A(3552);PTD00 4(29789);CASP10(843);CREB1(1385);SEPT2(4735);ZAK(51776);RBMS3(27303);EIF4E3(317649);KALRN(8997); SLC9A9(285195);SLC2A2(6514);TNFSF10(8743);DKFZP686A01247(22998);GABRA4(2557);FLJ38991(285521); SNCA(6622);CASP6(839);ADCY2(108);PRLR(5618);LOC153561(153561);ZBED3(84327);APC(324);DNAJC18(2 02052);PCDHGB4(8641);UNC5A(90249);PRPF4B(8899);FLJ45422(441140);FKBP5(2289);RPL7L1(285855);SYN CRIP(10492);PLAGL1(5325);RABGEF1(27342);PODXL(5420);ABCF2(10061);REXO1L1(254958);DLGAP2(9228) ;EFHA2(286097);UNC5D(137970);CHD7(55636);MTFR1(9650);UBE2W(55284);ATP6V0D2(245972);CCNE2(9134 );PTPLAD2(401494);SMU1(55234);TLR4(7099);MAP3K8(1326);C10orf54(64115);KIF11(3832);TIAL1(7073);GAS 2(2620);SSSCA1(10534);PGM2L1(283209);HBXAP(51773);NDUFC2(4718);DLG2(1740);MTMR2(8898);SNF1LK2 (23235);CBL(867);LOC283174(283174);KRAS(3845);CBX5(23468);RBMS2(5939);TMCC3(57458);KIAA0152(976 1);TNFRSF19(55504);TFDP1(7027);RHOJ(57381);FLJ39531(400360);LOC405753(405753);PDCD7(10081);RAB 11A(8766);AP3S2(10239);IQGAP1(8826);PHLPPL(23035);CROP(51747);WBP2(23558);DLGAP1(9229);C18orf1( 753);AQP4(361);SMAD2(4087);DOK6(220164);TNFSF14(8740);DNAJB1(3337);APOE(348);ZNF347(84671);RNF 24(11237);TP53INP2(58476);RBL1(5933);ABCC13(150000);NF2(4771);SYN3(8224);RAXLX(91464);ATRX(546); |

**[Table 2-4]**

| | |
|---|---|
| | CD40G(959);AFF2(2334): |
| 21 | DFFA(1676);TNFRSF1B(7133);NBL1(4681);NUDC(10726);SESN2(83667);RCC1(1104);TTMB(399474);KIAA0319 L(79932);NFYC(4802);TESK2(10420);CDC7(8317);RAP1A(5906);NRAS(4893);SNX27(81609);CD84(8832);UHMK 1(127933);QSCN6(5768);PPP1R12B(4660);CCNL2(81669);E2F6(1876);RBJ(51277);KHK(3795);OXER1(165140); TGFA(7039);KCNIP3(30818);BCL2L11(10018);ANAPC1(64682);RIF1(55183);CASP10(843);OGG1(4968);FANCD 2(2177);STAG1(10274);CHRD(8646);TP73L(8626);PCGF3(10336);CD38(952);EREG(2069);BTC(685);SEPT11(55 752);KIAA1909(153478);UNG2(10309);F2R(2149);FGF1(2246);RNF130(55819);RIPK1(8737);LY86(9450);E2F3(18 71);MAPK13(5603);TRAM2(9697);DST(667);SASH1(23328);FLJ34503(285759);MGC11257(84310);TRIAD3(5447 6);INHBA(3624);PURB(5814);CALN1(83698);CDK6(1021);CASP2(835);PSD3(23362);POLR3D(661);BIN3(55909) ;TNFRSF10B(8795);BNIP3L(665);CLU(1191);UNC5D(137970);UBE2V2(7336);STK3(6788);KIAA1875(340390);G AS1(2619);SEMA4D(10507);FGD3(89846);TRIM14(9830);SPTAN1(6709);ADAMTS13(11093);CARD9(64170);ZM YND11(10771);ZWINT(11130);SIRT1(23411);UNC5B(219699);LZTS2(84445);SUFU(51684);C10orf46(143384);H RAS(3265);PARVA(55742);HIPK3(10114);DAK(26007);DPF2(5977);DLG2(1740);SCN4B(6330);MLL(4297);H2AF X(3014);SRPR(6734);RAD52(5893);CCND2(894);TNFRSF7(939);CDKN1B(1027);IAPP(3375);HDAC7A(51564);F AIM2(23017);C12orf22(81566);PDE1B(5153);DDIT3(1649);CENTG1(116986);PCTK2(5128);APAF1(317);HRK(87 39);EP400NL(347918);TNFRSF19(55504);RB1(5925);KLF12(11278);CUL4A(8451);ACIN1(22985);BCL2L2(599);J PH4(84502);MNAT1(4331);PSEN1(5663);TGFB3(7043);VASH1(22846);PPP1R13B(23368);MAPK6(5597);FLJ38 723(255180);KIF23(9493);IGF1R(3480);ALPK3(57538);RAB40C(57799);TRAF7(84231);PKMYT1(9088);DNAJA3( 9093);GSPT1(2935);PLK1(5347);ERN2(10595);PRRT2(112476);RNF40(9810);FTS(64400);CIAPIN1(57019);CTC F(10664);SMPD3(55512);CDK10(8558);ANAPC11(51529);MNT(4335);TNFSF12(8742);GAS7(8522);HCP1(11323 5);CDK5RAP3(80279);C17orf73(55018);MPO(4353);ABC1(63897);BIRC5(332);EMILIN2(84034);GNAL(2774);CA BLES1(91768);PHLPP(23239);BCL2(596);FZR1(51343);DPF1(8193);SIRT2(22933);SAMD4B(55095);LYPD5(284 348);APOE(348);OPA3(80207);BBC3(27113);BAX(581);FLJ38288(284309);GRLF1(2909);PLCB1(23236);CSE1L( 1434);ZGPAT(84619);MAPK1(5594);NF2(4771);CARD10(29775);NPTXR(23467);TNRC6B(23112);TOB2(10766); FIGF(2277);SMC1L1(8243);SEPT6(23157);MECP2(4204); |
| 22 | CDC2L2(985);TNFRSF14(8764);CHD5(26038);CDC42(998);E2F2(1870);WASF2(10163);RCC1(1104);KHDRBS1( 10657);LCK(3932);KIAA0319L(79932);RIMS3(9783);CDC20(991);CDKN2C(1031);NOTCH2(4853);CKS1B(1163); C1orf21(81563);PPP1R12B(4660);SYT2(127833);ADORA1(134);PIK3C2B(5287);IL10(3586);IL24(11009);PLXNA 2(5362);VANGL1(81839);KIF21B(23046);RHOB(388);MAPRE3(22924);TGFA(7039);HK2(3099);BRRN1(23397);IN HBB(3625);ERCC3(2071);PROC(5624);TAIP-2(80034);TLK1(9874);BARD1(580);TNS1(7145);PAX3(5077);HDAC 4(9759);BOK(666);ZAK(51776);ZNF651(92999);RASSF1(11186);HEMK1(51409);RBM15B(29890);RNF7(9616);TP 73L(8626);FAM43A(131583);LETM1(3954);HD(3064);EREG(2069);CCNG2(901);TPPP(11076);DAP(1611);PRKA A1(5562);RPL37(6167);ERBB2IP(55914);APC(324);MCC(4163);HDAC3(8841);CPLX2(10814);NSD1(64324);IER3 (8870);C2(717);BAK1(578);IHPK3(117283);PACSIN1(29993);MAPK13(5603);CDKN1A(1026);TREML2(79865);PT K7(5754);SRF(6722);VEGF(7422);SUPT3H(8464);IL17(3605);FOXO3A(2309);HECA(51696);ELMO1(9844);FOXK 1(221937);TRIAD3(54476);HDAC9(9734);ADCY1(107);IGFBP3(3486);EGFR(1956);FLJ37538(222950);CASP2(83 5);KIAA0773(9715);VIPR2(7434);LZTS1(11178);POLR3D(661);BIN3(55909);TNFRSF10A(8797);TRIM35(23087); PTK2B(2185);DUSP4(1846);UNC5D(137970);TERF1(7013);TP53INP1(94241);LYNX1(66004);MGC21881(389741 );UHRF2(115426);CDKN2B(1030);TAF1L(138474);BAG1(573);FANCG(2189);SEMA4D(10507);TNFSF15(9966);T NFSF8(944);TRAF1(7185);DAB2IP(153090);FAM102A(399665);ADARB2(105);ZWINT(11130);SIRT1(23411);SGP L1(8879);C10orf54(64115);CHST3(9469);KIF11(3832);LZTS2(84445);FGF8(2253);C10orf76(79591);SUFU(51684 );PDCD11(22984);NEURL(9148);BAG3(9531);FAM53B(9679);APBB1(322);MDK(4192);CDCA5(113130);DPF2(59 77);CLCF1(23529);PPP1CA(5499);FADD(8772);RAB30(27314);SCN2B(6327);H2AFX(3014);EI24(9538);CDCA3( 83461);BCAT1(586);KRAS(3845);HDAC7A(51564);MCRS1(10445);TEGT(7009);FAIM2(23017);CBX5(23468);PD E1B(5153);ITGA7(3679);MYL6(4637);CRADD(8738);FOXO1A(2308);RFP2(10206);POU4F1(5457);LOC283487(2 83487);BCL2L2(599);RGS6(9628);PSEN1(5663);LTBP2(4053);NEK9(91754);VASH1(22846);TRAF3(7187);BAG5 (9529);AKT1(207);JAG2(3714);BMF(90427);FLJ43339(388115);FLJ38723(255180);SNX1(6642);PDCD7(10081); CSK(1445);ABHD2(11057);IGF1R(3480);NME3(4832);TSC2(7249);TRAF7(84231);CCNF(899);TNFRSF12A(5133 0);ERN2(10595);P8(26471);PRRT2(112476);CYLD(1540);FTS(64400);LOC283849(283849);E2F4(1874);CTCF(10 664);SLC12A4(6560);KIAA0513(9764);PCOLN3(5119);ABR(29);HIC1(3090);PAFAH1B1(5048);GARNL4(23108); TNFSF12(8742);TP53(7157);GAS7(8522);TOM1L2(146691);POLDIP2(26073);TRAF4(9618);NF1(4763);MLLT6(4 302);PTRF(284119);BRCA1(672);TBKBP1(9755);IGF2BP1(10642);PHB(5245);NGFR(4804);CROP(51747);DYNL L2(140735);MPO(4353);PRKCA(5578);CDK3(1018);SOCS3(9021);BAIAP2(10458);CABLES1(91768);RNF165(49 4470);KIAA0427(9811);BCL2(596);NFATC1(4772);SF3A2(8175);GNG7(2788);FZR1(51343);PIN1(5300);CDC37(1 1140);TNPO2(30000);LPHN1(22859);SIRT2(22933);IGSF4C(199731);FOSB(2354);DMWD(1762);BBC3(27113);R PS11(6205);PPP2R1A(5518);GRLF1(2909);ZNF343(79175);C20orf116(65992);E2F1(1869);CEP250(11190);PPP 1R16B(26051);ZHX3(23051);RIMS4(140730);STK4(6789);DIDO1(11083);TNFRSF6B(8771);ZGPAT(84619);APP( 351);ADARB1(104);LSS(4047);HIC2(23119);MAPK1(5594);PPM1F(9647);MIF(4282);ADORA2A(135);NF2(4771); KIAA1904(114794);MGAT3(4248);ZC3H7B(23264);NHP2L1(4809);SEPT3(55964);GTSE1(51512);PCTK1(5127);I QSEC2(23096);TMEM28(27112);MLLT7(4303);RP6-213H19.1(51765);CD40LG(959);HCFC1(3054);PRKY(5616); |
| 23 | ANKRD36(375248) |

**[Table 2-5]**

| | |
|---|---|
| 24 | NFIX(4784) |
| 25 | SDF4(51150);B3GALT6(126792);UBE2J2(118424);CENTB5(116983);SKI(6497);C1orf93(127281);SPSB1(80176) ;PIK3CD(5293);TINAGL1(64129);KIAA0319L(79932);IGSF4B(57863);ZNF648(127665);WNT3A(89780);OR2W5(4 41932);OR2C3(81472);ATAD3C(219293);INPP5D(3635);ANKRD23(200539);RAMP1(10267);RIS1(25907);PH-4(5 4681);IHPK1(9807);PLXNA1(5361);CLDN18(51208);LIPH(200879);GP5(2814);CPLX1(10815);MAEA(10296);RGS 12(6002);LOC93622(93622);DCAMKL2(166614);DUX4(22947);AHRR(57491);TPPP(11076);CTNND2(1501);PITX 1(5307);PCDHA1(56147);PCDHA2(56146);PCDHA3(56145);PCDHA4(56144);PCDHA5(56143);PCDHA6(56142); PCDHA7(56141);PCDHA8(56140);PCDHA9(9752);PCDHA10(56139);PCDHA11(56138);PCDHA12(56137);PCDH A13(56136);PCDHAC1(56135);PCDHAC2(56134);SH3PXD2B(285590);UNC5A(90249);DBN1(1627);LOC51149(5 1149);C6orf85(63027);C6orf145(221749);TULP1(7287);COL12A1(1303);TCF21(6943);RPS6KA2(6196);VGF(742 5);BCAP29(55973);EPHA1(2041);ATG9B(285973);LRCH4(4034);KIAA1688(80728);CDKN2B(1030);B4GALT1(26 83);KLF9(687);IPPK(64768);DAB2IP(153090);BRD3(8019);PTGDS(5730);FLJ45224(401562);C9orf140(89958);M GC61598(441478);LOC399706(399706);C10orf114(399726);LOC439985(439985);C10orf116(10974);FRAT1(100 23);MARVELD1(83742);C10orf39(282973);STK32C(282974);INPP5A(3632);FBXO3(26273);CTTN(2017);DLG2(1 740);DKFZp547C195(257160);KIRREL3(84623);HOM-TES-103(25900);DDN(23109);METTL7A(25840);HOXC13( 3229);CDK4(1019);IPF1(3651);C13orf21(387923);SLITRK5(26050);ATP11A(23250);GPR68(8111);PPP2R5C(552 7);BRF1(2972);PACS2(23241);C15orf37(283687);UBE2I(7329);IFT140(9742);CASKIN1(57524);ZNF213(7760);ZN F500(26048);SEPT1(1731);FBXL19(54620);GPT2(84706);MON1B(22879);MGC51025(353149);TRAF4(9618);AN KRD13B(124930);CCDC55(84081);RAB11FIP4(84440);LHX1(3975);PCGF2(7703);HLF(3131);KIAA0195(9772);C 1QTNF1(114897);HOXB8(3218);C18orf43(10650);RNF165(494470);KIAA0427(9811);CDH20(28316);TXNL4A(10 907);WDR18(57418);MKNK2(2872);MOBKL2A(126308);LMNB2(84823);DAPK3(1613);TNFAIP8L1(126282);JMJD 2B(23030);TIMM44(10469);RAVER1(125950);NFIX(4784);F2RL3(9002);ZBTB32(27033);RASGRP4(115727);CAP N12(147968);PSCD2(9266);SPHK2(56848);BCAT2(587);LIN7B(64130);PRKCG(5582);LENG8(114823);LOC3529 09(352909);ICAM4(3386);EEF2(1938);GRLF1(2909);UBOX5(22888);BLCAP(10904);SPATA2(9825);D21S2056E( 8568);POFUT2(23275);SEPT5(5413);TBX1(6899);LOC150223(150223);BRD1(23774);EDA(1896);FAM11A(84548 );CHM(1121); |
| 26 | ZFP41(286128) |
| 27 | PRDM16(63976) |
| 28 | CLASP1(23332) |
| 29 | ZNF655(79027) |
| 30 | FAIM2(23017) |
| 31 | ZGPAT(84619) |
| 32 | E2F2(1870);CCDC21(64793);LAPTM5(7805);ZBTB8(127557);MGC33556(339541);RAB3B(5865);CDC42SE1(56 882);UHMK1(127933);IL24(11009);TRAF5(7188);C1orf95(375057);VANGL1(81839);SFXN5(94097);CASP10(843) ;CD28(940);COL4A3(1285);FLJ10996(54520);GRM7(2917);SRGAP3(9901);PHF4(54681);RASSF1(11186);PRICK LE2(166336);ST3GAL6(10402);PCNP(57092);RNF7(9616);HIP2(3093);TMPRSS11E(28983);OSMR(9180);F2R(21 49);TICAM2(353376);SEMA6A(57556);N4BP3(23138);ELOVL2(54898);IHPK3(117283);CCND3(896);HSP90AB1( 3326);PAQR8(85315);CCNC(892);ATG5(9474);FOXO3A(2309);SLC22A3(6581);ELMO1(9844);NUDCD3(23386); CALN1(83698);BCAP29(55973);MSR1(4481);DUSP4(1846);UNC5D(137970);TACC1(6867);UBE2V2(7336);YTH DF3(253943);EXT1(2131);LRRC6(23639);ST3GAL1(6482);C8orf30A(51236);MGC21881(389741);KIAA1815(799 56);RECK(8434);SEMA4D(10507);TNFSF8(944);C10orf9(219771);ARID5B(84159);SGPL1(8879);HIF1AN(55662); FAM26C(255022);C10orf137(26098);FLJ46154(196296);TRAF6(7189);ACAT1(38);SNF1LK2(23235);CDON(509 37);KCNA6(3742);PTMS(5763);BCAT1(586);KRAS(3845);HDAC7A(51564);TEGT(7009);C12orf22(81566);ZBTB3 9(9880);FLJ11259(55332);THRAP2(23389);RFP2(10206);STRN3(29966);C14orf118(55668);UBE3A(7337);KLF13 (51621);Gcom1(145781);THSD4(79875);GRIN2A(2903);CTCF(10664);MPHOSPH6(10200);POLDIP2(26073);SAR M1(23098);NF1(4763);EIF1(10209);TMEM101(84336);PPM1D(8493);HOXB9(3219);BCL2(596);PRX(57716);ERF( 2077);GRLF1(2909);BTBD4(140685);PARD6B(84612);MAPK1(5594);CACNA1I(8911);TNRC6B(23112);SEPT3(5 5964);SSX4B(548313);PCYT1B(9468);SRPX(8406);OATL1(4943);SMC1L1(8243);SPRY3(10251); |
| 33 | ADCY1(107) |
| 34 | CNNM4(26504) |
| 35 | FUT3(2525) |
| 36 | RER1(11079);SASS6(163786);CDC14A(8556);RAP1A(5906);MCL1(4170);CKS1B(1163);F11R(50848);BRP44(25 874);DNM3(26052);RGS2(5997);MFSD4(148808);PFKFB2(5208);PROX1(5629);CCNL2(81669);MSH2(4436);SER TAD2(9792);EDAR(10913);ZC3H6(376940);WDR33(55339);MCM6(4175);CYBRD1(79901);PTD004(29789);STAT 1(6772);CASP10(843);CUL3(8452);HDAC4(9759);FLJ45964(401040);SRGAP3(9901);TMEM16K(55129);TDGF1( |

**[Table 2-6]**

| | |
|---|---|
| | 6997);APPL(26060);AER61(285203);PROK2(60675);CLDND1(56650);ACPP(55);CLSTN2(64084);XRN1(54464);S MC4L1(10051);KLHL6(89857);YEATS2(55689);SENP2(59343);TP73L(8626);MGC21675(92070);HD(3064);DKFZ p761B107(91050);IL8(3576);FRAS1(80144);PRKG2(5593);DNAJB14(79982);TIFA(92610);MAD2L1(4085);FGF2( 2247);LOC134145(134145);PRLR(5618);LIFR(3977);PLCXD3(345557);MRPS30(10884);LOC153364(153364);AP C(324);MCC(4163);SLC12A2(6558);FGF1(2246);G3BP(10146);GCNT2(2651);TCF19(6941);TNFRSF21(27242);I CK(22858);COL19A1(1310);CNR1(1268);HACE1(57531);BVES(11149);ATG5(9474);SESN1(27244);SERINC1(57 515);HEY2(23493);TNFAIP3(7128);LOC90639(90639);TRIAD3(54476);LOC90693(90693);CYCS(54205);CREB5( 9586);CDK6(1021);NAPE-PLD(222236);RELN(5649);ZC3HAV1(56829);CNOT7(29883);MTMR7(9108);ADRA1A( 148);UNC5D(137970);SPFH2(11160);BRF2(55290);UBE2V2(7336);UBE2W(55284);LRRCC1(85444);E2F5(1875); MTSS1(9788);GNAQ(2776);C9orf47(286223);CDC14B(8555);NR4A3(8013);C1QL3(389941);PARD3(56288);ZNF 365(22891);SGPL1(8879);COX15(1355);NOLC1(9221);GPR26(2849);EIF4G2(1982);DLG2(1740);CUL5(8065);PP P2R1B(5519);ARHGEF12(23365);BCL2L14(79370);ATF7IP(55729);CPNE8(144402);TMEM16F(196527);SP1(666 7);EEA1(8411);APAF1(317);HCFC2(29915);FBXO21(23014);15E1.2(283459);P2RX7(5027);TMEM132B(114795); ETNK1(55500);DIP13B(55198);LATS2(26524);TNFRSF19(55504);FLT1(2321);HSPH1(10808);FOXO1A(2308);S UGT1(10910);RAP2A(5911);FLJ10769(55739);CUL4A(8451);METTL3(56339);KIAA1443(57594);PPM1A(5494);M AP3K9(4293);C14orf43(91748);LTBP2(4053);DIO2(1734);ITPK1(3705);EIF5(1983);JAG2(3714);GOLGA8G(2837 68);TP53BP1(7158);FGF7(2252);NARG2(79664);FEM1B(10116);IREB2(3658);ABHD2(11057);GSPT1(2935);MKL 2(57496);GABARAPL2(11345);GAN(8139);CDH13(1012);MNT(4335);TRAF4(9618);BRCA1(672);NPEPPS(9520); MXRA7(439921);CARD14(79092);RAB12(201475);VAPA(9218);CEP192(55125);C18orf1(753);CTAGE1(64693); MALT1(10892);PHLPP(23239);CLEC4M(10332);GGTL3(2686);C20orf117(140710);RBL1(5933);UBE2V1(7335);C STF1(1477);CABLES2(81928);SON(6651);SLC5A3(6526);CDC45L(8318);MAPK1(5594);MN1(4330);DEPDC5(96 81);MCM5(4174);TNRC6B(23112);LOC63929(63929);SEPT3(55964);ATXN10(25814);ZBED4(9889);PRKX(5613); AGTR2(186);SEPT6(23157);ATP1B4(23439);RP6-213H19.1(51765);FMR1(2332);DKC1(1736);AFF2(2334); |
| 37 | ZKSCAN1(7586) |
| 38 | TPPP(11076) |
| 39 | SRGAP3(S901) |
| 40 | DFFA(1676) |
| 41 | LIMD1(8994) |
| 42 | FSTL3(10272) |
| 43 | NUDCD3(23386) |
| 44 | GIPC3(126326) |
| 45 | IQCE(23288) |
| 46 | CMTM4(146223) |
| 47 | LETM1(3954) |
| 48 | GMFB(2764) |
| 49 | NFIX(4784) |
| 50 | PIK3RI(5295) |
| 51 | ATXN1(6310) |
| 52 | RP5-875H10.1(389432) |
| 53 | TPI1(7167) |
| 54 | ZNF192(7745) |
| 55 | ABO(28) |
| 56 | PRX(57716) |
| 57 | PALM2(114299) |
| 58 | NFIX(4784) |

**[Table 2-7]**

| | |
|---|---|
| 59 | RBMY1A1(5940) |
| 60 | TNFRSF8(943) |
| 61 | CRTC1(23373) |
| 62 | BBS5(129880) |
| 63 | COL27A1(85301) |
| 64 | DGCR14(8220) |
| 65 | EXOSC6(118460) |
| 66 | EIF2C4(192670) |
| 67 | RAB30(27314) |
| 68 | KCNMA1(3778) |
| 69 | CCND2(894) |
| 70 | ADARB2(105) |
| 71 | FYCO1(79443) |
| 72 | ADCY7(113) |
| 73 | IGSF4D(253559) |
| 74 | GNG13(51764) |
| 75 | ID7(57089) |
| 76 | TMEM110(375346) |
| 77 | CREB3L3(84699) |
| 78 | ATXN1(6310) |
| 79 | ADARB2(105) |
| 80 | PRLR(5618) |
| 81 | ADARB2(105) |
| 82 | XAB1(11321) |
| 83 | MGAT4A(11320) |
| 84 | NBPF11(200030) |
| 85 | LIN10(80262) |
| 86 | SPOCK2(9806) |
| 87 | NTN1(9423) |
| 88 | ISG20L2(81875) |
| 89 | GGTL3(2686) |
| 90 | LRPAP1(4043) |
| 91 | GGTL3(2686) |
| 92 | IGF2(3481) |
| 93 | NEURL(9148) |
| 94 | TNRC6B(23112) |
| 95 | FN3K(64122) |
| 96 | BRWD1(54014) |
| 97 | DISC1(27185) |
| 98 | ENAH(55740) |
| 99 | GDF11(10220) |
| 100 | RAB22A(57403) |
| 101 | APOE(348) |
| 102 | PSD3(23362) |
| 103 | PSD3(23362) |
| 104 | NKTR(4820) |

**[Table 2-8]**

| | |
|---|---|
| 105 | JDP2(122953) |
| 106 | RAB40C(57799) |
| 107 | CNTN2(6900) |
| 108 | PRX(57716) |
| 109 | APOL6(80830) |
| 110 | FOXKl(221937) |
| 111 | YIPF6(286451) |
| 112 | HOXB8(3218) |
| 113 | CLN8(2055) |
| 114 | PRX(57716) |
| 115 | EVC(2121) |
| 116 | VANGL1(81839) |
| 117 | THRC6B(23112) |
| 118 | JPH4(84502) |
| 119 | PHACTR2(9749) |
| 120 | LRRC27(80313) |
| 121 | TBL1 X(6907) |
| 122 | BBS7(55212) |
| 123 | BDKRB2(624) |
| 124 | LPP(4026) |
| 125 | SAMD11(148398) |
| 126 | TNRC6B(23112) |
| 127 | ZGPAT(84619) |
| 128 | TNRC6B(23112) |
| 129 | RASSF5(83593) |
| 130 | USH1G(124590) |
| 131 | HBXAP(51773) |
| 132 | HIF3A(64344) |
| 133 | LRPAP1(4043) |
| 134 | DIAPH2(1730) |
| 135 | RAB8A(4218) |
| 136 | PRDM16(63976) |
| 137 | STX3A(6809) |
| 138 | AK1(203) |
| 139 | ENAH(55740) |
| 140 | PURB(5814) |
| 141 | MON1B(22879) |
| 142 | EVC(2121) |
| 143 | PQLC2(54896) |
| 144 | BRSK2(9024) |
| 145 | ATF11A(23250) |
| 146 | TSPAN14(81619) |
| 147 | CYP4F3(4051) |
| 148 | COPG2(26958) |
| 149 | PTPRT(11122) |
| 150 | ZNF84(7637) |

**[Table 2-10]**

| | |
|---|---|
| 151 | CACNA1A(773) |
| 152 | GIPC3(126326) |
| 153 | RAB36(9609) |
| 154 | DNM3(26052) |
| 155 | GABRA4(2557) |
| 156 | FOXK1(221937) |
| 157 | LAT(27040) |
| 158 | ABHD2(11057) |
| 159 | ENTPD7(57089) |
| 160 | ATP11A(23250) |
| 161 | CLCA1(179) |
| 162 | MARCH3(115123) |
| 163 | USH1G(124590) |
| 164 | FOXK1(221937) |
| 165 | PRSS16(10279) |
| 166 | BLR1(643) |
| 167 | ANKRD30B(374860) |
| 168 | DCX(1641) |
| 169 | CACNA1A(773) |
| 170 | KATNAL1(84056) |
| 171 | PRTG(283659) |
| 172 | ADCY7(113) |
| 173 | EIF4EBP2(1979) |
| 174 | HEXDC(284004) |
| 175 | H6PD(9563) |
| 176 | APC2(10297) |
| 177 | SF4(57794) |
| 178 | BBS5(129880) |
| 179 | TBC1D16(125058) |
| 180 | CHD5(26038) |
| 181 | CBX5(23468) |
| 182 | TGOLN2(10618) |
| 183 | ICMT(23463) |
| 184 | SELI(85465) |
| 185 | WASF2(10163) |
| 186 | TMC6(11322) |
| 187 | MUC17(140453) |
| 188 | ADCY1(107) |
| 189 | UNQ6125(442092) |
| 190 | LRRC27(80313) |
| 191 | RAB30(27314) |
| 192 | CBL(867) |
| 193 | GOLGA3(2802) |
| 194 | PTGFR(5737) |
| 195 | CDK6(1021) |
| 196 | PURB(5814) |

**[Table 2-11]**

| | |
|---|---|
| 197 | PTGFR(5737) |
| 198 | FUT4(2526) |
| 199 | LYNXI(66004) |
| 200 | SNX8(29886) |
| 201 | CDK6(1021) |
| 202 | PRKAA2(5563) |
| 203 | ADCY1(107) |
| 204 | IP09(55705) |
| 205 | TBL3(10607) |
| 206 | MLLT6(4302) |
| 207 | ANKRD30B(374860) |
| 208 | MAP6(4135) |
| 209 | CLNB(2055) |
| 210 | GRM4(2914) |
| 211 | NTRK2(4915) |
| 212 | REX01 L1 (254958) |
| 213 | EPB41 L5(57669) |
| 214 | TMED4(222068) |
| 215 | ERBB4(2066) |
| 216 | RIPK5(25778) |
| 217 | APOE(348) |
| 218 | BOK(666) |
| 219 | BAG5(9529) |
| 220 | TRAF7(84231) |
| 221 | LRRC27(80313) |
| 222 | HOXBB(3218) |
| 223 | PRKAA2(5563) |
| 224 | RAB4OC(57799) |
| 225 | AFF4(27125) |
| 226 | GIPC3(126326) |
| 227 | H-pflc(51351) |
| 228 | PIK3CA(5290) |
| 229 | LRRC14(9684) |
| 230 | NAV1(89796) |
| 231 | PRTG(283659) |
| 232 | SPRY3(10251) |
| 233 | PDE4D(5144) |
| 234 | ZNF440L(284390) |
| 235 | PSD3(23362) |
| 236 | GABRA4(2557) |
| 237 | ADCY1(107) |
| 238 | F7(2155) |
| 239 | KLF7(8609) |
| 240 | RP6-166C19.1(255313) |
| 241 | NFAT5(10725) |
| 242 | PRTG(283659) |

**[Table 2-12]**

| | |
|---|---|
| 243 | SAMD11(148398) |
| 244 | ZGPAT(84619) |
| 245 | RAB4OC(57799) |
| 246 | GRIN2A(2903) |
| 247 | LIMD1(8994) |
| 248 | TNRC6B(23112) |
| 249 | GIPC3(126326) |
| 250 | GABRA4(2557) |
| 251 | TNRC6B(23112) |
| 252 | TMED4(222068) |
| 253 | MXRA7(439921) |
| 254 | AFG3Ll (172) |
| 255 | TIYH3(80727) |
| 256 | DCP2(167227) |
| 257 | QKI(9444) |
| 258 | NFIX(4784) |
| 259 | ENAH(55740) |
| 260 | WDR37(22884) |
| 261 | NUDT3(11165) |
| 262 | KBTBD8(84541) |
| 263 | CD59(966) |
| 264 | UNQ6125(442092) |
| 265 | SH3PXD2A(9644) |
| 266 | FOXK1(221937) |
| 267 | CREB3L2(64764) |
| 268 | HOXB8(3218) |
| 269 | YPEL1(29799) |
| 270 | PRX(57716) |
| 271 | CXorf15(55787) |
| 272 | FOXK1(221937) |
| 273 | GIPC3(126326) |
| 274 | SCLY(51540) |
| 275 | RP11-114H20.1(401589) |
| 276 | SMU1(55234) |
| 277 | CALML4(91860) |
| 278 | SNX8(29886) |
| 279 | TP73L(8626) |
| 280 | ENTPD7(57089) |
| 281 | AFG3L1(172) |
| 282 | RAPH1(65059) |
| 283 | SLC36A1(206358) |
| 284 | RBJ(51277) |
| 285 | MUCDHL(53841) |
| 286 | KRTHB5(3891) |
| 287 | SEMA3E(9723) |
| 288 | WDR35(57539) |

**[Table 2-13]**

| | |
|---|---|
| 289 | SIPA1L3(23094) |
| 290 | GGTL3(2686) |
| 291 | PCYT2(5833) |
| 292 | ENAH(55740) |
| 293 | ANKK(286) |
| 294 | GRIA1(2890) |
| 295 | GIPC3(126326) |
| 296 | VAPB(9217) |
| 297 | PDPR(55066) |
| 298 | BRCC3(79184) |
| 299 | LETMI(3954) |
| 300 | SARM1(23098) |
| 301 | DCP2(167227) |
| 302 | TAGLN(6876) |
| 303 | TPPP(11076) |
| 304 | SEC14L1(6397) |
| 305 | H-plk(51351) |
| 306 | GIPC3(126326) |
| 307 | HS6ST1(9394) |
| 308 | EVC(2121) |
| 309 | SLC6A3(6531) |
| 310 | SCRT1(83482) |
| 311 | RP11-114H20.1(401589) |
| 312 | JPH4(84502) |
| 313 | CREB3L3(84699) |
| 314 | BOK(666) |
| 315 | ZFPL(162967) |
| 316 | POU3F1(5453) |
| 317 | GM632(57473) |
| 318 | ZNF213(7760) |
| 319 | PHF15(23338) |
| 320 | PRX(57716) |
| 321 | TRAF6(7189) |
| 322 | ANKRD36(375248) |
| 323 | NFATC1(4772) |
| 324 | FBXO40(51725) |
| 325 | ZGPAT(84619) |
| 326 | PDXK(8566) |
| 327 | SFRS8(6433) |
| 328 | CACNAU(8911) |
| 329 | AGPAT4(56895) |
| 330 | EVC(2121) |
| 331 | JARID1AA(5927) |
| 332 | ZNF641(121274) |
| 333 | PGAP1(80055) |
| 334 | DSC2(1824) |

**[Table 2-14]**

| | |
|---|---|
| 335 | ABHD2(11057) |
| 336 | BDKRB2(624) |
| 337 | BCAM(4059) |
| 338 | SCYL1 (57410) |
| 339 | MARCH1(55016) |
| 340 | USP31(57478) |
| 341 | GDF6(392255) |
| 342 | BCL2(596) |
| 343 | DKFZP434B0335(25851) |
| 344 | PLCXD3(345557) |
| 345 | IGF2(3481) |
| 346 | MTHFR(4524) |
| 347 | WDR33(55339) |
| 348 | KCNA7(3743) |
| 349 | ADCY1(107) |
| 350 | CHD5(26038) |
| 351 | AFF2(2334) |
| 352 | SPN(6693) |
| 353 | JARID1A(5927) |
| 354 | HIF3A(64344) |
| 355 | TMC6(11322) |
| 356 | ENTPD7(57089) |
| 357 | LTB4R2(56413) |
| 358 | ADARB2(105) |
| 359 | TRO(7216) |
| 360 | ASB1(51665) |
| 361 | MAPK1(5594) |
| 362 | NFAT5(10725) |
| 363 | EEA1(8411) |
| 364 | ZNF705CP(389631) |
| 365 | CREB5(9586) |
| 366 | EXOSC6(118460) |
| 367 | FMNL3(91010) |
| 368 | LETM1(3954) |
| 369 | GGTL3(2686) |
| 370 | CDK6(1021) |
| 371 | SBK1(388228) |
| 372 | EIF4EBP2(1979) |
| 373 | OPCML(4978) |
| 374 | MECP2(4204) |
| 375 | TNFRSF13B(23495) |
| 376 | TNRC6B(23112) |
| 377 | STX7(8417) |
| 378 | CREBK1385) |
| 379 | CBX5(23468) |
| 380 | NFIX(4784) |

**[Table 2-15]**

| | |
|---|---|
| 381 | ADARB2(105) |
| 382 | TNS1(7145) |
| 383 | SEU(85465) |
| 384 | LETM1(3954) |
| 385 | FGFRL1(53834) |
| 386 | ZNF440L(284390) |
| 387 | GABRG1(2565) |
| 388 | DECR2(26063) |
| 389 | HTRA2(27429) |
| 390 | LPAL2(80350) |
| 391 | OPCML(4978) |
| 392 | USP37(57695) |
| 393 | ITR(160897) |
| 394 | SFRS8(6433) |
| 395 | APOE(348) |
| 396 | FGD3(89846) |
| 397 | LRRC27(80313) |
| 398 | H6PD(9563) |
| 399 | ADAM22(53616) |
| 400 | LRRC27(80313) |
| 401 | CREB3L3(84699) |
| 402 | OXCT2(64064) |
| 403 | DISC1(27185) |
| 404 | NEURL(9148) |
| 405 | WDR55(54853) |
| 406 | FAMBA1(51439) |
| 407 | GABRB2(2561) |
| 408 | NELF(26012) |
| 409 | H6PD(9563) |
| 410 | TNFRSF13B(23495) |
| 411 | WNT7B(7477) |
| 412 | ADARB2(105) |
| 413 | CXorf6(10046) |
| 414 | TPPP(11076) |
| 415 | DGCR14(8220) |
| 416 | PKN3(29941) |
| 417 | LRPAPK4043) |
| 418 | CER1(9350) |
| 419 | VGLL3(389136) |
| 420 | PCDHGB4(8641) |
| 421 | RAB40C(57799) |
| 422 | YOD1(55432) |
| 423 | CREB1(1385) |
| 424 | SLC7A1(6541) |
| 425 | CHTF18(63922) |
| 426 | CALN1(83698) |

**[Table 2-16]**

| | |
|---|---|
| 427 | BSDC1(55108) |
| 428 | NUAK1(9891) |
| 429 | GIPC3(126326) |
| 430 | RASGEF18(153020) |
| 431 | MAGI1(9223) |
| 432 | BSDC1(55108) |
| 433 | VGLL3(389136) |
| 434 | NF2(4771) |
| 435 | PDXK(8566) |
| 436 | GABRG1(2565) |
| 437 | LIMD1(8994) |
| 438 | L3MBTL2(83746) |
| 439 | TPPP(11076) |
| 440 | PRKAA2(5563) |
| 441 | BRWD1(54014) |
| 442 | SEU(85465) |
| 443 | LRRC27(80313) |
| 444 | CALN1(83698) |
| 445 | GATAD2A(54815) |
| 446 | SH3BP2(6452) |
| 447 | CDR1(1038) |
| 448 | POU3F3(5455) |
| 449 | TMEM56(148534) |
| 450 | CDR1(1038) |
| 451 | ASB1(51665) |
| 452 | GPIAP1(4076) |
| 453 | VAV2(7410) |
| 454 | JARID1A(5927) |
| 455 | TRM14(9830) |
| 456 | TPPP(11076) |
| 457 | BDKRB2(624) |
| 458 | E2F2(1870) |
| 459 | APBB3(10307) |
| 460 | LRPAP1(4043) |
| 461 | RGPD2(440872) |
| 462 | PALM2(114299) |
| 463 | APPL(26060) |
| 464 | TPPP(11076) |
| 465 | GGTL3(2686) |
| 466 | RAB4OC(57799) |
| 467 | PRMT8(56341) |
| 468 | POFUT2(23275) |
| 469 | STK38L(23012) |
| 470 | BOK(666) |
| 471 | CRTC1(23373) |
| 472 | FAM20B(9917) |

**[Table 2-17]**

| | |
|---|---|
| 473 | ICMT(23463) |
| 474 | BAK1(578) |
| 475 | SLC36A4(120103) |
| 476 | SEPT5(5413) |
| 477 | EVC(2121) |
| 478 | PRTG(283659) |
| 479 | TRIM33(51592) |
| 480 | ZFP41(286128) |
| 481 | PDXK(8566) |
| 482 | TEAD1(7003) |
| 483 | ENAH(55740) |
| 484 | PLXDC1(57125) |
| 485 | PRX(57716) |
| 486 | AGPAT3(56894) |
| 487 | RAB40C(57799) |
| 488 | LRPAP1(4043) |
| 489 | PRELP(5549) |
| 490 | ZNF44(51710) |
| 491 | Gong13(51764) |
| 492 | PRKAA2(5563) |
| 493 | PRTG(283659) |
| 494 | GABRA4(2557) |
| 495 | CRTAP(10491) |
| 496 | PSD3(23362) |
| 497 | BCAT1(586) |
| 498 | RP11-308D16.4(441522) |
| 499 | LRPAP1(4043) |
| 500 | PFKFB2(5208) |
| 501 | PPM1F(9647) |
| 502 | GPR123(84435) |
| 503 | FMNL3(91010) |
| 504 | PRTG(283659) |
| 505 | GRIN2A(2903) |
| 506 | POU3F3(5455) |
| 507 | SNX27(81609) |
| 508 | BOK(666) |
| 509 | RAB8A(4218) |
| 510 | TNRC6B(23112) |
| 511 | ERBB4(2066) |
| 512 | GABRA4(2557) |
| 513 | ASB1(51665) |
| 514 | MECP2(4204) |
| 515 | ZNF264(9422) |
| 516 | ASB1(51665) |
| 517 | RBMS2(5939) |
| 518 | GRIN2A(2903) |

**[Table 2-18]**

| | |
|---|---|
| 519 | SOX11(6664) |
| 520 | KLF12(11278) |
| 521 | CBL(867) |
| 522 | PGAP1(80055) |
| 523 | DDEF2(8853) |
| 524 | MINA(84864) |
| 525 | TNRC6B(23112) |
| 526 | LRPAPI(4043) |
| 527 | AXL(558) |
| 528 | ZNF289(84364) |
| 529 | NEURL(9148) |
| 530 | FZD4(8322) |
| 531 | PRLR(5618) |
| 532 | PB1(55193) |
| 533 | ARF3(377) |
| 534 | JARID1A(5927) |
| 535 | POLR2J2(246721) |
| 536 | SYNCRIP(10492) |
| 537 | PAG1(55824) |
| 538 | EXOC5(10640) |
| 539 | DENR(8562) |
| 540 | RAB40C(57799) |
| 541 | RNF165(494470) |
| 542 | SLC38A5(92745) |
| 543 | FOXK1(221937) |
| 544 | SOCS7(30837) |
| 545 | RNF40(9810) |
| 546 | LRPAP1(4043) |
| 547 | STX7(8417) |
| 548 | EVC(2121) |
| 549 | ERGIC1(57222) |
| 550 | LPAL2(80350) |
| 551 | DDR2(4921) |
| 552 | ENAH(55740) |
| 553 | ENAH(55740) |
| 554 | FN3K(64122) |
| 555 | ZNF213(7760) |
| 556 | LIMD1(8994) |
| 557 | SNPH(9751) |
| 558 | PRX(57716) |
| 559 | SAMD11(148398) |
| 560 | FREQ(23413) |
| 561 | HNRPU(3192) |
| 562 | TMC6(11322) |
| 563 | RAB40C(57799) |
| 564 | WDR37(22884) |

**[Table 2-19]**

| | |
|---|---|
| 565 | EGFR(1956) |
| 566 | FAM9C(171484) |
| 567 | TMED4(222068) |
| 568 | FAM102B(284611) |
| 569 | GNG4(2786) |
| 570 | RP6-166C19,1(255313) |
| 571 | KSR1(8844) |
| 572 | DZlPl(22873) |
| 573 | TPPP(11076) |
| 574 | RP6-166C19.1(255313) |
| 575 | ISG20L2(81875) |
| 576 | BMPR2(659) |
| 577 | ATP9A(10079) |
| 578 | GAS7(8522) |
| 579 | ENAH(55740) |
| 580 | BTRC(8945) |
| 581 | SUPT3H(8464) |
| 582 | RPS15A(6210) |
| 583 | GATAD1(57798) |
| 584 | FOXK1(221937) |
| 585 | AP3S2(10239) |
| 586 | HEMK1(51409) |
| 587 | ARNT2(9915) |
| 588 | H6PD(9563) |
| 589 | APOE(348) |
| 590 | PRX(57716) |
| 591 | LETM1(3954) |
| 592 | CRAMP1L(57585) |
| 593 | LTB4R2(56413) |
| 594 | RASSF2(9770) |
| 595 | TPPP(11076) |
| 596 | ARGFX(503582) |
| 597 | SRGAP3(9901) |
| 598 | DUSP8(1850) |
| 599 | PRX(57716) |
| 600 | SEPT5(5413) |
| 601 | RBMS3(27303) |
| 602 | MECP2(4204) |
| 603 | SORD(6652) |
| 604 | ARFGAP1 (55738) |
| 605 | GIPC3(126326) |
| 1606 | MGAT2(4247) |
| 607 | ITPIC1(3705) |
| 608 | SPFH2(11160) |
| 609 | TNRCBB(23112) |
| 610 | CREB1(1385) |

**[Table 2-20]**

| | |
|---|---|
| 611 | HOXBB(3218) |
| 612 | GGTL3(2686) |
| 613 | RBMS3(27303) |
| 614 | PSD3(23362) |
| 615 | BACH2(60468) |
| 616 | PIP5K1C(23396) |
| 617 | MTHFR(4524) |
| 618 | RBM14(10432) |
| 619 | LRRC27(80313) |
| 620 | APC2(10297) |
| 621 | LRRC14(9684) |
| 622 | PTGFR(5737) |
| 623 | TBC1016(125058) |
| 624 | Ells1(222166) |
| 625 | ILF3(3609) |
| 626 | TNRC6B(23112) |
| 627 | RBM3(5935) |
| 628 | HCN2(610) |
| 629 | NT5DC2(64943) |
| 630 | MAPK1(5594) |
| 631 | PRX(57716) |
| 632 | AK2(204) |
| 633 | MTHFR(4524) |
| 634 | SEC24C(9632) |
| 635 | ING5(84289) |
| 636 | SCN5A(6331) |
| 637 | PTGDS(5730) |
| 638 | ZXDC(79364) |
| 639 | HIP1(3092) |
| 640 | FGFRL1(53834) |
| 641 | BIRC4(331) |
| 642 | FAN2(23017) |
| 643 | CREB3L2(64764) |
| 644 | PAX2(5076) |
| 645 | GLS2(27165) |
| 646 | PCYT2(5833) |
| 647 | SRGAP3(9901) |
| 648 | GIPC3(126326) |
| 649 | PRRT2(112476) |
| 650 | SCRT1(83482) |
| 651 | GIPC3(126326) |
| 652 | SCRT1(83482) |
| 653 | ZYG11B(79699) |
| 654 | DRCTNNB1A(84668) |
| 655 | GGTL3(2686) |
| 656 | TPPP(11076) |

**[Table 2-21]**

| | |
|---|---|
| 657 | FSTL4(23105) |
| 658 | EVC(2121) |
| 659 | BRCA1(672) |
| 660 | AXL(558) |
| 661 | H-plk(51351) |
| 662 | TMEM10(93377) |
| 663 | DISC1(27185) |
| 664 | RPE(6120) |
| 665 | EVC(2121) |
| 666 | TPPP(11076) |
| 667 | FDPS(2224) |
| 668 | ZNF721(170960) |
| 669 | WNK3(65267) |
| 670 | SLC6A3(6531) |
| 671 | RBMS3(27303) |
| 672 | TRIM14(9830) |
| 673 | CBX5(23468) |
| 674 | ZNF493(284443) |
| 675 | GABRA4(2557) |
| 676 | CACNB4(785) |
| 677 | GPR123(84435) |
| 678 | TMEM132B(114795) |
| 679 | PMS2L2(5380) |
| 680 | KLF13(51621) |
| 681 | DAPK3(1613) |
| 682 | STK17A(9263) |
| 683 | SNX8(29886) |
| 684 | KCNMA1(3778) |
| 685 | GIPC3(126326) |
| 686 | AFG3L1(172) |
| 687 | TUSC5(286753) |
| 688 | TPPP(11076) |
| 689 | FN3K(64122) |
| 690 | WARS2(10352) |
| 691 | CSNK1G1(53944) |
| 692 | SH3BP2(6452) |
| 693 | FMNL3(91010) |
| 694 | GM632(57473) |
| 695 | PALM2(114299) |
| 696 | ZFP41(286128) |
| 697 | FOXK1(221937) |
| 698 | PRKAA2(5563) |
| 699 | LRRC27(80313) |
| 700 | ZF(58487) |
| 701 | BRCA1(672) |
| 702 | MGAT4A(11320) |

**[Table 2-22]**

| | |
|---|---|
| 703 | TRIM33(51592) |
| 704 | LTBP3(4054) |
| 705 | CUGBP2(10659) |
| 706 | ZNF440L(284390) |
| 707 | ISG20L1(64782) |
| 708 | BDKRB2(624) |
| 709 | ALPK3(57538) |
| 710 | TFRC(7037) |
| 711 | PCYT2(5833) |
| 712 | YEATS2(55689) |
| 713 | FDPS(2224) |
| 714 | BOK(666) |
| 715 | BBS5(129880) |
| 716 | ZBTB7A(51341) |
| 717 | TUBB1(81027) |
| 718 | DID01(11083) |
| 719 | SEC24C(9632) |
| 720 | GNG13(51764) |
| 721 | SAMD11(148398) |
| 722 | P2RXL1(9127) |
| 723 | SNN(8303) |
| 724 | GRM4(2914) |
| 725 | HD(3064) |
| 726 | BRCA1(672) |
| 727 | PCYT2(5833) |
| 728 | FAM26C(255022) |
| 729 | BOK(666) |
| 730 | MMP24(10893) |
| 731 | ADARB2(105) |
| 732 | TTYH3(80727) |
| 733 | SYT2(127833) |
| 734 | TIBK1(84630) |
| 735 | STK1(8576) |
| 736 | LRPAP1(4043) |
| 737 | TNFRSF13B(23495) |
| 738 | SNX8(29886) |
| 739 | SPN(6693) |
| 740 | PAG1(55824) |
| 741 | CNTN2(6900) |
| 742 | BRCA1(672) |
| 743 | SOX1(6656) |
| 744 | EML3(256364) |
| 745 | TENC1(23371) |
| 746 | GIPC3(126326) |
| 747 | CYCS(54205) |
| 748 | ZFP41(286128) |

**[Table 2-23]**

| | |
|---|---|
| 749 | JMJD2B(23030) |
| 750 | ZDHHC11(79844) |
| 751 | SNXB(29886) |
| 752 | ZNF721(170960) |
| 753 | LETM1(3954) |
| 754 | LETM1(3954) |
| 755 | NUFIP2(57532) |
| 756 | CBX5(23468) |
| 757 | CDK6(1021) |
| 758 | LRRC27(80313) |
| 759 | TCF2(6928) |
| 760 | ZNF24(7572) |
| 761 | SNX27(81609) |
| 762 | ZNF468(90333) |
| 763 | HIST2H4(8370) |
| 764 | VKORC1L1(154807) |
| 765 | ABHD2(11057) |
| 766 | ZNF226(7769) |
| 767 | TSPAN18(90139) |
| 768 | MCFD2(90411) |
| 769 | FDPS(2224) |
| 770 | TNRC6B(23112) |
| 771 | FGD3(89846) |
| 772 | BOK(666) |
| 773 | STAC2(342667) |
| 774 | RAB40C(57799) |
| 775 | SAPS3(55291) |
| 776 | BCAM(4059) |
| 777 | LEPR(3953) |
| 778 | RNF12(51132) |
| 779 | MECP2(4204) |
| 780 | MAPK1(5594) |
| 781 | MAP2K1IP1(8649) |
| 782 | FAM105B(90268) |
| 783 | TMC6(11322) |
| 784 | CBX5(23468) |
| 785 | LETM1(3954) |
| 786 | N4BP3(23138) |
| 787 | POU2AF1(5450) |
| 788 | NISCH(11188) |
| 789 | GPR123(84435) |
| 790 | EVC(2121) |
| 791 | ENAH(55740) |
| 792 | TMC6(11322) |
| 793 | 8857(55212) |
| 794 | RAB40C(57799) |

**[Table 2-24]**

| | |
|---|---|
| 795 | SLC5A3(6526) |
| 796 | RNF31(55072) |
| 797 | LIFR(3977) |
| 798 | DIP(23151) |
| 799 | LEPR(3953) |
| 800 | DCP2(167227) |
| 801 | HIF3A(64344) |
| 802 | RFP2(10206) |
| 803 | JM11(90060) |
| 804 | BOK(666) |
| 805 | PRX(57716) |
| 806 | TPPP(11076) |
| 807 | PRX(57716) |
| 808 | JARID1A(5927) |
| 809 | PAPD1(55149) |
| 810 | FGFRL1(53834) |
| 811 | VAPB(9217) |
| 812 | RBM33(155435) |
| 813 | TBL3(10607) |
| 814 | GABRA4(2557) |
| 815 | ZNF721(170960) |
| 816 | PRDM10(56980) |
| 817 | APOE(348) |
| 818 | NEURL(9148) |
| 819 | KPNA1(3836) |
| 820 | LIFR(3977) |
| 821 | RAB40C(57799) |
| 822 | GRIN2A(2903) |
| x823 | FGF2(2247) |
| 824 | DIO2(1734) |
| 825 | SLC30A7(148867) |
| 826 | SNX27(81609) |
| 827 | EV15(7813) |
| 828 | LRRC27(80313) |
| 829 | PPCDC(60490) |
| 830 | ITGAM(3684) |
| 831 | RNF12(51132) |
| 832 | TSPAN18(90139) |
| 833 | LRRC14(9684) |
| 834 | ADCY1(107) |
| 835 | PAG1(55824) |
| 836 | SOX11(6664) |
| 837 | AFF2(2334) |
| 838 | FSTL3(10272) |
| 839 | DISC1(27185) |
| 840 | ZDHHC2(51201) |

**[Table 2-25]**

| | |
|---|---|
| 841 | FSTL3(10272) |
| 842 | PAG1(55824) |
| 843 | MXRA7(439921) |
| 844 | SBK1(388228) |
| 845 | APOL6(80830) |
| 846 | GAB2(9846) |
| 847 | GLS2(27165) |
| 848 | SLC7A11(23657) |
| 849 | AFF2(2334) |
| 850 | MOBKL2B(79817) |
| 851 | PSD3(23362) |
| 852 | CHML(1122) |
| 853 | TNRC6B(23112) |
| 854 | NAPE-PLD(222236) |
| 855 | SOX5(6660) |
| 856 | APOL6(80830) |
| 857 | PDXK(8566) |
| 858 | FDPS(2224) |
| 859 | CBX5(23468) |
| 860 | CBX5(23468) |
| 861 | EIF4E3(317649) |
| 862 | SNX8(29886) |
| 863 | NKTR(4820) |
| 864 | SH3BP2(6452) |
| 865 | RAB40C(57799) |
| 866 | BAIAP2(10458) |
| 867 | RAB40C(57799) |
| 868 | CALML4(91860) |
| 869 | BRWD1(54014) |
| 870 | POU3F3(5455) |
| 871 | RBBP4(5928) |
| 872 | STK35(140901) |
| 873 | FMNL3(91010) SNX1(6642) |
| 874 | |
| 875 | SNX8(29886) |
| 876 | SGCD(6444) |
| 877 | TRIM14(9830) |
| 878 | FBX028(23219) |
| 879 | QKI(9444) |
| 880 | PRR11(55771) |
| 881 | XPO(64328) |
| 882 | LIMD1(8994) |
| 883 | PNMA5(114824) |
| 884 | MECP2(4204) |
| 885 | TNRC6B(23112) |
| 886 | SLC5A3(6526) |

**[Table 2-26]**

| | |
|---|---|
| 887 | GABRA4(2557) |
| 888 | SLC1A2(6506) |
| 889 | IHPK1(9807) |
| 890 | CD93(22918) |
| 891 | PGAP1(80055) |
| 892 | DCP2(167227) |
| 893 | SELI(85465) |
| 894 | GGTL3(2686) |
| 895 | CBX5(23468) |
| 896 | LIMD1(8994) |
| 897 | PARP11(57097) |
| 898 | TNRC6B(23112) |
| 899 | H6PD(9563) |
| 900 | BRWD1(54014) |
| 901 | SLC7A11(23657) |
| 902 | TMEM110(375346) |
| 903 | PRX(57716) |
| 904 | TBC1D16(125058) |
| 905 | POU3F3(5455) |
| 906 | SPN(6693) |
| 907 | NKTR(4820) |
| 908 | GABRA4(2557) |
| 909 | PNPO(55163) |
| 910 | TFB1M(51106) |
| 911 | WNK3(65267) |
| 912 | FOXK1(221937) |
| 913 | PLCXD3(345557) |
| 914 | PODXL(5420) |
| 915 | CBX5(23468) |
| 916 | CDK6(1021) |
| 917 | FSTL3(10272) |
| 918 | NEURL(9148) |
| 919 | LRPAP1(4043) |
| 920 | LETM1(3954) |
| 921 | NKTR(4820) |
| 922 | VANGL1(81839) |
| 923 | TNRC6B(23112) |
| 924 | H6PD(9563) |
| 925 | LRAP1(4043) |
| 926 | ANKRD36(375248) |
| 927 | SEC22L3(9117) |
| 928 | RGPD5(84220) |
| 929 | CBX5(23468) |
| 930 | PACS1(55690) |
| 931 | MECP2(4204) |
| 932 | SLC1A2(6506) |

**[Table 2-27]**

| | |
|---|---|
| 933 | BDKRB2(624) |
| 934 | RAB40C(57799) |
| 935 | CBX5(23468) |
| 936 | SYNCRIP(10492) |
| 937 | GIPC3(126326) |
| 938 | TNRC6B(23112) |
| 939 | GOLGA3(2802) |
| 940 | LRRC27(80313) |
| 941 | HRB(3267) |
| 942 | WHSC1(7468) |
| 943 | RPE(6120) |
| 944 | RBL1(5933) |
| 945 | ST6GAL2(84620) |
| 946 | AFF3(3899) |
| 947 | BMPR2(659) |
| 948 | KPNA1(3836) |
| 949 | G3BP(10146) |
| 950 | G3BP(10146) |
| 951 | LRRC27(80313) |
| 952 | RP6-166C19.1(255313) |
| 953 | ZNF213(7760) |
| 954 | MBD3(53615) |
| 955 | NUDT3(11165) |
| 956 | TMC6(11322) |
| 957 | LRPAP1(4043) |
| 958 | ZNF721(170960) |
| 959 | RP6-166C19.1(255313) |
| 960 | DCP2(167227) |
| 961 | SARM1(23098) |
| 962 | ZNF510(22869) |
| 963 | CACNA2D2(9254) |
| 964 | JARID1A(5927) |
| 965 | CER1(9350) |
| 966 | SH3GLB2(56904) |
| 967 | ILF3(3609) |
| 968 | SYNGAP1(8831) |
| 969 | PEX19(5824) |
| 970 | LUZP1(7798) |
| 971 | LRRC27(80313) |
| 972 | GRHL2(79977) |
| 973 | FAM53A(152877) |
| 974 | HOXB8(3218) |
| 975 | GRIN2A(2903) |
| 976 | KIF136(23303) |
| 977 | RAI1(10743) |
| 978 | ADCY1(107) |

**[Table 2-28]**

| | |
|---|---|
| 979 | FGD3(89846) |
| 980 | BCR(613) |
| 981 | RIMS3(9783) |
| 982 | FAM53A(152877) |
| 983 | TMED4(222068) |
| 984 | SLA(6503) |
| 985 | TMTC1(83857) |
| 986 | ARLSB(221079) |
| 987 | PERQ1(64599) |
| 988 | FADS1(3992) |
| 989 | TRAM2(9697) |
| 990 | NRCAM(4897) |
| 991 | BIRC4BP(54739) |
| 992 | IQCE(23288) |
| 993 | SYNJ2BP(55333) |
| 994 | HIF3A(64344) |
| 995 | RBMS2(5939) |
| 996 | ABC1(63897) |
| 997 | ANGEL2(90806) |
| 998 | PHOX2B(8929) |
| 999 | SLC6A3(6531) |
| 1000 | ZNF490(57474) |
| 1001 | ASB1(51665) |
| 1002 | NAV1(89796) |
| 1003 | KLK4(9622) |
| 1004 | NRXN3(9369) |
| 1005 | CIITA(4261) |
| 1006 | DLGAP2(9228) |
| 1007 | LRRC27(80313) |
| 1008 | LRRC27(80313) |
| 1009 | PURA(5813) |
| 1010 | ILF3(3609) |
| 1011 | ADAM22(53616) |
| 1012 | KATNAL1(84056) |
| 1013 | GOSR1(9527) |
| 1014 | DLEC1(9940) |
| 1015 | GALNTL2(117248) |
| 1016 | CER1(9350) |
| 1017 | SBF1(6305) |
| 1018 | RNF165(494470) |
| 1019 | FOXK1(221937) |
| 1020 | LRRC27(80313) |
| 1021 | TBC1016(125058) |
| 1022 | FBX042(54455) |
| 1023 | SYT8(90019) |
| 1024 | CALD1(800) |

**[Table 2-29]**

| | |
|---|---|
| 1025 | TMEM63A(9725) |
| 1026 | MAPK1(5594) |
| 1027 | PPP1R12B(4660) |
| 1028 | LETM1(3954) |
| 1029 | PARVA(55742) |
| 1030 | ELAVL1(1994) |
| 1031 | PSCD3(9265) |
| 1032 | WHSC1(7468) |
| 1033 | TCL1A(8115) |
| 1034 | SEPT5(5413) |
| 1035 | APBB3(10307) |
| 1036 | ADM2(79924) |
| 1037 | TMEM132B(114795) |
| 1038 | ATXN1(6310) |
| 1039 | LRPAP1(4043) |
| 1040 | PALM2(114299) |
| 1041 | TBC1D16(125058) |
| 1042 | LAMP1(3916) |
| 1043 | GRIN1(2902) |
| 1044 | PLXNA1(5361) |
| 1045 | VGLL3(389136) |
| 1046 | TRAF3(7187) |
| 1047 | ZFP41(286128) |
| 1048 | CREB3L3(84699) |
| 1049 | ERBB4(2066) |
| 1050 | NAV2(89797) |
| 1051 | PDLIM7(9260) |
| 1052 | IL17RB(55540) |
| 1053 | GTDC1(79712) |
| 1054 | DNAJC18(202052) |
| 1055 | ZNF468(90333) |
| 1056 | ZDHHC11(79844) |
| 1057 | BOLA2(552900) |
| 1058 | BRWD1(54014) |
| 1059 | LRRC15(131578) |
| 1060 | ENAH(55740) |
| 1061 | STK35(140901) |
| 1062 | SUPT7L(9913) |
| 1063 | PTDSS2(81490) |
| 1064 | THSD4(79875) |
| 1065 | IGF1(3479) |
| 1066 | PAX8(7849) |
| 1067 | ENAH(55740) |
| 1068 | ZNF621(285268) |
| 1069 | AFG3L1(172) |
| 1070 | EPB41 L4B(54566) |

**[Table 2-30]**

| | |
|---|---|
| 1071 | PBX1(5087) |
| 1072 | ZNF721(170960) |
| 1073 | LRRC27(80313) |
| 1074 | ABCC3(8714) |
| 1075 | OXCT2(64064) |
| 1076 | CPLX1(10815) |
| 1077 | UBE3B(89910) |
| 1078 | INGS(84289) |
| 1079 | LRPAP1(4043) |
| 1080 | HIF3A(64344) |
| 1081 | PTGDS(5730) |
| 1082 | BOK(666) |
| 1083 | DMWD(1762) |
| 1084 | NFIX(4784) |
| 1085 | ZFP41(286128) |
| 1086 | SNX8(29886) |
| 1087 | AFG3L1(172) |
| 1088 | GIPC3(126326) |
| 1089 | SLC36A4(120103) |
| 1090 | MBNL2(10150) |
| 1091 | WDR37(22884) |
| 1092 | RAPH1(65059) |
| 1093 | SPANX-N1(494118) |
| 1094 | ITTH5(80760) |
| 1095 | PDPK1(5170) |
| 1096 | RAB40C(57799) |
| 1097 | SYT7(9066) |
| 1098 | RAB4OC(57799) |
| 1099 | WIP12(26100) |
| 1100 | ARGFX(503582) |
| 1101 | AP3S2(10239) |
| 1102 | EXOSC6(118460) |
| 1103 | MECP2(4204) |
| 1104 | PDXK(8566) |
| 1105 | TFCP2(7024) |
| 1106 | EIF3S1(8669) |
| 1107 | SNRP70(6625) |
| 1108 | ADCY1(107) |
| 1109 | PDPK1(5170) |
| 1110 | FGD3(89846) |
| 1111 | CBL(867) |
| 1112 | BOK(666) |
| 1113 | ADARB2(105) |
| 1114 | KLK9(284366) |
| 1115 | FN3K(64122) |
| 1116 | NFIX(4784) |

**[Table 2-31]**

| | |
|---|---|
| 1117 | IL17RE(132014) |
| 1118 | PIGA(5277) |
| 1119 | PRLR(5618) |
| 1120 | EVC(2121) |
| 1121 | SNX8(29886) |
| 1122 | CREB3L2(64764) |
| 1123 | KBTBD11(9920) |
| 1124 | SYNCRIP(10492) |
| 1125 | KIF1B(23095) |
| 1126 | APOL6(80830) |
| 1127 | CBX5(23468) |
| 1128 | RBMS2(5939) |
| 1129 | ZNF721(170960) |
| 1130 | PLXNA1(5361) |
| 1131 | ZNF440L(284390) |
| 1132 | APOE(348) |
| 1133 | NFATC1(4772) |
| 1134 | DISC1(27185) |
| 1135 | MAPK1(5594) |
| 1136 | POLR3H(171568) |
| 1137 | NKTR(4820) |
| 1138 | ABI2(10152) |
| 1139 | APOL6(80830) |
| 1140 | HOXA11(3207) |
| 1141 | RNF12(51132) |
| 1142 | TP53INP1(94241) |
| 1143 | CREB5(9586) |
| 1144 | TPPP(11076) |
| 1145 | H-plk(51351) |
| 1146 | ENTPD7(57089) |
| 1147 | ZNF440L(284390) |
| 1148 | AKNA(80709) |
| 1149 | DFFA(1676) |
| 1150 | FGFRL1(53834) |
| 1151 | RNF12(51132) |
| 1152 | PRX(57716) |
| 1153 | GIPC3(126326) |
| 1154 | RAB30(27314) |
| 1155 | NKTR(4820) |
| 1156 | SEPT5(5413) |
| 1157 | CD59(966) |
| 1158 | CBL(867) |
| 1159 | DDEF2(8853) |
| 1160 | LRPAP1(4043) |
| 1161 | LRPAP1(4043) |
| 1162 | JPH4(84502) |

**[Table 2-32]**

| | |
|---|---|
| 1163 | TP53INP1(94241) |
| 1164 | ELAVL1(1994) |
| 1165 | ZNF721(170960) |
| 1166 | CMTM4(146223) |
| 1167 | ERBB4(2066) |
| 1168 | GFPT1(2673) |
| 1169 | TOMM20(9804) |
| 1170 | AGPAT3(56894) |
| 1171 | GGTL3(2686) |
| 1172 | ADCY1(107) |
| 1173 | MLX(6945) |
| 1174 | AKNA(80709) |
| 1175 | ADCY1(107) |
| 1176 | OPCML(4978) |
| 1177 | NAV1(89796) |
| 1178 | RIMS3(9783) |
| 1179 | LYNX1(66004) |
| 1180 | LRRC14(9684) |
| 1181 | KCNA7(3743) |
| 1182 | TNRC6B(23112) |
| 1183 | SKIL(6498) |
| 1184 | SBK1(388228) |
| 1185 | ZNF264(9422) |
| 1186 | WDR33(55339) |
| 1187 | EXOSC6(118460) |
| 1188 | APOL6(80830) |
| 1189 | HCP1(113235) |
| 1190 | RASGRP4(115727) |
| 1191 | TFCP2L1(29842) |
| 1192 | CYP4F3(4051) |
| 1193 | BBS5(129880) |
| 1194 | ILF3(3609) |
| 1195 | LYNX1(66004) |
| 1196 | APOE(348) |
| 1197 | TBC1D16(125058) |
| 1198 | RAB40C(57799) |
| 1199 | FUT3(2525) |
| 1200 | PRX(57716) |
| 1201 | PRTG(283659) |
| 1202 | SNX27(81609) |
| 1203 | PIK3CD(5293) |
| 1204 | SLC1A2(6506) |
| 1205 | GGTL3(2686) |
| 1206 | ATP11A(23250) |
| 1207 | ZFP41(286128) |
| 1208 | SOX11(6664) |

**[Table 2-33]**

| | |
|---|---|
| 1209 | AFG3L1(172) |
| 1210 | FNDC6(152028) |
| 1211 | WNK3(65267) |
| 1212 | ZBTB7A(51341) |
| 1213 | JPH4(84502) |
| 1214 | PRX(57716) |
| 1215 | APOE(348) |
| 1216 | SGCD(6444) |
| 1217 | PRLR(5618) |
| 1218 | STOX2(56977) |
| 1219 | CBX5(23468) |
| 1220 | RNF12(51132) |
| 1221 | EVC(2121) |
| 1222 | DIDO1(11083) |
| 1223 | PRX(57716) |
| 1224 | TBC1D16(125058) |
| 1225 | CBX5(23468) |
| 1226 | SNX8(29886) |
| 1227 | AFG3L1(172) |
| 1228 | MAK10(60560) |
| 1229 | KCNK6(9424) |
| 1230 | EXOC5(10640) |
| 1231 | CDKS(1021) |
| 1232 | QSCN6(5768) |
| 1233 | EVC(2121) |
| 1234 | LRPAP1(4043) |
| 1235 | MDGA1(266727) |
| 1236 | ENTPD7(57089) |
| 1237 | CDR1(1038) |
| 1238 | ADCY1(107) |
| 1239 | CUGBP2(10659) |
| 1240 | ZC3HAV1(56829) |
| 1241 | LRPAP1(4043) |
| 1242 | LASS1(10715) |
| 1243 | FZR1(51343) |
| 1244 | CBX5(23468) |
| 1245 | LRRC14(9684) |
| 1246 | ENTPD7(57089) |
| 1247 | AFG3L1(172) |
| 1248 | DISC1(27185) |
| 1249 | TNRC6B(23112) |
| 1250 | PALM2(114299) |
| 1251 | ZNF721(170960) |
| 1252 | GIPC3(126326) |
| 1253 | CHKB(1120) |
| 1254 | PTPRT(11122) |

**[Table 2-34]**

| | |
|---|---|
| 1255 | ZNF660(285349) |
| 1256 | ADCY1(107) |
| 1257 | MECP2(4204) |
| 1258 | ZNF264(9422) |
| 1259 | PRX(57716) |
| 1260 | BOK(666) |
| 1261 | RNF12(51132) |
| 1262 | GABRA4(2557) |
| 1263 | WASF2(10163) |
| 1264 | TBC1D16(125058) |
| 1265 | RAB40C(57799) |
| 1266 | SEPT5(5413) |
| 1267 | CHES1(1112) |
| 1268 | TPPP(11076) |
| 1269 | TNRC6B(23112) |
| 1270 | ASB1(51665) |
| 1271 | AP4E1(23431) |
| 1272 | SEC14L1(6397) |
| 1273 | TNNI1(7135) |
| 1274 | POU3F3(5455) |
| 1275 | SEMA5A(9037) |
| 1276 | ABO(28) |
| 1277 | BRWD1(54014) |
| 1278 | MECP2(4204) |
| 1279 | GIPC3(126326) |
| 1280 | THUMPD1(55623) |
| 1281 | GDA(9615) |
| 1282 | FANCC(2176) |
| 1283 | AXL(558) |
| 1284 | POU2AF1(5450) |
| 1285 | PRKAA2(5563) |
| 1286 | RP6-166C19.1(255313) |
| 1287 | REEP3(221035) |
| 1288 | ADAM19(8728) |
| 1289 | TPPP(11076) |
| 1290 | KLF15(28999) |
| 1291 | VGLL4(9686) |
| 1292 | WHSC1(7468) |
| 1293 | TNRC6B(23112) |
| 1294 | TSPY2(64591) |
| 1295 | ZIC1(7545) |
| 1296 | QKI(9444) |
| 1297 | CBX5(23468) |
| 1298 | MAPK1(5594) |
| 1299 | FUT3(2525) |
| 1300 | GNG13(51764) |

**[Table 2-35]**

| | |
|---|---|
| 1301 | NKTR(4820) |
| 1302 | GOSR1 (9527) |
| 1303 | TRIM2(23321) |
| 1304 | BRWD1(54014) |
| 1305 | MECP2(4204) |
| 1306 | NF1(4763) |
| 1307 | GFPT1(2673) |
| 1308 | EVC(2121) |
| 1309 | RP11-114H20.1(401589) |
| 1310 | CPLX2(10814) |
| 1311 | RASSF6(166824) |
| 1312 | NTRK2(4915) |
| 1313 | CALML4(91860) |
| 1314 | RBM33(155435) |
| 1315 | KLK9(284366) |
| 1316 | BRCA1(672) |
| 1317 | FMNL3(91010) |
| 1318 | THRB(7068) |
| 1319 | KLF12(11278) |
| 1320 | CALN1 (83698) |
| 1321 | MAGI1(9223) |
| 1322 | TMEM132B(114795) |
| 1323 | FAM78A(286336) |
| 1324 | VGLL3(389136) |
| 1325 | CPLX2(10814) |
| 1326 | NTRK2(4915) |
| 1327 | RIMS4(140730) |
| 1328 | SPTLC2(9517) |
| 1329 | AFG3L1(172) |
| 1330 | RP11-145H9.1(340156) |
| 1331 | NDE1(54820) |
| 1332 | THRB(7068) |
| 1333 | GABRB2(2561) |
| 1334 | RCP9(27297) |
| 1335 | AFG3L1(172) |
| 1336 | PRLR(5618) |

The method of screening a substance that promotes or suppresses the expression or function of a nucleic acid such as a micro-RNA or a micro-RNA precursor using a nucleic acid of the present invention may be any method of screening for a substance that promotes or suppresses the expression or function of a nucleic acid such as a micro-RNA or a micro-RNA precursor using a nucleic acid of the present invention. For example, a method can be mentioned wherein a vector that expresses a nucleic acid, such as a micro-RNA or a micro-RNA precursor, of the present invention, is introduced into a cell, and a substance that suppresses the expression of a target gene thereof, or a substance that promotes the expression of a target gene thereof, is screened for.

Although the substance that suppresses the expression of a target gene may be any substance that suppresses the expression of the mRNA of the target gene, the substance is preferably a nucleic acid, more preferably an siRNA against the target gene. Although the substance that promotes the expression of a target gene may be any substance that promotes the expression of the mRNA of the target gene, the substance is preferably a nucleic acid, more preferably an siRNA against a micro-RNA that suppresses the expression of the target gene.

The cell incorporating a nucleic acid or vector of the present invention may be any cell incorporating the nucleic acid or vector of the present invention introduced in vitro. Specifically, mast cells and mast cell precursor cells, or, mesenchymal stem cells and cells resulting from differentiation of mesenchymal stem cells, for example, cells that are present in tissues such as the skin, lung, small intestine, nose, tonsil, blepharal conjunctiva, vascular walls, and bone marrow, or cells that are present in tissues such as bone marrow, fat tissue, umbilical blood, endometrium, dermis, skeletal muscles, periosteum, dental follicles, periodontal membranes, dental pulps, and dental germs, for example, osteoblasts, adipocytes, and muscle cells and the like, can be mentioned.

In the present invention, a mast cell refers to a cell that becomes activated by various stimuli to undergo degranulation and release or produce many inflammatory mediators, and is involved in the pathogenesis of various allergic diseases.
In the present invention, a mesenchymal stem cell refers to a cell that is present in mesenchymal tissues such as bone marrow, fat tissue, umbilical blood, endometrium, dermis, skeletal muscles, periosteum, dental follicles, periodontal membranes, dental pulps, and dental germs, and has the potential for differentiating at least into mesenchymal cells such as osteoblasts, adipocytes, and muscle cells.

A pharmaceutical with a nucleic acid of the present invention as an active ingredient can be used to diagnose or treat a disease caused by a mast cell abnormality. Because mast cell abnormalities include degranulation abnormalities, a nucleic acid of the present invention can also be used as a mast cell degranulation promoter or degranulation suppressant. A substance that promotes or suppresses the expression or function of a nucleic acid, such as a micro-RNA or a micro-RNA precursor, of the present invention, and a substance that suppresses or promotes the expression of a target gene of a nucleic acid, such as a micro-RNA, of the present invention, can also be used to diagnose or treat a disease caused by a mast cell abnormality. These substances can also be used as mast cell degranulation promoters or degranulation suppressants.

As diseases caused by a mast cell abnormality, specifically, atopic dermatitis, asthma, chronic obstructive lung disease, and allergic diseases and the like can be mentioned.
A pharmaceutical with a nucleic acid of the present invention as an active ingredient can be used to diagnose or treat a disease caused by an abnormality of mesenchymal stem cell proliferation and/or differentiation. A substance that promotes or suppresses the expression or function of a nucleic acid, such as a micro-RNA or a micro-RNA precursor, of the present invention, and a substance that suppresses or promotes the expression of a target gene of a nucleic acid, such as a micro-RNA, of the present invention, can also be used to diagnose or treat a disease caused by an abnormality of mesenchymal stem cell proliferation and/or differentiation.

As diseases caused by an abnormality of mesenchymal stem cell proliferation and/or differentiation, specifically, cancers and dysosteogenesis, achondroplasia, diabetes and the like can be mentioned.
A pharmaceutical with a nucleic acid of the present invention as an active ingredient can be used to diagnose or treat a disease caused by an abnormality of cell proliferation and the like, tissue hyperplasia and the like. A nucleic acid of the present invention can also be used as a cell proliferation suppressant or proliferation promoter. A substance that promotes or suppresses the expression or function of a nucleic acid, such as a micro-RNA or a micro-RNA precursor, of the present invention, and a substance that suppresses or promotes the expression of a target gene of a nucleic acid, such as a micro-RNA, of the present invention, can also be used to diagnose or treat a disease caused by an abnormality of cell proliferation and the like, tissue hyperplasia and the like. These substances can also be used as cell proliferation suppressants or proliferation promoters. Here, an abnormality of cell proliferation refers to a condition wherein cells are proliferating at a rate that is not a normal proliferation rate in a living organism.

As diseases caused by a cell proliferation abnormality, tissue hyperplasia and the like, specifically, cancers, arteriosclerosis, rheumatoid arthritis, prostatic hyperplasia, blood vessel restenosis after percutaneous transvascular coronary angioplasty, fibroid lung, glomerulonephritis, autoimmune diseases and the like can be mentioned.
Hereinafter, the present invention is described in detail with reference to cases wherein the nucleic acid of the present invention is micro-RNA or micro-RNA precursor.

### 1. Identification of micro-RNAs and micro-RNA precursors expressed in mast cells

### (1-1) Acquirement and cultivation of mast cells

The method of acquiring human mast cells is not particularly limited, as far as it ensures safe and efficient acquirement; for example, human mast cells can be prepared from the human lung, skin, fetal liver and the like by known methods [J. Immunol. Methods, 169, 153 (1994); J. Immunol., 138, 861 (1987); J. Allergy Clin. Immunol., 107, 322 (2001); J. Immunol. Methods., 240, 101 (2000)]. Human mast cells can also be prepared by culturing mononuclear cells prepared from human umbilical blood, peripheral blood, bone marrow, lung or skin in the presence of stem cell factor (hereinafter also referred to as SCF) to differentiate them into mast cells in accordance with known methods [J. Immunol., 157, 343, (1996); Blood, 91, 187 (1998); J. Allergy Clin. Immunol., 106, 141 (2000); Blood, 97, 1016 (2001); Blood, 98, 1127 (2001); Blood, 100, 3861 (2002); Blood, 97, 2045 (2001)].

A cell line established from a human mast cell can also be used. As a human mast cell line, LAD2, which is known to well retain the nature of human mast cells [Leuk. Res., 27, 671 (2003); Leuk. Res., 27, 677 (2003)] and the like can be mentioned.

### (1-2) Acquirement of low-molecular RNA and sequence information

Total RNA is extracted from mast cells acquired by the various methods described above, and using the RNA, a low-molecular RNA comprising a micro-RNA expressed in mast cells can be acquired as described below.

As a method of acquiring a low-molecular RNA, specifically, a method wherein separation and cutting out of a low-molecular RNA by 15% polyacrylamide gel electrophoresis, 5' terminal dephosphorylation, 3'-adapter ligation, phosphorylation, 5'-adapter ligation, reverse transcription, PCR amplification, concatemerization, and ligation to a vector are performed sequentially, thereafter the low-molecular RNA is cloned, and the nucleotide sequence of the clone is determined, as described in Genes & Development 15, 188-200 (2000), and the like can be mentioned. Alternatively, for example, a method wherein separation and cutting off of a low-molecular RNA by 15% polyacrylamide gel electrophoresis, 5'-adenylation 3'-adapter ligation, 5'-adapter ligation, reverse transcription, PCR amplification, concatemerization, and ligation to a vector are performed sequentially, thereafter the low-molecular RNA is cloned, and the nucleotide sequence of the clone is determined, as described in Science 294, 858-862 (2001), and the like can be mentioned.

Alternatively, separation and cutting off of a low-molecular RNA by 15% polyacrylamide gel electrophoresis, 5' terminal dephosphorylation, 3'-adapter ligation, phosphorylation, 5'-adapter ligation, reverse transcription, PCR amplification, and ligation to a microbead vector are performed sequentially, thereafter the low-molecular RNA is cloned, and the nucleotide sequence of the microbeads is read to determine the nucleotide sequence, whereby the low-molecular RNA can also be acquired, as described in Nucleic Acids Research 34, 1765-1771 (2006).

A low-molecular RNA can also be acquired using a small RNA Cloning Kit (manufactured by Takara Bio Inc.).

### (1-3) Identification of micro-RNA

Whether or not the low-molecular RNA sequence acquired is a micro-RNA can be determined on the basis of whether or not the criteria described in RNA, 9, 277-279 (2003) are met. For example, in cases where the low-molecular RNA was acquired and the nucleotide sequence thereof was determined by a method described above, this can be performed as described below.

Specifically, a surrounding genome sequence wherein a DNA sequence corresponding to the nucleotide sequence of the low-molecular RNA acquired is extended by about 50 nt toward the 5' terminal side and the 3' terminal side, respectively, is acquired, and the secondary structure of the RNA expected to be transcribed from the genome sequence is predicted. If the result shows that a hairpin structure is present and the nucleotide sequence of the low-molecular RNA is located in one chain of the hairpin, the low-molecular RNA can be judged to be a micro-RNA. Genome sequences are open to the general public, and are available from, for example, UCSC Genome Bioinformatics (http://genome.ucsc.edu/). For prediction of secondary structures, various programs are open; for example, RNAfold [Nucleic Acids Research 31, 3429-3431 (2003)], Mfold [Nucleic Acids Research 31, 3406-3415 (2003)] and the like can be used. Existing micro-RNA sequences are registered in a database called miRBase (http://microrna.sanger.ac.uk/); whether or not a micro-RNA is identical to an existing micro-RNA can be determined on the basis of whether or not the sequence thereof is identical to one of the sequences listed therein.

As examples of the thus-identified micro-RNA expressed in mast cells, a nucleic acid having the nucleotide sequence of any one of SEQ ID NOs:1 to 1336 can be mentioned.
A genome sequence corresponding to the micro-RNA identified and the genome sequence of another organism may be compared, and a nucleic acid having a nucleotide sequence having an identity of 60% or more to the nucleotide sequence of any of SEQ ID NOs:1 to 1336, preferably a nucleic acid having a nucleotide sequence having an identify of 90% or more and more preferably 95% or more, can be identified as a micro-RNA in the organism.

### (1-4) Identification of micro-RNA precursor

On the basis of the nucleotide sequence of the micro-RNAs identified in (1-3) above, a sequence comprising the sequence that encodes a micro-RNA can be identified as the sequence that encodes a micro-RNA precursor. As examples of a micro-RNA precursor of the present invention expressed in mast cells, a nucleic acid having the nucleotide sequence of any one of SEQ ID NOs:1337 to 2851 and the like can be mentioned.

Furthermore, a genome sequence corresponding to the micro-RNA precursor identified and the genome sequence of another organism may be compared, and a nucleic acid having a nucleotide sequence having an identity of 80% or more, preferably 90% or more, and more preferably 95% or more, and still more preferably 97% or more, to the nucleotide sequence of any one of SEQ ID NOs:1337 to 2851, can be identified as a micro-RNA precursor in the organism.

### 2. Synthesis of nucleic acid

After a micro-RNA and a micro-RNA precursor expressed in mast cells are once identified, as described in 1 above, not only an RNA, which is a polymer of a ribonucleotide, but also a DNA, which is a polymer of a deoxyribonucleotide, can be synthesized on the basis of the nucleotide sequences. For example, on the basis of the nucleotide sequence of the RNA identified in 1 above, the nucleotide sequence of a DNA can be determined. The nucleotide sequence of a DNA corresponding to the nucleotide sequence of an RNA can be determined, without exception, by reading the U (uracil) contained in the sequence of the RNA as T (thymine). A polymer being a mixture of a ribonucleotide and a deoxyribonucleotide and a polymer comprising a nucleotide analogue can also be synthesized in the same manner.

The method of synthesizing a nucleic acid such as micro-RNA or micro-RNA precursor of the present invention is not particularly limited; the same can be produced by a method using a known chemical synthesis, or an enzymatic transcription method and the like. As methods using a known chemical synthesis, the phosphoroamidite method, the phosphorothioate method, the phosphotriester method, the CEM method [Nucleic Acid Research, 35, 3287 (2007)] and the like can be mentioned; for example, a nucleic acid such as micro-RNA or micro-RNA precursor of the present invention can be synthesized using the ABI3900 high throughput nucleic acid synthesizer (manufactured by Applied Biosystems). As an enzymatic transcription method, transcription with a plasmid or DNA having a desired nucleotide sequence as the template using a typical phage RNA polymerase, for example, T7, T3, or SP6RNA polymerase, can be mentioned.

### 3. Method of detecting functions of micro-RNA and precursor of the micro-RNA

A micro-RNA is produced via processing of a micro-RNA precursor having a hairpin structure by a protein called Dicer, a kind of RNaseIII endonuclease, in cytoplasm, and suppresses the translation of an mRNA having a target nucleotide sequence. Therefore, whether or not the nucleic acid obtained is a micro-RNA can be determined on the basis of whether or not the function is present.

For example, on the basis of whether or not the RNA undergoes processing by RNaseIII endonuclease, whether or not the same functions as a micro-RNA precursor can be measured. Specifically, a single-stranded RNA whose function is to be detected is reacted with RNaseIII endonuclease, and the reaction product is electrophoresed; if a function as a micro-RNA precursor is possessed, a band about 20 to 25 nucleotides long resulting from the processing will be detected, whereby the RNA is judged to possess a function as a micro-RNA. Although an RNaseIII endonuclease is not particularly limited, as far as it possesses an activity to process the micro-RNA precursor, it is preferable to use a Dicer protein. Specifically, si-RNAse III^{™} (manufactured by Takara Bio Inc.), Cold Shock-DICER (manufactured by Takara Bio Inc.), Recombinant Dicer Enzyme (manufactured by Stratagene), BLOCK-iT Dicer RNAi Transfection Kit (manufactured by Invitrogen), X-treme GENE siRNA Dicer Kit (manufactured by Roche-Applied Science) and the like can be used, and the measurement can be made according to the reaction conditions given in the attached instructions.

As a method of detecting a function of a micro-RNA, a method can be mentioned wherein the function is measured on the basis of whether or not the translation of a mRNA having a target nucleotide sequence is suppressed.
Micro-RNAs are known to suppress the translation of an mRNA comprising a target nucleotide sequence thereof in the untranslated region on the 3' side (3'UTR) [Current Biology, 15, R458-R460 (2005)]. Hence, a DNA wherein a target nucleotide sequence for the single-stranded RNA to be measured is inserted into the 3'UTR of an appropriate reporter gene expression vector is prepared and introduced into a host cell suitable for the expression vector, and the expression of the reporter gene is measured when the cell is allowed to express the single-stranded RNA, whereby whether or not a function of a micro-RNA is possessed can be detected.

The reporter gene expression vector may be any one, as far as it has a promoter upstream of a reporter gene, and is capable of expressing the reporter gene in the host cell. Any reporter gene can be used; for example, the firefly luciferase gene, the Renilla luciferase gene, the chloramphenicol acetyltransferase gene, the β-glucuronidase gene, the β-galactosidase gene, the β-lactamase gene, the aequorin gene, the green fluorescent protein gene, the DsRed fluorescent gene and the like can be utilized. As examples of reporter gene expression vectors having these properties, psiCHECK-1 (manufactured by Promega), psiCHECK-2 (manufactured by Promega), pGL3-Control (manufactured by Promega), pGL4 (manufactured by Promega), pRNAi-GL (manufactured by Takara Bio Inc.), pCMV-DsRed-Express (manufactured by CLONTECH) and the like can be mentioned. An RNA can be expressed by the method described in 6 below.

A function of a micro-RNA can be detected, specifically as described below. First, a host cell is cultured on a multiwell plate or the like, and a reporter gene expression vector having a target nucleotide sequence and an RNA are expressed. Thereafter, reporter activity is measured, and a reduction in the reporter activity is detected when the RNA is expressed compared with the RNA being not expressed, whereby a function of the micro-RNA can be detected.

### 4. Method of detecting the expression of a nucleic acid such as a micro-RNA or a micro-RNA precursor using a nucleic acid of the present invention

Hereinafter, methods of detecting the expression of a nucleic acid such as a micro-RNA or a precursor thereof using a nucleic acid of the present invention are described.

As examples of methods of detecting the expression of a micro-RNA, a micro-RNA precursor and the like, (1) Northern hybridization, (2) dot blot hybridization, (3) in situ hybridization, (4) quantitative PCR, (5) differential hybridization, (6) microarray, (7) ribonuclease protection assay and the like can be mentioned.

The Northern blot method is a method wherein a sample-derived RNA is separated by gel electrophoresis, then transferred to a support such as a nylon filter, and an appropriately-labeled probe is prepared on the basis of the nucleotide sequence of a nucleic acid of the present invention, and hybridization and washing are performed, whereby a band specifically bound to the nucleic acid of the present invention is detected; specifically, for example, this method can be performed as described in Science 294, 853-858 (2001) and the like.

A labeled probe can be prepared by incorporating a radioisotope, biotin, digoxigenin, a fluorescent group, a chemiluminescent group and the like in a DNA, RNA, or LNA and the like having a sequence complementary to the nucleotide sequence of a nucleic acid of the present invention by a method, for example, nick translation, random priming or 5'-terminal phosphorylation. Because the amount of labeled probe bound reflects the expression level of a nucleic acid such as a micro-RNA or a micro-RNA precursor, the expression level of a micro-RNA, micro-RNA precursor or the like can be quantified by quantifying the amount of labeled probe bound. Electrophoresis, membrane transfer, probe preparation, hybridization, and nucleic acid detection can be achieved by a method described in Molecular Cloning, 3rd edition, and Cold Spring Harbor Laboratory Press, NY, USA (2001).

Dot blot hybridization is a method whereina nucleic acid extracted from a tissue or a cell is spotted in dot forms and immobilized on a membrane, and hybridized with a probe, and a nucleic acid that specifically hybridizes with the probe is detected. The probe used may be the same as that used for Northern hybridization. A nucleic acid preparation, spotting, hybridization, and detection can be achieved by a method described in Molecular Cloning, 3rd edition.

In situ hybridization is a method wherein a paraffin-embedded or cryostat-treated section of a tissue acquired from a living organism, or a cell fixed, is used as a sample and subjected to steps for hybridization with a labeled probe and washing, and the distribution and localization of a micro-RNA, micro-RNA precursor and the like in the tissue or cell are examined by microscopic examination [Methods in Enzymology, 254, 419 (1995)]. The probe used may be the same as that used for Northern hybridization. Specifically, a micro-RNA, micro-RNA precursor and the like can be detected in accordance with a method described in Nature Method 3, 27 (2006).

In quantitative PCR, a cDNA synthesized from a sample-derived RNA using a primer for reverse transcription and a reverse transcriptase (hereunder, this cDNA is referred to as a sample-derived cDNA) is used for the measurement. As a primer for reverse transcription to be supplied for cDNA synthesis, a random primer or a specific RT primer and the like can be used. A specific RT primer refers to a primer having a sequence complementary to a nucleotide sequence corresponding to a micro-RNA, micro-RNA precursor of the present invention and the like, and a genome sequence therearound.

For example, a sample-derived cDNA is synthesized, after which a PCR is performed with this cDNA as the template, using a template-specific primer designed from a nucleotide sequence corresponding to a micro-RNA, micro-RNA precursor and a genome sequence therearound, or from a nucleotide sequence corresponding to a primer for reverse transcription, to amplify a cDNA fragment and the amount of the micro-RNA and micro-RNA precursor contained in the sample-derived RNA is detected from the number of cycles for reach to a given amount of the fragment. As the template-specific primer, an appropriate region corresponding to a micro-RNA, micro-RNA precursor and a genome sequence therearound is selected, and a pair of a DNA or LNA consisting of a sequence of 20 to 40 nucleotides at the 5' terminus of the nucleotide sequence of the region, and a DNA or LNA consisting of a sequence complementary to a sequence of 20 to 40 nucleotides at the 3' terminus can be used. Specifically, this can be performed in accordance with a method described in Nucleic Acids Research, 32, e43 (2004) and the like.

Alternatively, as the primer for reverse transcription to be supplied for cDNA synthesis, a specific RT primer having a stem-loop structure can also be used. Specifically, this can be performed using a method described in Nucleic Acid Research, 33, e179 (2005), or TaqMan MicroRNA Assays (manufactured by Applied Biosystems).
As another method of synthesizing a sample-derived cDNA, a polyA sequence is added to a sample-derived RNA by means of polyA polymerase, and a nucleotide sequence comprising an oligodT sequence is used as a primer for reverse transcription, whereby a reverse transcription reaction can be performed. Specifically, this can be performed using the miScript System (manufactured by QIAGEN) or the QuantiMir RT Kit (manufactured by System Biosciences).

In addition, by hybridizing a sample-derived cDNA to a substrate such as a filter, glass slide, or silicone having a DNA or LNA corresponding to a nucleotide sequence comprising at least one or more of a nucleic acid such as micro-RNA, micro-RNA precursor of the present invention and the like immobilized thereon, and performing washing, a change in the amount of the micro-RNA, micro-RNA precursor of the present invention and the like can be detected. As such methods based on hybridization, methods using differential hybridization [Trends Genet., 7, 314 (1991)] or a microarray [Genome Res., 6, 639 (1996)] can be mentioned. Both methods enable accurate detection of a difference in the amount of a micro-RNA, a micro-RNA precursor or the like between a control sample and a target sample by immobilizing an internal control, such as a nucleotide sequence corresponding to U6 RNA, on a filter or a substrate. Also, by synthesizing labeled cDNAs using differently labeled dNTPs (mixtures of dATP, dGTP, dCTP, and dTTP) on the basis of RNAs derived from a control sample and a target sample, and simultaneously hybridizing the two labeled cDNAs to a single filter or a single substrate, accurate quantitation of the micro-RNA, micro-RNA precursor and the like can be performed. Furthermore, quantitation of a micro-RNA, a micro-RNA precursor or the like can also be performed by directly labeling and hybridizing an RNA derived from a control sample and/or a target sample. For example, a micro-RNA or the like can be detected using microarrays described in Proc. Natl. Acad. Sci. USA, 101, 9740-9744 (2004), Nucleic Acid Research, 32, e188 (2004) and the like. Specifically, a micro-RNA or the like can be detected or quantified using mirVana miRNA Bioarray (manufactured by Ambion).

In ribonuclease protection assay, first, a promoter sequence such as the T7 promoter or the SP6 promoter is bound to the 3' terminus of a nucleotide sequence corresponding to a micro-RNA, micro-RNA precursor of the present invention or a genome sequence therearound, and a labeled antisense RNA is synthesized with an in vitro transcription system using a labeled NTP (a mixture of ATP, GTP, CTP, and UTP) and an RNA polymerase. The labeled antisense RNA is bound to a sample-derived RNA to form an RNA-RNA hybrid, after which the hybrid is digested with ribonuclease A, which degrades single-stranded RNAs only. The digest is subjected to gel electrophoresis to detect or quantify an RNA fragment protected against the digestion by forming the RNA-RNA hybrid, as a micro-RNA or micro-RNA precursor. Specifically, the fragment can be detected or quantified a micro-RNA and the like using the mirVana miRNA Detection Kit (manufactured by Ambion).

### 5. Method of detecting mutations of a nucleic acid such as micro-RNA, micro-RNA precursor and the like using a nucleic acid of the present invention

Hereinafter, methods of detecting mutations of a nucleic acid such as a micro-RNA or a micro-RNA precursor using a nucleic acid of the present invention are described.

As a method of detecting mutations of a micro-RNA, micro-RNA precursor and the like, a method can be used wherein a heteroduplex formed by hybridization of the normal form of the micro-RNA and a mutated form of the micro-RNA, or of the normal form of the micro-RNA precursor and a mutated form of the micro-RNA precursor, is detected.
As methods of detecting a heteroduplex, (1) detection of a heteroduplex by polyacrylamide gel electrophoresis [Trends genet., 7, 5 (1991)], (2) single-strand conformation polymorphism analysis [Genomics, 16, 325-332 (1993)], (3) chemical cleavage of mismatches (CCM) [Human Genetics (1996), Tom Strachan and Andrew P. Read, BIOS Scientific Publishers Limited], (4) enzymatic cleavage of mismatches [Nature Genetics, 9, 103-104 (1996)], (5) denatured gel electrophoresis [Mutat. Res., 288, 103-112 (1993)] and the like can be mentioned.

Detection of a heteroduplex by polyacrylamide gel electrophoresis is, for example, performed as described below. First, with a sample-derived DNA or a sample-derived cDNA as the template, and using a primer designed on the basis of a genome nucleotide sequence comprising the nucleotide sequence of a micro-RNA and micro-RNA precursor of the present invention and the like, a fragment smaller than 200 bp is amplified. Heteroduplexs, if formed, are slower in mobility than mutation-free homo-double-strands, and can be detected as extra bands. Better separation is achieved using a custom-made gel (Hydro-link, MDE and the like). In the case of search for a fragment smaller than 200 bp, insertions, deletions, and most single-nucleotide substitutions can be detected. It is desirable that heteroduplex analysis be performed using a single gel in combination with the single strand conformation analysis described below.

In single strand conformation polymorphism analysis (SSCP analysis), a DNA amplified as a fragment smaller than 200 bp with a sample-derived DNA or sample-derived cDNA as the template, using a primer designed on the basis of a genome nucleotide sequence comprising the nucleotide sequence of a micro-RNA, micro-RNA precursor and the like, is denatured, after which it is electrophoresed in non-denatured polyacrylamide gel. By labeling the primer with an isotope or a fluorescent dye at the time of DNA amplification, or by silver-staining the non-labeled amplification product, the amplified DNA can be detected as a band. To clarify a difference from the wild type pattern, a control sample may be electrophoresed simultaneously, whereby a fragment with a mutation can be detected on the basis of a difference in mobility.

In chemical cleavage of mismatches (CCM method), a DNA fragment amplified with a sample-derived DNA or sample-derived cDNA as the template, using a primer designed on the basis of a genome nucleotide sequence comprising the nucleotide sequence of a micro-RNA, micro-RNA precursor and the like, is hybridized to a labeled nucleic acid prepared by allowing a nucleic acid of the present invention to incorporate an isotope or a fluorescent target, and treated with osmium tetraoxide to cleave one strand of the DNA at the mismatched portion, whereby a mutation can be detected. CCM is one of the most sensitive methods of detection, and can be applied to samples of kilobase length.

In place of osmium tetraoxide used above, T4 phage resolvase and an enzyme involved in mismatch repair in cells, such as endonuclease VII, and RNaseA may be used in combination to enzymatically cleave a mismatch.
In denaturing gradient gel electrophoresis (DGGE method), a DNA amplified with a sample-derived DNA or sample-derived cDNA as the template, using a primer designed on the basis of a genome nucleotide sequence comprising the nucleotide sequence of a micro-RNA, micro-RNA precursor and the like, is electrophoresed using a gel having a chemical denaturant density gradient or a temperature gradient. The DNA fragment amplified migrates in the gel to a position where it denatures to a single strand, and no longer migrates after the denaturation. Because the migration of the amplified DNA in the gel differs between the presence and absence of a mutation, the presence of the mutation can be detected. To increase the detection sensitivity, the addition of a poly (G:C) end to each primer is effective.

By directly determining and analyzing the nucleotide sequence of a sample-derived DNA or sample-derived cDNA, a mutation of a micro-RNA, micro-RNA precursor and the like can also be detected.

### 6. Method of expressing a nucleic acid such as micro-RNA or micro-RNA precursor of the present invention, and the like

A nucleic acid of the present invention, such as a micro-RNA or a micro-RNA precursor, can be expressed by using an expression vector that encodes the nucleic acid.

As an expression vector, a plasmid, viral vector or the like capable of self-replication in the host cell, or capable of being incorporated in the chromosome, that comprises a promoter at a position enabling the transcription of the gene of a nucleic acid, such as a micro-RNA or a micro-RNA precursor, of the present invention of the present invention is preferably used. The promoter may be any one, as far as it is capable of expressing in the host cell; for example, a RNA polymerase II (pol II) system promoter, a RNA polymerase III (pol III) system promoter being a U6 RNA and H1 RNA transcription system and the like can be mentioned. As examples of pol II system promoters, the promoter of the cytomegalovirus (human CMV) IE (immediate early) gene, the early promoter of SV40 and the like can be mentioned. As examples of expression vectors using them, pCDNA6.2-GW/miR (manufactured by Invitrogen), pSilencer 4.1-CMV (manufactured by Ambion) and the like can be mentioned. As pol III system promoters, U6 RNA, H1 RNA or tRNA promoters can be mentioned. As examples of expression vectors using them, pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned. When a micro-RNA, a micro-RNA precursor or the like is to be expressed in vitro, an expression vector having the T7 promoter, the T3 promoter or the SP6 promoter is preferably used. As examples of vectors having these promoters, pBluescript II SK(+) (manufactured by Stratagene) and the like can be mentioned.

When a plasmid is used, by preparing a DNA fragment comprising a hairpin portion on the basis of the nucleotide sequence of a micro-RNA, a micro-RNA precursor or the like of the present invention, or the nucleotide sequence of a genome comprising the foregoing nucleotide sequence, and inserting the fragment downstream of a promoter of the plasmid to construct a recombinant plasmid, and then introducing the plasmid into a host cell suitable for the plasmid, or mixing the plasmid with RNA polymerase, a nucleotide or the like in vitro, a nucleic acid, such as a micro-RNA or a micro-RNA precursor, of the present invention, can be expressed.

When a viral vector is used, by inserting a gene comprising the nucleotide sequence of a nucleic acid, such as a micro-RNA or a micro-RNA precursor, of the present invention, downstream of a promoter in the viral vector to construct a recombinant viral vector, and introducing the vector into a packaging cell to produce a recombinant virus, the gene of the nucleic acid such as the micro-RNA or micro-RNA precursor can be expressed.

The packaging cell may be any cell, as far as it is capable of supplementing a recombinant viral vector deficient in any one of the genes that encode the proteins necessary for the packaging of the virus with the lacked protein; for example, human kidney-derived HEK293 cells, mouse fibroblasts NIH3T3-derived cells and the like can be used. As the protein supplemented by the packaging cell, in the case of a retrovirus vector, proteins derived from a mouse retrovirus, such as gag, pol, and env, can be used; in the case of a lentivirus vector, proteins derived from a HIV virus, such as gag, pol, env, vpr, vpu, vif, tat, rev, and nef, can be used; in the case of an adenovirus vector, proteins derived from an adenovirus, such as E1A and E1B, can be used; in the case of an adeno-associated viral vector, proteins such as Rep (p5, p19, p40) and Vp(Cap), can be used.

In addition to using an expression vector, a nucleic acid such as micro-RNA and micro-RNA precursor of the present invention and the like can also be introduced directly into a cell, without using a vector. As the nucleic acid used in this technique, a DNA, an RNA, or a nucleotide analogue, as well as a chimeric molecule thereof, or a derivative of the nucleic acid can also be used. Specifically, Pre-miR^{™} miRNA Precursor Molecules (manufactured by Ambion) and miRIDIAN microRNA Mimics (manufactured by GE Healthcare) can be used.

### 7. Methods of promoting or suppressing or promoting the expression or function of a nucleic acid such as a micro-RNA or micro-RNA precursor of the present invention

The expression or function of a nucleic acid such as micro-RNA and micro-RNA precursor of the present invention and the like can be suppressed using an antisense technology [Baiosaiensu To Indasutorii, 50, 322 (1992); Kagaku, 46, 681 (1991), Biotechnology, 9, 358 (1992), Trends in Biotechnology, 10, 87 (1992), Trends in Biotechnology, 10, 152 (1992); SAIBO KOGAKU, 16, 1463 (1997)], triple helix technology [Trends in Biotechnology, 10, 132 (1992)], ribozyme technology [Current Opinion in Chemical Biology, 3, 274 (1999), FEMS Microbiology Reviews, 23, 257 (1999), Frontiers in Bioscience, 4, D497 (1999), Chemistry & Biology, 6, R33 (1999), Nucleic Acids Research, 26, 5237 (1998), Trends In Biotechnology, 16, 438 (1998)], decoy DNA method [Nippon Rinsho - Japanese Journal of Clinical Medicine, 56, 563 (1998), Circulation Research, 82, 1023 (1998), Experimental Nephrology, 5, 429 (1997), Nippon Rinsho - Japanese Journal of Clinical Medicine, 54, 2583 (1996)], or a siRNA (short interfering RNA).

An antisense refers to one that allows nucleotide sequence-specific hybridization of a nucleic acid having a nucleotide sequence complementary to a certain target nucleic acid to suppress the expression or function of the target nucleic acid. As the nucleic acid used as the antisense, a DNA, an RNA or a nucleotide analogue, as well as a chimeric molecule thereof, or a derivative of the nucleic acid can also be used. Specifically, an antisense can be prepared by following the method described in Nature 432, 226 (2004) and the like, and the expression or function can be suppressed. Specifically, by using Anti-miR^{™} miRNA Inhibitors (manufactured by Ambion) or miRIDIAN microRNA Inhibitors (manufactured by GE Healthcare), the expression or function of a nucleic acid, such as a micro-RNA or a micro-RNA precursor, of the present invention, can be suppressed.

An siRNA refers to a short double-stranded RNA comprising the nucleotide sequence of a certain target nucleic acid, that is capable of suppressing the expression or function of the target nucleic acid by RNA interference (RNAi). The sequence of an siRNA can be designed as appropriate from the target nucleotide sequence on the basis of conditions shown in the literature [Genes Dev., 13, 3191 (1999)]. By synthesizing two RNAs having a sequence of 17 to 30 nucleotides, preferably 18 to 25 nucleotides, more preferably 19 to 23 nucleotides, selected and a complementary sequence, with TT added to the 3' terminus of each thereof, using a nucleic acid synthesizer, and performing annealing, an siRNA can be prepared. By inserting a DNA corresponding to the above-described selected sequence of 17 to 30 nucleotides, preferably 18 to 25 nucleotides, more preferably 19 to 23 nucleotides, into an siRNA expression vector such as pSilencer 1.0-U6 (manufactured by Ambion) or pSUPER (OligoEngine), a vector that expresses an siRNA capable of suppressing the expression or function of the gene can also be prepared.

Using an antisense or siRNA specific for a micro-RNA or micro-RNA precursor expressed in mast cells, mesenchymal stem cells or cancer cells, the expression or function of a micro-RNA or micro-RNA precursor expressed in mast cells, mesenchymal stem cells or cancer cells can be suppressed. Specifically, by administering the antisense or siRNA specific for a micro-RNA, the expression or function of the micro-RNA can be suppressed, and the action of the micro-RNA or micro-RNA precursor in mast cells, mesenchymal stem cells or cancer cells can be controlled.

Referring to a specific example, an antisense or siRNA specific for a micro-RNA of any one of SEQ ID NOs:1, 2, 3, 8, 14, 20, 22, 25, 32 and 36 or a micro-RNA precursor of any one of SEQ ID NOs:1337, 1338, 1339, 1352, 1363, 1371, 1373, 1377, 1386 and 1390 can be used as a mast cell degranulation suppressant. An antisense or siRNA specific for a micro-RNA of SEQ ID NOs:1, 8, 21 and 36 or a micro-RNA precursor of any one of SEQ ID NOs:1337, 1352, 1372 and 1390 can be used as a mesenchymal stem cell proliferation suppressant or proliferation promoter. Furthermore, an antisense or siRNA specific for a micro-RNA of any one of SEQ ID NOs:1, 3, 8, 20, 21, 22, 32 and 36 or a micro-RNA precursor of any one of SEQ ID NOs:1337, 1339, 1352, 1371, 1372, 1373, 1386 and 1390 can be used as a cell proliferation promoter.

In the case of a patient affected by an abnormality of the expression of a micro-RNA or micro-RNA precursor expressed in mast cells, mesenchymal stem cells or cancer cells, by administering an antisense or siRNA specific for the micro-RNA or precursor thereof to the patient, it is possible to control a function of mast cells, mesenchymal stem cells or cancer cells to treat a disease that develops as a result of the above-described expressional abnormality. Hence, an antisense or siRNA that is specific for the micro-RNA or precursor thereof is useful as a therapeutic agent for a disease caused by an abnormality of mast cells or mesenchymal stem cells, or a disease caused by a cell proliferation abnormality.

When an antisense or siRNA that is specific for the micro-RNA or precursor thereof is used as the above-described therapeutic agent, the antisense or siRNA, alone or after being inserted into an appropriate vector such as a retrovirus vector, adenovirus vector, or adeno-associated viral vector, can be administered in the form of a pharmaceutical preparation prepared by the conventional method described in 11 below.
Meanwhile, as substances that promote the expression or function of a nucleic acid, such as a micro-RNA or a micro-RNA precursor, of the present invention, the micro-RNA and the micro-RNA precursor, a micro-RNA and micro-RNA precursor that share the same target nucleotide sequence therewith, and an expression vector that encodes the same, as well as factors involved in the processing of micro-RNA precursors, such as Dicer, can be mentioned. An expression vector that encodes a micro-RNA, micro-RNA precursor or the like can be produced by the methods mentioned in the foregoing 6.

Using a micro-RNA or micro-RNA precursor expressed in mast cells, mesenchymal stem cells or cancer cells, a micro-RNA or micro-RNA precursor that shares the same target nucleotide sequence therewith, and an expression vector that encodes the same, the expression or function of a micro-RNA or micro-RNA precursor expressed in mast cells, mesenchymal stem cells or cancer cells can be promoted. Hence, by administering them, it is possible to promote the expression or function of the micro-RNA to control the action of micro-RNA and micro-RNA precursor in mast cells, mesenchymal stem cells or cancer cells.

Referring to specific examples, the micro-RNA of any one of SEQ ID NOs:1, 2, 3, 8, 14, 20, 22, 25, 32 and 36 or the micro-RNA precursor of any one of SEQ ID NOs:1337, 1338, 1339, 1352, 1363, 1371, 1373, 1377, 1386 and 1390, a micro-RNA or micro-RNA precursor that shares the same target nucleotide sequence therewith, and an expression vector that encodes the same can be used as mast cell degranulation promoters. The micro-RNA of any one of SEQ ID NOs:1, 8, 21 and 36 or the micro-RNA precursor of any one of SEQ ID NOs:1337, 1352, 1372 and 1390, a micro-RNA or micro-RNA precursor that shares the same target nucleotide sequence therewith, and an expression vector that encodes the same can be used as proliferation promoters or proliferation suppressants of mesenchymal stem cells. Furthermore, the micro-RNA of any one of SEQ ID NOs:1, 3, 8, 20, 21, 22, 32 and 36 or the micro-RNA precursor of any one of SEQ ID NOs:1337, 1339, 1352, 1371, 1372, 1373, 1386 and 1390, a micro-RNA or micro-RNA precursor that shares the same target nucleotide sequence therewith, and an expression vector that encodes the same can be used as cell proliferation suppressants.

In the case of a patient affected by an abnormality of the expression of a micro-RNA or micro-RNA precursor expressed in mast cells, mesenchymal stem cells or cancer cells, by administering the micro-RNA, precursor thereof, an expression vector that encodes the same, a micro-RNA or micro-RNA precursor that shares the same target nucleotide sequence therewith, and an expression vector that encodes the same to the patient, it is possible to control a function of mast cells, mesenchymal stem cells or cancer cells to treat the above-described disease that develops as a result of an expressional abnormality. Hence, the micro-RNA or precursor thereof, an expression vector that encodes the same, a micro-RNA or micro-RNA precursor that shares the same target nucleotide sequence therewith, and an expression vector that encodes the same are useful as therapeutic agents for a disease caused by an abnormality of mast cells or mesenchymal stem cells, or a disease caused by a cell proliferation abnormality.

When the micro-RNA or a precursor thereof, and an expression vector that encodes the same are used as the above-described therapeutic agent, the micro-RNA or precursor thereof, and the expression vector that encodes the same, alone or using an appropriate vector such as a retrovirus vector, adenovirus vector, or adeno-associated viral vector, can be administered in the form of a pharmaceutical preparation prepared by the conventional method described in 11 below.

### 8. Methods of suppressing or promoting the expression of a target gene of a nucleic acid, such as a micro-RNA, of the present invention

The method of suppressing the expression of a target gene of a nucleic acid, such as a micro-RNA, of the present invention, may be any method, as far as the expression of the target gene is suppressed. For example, a method can be mentioned wherein a nucleic acid, such as a micro-RNA or a micro-RNA precursor, of the present invention, is expressed or administered to increase the amount of micro-RNA in the cell, whereby the expression of an mRNA having the target sequence is suppressed to suppress the expression of the target gene. Here, a nucleic acid, such as a micro-RNA or a micro-RNA precursor, of the present invention, can be expressed by the methods described in 6 above.

The method of promoting the expression of a target gene of a nucleic acid, such as a micro-RNA, of the present invention, may be any method, as far as the expression of the target gene is promoted. For example, a method can be mentioned wherein an antisense or siRNA against a nucleic acid, such as a micro-RNA or a micro-RNA precursor, of the present invention, is expressed or administered to suppress the expression or function of the micro-RNA, micro-RNA precursor and the like, whereby the expression of the target gene is promoted. The antisense and siRNA can be prepared by the methods described in 7 above.

As examples of a target gene of a micro-RNA consisting of the nucleotide sequence of any one of SEQ ID NOs:1 to 1336, the above-described gene cluster shown in Table 2 can be mentioned.

### 9. Method of separating cells using a nucleic acid such as micro-RNA and micro-RNA precursor of the present invention, and the like

As a method of separating cells that express a nucleic acid such as the micro-RNA and micro-RNA precursor of the present invention, and the like from various cells taken out from a living organism, a probe prepared by fluorescently labeling a nucleic acid having a nucleotide sequence complementary to the nucleotide sequence of a nucleic acid such as micro-RNA, micro-RNA precursor and the like is introduced to a cell to hybridize the same to the nucleic acid such as micro-RNA, micro-RNA precursor and the like, and only the cells that have hybridized with the labeled probe are separated using a flow cytometer with a sorting function.

The fluorescently labeled probe may be any one, as far as it emits specific fluorescence upon hybridization; for example, a molecular beacon [Biochimica et Biophysica Acta 1479, 178 (1998)], FRET [Proc.Natl.Acad.Sci. USA 103, 263 (2006)] and the like can be mentioned.
A molecular beacon is a nucleic acid wherein a fluorescent functional group has been introduced via a covalent bond at one end thereof, and a dabsyl group and the like that cause fluorescence quenching has been introduced at the other end, and the nucleotide sequence thereof is designed to assume a hairpin structure in an ordinary aqueous solution. Because the fluorescent functional group and the fluorescence quenching group introduced to both ends are adjacent to each other, no fluorescence is observed with the probe alone, but if the probe is hybridized with a nucleic acid such as micro-RNA and micro-RNA precursor of the present invention, and the like, the fluorescent functional group and the fluorescence quenching group are disjoined, and intense fluorescence is observable.

FRET is the phenomenon in which molecule excitation energy transfer occurs between two kinds or more of fluorescent functional groups. Specifically, two nucleic acid probes are provided: a nucleic acid probe incorporating an energy donor at one end thereof and another nucleic acid probe incorporating an energy receptor at the other end. If the nucleotide sequences of the two probes are designed to allow the two fluorescent functional groups, introduced after hybridization with a nucleic acid such as micro-RNA, micro-RNA precursor and the like, to come into close contact with each other, only emission from the donor is observed with the probe alone, but if the probes are hybridized with a nucleic acid such as the micro-RNA and micro-RNA precursor of the present invention, and the like, the two probes come in close contact with each other, the energy of the donor transfers to the acceptor, and emission based on the acceptor is mainly observable.

As methods of cell separation using a flow cytometer with a sorting function, the water charge method, the cell capture method and the like can be mentioned (Huro Saitometa Jiyu Jizai, p14-23, SHUJUNSHA, 1999). In both methods, cell fluorometry is performed and fluorescence intensity is converted to electrical signals to quantify fluorescence intensity, whereby cells can be separated according to the amount quantified. Specifically, fluorescence intensity can be measured using the BD FACS Aria cell sorter (manufactured by Becton Dickinson Immunocytometry Systems), EPICS ALTRA HyPerSort (manufactured by Beckman Coulter, K.K.) and the like, and the cells can be separated.

### 10. Method of screening for a substance that promotes or suppresses the expression or function of a nucleic acid such as a micro-RNA or micro-RNA precursor of the present invention

Using a nucleic acid, such as a micro-RNA or a micro-RNA precursor, of the present invention, a substance that promotes or suppresses the expression or function of the micro-RNA or precursor thereof can be screened for. For example, from among the nucleotide sequences of micro-RNAs and micro-RNA precursors of the present invention, a nucleotide sequence to be targeted for the screening is chosen, and by means of a cell that expresses a nucleic acid having the nucleotide sequence, a substance that promotes or suppresses the expression or function of the chosen micro-RNA or precursor thereof can be screened for.

As cells that express a nucleic acid having the nucleotide sequence of a micro-RNA or micro-RNA precursor, used for screening, mast cells, mesenchymal stem cells or cancer cells, as well as transformant cells obtained by introducing a vector that expresses a nucleic acid having the nucleotide sequence into a host cell such as an animal cell or yeast, cells incorporating a nucleic acid having the nucleotide sequence introduced directly without using a vector and the like as described in 6 above can also be used.

As specific methods of screening, (a) a method wherein a change in the expression level of a micro-RNA or precursor thereof being a target for screening is used as an index, as well as (b) a method wherein a change in the expression level of an mRNA having a target sequence of a micro-RNA or precursor thereof being a target for screening is used as an index, since a micro-RNA suppresses the translation of an mRNA having a target sequence, can be mentioned.
(a) Screening method wherein a change in the expression level of a micro-RNA or precursor thereof being a target for screening is used as an index
   A test substance is brought into contact with a cell that expresses a nucleic acid having the nucleotide sequence, and with a change in the expression level of the nucleic acid selected as an index, a substance that promotes or suppresses the expression of a micro-RNA and precursor thereof is obtained. The expression level of a nucleic acid can be detected by the method described in 4 above.
(b) Screening method wherein a change in the expression level of an mRNA having a target sequence of a micro-RNA or precursor thereof being a target for screening is used as an index
   A test substance is brought into contact with a cell that expresses a nucleic acid having the nucleotide sequence, and with a change in the expression level of an mRNA having a target sequence of the nucleic acid selected as an index, a substance that promotes or suppresses the expression or function of a micro-RNA and a precursor thereof is obtained. Alternatively, a DNA incorporating a target sequence for a single-stranded RNA having a nucleotide sequence of the present invention introduced into the 3' UTR of an appropriate reporter gene expression vector is prepared and introduced into a host cell suitable for the expression vector, a test substance is brought into contact with the cell, and with a change in the expression level of the reporter gene as an index, a substance that promotes or suppresses the expression or function of a micro-RNA and precursor thereof is obtained.

A target sequence can be selected by the method described above; as examples of a target gene of a micro-RNA consisting of the nucleotide sequence of any one of SEQ ID NOs:1 to 1336, the above-described gene cluster shown in Table 2 can be mentioned.

### 11. Diagnostic reagents and therapeutic agents comprising a nucleic acid of the present invention and the like

A nucleic acid, such as a micro-RNA or a micro-RNA precursor, of the present invention, and a nucleic acid having a nucleotide sequence complementary to the nucleotide sequence thereof can be utilized as a therapeutic agent for a disease caused by an abnormality of mast cells or mesenchymal stem cells and the like, or a disease caused by a cell proliferation abnormality, by controlling the expression of a target gene thereof or a nucleic acid of the present invention. A nucleic acid of the present invention can also be utilized as a diagnostic reagent for a disease caused by an abnormality of mast cells or mesenchymal stem cells and the like, or a disease caused by a cell proliferation abnormality, by quantifying, or detecting a mutation of, a nucleic acid, such as a micro-RNA or a micro-RNA, of the present invention.

As mast cell abnormalities, abnormalities of mast cell differentiation and degranulation, inflammatory mediator production, cytokine production, chemokine production and the like can be mentioned; as diseases caused thereby, atopic dermatitis, asthma, chronic obstructive lung disease, allergic disease and the like can be mentioned. As abnormalities of mesenchymal stem cells, an abnormality of proliferation or differentiation and the like can be mentioned; as diseases caused thereby, cancers, dysosteogenesis, achondroplasia, diabetes and the like can be mentioned. As diseases caused by a cell proliferation abnormality, cancers and diseases caused by abnormal proliferation of cells, tissue hyperplasia and the like, such as arteriosclerosis, rheumatoid arthritis, prostatic hyperplasia, blood vessel restenosis after percutaneous transvascular coronary angioplasty, fibroid lung, glomerulonephritis, and autoimmune diseases can be mentioned.

Referring to specific examples of therapeutic agents or diagnostic reagents, the micro-RNA of any one of SEQ ID NOs:1 to 1336 or the micro-RNA precursor of any one of SEQ ID NOs:1337 to 2851, and a nucleic acid having a nucleotide sequence complementary to the nucleotide sequence thereof can be used as a diagnostic reagent or a therapeutic agent for a disease caused by a mast cell abnormality. The micro-RNA of any one of SEQ ID NOs:1, 8, 21 and 36 or the micro-RNA precursor of any one of SEQ ID NOs:1337, 1352, 1372 and 1390, and a nucleic acid having a nucleotide sequence complementary to the nucleotide sequence thereof can be used as a diagnostic reagent or a therapeutic agent for a disease caused by an abnormality of mesenchymal stem cell proliferation and/or differentiation. Furthermore, the micro-RNA of any one of SEQ ID NOs:1, 3, 8, 20, 21, 22, 32 and 36 or the micro-RNA precursor of any one of SEQ ID NOs:1337, 1339, 1352, 1371, 1372, 1373, 1386 and 1390, and a nucleic acid having a nucleotide sequence complementary to the nucleotide sequence thereof can be used as a diagnostic reagent or a therapeutic agent for a disease caused by a cell proliferation abnormality.

A diagnostic reagent comprising a nucleic acid of the present invention, according to the desired diagnostic method, may comprise reagents necessary for quantitation or detection of a mutation of a nucleic acid such as micro-RNA and micro-RNA precursor of the present invention, and the like, for example, buffering agents, salts, reaction enzymes, labeled proteins that bind to a nucleic acid such as micro-RNA and micro-RNA precursor of the present invention, and the like, and a color developer for detection and the like.
Although a pharmaceutical containing a nucleic acid of the present invention or a nucleic acid, having a nucleotide sequence complementary to the nucleotide sequence thereof as an active ingredient can be administered alone, the same is normally desirably administered as a pharmaceutical preparation produced by an optionally chosen method known well in the technical field of pharmaceutical making with one or more pharmacologically acceptable carriers blended therein.

The route of administration used is desirably the most effective one in treatment; oral administration, or parenteral administration such as intraoral administration, airway administration, intrarectal administration, subcutaneous administration, intramuscular administration and intravenous administration can be mentioned, and desirably intravenous administration can be mentioned.

As dosage forms, sprays, capsules, tablets, granules, syrups, emulsions, suppositories, injection formulations, ointments, tapes and the like can be mentioned.
As preparations appropriate for oral administration, emulsions, syrups, capsules, tablets, powders, granules and the like can be mentioned.
Liquid preparations like emulsions and syrups can be produced using water, saccharides such as sucrose, sorbitol, and fructose, glycols such as polyethylene glycol and propylene glycol, oils such as sesame oil, olive oil, and soybean oil, antiseptics such as p-hydroxybenzoic acid esters, flavors such as strawberry flavor and peppermint and the like as additives.

Capsules, tablets, powders, granules and the like can be produced using excipients such as lactose, glucose, sucrose, and mannitol, disintegrants such as starch and sodium alginate, lubricants such as magnesium stearate and talc, binders such as polyvinyl alcohol, hydroxypropylcellulose, and gelatin, surfactants such as fatty acid esters, plasticizers such as glycerin and the like as additives.

As appropriate preparations for parenteral administration, injection formulations, suppositories, sprays and the like can be mentioned.
An injection formulation is prepared using a carrier consisting of a salt solution, a glucose solution or a mixture of both and the like. A suppository is prepared using a carrier such as cacao butter, hydrogenated fat or carboxylic acid. A spray is prepared using a carrier that does not stimulate the recipient's oral cavity and airway mucosa, and that disperses the active ingredients as fine particles to facilitate the absorption thereof, and the like.

As examples of the carrier, specifically, lactose, glycerin and the like can be exemplified. Depending on the nature of the nucleic acid of the present invention, and of the carrier used, preparations such as aerosols and dry powders are possible. In these parenteral preparations, components exemplified as additives for oral preparations can be added.
The dose or frequency of administration varies depending on desired therapeutic effect, method of administration, duration of treatment, age, body weight and the like, and is normally 10 µg/kg to 20 mg/kg per day for an adult.

A therapeutic agent comprising a nucleic acid of the present invention or a nucleic acid having a nucleotide sequence complementary to the nucleotide sequence thereof as an active ingredient can also be produced by blending a vector that expresses the nucleic acids and a base used in a nucleic acid therapeutic agent [Nature Genet., 8, 42(1994)].
The base used in the nucleic acid therapeutic agent may be any base for ordinary use in injection formulations; distilled water, solutions of salts such as sodium chloride or a mixture of sodium chloride and an inorganic salt, solutions of mannitol, lactose, dextran, and glucose, solutions of amino acids such as glycine and arginine, mixed solutions of organic acid solutions or salt solutions and glucose solution and the like can be mentioned. In accordance with a conventional method, using auxiliary agents such as an osmotic pressure regulator, a pH regulator, a vegetable oil such as sesame oil or soybean oil, lecithin, and a surfactant in these bases, an injection formulation may be prepared as a solution, suspension, or dispersion. These injection formulations can also be prepared as preparations for dissolution before use, by procedures such as powdering and lyophilization. A therapeutic agent of the present invention can be used for treatment as is in the case of a liquid, or after being dissolved in a base described above, optionally sterilized, in the case of a solid, just before treatment.

As a vector encoding a nucleic acid of the present invention, the recombinant viral vector prepared in 6 above can be mentioned, more specifically, retrovirus vector and lentivirus vector and the like can be mentioned.
For example, by combining a vector encoding a nucleic acid of the present invention with a polylysine-conjugated antibody that is specific for adenovirus hexon protein to prepare a complex, and binding the complex obtained to an adenovirus vector, a viral vector can be prepared. The viral vector is capable of stably reaching the desired cell, being incorporated into cells by endosome, being decomposed in the cells, and efficiently expressing the nucleic acid.

A viral vector based on Sendai virus, which is a (-) strand RNA virus, has been developed (WO97/16538, WO97/16539), and a Sendai virus incorporating a nucleic acid such as micro-RNA and micro-RNA precursor of the present invention and the like can be prepared using the Sendai virus.
A nucleic acid of the present invention and a vector encoding the same can also be migrated by a non-viral nucleic acid migration method. The same can be migrated by, for example, calcium phosphate co-precipitation [Virology, 52, 456-467 (1973); Science, 209, 1414-1422 (1980)], microinjection method [Proc.Natl.Acad.Sci. USA, 77, 5399-5403 (1980); Proc.Natl.Acad.Sci. USA, 77, 7380-7384 (1980); Cell, 27, 223-231 (1981); Nature, 294, 92-94 (1981)], membrane fusion-mediated migration mediated by liposome [Proc.Natl.Acad.Sci. USA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)] or direct DNA uptake and receptor-mediated DNA migration method [Science, 247, 1465-1468 (1990); J. Biol. Chem., 266, 14338-14342 (1991); Proc.Natl.Acad.Sci. USA, 87, 3655-3659 (1991); J. Biol. Chem., 264, 16985-16987 (1989); BioTechniques, 11, 474-485 (1991); Proc.Natl.Acad.Sci. USA, 87, 3410-3414 (1990); Proc.Natl.Acad.Sci. USA, 88, 4255-4259 (1991); Proc.Natl.Acad.Sci. USA, 87, 4033-4037 (1990); Proc.Natl.Acad.Sci. USA, 88, 8850-8854 (1991); Hum. Gene Ther., 3, 147-154 (1991)] and the like.

Membrane fusion-mediated migration mediated by liposome allows a nucleic acid of the present invention and a vector encoding the same to be incorporated locally in the tissue, and to be expressed, by administering a liposome preparation directly to the target tissue [Hum. Gene Ther., 3, 399 (1992)]. For direct targeting of a DNA to a focus, direct DNA uptake technology is preferable.
For receptor-mediated DNA transfer, for example, a method performed by binding a DNA (usually assuming the form of a covalently cyclized supercoiled plasmid) to a protein ligand via polylysine can be mentioned. A ligand is chosen on the basis of the presence of a corresponding ligand receptor on the cell surface of the desired cell or tissue. The ligand-DNA conjugate can be injected directly into a blood vessel as desired, and can be directed to a target tissue wherein receptor binding and DNA-protein complex internalization occur. To prevent the destruction of the DNA in a cell, an adenovirus may be infected simultaneously to destroy the endosome function.

### 12. Methods of measuring the degree of mast cell activation

The fact that a certain nucleic acid exhibits at least one of the actions to suppress activation, suppress degranulation, suppress inflammatory mediator production, suppress cytokine production and suppress chemokine production on mast cells can be confirmed by, for example, introducing a nucleic acid, such as a micro-RNA or a micro-RNA, of the present invention, or an antisense or siRNA against a target gene of the micro-RNA, into mast cells, and culturing the cells with the addition of IgE, thereafter activating human mast cells by the addition of an anti-IgE antibody and the like, measuring a released substance such as (i) histamine or β-hexosaminidase, which can serve as an index of degranulation, (ii) an inflammatory mediator such as LTC4, LTD4, LTE4, or PGD2, (iii) a cytokine such as TNF-α or GM-CSF, (iv) a chemokine such as IL-8, I-309, or MIP-1α, or the like, and comparing the result with that obtained when nothing is introduced. As described above, mast cell activation can also be examined by measuring, in place of degranulation, production of cytokines such as TNF-α and GM-CSF, production of chemokines such as IL-8, I-309, and MIP-1α, production of inflammatory mediators such as LTC4, LTD4, LTE4, and PGD2, and the like [Blood 100, 3861(2002)].

The fact that a nucleic acid, such as a micro-RNA or a micro-RNA precursor, of the present invention, or an antisense, siRNA or the like against a target gene of the micro-RNA possess apoptosis inducing action can be detected by introducing them into mast cells, and performing a measurement of the fragmentation of chromatin DNA or a measurement by the TUNEL method and the like.

### 13. Methods of proliferation, differentiation into osteoblasts and evaluation of mesenchymal stem cells

The method of acquiring mesenchymal stem cells is not particularly limited, as far as it ensures safe and efficient acquirement; as an example of a method of acquirement from human bone marrow, the method described in S. E. Haynesworth et al. Bone, 13, 81 (1992) can be mentioned.

In the sternal bone or iliac bone, the skin at the site for bone marrow paracentesis is disinfected, and particularly the subperiosteal region is fully anesthetized topically. The inner cylinder of the bone marrow paracentesis needle is removed, a 10-ml syringe containing 5,000 units of heparin is attached, and a necessary volume, usually 10 ml to 20 ml, of bone marrow fluid is aspirated. The bone marrow paracentesis needle is detached, and compressive hemostasis is performed for about 10 minutes. The bone marrow fluid acquired is centrifuged at 1,000×g, and bone marrow cells are recovered, thereafter the bone marrow cells are washed with phosphate buffer solution (phosphate-buffered saline) (PBS). After centrifugation and washing are repeated in 2 cycles, the bone marrow cells are suspended in a medium for cell culture such as α-MEM (α-modified MEM), DMEM (Dulbecco's modified MEM) or IMDM (Isocove's modified Dulbecco's medium), containing 10% fetal bovine serum (FBS), whereby a bone marrow cell fluid is obtained. The method of isolating mesenchymal stem cells from the bone marrow cell fluid is not particularly limited, as far as it allows the removal of other cells that are also present in the bone marrow cell fluid, for example, corpuscular cells, hematopoietic stem cells, vascular stem cells, fibroblasts and the like; for example, the method described in M.F. Pittenger et al. Science, 284, 143-147 (1999) can be mentioned. The bone marrow cell fluid is overlaid on Percoll having a density of 1.073 g/ml, and then centrifuged at 1,100×g for 30 minutes, whereby the cells at the interface can be isolated as mesenchymal stem cells. Also, an equal volume of Percoll diluted to 9/10 by the addition of a 10-fold concentration of PBS is added to, and mixed with, the bone marrow cell fluid, after which the mixture is centrifuged at 20,000×g for 30 minutes, whereby the cells in a fraction having a density of 1.075 to 1.060 can be isolated as mesenchymal stem cells.

Mesenchymal stem cells derived from human bone marrow can also be purchased from Cambrex and Takara Bio Inc.
As an example of a method of acquiring mesenchymal stem cells from the umbilical cord, the method described in Stem Cells, 21, 105-110 (2003) can be mentioned. A cannula is inserted into each end of the umbilical vein, which is washed with an appropriate buffer solution, for example, EBSS (Earle's balanced salt solution). An antibiotic is added to a 199 medium containing a protease, for example, 0.1% collagenase, and this is injected into the blood vessel, and incubated at 4 to 40°C, preferably at 37°C, for 1 to 60 minutes. The blood vessel is washed with EBSS, and the umbilical cord is gently massaged, after which a suspension of endothelial cells and subendothelial cells is recovered. The suspension is centrifuged at 600xg for 10 minutes, and the cells obtained are suspended in, for example, a medium prepared by adding 20 mM HEPES, 100 units/ml penicillin, 100 µg/ml streptomycin, 2 mM L-glutamine, 1 mM sodium pyruvate and 10% FBS to a DMEM medium containing a low concentration of glucose (DMEM-LG, Gibco). The cell density is adjusted to 10² to 10⁶ cells/cm², and the cells are inoculated to a culture flask and cultured under the conditions of 37°C and 5% CO₂. While renewing the medium every 1 to 7 days, the cultivation is continued for 1 to 3 weeks, whereby mesenchymal stem cells can be acquired.

As an example of a method of acquiring mesenchymal stem cells from the endometrium, the method described in Am. J. Pathol., 163, 2259-2269 (2003) can be mentioned. Human endometrial tissue extirpated by surgical operation is shredded, and cultured with a medium that allows cell culture, preferably a medium prepared by adding 1 to 20% animal-derived serum, preferably 5 to 10% FBS, to α-MEM, DMEM, IMDM and the like. The medium may be supplemented with antibiotics such as penicillin and streptomycin. Furthermore, to facilitate cell separation, a collagen-decomposing enzyme such as type 3 collagenase and a DNase such as deoxyribonuclease I are added to the medium, and the medium is gently shaken at 20 to 40°C, preferably at 37°C, for 10 minutes to 5 hours, preferably for 1 hour. Each endometrial gland is separated while examining microscopically, and cultured in an appropriate culture vessel, for example, a 24-well culture dish, under the conditions of 37°C and 5% CO₂ whereby mesenchymal stem cells can be acquired.

As an example of a method of acquiring mesenchymal stem cells from a tooth, a dental germ, or periodontal tissue, the methods described in Lancet, 364, 149-155(2004), Proc. Natl. Acad. Sci. USA, 97, 13625-13630 (2000) can be mentioned. The human tooth used may be any of deciduous teeth and permanent teeth such as incisor teeth, canine teeth, premolar teeth, and molar teeth. For example, the periodontal ligament is carefully separated from the surface of the root of a third molar (wisdom tooth) extracted, and a digestive reaction is carried out using a protease such as collagenase, trypsin, pronase, elastase, dispase, or hyaluronidase at 37°C for 1 hour. The tissue residue is removed using a strainer, a mesh, a filter and the like, whereby mesenchymal stem cells can be acquired. Mesenchymal stem cells can also be acquired by washing the surface of an extracted third molar with PBS and the like, thereafter cutting the joint of the cement and the enamel to expose the pulp, carefully separating the dental pulp tissue from the dental crown and root, and treating the dental pulp tissue with a protease as described above, thereafter removing the tissue residue.

As methods of mesenchymal stem cell isolation other than those described above, methods can be mentioned wherein mesenchymal stem cells are isolated using a surface antigen expressed in mesenchymal stem cells or a reporter vector having a promoter and enhancer of a gene that is specific for mesenchymal stem cells. Specifically, a method of isolating stem cells using the AC133 antigen (US6468794), methods of isolating mesenchymal stem cells using a reporter vector having a promoter and enhancer of the Sox gene (US2002/0135539), the Nestin gene or the Musashi gene (JP-A-2002-034580), and the like can be mentioned.

Stem cells can also be separated using a method wherein FACS fractionation with the potential for extracellular discharge of Hoechst33342 as an index is used to concentrate stem cells in a side population (SP) [Journal of Experimental Medicine, 183, 1797-806 (1996)]. A method wherein SH2-positive, SH4-positive, CD29-positive, CD44-positive, CD71-positive, CD90-positive, CD106-positive, CD120a-positive, CD124-positive, CD14-negative, CD34-negative, and CD45-negative cells are separated as mesenchymal stem cells using a cell sorter or magnetic beads [Science, 284, 143-147 (1999)] can also be used.

As examples of media used to culture mesenchymal stem cells, the media for cell culture described in "Soshiki Baiyou No Gijyutsu, Kiso-hen, 3rd edition", Asakura Shoten (1996) and the like can be mentioned; media for cell culture such as α-MEM, DMEM, and IMDM, supplemented with 1 to 20% of a serum such as bovine or human serum, are preferably used. Although the culture conditions may be any conditions that allow cultivation of the cells, culturing temperature is preferably 33 to 37°C, and the cultivation is preferably performed in an incubator filled with 5 to 10% gaseous CO₂. Mesenchymal stem cells are preferably proliferated in adhesion to a plastic culture dish for ordinary tissue culture. When the cells have proliferated over the entire surface of the culture dish, the medium is removed, and a trypsin-EDTA solution is added to suspend the cells. The cells suspended are washed with PBS or a medium for cell culture, after which the cells are 2 fold to 20 fold diluted with a medium for cell culture, and sown to a new culture dish, whereby further subculture can be performed.

Whether a certain nucleic acid controls the proliferation of mesenchymal stem cells can be confirmed by, for example, introducing a nucleic acid, such as a micro-RNA or a micro-RNA precursor, of the present invention, or an antisense, siRNA or the like against a target gene of the micro-RNA, into the mesenchymal stem cells, and comparing the degree of cell proliferation with that of a negative control. A method of measuring the degree of cell proliferation can be performed by the method described in 14 below.

As a method of examining the influence on the process of differentiation from mesenchymal stem cells to osteoblasts, for example, confirmation is made as described below. Specifically, under conditions that induce differentiation from mesenchymal stem cells to osteoblasts, a nucleic acid, micro-RNA or micro-RNA precursor of the present invention, or an antisense, siRNA or the like against a target gene of the micro-RNA, is introduced into the mesenchymal stem cells, and the cells are cultured. Genes or proteins whose expression increased with the progression of differentiation into osteoblasts are analyzed, and compared with negative control.

As a method of inducing differentiation from mesenchymal stem cells to osteoblasts, any method can be used, as far as it enables induction of differentiation from mesenchymal stem cells to osteoblasts; for example, a method described in Science, 284, 143-147 (1999) can be mentioned. Specifically, by sowing mesenchymal stem cells to an incubator, and thereafter continuing to culture the cells in a medium for cell culture containing dexamethasone, ascorbic acid-diphosphate, and β-glycerophosphate for 1 to 4 weeks, mesenchymal stem cells can be differentiated into osteoblasts.

As quantitative analytical methods for genes whose expression increases as a result of differentiation into osteoblasts, analysis by RT-PCR (reverse transcription-polymerase chain reaction), Northern blot analysis, dot blot hybridization, DNA microarray and the like can be mentioned.
As quantitative analytical methods for proteins whose expression increases as a result of differentiation into osteoblasts, Western blot analysis, immunohistological staining, ELISA and the like using an antibody that specifically reacts on the protein can be mentioned.

As genes or proteins whose expression increases as a result of differentiation into osteoblasts, type I collagen, osteocalcin, osteonectin, osteopontin, bone sialoprotein, Runx2 (runt-related gene 2), alkaline phosphatase (ALP) and the like can be mentioned.
As methods of evaluating the degree of differentiation into osteoblasts, staining cells by means of the ALP enzyme activity in the osteoblasts, and a method wherein ALP enzyme activity is measured can be mentioned. More specifically, as a method of such cell staining, a method can be mentioned wherein the alcohol moiety of the phosphoric acid ester of the substrate hydrolyzed by ALP enzyme in osteoblasts is coupled with a diazonium salt, and precipitated with azo dye in the enzyme activity portion. As an example of the substrate, Naphthol AS-MX phosphate can be mentioned; as an example of the azo dye, Fast Violet Blue can be mentioned. A kit comprising the same, for example, leukocyte alkaline phosphatase (manufactured by Sigma) and the like may be used. As kits for measuring ALP enzyme activity, for example, Alkaline Phospha B-Test Wako (manufactured by Wako Pure Chemical Industries, Ltd.) and the like may be used.

Furthermore, by detecting a calcified component produced by osteoblasts, differentiation into osteoblasts can also be confirmed. As methods of detecting a calcified component, staining methods such as von Kossa staining and Alizarin Red staining can be mentioned.
Von Kossa staining is a method of detecting calcium phosphate, a calcified component, using silver nitrate. Specifically, a 1 to 5% aqueous solution of silver nitrate is reacted with cells fixed with paraffin and the like and exposed to light, and the portion that develops a black color in which calcium phosphate is present is quantified by, for example, measuring the area where the color is developed, whereby the degree of differentiation into osteoblasts can be evaluated.

Alizarin Red staining is a method based on the fact that Alizarin Red S exhibits specific binding to calcium to form a lake. Specifically, 0.01 to 5% Alizarin Red S solution is reacted with cells fixed with paraffin and the like, and the portion that develops a red-purple to orange-red color is quantified by, for example, measuring the area where the color is developed, whereby the degree of differentiation into osteoblasts can be evaluated.

### 14. Method of measuring proliferation of cancer cells and other cells

The method of measuring cell proliferation is not particularly limited, as far as it enables a measurement of an index that reflects cell count or cell proliferation rate. Viable cell counting, DNA synthesis rate measurements, total protein content measurements and the like can be used.

As a method of evaluating viable cell counts, a method wherein the ATP content in cells is measured can be mentioned. It is known that the ATP content in cells is proportional to the number of cells in culture (J. Immunol. Meth. 160, 81-88 (1993)). As more specific methods of measuring the ATP content in cells, the MTT method, the XTT method and the like can be mentioned (J. Immunol. Meth. 65, 55-63 (1983)). A method can also be mentioned wherein ATP content is measured by luminescence of a luciferin substrate by the ATP-dependent enzyme luciferase. As a kit for measuring the ATP content in cells, for example, Cell Titer-Glo^{R} Luminescent Cell viability Assay (manufactured by Promega) and the like may be used.

### 15. Methods of measuring the degree of cell death of cancer cells and other cells

As methods of measuring the degree of cell death, a method wherein dead cells are stained with a dye such as Propium Iodide, a method wherein the activity of an enzyme leaked extracellularly as a result of cell death is measured, and the like can be used. For the latter, for example, a method wherein the enzyme activity of extracellularly leaked adenylate kinase is measured can be utilized. More specifically, ToxiLight^{®} Non-Destructive Cytotoxicity BioAssay Kit (manufactured by Lonza) and the like may be used.

It is also possible to measure the degree exclusively of apoptosis (programmed cell death), which is important in relation to cancers, out of the various types of cell death.
As methods of evaluating cell apoptosis, a method wherein the degree of DNA fragmentation is measured, a method wherein changes in cell membrane constituent lipids are measured, and a method wherein the activity of caspase 3/7, an intracellular protease induced upon apoptosis, is measured, can be mentioned. As a more specific method of measuring caspase 3/7 activity in cells, a method wherein a luciferin substrate liberated by caspase 3/7 activity is measured by luciferin luminescence by a luciferase enzyme reaction can be mentioned. As kits for measuring caspase 3/7 activity in cells, for example, Caspase-Glo^{R} 3/7 Assay (manufactured by Promega) and the like may be used.

Hereinafter, the present invention is described specifically by means of the following examples.

### [Example 1]

### Extraction of micro-RNAs expressed in human mast cells

### (1) RNA extraction

LAD2 is a recently established human mast cell line, and is known to well retain the nature of human mast cells [Leuk. Res., 27, 671 (2003); Leuk. Res., 27, 677 (2003)]. Hence, extraction of micro-RNAs from LAD2 was performed. LAD2 was obtained from the National Institute of Allergy and Infectious Diseases, National Institutes of Health (Bethesda, MD 20892-1881, USA), and cultured with a Stem Pro-34 medium [manufactured by Invitrogen] containing 100 ng/mL SCF in a 37°C 5% CO₂ concentration incubator.

### (2) Cloning of low-molecular RNAs

Using 200 µg of the LAD2-derived total RNA acquired in (1) above, and according to the method of Lau et al. (Science 294, 858-862, 2001), excision for low-molecular RNAs by means of 15% polyacrylamide gel electrophoresis, 5'-adenylated 3'-adapter ligation, 5'-adapter ligation, reverse transcription, PCR amplification, concatemerization, and ligation to the pCR2.1-TOPO vector were performed sequentially, to achieve cloning of the low-molecular RNAs. Next, the nucleotide sequences of the low-molecular RNAs cloned were determined. The 5'-adenylated 3'-adapter used was miRNA Cloning Linker, manufactured by Integrated DNA Technologies.

Separately from the above-described method, the nucleotide sequence was also determined by performing separation and cutting out of a low-molecular RNA by 15% polyacrylamide gel electrophoresis, 5' terminal dephosphorylation, 3'-adapter ligation, phosphorylation, 5'-adapter ligation, reverse transcription, PCR amplification, and ligation to a microbead vector sequentially to achieve cloning of the low-molecular RNA, and reading the nucleotide sequence of the microbead, using 200 µg of the LAD2-derived total RNA acquired in (1) above, according to the method of Mineno et al. [Nucleic Acids Research, 34, 1765-1771, (2006)].

### [Example 2]

### Identification of micro-RNAs

From among the low-molecular RNAs obtained in Example 1, first, those whose nucleotide sequences did not agree with any one in miRBase (http://microrna.sanger.ac.uk/), which is a database for known micro-RNAs, were selected. Surrounding genome sequences wherein DNA sequences corresponding to those nucleotide sequences were extended by about 50 nt toward the 5' side and the 3' side, respectively, were acquired from UCSC Genome Bioinformatics (http://genome.ucsc.edu/), and the secondary structures of the RNAs expected to be transcribed from the genome sequences were predicted using RNAfold. As a result, 1336 types were found to be novel micro-RNAs located in one chain of the hairpin structure. The nucleotide sequences thereof and the nucleotide sequences of micro-RNA precursors comprising these micro-RNAs are shown in Tables 1. The micro-RNAs having the respective nucleotide sequences were given the names KHK_miR_1001 to 2344 (Table 1). If one micro-RNA can assume hairpin structures derived from genome sequences at different positions, all thereof are shown.

As an example secondary structure, the hairpin structure of KHK_miR_1194 is shown in FIG. 1.

### [Example 3]

### Detection of functions of micro-RNAs

By determining whether or not a micro-RNA obtained in Example 2 undergoes processing by Dicer protein, whether or not the same functions as a micro-RNA can be determined.
Of the micro-RNAs obtained in Example 2, the micro-RNA of KHK_miR_1194 can have a function thereof detected as described below. First, a single-stranded RNA having the nucleotide sequence of SEQ ID NO:1580 is synthesized, and reacted with the Dicer Enzyme attached to the X-treme GENE siRNA Dicer Kit (manufactured by Roche-Applied Science). Next, the reaction product is electrophoresed with 15% polyacrylamide gel; detection of a band 20 to 25 nucleotides in size indicates that the possession of a function as a micro-RNA.

### [Example 4]

### Action on degranulation of human mast cells with micro-RNA expressed forcibly therein

Each micro-RNA precursor obtained in Example 2 was introduced to LAD2, a human mast cell line, to induce degranulation, and the influence of the micro-RNA precursor was examined.
The LAD2 was cultured with a Stem Pro-34 medium (manufactured by Invitrogen) containing 100 ng/mL SCF.
The LAD2 was sown to a 6-well plate at about 5x10⁵ cells per well, and a micro-RNA precursor was introduced using a lipofection method, specifically, Gene Silencer (manufactured by Genlantis), to obtain a final concentration of 30 nM. The micro-RNA (hereinafter also referred to as miRNA) precursors used were KHK_miR_1001, 1002, 1003, 1008, 1014, 1020, 1021, 1022, 1025, 1032, and 1036, synthesized as Pre-miR^{™} miRNA Precursor Molecules by Ambion. These were chemically synthesized double-stranded nucleic acid molecules, designed to allow nucleic acids consisting of the nucleotide sequences of SEQ ID NOs:1, 2, 3, 8, 14, 20, 21, 22, 25, 32, and 36, respectively, to be incorporated by a complex similar to RISC, which is a factor for the activity of an miRNA, to exhibit the same function as the miRNA. For a sequence corresponding to the human genome sequence of SEQ ID NO:1, a nucleic acid consisting of a sequence having 1 nucleotide on the 5' side and 4 nucleotides on the 3' side deleted from SEQ ID NO:1 (SEQ ID NO:2852) was provided, and KHK_miR_1001_2, synthesized as Pre-miR^{™} miRNA Precursor Molecule by Ambion, was also used. For negative control, Pre-miR^{™} miRNA Precursor Molecules-Negative Control #2 (hereinafter referred to as miR-negacon #2) (manufactured by Ambion) was introduced into the LAD2 in the same manner. Lipofection was performed per the directions attached to the product.

Two days after introduction of the micro-RNA precursor by a lipofection method, 1 µg/mL human myeloma IgE (manufactured by Cosmo Bio Co., Ltd.) was added, and the cells were cultured in a 37°C 5% CO₂ concentration incubator overnight. On the day that followed, the medium was removed via centrifugation, and the plate was washed with a Tyrode buffer solution (126.1 mmol/L NaCl, 4.0 mmol/L KCl, 1.0 mmol/L CaCl₂, 0.6 mmol/L MgCl₂, 0.6 mmol/L KH₂PO₄, 10 mM HEPES, 5.6 mmol/L D-glucose, 0.1% bovine serum albumin, pH 7.4), after which 3.9 mL of the Tyrode buffer solution was added to suspend the cells, and the suspension was dispensed to a 96-well plate at 100 µL per well. Next, a rabbit anti-human IgE antibody (manufactured by DAKO) was added to obtain a final concentration of 10 µg/mL, and this was followed by incubation in a 37°C 5% CO₂ concentration incubator for 20 minutes to induce degranulation. The supernatant was recovered via centrifugation, and the β-hexosaminidase activity in the supernatant was measured, whereby the degree of degranulation was determined. The β-hexosaminidase activity was measured by adding 50 µL of 4 mmol/L p-nitrophenyl N-acetyl-β-glucosaminide (manufactured by Sigma) dissolved in 40 mmol/L citrate buffer solution (pH 4.5) to 50 µL of the supernatant recovered, and incubating the mixture at 37°C for 1 hour, thereafter adding 100 µL of 0.2 mol/L glycine (pH 10.7), and measuring the absorbance of the sample at 405 nm using the plate reader 1420 ARVOsx (manufactured by Perkin Elmer). Also, by performing the same experiment but with the addition of Triton X-100 at a final concentration of 1% in place of the rabbit anti-human IgE antibody, the total β-hexosaminidase activity in LAD2 was measured. The ratio of degranulation was calculated as the ratio (%) of the β-hexosaminidase activity in the supernatant relative to total β-hexosaminidase activity; taking the degranulation ratio of a control plot (Gene Silencer only) as 1.0, the relative degranulation activity of each was calculated. The results are shown in Table 3.

**[Table 3]**

| introduced micro-RNA precursor | relative degranulation activity |
|---|---|
| KHK_miR_1001 | 1.34 |
| KHK_miR_1001_2 | 1.25 |
| KHK_miR_1002 | 1.80 |
| KHK_miR_1003 | 1.42 |
| KHK_miR_1008 | 1.33 |
| KHK_miR_1014 | 1.38 |
| KHK_miR_1020 | 1.31 |
| KHK_miR_1022 | 1.25 |
| KHK_miR_1025 | 1.24 |
| KHK_miR_1032 | 1.53 |
| KHK_miR_1036 | 1.43 |
| miR-negacon#2 | 1.01 |

As a result, it was found that with introduction of KHK_miR_1001, 1001_2, 1002, 1003, 1008, 1014, 1020, 1022, 1025, 1032, or 1036, the degranulation of LAD2 stimulated by IgE receptor is promoted.

### [Example 5]

### Proliferation activity, osteoblast differentiation, and viable cell count of mesenchymal stem cells with micro-RNAs expressed forcibly therein

Each micro-RNA precursor obtained in Example 2 was introduced into human mesenchymal stem cells (hereinafter also referred to as hMSCs), and the effects of the micro-RNA precursors on the proliferation and osteoblast differentiation were examined.
The human mesenchymal stem cells were obtained from Cambrex, and cultured with an IMDM medium (manufactured by Invitrogen) containing 20% fetal bovine serum (FBS) (manufactured by JRH Bioscience) in a 37°C 5% CO₂ concentration incubator. The hMSCs were sown to a 24-well plate at about 6.2x10³ cells per well, and cultured with the IMDM medium containing 20% FBS overnight. One day later, the micro-RNA precursor was introduced into the hMSCs using a lipofection method, specifically, Lipofectamine 2000 (manufactured by Invitrogen), to obtain a final concentration of 20 nM. The micro-RNA precursors used were KHK_miR_1001, 1001_2, 1002, 1003, 1008, 1014, 1020, 1021, 1022, 1025, 1032, and 1036, synthesized as Pre-miR^{™} miRNA Precursor Molecules by Ambion. Lipofection was performed per the directions attached to the product.

On the day after introduction of the micro-RNA precursor by the lipofection method, the medium was replaced with an osteoblast differentiation induction medium [an IMDM medium containing 20% FBS, supplemented with 0.1 µmol/L dexamethasone, 50 µmol/L ascorbic acid-diphosphoric acid (manufactured by Sigma), and 10 mmol/L β-glycerophosphite (manufactured by Sigma)], and cultivation was continued with renewal of the osteoblast differentiation induction medium at a frequency of once per 3 days.
Two weeks after the start of the cultivation, cell morphology was examined under a phase-contrast microscope (manufactured by Nikon), and alkaline phosphatase staining was performed, to detect osteoblasts. Specifically, first, the cells were once washed with phosphate buffer solution (hereinafter also referred to as PBS (phosphate-buffered saline)) (manufactured by Invitrogen), and fixed with a fixative solution (10% formalin/PBS) for 5 minutes. The cells were washed with distilled water, and thereafter reacted with a mixed solution of Naphthol AS-MX phosphate (manufactured by Sigma) and Fast Violet B solution (manufactured by Sigma) in the dark for 30 minutes to cause an alkaline phosphatase reaction. Furthermore, the cells were washed with distilled water, and osteoblasts stained red were examined under a phase contrast microscope and photographed using a digital camera (manufactured by Nikon).

As a result, it was found that the hMSCs incorporating Pre-miR^{™} miRNA Precursor Molecules of KHK_miR_1008, 1021, or 1036 had fewer cells than the hMSCs not incorporating any micro-RNA precursor, and also had fewer positive cells stained with alkaline phosphatase. Micro-RNA precursors are converted to micro-RNAs in cells; hence, it was found that the micro-RNAs derived from these precursors exhibit a suppressive activity on the proliferation of hMSCs and suppressive activity on the differentiation thereof into osteoblasts. Conversely, it was found that the hMSCs incorporating Pre-miR^{™} miRNA Precursor Molecules of KHK_miR_1001 or 1001_2 had a larger number of positive cells stained with alkaline phosphatase than the hMSCs not incorporating any micro-RNA precursor. From this, it was found that the micro-RNAs derived from these precursors exhibit a promotive activity on the differentiation of hMSCs into osteoblasts.

Four days after introduction of each micro-RNA precursor by the lipofection method, viable cell ratios were measured using CellTiter-Glo^{™} Luminescent Cell Viability Assay (manufactured by Promega). The results are shown in Table 4. As shown in Table 4, it was found that with introduction of KHK_miR_1001, the viable cell ratio increased than usual.

**[Table 4]**

| introduced micro-RNA precursor | viable cell ratio |
|---|---|
| KHK_miR_1001 | 1.28 |
| miR_negacon#2 | 1.00 |

### [Example 6]

### Viable cell ratio and apoptotic activity in colon cancer-derived cell line with micro-RNA expressed forcibly therein

Each micro-RNA precursor obtained in Example 2 was introduced to a colon cancer-derived cell line, and the effects of the micro-RNA precursor on viable cell ratio and apoptotic activity were examined.

The DLD-1 human colorectal cancer-derived cell line (hereinafter to be sometimes referred to as DLD-1) was obtained from the American Type Culture Collection (ATCC) (hereinafter referred to as ATCC) (ATCC CCL-221). DLD-1 was cultured with an RPMI1640 medium (manufactured by Invitrogen) containing 10% fetal bovine serum (FBS) (manufactured by JRH Biosciences) in a 37°C 5% CO₂ concentration incubator.
DLD-1 was sown to a 96-well plate at about 2500 cells per well, and cultured in an RPMI medium containing 10% FBS overnight. After 1 day, a micro-RNA precursor was introduced to the DLD-1 using a lipofection method, specifically Lipofectamine 2000 (manufactured by Invitrogen), to obtain a final concentration of 5 nM or 25 nM. The micro-RNA precursors used were KHK_miR_1001, 1001_2, 1002, 1003, 1008, 1014, 1020, 1021, 1022, 1025, 1032, and 1036, synthesized as Pre-miR^{™} miRNA Precursor Molecules by Ambion. In addition, miR-negacon #2 (manufactured by Ambion) was also introduced to DLD-1, and this was used as the negative control. Lipofection was performed per the directions attached to the product.

Three days after introduction of the micro-RNA precursor by a lipofection method, viable cell ratios were measured using CellTiter-Glo^{™} Luminescent Cell Viability Assay (manufactured by Promega). Taking the viable cell ratio of DLD-1 in a control plot (Lipofectamine 2000 only) as 1.0, the relative viable cell ratio of each was calculated. As a result, as shown in Table 5, with introduction of KHK_miR_1001, 1001_2, 1032, or 1036, a decrease of 40% or more in viable cell ratio was observed.

**[Table 5]**

| introduced micro-RNA precursor | viable cell ratio (5 nM) | viable cell ratio (25 nM) |
|---|---|---|
| KHK_miR_1001 | 0.23 | 0.11 |
| KHK_miR_1001_2 | 0.32 | 0.19 |
| KHK_miR_1032 | 0.58 | 0.59 |
| KHK_miR_1036 | 0.70 | 0.46 |
| miR_negacon#2 | 0.87 | 0.87 |

Two days after introduction of the micro-RNA precursor by a lipofection method, caspase 3/7 activity was measured using Caspase-Glo^{R} 3/7 assay (manufactured by Promega) per the directions attached to the product. Taking the caspase 3/7 activity value of DLD-1 in a control plot (Lipofectamine 2000 only) as 1.0, the relative caspase 3/7 activity value of each was calculated. The results are shown in Table 6. As shown in Table 6, with introduction of KHK_miR_1001, 1001_2, or 1036, an increase of 50% or more in caspase 3/7 activity was observed.

**[Table 6]**

| introduced micro-RNA precursor | caspase 3/7 activity |
|---|---|
| KHK_miR_1036 | 2.50 |
| KHK_miR_1001 | 2.07 |
| KHK_miR_1001_2 | 1.84 |
| miR_negacon#2 | 0.85 |

### [Example 7]

### Viable cell ratio in ovarian cancer-derived cell line with micro-RNA expressed forcibly therein

Each micro-RNA precursor obtained in Example 2 was introduced to an ovarian cancer-derived cell line, and the effects of the micro-RNA precursor on viable cell ratio was examined.
The A2780 human ovarian cancer-derived cell line (Nature, 295, 116-119 (1982); Science, 224, 994-996 (1984); Semin. Oncol., 11, 285-298 (1984); hereinafter to be referred to as A2780) was cultured with an RPMI1640 medium (manufactured by Invitrogen) containing 5% FBS (manufactured by JRH Biosciences) in a 37°C 5% CO₂ concentration incubator.

A2780 was sown to a 96-well plate at about 2500 cells per well, and cultured with an RPMI medium containing 10% FBS overnight. After 1 day, the micro-RNA precursor was introduced to A2780 using a lipofection method, specifically Lipofectamine 2000 (manufactured by Invitrogen), to obtain a final concentration of 5 nM or 25 nM. The micro-RNA precursors used were KHK_miR_1001, 1001_2, 1002, 1003, 1008, 1014, 1020, 1021, 1022, 1025, 1032, and 1036, synthesized as Pre-miR^{™} miRNA Precursor Molecules by Ambion. miR-negacon #2 (manufactured by Ambion) was also introduced into A2780, and this was used for negative control.

Three days after introduction of the micro-RNA precursor by a lipofection method, viable cell ratios were measured using CellTiter-Glo^{™} Luminescent Cell Viability Assay (manufactured by Promega). Taking the viable cell ratio of A2780 in a control plot (Lipofectamine 2000 only) as 1.0, the relative viable cell ratio of each was calculated. The results are shown in Table 7. As shown in Table 7, with introduction of KHK_miR_1003, 1008, 1020, 1021, 1022, 1032, or 1036, a decrease of 40% or more in viable cell ratio was observed.

**[Table 7]**

| introduced micro-RNA precursor | viable cell ratio (5 nM) | viable cell ratio (25 nM) |
|---|---|---|
| KHK_miR_1032 | 0.36 | 0.43 |
| KHK_miR_1003 | 0.41 | 0.45 |
| KHK_miR_1008 | 0.43 | 0.43 |
| KHK__miR_1036 | 0.44 | 0.36 |
| KHK__miR_1021 | 0.46 | 0.64 |
| KHK_miR_1020 | 0.48 | 0.57 |
| KHK_miR_1022 | 0.52 | 0.52 |
| miR_negacon#2 | 1.00 | 1.05 |

### Industrial Applicability

The present invention provides a nucleic acid such as a micro-RNA or a micro-RNA precursor, having a novel sequence. The nucleic acid of the present invention makes it possible to detect the expression or a mutation of a micro-RNA, to separate cells, to suppress the expression of a target sequence gene, to screen for a substance that promotes or suppresses a function of a micro-RNA, and to diagnose or treat a disease caused by a mast cell abnormality, a disease caused by an abnormality of mesenchymal stem cell proliferation or differentiation, cancers, and a disease caused by abnormal proliferation of cells, tissue hyperplasia or the like.

## Claims

1. A nucleic acid consisting of the nucleotide sequence of any one of SEQ ID NOs:1 to 1336.

2. A nucleic acid consisting of a nucleotide sequence having an identity of 90% or more to the nucleotide sequence of any one of SEQ ID NOs:1 to 1336.

3. A nucleic acid that hybridizes under stringent conditions with a strand complementary to a nucleic acid consisting of the nucleotide sequence of any one of SEQ ID NOs:1 to 1336.

4. A nucleic acid comprising the nucleic acid described in any one of claims 1 to 3.

5. A nucleic acid consisting of the nucleotide sequence of any one of SEQ ID NOs:1337 to 2851.

6. A nucleic acid consisting of a nucleotide sequence having an identity of 80% or more to the nucleotide sequence of any one of SEQ ID NOs:1337 to 2851.

7. A nucleic acid that hybridizes under stringent conditions with a strand complementary to a nucleic acid consisting of the nucleotide sequence of any one of SEQ ID NOs:1337 to 2851.

8. A nucleic acid consisting of a nucleotide sequence complementary to the nucleic acid described in any one of claims 1 to 7.

9. A double-stranded nucleic acid consisting of the nucleic acid described in any one of claims 1 to 7 and a nucleic acid consisting of a nucleotide sequence complementary to the nucleotide sequence of the nucleic acid.

10. A vector which expresses the nucleic acid described in any one of claims 1 to 9.

11. A method of detecting the expression or mutation of the nucleic acid described in any one of claims 1 to 9, comprising using the nucleic acid described in any one of claims 1 to 9.

12. A method for screening a substance that promotes or suppresses the expression or function of the nucleic acid described in any one of claims 1 to 9, comprising using the nucleic acid described in any one of claims 1 to 9.

13. A method of separating a cell that expresses the nucleic acid described in any one of claims 1 to 9, comprising using the nucleic acid described in any one of claims 1 to 9.

14. A method of suppressing the expression of a target gene of the nucleic acid described in any one of claims 1 to 9, comprising using the nucleic acid described in any one of claims 1 to 9.

15. A diagnostic reagent or a therapeutic agent for a disease caused by a mast cell abnormality, comprising the nucleic acid described in any one of claims 1 to 9 as an active ingredient.

16. A diagnostic reagent or a therapeutic agent for a disease caused by a mast cell abnormality, comprising a substance that promotes or suppresses the expression or function of the nucleic acid described in any one of claims 1 to 9 as an active ingredient.

17. A method for screening a diagnostic reagent or a therapeutic agent for a disease caused by a mast cell abnormality, wherein promotion or suppression of the expression or function of the nucleic acid described in any one of claims 1 to 9 serves as an index.

18. A diagnostic reagent or a therapeutic agent for a disease caused by a mast cell abnormality, comprising a substance that suppresses the expression of a target gene of the nucleic acid described in any one of claims 1 to 9 as an active ingredient.

19. A method for screening a diagnostic reagent or a therapeutic agent for a disease caused by a mast cell abnormality, wherein suppression of the expression of a target gene of the nucleic acid described in any one of claims 1 to 9 serves as an index.

20. A cell incorporating the nucleic acid or vector described in any one of claims 1 to 10.

21. A mast cell degranulation promoter comprising the nucleic acid described in any one of claims 1 to 4 wherein the SEQ ID NO: is any one of 1, 2, 3, 8, 14, 20, 22, 25, 32 and 36 as an active ingredient.

22. A mast cell degranulation promoter comprising the nucleic acid described in any one of claims 5 to 7 wherein the SEQ ID NO: is any one of 1337, 1338, 1339, 1352, 1363, 1371, 1373, 1377, 1386 and 1390 as an active ingredient.

23. A mast cell degranulation suppressant comprising a nucleic acid consisting of a nucleotide sequence complementary to the nucleic acid of claim 21 or 22 as an active ingredient.

24. A mast cell degranulation promoter or degranulation suppressant comprising a double-stranded nucleic acid consisting of the nucleic acid described in claim 21 or 22 and a nucleic acid consisting of a nucleotide sequence complementary to the nucleotide sequence of the nucleic acid, as an active ingredient.

25. A mast cell degranulation promoter or degranulation suppressant comprising a substance that promotes or suppresses the expression or function of the nucleic acid described in claim 21 or 22 as an active ingredient.

26. A method for screening a mast cell degranulation promoter or degranulation suppressant, wherein promotion or suppression of the expression or function of the nucleic acid described in claim 21 or 22 serves as an index.

27. A mast cell degranulation promoter or degranulation suppressant comprising a substance that suppresses or promotes the expression of a target gene of the nucleic acid of claim 21 or 22 as an active ingredient.

28. A method for screening a mast cell degranulation promoter or degranulation suppressant, wherein suppression or promotion of the expression of a target gene of the nucleic acid described in claim 21 or 22 serves as an index.

29. A diagnostic reagent or a therapeutic agent for a disease caused by an abnormality of mesenchymal stem cell proliferation and/or differentiation, comprising the nucleic acid described in any one of claims 1 to 4 wherein the SEQ ID NO: is any one of 1, 8, 21 and 36, as an active ingredient.

30. A diagnostic reagent or a therapeutic agent for a disease caused by an abnormality of mesenchymal stem cell proliferation and/or differentiation, comprising the nucleic acid described in any one of claims 5 to 7 wherein the SEQ ID NO: is any one of 1337, 1352, 1372 and 1390, as an active ingredient.

31. A diagnostic reagent or a therapeutic agent for a disease caused by an abnormality of mesenchymal stem cell proliferation and/or differentiation, comprising a nucleic acid consisting of a nucleotide sequence complementary to the nucleic acid described in claim 29 or 30 as an active ingredient.

32. A diagnostic reagent or a therapeutic agent for a disease caused by an abnormality of mesenchymal stem cell proliferation and/or differentiation, comprising a double-stranded nucleic acid consisting of the nucleic acid described in claim 29 or 30 and a nucleic acid consisting of a nucleotide sequence complementary to the nucleotide sequence of the nucleic acid as an active ingredient.

33. A diagnostic reagent or a therapeutic agent for a disease caused by an abnormality of mesenchymal stem cell proliferation and/or differentiation, comprising a substance that promotes or suppresses the expression or function of the nucleic acid described in claim 29 or 30 as an active ingredient.

34. A method for screening a diagnostic reagent or a therapeutic agent for a disease caused by an abnormality of mesenchymal stem cell proliferation and/or differentiation, wherein promotion or suppression of the expression or function of the nucleic acid described in claim 29 or 30 serves as an index.

35. A diagnostic reagent or a therapeutic agent for a disease caused by an abnormality of mesenchymal stem cell proliferation and/or differentiation, comprising a substance that suppresses or promotes the expression of a target gene of the nucleic acid described in claim 29 or 30 as an active ingredient.

36. A method for screening a diagnostic reagent or a therapeutic agent for a disease caused by an abnormality of mesenchymal stem cell proliferation and/or differentiation, wherein suppression or promotion of the expression of a target gene of the nucleic acid described in claim 29 or 30 serves as an index.

37. A mesenchymal stem cell proliferation promoter comprising the nucleic acid described in any one of claims 1 to 4 wherein the SEQ ID NO: is 1, as an active ingredient.

38. A mesenchymal stem cell proliferation promoter comprising the nucleic acid described in any one of claims 5 to 7 wherein the SEQ ID NO: is 1337, as an active ingredient.

39. A mesenchymal stem cell proliferation suppressant comprising a nucleic acid consisting of a nucleotide sequence complementary to the nucleic acid described in claim 37 or 38 as an active ingredient.

40. A mesenchymal stem cell proliferation suppressant comprising the nucleic acid described in any one of claims 1 to 4 wherein the SEQ ID NO: is any one of 8, 21 and 36, as an active ingredient.

41. A mesenchymal stem cell proliferation suppressant comprising the nucleic acid described in any one of claims 5 to 7 wherein the SEQ ID NO: is any one of 1352, 1372 and 1390, as an active ingredient.

42. A mesenchymal stem cell proliferation promoter comprising a nucleic acid consisting of a nucleotide sequence complementary to the nucleic acid described in claim 37 or 38 as an active ingredient.

43. A mesenchymal stem cell proliferation promoter or proliferation suppressant comprising a double-stranded nucleic acid consisting of the nucleic acid described in any one of claims 37 to 42 and a nucleic acid consisting of a nucleotide sequence complementary to the nucleotide sequence of the nucleic acid as an active ingredient.

44. A mesenchymal stem cell proliferation promoter or proliferation suppressant comprising a substance that promotes or suppresses the expression or function of the nucleic acid described in any one of claims 37 to 42 as an active ingredient.

45. A method for screening a mesenchymal stem cell proliferation promoter or proliferation suppressant, wherein promotion or suppression of the expression or function of the nucleic acid described in any one of claims 37 to 42 serves as an index.

46. A mesenchymal stem cell proliferation promoter or proliferation suppressant comprising a substance that suppresses or promotes the expression of a target gene of the nucleic acid described in any one of claims 37 to 42 as an active ingredient.

47. A method for screening a mesenchymal stem cell proliferation promoter or proliferation suppressant, wherein suppression or promotion of the expression of a target gene of the nucleic acid described in any one of claims 37 to 42 serves as an index.

48. A diagnostic reagent or a therapeutic agent for a disease caused by a cell proliferation abnormality, comprising the nucleic acid described in any one of claims 1 to 4 wherein the SEQ ID NO: is any one of 1, 3, 8, 20, 21, 22, 32 and 36, as an active ingredient.

49. A diagnostic reagent or a therapeutic agent for a disease caused by a cell proliferation abnormality, comprising the nucleic acid described in any one of claims 5 to 7 wherein the SEQ ID NO: is any one of 1337, 1339, 1352, 1371, 1372, 1373, 1386 and 1390, as an active ingredient.

50. A diagnostic reagent or a therapeutic agent for a disease caused by a cell proliferation abnormality, comprising a nucleic acid consisting of a nucleotide sequence complementary to the nucleic acid described in claim 48 or 49 as an active ingredient.

51. A diagnostic reagent or a therapeutic agent for a disease caused by a cell proliferation abnormality, comprising a double-stranded nucleic acid consisting of the nucleic acid described in claim 48 or 49 and a nucleic acid consisting of a nucleotide sequence complementary to the nucleic acid as an active ingredient.

52. A diagnostic reagent or a therapeutic agent for a disease caused by a cell proliferation abnormality, comprising a substance that promotes or suppresses the expression or function of the nucleic acid described in claim 48 or 49 as an active ingredient.

53. A method for screening a diagnostic reagent or a therapeutic agent for a disease caused by a cell proliferation abnormality, wherein promotion or suppression of the expression or function of the nucleic acid described in claim 48 or 49 serves as an index.

54. A diagnostic reagent or a therapeutic agent for a disease caused by a cell proliferation abnormality, comprising a substance that suppresses or promotes the expression of a target gene of the nucleic acid described in claim 48 or 49 as an active ingredient.

55. A method for screening a diagnostic reagent or a therapeutic agent for a disease caused by a cell proliferation abnormality, wherein suppression or promotion of the expression of a target gene of the nucleic acid described in claim 48 or 49 serves as an index.

56. The diagnostic reagent, the therapeutic agent or the screening method described in any one of claims 48 to 55, wherein the disease caused by a cell proliferation abnormality is a disease selected from the group consisting of cancers, arteriosclerosis, rheumatoid arthritis, prostatic hyperplasia, blood vessel restenosis after percutaneous transvascular coronary angioplasty, fibroid lung, glomerulonephritis and autoimmune diseases.

57. A cell proliferation suppressant comprising the nucleic acid described in any one of claims 1 to 4 wherein the SEQ ID NO: is any one of 1, 3, 8, 20, 21, 22, 32 and 36, as an active ingredient.

58. A cell proliferation suppressant comprising the nucleic acid described in any one of claims 5 to 7 wherein the SEQ ID NO: is any one of 1337, 1339, 1352, 1371, 1372, 1373, 1386 and 1390, as an active ingredient.

59. A cell proliferation promoter comprising a nucleic acid consisting of a nucleotide sequence complementary to the nucleic acid described in claim 57 or 58 as an active ingredient.

60. A cell proliferation suppressant or proliferation promoter comprising a double-stranded nucleic acid consisting of the nucleic acid described in claim 57 or 58 and a nucleic acid consisting of a nucleotide sequence complementary to the nucleotide sequence of the nucleic acid as an active ingredient.

61. A cell proliferation suppressant or proliferation promoter comprising a substance that promotes or suppresses the expression or function of the nucleic acid described in claim 57 or 58 as an active ingredient.

62. A method for screening a cell proliferation suppressant or proliferation promoter, wherein promotion or suppression of the expression or function of the nucleic acid described in claim 57 or 58 serves as an index.

63. A cell proliferation suppressant or proliferation promoter comprising a substance that suppresses or promotes the expression of a target gene of the nucleic acid described in claim 57 or 58 as an active ingredient.

64. A method for screening a cell proliferation suppressant or proliferation promoter, wherein suppression or promotion of the expression of a target gene of the nucleic acid described in claim 57 or 58 serves as an index.
